(19) Europäisches Patentamt
European Patent Office
Office européen des brevets

(11) EP 4 108 663 A1

(12) **EUROPEAN PATENT APPLICATION**
published in accordance with Art. 153(4) EPC

(43) Date of publication:
**28.12.2022 Bulletin 2022/52**

(21) Application number: **21757764.2**

(22) Date of filing: **19.02.2021**

(51) International Patent Classification (IPC):
***C07D 417/04*** $^{(2006.01)}$ ***C07D 417/14*** $^{(2006.01)}$
***C07D 277/28*** $^{(2006.01)}$ ***C07D 277/42*** $^{(2006.01)}$
***C07D 277/60*** $^{(2006.01)}$ ***C07D 513/04*** $^{(2006.01)}$
***C07D 471/10*** $^{(2006.01)}$ ***A61K 31/425*** $^{(2006.01)}$
***A61P 35/00*** $^{(2006.01)}$ ***A61P 37/00*** $^{(2006.01)}$
***A61P 25/00*** $^{(2006.01)}$

(52) Cooperative Patent Classification (CPC):
**A61K 31/425; A61P 25/00; A61P 35/00; A61P 37/00; C07D 277/28; C07D 277/42; C07D 277/60; C07D 417/04; C07D 417/14; C07D 471/10; C07D 513/04**

(86) International application number:
**PCT/CN2021/076939**

(87) International publication number:
**WO 2021/164746 (26.08.2021 Gazette 2021/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.02.2020 CN 202010102773**
**28.07.2020 CN 202010738095**

(71) Applicants:
- **Simcere Pharmaceutical Co., Ltd.**
  **Nanjing, Jiangsu 210032 (CN)**
- **Jiangsu Simcere Pharmaceutical Co., Ltd.**
  **Nanjing, Jiangsu 210042 (CN)**

(72) Inventors:
- **GU, Peng**
  **Shanghai 201321 (CN)**
- **LIU, Lei**
  **Shanghai 201321 (CN)**
- **ZHANG, Guobao**
  **Shanghai 201321 (CN)**
- **ZHAO, Chunyan**
  **Shanghai 201321 (CN)**
- **TANG, Renhong**
  **Shanghai 201321 (CN)**
- **REN, Jinsheng**
  **Nanjing, Jiangsu 210041 (CN)**

(74) Representative: **Grünecker Patent- und Rechtsanwälte**
**PartG mbB**
**Leopoldstraße 4**
**80802 München (DE)**

(54) **SUBSTITUTED ARYL COMPOUND**

(57) The present application describes a substituted aryl compound used as an RAD51 inhibitor and a pharmaceutically acceptable salt thereof. The compound has the structure as represented by Formula (I) and has the substituents and structural features as described in the present application. Furthermore, the present application describes a pharmaceutical composition containing the compound as represented by Formula (I) or the pharmaceutically acceptable salt thereof, and the use of the compound as represented by Formula (I) or the pharmaceutically acceptable salt thereof and the pharmaceutical composition containing same in the preparation of a drug for preventing or treating RAD51 mediated diseases.

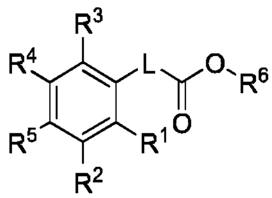

(I)

EP 4 108 663 A1

## Description

The present application claims the priority of two earlier applications, *i.e.,* Chinese Patent Application No. 202010102773.6 entitled "Substituted Aryl Compounds" and filed with the National Intellectual Property Administration, PRC on February 19, 2020, and Chinese Patent Application No. 202010738095.2 entitled "Substituted Aryl Compounds" and filed with the National Intellectual Property Administration, PRC on July 28, 2020, the disclosures of which are incorporated herein by reference in their entirety.

## TECHNICAL FIELD

The present disclosure relates to a novel substituted aryl compound or a pharmaceutically acceptable salt thereof, a pharmaceutical composition containing the same, and the use thereof as an RAD51 inhibitor.

## BACKGROUND

RAD51 is a eukaryotic gene. The protein encoded by this gene is a member of the RAD51 protein family and is capable of helping repair DNA double strand breaks. RAD51 family members are homologous to bacterial RecA, archaeal RadA, and yeast RAD51. From yeast to humans, this kind of protein is highly conserved in most eukaryotes. In humans, RAD51 is a protein consisting of 339 amino acids and has a DNA-dependent ATP kinase activity. It plays a major role in homologous recombination during the repair process of DNA double strand break (DSB). RAD51 is involved in the reciprocal translocation between a broken sequence and its undamaged homologous sequence to enable the re-synthesis of the damaged region.

DNA damage response plays a significant role in the maintenance of genome stability of cells and cell survival. DNA double strand breaks (DSBs) are the most severe form among DNA damages. Homologous recombination repair is one of the important mechanisms involved in the repair of DSB damage *in vivo,* in which RAD51 is a key factor involved in the DNA repair via homologous recombination *in vivo.* RAD51 is highly expressed in a variety of human tumor tissues such as the tumor tissues of breast cancer, non-small cell lung cancer, prostate cancer and the like, and is associated with the metastasis and aggravation of tumors (Klein et al., DNA Repair (Amst). 2008 May 3;7(5):686-693). How to effectively down-regulate the RAD51 level in tumor tissues and reduce the capacity of tumor cells to repair the damaged DNA and thus improve the efficacy of tumor therapy has potential value in clinical applications.

Homologous recombination has multiple roles in the repair of DNA damage, including the repair of DNA double strand breaks and the recovery from the damage of the block of DNA replication caused by DNA crosslinking agents. Homologous recombination repairs DNA double strand breaks by locating a homologous fragment of DNA and replicating the missing genetic information from a homologous template. Numerous studies have shown that homologous recombination is essential in the maintenance of genome stability. Studies have shown that the defects in the proteins that promote homologous recombination repair in cells are associated with the sensitivity to therapies for certain DNA damages. Such sensitization effect is particularly pronounced in terms of DNA crosslinking chemotherapeutics and ionizing radiation (Takata et al., Mol Cell Biol. 2001 Apr; 21(8):2858-2866; Godthelp et al., Nucleic Acids Res. 2002 May 15; 30(10):2172-2182).

A research groups has recently demonstrated that the sensitivity of cells to therapies for DNA damages may be further enhanced by the partial inhibition of homologous recombination. For example, a synthetic peptide corresponding to another paralogous protein may be used to inhibit XRCC3 (a paralogous protein of Rad51). This synthetic peptide enables Chinese hamster ovary (CHO) cells more sensitive to cisplatin and inhibits the formation of Rad51foci resulted from DNA damages (Connell et al., Cancer Res. 2004 May 1; 64(9):3002-3005).

Therefore, in view of the fact that the defects in proteins associated with DNA homologous recombination repair may increase the sensitivity of cells to therapies for DNA damages, there is a need to develop small molecules capable of inhibiting the activity of RAD51. Cyteir Therapeutics, Inc. has currently published two patent applications (WO2019014315A1 and WO2019051465A1) related to RAD51 inhibitors and has disclosed a series of RAD51 inhibitors with novel structures as well as the medical use thereof. In particular, these RAD51 inhibitors are useful in the treatment of conditions such as cancers, autoimmune diseases, immunodeficiency, or neurodegenerative diseases.

In spite of some progress currently has been achieved in the treatment of diseases such as tumors and autoimmune diseases, there are still a large number of patients in need of better and more effective therapeutic drugs and regimens in clinical practice. In view of the huge unmet clinical needs and the potential application value of RAD51 inhibitors in the area of diseases such as tumors, a series of compounds has been designed in the present disclosure based on the prior art, so as to provide RAD51 inhibitors with novel structures, better efficacy, high bioavailability and great druggability.

## SUMMARY

The present disclosure provides a compound represented by formula (I) or a pharmaceutically acceptable salt thereof,

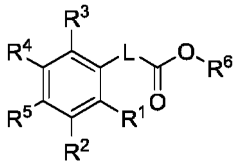

(I)

wherein

$R^1$ is

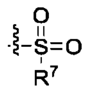, 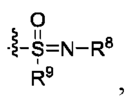, 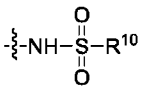, 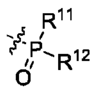, or 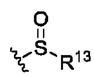;

$R^7$ is

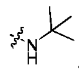,

$C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{7a}$;

$R^{7a}$ is F, Cl, Br, I, OH, CN, =O, $NO_2$, or the following group optionally substituted with one or more $R^{7b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{7b}$ is F, Cl, Br, I, OH, CN, =O, $NO_2$, or the following group optionally substituted with one or more $R^{7c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{7c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^8$ is H, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{8a}$;

$R^{8a}$ is F, Cl, Br, I, OH, CN, =O, $NO_2$, or the following group optionally substituted with one or more $R^{8b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{8b}$ is F, Cl, Br, I, OH, CN, =O, $NO_2$, or the following group optionally substituted with one or more $R^{8c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{8c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^9$ is $C_1$-$C_{10}$ alkyl, $C_3$-$C_{10}$ cycloalkyl, 3- to 10-membered heterocyclyl, $C_6$-$C_{10}$ aryl, or 5-to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{9a}$;

$R^{9a}$ is F, Cl, Br, I, OH, CN, =O, $NO_2$, or the following group optionally substituted with one or more $R^{9b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{9b}$ is F, Cl, Br, I, OH, CN, =O, $NO_2$, or the following group optionally substituted with one or more $R^{9c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{9c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^{10}$ is $C_1$-$C_{10}$ alkyl, $C_3$-$C_{10}$ cycloalkyl, 3- to 10-membered heterocyclyl, $C_6$-$C_{10}$ aryl, or 5-to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{10a}$;

$R^{10a}$ is F, Cl, Br, I, OH, CN, =O, $NO_2$, or the following group optionally substituted with one or more $R^{10b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{10b}$ is F, Cl, Br, I, OH, CN, =O, $NO_2$, or the following group optionally substituted with one or more $R^{10c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{10c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^{11}$, $R^{12}$, and $R^{13}$ are independently $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{11a}$; or

$R^{11}$ and $R^{12}$, together with the atoms to which they are attached, form 3- to 10-membered heterocyclyl, said 3- to 10-membered heterocyclyl is optionally substituted with one or more $R^{11a}$;

$R^{11a}$ is F, Cl, Br, I, OH, CN, =O, $NO_2$, or the following group optionally substituted with one or more $R^{11b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{11b}$ is F, Cl, Br, I, OH, CN, =O, $NO_2$, or the following group optionally substituted with one or more $R^{11c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{11c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^2$, $R^3$, and $R^4$ are independently H, F, Cl, Br, I, OH, CN, $NO_2$, or the following group optionally substituted with one or more $R^a$: $NH_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^a$ is F, Cl, Br, I, OH, CN, =O, $NO_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

Lis

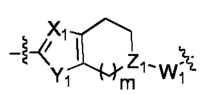 , 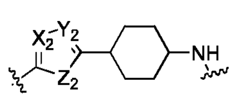 , or 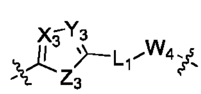 ;

$X_1$, $X_2$, $X_3$, $Y_2$, $Y_3$, and $Z_1$ are independently $CR^{15}$ or N;

$Y_1$, $Z_2$, and $Z_3$ are independently O, NH, or S;

m is 0, 1, or 2;

$R^{15}$ is H, F, Cl, Br, I, OH, CN, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{15a}$;

said $R^{15a}$ is F, Cl, Br, I, OH, CN, =O, $NO_2$, $NH_2$, SH, $C_1$-$C_{10}$ alkyl, $C_3$-$C_{10}$ cycloalkyl, 3-to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$L_1$ is phenyl, $C_7$-$C_{12}$ spirocyclyl, $C_6$-$C_{12}$ fused cyclyl, $C_5$-$C_{12}$ bridged cyclyl, 7- to 12-membered spiroheterocyclyl, 6- to 12-membered fused heterocyclyl, 3- to 10-membered monocyclic heterocyclyl, or 5- to 12-membered bridged heterocyclyl, said 7- to 12-membered spiroheterocyclyl, 6- to 12-membered fused heterocyclyl, 3- to 10-membered monocyclic heterocyclyl, or 5- to 12-membered bridged heterocyclyl contains 1 to 3 heteroatoms selected from O, N, or S; said $C_7$-$C_{12}$ spirocyclyl, $C_6$-$C_{12}$ fused cyclyl, $C_5$-$C_{12}$ bridged cyclyl, 7- to 12-membered spiroheterocyclyl, 6- to 12-membered fused heterocyclyl, 3- to 10-membered monocyclic heterocyclyl, or 5- to 12-membered bridged heterocyclyl is optionally substituted with one or more $R^{28}$;

said $R^{28}$ is F, Cl, Br, I, OH, CN, $NO_2$, or the following group optionally substituted with one or more $R^{28a}$: $NH_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{28a}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$W_1$ and $W_4$ are independently a chemical bond, $NR^{16}$, or O;

$R^{16}$ is H, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{16a}$;

said $R^{16a}$ is F, Cl, Br, I, OH, CN, =O, $NO_2$, $NH_2$, SH, $C_1$-$C_{10}$ alkyl, $C_3$-$C_{10}$ cycloalkyl, 3-to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^5$ is

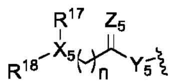 , 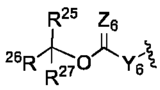 , 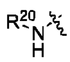 , 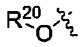 ,

3- to 10-membered heterocyclyl, or 5-to 10-membered heteroaryl; said 3- to 10-membered heterocyclyl or 5- to 10-membered heteroaryl is optionally substituted with one or more $R^{20a}$;

n is 0 or 1;

$X_5$ is N or $CR^{21}$;

$Y_5$ and $Y_6$ are independently a chemical bond, NH, or $CHR^{22}$;

$Z_5$ and $Z_6$ are independently O or $NR^{23}$;

$R^{17}$, $R^{18}$, $R^{25}$, and $R^{26}$ are independently H, $C_1$-$C_{10}$ alkyl, $C_3$-$C_{10}$ cycloalkyl, 3- to 10-membered heterocyclyl, $C_6$-$C_{10}$ aryl, 5- to 10-membered heteroaryl, $C_6$-$C_{10}$ aryloxy, or 5- to 10-membered heteroaryloxy, said $C_1$-$C_{10}$ alkyl, $C_3$-$C_{10}$ cycloalkyl, 3- to 10-membered heterocyclyl, $C_6$-$C_{10}$ aryl, 5- to 10-membered heteroaryl, $C_6$-$C_{10}$ aryloxy, or 5- to 10-membered heteroaryloxy is optionally substituted with one or more $R^{17a}$;

$R^{17a}$ is F, Cl, Br, I, OH, CN, =O, $NO_2$, or the following group optionally substituted with one or more $R^{17b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{17b}$ is F, Cl, Br, I, OH, CN, or the following group optionally substituted with one or more $R^{17c}$: $C_1$-$C_{10}$ alkyl, $C_3$-$C_{10}$ cycloalkyl, 3- to 10-membered heterocyclyl, or $C_6$-$C_{10}$ aryl;

$R^{17c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl; or

$R^{17}$ and $R^{18}$, together with the atom $X_5$ to which they are attached, form 3- to 10-membered heterocyclyl or $C_3$-$C_{10}$ cycloalkyl, said 3- to 10-membered heterocyclyl or $C_3$-$C_{10}$ cycloalkyl is optionally substituted with one or more $R^{17a}$;

$R^{20}$ is the following group optionally substituted with one or more $R^{20a}$: 5- to 10-membered heteroaryl, -C(O)-5- to 10-membered heteroaryl, 5- to 10-membered heterocyclyl, $C_6$-$C_{10}$ aryl, or -C(O)-$C_6$-$C_{10}$ aryl;

$R^{20a}$ is F, Cl, Br, I, OH, CN, =O, $NO_2$, $NH_2$, SH, $C_1$-$C_{10}$ alkyl, $C_1$-$C_{10}$ alkoxy, $C_3$-$C_{10}$ cycloalkyl, 3- to 10-membered heterocyclyl, $C_6$-$C_{10}$ aryl, or 5- to 10-membered heteroaryl; said $C_1$-$C_{10}$ alkyl, $C_1$-$C_{10}$ alkoxy, $C_3$-$C_{10}$ cycloalkyl, 3- to 10-membered heterocyclyl, $C_6$-$C_{10}$ aryl, or 5- to 10-membered heteroaryl is optionally substituted with one or more $R^{20b}$;

$R^{20b}$ is F, Cl, Br, I, OH, CN, =O, $NO_2$, $NH_2$, SH, or $C_3$-$C_{10}$ cycloalkyl;

$R^{21}$, $R^{22}$, and $R^{27}$ are independently H, $C_1$-$C_{10}$ alkyl, or $C_6$-$C_{10}$ aryl, said $C_1$-$C_{10}$ alkyl or $C_6$-$C_{10}$ aryl is optionally

substituted with one or more $R^{21a}$;

$R^{21a}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^{23}$ is H, CN, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{23a}$;

$R^{23a}$ is F, Cl, Br, I, OH, CN, =O, $NO_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy; or

$R^4$ and $R^5$, together with the atoms to which they are attached, form 4- to 6-membered heterocyclyl, phenyl, or 5- to 6-membered heteroaryl, said 4- to 6-membered heterocyclyl, phenyl, or 5- to 6-membered heteroaryl is optionally substituted with one or more $R^{24a}$;

$R^{24a}$ is F, Cl, Br, I, OH, CN, =O, $NO_2$, or the following group optionally substituted with one or more $R^{24b}$: $NH_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{24b}$ is F, Cl, Br, I, OH, CN, =O, $NO_2$, or the following group optionally substituted with one or more $R^{24c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{24c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^6$ is H or the following group optionally substituted with one or more $R^{6a}$: $NH_2$, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^{6a}$ is F, Cl, Br, I, OH, CN, =O, $NO_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy,

provided that when $L_1$ is phenyl, or when $R^5$ is 3- to 10-membered heterocyclyl or 5- to 10-membered heteroaryl, $R^7$ is not

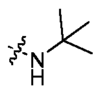.

In some embodiments, $R^{11}$ and $R^{12}$ are independently $C_1$-$C_{10}$ alkyl, $C_3$-$C_{10}$ cycloalkyl, 3-to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{11a}$.

In some embodiments, said $R^5$ is

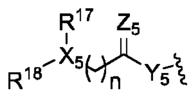, 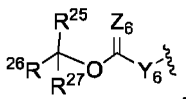, or 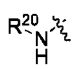,

and said $Y_5$ and $Y_6$ are independently NH or $CHR^{22}$.

In some embodiments, $R^{20}$ is 5- to 10-membered heteroaryl or $C_6$-$C_{10}$ aryl, said 5- to 10-membered heteroaryl or $C_6$-$C_{10}$ aryl is optionally substituted with $R^{20a}$.

In some embodiments, said $R^{20a}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl; said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with $R^{20b}$, said $R^{20b}$ is F, Cl, Br, I, OH, CN, =O, $NO_2$, $NH_2$, or SH.

In some embodiments, said $R^{20a}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl.

In some embodiments, $L_1$ is $C_7$-$C_{12}$ spirocyclyl, $C_6$-$C_{12}$ fused cyclyl, $C_5$-$C_{12}$ bridged cyclyl, 7- to 12-membered spiroheterocyclyl, 6- to 12-membered fused heterocyclyl, 3- to 10-membered monocyclic heterocyclyl, or 5- to 12-membered bridged heterocyclyl, said 7- to 12-membered spiroheterocyclyl, 6- to 12-membered fused heterocyclyl, 3- to 10-membered monocyclic heterocyclyl, or 5- to 12-membered bridged heterocyclyl contains 1 to 3 heteroatoms selected from O, N, or S; said $C_7$-$C_{12}$ spirocyclyl, $C_6$-$C_{12}$ fused cyclyl, $C_5$-$C_{12}$ bridged cyclyl, 7- to 12-membered spiroheterocyclyl,

6- to 12-membered fused heterocyclyl, 3-to 10-membered monocyclic heterocyclyl, or 5- to 12-membered bridged heterocyclyl is optionally substituted with $R^{28a}$.

In some embodiments, $L_1$ is $C_7$-$C_{12}$ spirocyclyl, $C_6$-$C_{12}$ fused cyclyl, $C_5$-$C_{12}$ bridged cyclyl, 7- to 12-membered spiroheterocyclyl, 6- to 12-membered fused heterocyclyl, 3- to 10-membered monocyclic heterocyclyl, or 5- to 12-membered bridged heterocyclyl, said 7- to 12-membered spiroheterocyclyl, 6- to 12-membered fused heterocyclyl, 3- to 10-membered monocyclic heterocyclyl, or 5- to 12-membered bridged heterocyclyl contains 1 to 3 heteroatoms selected from O, N, or S.

In some embodiments, the compound of formula (I) or a pharmaceutically acceptable salt thereof is a compound of formula (Ia) or a pharmaceutically acceptable salt thereof:

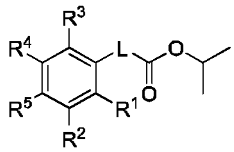

(Ia)

wherein $R^1$, $R^2$, $R^3$, $R^4$, and $R^5$ are as defined in formula (I), L is

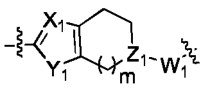

or

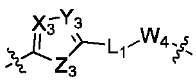

,

$X_1$, $X_3$, $Y_1$, $Y_3$, $Z_1$, $Z_3$, $L_1$, $W_1$, $W_4$, and m are as defined in formula (I), provided that when $L_1$ is 3- to 10-membered monocyclic heterocyclyl optionally substituted with $R^{28}$, $W_4$ is not a chemical bond, wherein $R^{28}$ is as defined in formula (I).

In some embodiments, the compound of formula (I) or a pharmaceutically acceptable salt thereof is a compound of formula (Ia) or a pharmaceutically acceptable salt thereof:

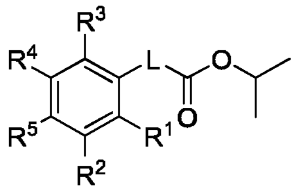

(Ia)

wherein $R^1$, $R^2$, $R^3$, $R^4$, and $R^5$ are as defined in formula (I), L is

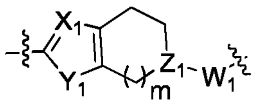

,

wherein $X_1$, $Y_1$, $Z_1$, $W_1$, and m are as defined in formula (I).

In some embodiments, the compound of formula (I) or a pharmaceutically acceptable salt thereof is a compound of formula (Ia) or a pharmaceutically acceptable salt thereof:

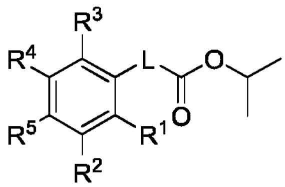

(Ia)

wherein $R^1$, $R^2$, $R^3$, $R^4$, and $R^5$ are as defined in formula (I),

Lis

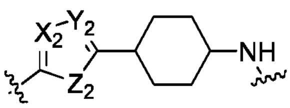

;

$X_2$, $Y_2$, and $Z_2$ are as defined in formula (I),

provided that when $R^1$ is tert-butylsulfamoyl, $R^5$ is not

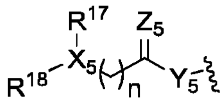

or

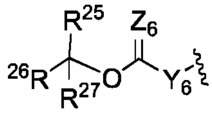

,

wherein $X_5$, $Y_5$, $Y_6$, $Z_5$, $Z_6$, $R^{17}$, $R^{18}$, $R^{25}$, $R^{26}$, $R^{27}$, and n are as defined in formula (I).

In some embodiments, the compound of formula (I) or a pharmaceutically acceptable salt thereof is a compound of formula (Ia) or a pharmaceutically acceptable salt thereof:

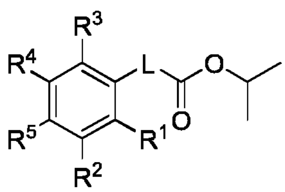

(Ia)

wherein $R^1$, $R^2$, $R^3$, $R^4$, and $R^5$ are as defined in formula (I),

Lis

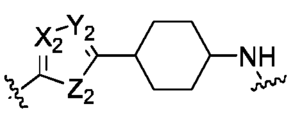

;

$X_2$, $Y_2$, and $Z_2$ are as defined in formula (I),

provided that when $R^1$ is tert-butylsulfamoyl, $R^5$ is

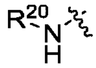

and $R^{20}$ is as defined in formula (I).

In some embodiments, the compounds of formula (Ia) do not comprise

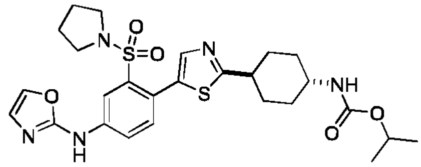

,

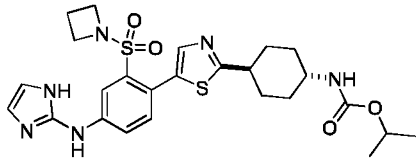

,

or

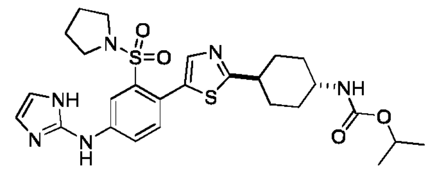

.

In some embodiments, the compound of formula (I) or a pharmaceutically acceptable salt thereof is a compound of formula (Ia) or a pharmaceutically acceptable salt thereof:

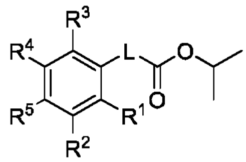

(Ia)

wherein $R^1$, $R^2$, $R^3$, and $R^4$ are as defined in formula (I),

Lis

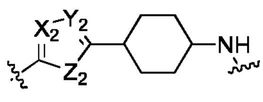

,

wherein $X_2$, $Y_2$, and $Z_2$ are as defined in formula (I);

$R^5$ is

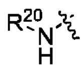

,

provided that $R^1$ is not tert-butylsulfamoyl.

In some embodiments, the compound of formula (I) or a pharmaceutically acceptable salt thereof is a compound of formula (Ib) or a pharmaceutically acceptable salt thereof:

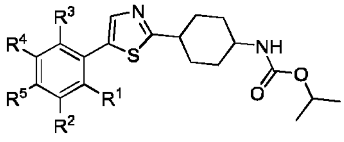

(Ib)

wherein $R^1$, $R^2$, $R^3$, $R^4$, and $R^5$ are as defined in formula (I), provided that $R^1$ is not tert-butyl sulfamoyl.

In some embodiments, the compound of formula (I) or a pharmaceutically acceptable salt thereof is a compound of formula (II) or a pharmaceutically acceptable salt thereof:

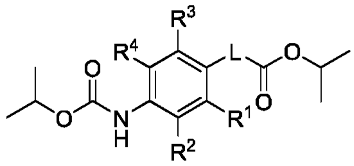

(II)

wherein $R^1$, $R^2$, $R^3$, $R^4$, and L are as defined in formula (I).

In some embodiments, the compound of formula (I) or a pharmaceutically acceptable salt thereof is a compound of formula (II) or a pharmaceutically acceptable salt thereof:

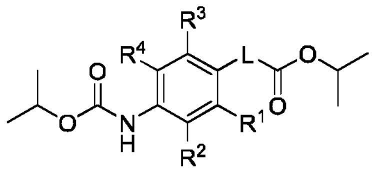

(II)

wherein $R^1$, $R^2$, $R^3$, and $R^4$ are as defined in formula (I); L is

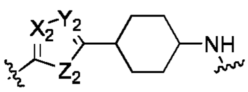

,

wherein $X_2$, $Y_2$, and $Z_2$ are as defined in formula (I),
provided that when $R^1$ is tert-butylsulfamoyl and $X_2$ is $CR^{15}$ together with $Y_2$ is N, $Z_2$ is not S.

In some embodiments, the compound of formula (I) or a pharmaceutically acceptable salt thereof is a compound of formula (II) or a pharmaceutically acceptable salt thereof:

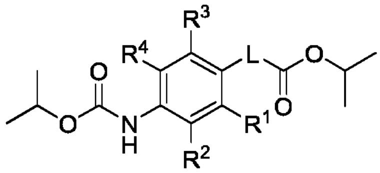

(II)

wherein $R^1$, $R^2$, $R^3$, and $R^4$ are as defined in formula (I); L is

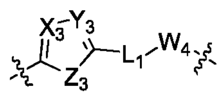

,

wherein $X_3$, $Y_3$, $Z_3$, $L_1$, and $W_4$ are as defined in formula (I), provided that when $L_1$ is 3- to 10-membered monocyclic heterocyclyl optionally substituted with $R^{28}$, $W_4$ is not a chemical bond, wherein $R^{28}$ is as defined in formula (I).

In some embodiments, the compound of formula (I) or a pharmaceutically acceptable salt thereof is a compound of formula (II) or a pharmaceutically acceptable salt thereof:

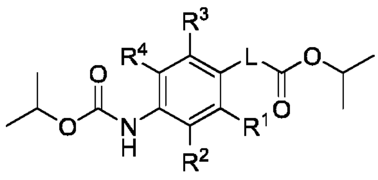

(II)

wherein $R^1$, $R^2$, $R^3$, and $R^4$ are as defined in formula (I); L is

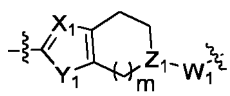

,

and $X_1$, $Y_1$, $Z_1$, $W_1$, and m are as defined in formula (I).

In some embodiments, the compound of formula (I) or a pharmaceutically acceptable salt thereof is a compound of formula (IIa) or a pharmaceutically acceptable salt thereof:

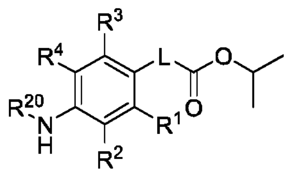

(IIa)

wherein $R^1$, $R^2$, $R^3$, $R^4$, $R^{20}$, and L are as defined in formula (I); and $R^1$ is not tert-butyl sulfamoyl.

In some embodiments, the compound of formula (I) or a pharmaceutically acceptable salt thereof is a compound of formula (IIa) or a pharmaceutically acceptable salt thereof:

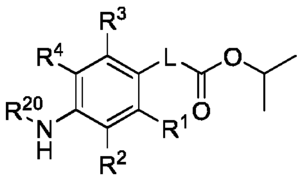

(IIa)

wherein $R^2$, $R^3$, $R^4$, and $R^{20}$ are as defined in formula (I); L is

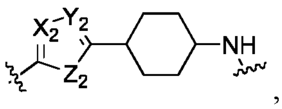

wherein $X_2$, $Y_2$, and $Z_2$ are as defined in formula (I); $R^1$ is

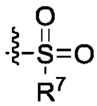

and $R^7$ is not

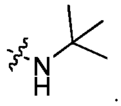

In some embodiments, the compound of formula (I) or a pharmaceutically acceptable salt thereof is a compound of formula (IIb) or a pharmaceutically acceptable salt thereof:

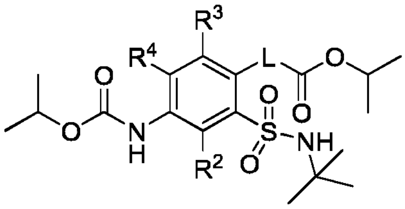

(IIb)

wherein $R^2$, $R^3$, and $R^4$ are as defined in formula (I);
Lis

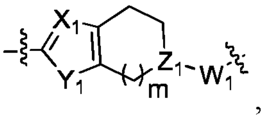

and $X_1$, $Y_1$, $Z_1$, $W_1$, and m are as defined in formula (I).

In some embodiments, the compound of formula (I) or a pharmaceutically acceptable salt thereof is a compound of formula (II) or a pharmaceutically acceptable salt thereof:

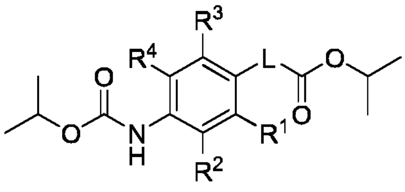

(II)

wherein $R^1$, $R^2$, $R^3$, and $R^4$ are as defined in formula (I), L is

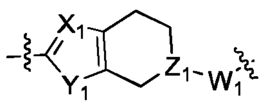 or

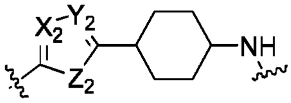 ;

$X_1$, $X_2$, $Y_2$, and $Z_1$ are independently $CR^{15}$ or N;
$Y_1$ and $Z_2$ are independently O, NH, or S;
$W_1$ is a chemical bond or NH;
$R^{15}$ is H, F, Cl, Br, I, OH, CN, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl.

In some embodiments, the compound of formula (I) or a pharmaceutically acceptable salt thereof is a compound of formula (II) or a pharmaceutically acceptable salt thereof:

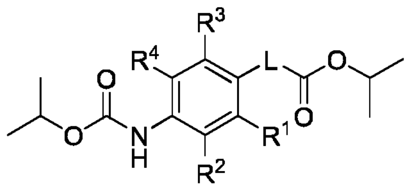

(II)

wherein $R^1$, $R^2$, $R^3$, and $R^4$ are as defined in formula (I), L is

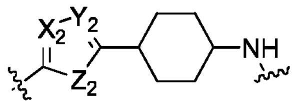

or

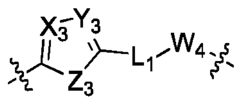

,

provided that when $R^1$ is tert-butylsulfamoyl and $X_2$ is $CR^{15}$ together with $Y_2$ is N, $Z_2$ is not S, wherein $R^{15}$, $X_2$, $Y_2$, $Z_2$, $X_3$, $Y_3$, $Z_3$, $L_1$, and $W_4$ are as defined in formula (I).

In some embodiments, the compound of formula (I) or a pharmaceutically acceptable salt thereof is a compound of formula (IV) or a pharmaceutically acceptable salt thereof:

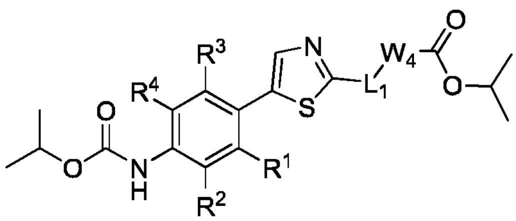

(IV)

wherein $R^1$, $R^2$, $R^3$, $R^4$, $L_1$, and $W_4$ are as defined in formula (I).

In some embodiments, the compound of formula (I), formula (II) or formula (IV), or a pharmaceutically acceptable salt thereof is a compound of formula (V) or a pharmaceutically acceptable salt thereof:

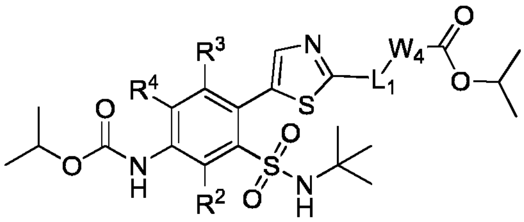

(V)

wherein $R^2$, $R^3$, $R^4$, $L_1$, and $W_4$ are as defined in formula (I).

In some embodiments, the compound of formula (I) or a pharmaceutically acceptable salt thereof is a compound of formula (Ia) or a pharmaceutically acceptable salt thereof:

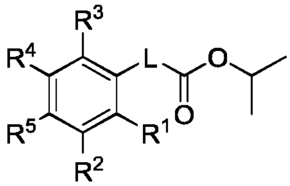

(Ia)

wherein $R^1$, $R^2$, $R^3$, $R^4$, $R^5$, and L are as defined in formula (I), and $R^5$ is not

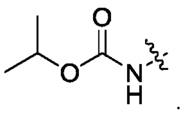

In some embodiments, the compound of formula (I) or a pharmaceutically acceptable salt thereof is a compound of formula (VII) or a pharmaceutically acceptable salt thereof:

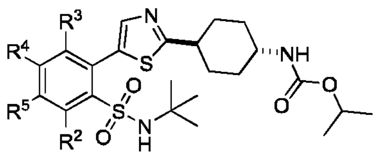

(VII)

wherein $R^2$, $R^3$, $R^4$, and $R^5$ are as defined in formula (I), and $R^5$ is not

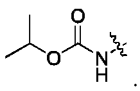

In some embodiments, the compound of formula (I) or a pharmaceutically acceptable salt thereof is a compound of formula (VII) or a pharmaceutically acceptable salt thereof:

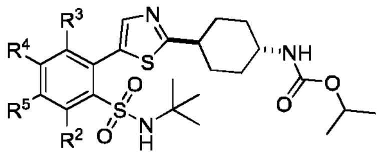

(VII)

wherein $R^2$, $R^3$, and $R^4$ are as defined in formula (I), $R^5$ is

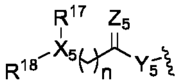

or

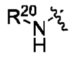

,

provided that when $R^5$ is

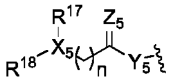

,

$Z_5$ is $NR^{23}$, or $Y_5$ is $CHR^{22}$.

In some embodiments, the compound of formula (I) or a pharmaceutically acceptable salt thereof is a compound of formula (VIII) or a pharmaceutically acceptable salt thereof:

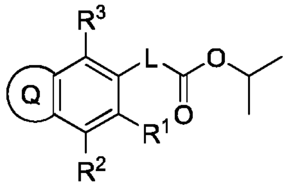

(VIII)

wherein ring Q is 4- to 6-membered heterocyclyl, phenyl, or 5- to 6-membered heteroaryl, said 4- to 6-membered heterocyclyl, phenyl, or 5- to 6-membered heteroaryl is optionally substituted with one or more $R^{24a}$;
$R^1$, $R^2$, $R^3$, L, and $R^{24a}$ are as defined in formula (I).

In some embodiments, the compound of formula (I) or a pharmaceutically acceptable salt thereof is a compound of formula (IX) or a pharmaceutically acceptable salt thereof:

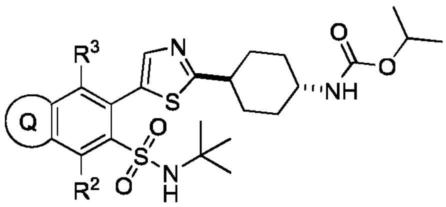

(IX)

wherein ring Q is 4- to 6-membered heterocyclyl, phenyl, or 5- to 6-membered heteroaryl, said 4- to 6-membered heterocyclyl, phenyl, or 5- to 6-membered heteroaryl is optionally substituted with one or more $R^{24a}$;
$R^2$, $R^3$, and $R^{24a}$ are as defined in formula (I).

In some embodiments, the compound of formula (I) or a pharmaceutically acceptable salt thereof is a compound of formula I-1, formula I-7 or formula I-11, or a pharmaceutically acceptable salt thereof:

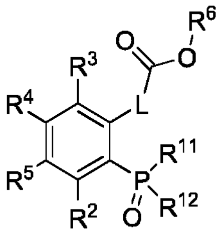
I-1
,
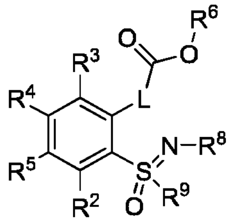
I-7
, or
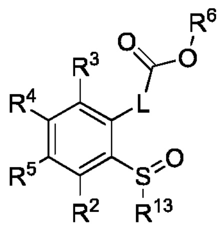
I-11
,

wherein L, $R^2$, $R^3$, $R^4$, $R^5$, $R^6$, $R^8$, $R^9$, $R^{11}$, $R^{12}$, and $R^{13}$ are as defined in formula (I).

In some embodiments, the compound of formula (I) or a pharmaceutically acceptable salt thereof is a compound of formula 1-13, formula 1-23 or formula 1-27, or a pharmaceutically acceptable salt thereof:

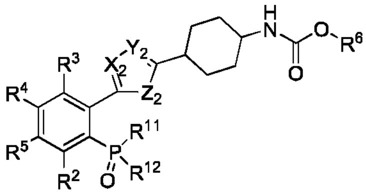
I-13
,
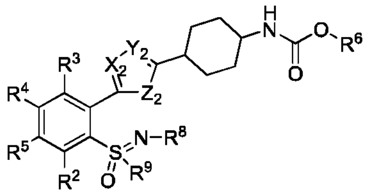
I-23
, or
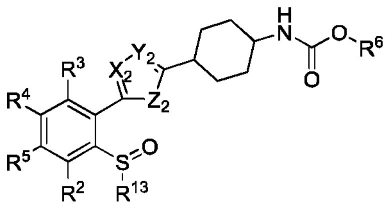
I-27
,

wherein $R^2$, $R^3$, $R^4$, $R^5$, $R^6$, $R^8$, $R^9$, $R^{11}$, $R^{12}$, $R^{13}$, $X_2$, $Z_2$, and $Y_2$ are as defined in formula (I).

In some embodiments, $R^1$ is

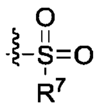
,
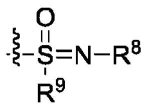
,
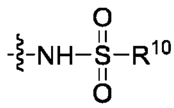
, or
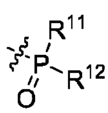
.

In some embodiments, $R^1$ is

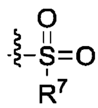
,
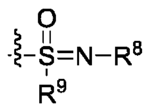
, or
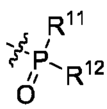
.

In some embodiments, $R^1$ is

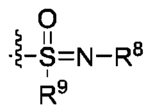
,
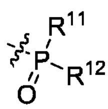
, or
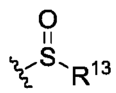
.

In some embodiments, $R^1$ is

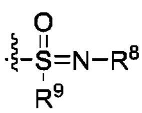
or
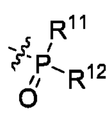
.

In some embodiments, $R^1$ is

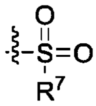 or 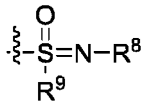 .

In some embodiments, $R^1$ is

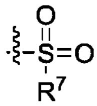 .

In some embodiments, $R^7$ is

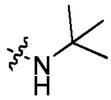 ,

$C_1$-$C_{10}$ alkyl, $C_3$-$C_{10}$ cycloalkyl, 3- to 10-membered heterocyclyl, or $C_6$-$C_{10}$ aryl, said $C_1$-$C_{10}$ alkyl, $C_3$-$C_{10}$ cycloalkyl, 3- to 10-membered heterocyclyl, or $C_6$-$C_{10}$ aryl is optionally substituted with one or more $R^{7a}$. In some embodiments, $R^7$ is

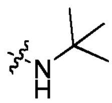 ,

$C_1$-$C_{10}$ alkyl, $C_3$-$C_{10}$ cycloalkyl, or $C_6$-$C_{10}$ aryl, said $C_1$-$C_{10}$ alkyl, $C_3$-$C_{10}$ cycloalkyl, or $C_6$-$C_{10}$ aryl is optionally substituted with one or more $R^{7a}$.

In some embodiments, $R^7$ is

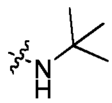

or $C_1$-$C_{10}$ alkyl, said $C_1$-$C_{10}$ alkyl is optionally substituted with one or more $R^{7a}$.

In some embodiments, $R^7$ is 3- to 10-membered heterocyclyl, said 3- to 10-membered heterocyclyl is optionally substituted with one or more $R^{7a}$.

In some embodiments, $R^7$ is

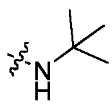 ,

$C_1$-$C_6$ alkyl, $C_3$-$C_6$ cycloalkyl, 3- to 7-membered heterocyclyl, or $C_6$-$C_{10}$ aryl, said $C_1$-$C_6$ alkyl, $C_3$-$C_6$ cycloalkyl, 3- to 7-membered heterocyclyl, or $C_6$-$C_{10}$ aryl is optionally substituted with one or more $R^{7a}$.

In some embodiments, $R^7$ is oxetanyl,

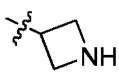 ,

or oxacyclopentyl, said oxetanyl,

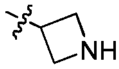,

or oxacyclopentyl is optionally substituted with one or more $R^{7a}$.

In some embodiments, $R^7$ is $C_3$-$C_{10}$ cycloalkyl, oxetanyl,

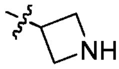,

oxacyclopentyl, or $C_6$-$C_{10}$ aryl, said $C_3$-$C_{10}$ cycloalkyl, oxetanyl,

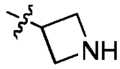,

oxacyclopentyl, or $C_6$-$C_{10}$ aryl is optionally substituted with one or more $R^{7a}$.

In some embodiments, $R^7$ is $C_3$-$C_6$ cycloalkyl, oxetanyl,

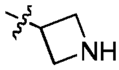,

or oxacyclopentyl, said $C_3$-$C_6$ cycloalkyl, oxetanyl,

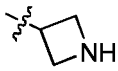,

or oxacyclopentyl is optionally substituted with one or more $R^{7a}$.

In some embodiments, $R^7$ is

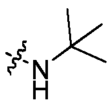, 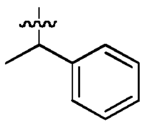,

$CF_3$, $CH_2F$, isopropyl, neopentyl, cyclopropyl, cyclobutyl, tert-butyl, oxetanyl, oxacyclopentyl, azetidinyl, or phenyl.

In some embodiments, $R^7$ is

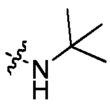, 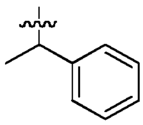,

$CF_3$, $CH_2F$, isopropyl, neopentyl, cyclopropyl, cyclobutyl, tert-butyl, oxetanyl,

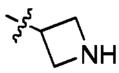,

oxacyclopentyl, or phenyl.

In some embodiments, $R^7$ is

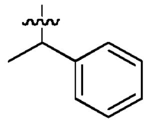,

$CF_3$, $CH_2F$, isopropyl, neopentyl, cyclopropyl, cyclobutyl, tert-butyl, oxetanyl,

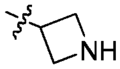,

oxacyclopentyl, or phenyl.

In some embodiments, $R^7$ is

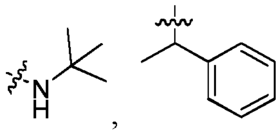, ,

$CF_3$, $CH_2F$, isopropyl, neopentyl, cyclopropyl, tert-butyl, or phenyl.

In some embodiments, $R^7$ is cyclopropyl, cyclobutyl, oxetanyl,

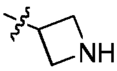,

or oxacyclopentyl.

In some embodiments, $R^8$ is H, $C_1$-$C_{10}$ alkyl, or $C_3$-$C_{10}$ cycloalkyl, said $C_1$-$C_{10}$ alkyl or $C_3$-$C_{10}$ cycloalkyl is optionally substituted with one or more $R^{8a}$.

In some embodiments, $R^8$ is H, $C_1$-$C_{10}$ alkyl, or $C_3$-$C_{10}$ cycloalkyl, said $C_1$-$C_{10}$ alkyl or $C_3$-$C_{10}$ cycloalkyl is optionally substituted with one or more $R^{8a}$.

In some embodiments, $R^8$ is H, $C_1$-$C_6$ alkyl, or $C_3$-$C_6$ cycloalkyl, said $C_1$-$C_6$ alkyl or $C_3$-$C_6$ cycloalkyl is optionally substituted with one or more $R^{8a}$.

In some embodiments, $R^8$ is H, methyl, cyclopropyl, or

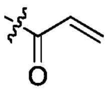.

In some embodiments, $R^9$ is $C_1$-$C_{10}$ alkyl, $C_3$-$C_{10}$ cycloalkyl, or 3- to 10-membered heterocyclyl, said $C_1$-$C_{10}$ alkyl, $C_3$-$C_{10}$ cycloalkyl, or 3- to 10-membered heterocyclyl is optionally substituted with one or more $R^{9a}$.

In some embodiments, $R^9$ is $C_1$-$C_{10}$ alkyl or $C_3$-$C_{10}$ cycloalkyl, said $C_1$-$C_{10}$ alkyl is optionally substituted with one or more $R^{9a}$.

In some embodiments, $R^9$ is $C_1$-$C_{10}$ alkyl, said $C_1$-$C_{10}$ alkyl is optionally substituted with one or more $R^{9a}$.

In some embodiments, $R^9$ is $C_3$-$C_{10}$ cycloalkyl or 3- to 10-membered heterocyclyl, said $C_3$-$C_{10}$ cycloalkyl or 3- to 10-membered heterocyclyl is optionally substituted with one or more $R^{9a}$.

In some embodiments, $R^9$ is $C_1$-$C_6$ alkyl, $C_3$-$C_6$ cycloalkyl, or 3- to 7-membered heterocyclyl, said $C_1$-$C_6$ alkyl, $C_3$-$C_6$ cycloalkyl, or 3- to 7-membered heterocyclyl is optionally substituted with one or more $R^{9a}$.

In some embodiments, $R^9$ is methyl, isopropyl, cyclobutyl, oxetanyl, or cyclopropyl.

In some embodiments, $R^9$ is methyl, isopropyl or cyclopropyl.

In some embodiments, $R^9$ is cyclopropyl.

In some embodiments, $R^{10}$ is $C_1$-$C_{10}$ alkyl, said $C_1$-$C_{10}$ alkyl is optionally substituted with one or more $R^{10a}$.

In some embodiments, $R^{10}$ is $C_1$-$C_6$ alkyl, said $C_1$-$C_6$ alkyl is optionally substituted with one or more $R^{10a}$.

In some embodiments, $R^{10}$ is tert-butyl. In some embodiments, $R^{11}$ and $R^{12}$ are independently $C_1$-$C_{10}$ alkyl, said $C_1$-$C_{10}$ alkyl is optionally substituted with one or more $R^{11a}$.

In some embodiments, $R^{11}$ and $R^{12}$ are independently $C_1$-$C_6$ alkyl, said $C_1$-$C_6$ alkyl is optionally substituted with one or more $R^{11a}$.

In some embodiments, $R^{11}$ and $R^{12}$ are independently methyl, ethyl, or isopropyl.

In some embodiments, $R^{11}$ and $R^{12}$, together with the atoms to which they are attached, form 3- to 10-membered heterocyclyl, said 3- to 10-membered heterocyclyl is optionally substituted with one or more $R^{11a}$.

In some embodiments, $R^{11}$ and $R^{12}$, together with the atoms to which they are attached, form 3- to 7-membered heterocyclyl, said 3- to 7-membered heterocyclyl is optionally substituted with one or more $R^{11a}$.

In some embodiments, $R^{11}$ and $R^{12}$, together with the atoms to which they are attached, form

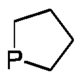 or 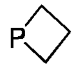.

In some embodiments, $R^{11}$ and $R^{12}$, together with the atoms to which they are attached, form

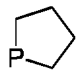.

In some embodiments, $R^{13}$ is $C_1$-$C_{10}$ alkyl or $C_3$-$C_{10}$ cycloalkyl, said $C_1$-$C_{10}$ alkyl or $C_3$-$C_{10}$ cycloalkyl is optionally substituted with one or more $R^{11a}$.

In some embodiments, $R^{13}$ is $C_1$-$C_6$ alkyl or $C_3$-$C_6$ cycloalkyl, said $C_1$-$C_6$ alkyl or $C_3$-$C_6$ cycloalkyl is optionally substituted with one or more $R^{11a}$.

In some embodiments, $R^{13}$ is isopropyl or cyclopropyl.

In some embodiments, $R^{7a}$, $R^{8a}$, $R^{9a}$, $R^{10a}$, and $R^{11a}$ are independently F, Cl, Br, I, =O, $C_2$-$C_{10}$ alkenyl, $C_1$-$C_{10}$ alkyl, or $C_6$-$C_{10}$ aryl.

In some embodiments, $R^{7a}$, $R^{8a}$, $R^{9a}$, $R^{10a}$, and $R^{11a}$ are independently $C_1$-$C_{10}$ alkyl, $C_6$-$C_{10}$ aryl, $C_2$-$C_{10}$ alkenyl, or =O.

In some embodiments, $R^{7a}$, $R^{8a}$, $R^{9a}$, $R^{10a}$, and $R^{11a}$ are independently F, Cl, Br, I, =O, $C_2$-$C_{10}$ alkenyl, or $C_6$-$C_{10}$ aryl.

In some embodiments, $R^{7a}$, $R^{8a}$, $R^{9a}$, $R^{10a}$, and $R^{11a}$ are independently F, Cl, Br, =O, $C_2$-$C_4$ alkenyl, $C_1$-$C_6$ alkyl, or $C_6$-$C_{10}$ aryl.

In some embodiments, $R^{7a}$ is F or phenyl.

In some embodiments, $R^{8a}$ is =O or vinyl.

In some embodiments, $R^1$ is

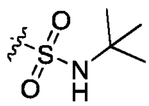, 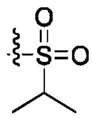, 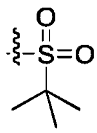, 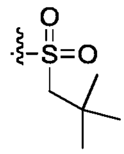, 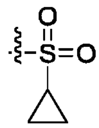,

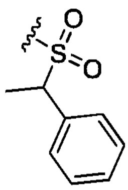, 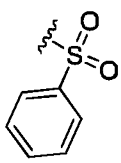, 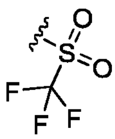, 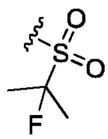, 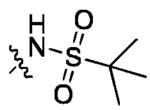, 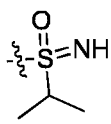, 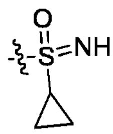, 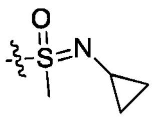,

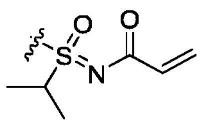, 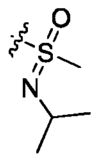, 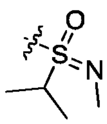, 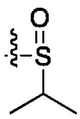, 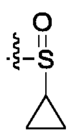, 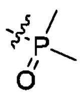, 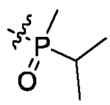, 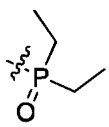, 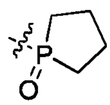,

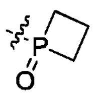, 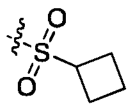, 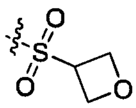, 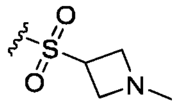, 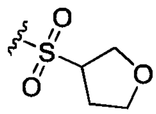, 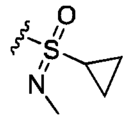, 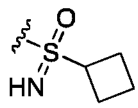,

or

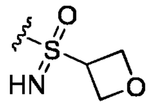.

In some embodiments, $R^1$ is

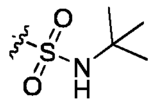, 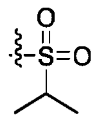, 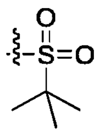, 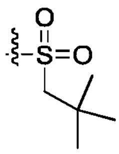, 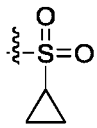,

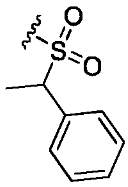, 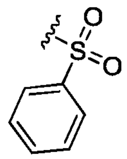, 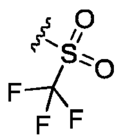, 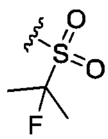, 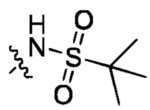, 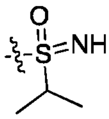, 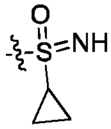, 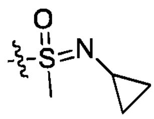,

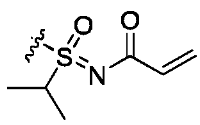, 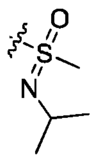, 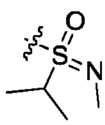, 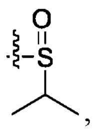, 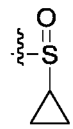, 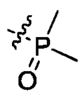, 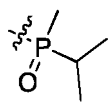, 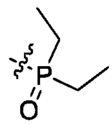, or 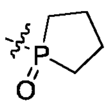.

In some embodiments, $R^1$ is

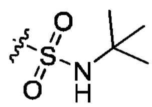, 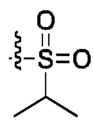, 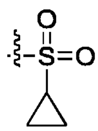, 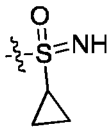, 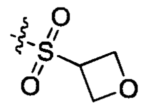,

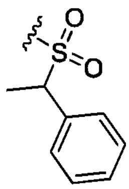, 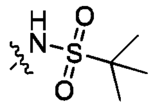, 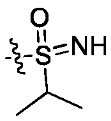, 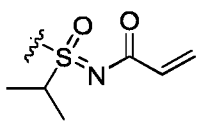, 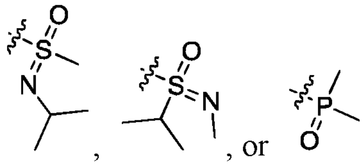, , or .

In some embodiments, $R^2$, $R^3$, and $R^4$ are independently H, F, Cl, Br, I, OH, CN, or the following group optionally substituted with $R^a$: $NH_2$, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl.

In some embodiments, $R^2$, $R^3$, and $R^4$ are independently H, F, Cl, Br, I, OH, CN, $NH_2$, methyl, ethyl, isopropyl, cyclopropyl, or phenyl.

In some embodiments, $R^2$, $R^3$, and $R^4$ are independently H.

In some embodiments, L is

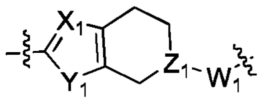, 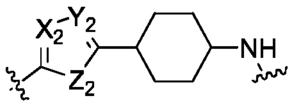,

or

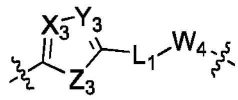.

In some embodiments, $X_1$, $X_2$, $X_3$, $Y_2$, $Y_3$, and $Z_1$ are independently N or CH.

In some embodiments, $Y_1$, $Z_2$, and $Z_3$ are independently S.

In some embodiments, m is 1 or 2.

In some embodiments, m is 1.

In some embodiments, $W_1$ and $W_4$ are independently NH or a chemical bond.

In some embodiments, L is

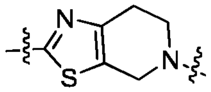, 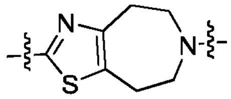, 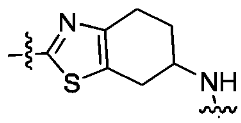,

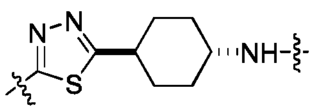, 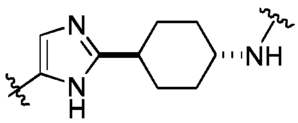, 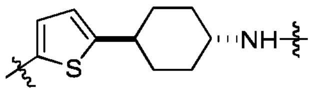,

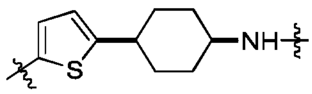, 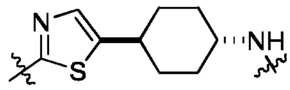, 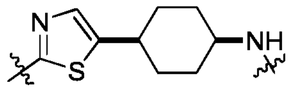,

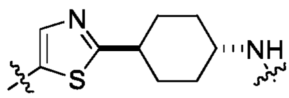, or 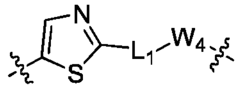.

In some embodiments, L is

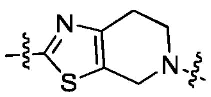, 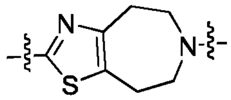, 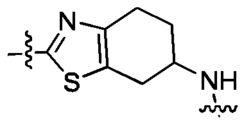,

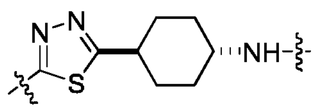, 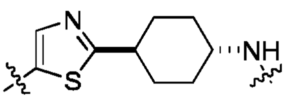, or 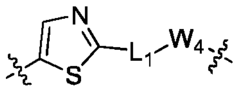.

In some embodiments, L is

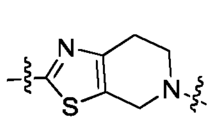 , 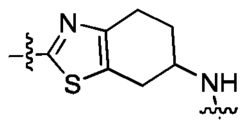 ,

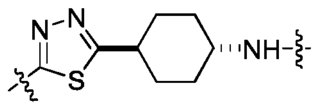 , 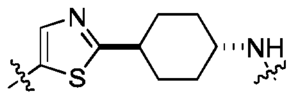 , or 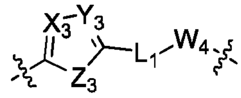 .

In some embodiments, said

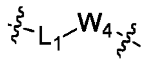

is phenyl-NH-, $C_7$-$C_{12}$ spirocyclyl-NH-, 7- to 12-membered spiroheterocyclyl, $C_6$-$C_{12}$ fused cyclyl-NH-, 6- to 12-membered fused heterocyclyl-NH-, 3- to 10-membered monocyclic heterocyclyl-NH-, or $C_5$-$C_{12}$ bridged cyclyl-NH-.

In some embodiments, said

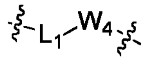

is $C_7$-$C_{12}$ spirocyclyl-NH-, 7- to 12-membered spiroheterocyclyl, 6- to 12-membered fused heterocyclyl-NH-, 3- to 10-membered monocyclic heterocyclyl-NH-, or $C_5$-$C_{12}$ bridged cyclyl-NH-.

In some embodiments,

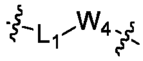

is 7- to 12-membered spiroheterocyclyl, 6- to 12-membered fused heterocyclyl-NH-, 3- to 10-membered monocyclic heterocyclyl-NH-, or $C_5$-$C_{12}$ bridged cyclyl-NH-.

In some embodiments, said 7- to 12-membered spiroheterocyclyl, 6- to 12-membered fused heterocyclyl, or 3- to 10-membered monocyclic heterocyclyl contains 1 to 3 nitrogen atoms.

In some embodiments, said is

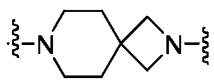 , 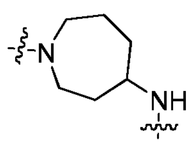 ,

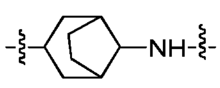 , 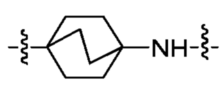 , 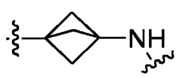 , 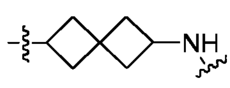 , 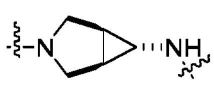 ,

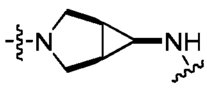 , 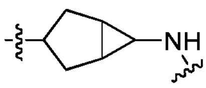 , 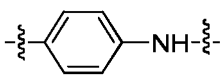 , or 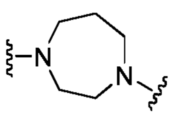 .

In some embodiments, said

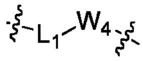

is

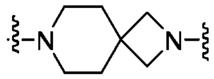 , 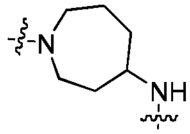 ,

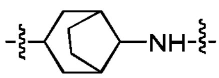 , 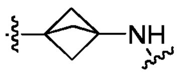 , 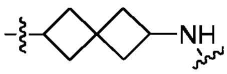 , 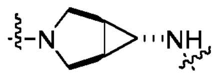 , 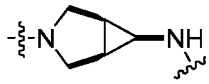 ,

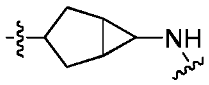 , 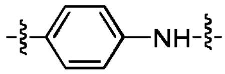 , or 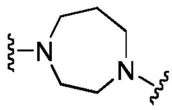 .

In some embodiments, said

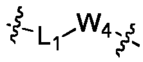

is

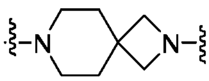 , 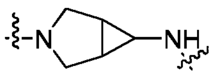 ,

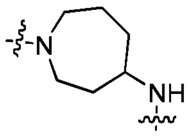 , 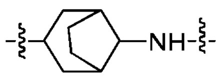 , 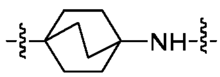 , 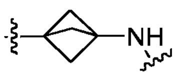 , 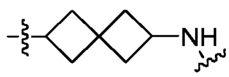 ,

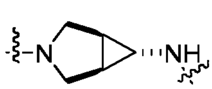 , or 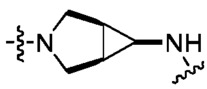 .

In some embodiments, said

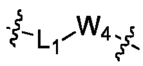

is

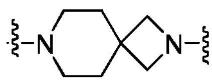 , 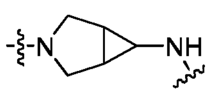 ,

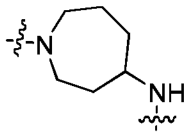, 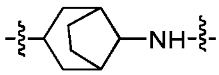, 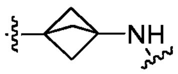, 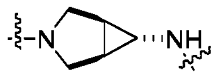, or 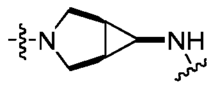.

In some embodiments, $R^{17}$, $R^{18}$, $R^{25}$, and $R^{26}$ are independently H, $C_1$-$C_{10}$ alkyl, $C_3$-$C_{10}$ cycloalkyl, or $C_6$-$C_{10}$ aryloxy, said $C_1$-$C_{10}$ alkyl, $C_3$-$C_{10}$ cycloalkyl, or $C_6$-$C_{10}$ aryloxy is optionally substituted with one or more $R^{17a}$.

In some embodiments, $R^{17}$, $R^{18}$, $R^{25}$, and $R^{26}$ are independently H, $C_1$-$C_6$ alkyl, $C_3$-$C_6$ cycloalkyl, or $C_6$-$C_{10}$ aryloxy, said $C_1$-$C_6$ alkyl, $C_3$-$C_6$ cycloalkyl, or $C_6$-$C_{10}$ aryloxy is optionally substituted with one or more $R^{17a}$.

In some embodiments, $R^{17a}$ is F, Cl, Br, I, OH, CN, or the following group optionally substituted with one or more $R^{17b}$: $C_1$-$C_{10}$ alkyl, $C_6$-$C_{10}$ aryl.

In some embodiments, $R^{17a}$ is F or phenyl.

In some embodiments, $R^{17a}$ is phenyl.

In some embodiments, $R^{17}$ and $R^{18}$ are independently H, ethyl, phenyl, phenoxy, isopropyl, benzyl, or

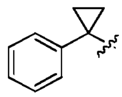.

In some embodiments, $R^{17}$ and $R^{18}$ are independently H, phenyl, phenoxy, isopropyl, or benzyl.

In some embodiments, $R^{25}$ and $R^{26}$ are independently H, methyl, cyclopropyl, or

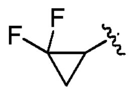.

In some embodiments, $R^{25}$ and $R^{26}$ are independently H, methyl, or cyclopropyl.

In some embodiments, $R^{21}$, $R^{22}$, and $R^{27}$ are H or $C_1$-$C_{10}$ alkyl.

In some embodiments, $R^{21}$, $R^{22}$, and $R^{27}$ are H or methyl.

In some embodiments, $R^{17}$ and $R^{18}$, together with the atom $X_5$ to which they are attached, form 3- to 10-membered heterocyclyl or $C_3$-$C_{10}$ cycloalkyl, said 3- to 10-membered heterocyclyl or $C_3$-$C_{10}$ cycloalkyl is optionally substituted with one or more $R^{17a}$.

In some embodiments, $R^{17}$ and $R^{18}$, together with the atom $X_5$ to which they are attached, form 10-membered heterocyclyl, cyclopropyl, or phenyl-substituted cyclopropyl, said 10-membered heterocyclyl is

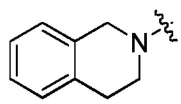, 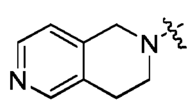, 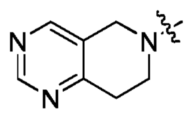,

or

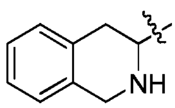.

In some embodiments, $R^{17}$ and $R^{18}$, together with the atom $X_5$ to which they are attached, form 10-membered heterocyclyl or phenyl-substituted cyclopropyl, said 10-membered heterocyclyl is

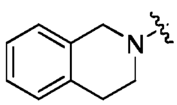, 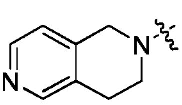, 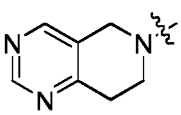, or 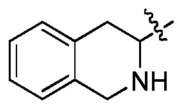.

In some embodiments, $R^{20}$ is 5- to 10-membered heteroaryl, 5- to 10-membered heterocyclyl, or -C(O)-$C_6$-$C_{10}$ aryl, said 5- to 10-membered heteroaryl, 5- to 10-membered heterocyclyl, or -C(O)-$C_6$-$C_{10}$ aryl is optionally substituted with one or more $R^{20a}$.

In some embodiments, $R^{20}$ is 5- to 10-membered heteroaryl, 5- to 10-membered heterocyclyl, or -C(O)-$C_6$-$C_{10}$ aryl, said 5- to 10-membered heteroaryl or 5- to 10-membered heterocyclyl is optionally substituted with one or more $R^{20a}$.

In some embodiments, $R^{20}$ is 5- to 10-membered heteroaryl, said 5- to 10-membered heteroaryl is optionally substituted with one or more $R^{20a}$.

In some embodiments, $R^{20}$ is 5- to 6-membered heteroaryl, said 5- to 6-membered heteroaryl is optionally substituted with one or more $R^{20a}$.

In some embodiments, $R^{20a}$ is F, =O, OH, $C_1$-$C_{10}$ alkyl, $C_1$-$C_{10}$ alkoxy, $C_3$-$C_{10}$ cycloalkyl, $C_6$-$C_{10}$ aryl, or 3- to 10-membered heterocyclyl, wherein said $C_1$-$C_{10}$ alkyl, $C_1$-$C_{10}$ alkoxy, $C_3$-$C_{10}$ cycloalkyl, $C_6$-$C_{10}$ aryl, or 3- to 10-membered heterocyclyl is optionally substituted with one or more $R^{20b}$.

In some embodiments, $R^{20a}$ is =O, $C_1$-$C_{10}$ alkyl, $C_3$-$C_{10}$ cycloalkyl, or $C_6$-$C_{10}$ aryl, wherein said $C_1$-$C_{10}$ alkyl is optionally substituted with one or more $R^{20b}$.

In some embodiments, $R^{20b}$ is F or OH.

In some embodiments, $R^{20b}$ is F.

In some embodiments, $R^{20a}$ is F, =O, OH, $CH_3$, $CF_3$, $-CH_2OH$, $-CH_2CH_2OH$, $-CH_2CH_2F$, cyclopropyl, methoxy, oxetanyl, or phenyl.

In some embodiments, $R^{20a}$ is =O, $CH_3$, $CF_3$, cyclopropyl, or phenyl.

In some embodiments, $R^{20}$ is 5- to 10-membered heteroaryl, 5- to 10-membered heterocyclyl, or -C(O)-$C_6$-$C_{10}$ aryl, said 5- to 10-membered heteroaryl is pyrazolyl, pyridyl, pyrimidinyl, oxazolyl, isoxazolyl, oxadiazolyl, isothiazolyl, 1,2,3-triazolyl, pyridazinyl, triazinyl, benzopyrazolyl, or pyridopyrazolyl, said 5- to 10-membered heterocyclyl is dihydropyridinyl, and said $C_6$-$C_{10}$ aryl is phenyl.

In some embodiments, $R^{20}$ is 5- to 10-membered heteroaryl, 5- to 10-membered heterocyclyl, or C(O)-$C_6$-$C_{10}$ aryl, said 5- to 10-membered heteroaryl is pyrazolyl, isoxazolyl, 1,2,3-triazolyl, benzopyrazolyl, or pyridopyrazolyl, said 5- to 10-membered heterocyclyl is dihydropyridinyl, and said $C_6$-$C_{10}$ aryl is phenyl.

In some embodiments, $R^{20}$ is 5- to 10-membered heteroaryl, 5- to 10-membered heterocyclyl, or -C(O)-$C_6$-$C_{10}$ aryl, said 5- to 10-membered heteroaryl is pyrazolyl, isoxazolyl, isothiazolyl, 1,2,3-triazolyl, pyridazinyl, benzopyrazolyl, or pyridopyrazolyl, said 5- to 10-membered heterocyclyl is dihydropyridinyl, and said $C_6$-$C_{10}$ aryl is phenyl.

In some embodiments, $R^{20}$ is 5- to 10-membered heteroaryl, 5- to 10-membered heterocyclyl, or -C(O)-$C_6$-$C_{10}$ aryl, said 5- to 10-membered heteroaryl is pyrazolyl, isoxazolyl, isothiazolyl, 1,2,3-triazolyl, benzopyrazolyl, or pyridopyrazolyl, said 5- to 10-membered heterocyclyl is dihydropyridinyl, and said $C_6$-$C_{10}$ aryl is phenyl.

In some embodiments, $R^{23}$ is CN or $C_1$-$C_{10}$ alkyl.

In some embodiments, $R^{23}$ is CN.

In some embodiments, $X_5$ is N or CH.

In some embodiments, $Y_5$ and $Y_6$ are independently a chemical bond, NH, or $CH_2$.

In some embodiments, $Y_5$ and $Y_6$ are independently NH or $CH_2$.

In some embodiments, $Y_5$ is a chemical bond, NH, or $CH_2$.

In some embodiments, $Y_5$ is NH or $CH_2$.

In some embodiments, $Y_6$ is NH.

In some embodiments, $Z_5$ and $Z_6$ are independently O or N-CN.

In some embodiments, $Z_5$ is O or N-CN.

In some embodiments, $Z_6$ is O.

In some embodiments, $R^5$ is

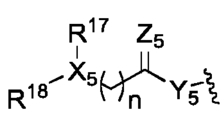, 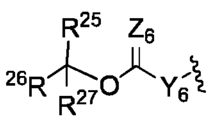, 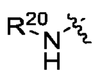, 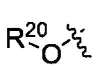, 3- to 7-membered heterocyclyl, or 5- to 6-membered heteroaryl; said 3- to 7-membered heterocyclyl or 5- to 6-membered heteroaryl is optionally substituted with one or more $R^{20a}$.

In some embodiments, $R^5$ is

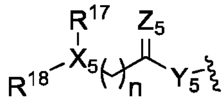, 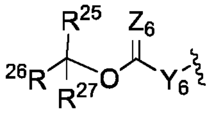, or 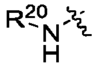.

In some embodiments, $R^5$ is

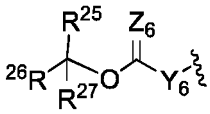 or 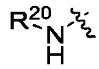.

In some embodiments, $R^5$ is

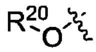,

3- to 7-membered heterocyclyl, or 5- to 6-membered heteroaryl.

In some embodiments, $R^5$ is

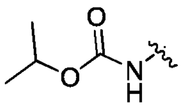, 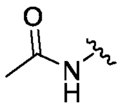, 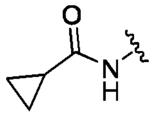, 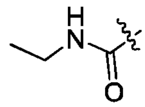,

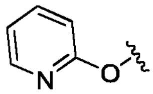, 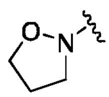, 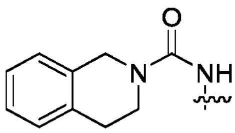, 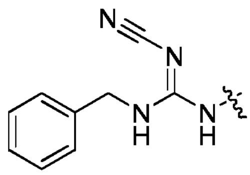, 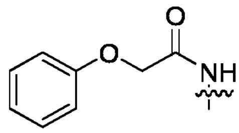,

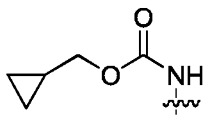, 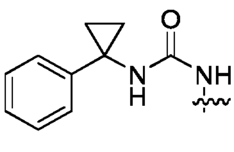, 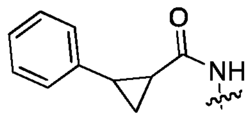, 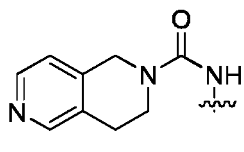,

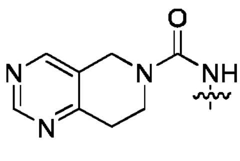, 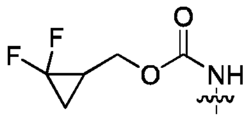, 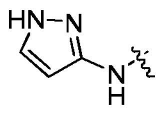, 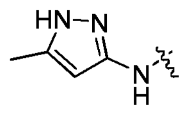, 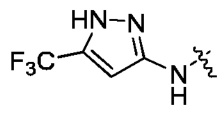,

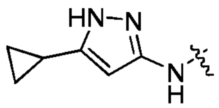, 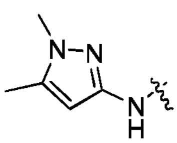, 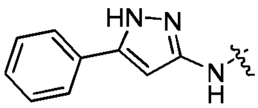, 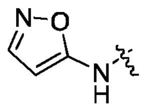, 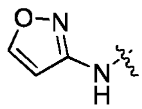,

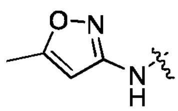, 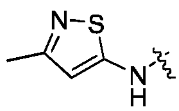, 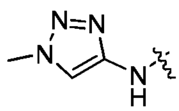, 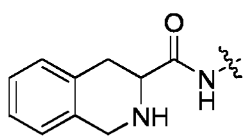, 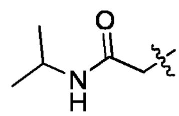,

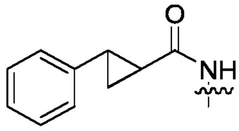, 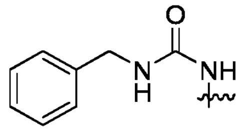, 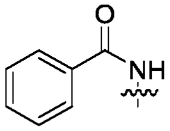, 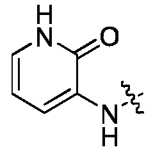, 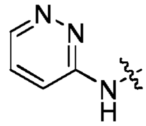,

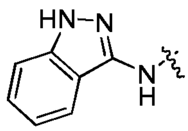, 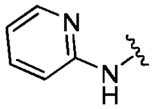, 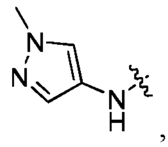, 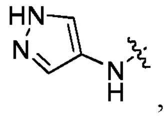, 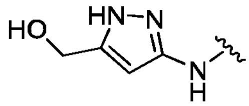, 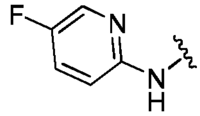,

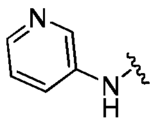, 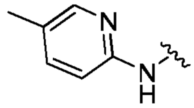, 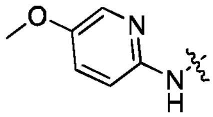, 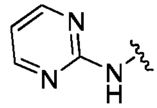, 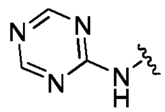,

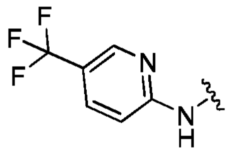, 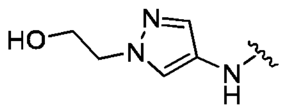, 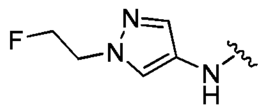, 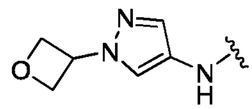, 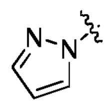,

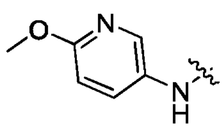, 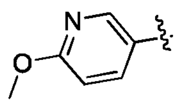, 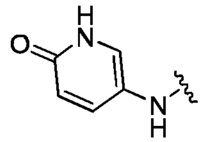, 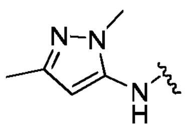, 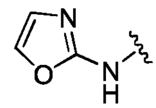, 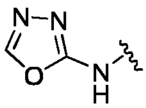,

or

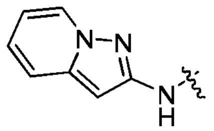.

In some embodiments, $R^5$ is

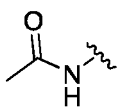, 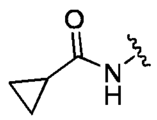, 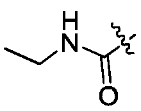, 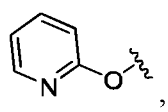, 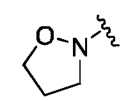,

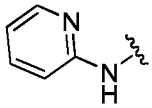, 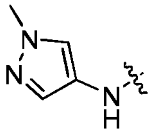, 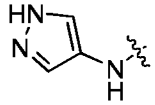, 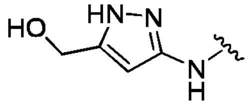, 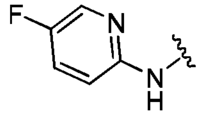, 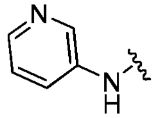,

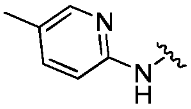, 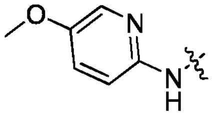, 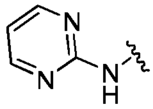, 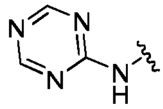, 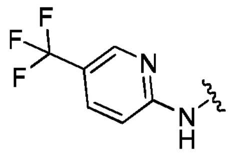,

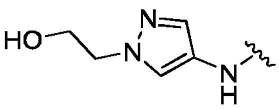, 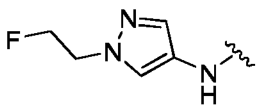, 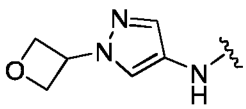, 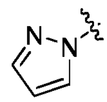, 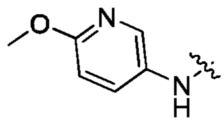,

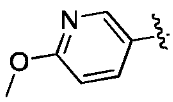, 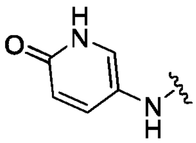, 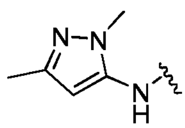, 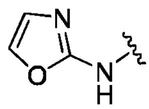, or 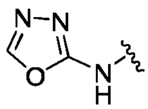.

In some embodiments, $R^5$ is

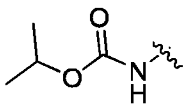, 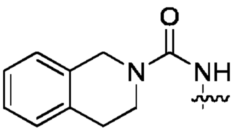, 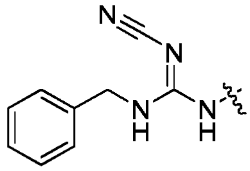,

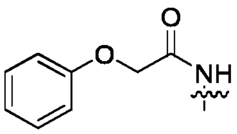, 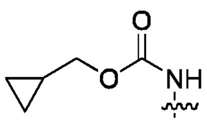, 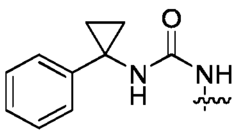, 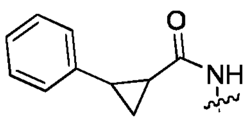,

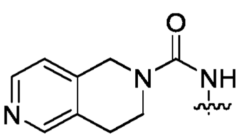, 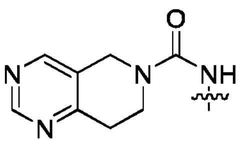, 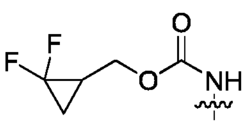, 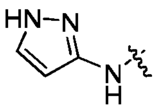, 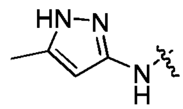,

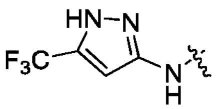, 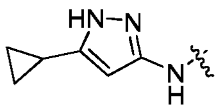, 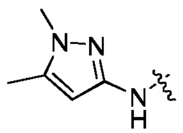, 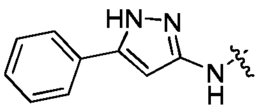, 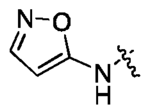,

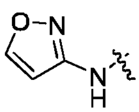, 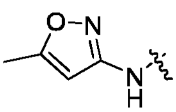, 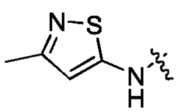, 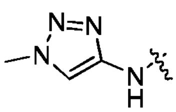, 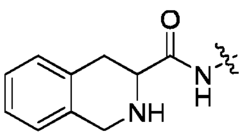, 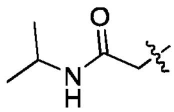,

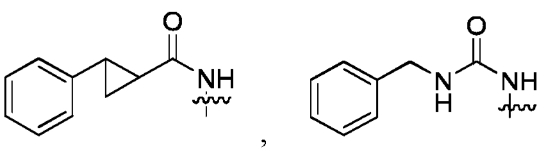, 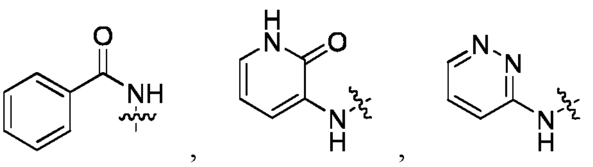,

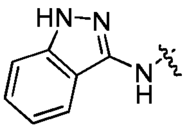, or 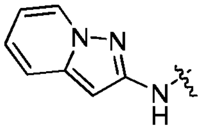.

In some embodiments, $R^5$ is

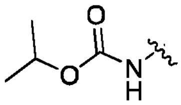, 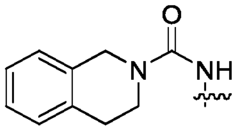, 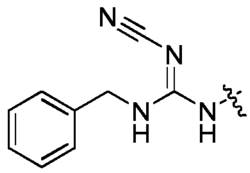,

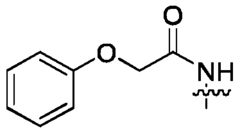, 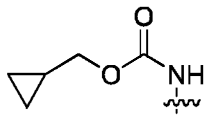, 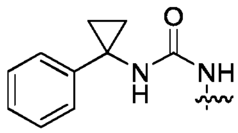, 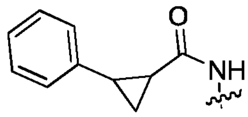,

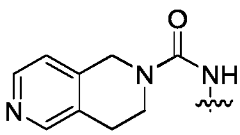, 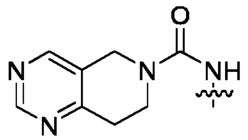, 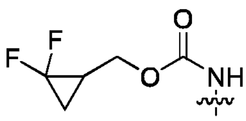, 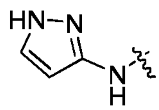,

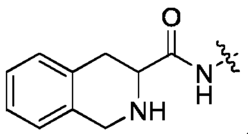, or 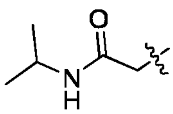.

In some embodiments, $R^4$ and $R^5$, together with the atoms to which they are attached, form 4- to 6-membered heterocyclyl, phenyl, or 5- to 6-membered heteroaryl, said 4- to 6-membered heterocyclyl, phenyl, or 5- to 6-membered heteroaryl is optionally substituted with one or more $R^{24a}$.

In some embodiments, $R^4$ and $R^5$, together with the atoms to which they are attached, form 4- to 6-membered heterocyclyl or 5- to 6-membered heteroaryl, said 4- to 6-membered heterocyclyl or 5- to 6-membered heteroaryl is optionally substituted with one or more $R^{24a}$.

In some embodiments, $R^4$ and $R^5$, together with the atoms to which they are attached, form 6-membered heterocyclyl or 5-membered heteroaryl, said 6-membered heterocyclyl or 5-membered heteroaryl is optionally substituted with one or more $R^{24a}$.

In some embodiments, ring Q in

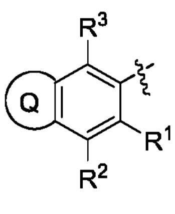

is 6-membered heterocyclyl or 5-membered heteroaryl, said 6-membered heterocyclyl or 5-membered heteroaryl is optionally substituted with $R^{24a}$.

In some embodiments,

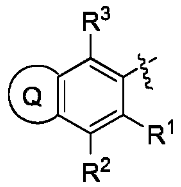

is

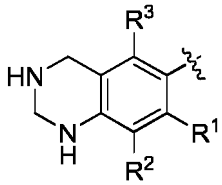

optionally substituted with one or more $R^{24a}$, or

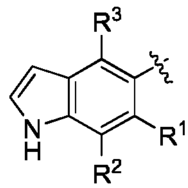

optionally substituted with one or more $R^{24a}$.

In some embodiments,

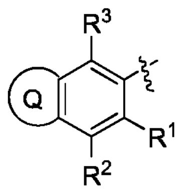

is

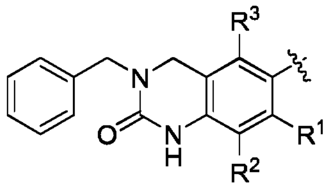

or

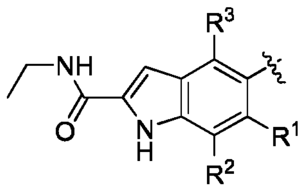

.

In some embodiments, $R^{24a}$ is F, Cl, Br, I, OH, CN, =O, or the following group optionally substituted with one or more $R^{24b}$: $NH_2$, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl.

In some embodiments, $R^{24a}$ is =O or $C_1$-$C_{10}$ alkyl optionally substituted with one or more $R^{24b}$.

In some embodiments, $R^{24a}$ is =O or methyl optionally substituted with one or more $R^{24b}$.

In some embodiments, $R^{24b}$ is F, Cl, Br, I, OH, CN, =O, or the following group optionally substituted with one or more $R^{24c}$: $NH_2$, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl.

In some embodiments, $R^{24b}$ is =O, $NH_2$ optionally substituted with one or more $R^{24c}$, or $C_6$-$C_{10}$ aryl optionally substituted with one or more $R^{24c}$.

In some embodiments, $R^{24b}$ is =O, $-NHCH_2CH_3$, or phenyl.

In some embodiments, $R^{24c}$ is F, Cl, Br, I, OH, CN, =O, $NO_2$, $NH_2$, SH, or $C_1$-$C_{10}$ alkyl.

In some embodiments, $R^{24c}$ is $C_1$-$C_{10}$ alkyl.

In some embodiments, $R^{24c}$ is ethyl.

In some embodiments, $R^{24a}$ is =O,

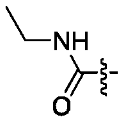 , or 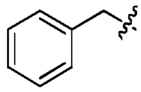 .

In some embodiments, $R^6$ is the following group optionally substituted with one or more $R^{6a}$: $NH_2$, $C_1$-$C_{10}$ alkyl, $C_3$-$C_{10}$ cycloalkyl, 3- to 10-membered heterocyclyl, $C_6$-$C_{10}$ aryl, or 5-to 10-membered heteroaryl.

In some embodiments, $R^{6a}$ is F, Cl, Br, I, OH, CN, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl.

In some embodiments, $R^6$ is $C_1$-$C_{10}$ alkyl.

In some embodiments, $R^6$ is isopropyl.

In some embodiments, when $R^7$ is

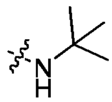 ,

$R^{17}$ and $R^{18}$, together with the atom $X_5$ to which they are attached, form 3- to 10-membered heterocyclyl or $C_3$-$C_{10}$ cycloalkyl, said 3- to 10-membered heterocyclyl or $C_3$-$C_{10}$ cycloalkyl is optionally substituted with one or more $R^{17a}$.

In some embodiments, when $R^7$ is

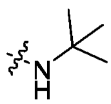 ,

n is 1.

In some embodiments, when $R^7$ is

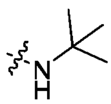 ,

$X_5$ is $CR^{21}$.

In some embodiments, when $R^7$ is

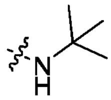,

$Y_5$ and $Y_6$ are independently $CHR^{22}$.

In some embodiments, when $R^7$ is

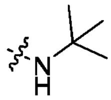,

$Z_5$ and $Z_6$ are independently $NR^{23}$.

In some embodiments, when $R^7$ is

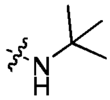

and $R^5$ is

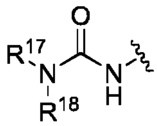,

$R^{17}$ and $R^{18}$ are independently H, $C_1$-$C_{10}$ alkyl, $C_3$-$C_{10}$ cycloalkyl, 3- to 10-membered heterocyclyl, 5- to 10-membered heteroaryl, $C_6$-$C_{10}$ aryl, or $C_6$-$C_{10}$ aryloxy, said $C_1$-$C_{10}$ alkyl, $C_3$-$C_{10}$ cycloalkyl, 3- to 10-membered heterocyclyl, 5- to 10-membered heteroaryl, $C_6$-$C_{10}$ aryl, or $C_6$-$C_{10}$ aryloxy is optionally substituted with one or more $R^{17a}$; or $R^{17}$ and $R^{18}$, together with the N atom to which they are attached, form 3- to 10-membered heterocyclyl, said 3- to 10-membered heterocyclyl is optionally substituted with one or more $R^{17a}$.

In some embodiments, when $R^7$ is

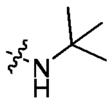

and $R^5$ is

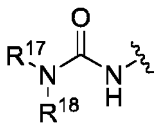,

$R^{17}$ and $R^{18}$ are independently H, $C_3$-$C_{10}$ cycloalkyl, or 3- to 10-membered heterocyclyl, said $C_3$-$C_{10}$ cycloalkyl or 3- to 10-membered heterocyclyl is optionally substituted with one or more $R^{17a}$; or $R^{17}$ and $R^{18}$, together with the N atom to which they are attached, form 9- to 10-membered heterocyclyl, said 9- to 10-membered heterocyclyl is optionally substituted with one or more $R^{17a}$.

In some embodiments, when $R^7$ is 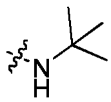, $R^{25}$ and $R^{26}$ are independently H, $C_1$-$C_{10}$ alkyl, $C_3$-$C_{10}$ cycloalkyl, 3- to 10-membered heterocyclyl, 5- to 10-membered heteroaryl, $C_6$-$C_{10}$ aryl, or $C_6$-$C_{10}$ aryloxy, said $C_1$-$C_{10}$ alkyl, $C_3$-$C_{10}$

cycloalkyl, 3- to 10-membered heterocyclyl, 5- to 10-membered heteroaryl, $C_6$-$C_{10}$ aryl, or $C_6$-$C_{10}$ aryloxy is optionally substituted with one or more $R^{17a}$.

In some embodiments, when $R^7$ is 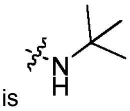, $R^{25}$ and $R^{26}$ are independently H or $C_3$-$C_{10}$ cycloalkyl, $R^{27}$ is H, said $C_3$-$C_{10}$ cycloalkyl is optionally substituted with one or more $R^{17a}$.

In some embodiments, when $R^7$ is 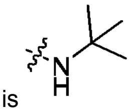, $R^{25}$ is $C_3$-$C_6$ cycloalkyl, both of $R^{26}$ and $R^{27}$ are H, said $C_3$-$C_6$ cycloalkyl is optionally substituted with one or more F, Cl, Br, or I.

In some embodiments, the compound of formula (I) or a pharmaceutically acceptable salt thereof is the following compound or a pharmaceutically acceptable salt thereof:

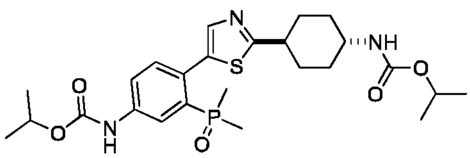
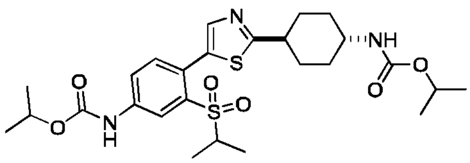

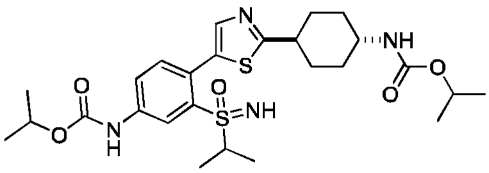
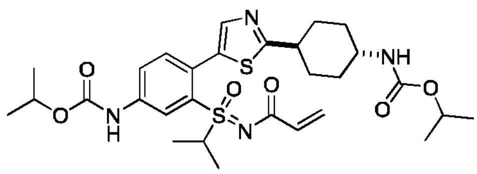

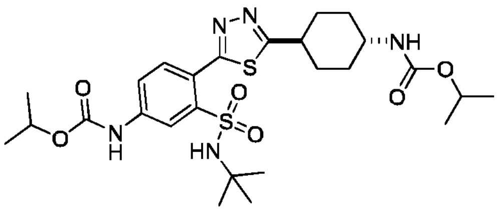
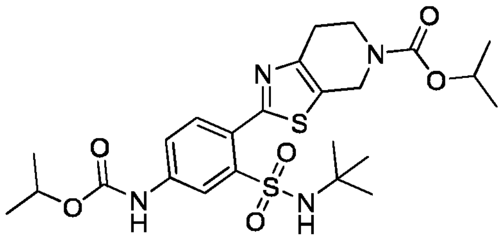

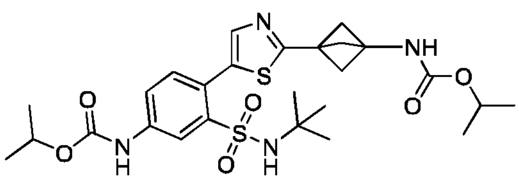
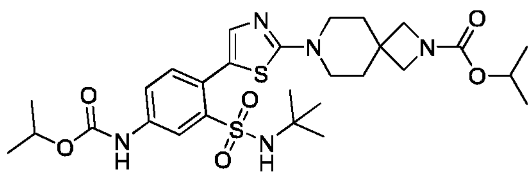

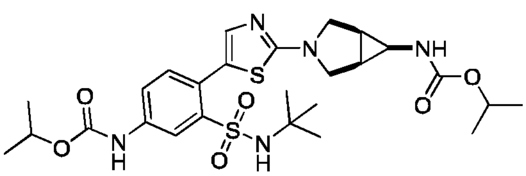
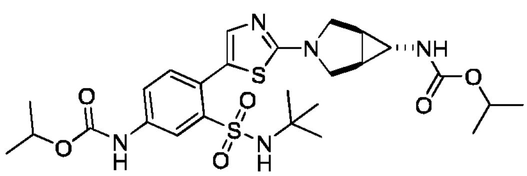

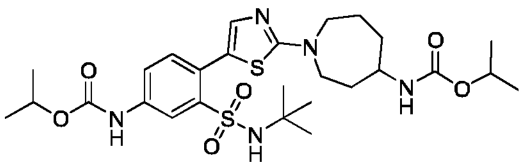
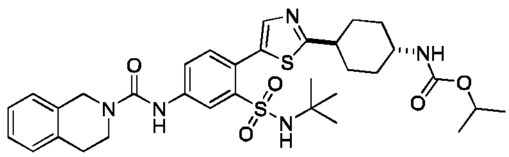

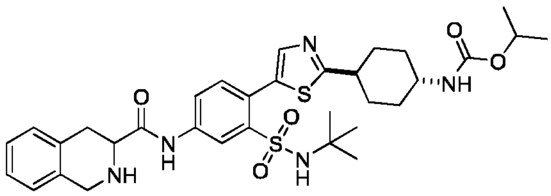
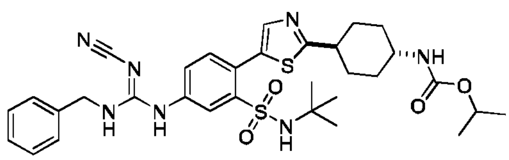

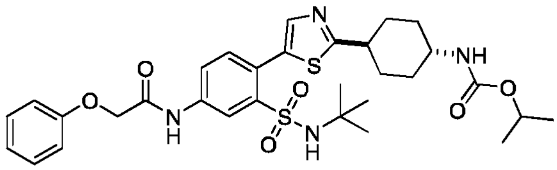
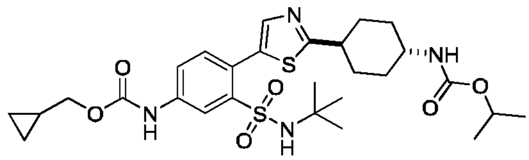

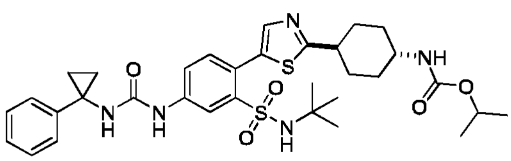
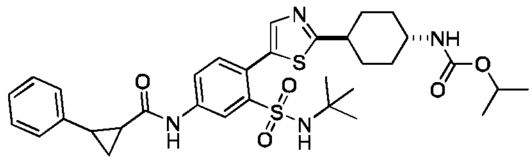

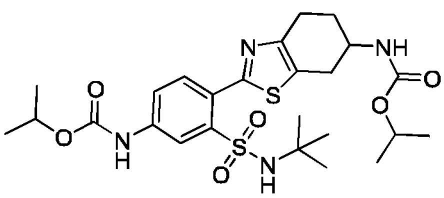
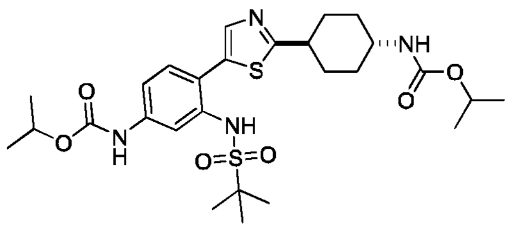

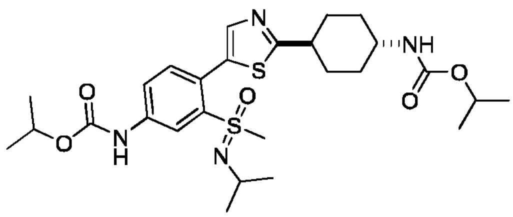
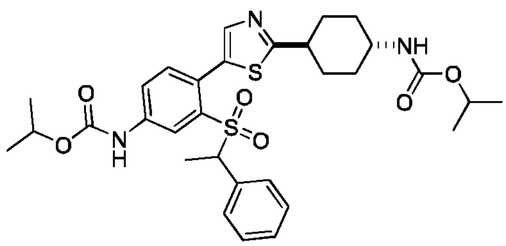

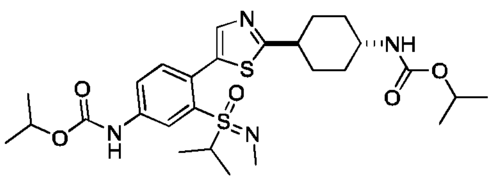
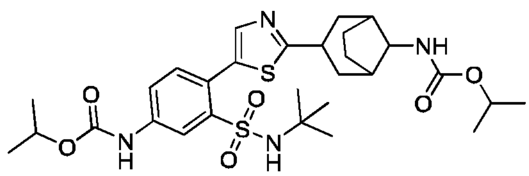

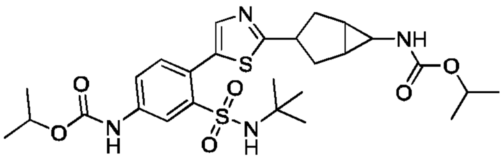
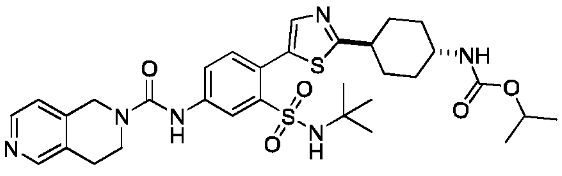

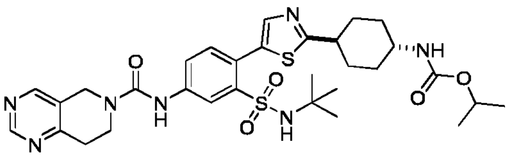
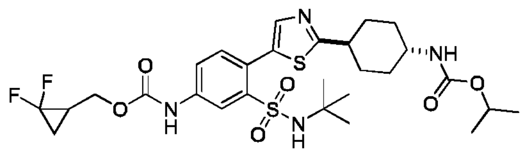

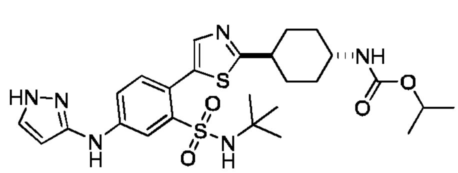
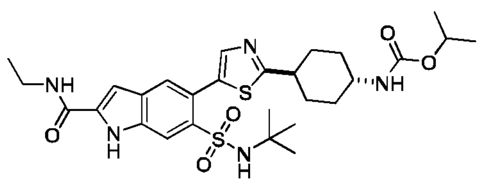

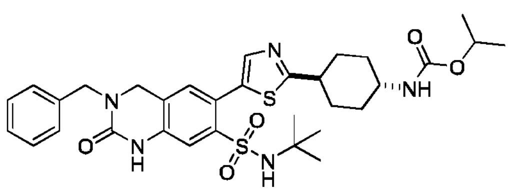
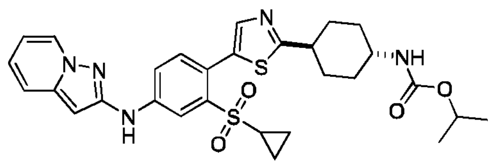

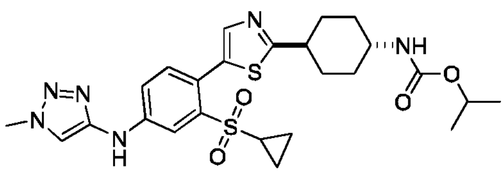
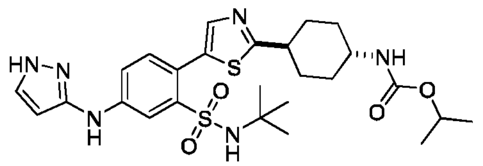

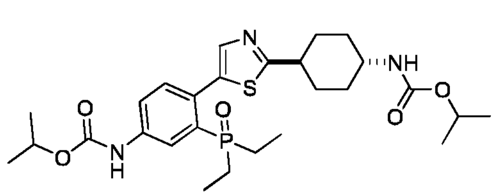
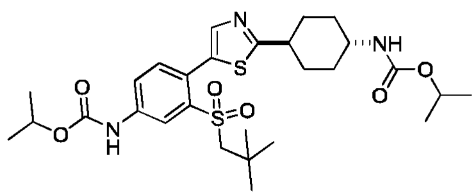

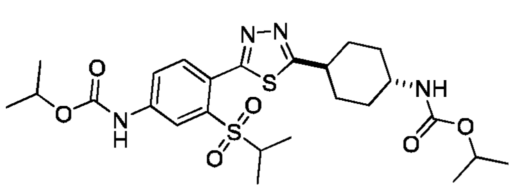
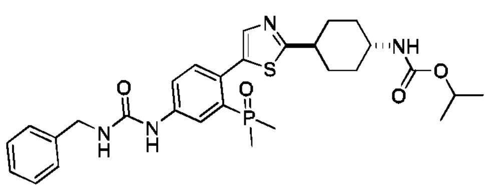

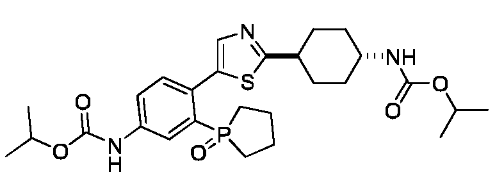
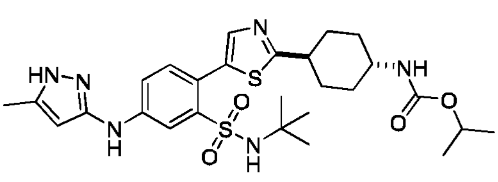

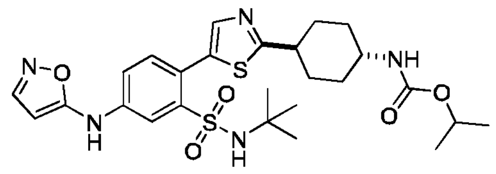
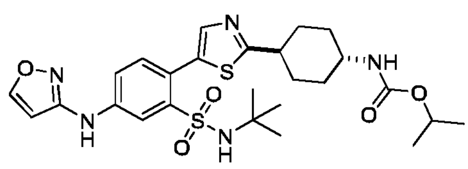

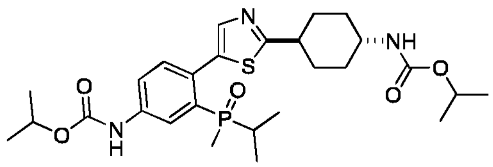
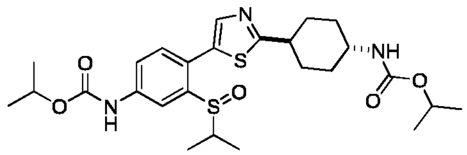

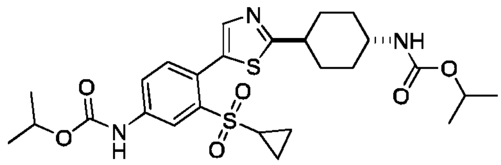
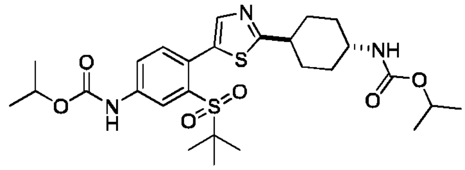

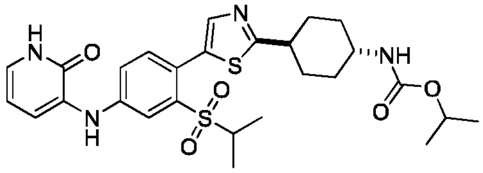
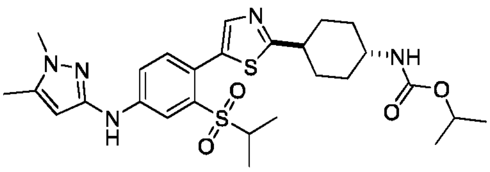

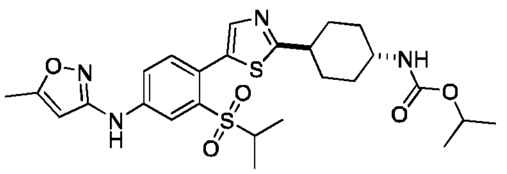
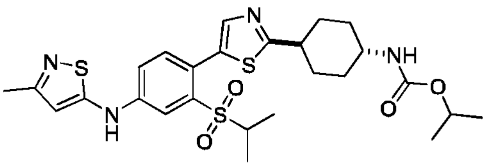

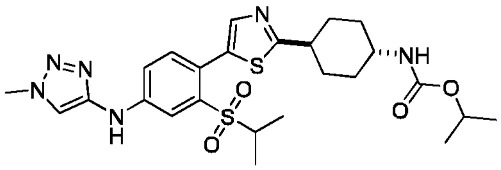
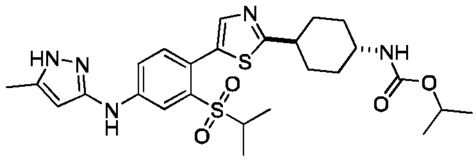

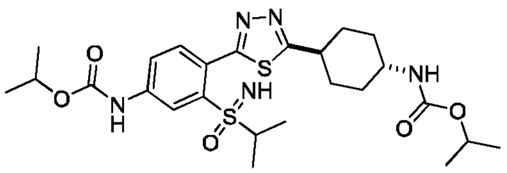
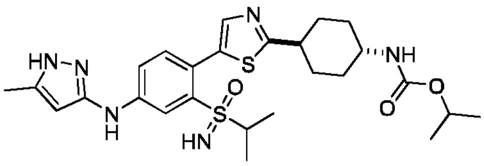

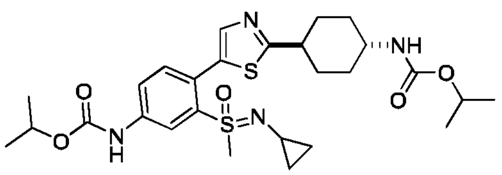
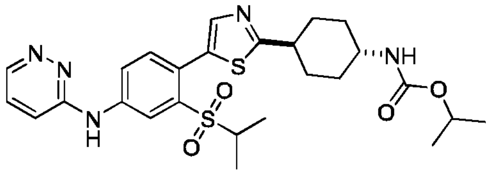

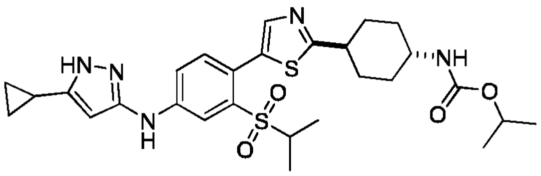
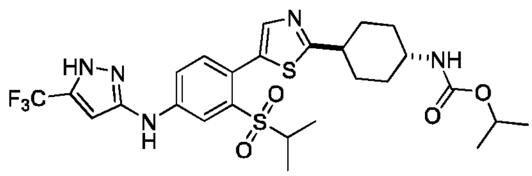

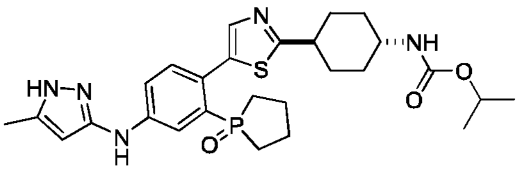
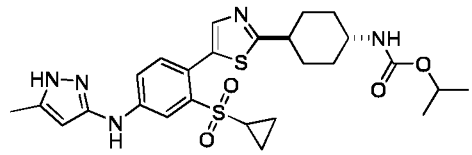

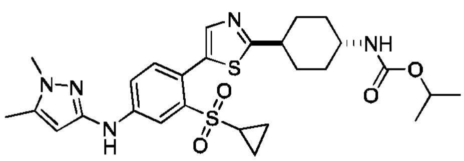
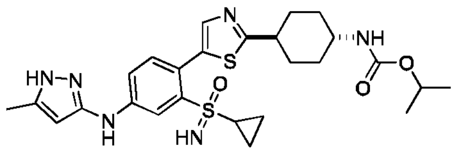

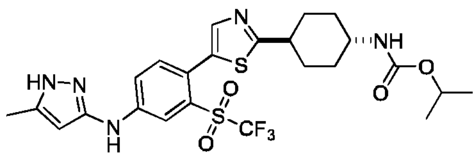
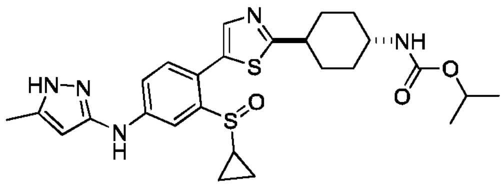

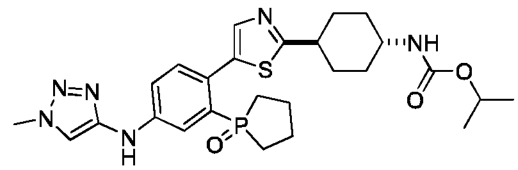
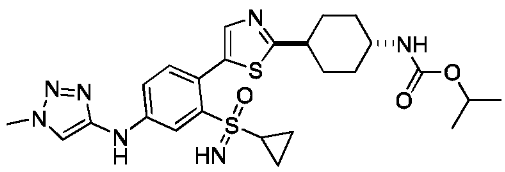

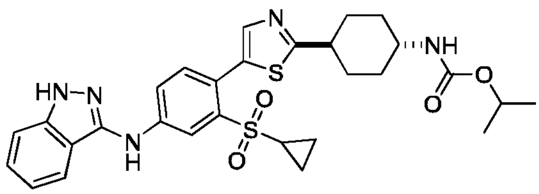
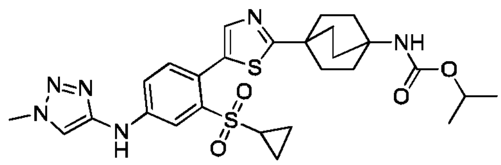

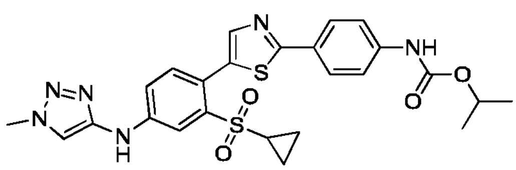
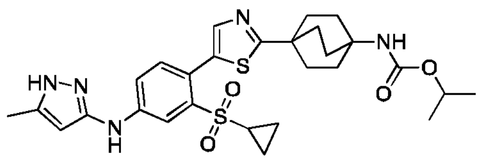

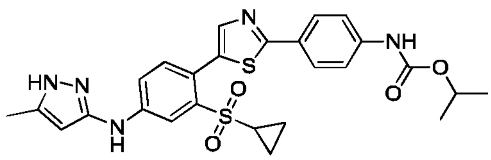
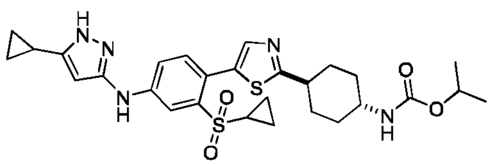

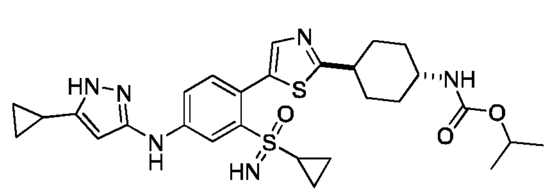
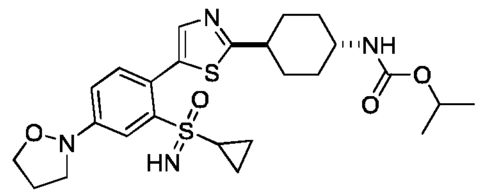

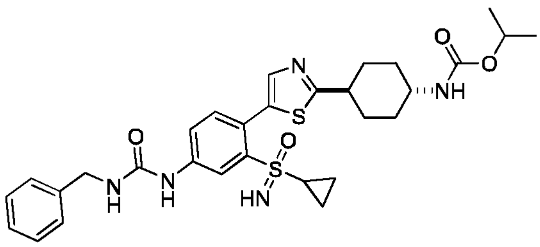
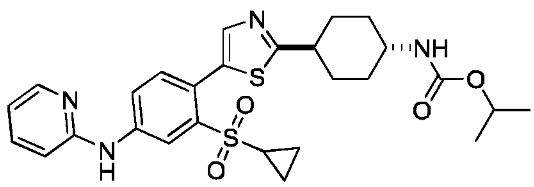

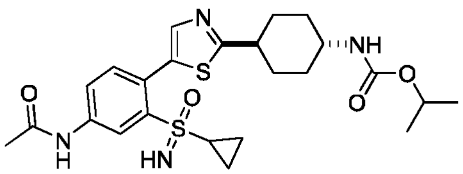
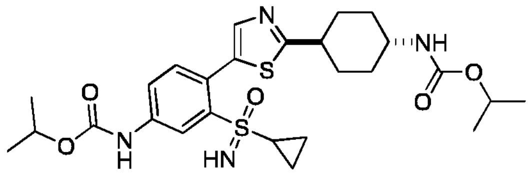

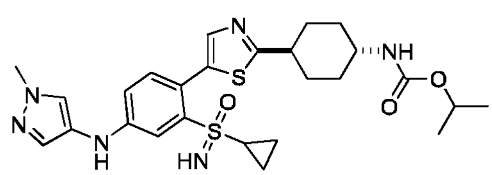
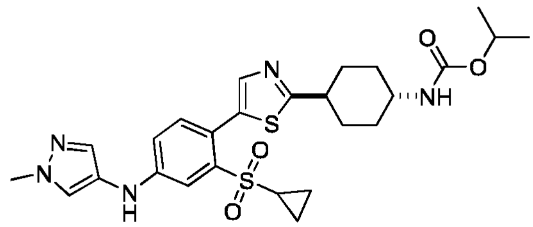

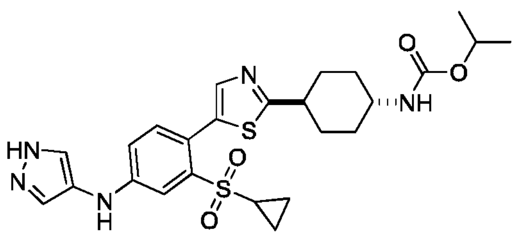
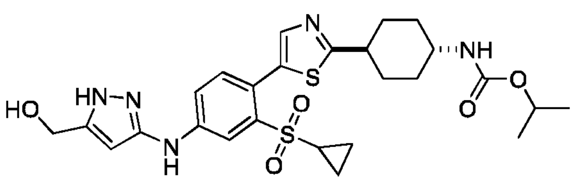

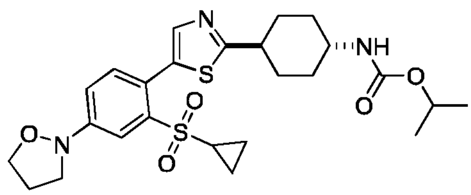
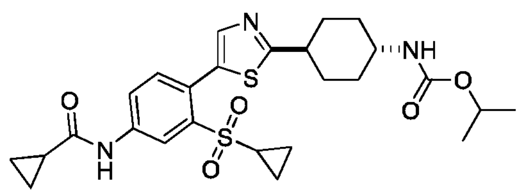

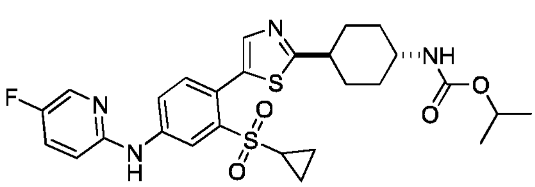
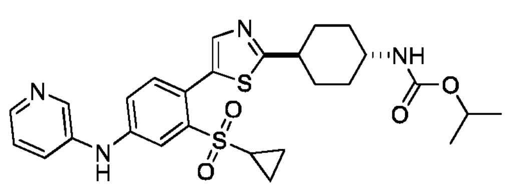

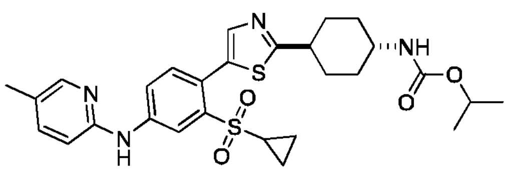

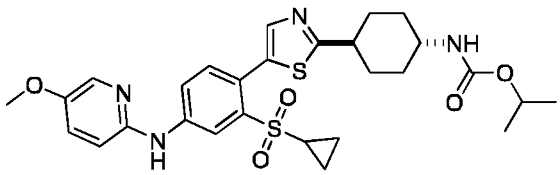

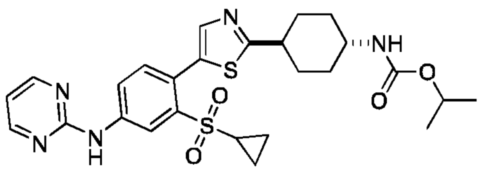

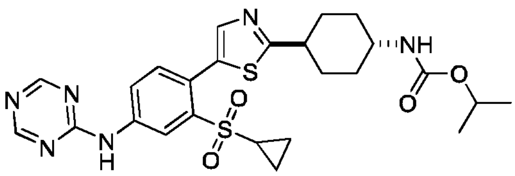

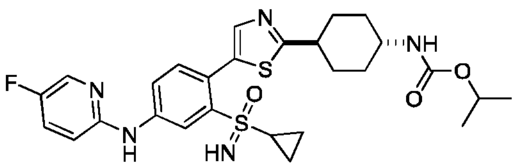

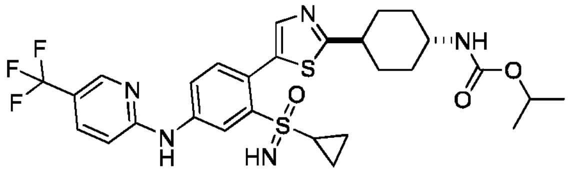

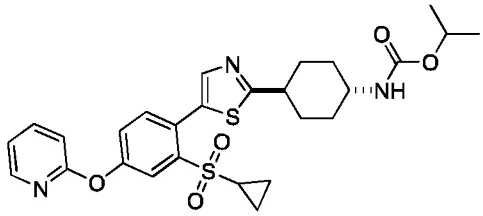

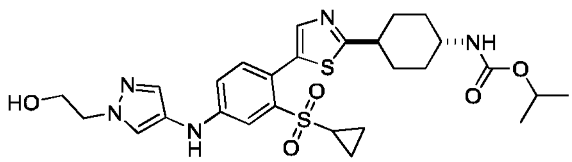

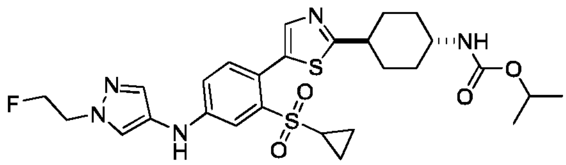

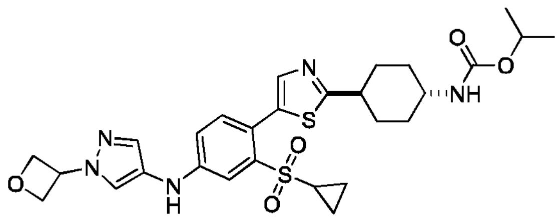

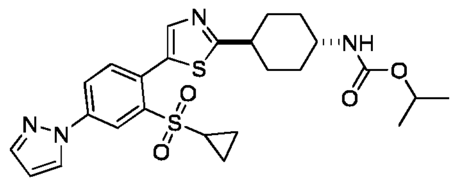

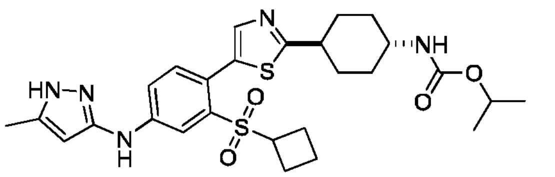

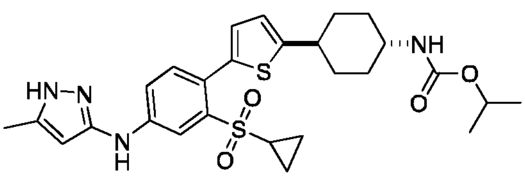

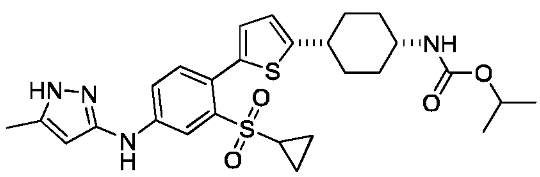

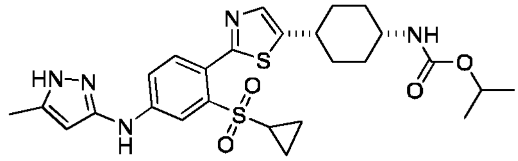

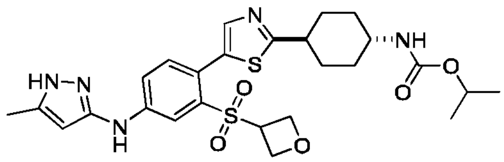

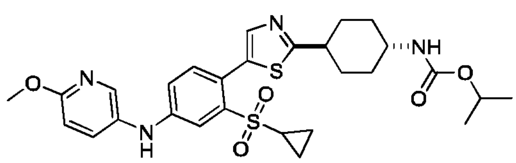

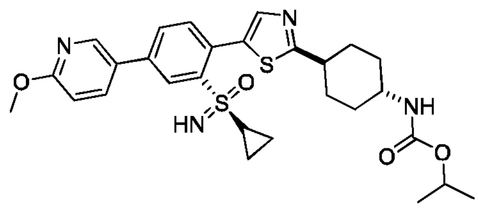

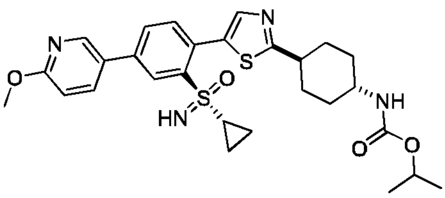

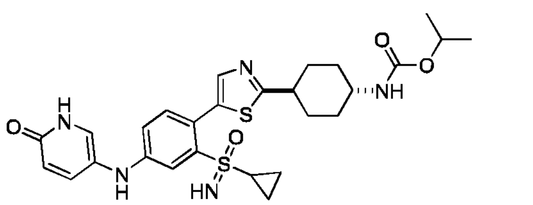

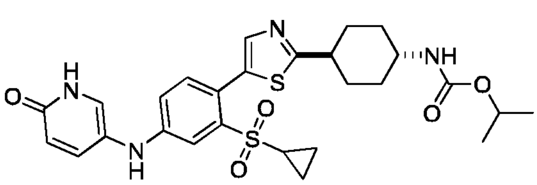

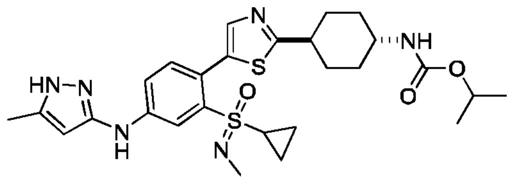

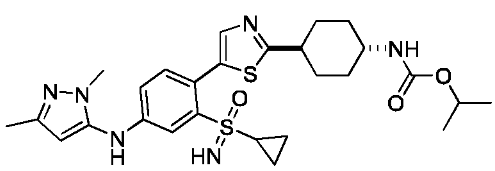

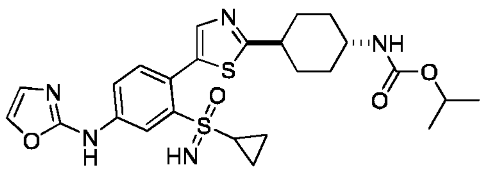

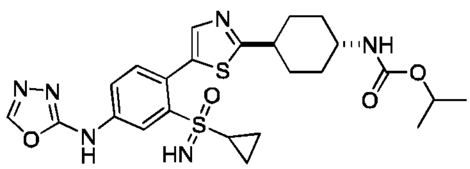

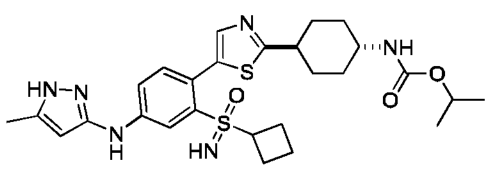

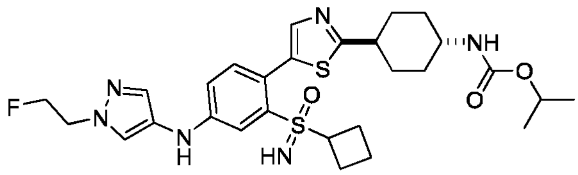

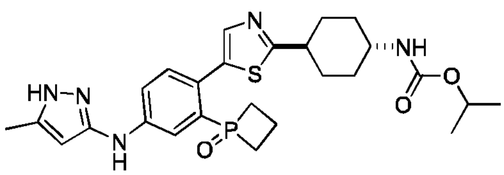

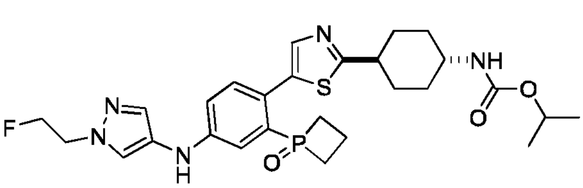

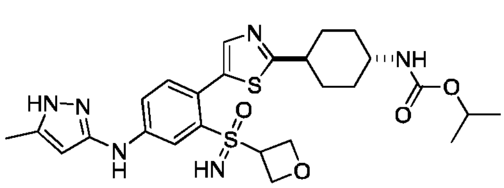

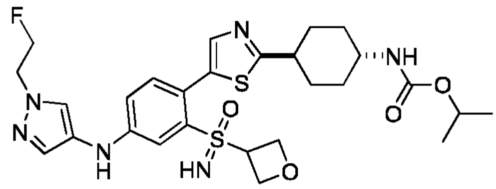 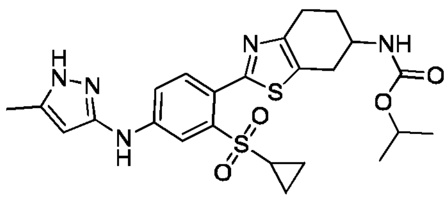

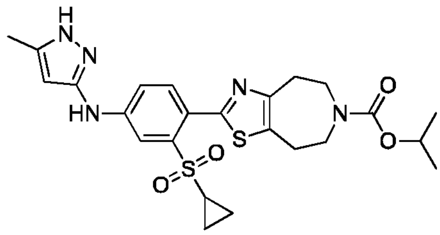 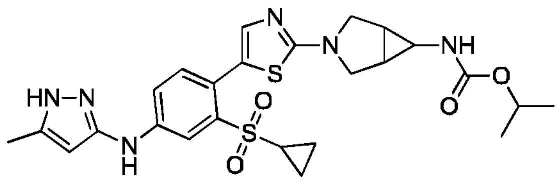

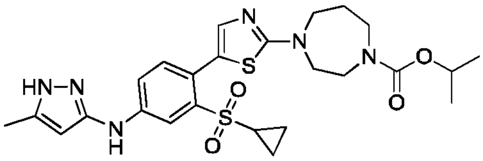 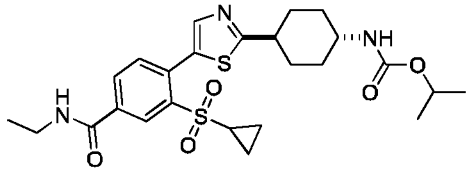

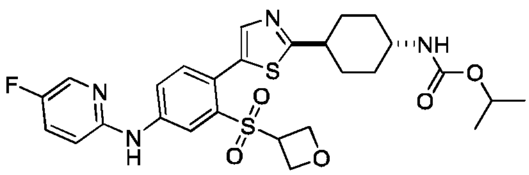 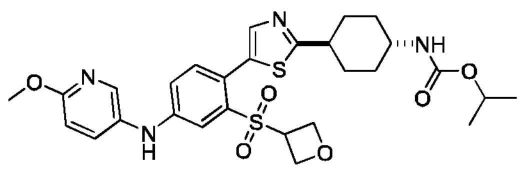

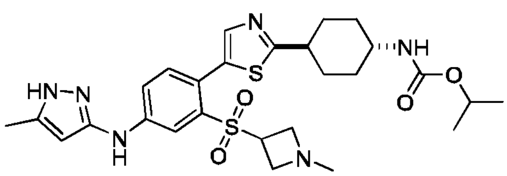 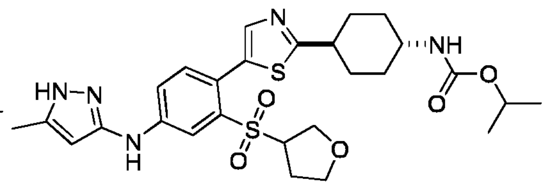

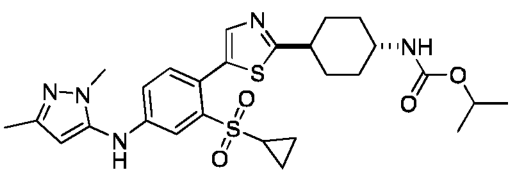 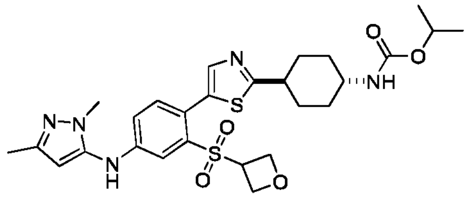

,

or

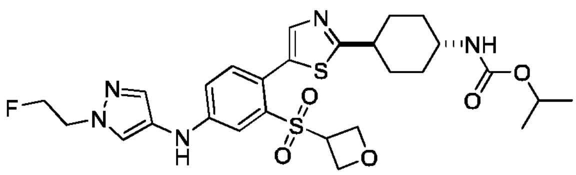

.

The present disclosure further provides a pharmaceutical composition, comprising a compound represented by formula (I), formula (Ia), formula (Ib), formula (II), formula (IIa), formula (IIb), formula (IV), formula (V), formula (VII), formula (VIII), formula (IX), formula I-1, formula I-7, formula I-11, formula I-13, formula I-23, or formula 1-27 or a pharmaceutically acceptable salt thereof, and a pharmaceutically acceptable excipient.

Furthermore, the present disclosure relates to the use of a compound represented by formula (I), formula (Ia), formula (Ib), formula (II), formula (IIa), formula (IIb), formula (IV), formula (V), formula (VII), formula (VIII), formula (IX), formula I-1, formula I-7, formula I-11, formula I-13, formula I-23, or formula 1-27 or a pharmaceutically acceptable salt thereof, or a pharmaceutical composition comprising the same in the preparation of a medicament for preventing or treating RAD51-associated diseases.

Furthermore, the present disclosure relates to the use of a compound represented by formula (I), formula (Ia), formula (Ib), formula (II), formula (IIa), formula (IIb), formula (IV), formula (V), formula (VII), formula (VIII), formula (IX), formula I-1, formula I-7, formula I-11, formula I-13, formula I-23, or formula 1-27 or a pharmaceutically acceptable salt thereof, or a pharmaceutical composition comprising the same for preventing or treating RAD51-associated diseases.

Furthermore, the present disclosure relates to a compound represented by formula (I), formula (Ia), formula (Ib), formula (II), formula (IIa), formula (IIb), formula (IV), formula (V), formula (VII), formula (VIII), formula (IX), formula I-1, formula I-7, formula I-11, formula I-13, formula I-23, or formula 1-27 or a pharmaceutically acceptable salt thereof or a pharmaceutical composition comprising the same, for use in prevention or treatment of RAD51-associated diseases.

The present disclosure further relates to a method for treating RAD51-associated diseases, the method comprises administering to a subject a therapeutically effective amount of a pharmaceutical preparation comprising the compound of formula (I), formula (Ia), formula (Ib), formula (II), formula (IIa), formula (IIb), formula (IV), formula (V), formula (VII), formula (VIII), formula (IX), formula I-1, formula I-7, formula I-11, formula I-13, formula I-23, or formula 1-27 of the present disclosure or a pharmaceutically acceptable salt thereof.

In a preferred embodiment of the present disclosure, the RAD51-associated diseases include, but are not limited to, tumors (such as breast cancer, non-small cell lung cancer, and prostate cancer), autoimmune diseases (such as rheumatic arthritis, inflammatory bowel disease, and systemic lupus erythematosus), and the like.

Definitions and Description of Terms

Unless otherwise specified, the definitions of the groups and terms as set forth in the specification and claims of the present disclosure include their definitions as examples, exemplary definitions, preferred definitions, definitions listed in tables, definitions of specific compounds in the Examples, etc., which may be arbitrarily combined or incorporated with one another. The definitions of the groups and the structures of the compounds derived from such combination and incorporation shall fall within the scope described in the specification of the disclosure.

In the present disclosure, "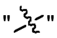" denotes a linking site.

The term "pharmaceutically acceptable salt" refers to a pharmaceutically acceptable salt of a non-toxic acid or base, including salts of inorganic acids and bases and salts of organic acids and bases.

The term "stereoisomer" refers to isomers resulting from different spatial arrangements of the atoms in a molecule, including cis-trans-isomers, enantiomers, diastereomers, and conformers.

The compounds of the present disclosure may have asymmetric atom(s), such as asymmetric carbon atom(s), asymmetric sulfur atom(s), asymmetric nitrogen atom(s), and asymmetric phosphorus atom(s) (optical center(s)), or asymmetric double bond(s). Racemates, enantiomers, diastereomers, geometric isomers, and single isomers are all included within the scope of the present disclosure.

The graphical representation of racemates or enantiomerically pure compounds provided herein comes from Maehr, J. Chem. Ed. 1985, 62:114-120. Unless otherwise specified, the wedge-shaped solid bond and the wedge-shaped dashed-line bond (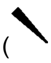 and 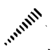) are used to represent the absolute configuration of a stereogenic center, and the black solid-line bond and the dashed-line bond (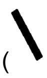 and 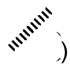) are used to represent cis- and trans-configuration of a alicyclic compound. When containing an olefinic double bond or additional geometrically asymmetrical center(s), the compounds provided herein include E and Z geometric isomers unless otherwise specified. Likewise, all tautomeric forms are included within the scope of the present disclosure.

The compounds of the present disclosure may have specific geometrically isomeric form or stereoisomeric form. The present disclosure contemplates all such compounds, including cis- and trans-isomers, (-)- and (+)-enantiomers, (R)- and (S)-enantiomers, diastereomers, (D)-isomers, (L)-isomers, as well as the racemic mixtures and other mixtures thereof, such as enantiomerically or diastereomerically enriched mixtures, and all these mixtures fall within the scope of the present disclosure. Additional asymmetric carbon atom(s), asymmetric sulfur atom(s), asymmetric nitrogen atom(s), or asymmetric phosphorus atom(s) may be present in the substituent(s) such as alkyl. All these isomers and the mixtures thereof are included within the scope of the present disclosure. The compounds containing asymmetric

atom(s) in the present application may be isolated in an enantiomerically pure form or in a racemic form. The enantiomerically pure form may be obtained by resolution from racemic mixtures or by synthesis using chiral raw materials or chiral reagents.

The term "tautomer" refers to an isomer in which functional group isomerism is formed due to the rapid movement of a certain atom between two positions in the molecule. The compounds of the present disclosure may exhibit tautomerism. Tautomeric compounds may exist as two or more interconvertible species. Prototropic tautomers arise from the migration of a hydrogen atom through which two atoms are covalently bonded. Tautomers generally exist in equilibrium. In an attempt to isolate a single tautomer, a mixture is often produced, and the physical and chemical properties of the single tautomer are consistent with those of the mixture of compounds. The position of the equilibrium depends on intramolecular chemical properties. For example, in many aliphatic aldehydes and ketones such as acetaldehyde, the keto-type isomers predominate, whereas in phenols, the enol-type isomers predominate. The present disclosure includes all tautomeric forms of compounds.

The term "pharmaceutical composition" refers to a mixture of one or more compounds described herein or a physiologically/pharmaceutically acceptable salt thereof or a prodrug thereof and other chemical components, e.g., a physiologically/pharmaceutically acceptable excipient. A pharmaceutical composition aims at facilitating the administration of a compound to an organism.

The term "substituted" means that any one or more of the hydrogen atom(s) on a specific atom are replaced with a substituent as long as the valence state of the specific atom is normal and the substituted compound is stable. When the substituent is an oxo (i.e. =O), it means that two hydrogen atoms are substituted. Oxo does not appear on an aromatic group.

The term "optional" or "optionally" means that the event or situation described later may or may not occur, and such description includes a case where said event or situation occurs and a case where said event or situation does not occur. For example, an ethyl group being "optionally" substituted with halogen means that said ethyl group may be unsubstituted ($-CH_2CH_3$), monosubstituted (such as $-CH_2CH_2F$), polysubstituted (such as $-CHFCH_2F$ or $-CH_2CHF_2$), or fully substituted ($-CF_2CF_3$). It is understandable to a person skilled in the art that, as for any group containing one or more substituents, any substitution or substitution pattern that is sterically impossible to exist and/or cannot be synthesized would not be introduced therein.

When any variable (such as $R^{7a}$) appears once or more in the composition or the structure of a compound, its definition in each case is independent.

The term "halo" or "halogen" refers to fluorine, chlorine, bromine, and iodine.

The term "$C_1$-$C_{10}$ alkyl" should be construed to refer to a linear or branched saturated monovalent hydrocarbyl having 1, 2, 3, 4, 5, 6, 7, 8, 9, or 10 carbon atom(s). Said alkyl is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl; "$C_1$-$C_6$ alkyl" should be construed to refer to a linear or branched saturated monovalent hydrocarbyl having 1, 2, 3, 4, 5, or 6 carbon atom(s). Preferably, "$C_1$-$C_{10}$ alkyl" may include "$C_1$-$C_6$ alkyl".

The term "$C_2$-$C_{10}$ alkenyl" should be construed to preferably refer to a linear or branched monovalent hydrocarbyl containing one or more double bonds and having 2, 3, 4, 5, 6, 7, 8, 9, or 10 carbon atoms.

The term "$C_3$-$C_{12}$ cycloalkenyl" should be construed to preferably refer to a monocyclic or bicyclic hydrocarbyl containing one or more double bonds and having 3 to 12 carbon atoms, such as cyclopropenyl, cyclobutenyl, or cyclopentenyl.

The term "$C_2$-$C_{10}$ alkynyl" should be construed to preferably refer to a linear or branched monovalent hydrocarbyl containing one or more triple bonds and having 2, 3, 4, 5, 6, 7, 8, 9, or 10 carbon atoms.

The term "$C_3$-$C_{10}$ cycloalkyl" should be construed to refer to a saturated monovalent monocyclic or bicyclic hydrocarbyl having 3 to 10 carbon atoms, for example, cyclopropyl, cyclobutyl, cyclopentyl, cyclohexyl, cycloheptyl, cyclooctyl, cyclononyl, or cyclodecyl, or bicyclic hydrocarbyls such as decalinyl. The term "$C_3$-$C_6$ cycloalkyl" should be construed to refer to a saturated monovalent monocyclic or bicyclic hydrocarbyl having 3 to 6 carbon atoms. Preferably, "$C_3$-$C_{10}$ cycloalkyl" may include "$C_3$-$C_6$ cycloalkyl".

The term "heterocyclyl" refers to a saturated or partially saturated monocyclic or polycyclic (such as bicyclic, e.g., fusedcyclic, bridged cyclic, or spirocyclic) non-aromatic group, whose ring atoms consist of carbon atoms and at least one heteroatom selected from nitrogen, oxygen, and sulfur.

The term "3- to 10-membered heterocyclyl" means a saturated or partially saturated monocyclic or bicyclic hydrocarbyl having 3 to 10 ring atoms, which contains 1 to 5, preferably 1 to 3 heteroatoms selected from N, O, and S. The term "3- to 7-membered heterocyclyl" means a saturated or partially saturated monocyclic or bicyclic hydrocarbyl having 3 to 7 ring atoms, which contains 1 to 5, preferably 1 to 3 heteroatoms selected from N, O, and S. In particular, said heterocyclyl may include, for example, a 4-membered cyclic group, such as azetidinyl or oxetanyl; a 5-membered cyclic group, such as tetrahydrofuranyl, dioxolyl, pyrrolidinyl, imidazolidinyl, pyrazolidinyl, or pyrrolinyl; or a 6-membered

cyclic group, such as tetrahydropyranyl, piperidinyl, morpholinyl, dithianyl, thiomorpholinyl, piperazinyl, or trithianyl; or a partially saturated 6-membered cyclic group, such as tetrahydropyridyl; or a 7-membered cyclic group, such as azepanyl or diazepanyl. Optionally, said 3- to 10-membered heterocyclyl may be an 8- to 10-membered benzo-fused heterocyclyl or an 8- to 10-membered heteroaryl-fused heterocyclyl, including but not limited to, for example, benzopiperidinyl, pyridopiperidinyl, pyrimidinopiperidinyl, or the like; said 3- to 10-membered heterocyclyl may further include but is not limited to a five/five-membered bicyclic group, such as hexahydrocyclopenta[c]pyrrol-2(1H)-yl cyclic group, or a five/six-membered bicyclic group, such as hexahydropyrrolo[1,2-a]pyrazin-2(1H)-yl cyclic group. The nitrogen-containing cyclic group may be partially unsaturated, that is, it may include one or more double bonds, it may be exemplified by, but not limited to, 2,5-dihydro-1H-pyrrolyl, 4H-[1,3,4]thiadiazinyl, 4,5-dihydrooxazolyl, or 4H-[1,4]thiazinyl, or it may be a benzo-fused cyclic group and may be exemplified by, but not limited to, dihydroisoquinolinyl. According to the present disclosure, said heterocyclyl is non-aromatic. Said bicyclic hydrocarbon ring comprises a bridged cyclic structure, a spirocyclic structure, or a fused cyclic structure. Preferably, "3- to 10-membered heterocyclyl" may include "3- to 7-membered heterocyclyl".

"3- to 10-membered monocyclic heterocyclyl" refers to a saturated or partially saturated monocyclic hydrocarbyl having 3 to 10 ring atoms, which contains 1 to 5, preferably 1 to 3 heteroatoms selected from N, O and S. Preferably, "3- to 10-membered monocyclic heterocyclyl" may include "3- to 7-membered monocyclic heterocyclyl".

The term "$C_7$-$C_{12}$ spirocyclyl" refers to a monovalent non-aromatic ring system in which two monocyclic rings share one carbon atom. It consists solely of carbon atoms and hydrogen atoms. It may be saturated or partially saturated, and it contains 7, 8, 9, 10, 11 or 12 ring atoms and is linked to the parent nucleus via one single bond. "$C_7$-$C_{12}$ spirocyclyl" includes, but is not limited to, spiro[2.4]heptan-1-yl, spiro[3.5]nonan-2-yl,

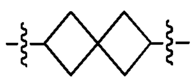
,
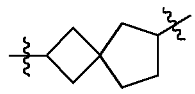
,

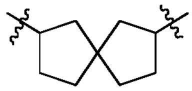
,

etc.

The term "7- to 12-membered spiroheterocyclyl" refers to a monovalent non-aromatic ring system in which two monocyclic rings share one carbon atom. It consists of carbon atoms and 1 to 3 heteroatoms selected from nitrogen, oxygen, sulfur and phosphorus. It may be saturated or partially saturated, and it contains 7, 8, 9, 10, 11 or 12 ring atoms and is linked to the parent nucleus via one single bond. "7- to 12-membered spiroheterocyclyl" includes, but is not limited to, 6-oxaspiro[3.3]heptan-2-yl, 7-azaspiro[3.5]nonan-2-yl, 2,7-diazaspiro[3.5]nonan-2-yl,

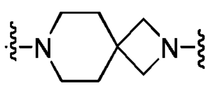
,
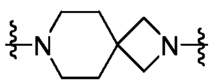
;

etc.

The term "$C_6$-$C_{12}$ fused cyclyl" refers to a polycyclic group in which each of the rings in the system shares an adjacent pair of ring atoms with the other ring(s) in the system, wherein one or more rings may contain 0 or more double bonds. It contains 6, 7, 8, 9, 10, 11 or 12 ring atoms, and the ring atoms consist solely of carbon atoms and may be substituted or unsubstituted. "$C_6$-$C_{12}$ fused cyclyl" includes, but is not limited to,

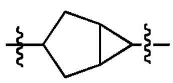
,

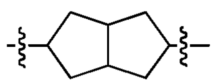
,

etc.

The term "6- to 12-membered fused heterocyclyl" refers to a polycyclic group in which each of the rings in the system shares an adjacent pair of ring atoms with the other ring(s) in the system, wherein one or more rings may contain 0 or more double bonds. It contains 6, 7, 8, 9, 10, 11 or 12 ring atoms, and the ring atoms consist of carbon atoms and 1 to 3 heteroatoms selected from nitrogen, oxygen, sulfur and phosphorus and may be substituted or unsubstituted. "6- to 12-membered fused heterocyclyl" includes, but is not limited to,

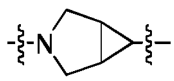,

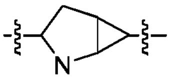, 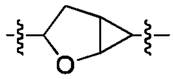,

etc.

The term "$C_5$-$C_{12}$ bridged cyclyl" refers to a monovalent non-aromatic ring system in which any two monocyclic rings share two carbon atoms that are not linked to each other directly. It consists solely of carbon atoms and hydrogen atoms. It may be saturated or partially saturated, and it contains 5, 6, 7, 8, 9, 10, 11 or 12 ring atoms and is linked to the parent nucleus via one single bond. "$C_5$-$C_{12}$ bridged cyclyl" includes, but is not limited to,

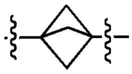,

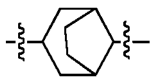, 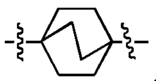,

etc.

The term "5- to 12-membered bridged heterocyclyl" refers to a monovalent non-aromatic ring system in which any two monocyclic rings share two carbon atoms that are not linked to each other directly. It consists of carbon atoms and 1 to 3 heteroatoms selected from nitrogen, oxygen, sulfur and phosphorus. It may be saturated or partially saturated, and it contains 5, 6, 7, 8, 9, 10, 11 or 12 ring atoms and is linked to the parent nucleus via one single bond. "5- to 12-membered bridged heterocyclyl" includes, but is not limited to,

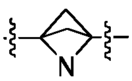, 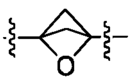,

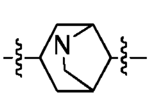, 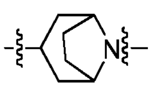, 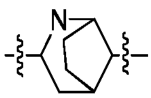, 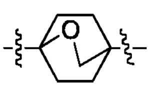,

etc.

The term "$C_6$-$C_{10}$ aryl" should be construed to preferably refer to a monovalent aromatic or partially aromatic monocyclic, bicyclic or tricyclic hydrocarbyl having 6 to 10 carbon atoms, and in particular, a cyclic group having 6 carbon atoms ("$C_6$ aryl"), such as phenyl; or a cyclic group having 9 carbon atoms ("$C_9$ aryl"), such as indanyl or indenyl, or a cyclic group having 10 carbon atoms ("$C_{10}$ aryl"), such as tetrahydronaphthyl, dihydronaphthyl, or naphthyl.

The term "5- to 10-membered heteroaryl" should be construed to include such a monovalent monocyclic, bicyclic or tricyclic aromatic ring system that has 5, 6, 7, 8, 9 or 10 ring atoms, in particular 5, 6, 9 or 10 ring atoms, contains 1 to 5, preferably 1 to 3 heteroatoms independently selected from N, O and S, and may be benzo-fused in each case. In particular, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl and the like, and the benzo derivatives thereof, such as benzofuranyl, benzothienyl, benzothiazolyl, benzoxazolyl, benzisoxazolyl, benzimidazolyl, benzotriazolyl, indazolyl, indolyl, isoindolyl, and the like; or pyridyl, pyridazinyl, pyrimidinyl, pyrazinyl, triazinyl and the like, and the benzo derivatives thereof, such as quinolinyl, quinazolinyl, isoquinolinyl and the like; or azocinyl, indolizinyl, purinyl and the like, and the benzo derivatives thereof; or cinnolinyl, phthalazinyl, quinazolinyl, quinoxalinyl, naphthyridinyl, pteridinyl, carbazolyl, acridinyl, phenazinyl,

phenothiazinyl, phenoxazinyl, and the like. Preferably, "5- to 10-membered heteroaryl" may include "5- to 6-membered heteroaryl".

The term "$C_1$-$C_{10}$ alkoxy" refers to $C_1$-$C_{10}$ alkyl linked to the remaining moiety of the molecule via an oxygen atom. $C_1$-$C_{10}$ alkoxy includes, for example, methoxy, ethoxy, n-propoxy, iso-propoxy, n-butoxy, iso-butoxy, tert-butoxy, pentyloxy, hexyloxy, and the like. Preferably, "$C_1$-$C_{10}$ alkoxy" may include "$C_1$-$C_6$ alkoxy".

The term "$C_3$-$C_{10}$ cycloalkyloxy" refers to $C_3$-$C_{10}$ cycloalkyl linked to the remaining moiety of the molecule via an oxygen atom. $C_3$-$C_{10}$ cycloalkyloxy includes, for example, cyclopropyloxy, cyclobutyloxy, cyclopentyloxy, cyclohexyloxy, and the like. Preferably, "$C_3$-$C_{10}$ cycloalkyloxy" may include "$C_3$-$C_6$ cycloalkyloxy".

The term "3- to 10-membered heterocyclyloxy" refers to 3- to 10-membered heterocyclyl linked to the remaining moiety of the molecule via an oxygen atom. 3- to 10-membered heterocyclyloxy includes, for example, oxetanyloxy, tetrahydrofuranyloxy, pyrrolidinyloxy, piperidinyloxy, and the like. Preferably, "3- to 10-membered heterocyclyloxy" may include "3-to 7-membered heterocyclyloxy".

The term "$C_6$-$C_{10}$ aryloxy" refers to $C_6$-$C_{10}$ aryl linked to the remaining moiety of the molecule via an oxygen atom. $C_6$-$C_{10}$ aryloxy includes, for example, phenyloxy and naphthyloxy.

The term "5- to 10-membered heteroaryloxy" refers to 5- to 10-membered heteroaryl linked to the remaining moiety of the molecule via an oxygen atom. 5- to 10-membered heteroaryloxy includes, for example, pyrazolyloxy, oxazolyloxy, pyridyloxy, indazolyloxy, and the like.

The present application further comprises isotopically labeled compounds of the present application that are identical to those described herein, except that one or more atoms are replaced by atom(s) having an atomic mass or mass number different from the atomic mass or mass number usually found in nature. Examples of isotopes that may be incorporated into the compounds of the present application include isotopes of hydrogen, carbon, nitrogen, oxygen, phosphorous, sulfur, fluorine, iodine and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$, respectively.

Some of the isotopically labeled compounds (e.g., compounds labeled with $^{3}H$ and $^{14}C$) of the present application may be used in the tissue distribution assay of a compound and/or a substrate. Tritiated (*i.e.* $^{3}H$) and carbon-14 (*i.e.* $^{14}C$) isotopes are particularly preferred due to their ease of preparation and detectability. Positron-emitting isotopes, such as $^{15}O$, $^{13}N$, $^{11}C$ and $^{18}F$, may be used in positron emission topography (PET) studies to determine substrate occupancy. The isotopically labeled compounds of the present application may generally be prepared by substituting an isotopically labeled reagent for a non-isotopically labeled reagent according to the procedures similar to those disclosed in the schemes and/or Examples below.

In addition, substitution with heavier isotopes (such as deuterium, *i.e.* $^{2}H$) may afford certain therapeutic advantages resulting from greater metabolic stability (e.g., prolonged *in-vivo* half-life or reduced dosage requirements) and may thus be preferred in some situations. Deuterium substitution may be partial substitution or complete substitution, and partial deuterium substitution means that at least one hydrogen is substituted with at least one deuterium.

All variations of the isotopic composition of the compounds of the present disclosure are included within the scope of the present disclosure regardless of the radioactivity.

The term "treating" means administering a compound or a preparation of the present application to prevent, ameliorate or eliminate a disease or one or more symptoms associated with the disease, and includes:

(i) preventing a disease or a disease state from occurring in a mammal, especially when such mammal is susceptible to the disease state but has not yet been diagnosed as having the disease state;
(ii) inhibiting a disease or a disease state, i.e. restraining its development; and
(iii) alleviating a disease or a disease state, i.e. causing the regression of the disease or the disease state.

The term "therapeutically effective amount" refers to an amount of the compound of the present disclosure used to (i) treat or prevent a specific disease, condition, or disorder, (ii) alleviate, ameliorate or eliminate one or more symptoms of a specific disease, condition, or disorder, or (iii) prevent or delay the onset of one or more symptoms of the specific disease, condition, or disorder described herein. The amount of the compound of the present disclosure that constitutes a "therapeutically effective amount" varies depending on the compound, the disease state and its severity, the route of administration and the age of the mammal to be treated, but it may be determined routinely by a person skilled in the art based on his/her own knowledge and the contents of the present disclosure.

The term "excipient" refers to a pharmaceutically acceptable inert ingredient. Examples of the term "excipient" include, without limitation, binders, disintegrants, lubricants, glidants, stabilizers, fillers, diluents, and the like. Excipients are capable of reinforcing the handling characteristics of the pharmaceutical formulation, *i.e.* enabling the preparation to be more suitable for direct compression by increasing flowability and/or adhesiveness. Examples of typical "pharmaceutically acceptable excipients" suitable for use in the above preparation are excipients commonly used in pharmaceutical preparations, such as saccharides, starches, cellulose and the derivatives thereof, and the like.

The term "pharmaceutically acceptable excipients" refer to those excipients that are not remarkably irritating

to the organism and do not impair the bioactivity and properties of the active compound. Suitable excipients are those well known to a person skilled in the art, for example, carbohydrates, waxes, water-soluble and/or water-swellable polymers, hydrophilic or hydrophobic materials, gelatin, oils, solvents, water, etc.

The wording "comprise" and the variants thereof, such as "comprises" or "comprising", should be understood as having an open and non-exclusive meaning, namely, "including but not limited to".

The pharmaceutical composition of the present application may be prepared by combining a compound of the present application with suitable pharmaceutically acceptable excipients, for example, the pharmaceutical composition may be formulated into a solid preparation, a semi-solid preparation, a liquid preparation, or a gaseous preparation, such as a tablet, a pill, a capsule, a powder, a granule, an ointment, an emulsion, a suspension, a suppository, an injection, an inhalant, a gel, a microsphere, an aerosol, and the like.

Typical routes of administering a compound of the present application or a pharmaceutically acceptable salt thereof or a pharmaceutical composition thereof include, but are not limited to, oral, rectal, topical, inhalation, parenteral, sublingual, intravaginal, intranasal, intraocular, intraperitoneal, intramuscular, subcutaneous, and intravenous administration.

The pharmaceutical composition of the present application may be manufactured by a method well known in the art, such as a conventional mixing method, dissolution method, granulation method, sugar-coating method, grinding method, emulsion method, lyophilizing method, and the like.

In some embodiments, the pharmaceutical composition is in an oral form. In case of oral administration, the pharmaceutical composition may be formulated by mixing the active compound with pharmaceutically acceptable excipients well known in the art. Such excipients enable the compound of the present application to be formulated into a tablet, a pill, a troche, a dragee, a capsule, a liquid, a gel, a syrup, a suspension or the like for oral administration to a patient.

Solid oral compositions may be prepared by a conventional mixing method, filling method or tableting method. The solid oral compositions may be obtained, for example, by the following method: mixing the active compound with a solid excipient, optionally grinding the resulting mixture, adding other suitable excipients if desired, and then processing this mixture into granules to obtain the cores of tablets or dragees. Suitable excipients include, but are not limited to, binders, diluents, disintegrants, lubricants, glidants, sweetening agents, flavoring agents, or the like.

The pharmaceutical compositions may also be suitable for parenteral administration, such as sterile solutions, suspensions, or lyophilized products in appropriate unit dosage forms.

In all administration methods of the compounds of formula (I) described herein, the daily dose is 0.01 to 100 mg/kg body weight, preferably 0.05 to 50 mg/kg body weight and more preferably 0.1 to 30 mg/kg body weight in the form of single dose or separate dose. The compounds of the present disclosure may be prepared by a plurality of synthetic methods well known to a person skilled in the art, including the specific embodiments listed below, embodiments resulting from their combination with other chemical synthesis methods, and equivalent alternatives well known to a person skilled in the art. Preferred embodiments include, but are not limited to, the Examples of the present disclosure.

The compounds of the present disclosure may be prepared by a plurality of synthetic methods well known to a person skilled in the art, including the specific embodiments listed below, embodiments resulting from their combination with other chemical synthesis methods, and equivalent alternatives well known to a person skilled in the art. Preferred embodiments include, but are not limited to, the Examples of the present disclosure.

The chemical reactions in the specific embodiments of the present disclosure are carried out in suitable solvents. The solvents must be suitable for the chemical changes of the present disclosure and the reagents and materials desired herein. In order to obtain the compounds of the present disclosure, it is sometimes necessary for a person skilled in the art to modify or select the synthetic steps or reaction processes on the basis of the existing embodiments.

## DETAILED DESCRIPTION

The technical solutions of the present disclosure will be illustrated in detail by way of the Examples below. However, the protection scope of the present disclosure includes but is not limited thereto.

The solvents used in the present disclosure are commercially available. The commercially available compounds are referred to as the names in the catalogs of suppliers.

The structure of a compound is determined by nuclear magnetic resonance (NMR) and/or mass spectrometry (MS). The unit of NMR shift is $10^{-6}$ (ppm). Solvents for NMR assay are deuterated dimethyl sulfoxide, deuterated chloroform, deuterated methanol, etc., and the internal standard is tetramethylsilane (TMS). "IC50" refers to a half maximal inhibitory concentration, *i.e.* the concentration at which half of the maximal inhibitory effect is achieved. The eluent described below may be a mixed eluent formed by two or more solvents, and the percentage is the volume ratio of the solvents. For example, "tetrahydrofuran/ petroleum ether = 0% to 20%" means that during the gradient elution, the volume ratio of the amount of tetrahydrofuran to the amount of petroleum ether in the mixed eluent is 0:100 to 20:100.

The following abbreviations are used in the present disclosure:

| Abbreviation | Chemical Name | Abbreviation | Chemical Name |
|---|---|---|---|
| BPin | 4,4,5,5-Tetramethyl-1,3,2-dioxaborolan-2-yl | $B_2Pin_2$ | 4,4,5,5-Tetramethyl-2-(4,4,5,5-tetramethyl-1,3,2-dioxaborolan-2-yl)-1,3,2-dioxaborolane |
| HATU | O-(7-azabenzotriazol-1-yl)-N,N ,N,N -tetramethyluronium hexafluorophosphate | EDCI | 1-(3-Dimethylaminopropyl)-3 -ethylcarbodiimide |
| DMAP | 4-Dimethylaminopyridine | 2-Me-THF | 2-Methyltetrahydrofuran |
| DCM | Dichloromethane | DMA | N,N -dimethylacetamide |
| EtOH | Ethanol | AcOH | Acetic acid |
| $Pd(dppf)Cl_2$ | 1,1-Bis(diphenylphosphino)ferrocene dichloropalladium | DMSO | Dimethyl sulfoxide |
| KOAc | Potassium acetate | TFA | Trifluoroacetic acid |
| THF | Tetrahydrofuran | MeCN | Acetonitrile |
| TEA | Triethylamine | $T_3P$ | Tri-n-propylphosphoric anhydride |
| M | Mole per liter | $MeB(OH)_2$ | Methylboronic acid |
| *m*CPBA | m-Chloroperoxybenzoic acid | $Cu(OAc)_2$ | Copper acetate |
| DMF | N,N-dimethylform amide | $Ti(OiPr)_4$ | Titanium tetraisopropoxide |
| TsOH | p-Toluenesulfonic acid | tBuONO | tert-Butyl nitrite |
| (R)-(S)-$cy_2$PF-P*t*$Bu_z$ | (R)-1-[(S)-2-(dicyclohexylphos phino) ferrocenyl]ethyl-tert-butylphosphorus trihydride | BrettPhos Pd $G_3$ | Methanesulfonato(2-dicyclohexylphosphino)-3,6-dimethoxy-2,4,6-triisopropyl-1,1-biphenyl)(2-amino-1,1-biphenyl-2-yl) palladium (II) |
| $Pd(PPh_3)_4$ | Tetrakis(triphenylphosphine) palladium | $Et_2O$ | Ethyl ether |
| Boc | tert-Butyloxycarbonyl | NBS | N-bromosuccinimide |
| DIEA | N,N -diisopropylethylamine | DME | Glycol dimethyl ether |
| $PhI(OAc)_2$ | Iodobenzene diacetate | LiHMDS | Lithium bis(trimethylsilyl)amide |
| MeOH | Methanol | THP | Tetrahydropyranyl |
| MW | Microwave | $Boc_2O$ | Di-tert-butyl dicarbonate |
| Xphos-Pd $G_2$ | Chloro(2-dicyclohexylphosphin o-2,4,6-triisopropyl-1,1-biphen yl)[2-(2-amino-1,1-biphenyl)] palladium (II) | t-BuBrettPhos-Pd $G_3$ | Methanesulfonato-2-(di-tert-butylphosphino)-3,6-dimethoxy-2,4,6-triisopropyl-1,1-biphenyl(2-amino-1,1-biphenyl-2-yl) palladium (II) |
| xantphos | 4,5-Bis(diphenylphosphino)-9, 9-dimethylxanthene | DMEDA | N,N -dimethylethylenediamine |
| $Pd_2(dba)_3$ | Tris(dibenzylideneacetone) dipalladium | NIS | N-iodosuccinimide |
| Xphos | 2-Dicyclohexylphosphino-2,4,6 -triisopropylbiphenyl | Pd(AmPhos) $Cl_2$ | Bis[di-tert-butyl-(4-dimethylaminophenyl ) phosphine]dichloropalladium (II) |
| $Pd(OAc)_2$ | Palladium acetate | n-BuOH | n-Butanol |

Intermediate 1: Synthesis of 5-amino-N-tert-butyl-2-(4,4,5,5-tetramethyl-1,3,2-dioxaborolan-2-yl)benzenesulfonamide

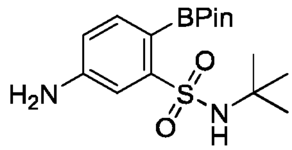

Synthetic Method

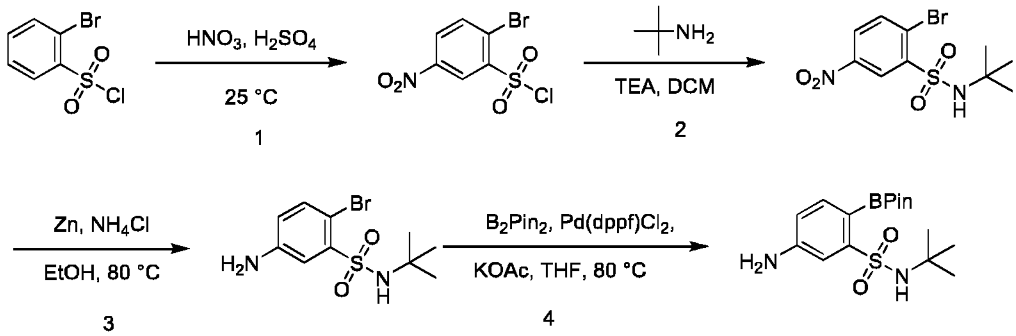


Step 1: Synthesis of 2-bromo-5-nitro-benzenesulfonyl chloride

2-Bromobenzenesulfonyl chloride (50.0 g, 195.7 mmol) was dissolved in sulfuric acid (750 mL), and a solution of nitric acid (37.0 g, 587.1 mmol, 26.5 mL) in sulfuric acid (750 mL) was slowly added. The whole dropwise addition process took 30 minutes. The temperature of the system was kept at 0°C in an ice-salt bath environment. After the dropwise addition, the clear reaction solution was stirred and reacted at 10°C for 2 hours. After the completion of the reaction was detected, the reaction solution was slowly added into ice water (2.5L) while stirring. A white solid precipitated with stirring. The white solid was filtered, washed with 1.5L (0.5L × 3) of water, and then dried to obtain 2-bromo-5-nitro-benzenesulfonyl chloride (48.0 g).

Step 2: Synthesis of 2-bromo-N-tert-butyl-5-nitrobenzenesulfonamide

Tert-butylamine (5.9 g, 79.9 mmol) was dissolved in dichloromethane (0.2L), and 2-bromo-5-nitro-benzenesulfonyl chloride (8.0 g, 26.6 mmol) was added in batches at 0°C. Triethylamine (5.4 g, 53.3 mmol) was added into the reaction solution. The light yellow reaction solution was stirred and reacted at 20°C for 1 hour. The completion of the reaction was detected by LCMS. The reaction solution was added with dilute hydrochloric acid (1M, 200 mL) and subjected to the liquid-liquid separation. Then, a saturated sodium carbonate solution (200 mL) was added into the organic phase. The organic phase was dried over anhydrous sodium sulfate and filtered. Thereafter, the organic phase was concentrated to dryness under reduced pressure. Methanol (50 mL) was added, a white solid precipitated with stirring, and the solid was slurried to obtain 2-bromo-N-tert-butyl-5- nitrobenzenesulfonamide (8.0 g).

$^1$HNMR (400 MHz, DMSO-d$_6$) δ 8.69 (d, J = 2.75 Hz, 1H), 8.29 (dd, J = 8.63, 2.75 Hz, 1H), 8.18-8.04 (m, 2H), 1.22-1.07 (m, 9H).

Step 3: Synthesis of 5-amino-2-bromo-N-tert-butylbenzenesulfonamide

2-Bromo-N-tert-butyl-5-nitrobenzenesulfonamide (5.0 g, 14.8 mmol) was dissolved in ethanol (0.2L), and zinc powder (4.9 g, 74.1 mmol) and ammonium chloride (4.0 g, 74.1 mmol) were added. The reaction solution was stirred and reacted at 40°C for 1 hour. The reaction solution was subjected to suction filtration to remove zinc powder. The mother liquor was concentrated to dryness under reduced pressure, water (200 mL) was added thereto, and the resulting mixture was extracted with 600 mL (300 mL × 2) of dichloromethane. The organic phase was dried over anhydrous sodium sulfate and then evaporated to dryness via rotary evaporation under reduced pressure to obtain a crude product of 5-amino-2-bromo-N-tert-butylbenzenesulfonamide (4.9 g).

MS m/z (ESI): 307.1, 309.1 [M+H]$^+$;

Step 4: Synthesis of 5-amino-N-tert-butyl-2-(4,4,5,5-tetramethyl-1,3,2-dioxaborolan-2-yl)benzenesulfonamide

5-Amino-2-bromo-N-tert-butylbenzenesulfonamide (1.0 g, 3.3 mmol) was dissolved in tetrahydrofuran (100 mL), and 4,4,5,5-tetramethyl-2-(4,4,5,5-tetramethyl-1,3,2-dioxaborolan-2-yl)-1,3,2-dioxaborolane (3.3 g, 13.0 mmol) and potassium acetate (958.4 mg, 9.8 mmol) were added. Under the protection of nitrogen, a mixture of 1,1-bis(diphenylphosphino)ferrocenedichloropalladium and dichloromethane (132.9 mg, 162.8 μmol) was added into the reaction solution. Under the protection of nitrogen, the dark red reaction solution was stirred and reacted at 100°C for 2 hours. The reaction solution was subjected to suction filtration. The filtrate was concentrated to dryness under reduced pressure and purified by column chromatography (silica, tetrahydrofuran/petroleum ether = 0% to 20%) to obtain 5-amino-N-tert-butyl-2-(4,4,5,5-tetramethyl-1,3,2-dioxaborolan-2-yl)benzenesulfonamide (0.6 g).
MS m/z (ESI): 355.2 $[M+H]^+$.

Intermediate 2: Synthesis of isopropyl trans-N-[4-[5-[4-amino-2-(tert-butylsulfamoyl)phenyl]thiazol-2-yl]cyclohexyl]carbamate

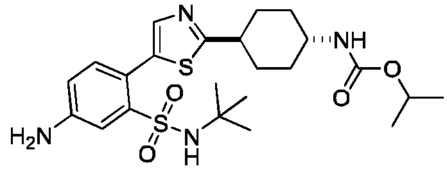

Synthetic Method

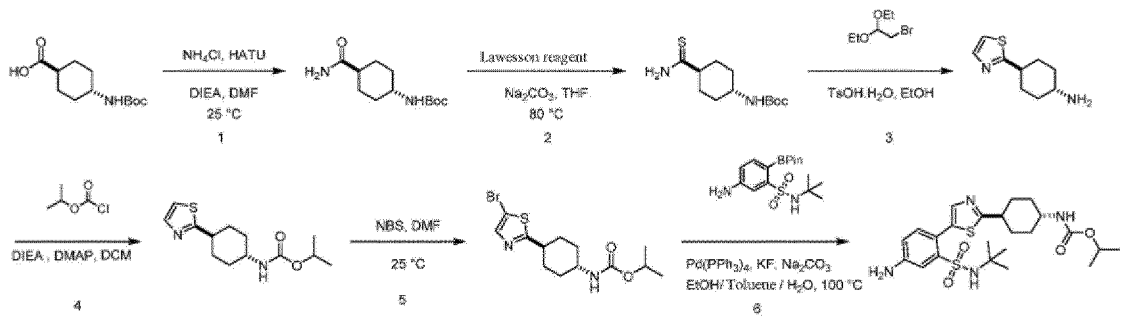


Step 1: Synthesis of tert-butyl trans-N-(4-carbamoylcyclohexyl)carbamate

Trans-4-(tert-butoxycarbonylamino)cyclohexanecarboxylic acid (20.0 g, 82.2 mmol) was dissolved in N,N-dimethylformamide (1.0L), and O-(7-azabenzotriazol-1-yl)-N,N,N,N-tetramethyluronium hexafluorophosphate (117.2 g, 308.3 mmol), ammonium chloride (66.0 g, 1.2 mol) and N,N-diisopropylethylamine (31.4 mg, 242.6 μmol, 42.3 μL) were added. The reaction solution was stirred and reacted at 25°C for 1 hour. The reaction solution was concentrated to dryness under reduced pressure. Methanol (0.2L) and water (1.0L) were added, and a white solid precipitated, followed by stirring at room temperature for 30 minutes. The resultant was filtered and then washed with 0.9L (0.3L × 3) of water to obtain tert-butyl trans-N-(4-carbamoylcyclohexyl)carbamate (13.0 g).
MS m/z (ESI): 187.1 $[M-55]^+$;

Step 2: Synthesis of tert-butyl trans-N-(4-carbamothioylcyclohexyl)carbamate

Tert-butyl trans-N-(4-carbamoylcyclohexyl)carbamate (12.0 g, 49.5 mmol) was dissolved in tetrahydrofuran (0.5L), and Lawesson reagent (20.0 g, 49.5 mmol) was added. The reaction solution was stirred and reacted at 80°C for 30 minutes. The reaction solution was added with a saturated aqueous sodium carbonate solution (0.8L) so that the pH of the mixture was adjusted to 8~9, and the resulting mixture was extracted with ethyl acetate (1.6L) three times. The organic layer was dried over anhydrous magnesium sulfate and subjected to suction filtration. The filtrate was

concentrated to dryness under reduced pressure and purified by column chromatography (silica, tetrahydrofuran/petroleum ether = 0% to 50%) to obtain tert-butyl trans-N-(4-carbamothioylcyclohexyl)carbamate (6.0 g).
MS m/z (ESI): 203.1 $[M-55]^+$;

Step 3: Synthesis of trans-4-thiazol-2-ylcyclohexylamine

Tert-butyl trans-N-(4-carbamothioylcyclohexyl)carbamate (2.0 g, 7.7 mmol) and bromoacetaldehyde diethyl acetal (3.1 g, 15.5 mmol, 2.4 mL) were dissolved in anhydrous ethanol (80 mL), and p-toluenesulfonic acid (2.7 g, 15.5 mmol) was added. The clear reaction solution was stirred and reacted at 80°C for 1 hour. The reaction solution was added with a saturated aqueous sodium carbonate solution (100 mL) so that the pH of the mixture was adjusted to 8~9, and the resulting mixture was extracted with dichloromethane (300 mL) three times. The organic layer was dried over anhydrous magnesium sulfate and subjected to suction filtration. The filtrate was concentrated to dryness under reduced pressure to obtain a crude product of trans-4-thiazol-2-ylcyclohexylamine (1.5 g).
MS m/z (ESI): 183.1 $[M+H]^+$;

Step 4: Synthesis of isopropyl trans-N-(4-thiazol-2-ylcyclohexyl)carbamate

Trans-4-thiazol-2-ylcyclohexylamine (1.4 g, 7.7 mmol) and isopropyl chloroformate (2.9 g, 23.3 mmol, 3.3 mL) were dissolved in dichloromethane (200 mL), and N,N-diisopropylethylamine (2.0 g, 15.5 mmol, 2.7 mL) and 4-dimethylaminopyridine (472.8 mg, 3.9 mmol) were added. The reaction solution was stirred and reacted at 20°C for 30 minutes. The reaction solution was concentrated to dryness under reduced pressure and purified by column chromatography (silica, ethyl acetate/petroleum ether = 0% to 15%) to obtain isopropyl trans-N-(4-thiazol-2-ylcyclohexyl)carbamate (0.3 g).
MS m/z (ESI): 269.1 $[M+H]^+$;

Step 5: Synthesis of isopropyl trans-N-[4-(5-bromothiazol-2- yl)cyclohexyl]carbamate

Isopropyl trans-N-(4-thiazol-2-ylcyclohexyl)carbamate (0.3 g, 1.1 mmol) was dissolved in N,N-dimethylformamide (1.5 mL), and N-bromosuccinimide (0.4 g, 2.3 mmol) was added. The reaction solution was stirred and reacted at 50°C for 2 hours. The reaction solution was concentrated to dryness under reduced pressure and purified by column chromatography (silica, tetrahydrofuran/petroleum ether = 0% to 10%) to obtain isopropyl trans-N-[4-(5-bromothiazol-2-yl)cyclohexyl]carbamate (0.1 g).
MS m/z (ESI): 347.0, 349.0 $[M+H]^+$;

Step 6: Synthesis of isopropyl trans-N-[4-[5-[4-amino-2-(tert-butylsulfamoyl)phenyl] thiazol-2-yl]cyclohexyl]carbamate

5-Amino-N-tert-butyl-2-(4,4,5,5-tetramethyl-1,3,2-dioxaborolan-2-yl)benzenesulfonamide (61.0 mg, 172.8 μmol) and isopropyl trans-N-[4-(5-bromothiazol-2-yl)cyclohexyl]carbamate (0.1 g, 288.0 μmol) were dissolved in a mixture of ethanol (2.0 mL), toluene (2.0 mL) and water (0.7 mL), and sodium carbonate (30.5 mg, 288.0 μmol), potassium fluoride (16.7 mg, 288.0 μmol) and tetrakis(triphenylphosphine)palladium (16.6 mg, 14.4 μmol) were added. Under the protection of nitrogen, the reaction solution was stirred and reacted at 100°C for 2 hours. The reaction solution was concentrated to dryness under reduced pressure. The reaction solution was added with water (2 mL) and extracted with dichloromethane (6 mL) three times. The organic layer was dried over anhydrous magnesium sulfate and subjected to suction filtration. The filtrate was concentrated to dryness under reduced pressure to obtain a crude product of isopropyl trans-N-[4-[5-[4-amino-2-(tert-butylsulfamoyl)phenyl]thiazol-2-yl]cyclohexyl]carbamate (60.0 mg).
MS m/z (ESI): 495.3 $[M+H]^+$.

Intermediate 3: Synthesis of isopropyl trans-N-[4-[2-[4-(isopropoxycarbonylamino) cyclohexyl]thiazol-5-yl]-3-(methylsulfonimidoyl)phenyl]carbamate

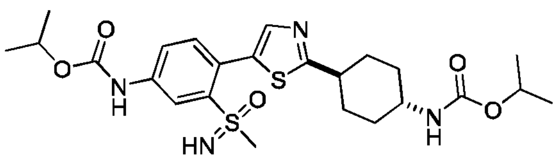

Synthetic Method

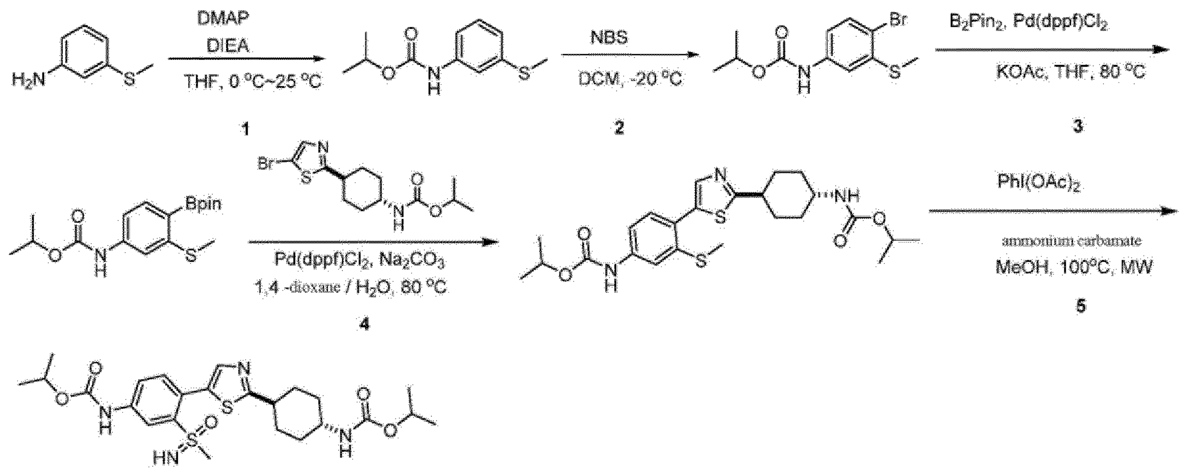


Step 1: Synthesis of isopropyl N-(3-methylsulfanylphenyl)carbamate

3-Methylthioaniline (806 mg, 5.79 mmol) as a reactant was dissolved in anhydrous tetrahydrofuran (20 mL), and N,N-diisopropylethylamine (1.50 g, 11.58 mmol, 2.02 mL) and 4-dimethylaminopyridine (353.65 mg, 2.89 mmol) were added at 0°C. Thereafter, isopropyl chloroformate (2.13 g, 17.37 mmol, 2.41 mL) was added into the reaction system at 0°C. The reaction solution was stirred and reacted at 25°C for 3 hours. The completion of the reaction was detected by LCMS. The reaction solution was concentrated under reduced pressure to remove the solvent tetrahydrofuran, added with 20 mL of dichloromethane, and extracted three times with a saturated aqueous sodium chloride solution (20mL × 3). The organic phase was separated, dried over anhydrous sodium sulfate, concentrated to dryness under reduced pressure, and purified by thin-layer chromatography (silica, petroleum ether:ethyl acetate = 10:1) to obtain isopropyl N-(3-methylsulfanylphenyl)carbamate (1.16 g) as a yellow liquid.
MS m/z (ESI): 226.1 $[M+H]^+$.

Step 2: Synthesis of isopropyl N-(4-bromo-3-methylsulfanyl-phenyl)carbamate

Isopropyl N-(3-methylsulfanylphenyl)carbamate (50 mg, 221.92 μmol) as a reactant was dissolved in dichloromethane (2 mL), and N-bromosuccinimide (39.50 mg, 221.92 μmol) was added into the reaction system. The reaction solution was stirred at -70°C for 3 hours. The completion of the reaction was detected by LCMS. The reaction solution was concentrated to dryness under reduced pressure and purified by thin-layer chromatography (silica, petroleum ether:ethyl acetate = 10:1) to obtain isopropyl N-(4-bromo-3-methylsulfanyl-phenyl)carbamate as a white solid (50 mg).
MS m/z (ESI): 262.0 $[M+H-iPr]^+$;
$^1H$ NMR(400 MHz, DMSO-$d_6$) δ ppm 9.75 (s, 1 H) 7.50-7.45 (m, 2 H) 7.20 (dd, J =8.57, 1.94 Hz, 1 H) 4.97-4.87 (m, 1 H) 2.44 (s, 3 H) 1.27 (d, J =6.25 Hz, 6 H).

Step 3: Synthesis of isopropyl N-[3-methylsulfanyl-4-(4,4,5,5-tetramethyl-1,3,2-dioxaborolan-2-yl)phenyl]carbamate

Isopropyl N-(4-bromo-3-methylsulfanyl-phenyl)carbamate (153 mg, 0.51 mmol) and bis(pinacolato)diboron (510.88 mg, 2.01 mmol) as reactants were dissolved in anhydrous tetrahydrofuran (3 mL). Under the protection of nitrogen, potassium acetate (148.08 mg, 1.51 mmol) and [1,1-bis(diphenylphosphino)ferrocene]dichloropalladium - dichloromethane complex (20.54 mg, 25.15 μmol) were added. The reaction solution was stirred at 80°C for 2 hours. LCMS showed that the reaction was complete. The reaction system was evaporated to dryness via rotary evaporation, and then the resultant was purified by column chromatography (silica, petroleum ether:ethyl acetate = 10:1) to obtain isopropyl N-[3-methylsulfanyl-4-(4,4,5,5-tetramethyl-1,3,2-dioxaborolan-2- yl)phenyl]carbamate (141 mg) as a yellow solid.
MS m/z (ESI): 352.2 $[M+H]^+$.

Step 4: Synthesis of isopropyl trans-N-[4-[2-[4-(isopropoxycarbonylamino) cyclohexyl]thiazol-5-yl]-3-methylsulfanyl-phenyl]carbamate

Isopropyl trans-N-[4-(5-bromothiazol-2-yl)cyclohexyl]carbamate (100 mg, 287.96 μmol), isopropyl N-[3-methylsulfanyl-4-(4,4,5,5-tetramethyl-1,3,2-dioxaborolan-2-yl)phenyl]carbamate (121.38 mg, 345.55 μmol) and sodium carbonate (61.04 mg, 575.92 μmol) were dissolved in water (600 μL) and 1,4-dioxane (6 mL). Under the protection of nitrogen, [1,1-bis(diphenylphosphino)ferrocene]dichloropalladium - dichloromethane (23.52 mg, 28.80 μmol) was added. The reaction solution was stirred at 80°C for 6 hours. LCMS showed that the reaction was complete. The reaction solution was filtered. The filtrate was evaporated to dryness via rotary evaporation to remove 1,4-dioxane. The resultant was added with water (10 mL), and the resulting mixture was extracted three times with dichloromethane (10 mL × 3). After dried over anhydrous sodium sulfate, the resultant was purified by thin-layer chromatography (silica, dichloromethane/methanol = 15:1) to obtain isopropyl trans-N-[4-[2-[4-(isopropoxycarbonylamino)cyclohexyl]thiazol-5-yl]-3-methylsulfanyl-phenyl]carbamate (133 mg) as a brown solid.

MS m/z (ESI): 492.3 $[M+H]^+$.

Step 5: Synthesis of isopropyl trans-N-[4-[2-[4-(isopropoxycarbonylamino)cyclohexyl] thiazol-5-yl]-3-(methylsulfonimidoyl)phenyl]carbamate

Isopropyl trans-N-[4-[2-[4-(isopropoxycarbonylamino)cyclohexyl]thiazol-5-yl]-3-methylsulfanyl-phenyl]carbamate (140 mg, 284.75 μmol), ammonium carbamate (444.60 mg, 5.69 mmol) and iodobenzene diacetate (917.17 mg, 2.85 mmol) were added into a 4-mL microwave reaction flask, and anhydrous methanol (1 mL) was added. The reaction was carried out under microwave irradiation at 100°C for 3 hours. LCMS showed that the reaction was complete. The reaction solution was concentrated under reduced pressure to remove methanol, added with water (4 mL), extracted with dichloromethane (4 mL × 3), and subjected to the liquid-liquid separation. The organic phase was dried over anhydrous sodium sulfate, evaporated to dryness via rotary evaporation under reduced pressure, and purified by thin-layer chromatography (silica, dichloromethane/methanol = 15:1) to obtain isopropyl trans-N-[4-[2-[4-(isopropoxycarbonylamino)cyclohexyl]thiazol-5-yl]-3-(methylsulfonimidoyl) phenyl]carbamate (47.4 mg) as a yellow solid compound.

MS m/z (ESI): 523.2 $[M+H]^+$.

Example 1: Synthesis of isopropyl trans-N-[3-dimethylphosphoryl-4-[2-[4-(isopropoxycarbonylamino)cyclohexyl]thiazol-5-yl]phenyl]carbamate

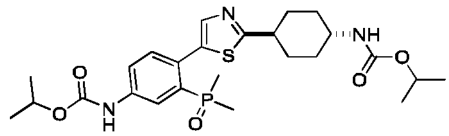

Synthetic Method

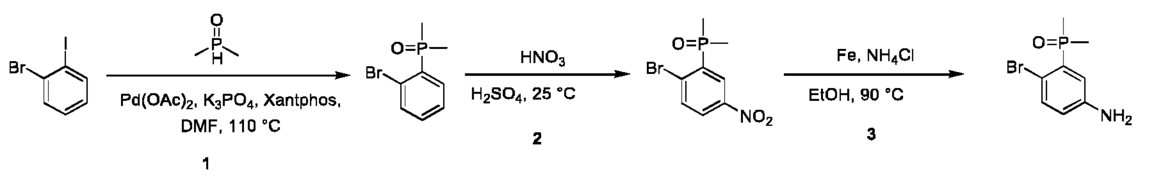


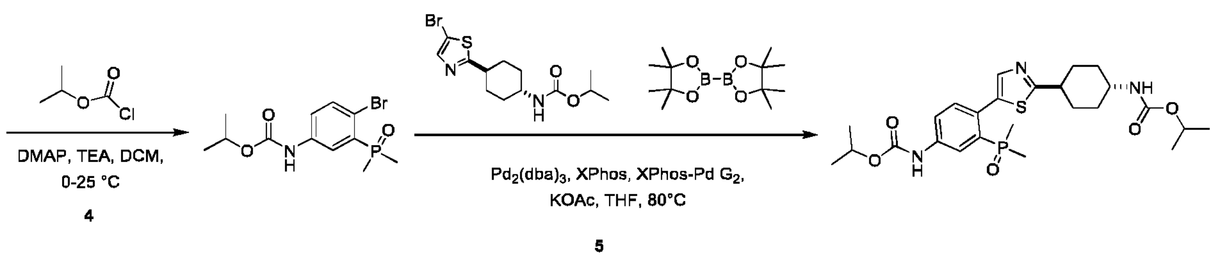

Step 1: Synthesis of (2-bromophenyl)dimethylphosphine oxide

1-Bromo-2-iodobenzene (5.0 g, 17.6 mmol), potassium phosphate (5.6 g, 26.5 mmol) and 4,5-bis(diphenylphosphino)-9,9-dimethylxanthene (613.6 mg, 1.06 mmol) were dissolved in N,N-dimethylformamide (50 mL), and dimethylphosphine oxide (1.7 g, 21.2 mmol) and palladium acetate (198.4 mg, 0.88 mmol) were added. The reaction solution was stirred and reacted at 110°C for 16 hours. The reaction solution was filtered. The filtrate was collected and concentrated to dryness under reduced pressure, and then purified by column chromatography (silica, dichloromethane/methanol = 20/1) to obtain (2-bromophenyl)dimethylphosphine oxide as the product (2.3 g).
MS m/z (ESI): 233.0, 235.0 $[M+H]^+$;

Step 2: Synthesis of (2-bromo-5-nitrophenyl)dimethylphosphine oxide

(2-Bromophenyl)dimethylphosphine oxide (1.0 g, 4.3 mmol) was dissolved in concentrated sulfuric acid (15 mL), and concentrated nitric acid (876.6 mg, 13.2 mmol, 626.1 μL) was added. The reaction solution was stirred and reacted at 25°C for 1 hour. The reaction solution was dissolved in water (200 mL) and extracted with ethyl acetate (100 mL) three times. The organic phase was concentrated to dryness under reduced pressure to obtain (2-bromo-5-nitrophenyl)dimethylphosphine oxide as the product (1.0 g).
MS m/z (ESI): 277.9, 279.9 $[M+H]^+$;

Step 3: Synthesis of (5-amino-2-bromophenyl)dimethylphosphine oxide

(2-Bromo-5-nitrophenyl)dimethylphosphine oxide (0.9 g, a crude product) was dissolved in ethanol (10 mL), and iron powder (903.8 mg, 16.2 mmol) and ammonium chloride (865.7 mg, 16.2 μmol) were added. The reaction solution was stirred and reacted at 90°C for 1 hour. The reaction solution was filtered. The filtrate was concentrated to dryness under reduced pressure, and then purified by column chromatography (silica, dichloromethane/methanol = 20/1) to obtain (5-amino-2-bromophenyl)dimethylphosphine oxide as the product (0.15 g).
MS m/z (ESI): 248.0, 250.0 $[M+H]^+$;

Step 4: Synthesis of isopropyl (4-bromo-3-(dimethylphosphoryl)phenyl)carbamate

(5-Amino-2-bromophenyl)dimethylphosphine oxide (130 mg, 524.1 μmol) was dissolved in dichloromethane (10 mL), and isopropyl chloroformate (192.7 mg, 1.6 mmol, 218.2 μL) was added. Thereafter, 4-dimethylaminopyridine (32.1 mg, 262.1 μmol) and triethylamine (106.1 mg, 1.05 mmol, 145.9 μL) were added at 0°C. The reaction solution was stirred and reacted at 25°C for 2 hours. The reaction solution was filtered. The filtrate was concentrated to dryness under reduced pressure, and then purified by column chromatography (silica, dichloromethane/methanol = 20/1) to obtain isopropyl (4-bromo-3-(dimethylphosphoryl) phenyl)carbamate as the product (0.12 g).
MS m/z (ESI): 334.1, 336.1 $[M+H]^+$;

Step 5: Synthesis of isopropyl trans-N-[3-dimethylphosphoryl-4-[2-[4-(isopropoxycarbonylamino)cyclohexyl]thiazol-5-yl]phenyl]carbamate

Isopropyl (4-bromo-3-(dimethylphosphoryl)phenyl)carbamate (60 mg, 179.56 μmol), isopropyl trans-N-[4-(5-bromothiazol-2-yl)cyclohexyl]carbamate (62.36 mg, 179.56 μmol) and 4,4,5,5-tetramethyl-2-(4,4,5,5-tetramethyl-1,3,2-dioxaborolan-2-yl)-1,3,2-dioxaborolane (182.39 mg, 718.25 μmol) were dissolved in tetrahydrofuran (6 mL). Under the protection of nitrogen, 2-dicyclohexylphosphino-2,4,6-triisopropylbiphenyl (4.5 mg, 9.0 μmol), chloro(2-dicyclohexylphosphino-2,4,6-triisopropyl-1,1-biphenyl)[2-(2-amino-1,1-biphenyl)] palladium (II) (7.1 mg, 9.0 μmol), tris(dibenzylideneacetone)dipalladium (8.2 mg, 9.0 μmol) and potassium acetate (35.3 mg, 359.1 μmol) were added. The reaction solution was stirred and reacted at 80°C for 16 hours. The reaction solution was filtered. The filtrate was concentrated to dryness under reduced pressure, and purified by preparative liquid chromatography (PhenomenexGemini-NX column: 5 μm silica, 30 mm in diameter, 150 mm in length; using decreasingly polar mixtures of water (containing 0.05% aqueous ammonia) and acetonitrile as eluents) to obtain the compound isopropyl trans-N-[3-dimethylphosphoryl-4-[2-[4-(isopropoxycarbonylamino)cyclohexyl]thiazol-5-yl]phenyl]carbamate (10.4 mg).
MS m/z (ESI): 522.3 $[M+H]^+$;
$^1H$ NMR (400 MHz, DMSO-$d_6$) δ ppm 9.88 (s, 1H), 8.03 (dd, J = 14.26, 2.13 Hz, 1H), 7.82 (s, 1H), 7.69 (d, J = 8.50 Hz, 1H), 7.44-7.30 (m, 1H), 7.00 (d, J = 7.38 Hz, 1H), 4.92 (dt, J = 12.51, 6.25 Hz, 1H), 4.74 (dt, J = 12.41, 6.11 Hz, 1H), 3.40-3.25 (m, 1H), 2.99-2.85 (m, 1H), 2.12 (d, J = 12.13 Hz, 2H), 1.91 (d, J = 10.26 Hz, 2H), 1.63-1.49 (m, 2H), 1.44 (d, J = 13.26 Hz, 6H), 1.40-1.29 (m, 2H), 1.27 (d, J = 6.25 Hz, 6H), 1.16 (d, J = 6.25 Hz, 6H).

Example 2: Synthesis of isopropyl trans-N-[4-[2-[4-(isopropoxycarbonylamino) cyclohexyl]thiazol-5-yl]-3-isopropylsulfonyl-phenyl]carbamate

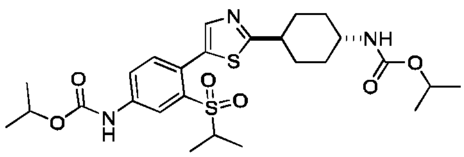

Synthetic Method

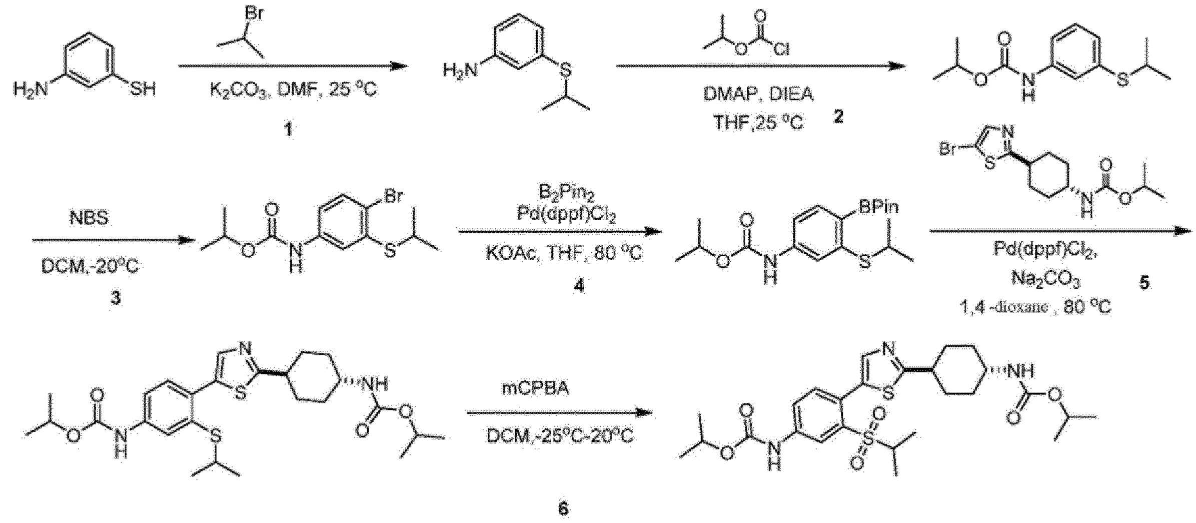


Step 1: Preparation of 3-isopropylsulfanylaniline

3-Aminothiophenol (1 g, 7.99 mmol) as a reactant was dissolved in N,N-dimethylformamide (10 mL), and isopropyl bromide (1.18 g, 9.59 mmol) and potassium carbonate (1.66 g, 11.98 mmol) were added. The reaction solution was stirred and reacted at 25°C for 3 hours. After the reaction was complete, the solvent N,N-dimethylformamide was evaporated to dryness by using a rotary evaporator, and then 5 mL of dichloromethane was added into the mixture to dissolve the mixture. The resulting mixture was added with 5 mL of water and extracted twice with dichloromethane (5 mL × 2) to separate the organic layer. The organic phase was dried over anhydrous sodium sulfate. The organic layer was concentrated to dryness under reduced pressure, and purified by column chromatography (silica, dichloromethane/methanol = 10:1) to obtain 3-isopropylsulfanylaniline as the product (1.4 g).
MS m/z (ESI): 168.0 $[M+H]^+$

Step 2: Synthesis of isopropyl N-(3-isopropylsulfanylphenyl)carbamate

3-Isopropylsulfanylaniline (0.1 g, 597.83 μmol) as a reactant was dissolved in anhydrous tetrahydrofuran (1 mL), and N,N-diisopropylethylamine (154.53 mg, 1.20 mmol) and 4-dimethylaminopyridine (36.52 mg, 298.92 μmol) were added. Thereafter, isopropyl chloroformate (87.92 mg, 717.40 μmol) was added into the reaction system. The reaction solution was stirred and reacted at 25°C for 3 hours. After the reaction was complete, the reaction solution was concentrated under reduced pressure to remove the solvent dichloromethane, added with 1 mL of water, and extracted three times with dichloromethane (1 mL × 3) to separate the organic phase. The organic phase was dried over anhydrous sodium sulfate and concentrated to dryness under reduced pressure. The resultant was purified by thin-layer chromatography (silica, petroleum ether:ethyl acetate = 3:1) to obtain isopropyl N-(3-isopropylsulfanylphenyl)carbamate (0.08 g).
MS m/z (ESI): 254.1 $[M+H]^+$

Step 3: Synthesis of isopropyl N-(4-bromo-3-isopropylsulfanyl-phenyl)carbamate

Isopropyl N-(3-isopropylsulfanylphenyl)carbamate (1.22 g, 4.82 mmol) as a reactant was dissolved in dichloromethane (15 mL). N-bromosuccinimide (857.03 mg, 4.82 mmol) was added into the reaction system. The reaction solution was stirred at -20°C for 3 hours. After the reaction was complete, the reaction solution was concentrated to dryness under reduced pressure and purified by column chromatography to obtain isopropyl N-(4-bromo-3-isopropylsulfanyl-phenyl)carbamate as the product (500 mg).
MS m/z (ESI): 332.0, 334.0 $[M+H]^+$

Step 4: Synthesis of isopropyl N-[3-isopropylsulfanyl-4-(4,4,5,5-tetramethyl-1,3,2-dioxaborolan-2-yl)phenyl]carbamate

Isopropyl N-(4-bromo-3-isopropylsulfanyl-phenyl)carbamate (450 mg, 1.35 mmol) and bis(pinacolato)diboron (1.38 g, 5.42 mmol) as reactants were dissolved in anhydrous tetrahydrofuran (5 mL). Under the protection of nitrogen, potassium acetate (398.77 mg, 4.06 mmol) and [1,1-bis(diphenylphosphino)ferrocene]dichloropalladium - dichloromethane (55.30 mg, 67.72 μmol) were added. The reaction solution was stirred at 80°C for 2 hours. After the reaction was complete, the system was evaporated to dryness via rotary evaporation and the resultant was purified by column chromatography (silica, petroleum ether:ethyl acetate = 5% to 15%) to obtain isopropyl N-[3-isopropylsulfanyl-4-(4,4,5,5-tetramethyl-1,3,2-dioxaborolan-2-yl)phenyl]carbamate (350 mg).
MS m/z (ESI): 380.1 $[M+H]^+$

Step 5: Synthesis of isopropyl trans-N-[4-[2-[4-(isopropoxycarbonylamino) cyclohexyl]thiazol-5-yl]-3-isopropylsulfanyl-phenyl]carbamate

Isopropyl N-[3-isopropylsulfanyl-4-(4,4,5,5-tetramethyl-1,3,2-dioxaborolan-2-yl) phenyl]carbamate (50 mg, 131.81 μmol) and isopropyl N-[4-(5-bromothiazol-2-yl)cyclohexyl]carbamate (50.35 mg, 145.00 μmol) as reactants were dissolved in 1,4-dioxane (1 mL). Under the protection of nitrogen, [1,1-bis(diphenylphosphino)ferrocene]dichloropalladium - dichloromethane (5.38 mg, 6.59 μmol) and sodium carbonate (2M, 131.81 μL) were added. The reaction solution was stirred at 80°C for 2 hours. The reaction system was evaporated to dryness via rotary evaporation to remove 1,4-dioxane, added with water (2 mL), and extracted three times with dichloromethane (2 mL × 3). The resultant was dried over anhydrous sodium sulfate, and then purified by thin-layer chromatography (silica, petroleum ether/tetrahydrofuran = 3:1) to obtain isopropyl trans-N-[4-[2-[4-(isopropoxycarbonylamino)cyclohexyl]thiazol-5-yl]-3-isopropylsulfanyl-phenyl]carbamate (33 mg).
MS m/z (ESI): 520.1 $[M+H]^+$

Step 6: Synthesis of isopropyl trans-N-[4-[2-[4-(isopropoxycarbonylamino)cyclohexyl]thiazol-5-yl]-3-isopropylsulfonyl-phen yl]carbamate

Isopropyl trans-N-[4-[2-[4-(isopropoxycarbonylamino)cyclohexyl]thiazol-5-yl]-3-isopropylsulfanyl-phenyl]carbamate (0.02 g, 38.48 μmol) was dissolved in anhydrous dichloromethane (1 mL), and m-chloroperoxybenzoic acid (15.63 mg, 76.96 μmol) was added at -25°C. The reaction solution was reacted at 20°C for 1 hour. The reaction solution was subjected to rotary evaporation to remove dioxane, added with water (2 mL), extracted three times with dichloromethane (2 mL × 3), and subjected to liquid-liquid separation to separate dichloromethane phase. The organic phase was dried over anhydrous sodium sulfate and concentrated to dryness under reduced pressure. 0.5 mL of methanol was added as a solvent to dissolve the resultant, and the resulting mixture was then purified. The resultant was purified by preparative high-performance liquid chromatography (YMCTriartC18 column, 7 μm silica, 50 mm in diameter, 250 mm in length) (using decreasingly polar mixtures of water (containing 0.05% aqueous ammonia) and acetonitrile (38% to 78%) as eluents) to obtain the compound isopropyl trans-N-[4-[2-[4-(isopropoxycarbonylamino)cyclohexyl]thiazol-5-yl]-3-isopropylsulfonyl-phenyl]carbamate (5.20 mg).
MS m/z (ESI): 552.2 $[M+H]^+$
$^1H$ NMR: (400 MHz, DMSO-$d_6$) δ ppm 10.13 (s, 1H), 8.32 (d, J = 2.25 Hz, 1H), 7.76 (dd, J = 8.44, 2.19 Hz, 1H), 7.69 (s, 1H), 7.49 (d, J = 8.38 Hz, 1H), 7.00 (br d, J = 7.75 Hz, 1H), 4.93 (quin, J = 6.22 Hz, 1H), 4.74 (dt, J = 12.44, 6.28Hz, 1H), 3.27 (br d, J = 3.75Hz, 1H), 3.01-2.78 (m, 2H), 2.12 (d, J = 12.01 Hz, 2H), 1.91 (d, J = 10.51 Hz, 2H), 1.64-1.46 (m, 2H), 1.41-1.30 (m, 2H), 1.27 (d, J = 6.25 Hz, 6H), 1.16 (d, J = 6.25 Hz, 6H), 1.05 (d, J = 6.75 Hz, 6H).

Example 3: Synthesis of isopropyl trans-N-[4-[2-[4-(isopropoxycarbonylamino) cyclohexyl]thiazol-5-yl]-3-(isopropylsulfonimidoyl)phenyl]carbamate

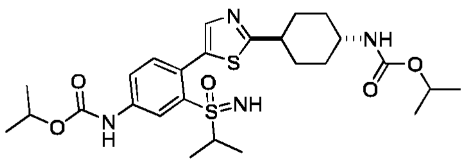

Synthetic Method

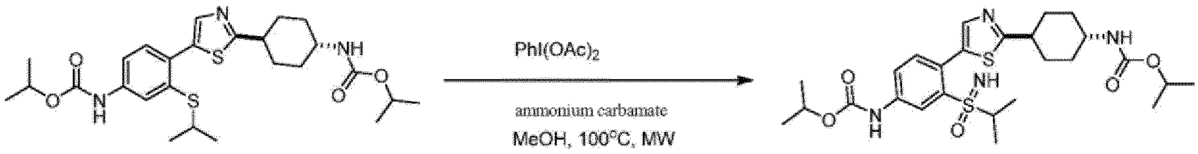


Isopropyl trans-N-[4-[2-[4-(isopropoxycarbonylamino)cyclohexyl]thiazol-5-yl]-3-isopropylsulfanyl-phenyl]carbamate (30 mg, 57.72 μmol), ammonium carbamate (90.13 mg, 1.15 mmol) and iodobenzene diacetate (185.93 mg, 577.24 μmol) were added into a 4-mL microwave reaction flask, and 1 mL of anhydrous methanol was added. The reaction was carried out under microwave irradiation at 100°C for 2 hours. The reaction solution was concentrated under reduced pressure to remove methanol, added with water (2 mL), extracted with dichloromethane (2 mL × 3), and subjected to liquid-liquid separation. The organic phase was dried over anhydrous sodium sulfate and evaporated to dryness via rotary evaporation under reduced pressure. The resultant was purified by preparative high-performance liquid chromatography (PhenomenexGemini-NXC18 column, 3 μm silica, 30 mm in diameter, 75 mm in length) (using decreasingly polar mixtures of water (containing 0.05% aqueous ammonia) and acetonitrile (30% to 70%) as eluents) to obtain isopropyl trans-N-[4-[2-[4-(isopropoxycarbonylamino)cyclohexyl]thiazol-5-yl]-3-(isopropylsulfonimidoyl)phenyl] carbamate (6.18 mg).

MS m/z (ESI): 551.3 $[M+H]^+$

$^1H$ NMR : (400 MHz, DMSO-$d_6$) δ ppm 10.04 (s, 1H), 8.34 (d, J = 2.13 Hz, 1H), 7.75 - 7.65 (m, 2H), 7.39 (d, J = 8.38 Hz, 1H), 7.00 (br d, J = 7.50 Hz, 1H), 4.93 (dt, J = 12.48, 6.33 Hz, 1H), 4.81-4.70 (m, 1H), 4.35 (s, 1H), 3.26 (br s, 1H), 2.96-2.88 (m, 1H), 2.87-2.79 (m, 1H), 2.12 (br d, J = 13.01 Hz, 2H), 1.92 (br d, J = 10.51 Hz, 2H), 1.62-1.49 (m, 2H), 1.40-1.31 (m, 2H), 1.28 (d, J = 6.25 Hz, 6H), 1.17 (d, J = 6.25 Hz, 6H), 1.08 (d, J = 6.75 Hz, 3H), 0.99 (d, J = 6.75 Hz, 3H).

Example 4: Synthesis of isopropyl trans-N-[4-[2-[4-(isopropoxycarbonylamino) cyclohexyl]thiazol-5-yl]-3-(isopropyl-N-prop-2-enoyl-sulfonimidoyl)phenyl]carbamate

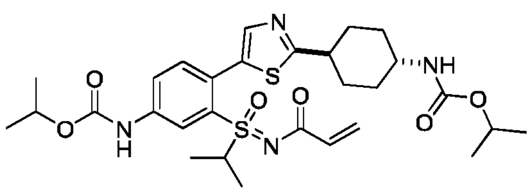

Synthetic Method

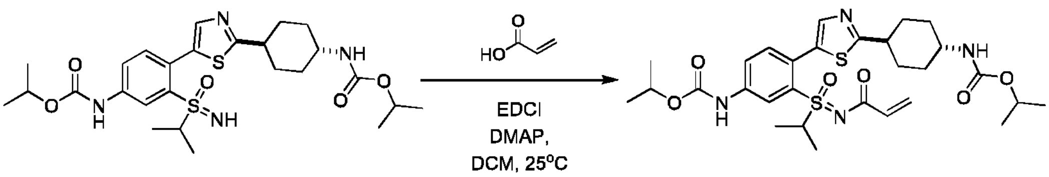


1

Isopropyl trans-N-[4-[2-[4-(isopropoxycarbonylamino)cyclohexyl]thiazol-5-yl]-3-(isopropylsulfonimidoyl)phenyl]carbamate (0.01 g, 18.16 μmol) and acrylic acid (1.44 mg, 19.97 μmol, 1.37 μL) were dissolved in anhydrous dichloromethane (1 mL). 4-Dimethylaminopyridine (1.11 mg, 9.08 μmol) and 1-(3-dimethylaminopropyl)-3-ethylcarbodiimide hydrochloride (4.18 mg, 21.79 μmol) were added into the reaction system. The reaction solution was stirred at 25°C for 1 hour. The reaction solution was concentrated to dryness under reduced pressure. The resultant was purified by preparative high-performance liquid chromatography (PhenomenexGemini-NXC18 column, 3 μm silica, 30mm in diameter, 75 mm in length) (using decreasingly polar mixtures of water (containing 0.05% aqueous ammonia) and acetonitrile (36% to 76%) as eluents) to obtain isopropyl trans-N-[4-[2-[4-(isopropoxycarbonylamino)cyclohexyl]thiazol-5-yl]-3-(isopropyl-N-prop-2-enoyl-sulfonimidoyl) phenyl]carbamate (0.84 mg).

MS m/z (ESI): 627.2 $[M+Na]^+$;

$^1H$ NMR: (400 MHz, DMSO-$d_6$) δ ppm 10.18 (s, 1H), 8.33 (d, J = 2.32 Hz, 1H), 7.75 (dd, J = 8.56, 2.08 Hz, 1H), 7.54 (s, 1H), 7.42 (d, J = 8.31 Hz, 1H), 7.02 (br d, J = 8.31 Hz, 1H), 6.09-5.90 (m, 2H), 5.68 (dd, J = 9.41, 2.69 Hz, 1H), 4.93 (quin, J = 6.36 Hz, 1H), 4.83-4.67 (m, 1H), 3.27-3.23 (m, 1H), 2.99-2.79 (m, 1H), 2.09 (br d, J = 9.78 Hz, 2H), 1.90 (br d, J = 9.78 Hz, 2H), 1.59-1.45 (m, 2H), 1.35 (d, J = 6.72 Hz, 5H) ,1.28 (d, J = 6.24 Hz, 6H), 1.23 (br s, 1H), 1.16 (d, J = 6.24 Hz, 6H), 0.99 (d, J = 6.85 Hz, 3H).

Example 5: Synthesis of isopropyl trans-N-[3-(tert-butylsulfamoyl)-4-[5-[4-(isopropoxycarbonylamino)cyclohexyl]-1,3,4-thiadiazol-2-yl]phenyl]carbamate

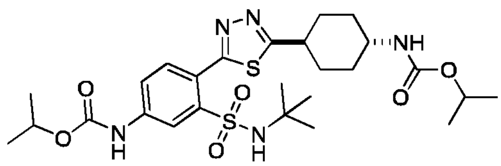

Synthetic Method

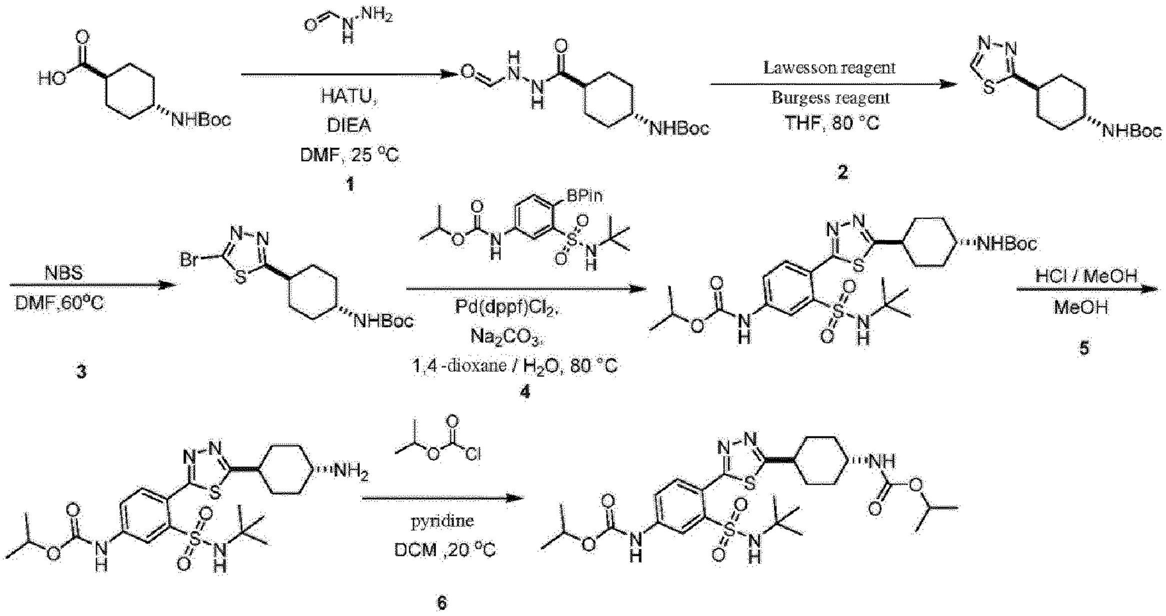


Step 1: Synthesis of tert-butyl trans-N-[4-(formamidocarbamoyl)cyclohexyl]carbamate

Trans-4-(tert-butoxycarbonylamino)cyclohexanecarboxylic acid (5 g, 20.55 mmol) and formylhydrazine (1.60 g, 26.72 mmol) were dissolved in N,N-dimethylformamide (80 mL), and O-(7-azabenzotriazol-1-yl)-N,N,N,N-tetramethyluronium hexafluorophosphate (9.38 g, 24.66 mmol) and N,N-diisopropylethylamine (6.91 g, 53.43 mmol, 9.31 mL)

were added. The reaction solution was stirred and reacted at 25°C for 0.5 hours. After the reaction was complete, the solvent N,N-dimethylformamide (80 mL) was evaporated to dryness by using a rotary evaporator. Thereafter, 400 mL of water and 4 mL of methanol were added into the mixture. The resultant was filtered to obtain tert-butyl trans-N-[4-(formamidocarbamoyl)cyclohexyl]carbamate as the product (3 g, 10.51 mmol).

MS m/z (ESI): 230.2 $[M-55]^+$;

Step 2: Synthesis of tert-butyl trans-N-[4-(1,3,4-thiadiazol-2-yl)cyclohexyl]carbamate

Tert-butyl trans-N-[4-(formamidocarbamoyl)cyclohexyl]carbamate (750 mg, 2.63 mmol) was dissolved in anhydrous tetrahydrofuran (30 mL). Afterwards, Lawesson reagent (1.28 g, 3.15 mmol) and Burgess reagent (626.37 mg, 2.63 mmol) were added into the reaction system. The reaction solution was stirred and reacted at 80°C for 16 hours. The reaction solution was subjected to rotary evaporation under reduced pressure to remove tetrahydrofuran. The remaining organic phase was added with water (30 mL) and extracted three times with ethyl acetate (10 mL × 3) to separate the organic phase. The organic phase was concentrated to dryness under reduced pressure, and purified by column chromatography (silica, ethyl acetate:petroleum ether = 2/1 to 3/1) to obtain tert-butyl trans-N-[4-(1,3,4-thiadiazol-2-yl)cyclohexyl]carbamate as the product (280 mg).

MS m/z (ESI): 227.9 $[M-55]^+$;

Step 3: Synthesis of tert-butyl N-[4-(5-bromo-1,3,4-thiadiazol-2-yl)cyclohexyl]carbamate

Tert-butyl trans-N-[4-(1,3,4-thiadiazol-2-yl)cyclohexyl]carbamate (0.1 g, 352.87 μmol) as a reactant was dissolved in N,N-dimethylformamide (3 mL), and N-bromosuccinimide (188.42 mg, 1.06 mmol) was added into the reaction system. The reaction solution was stirred at 60°C for 10 hours. The reaction solution was concentrated under reduced pressure to remove the solvent N,N-dimethylformamide (3 mL). The mixture was added with 3 mL of water and extracted with dichloromethane (3 mL × 2) to separate the organic layer. The organic phase was dried over anhydrous sodium sulfate and subjected to suction filtration. The filtrate was concentrated to dryness under reduced pressure. The resultant was purified by column chromatography (silica, petroleum ether:ethyl acetate = 0% to 20%) to obtain tert-butyl trans-N-[4-(5-bromo-1,3,4-thiadiazol-2-yl)cyclohexyl]carbamate as the product (0.030 g).

MS m/z (ESI): 307.8 $[M-55]^+$

Step 4: Synthesis of isopropyl trans-N-[4-[5-[4-(tert-butoxycarbonylamino)cyclohexyl]-1,3,4-thiadiazol-2-yl]-3-(tert-butylsulfamoyl)phenyl]carbamate

Tert-butyl trans-N-[4-(5-bromo-1,3,4-thiadiazol-2-yl)cyclohexyl]carbamate (0.02 g, 55.21 μmol) and isopropyl (3-(N-(tert-butyl)sulfamoyl)-4-(4,4,5,5-tetramethyl-1,3,2-dioxaborolan-2-yl)phenyl)carbamate (24.31 mg, 55.21 μmol) as reactants were dissolved in dioxane (1 mL). Under the protection of nitrogen, sodium carbonate (2M, 82.81 μL) and [1,1-bis(diphenylphosphino)ferrocene]dichloropalladium - dichloromethane (4.51 mg, 5.52 μmol) were added. The reaction solution was stirred at 80°C for 2 hours. The reaction system was evaporated to dryness via rotary evaporation, and the resultant was then purified by thin-layer chromatography (silica, petroleum ether:tetrahydrofuran = 3:1) to obtain isopropyl trans-N-[4-[5-[4-(tert-butoxycarbonylamino)cyclohexyl]-1,3,4-thiadiazol-2-yl]-3-(tert-butyl sulfamoyl)phenyl]carbamate (27 mg).

MS m/z (ESI): 596.3 $[M+H]^+$

Step 5: Synthesis of isopropyl trans-(4-(5-(4-aminocyclohexyl)-1,3,4-thiadiazol-2-yl)-3-(N-(tert-butyl)sulfamoyl)phenyl)carbamate

Isopropyl trans-N-[4-[5-[4-(tert-butoxycarbonylamino)cyclohexyl]-1,3,4-thiadiazol-2-yl]-3-(tert-butylsulfamoyl)phenyl]carbamate (0.027 g, 45.32 μmol) as a reactant was dissolved in anhydrous methanol (2 mL). A solution of hydrochloric acid in methanol (4M, 113.30 μL) was added. The reaction solution was stirred at 15°C for 1 hour. The reaction system was evaporated to dryness via rotary evaporation to obtain isopropyl trans-(4-(5-(4-aminocyclohexyl)-1,3,4-thiadiazol-2-yl)-3-(N-(tert-butyl)sulfamoyl)phenyl)carbamate (0.02 g).

MS m/z (ESI): 496.2 $[M+H]^+$;

Step 6: Synthesis of isopropyl trans-N-[3-(tert-butylsulfamoyl)-4-[5-[4-(isopropoxycarbonylamino)cyclohexyl]-1,3,4-thiadiazol-2-yl]phenyl]carbamate

Isopropyl trans-N-[4-[5-(4-aminocyclohexyl)-1,3,4-thiadiazol-2-yl]-3-(tert-butylsulfamoyl) phenyl]carbamate (22 mg, 44.39 μmol) as a reactant was dissolved in dichloromethane (1 mL), and pyridine (35.11 mg, 443.86 μmol) and

isopropyl chloroformate (16.32 mg, 133.16 μmol) were added. The reaction solution was stirred at 20°C for 3 hours. The reaction system was evaporated to dryness via rotary evaporation. The resultant was purified by preparative high-performance liquid chromatography (PhenomenexGemini-NXC18 column, 3 μm silica, 30 mm in diameter, 75 mm in length) (using decreasingly polar mixtures of water (containing 0.05% aqueous ammonia) and acetonitrile (36% to 76%) as eluents) to obtain isopropyl trans-N-[3-(tert-butylsulfamoyl)-4-[5-[4-(isopropoxycarbonylamino)cyclohexyl]-1,3,4-thiadiazol-2-yl]phenyl]carbamate (2.78 mg).

MS m/z (ESI): 582.3 $[M+H]^+$;

$^1$HNMR(400MHz, DMSO-$d_6$) δ 10.47-9.5 (m, 1H), 8.36 (d, J = 2.25 Hz, 1H), 7.74 (dd, J = 8.50, 2.13 Hz, 1H), 7.64 (d, J = 8.50 Hz, 1H), 7.30 (s, 1H), 7.03 (d, J = 8.50 Hz, 1H), 4.94 (quin, J = 6.28 Hz, 1H), 4.75 (dt, J = 12.32, 6.35 Hz, 1H), 3.25-3.03 (m, 2H), 2.16 (d, J = 12.38 Hz, 2H), 1.93 (br d, J = 10.26 Hz, 2H), 1.71-1.56 (m, 2H), 1.41-1.31 (m, 2H), 1.28 (d, J = 6.25 Hz, 6H), 1.17 (d, J = 6.25 Hz, 6H), 1.14 (s, 9H).

Example 6: Synthesis of isopropyl 2-[2-(2-tert-butylsulfamoyl)-4-(isopropoxycarbonylamino)phenyl]-6,7-dihydro-4H-thiazolo[5,4-c]pyridine-5-carboxylate

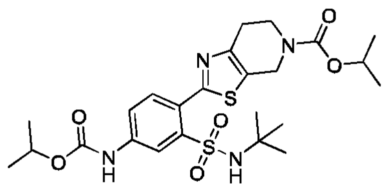

Synthetic Method

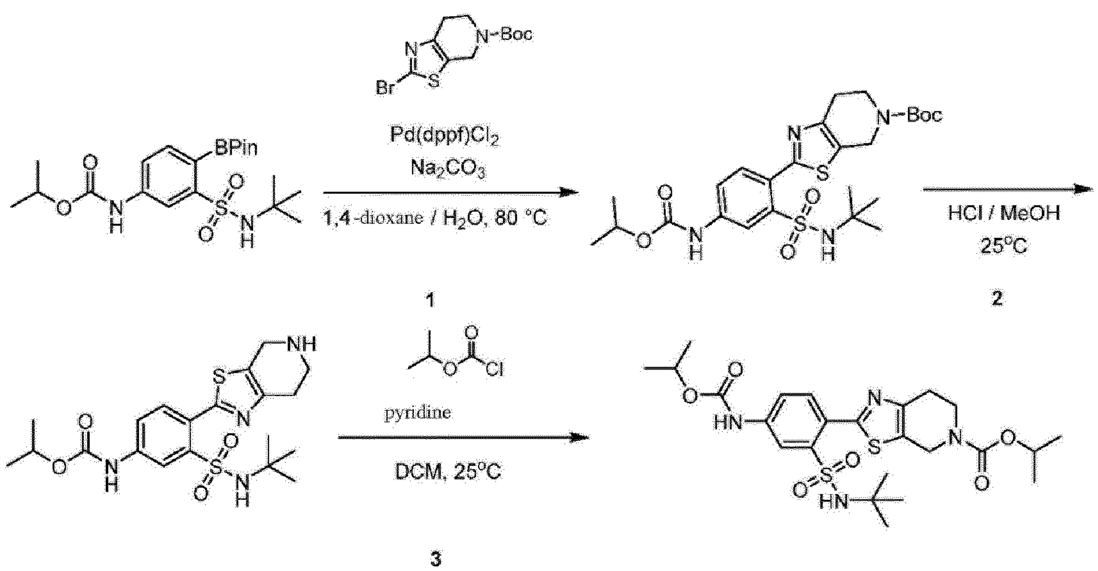


Step 1: Synthesis of 2-[2-(tert-butylsulfamoyl)-4-(isopropoxycarbonylamino) phenyl]tert-butyl-6,7-dihydro-4H-thiazolo[5,4-c]pyridine-5-carboxylate

Isopropyl (3-(N-(tert-butyl)sulfamoyl)-4-(4,4,5,5-tetramethyl-1,3,2-dioxaborolan-2-yl) phenyl)carbamate (100 mg, 313.27 μmol) and tert-butyl 2-bromo-6,7-dihydro-4H-thiazolo[5,4-c]pyridine-5-carboxylate (137.95 mg, 313.27 μmol) were dissolved in 1,4-dioxane (10 mL) and water (2 mL), and sodium carbonate (66.41 mg, 626.53 μmol) and 1,1-bis(diphenylphosphino)ferrocene dichloropalladium (II) - dichloromethane complex (25.58 mg, 31.33 μmol) were added. Under the protection of nitrogen, the reaction solution was stirred and reacted at 80°C for 1 hour. The reaction solution was added with water (1 mL), and extracted with dichloromethane (2 mL) three times. The organic layer was dried over anhydrous magnesium sulfate and subjected to suction filtration. The filtrate was concentrated to dryness under reduced pressure. The resultant was purified by thin-layer chromatography (silica, petroleum ether/ethyl acetate = 1/1) to obtain

2-[2-(tert-butylsulfamoyl)-4-(isopropoxycarbonylamino)phenyl]tert-butyl-6,7-dihydro-4H-thiazolo[5,4-c]pyridine-5-carboxylate as the product (120 mg).

MS m/z (ESI): 553.1 $[M+H]^+$;

Step 2: Synthesis of isopropyl N-[3-(tert-butylsulfamoyl)-4-(4,5,6,7-tetrahydrothiazolo[5,4-c]pyridin-2-yl)phenyl]carbamate

2-[2-(Tert-butylsulfamoyl)-4-(isopropoxycarbonylamino)phenyl]tert-butyl-6,7-dihydro-4H-thiazolo[5,4-c]pyridine-5-carboxylate (60 mg, 108.56 μmol) was dissolved in hydrochloric acid - methanol (5 mL, 4 mol/L). The reaction solution was stirred and reacted at 25°C for 1 hour. The reaction solution was concentrated to dryness under reduced pressure to obtain isopropyl N-[3-(tert-butylsulfamoyl)-4-(4,5,6,7-tetrahydrothiazolo[5,4-c]pyridin-2-yl) phenyl]carbamate as the product (45 mg).

MS m/z (ESI): 453.3 $[M+H]^+$

Step 3: Synthesis of isopropyl 2-[2-(2-tert-butylsulfamoyl)-4-(isopropoxycarbonylamino) phenyl]-6,7-dihydro-4H-thiazolo[5,4-c]pyridine-5-carboxylate

Isopropyl N-[3-(tert-butylsulfamoyl)-4-(4,5,6,7-tetrahydrothiazolo[5,4-c]pyridin-2-yl) phenyl]carbamate (60 mg, 132.57 μmol) and isopropyl chloroformate (81.23 mg, 662.85 μmol, 92.00 μL) were dissolved in anhydrous dichloromethane (4 mL), and pyridine (314.59 mg, 3.98 mmol, 321.01 μL) was added under the condition of ice bath. The reaction solution was stirred and reacted at 25°C for 1 hour. The completion of the reaction was detected by LCMS. The reaction solution was concentrated to dryness under reduced pressure. The resultant was purified by preparative liquid chromatography (column: YMCTriartC18; 5 μm silica, 25 mm in diameter, 150 mm in length; using decreasingly polar mixtures of water (containing 0.05% aqueous ammonia) and acetonitrile (65% to 85%) as eluents) to obtain the compound isopropyl 2-[2-(2-tert-butylsulfamoyl)-4-(isopropoxycarbonylamino)phenyl]-6,7-dihydro-4H-thiazolo[5, 4-c]pyridine-5-carboxylate (5.26 mg).

MS m/z (ESI): 539.3 $[M+H]^+$

$^1H$ NMR: (400MHz, DMSO-$d_6$) δ ppm 10.18 (s, 1H), 8.35 (d, J = 2.3 Hz, 1H), 7.88 (br s, 1H), 7.76-7.71 (m, 1H), 7.70-7.65 (m, 1H), 4.94 (quin, J = 6.2 Hz, 1H), 4.84 (td, J = 12.4, 6.2 Hz, 1H), 4.72 (s, 2H), 3.75 (t, J = 5.6 Hz, 2H), 2.84 (br s, 2H), 1.28 (d, J = 6.0 Hz, 6H), 1.23 (d, J = 6.3 Hz, 6H), 1.20 (s, 9H).

Example 7: Synthesis of isopropyl (3-(5-(4-(isopropoxycarbonyl)amino-2-(N-(tert-butyl) sulfamoyl)phenyl)thiazol-2-yl)bicyclo[1.1.1]pent-1-yl)carbamate

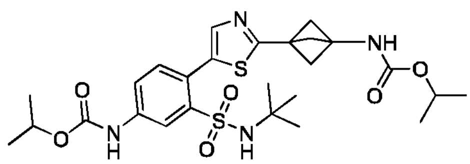

Synthetic Method

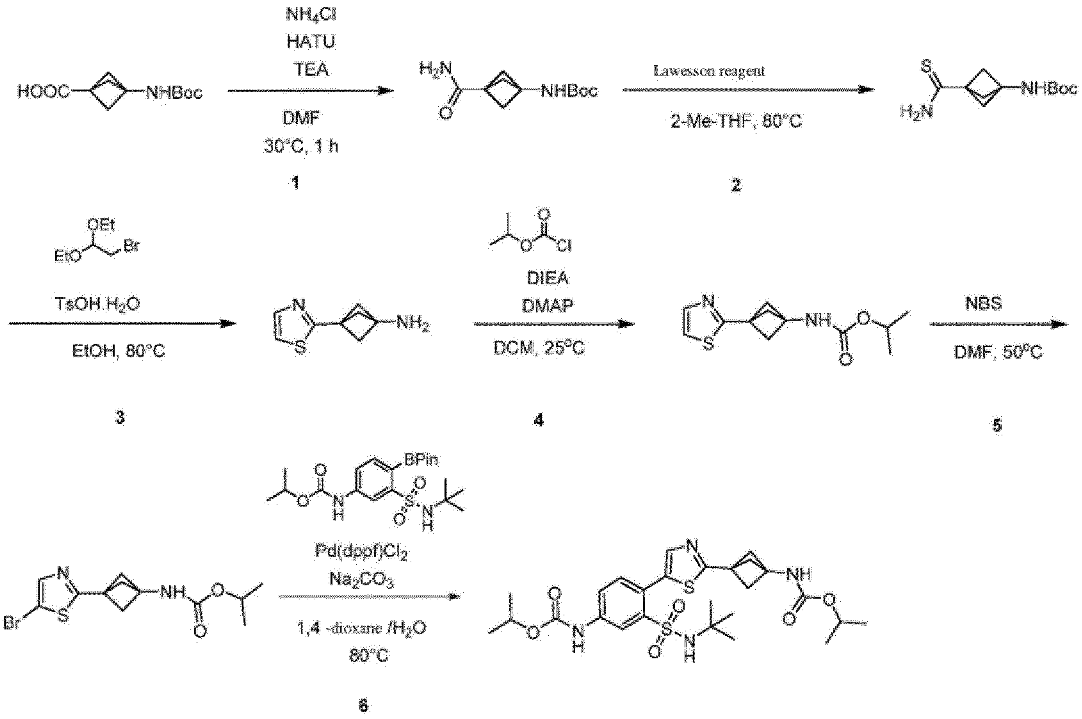


Step 1: Synthesis of tert-butyl (3-carbamoylbicyclo[1.1.1]pent-1-yl)carbamate

3-((Tert-butoxycarbonyl)amino)bicyclo[1.1.1]pentane-1-carboxylic acid (250 mg, 1.10 mmol) was dissolved in N,N-dimethylformamide (10 mL), and O-(7-azabenzotriazol-1-yl)-N,N,N,N-tetramethyluronium hexafluorophosphate (627.42 mg, 1.65 mmol), ammonium chloride (176.53 mg, 3.30 mmol) and triethylamine (333.95 mg, 3.30 mmol, 459.36 μL) were added. The reaction solution appeared as a yellow suspension and was stirred and reacted at 30°C for 1 hour. The completion of the reaction was detected by LCMS. The reaction solution was concentrated to dryness under reduced pressure and diluted by adding dichloromethane (50 mL). The organic layer was washed with water (20 mL), and a solid precipitated. The resultant was filtered to obtain tert-butyl (3-carbamoylbicyclo[1.1.1]pent-1-yl)carbamate (210 mg).

MS m/z (ESI): 227.0 $[M+H]^+$;

Step 2: Synthesis of tert-butyl (3-carbamothioylbicyclo[1.1.1]pent-1-yl)carbamate

Tert-butyl (3-carbamoylbicyclo[1.1.1]pent-1-yl)carbamate (230 mg, 1.02 mmol) was dissolved in 2-methyltetrahydrofuran (5.00 mL), and Lawesson reagent (411.13 mg, 1.02 mmol) was added. The reaction solution was stirred and reacted at 80°C for 1 hour. The reaction solution was added with a saturated aqueous sodium carbonate solution (10 mL) so that the pH of the mixture was adjusted to 8~9, and the resulting mixture was extracted with ethyl acetate (20 mL) three times. The organic layer was dried over anhydrous magnesium sulfate and subjected to suction filtration. The filtrate was concentrated to dryness under reduced pressure and purified by column chromatography (silica, petroleum ether/ethyl acetate = 2/1) to obtain tert-butyl (3-carbamothioylbicyclo[1.1.1]pent-1-yl)carbamate (170 mg).

MS m/z (ESI): 186.9 $[M-55]^+$;

Step 3: Synthesis of 3-(thiazol-2-yl)bicyclo[1.1.1]pentyl-1-amino

Tert-butyl (3-carbamothioylbicyclo[1.1.1]pent-1-yl)carbamate (170 mg, 701.50 μmol) and bromoacetaldehyde diethyl acetal (276.49 mg, 1.40 mmol, 211.06 μL) were dissolved in anhydrous ethanol (2 mL), and p-toluenesulfonic acid monohydrate (266.88 mg, 1.40 mmol) was added. The reaction solution was stirred and reacted at 80°C for 1 hour. The completion of the reaction was detected by LCMS. The reaction solution was added with a saturated aqueous sodium carbonate solution (10 mL) so that the pH of the mixture was adjusted to 8~9, and the resulting mixture was extracted with ethyl acetate (5 mL) six times. The organic layer was dried over anhydrous magnesium sulfate and subjected to suction filtration. The filtrate was concentrated to dryness under reduced pressure to obtain 3-(thiazol-2-yl)bicyclo[1.1.1]pentyl-1-amino (80 mg).

MS m/z (ESI): 167.0 $[M+H]^+$;

Step 4: Synthesis of isopropyl (3-(thiazol-2-yl)bicyclo[1.1.1]pent-1-yl)carbamate

3-(Thiazol-2-yl)bicyclo[1.1.1]pentyl-1-amino (80 mg, 481.22 μmol) and isopropyl chloroformate (117.95 mg, 962.45 μmol, 133.58 μL) were dissolved in dichloromethane (3 mL), and N,N-diisopropylethylamine (124.39 mg, 962.45 μmol, 167.64 μL) and 4-dimethylaminopyridine (29.40 mg, 240.61 μmol) were added. The reaction solution was stirred and reacted at 25°C for 1 hour. The reaction solution was concentrated to dryness under reduced pressure and purified by thin-layer chromatography (silica, petroleum ether/ethyl acetate = 2/1) to obtain isopropyl (3-(thiazol-2-yl)bicyclo[1.1.1]pent-1-yl)carbamate (31 mg).
MS m/z (ESI): 253.1 $[M+H]^+$;

Step 5: Synthesis of isopropyl (3-(5-bromothiazol-2-yl)bicyclo[1.1.1]pent-1-yl)carbamate

Isopropyl (3-(thiazol-2-yl)bicyclo[1.1.1]pent-1-yl)carbamate (31 mg, 122.85 μmol) was dissolved in N,N-dimethylformamide (4 mL), and N-bromosuccinimide (65.60 mg, 368.56 μmol) was added. The reaction solution was stirred and reacted at 50°C for 2 hours. The reaction solution was concentrated to dryness under reduced pressure and purified by thin-layer chromatography (silica, petroleum ether/ethyl acetate = 1/1) to obtain isopropyl (3 -(5 -bromothiazol-2-yl)bicyclo[1.1.1]pent-1-yl)carbamate (34 mg).
MS m/z (ESI): 331.1, 333.1 $[M+H]^+$;

Step 6: Synthesis of isopropyl (3-(5-(4-(isopropoxycarbonyl)amino-2-(N-(tert-butyl)sulfamoyl)phenyl)thiazol-2-yl)bicyclo[1.1.]pent-1-yl)carbamate

Isopropyl (3-(5-bromothiazol-2-yl)bicyclo[1.1.1]pent-1-yl)carbamate (50 mg, 150.95 μmol) and isopropyl (3-(N-(tert-butyl)sulfamoyl)-4-(4,4,5,5-tetramethyl-1,3,2-dioxaborolan-2-yl)phenyl)carbamate (66.47 mg, 150.95 μmol) were dissolved in 1,4-dioxane (2.5 mL) and water (0.5 mL), and sodium carbonate (32.00 mg, 301.91 μmol) and 1,1-bis(diphenylphosphino)ferrocene dichloropalladium (II) - dichloromethane complex (12.33 mg, 15.10 μmol) were added. Under the protection of nitrogen, the reaction solution was stirred and reacted at 80°C for 2 hours. The reaction solution was concentrated to dryness under reduced pressure. The reaction solution was added with water (1 mL) and extracted with dichloromethane (2 mL) three times. The organic layer was dried over anhydrous magnesium sulfate and subjected to suction filtration. The filtrate was concentrated to dryness under reduced pressure. The resultant was purified by preparative liquid chromatography (PhenomenexGeminiC18 column, 10 μm silica, 50 mm in diameter, 250 mm in length) (using decreasingly polar mixtures of water (containing 0.05% aqueous ammonia) and acetonitrile as eluents) to obtain the compound isopropyl (3-(5-(4-(isopropoxycarbonyl)amino-2-(N-(tert-butyl)sulfamoyl)phenyl)thiazol-2-yl)bicyclo[1.1.1]pent-1-yl)carbamate (11.3 mg).
MS m/z (ESI): 565.1 $[M+H]^+$
$^1H$ NMR (400MHz, DMSO-$d_6$) δ ppm 10.10 (s, 1H), 8.32 (d, J = 2.0 Hz, 1H), 7.94 (br s, 1H), 7.69-7.59 (m, 2H), 7.39 (d, J = 8.5 Hz, 1H), 7.17 (s, 1H), 4.93 (m, J = 6.3 Hz, 1H), 4.76 (m, J = 6.1 Hz, 1H), 2.34-2.32 (m, 1H), 2.33 (s, 5H), 1.28 (d, J = 6.3 Hz, 6H), 1.18 (br d, J = 5.8 Hz, 6H), 1.10 (s, 9H).

Example 8: Synthesis of isopropyl 7-(5-(2-(N-(tert-butyl)sulfamoyl)-4-((isopropoxycarbonyl)amino)phenyl)thiazol-2-yl)-2,7-diazaspiro[3.5]nonane-2-carboxylate

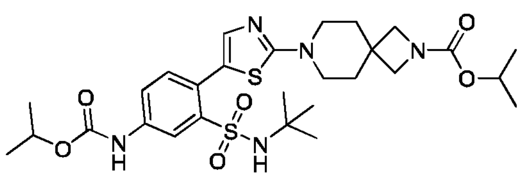

Synthetic Method

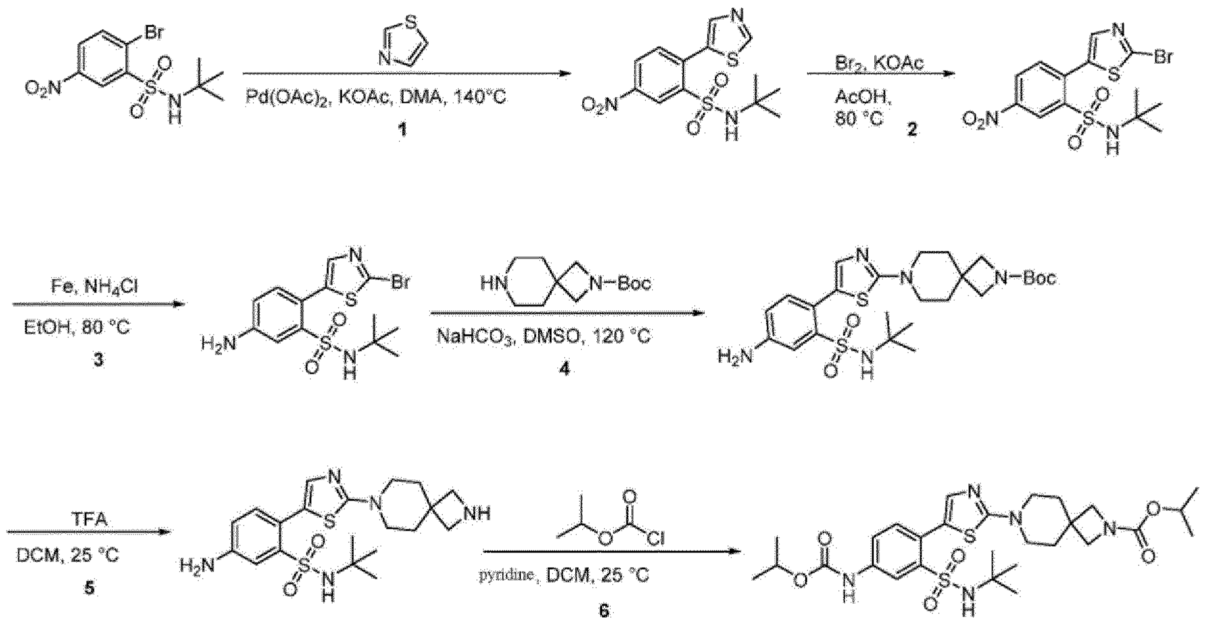


Step 1: Synthesis of N-(tert-butyl)-5-nitro-2-(thiazol-5-yl)benzenesulfonamide

2-Bromo-N-(tert-butyl)-5-nitrobenzenesulfonamide (2.0 g, 5.93 mmol) as a reactant was dissolved in N,N-dimethylacetamide (20 mL), and thiazole (1.51 g, 17.97 mmol), palladium acetate (133.16 mg, 593.14 μmol) and potassium acetate (1.75 g, 17.79 mmol) were added. The reaction solution appeared as a yellow suspension, and was stirred and reacted at 140°C for 16 hours. The reaction solution was dissolved in water (200 mL) and the resulting mixture was filtered. The filter cake was collected and concentrated to dryness under reduced pressure, and then purified by column chromatography (silica, petroleum ether/tetrahydrofuran = 3/1) to obtain N-(tert-butyl)-5-nitro-2-(thiazol-5-yl)benzenesulfonamide (0.9 g).
MS m/z (ESI): 342.1 $[M+H]^+$;

Step 2: Synthesis of 2-(2-bromothiazol-5-yl)-N-(tert-butyl)-5-nitrobenzenesulfonamide

N-(tert-butyl)-5-nitro-2-(thiazol-5-yl)benzenesulfonamide (0.8 g, 2.34 mmol) was dissolved in acetic acid (10.00 mL), and potassium acetate (1.15 g, 11.72 mmol) and liquid bromine (1.87 g, 11.72 mmol) were added. The reaction solution was stirred and reacted at 80°C for 3 hours. The reaction solution was added with a saturated aqueous sodium carbonate solution (100 mL) so that the pH of the mixture was adjusted to 8~9, and the resulting mixture was extracted with ethyl acetate (100 mL) three times. The organic phase was dried over anhydrous magnesium sulfate and subjected to suction filtration. The filtrate was concentrated to dryness under reduced pressure to obtain a crude product. The resulting crude 2-(2-bromothiazol-5-yl)-N-(tert-butyl)- 5-nitrobenzenesulfonamide (1.4 g, crude product) was directly used in the next reaction step.
MS m/z (ESI): 420.0, 422.0 $[M+H]^+$;

Step 3: Synthesis of 5-amino-2-(2-bromothiazol-5-yl)-N-(tert-butyl)benzenesulfonamide

2-(2-Bromothiazol-5-yl)-N-(tert-butyl)-5-nitrobenzenesulfonamide (1.4 g, 3.33 mmol) was dissolved in anhydrous ethanol (15 mL), and iron powder (1.86 g, 33.31 mmol) and ammonium chloride (1.78 g, 33.31 mmol) were added. The reaction solution was stirred and reacted at 80°C for 2 hours. The reaction solution was filtered. The filtrate was concentrated to dryness under reduced pressure, and then purified by column chromatography (silica, dichloromethane/methanol = 20/1) to obtain 5-amino-2-(2-bromothiazol-5-yl)-N-(tert-butyl)benzenesulfonamide (0.9 g).
MS m/z (ESI): 390.0, 392.0 $[M+H]^+$;

Step 4: Synthesis of tert-butyl 7-(5-(4-amino-2-(N-(tert-butyl)sulfamoyl)phenyl)thiazol-2-yl)-2,7-diazaspiro[3.5]nonane-2-carboxylate

5-Amino-2-(2-bromothiazol-5-yl)-N-(tert-butyl)benzenesulfonamide (20 mg, 51.24 μmol) was dissolved in dimethyl sulfoxide (2 mL), and sodium bicarbonate (12.91 mg, 153.72 μmol) and tert-butyl 2,7-diazaspiro[3.5]nonane-2-carboxylate (23.19 mg, 102.48 μmol) were added. The reaction solution was stirred and reacted under microwave irradiation at 120°C for 2 hours. The reaction solution was concentrated to dryness under reduced pressure, and purified by thin-layer chromatography (silica, dichloromethane/methanol = 20/1) to obtain tert-butyl 7-(5-(4-amino-2-(N-(tert-butyl)sulfamoyl)phenyl)thiazol-2-yl)-2,7-diazaspiro[3.5]nonane-2-carboxylate (18 mg).

MS m/z (ESI): 536.3 $[M+H]^+$;

Step 5: Synthesis of 2-(2-(2,7-diazaspiro[3.5]nonan-7-yl)thiazol-5-yl)-5-amino-N-(tert-butyl)benzenesulfonamide

Tert-butyl 7-(5-(4-amino-2-(N-(tert-butyl)sulfamoyl)phenyl)thiazol-2-yl)-2,7-diazaspiro[3. 5]nonane-2-carboxylate (18 mg, 33.60 μmol) was dissolved in dichloromethane (2 mL), and trifluoroacetic acid (180 mg, 1.58 mmol, 116.88 μL) was added. The reaction solution was stirred and reacted at 25°C for 1 hour. The reaction solution was concentrated to dryness under reduced pressure to obtain 2-(2-(2,7-diazaspiro[3.5]nonan-7-yl)thiazol-5-yl)-5-amino-N-(tert-butyl)benzenesulfonamide (0.14 g).

MS m/z (ESI): 436.2 $[M+H]^+$;

Step 6: Synthesis of isopropyl 7-(5-(2-(N-(tert-butyl)sulfamoyl)-4-((isopropoxycarbonyl) amino)phenyl)thiazol-2-yl)-2,7-diazaspiro[3.5]nonane-2-carboxylate

2-(2-(2,7-Diazaspiro[3.5]nonan-7-yl)thiazol-5-yl)-5-amino-N-(tert-butyl)benzenesulfonamide (0.14 g, crude product) was dissolved in dichloromethane (10 mL), and pyridine (254.22 mg, 3.21 mmol, 259.41 μL) and isopropyl chloroformate (157.55 mg, 1.29 mmol, 178.42 μL) were added. Under the protection of nitrogen, the reaction solution was stirred and reacted at 25°C for 16 hours. The reaction solution was concentrated to dryness under reduced pressure, and purified by preparative liquid chromatography (PhenomenexGeminiNXC18 column, 5 μm silica, 30 mm in diameter, 150 mm in length) (using decreasingly polar mixtures of water (containing 0.225% formic acid) and acetonitrile (45% to 65%) as eluents) to obtain the compound isopropyl 7-(5-(2-(N-(tert-butyl)sulfamoyl)-4-((isopropoxycarbonyl) amino)phenyl)thiazol-2-yl)-2,7-diazaspiro[3.5]nonane-2-carboxylate (3.8 mg).

MS m/z (ESI): 608.1 $[M+H]^+$

$^1$H NMR (400MHz, DMSO-$d_6$): δ ppm 10.00 (s, 1H), 8.28 (d, J = 2.2 Hz, 1H), 7.60 (dd, J = 8.3, 2.3 Hz, 1H), 7.33 (d, J = 8.4 Hz, 1H), 7.18 (s, 1H), 6.95 (s, 1H), 4.99-4.68 (m, 2H), 3.65 (br s, 4H), 3.39 (br s, 4H), 1.79 (br t, J=5.6 Hz, 4H), 1.31-1.05 (m, 21H).

Example 9 and Example 10: Synthesis of isopropyl endo-N-[3-(tert-butylsulfamoyl)-4-[2-[6-(isopropoxycarbonylamino)-3-azabicyclo[3.1.0]hex-3-yl]thiazol-5-yl]phenyl]carbamate and isopropyl exo-N-[3-(tert-butylsulfamoyl)-4-[2-[6-(isopropoxycarbonylamino)-3-azabicyclo[3.1.0]hex-3-yl] thiazol-5-yl]phenyl]carbamate

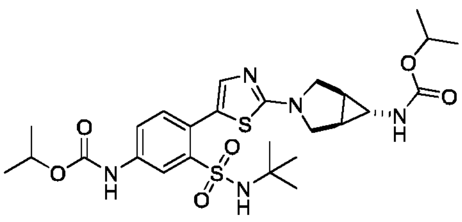

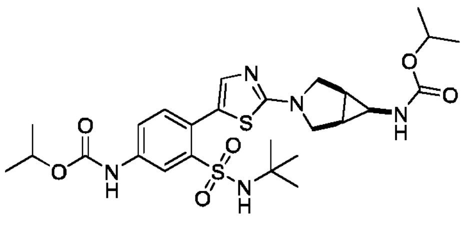

Synthetic Method

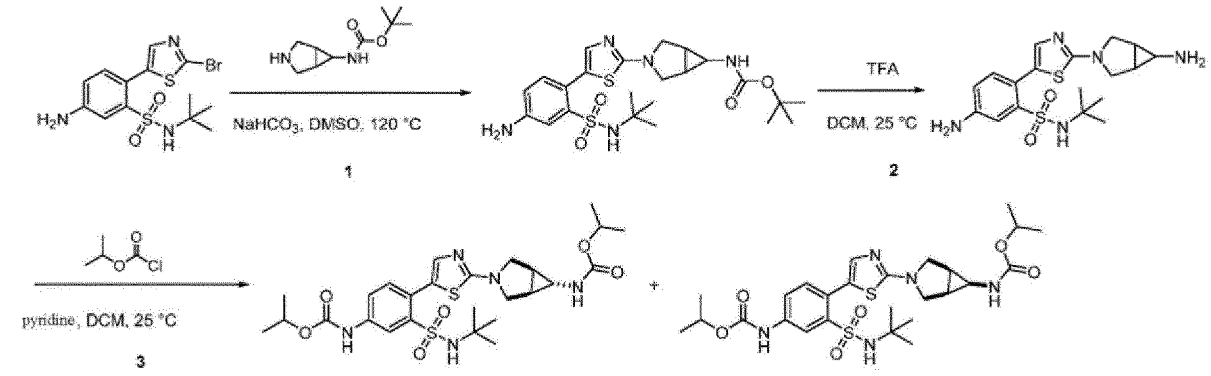


Step 1: Synthesis of tert-butyl (3-(5-(4-amino-2-(N-(tert-butyl)sulfamoyl)phenyl)thiazol-2-yl)-3-azabicyclo[3.1.0]hex-6-yl)carbamate

5-Amino-2-(2-bromothiazol-5-yl)-N-(tert-butyl)benzenesulfonamide (40 mg, 102.48 μmol) was dissolved in dimethyl sulfoxide (2 mL), and sodium bicarbonate (25.83 mg, 307.44 μmol) and tert-butyl 3-azabicyclo[3.1.0]hexyl-6-carbamate (60.95 mg, 307.44 μmol) were added. The reaction solution was stirred and reacted under microwave irradiation at 120°C for 2 hours. The completion of the reaction was detected by LCMS. The reaction solution was concentrated to dryness under reduced pressure and purified by thin-layer chromatography (silica, dichloromethane/methanol = 20/1) to obtain tert-butyl (3-(5-(4-amino-2-(N-(tert-butyl) sulfamoyl)phenyl)thiazol-2-yl)-3-azabicyclo[3.1.0]hex-6-yl)carbamate as the product (80 mg).

MS m/z (ESI): 508.3 $[M+H]^+$;

Step 2: Synthesis of 5-amino-2-(2-(6-amino-3-azabicyclo[3.1.0]hex-3-yl)thiazol-5-yl)-N-(tert-butyl)benzenesulfonamide

Tert-butyl (3-(5-(4-amino-2-(N-(tert-butyl)sulfamoyl)phenyl)thiazol-2-yl)-3-azabicyclo [3.1.0]hex-6-yl)carbamate (120 mg, 236.37 μmol) was dissolved in dichloromethane (12 mL), and trifluoroacetic acid (1.2 g, 10.52 mmol, 779.22 μL) was added. The reaction solution was stirred and reacted at 25°C for 1 hour. The completion of the reaction was detected by LCMS. The reaction solution was concentrated to dryness under reduced pressure to obtain 5-amino-2-(2-(6-amino-3-azabicyclo[3.1.0]hex-3-yl)thiazol-5-yl)-N-(tert-butyl)benzenesulfonamide as the product (0.1 g, crude product).

MS m/z (ESI): 408.1 $[M+H]^+$;

Step 3: Synthesis of isopropyl N-[3-(tert-butylsulfamoyl)-4-[2-[6-(isopropoxycarbonylamino)-3-azabicyclo[3.1.0]hex-3-yl]thiazol-5-yl]phenyl]carbamate

5-Amino-2-(2-(6-amino-3-azabicyclo[3.1.0]hex-3-yl)thiazol-5-yl)-N-(tert-butyl) benzenesulfonamide (0.1 g, crude product) was dissolved in dichloromethane (10 mL), and pyridine (194.09 mg, 2.45 mmol, 198.05 μL) and isopropyl chloroformate (180.42 mg, 1.47 mmol, 204.32 μL) were added. Under the protection of nitrogen, the reaction solution was stirred and reacted at 25°C for 16 hours. The completion of the reaction was detected by LCMS. The reaction solution was concentrated to dryness under reduced pressure and purified by preparative liquid chromatography (PhenomenexGeminiNXC18 column, 5 μm silica, 30 mm in diameter, 150 mm in length) (using decreasingly polar mixtures of water (containing 0.05% aqueous ammonia and 10mM ammonium bicarbonate) and acetonitrile (50% to 70%) as eluents) to obtain two products, *i.e.* isopropyl endo-N-[3-(tert-butylsulfamoyl)-4-[2-[6-(isopropoxycarbonylamino)-3-azabicyclo[3.1.0]hex-3-yl]thiazol-5-yl]phenyl]carbamate and isopropyl exo-N-[3-(tert-butylsulfamoyl)-4-[2-[6-(isopropoxycarbonylamino)-3-azabicyclo [3.1.0]hex-3-yl]thiazol-5-yl]phenyl]carbamate (17.0 mg/17.0 mg) respectively, the correspondence of specific configurations was unknown.

Product 1:

MS m/z (ESI): 580.3 $[M+H]^+$

$^1$H NMR (400MHz, DMSO-$d_6$) δ ppm 10.01 (s, 1H), 8.27 (d, J = 2.3 Hz, 1H), 7.59 (dd, J = 8.4, 2.1 Hz, 1H), 7.38-7.08 (m, 3H), 7.00-6.82 (m, 1H), 4.99-4.67 (m, 2H), 3.67-3.55 (m, 2H), 3.46 (br d, J = 10.3 Hz, 2H), 2.69-2.59 (m, 1H), 2.06-1.92 (m, 2H), 1.29-1.05 (m, 21H).

Product 2:

MS m/z (ESI): 580.3 $[M+H]^+$;

$^1H$ NMR (400MHz, DMSO-$d_6$) δ ppm 10.01 (s, 1H), 8.27 (d, J = 2.2 Hz, 1H), 7.60 (dd, J = 8.4, 2.3 Hz, 1H), 7.43-7.29 (m, 2H), 7.19 (s, 1H), 6.96 (s, 1H), 4.96-4.68 (m, 2H), 3.60-3.47 (m, 4H), 2.30 (br s, 1H), 1.83 (br s, 2H), 1.28-1.07 (m, 21H).

Example 11: Synthesis of isopropyl N-[3-(tert-butylsulfamoyl)-4-[2-[4-(isopropoxycarbonylamino)azepan-1-yl]thiazol-5-yl]phenyl]carbamate

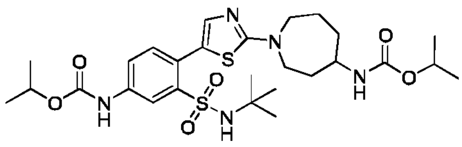

Synthetic Method

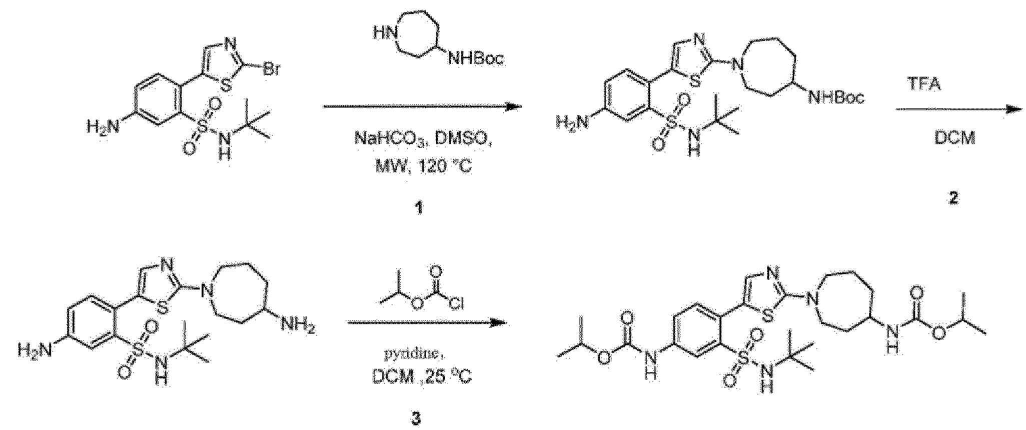


Step 1: Synthesis of tert-butyl N-[1-[1-[5-[4-amino-2-(tert-butylsulfamoyl)tert-butyl] phenyl]thiazol-2-yl]aza-4-yl]carbamate

5-Amino-2-(2-bromothiazol-5-yl)-N-tert-butylbenzenesulfonamide (0.03 g, 76.86 μmol), tert-butyl azepan-4-ylcarbamate (49.41 mg, 230.58 μmol) and sodium bicarbonate (19.37 mg, 230.58 μmol, 8.97 μL) as reactants were added into a 4-mL microwave reaction flask, dimethyl sulfoxide (1 mL) was added, and the mixture was reacted under microwave irradiation at 120°C for 5 hours. The reaction system was added with 2 mL of water, and extracted twice with dichloromethane (2 mL × 2) to separate the organic phase. The organic phase was dried over anhydrous sodium sulfate and concentrated to dryness under reduced pressure. The resultant was purified by thin-layer chromatography (silica, dichloromethane/methanol = 95:5) to obtain tert-butyl N-[1-[1-[5-[4-amino-2-(tert-butylsulfamoyl)tert-butyl]phenyl]thiazol-2-yl]aza-4-yl]carbamate as the product (40 mg).

MS m/z (ESI): 524.3 $[M+H]^+$;

Step 2: Synthesis of 5-amino-2-[2-(4-aminoaza-1-yl)thiazol-5-yl]-N-tert-butylbenzenesulfonamide

Tert-butyl N-[1-[1-[5-[4-amino-2-(tert-butylsulfamoyl)tert-butyl]phenyl]thiazol-2-yl]aza-4-yl]carbamate (0.04 g, 76.38 μmol) as a reactant was dissolved in anhydrous dichloromethane (5 mL), and trifluoroacetic acid (383.19 mg, 3.36 mmol, 248.82 μL) was added. The reaction solution was stirred and reacted at 25°C for 1 hour. The completion of the reaction was detected by LCMS. The resultant was concentrated under reduced pressure to remove the solvent dichloromethane to obtain crude 5-amino-2-[2-(4-aminoaza-1-yl)thiazol-5-yl]-N-tert-butylbenzenesulfonamide (80 mg, crude product).

MS m/z (ESI): 424.1 [M+H]$^+$;

Step 3: Synthesis of isopropyl N-[3-(tert-butylsulfamoyl)-4-[2-[4-(isopropoxycarbonylamino)azepan-1-yl]thiazol-5-yl]phenyl]carbamate

5-Amino-2-[2-(4-aminoaza-1-yl)thiazol-5-yl]-N-tert-butylbenzenesulfonamide (32 mg, 75.54 μmol) as a reactant was dissolved in anhydrous dichloromethane (2 mL). Pyridine (3.92 g, 49.56 mmol, 4 mL) and isopropyl chloroformate (55.55 mg, 453.26 μmol, 62.91 μL) were added into the reaction system. The reaction solution was stirred at 25°C for 5 hours. LCMS showed that the reaction was complete. The resulting mixture was concentrated under reduced pressure to remove dichloromethane. The resultant was purified by preparative high-performance liquid chromatography (YMCTriartC18 column, 7 μm silica, 50 mm in diameter, 250 mm in length) (using decreasingly polar mixtures of water (containing 0.05% aqueous ammonia) and acetonitrile (35% to 75%) as eluents) to obtain the compound isopropyl N-[3-(tert-butylsulfamoyl)-4-[2-[4-(isopropoxycarbonylamino)azepan-1-yl]thiazol-5-yl]phenyl]carbamate (6.42 mg).

MS m/z (ESI): 596.3 [M+H]$^+$;

$^1$H-NMR: (400 MHz, DMSO-$d_6$) δ 9.98 (s, 1H), 8.27 (d, J = 2.20 Hz, 1H), 7.59 (dd, J = 8.38, 2.26 Hz, 1H), 7.35 (d, J = 8.44 Hz, 1H), 7.18 (s, 1H), 7.09 (br d, J = 7.58 Hz, 1H), 6.84 (s, 1H), 4.92 (spt, J = 6.22 Hz, 1H), 4.80-4.64 (m, 1H), 3.79-3.65 (m, 1H), 3.61-3.47 (m, 2H), 3.46-3.36 (m, 2H), 2.01-1.85 (m, 2H), 1.84-1.70 (m, 2H), 1.69-1.59 (m, 1H), 1.51-1.35 (m, 1H), 1.27 (d, J = 6.24 Hz, 6H), 1.15 (d, J = 6.24 Hz, 6H), 1.10 (s, 9H).

Example 12: Synthesis of isopropyl trans-N-[4-[5-[2-(tert-butylsulfamoyl)-4-(3,4-dihydro-1H-isoquinoline-2-carbonylamino)phenyl]thiazol-2-yl]cyclohexyl]carbamate

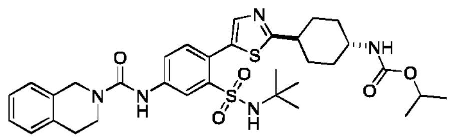

Synthetic Method

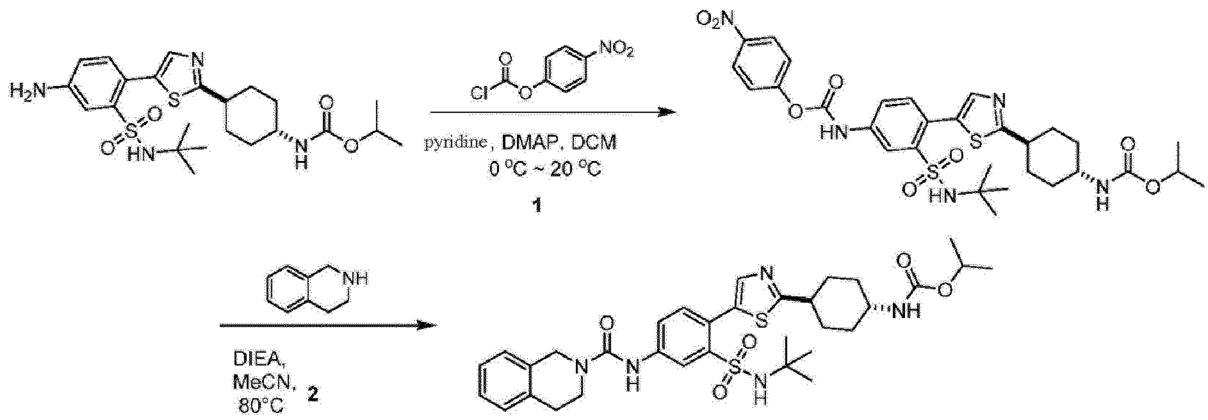


Step 1: Synthesis of 4-nitrophenyl trans-N-[3-(tert-butylsulfamoyl)-4-[2-[4-(isopropoxycarbonylamino)cyclohexyl]thiazol-5-yl]phenyl]carbamate

The above-mentioned intermediate 2, *i.e.* isopropyl trans-N-[4-[5-[4-amino-2-(tert-butylsulfamoyl)phenyl]thiazol-2-yl]cyclohexyl]carbamate (100 mg, 202.16 μmol) was dissolved in dichloromethane (6 mL). At 0°C, 4-nitrophenyl chloroformate (65.20 mg, 323.45 μmol) was added, and 4-dimethylaminopyridine (12.35 mg, 101.08 μmol) and pyridine (47.97 mg, 606.47 μmol, 48.95 μL) were added. The reaction solution was stirred and reacted at 20°C for 0.5 hours. The reaction solution was concentrated to dryness under reduced pressure and no further purification was conducted, so as to obtain 4-nitrophenyl trans-N-[3-(tert-butyl sulfamoyl)-4-[2-[4-(isopropoxycarbonylamino)cyclohexyl]thiazol-5-yl]phenyl]carbamate as the product (130 mg).

MS m/z (ESI): 660.3 $[M+H]^+$;

Step 2: Synthesis of isopropyl trans-N-[4-[5-[2-(tert-butylsulfamoyl)-4-(3,4-dihydro-1H-isoquinoline-2-carbonylamino)phenyl]thiazol-2-yl]cyclohexyl]carbamate

4-Nitrophenyl trans-N-[3-(tert-butylsulfamoyl)-4-[2-[4-(isopropoxycarbonylamino) cyclohexyl]thiazol-5-yl]phenyl]carbamate (133 mg, 201.58 μmol) and 1,2,3,4-tetrahydroisoquinoline (80.55 mg, 604.75 μmol, 75.99 μL) were dissolved in acetonitrile (4 mL), and N,N-diisopropylethylamine (65.13 mg, 503.96 μmol, 87.78 μL) was added. The reaction solution was stirred and reacted at 80°C for 1 hour. The reaction solution was cooled to 20°C and concentrated to dryness under reduced pressure. A solution of sodium carbonate (30 mg) in water (3 mL) was added to the residue and the resulting mixture was stirred for 3 minutes. The extracted organic phase was dried over anhydrous magnesium sulfate, filtered, and concentrated to dryness. The resultant was purified by preparative liquid chromatography (PhenomenexGeminiC18 column, 10 μm silica, 50 mm in diameter, 250 mm in length) (using decreasingly polar mixtures of water (containing 0.05% aqueous ammonia) and acetonitrile as eluents) to obtain isopropyl trans-N-[4-[5-[2-(tert-butylsulfamoyl)-4-(3,4-dihydro-1H-isoquinoline-2-carbonylamino)phenyl]thiazol-2-yl]cyclohexyl]carbamate as the product (36 mg).

MS m/z (ESI): 654.1 $[M+H]^+$;

$^1$H NMR (400 MHz, DMSO-$d_6$) δ ppm 9.05 (s, 1H), 8.33 (d, J = 2.25 Hz, 1H), 7.78 (dd, J = 8.50, 2.13 Hz, 1H), 7.66 (s, 1H), 7.34 (d, J = 8.50 Hz, 1H), 7.20 (s, 4H), 7.03 (br d, J = 7.50 Hz, 1H), 6.80 (s, 1H), 4.74 (dt, J = 12.44, 6.28 Hz, 1H), 4.67 (s, 2H), 3.73 (t, J = 5.88 Hz, 2H), 3.31 (br s, 1H), 2.95-2.84 (m, 3H), 2.13 (br d, J = 11.63 Hz, 2H), 1.91 (br d, J = 10.01 Hz, 2H), 1.66-1.49 (m, 2H), 1.42-1.28 (m, 2H), 1.16 (d, J = 6.25 Hz, 6H), 1.04 (s, 9H).

Example 13: Synthesis of isopropyl trans-N-[4-[5-[2-(tert-butylsulfamoyl)-4-(1,2,3,4-tetrahydroisoquinoline-3-carbonylamino)phenyl]thiazol-2-yl]cyclohexyl]carbamate

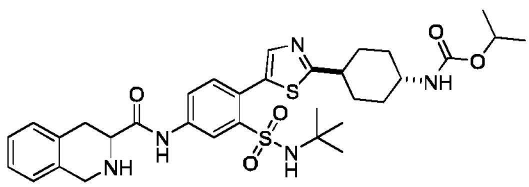

Synthetic Method

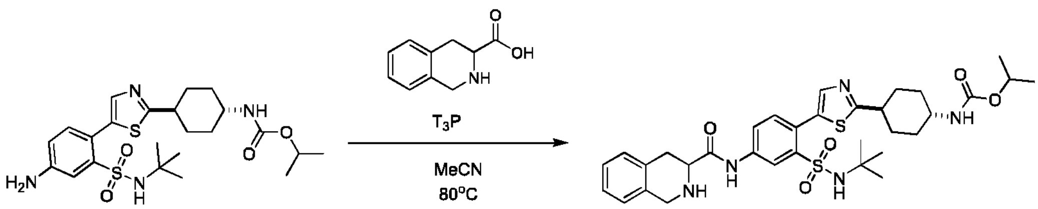


1

Isopropyl trans-N-[4-[5-[4-amino-2-(tert-butylsulfamoyl)phenyl]thiazol-2-yl]cyclohexyl] carbamate (20 mg, 40.43 μmol) and 1,2,3,4-tetrahydroisoquinoline-3-carboxylic acid (21.49 mg, 121.29 μmol) were dissolved in acetonitrile (0.5 mL), and tri-n-propylphosphoric anhydride (0.5 mL) was added. Under the protection of nitrogen, the reaction solution was stirred and reacted at 80°C for 1 hour. The reaction solution was concentrated to dryness under reduced pressure. The resultant was purified by preparative liquid chromatography (column: BostonPrimeC18; 5 μm silica, 30 mm in diameter, 150 mm in length; using decreasingly polar mixtures of water (containing 0.05% aqueous ammonia) and acetonitrile (43% to 73%) as eluents) to obtain the compound isopropyl trans-N-[4-[5-[2-(tert-butylsulfamoyl)-4-(1,2,3,4-tetrahydroisoquinoline-3-carbonylamino)phenyl]thiazol-2-yl]cyclohexyl]carbamate (11.87 mg).

MS m/z (ESI): 654.2 $[M+H]^+$;

$^1$HNMR (400MHz, DMSO-$d_6$) δ 10.37 (s, 1H), 8.56 (d, J = 2.3 Hz, 1H), 7.88 (dd, J = 8.4, 2.4 Hz, 1H), 7.69 (s,

1H), 7.43 (d, J = 8.5 Hz, 1H), 7.34-7.22 (m, 1H), 7.18-6.96 (m, 6H), 4.80-4.70 (m, 1H), 4.06-3.90 (m, 2H), 3.66 (br s, 1H), 3.41 (br d, J = 4.0 Hz, 1H), 3.05-2.81 (m, 3H), 2.14 (br d, J = 10.0 Hz, 2H), 1.92 (br d, J = 10.5 Hz, 2H), 1.65-1.50 (m, 2H), 1.41-1.27 (m, 2H), 1.17 (d, J = 6.3 Hz, 6H), 1.06 (s, 9H).

Example 14: Synthesis of isopropyl trans-N-[4-[5-[4-[[(E)-N-benzyl-N'-cyanocarbamoylamino]amino]-2-(tert-butylsulfamoyl)phenyl]thiazol-2-yl]cyclohexyl]carbamate

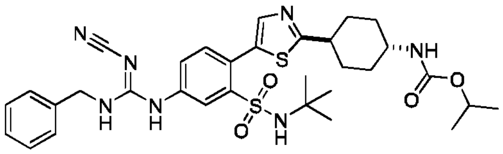

Synthetic Method

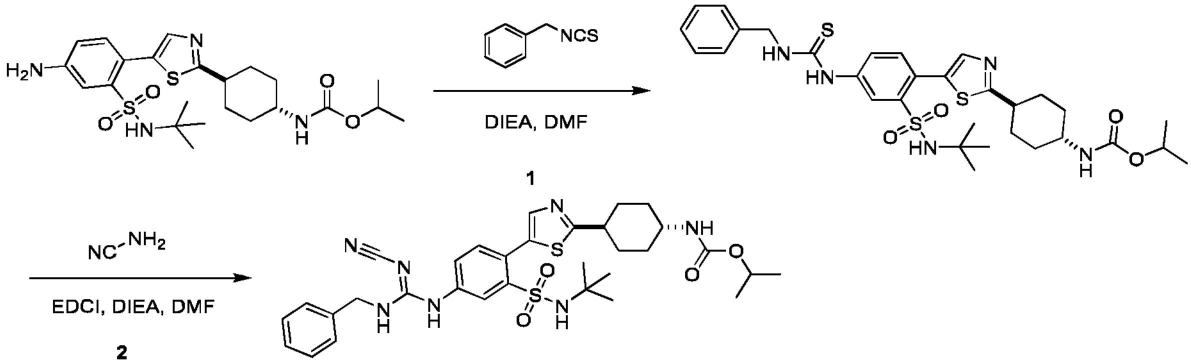


Step 1: Synthesis of isopropyl trans-N-[4-[5-[4-(benzylaminomethylthioamino)-2-(tert-butylsulfamoyl)phenyl]thiazol-2-yl]cyclohexyl]carbamate

Isopropyl trans-N-[4-[5-[4-amino-2-(tert-butylsulfamoyl)phenyl]thiazol-2-yl]cyclohexyl]carbamate (75.0 mg, 151.6 μmol) was dissolved in N,N-dimethylformamide (5.0 mL), and benzyl isothiocyanate (33.9 mg, 227.4 μmol, 30.0 μL) and N,N-diisopropylethylamine (39.2 mg, 303.2 μmol, 52.8μL) were added. The reaction solution was stirred and reacted at 20°C for 0.5 hours. The reaction solution was directly concentrated to dryness under reduced pressure and purified by column chromatography (silica, methanol/dichloromethane = 1:10) to obtain isopropyl trans-N-[4-[5-[4-(benzylaminomethylthioamino)-2-(tert-butylsulfamoyl)phenyl]thiazol-2-yl]cyclohexyl] carbamate (18.0 mg).
MS m/z (ESI): 644.3 $[M+H]^+$;

Step 2: Synthesis of isopropyl trans-N-[4-[5-[4-[[N-benzyl-N'-cyanocarbamoylamino] amino]-2-(tert-butylsulfamoyl)phenyl]thiazol-2-yl]cyclohexyl]carbamate

Isopropyl trans-N-[4-[5-[4-(benzylaminomethylthioamino)-2-(tert-butylsulfamoyl)phenyl] thiazol-2-yl]cyclohexyl]carbamate (66.0 mg, 105.1 μmol) and cyanamide (113.6 mg, 2.7 mmol) were dissolved in N,N-dimethylformamide (2.0 mL), and 1-(3-dimethylaminopropyl)-3-ethylcarbodiimide hydrochloride (30.2 mg, 157.7 μmol) and N,N-diisopropylethylamine (27.2 mg, 210.3 μmol, 36.6 μl) were added. The reaction solution was stirred and reacted at 40°C for 16 hours. The reaction solution was concentrated to dryness under reduced pressure and separated by high-performance liquid chromatography (PhenomenexGeminiC18 column, 10 μm silica, 50 mm in diameter, 250 mm in length) (using decreasingly polar mixtures of water (containing 0.05% aqueous ammonia) and acetonitrile (48% to 68%) as eluents) to obtain isopropyl trans-N-[4-[5-[4-[[N-benzyl-N'-cyanocarbamoylamino]amino]-2-(tert-butylsulfamoyl)phenyl] thiazol-2-yl]cyclohexyl]carbamate (1.58 mg).
MS m/z (ESI): 652.2 $[M+H]^+$
$^1$HNMR(400MHz, DMSO-$d_6$) δ 9.48 (br s, 1H), 8.20-8.09 (m, 1H), 8.04-7.95 (m, 1H), 7.72-7.64 (m, 1H), 7.54-7.47 (m, 1H), 7.45-7.40 (m, 1H), 7.39-7.32 (m, 5H), 7.07-6.95 (m, 1H), 7.14-6.95 (m, 1H), 4.82-4.65 (m, 1H), 4.49 (br s, 2H),

2.91 (tt, J = 11.8, 3.5 Hz, 1H), 2.56-2.52 (m, 1H), 2.20-2.07 (m, 2H), 1.91 (br d, J = 10.27 Hz, 2H), 1.57 (qd, J = 12.72, 2.81 Hz, 2H), 1.40-1.27 (m, 2H), 1.16 (d, J = 6.24 Hz, 6H), 1.05 (s, 9H).

Example 15: Synthesis of isopropyl trans-N-[4-[5-[2-(tert-butylsulfamoyl)-4-[(2-phenoxyacetyl)amino]phenyl]thiazol-2-yl]cyclohexyl]carbamate

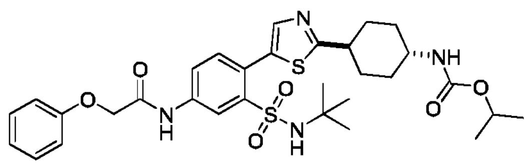

Synthetic Method

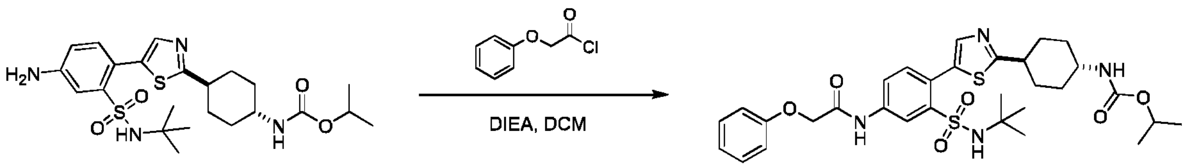


Isopropyl trans-N-[4-[5-[4-amino-2-(tert-butylsulfamoyl)phenyl]thiazol-2-yl]cyclohexyl] carbamate (60.0 mg, 121.3 μmol) was dissolved in dichloromethane (6.0 mL), and 2-phenoxyacetyl chloride (20.7 mg, 121.3 μmol, 16.7 μL) and N,N-diisopropylethylamine (31.4 mg, 242.6 μmol, 42.3 μl) were added. The reaction solution was stirred and reacted at 20°C for 1 hour. The completion of the reaction was detected by LCMS. The reaction solution was concentrated to dryness under reduced pressure and purified by preparative liquid chromatography (YMCTriartC18 column, 5 μm silica, 25 mm in diameter, 150 mm in length; using decreasingly polar mixtures of water (containing 0.05% aqueous ammonia) and acetonitrile as eluents) to obtain isopropyl trans-N-[4-[5-[2-(tert-butylsulfamoyl)-4-[(2-phenoxyacetyl)amino]phenyl]thiazol-2-yl]cyclohexyl]carbamate (22.3 mg).

MS m/z (ESI): 629.1 $[M+H]^+$

$^1$HNMR (400MHz, DMSO-$d_6$) δ 10.55 (s, 1H), 8.48 (d, J = 2.00 Hz, 1H), 7.88 (dd, J = 8.44, 2.06 Hz, 1H), 7.68 (s, 1H), 7.44 (d, J = 8.38 Hz, 1H), 7.32 (t, J = 7.94 Hz, 2H), 7.10-6.86 (m, 5H), 4.84-4.60 (m, 3H), 3.40-3.25 (m, 1H), 2.91 (br t, J = 11.94 Hz, 1H), 2.13 (br d, J = 11.51 Hz, 2H), 1.92 (br d, J = 9.63 Hz, 2H), 1.64-1.49 (m, 2H), 1.41-1.26 (m, 2H), 1.16 (d, J = 6.25 Hz, 6H), 1.05 (s, 9H).

Example 16: Synthesis of isopropyl trans-N-[4-[5-[2-(tert-butylsulfamoyl)-4-(cyclopropylmethoxycarbonylamino)phenyl]thiazol-2-yl]cyclohexyl]carbamate

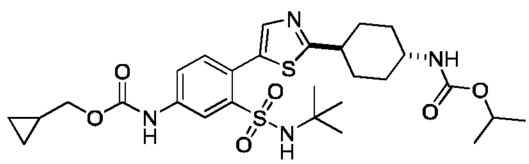

Synthetic Method

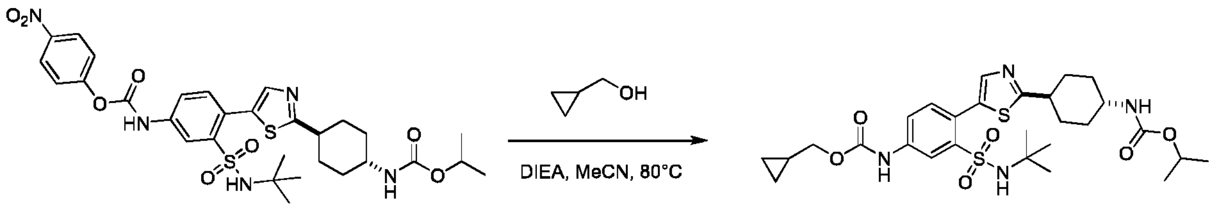


4-Nitrophenyl trans-N-[3-(tert-butylsulfamoyl)-4-[2-[4-(isopropoxycarbonylamino) cyclohexyl]thiazol-5-yl]phenyl]carbamate (50 mg, 75.78 μmol) prepared in Example 12 and cyclopropylmethanol (54.64 mg, 757.84 μmol, 59.92 μL) were dissolved in acetonitrile (4 mL), and N,N-diisopropylethylamine (24.49 mg, 189.46 μmol, 33.00 μL) was added. The reaction solution was stirred and reacted at 80°C for 1 hour. The reaction solution was concentrated to dryness under reduced pressure at 20°C and extracted with an aqueous sodium carbonate solution. The organic phase was dried over anhydrous sodium sulfate, filtered, concentrated to dryness under reduced pressure, and purified by preparative liquid chromatography (PhenomenexGeminiC18 column, 10 μm silica, 50 mm in diameter, 250 mm in length) (using decreasingly polar mixtures of water (containing 0.05% aqueous ammonia) and acetonitrile as eluents) to obtain isopropyl trans-N-[4-[5-[2-(tert-butylsulfamoyl)-4-(cyclopropylmethoxycarbonylamino)phenyl]thiazol-2-yl]cyclohexyl]carbamate as the product (9.2 mg).

MS m/z (ESI): 615.1 $[M+Na]^+$;

$^1$HNMR(400 MHz, DMSO-$d_6$) δ 10.20 (s, 1H), 10.27-10.13 (m, 1H), 8.33 (d, J = 2.01 Hz, 1H), 7.68-7.60 (m, 2H), 7.39 (d, J = 8.53 Hz, 1H), 7.62-7.09 (m, 2H), 4.80-4.62 (m, 1H), 3.96 (d, J = 7.28 Hz, 2H), 3.33-3.30 (m, 1H), 2.90 (br t, J = 12.17 Hz, 1H), 2.13 (br d, J = 11.54 Hz, 2H), 1.92 (br d, J=10.54 Hz, 2H), 1.63-1.51 (m, 2H), 1.40-1.28 (m, 2H), 1.17 (d, J = 6.02 Hz, 6H), 1.10-1.06 (m, 1H), 1.07 (s, 7H), 1.09-1.05 (m, 1H), 0.60-0.52 (m, 2H), 0.37-0.29 (m, 2H).

Example 17: Synthesis of isopropyl trans-N-[4-[5-[2-(tert-butylsulfamoyl)-4-[(1-phenylcyclopropyl)carbamoylamino]phenyl]thiazol-2-yl]cyclohexyl]carbamate

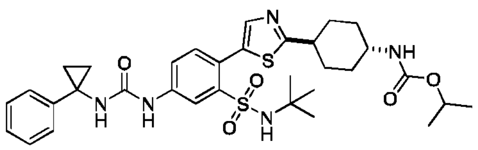

Synthetic Method

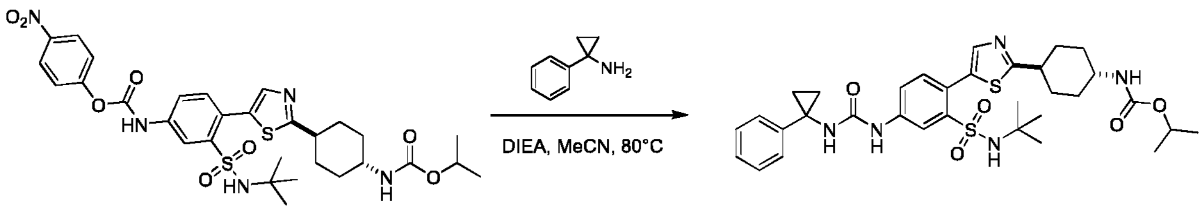


4-Nitrophenyl trans-N-[3-(tert-butylsulfamoyl)-4-[2-[4-(isopropoxycarbonylamino) cyclohexyl]thiazol-5-yl]phenyl]carbamate (50 mg, 75.78 μmol) and 1-phenylcyclopropanamine (20.19 mg, 151.57 μmol, 59.92 μL) were dissolved in acetonitrile (4 mL), and N,N-diisopropylethylamine (24.49 mg, 189.46 μmol, 33.00 μL) was added. The reaction solution was stirred and reacted at 80°C for 1 hour. The completion of the reaction was detected by LC-MS. The reaction solution was concentrated to dryness under reduced pressure at 20°C. The residue was extracted with an aqueous sodium carbonate solution/dichloromethane, and the resulting mixture was subjected to liquid-liquid separation. The resulting organic phase was dried over anhydrous sodium sulfate and filtered under reduced pressure. The filtrate was concentrated to dryness and purified by preparative liquid chromatography (PhenomenexGeminiC18 column, 10 μm silica, 50 mm in diameter, 250 mm in length) (using decreasingly polar mixtures of water (containing 0.05% aqueous ammonia) and acetonitrile as eluents) to obtain isopropyl trans-N-[4-[5-[2-(tert-butylsulfamoyl)-4-[(1-phenylcyclopropyl)carbamoylamino]phenyl]thiazol-2-yl]cyclohexyl]carbamate as the product (6.18 mg).

MS m/z (ESI):654.3 [M+H]+;

$^1$HNMR(400 MHz, DMSO-$d_6$) δ 8.97 (br s, 1H), 8.21 (d, J = 2.26 Hz, 1H), 7.64 (s, 1H), 7.60 (dd, J = 8.41, 2.13 Hz, 1H), 7.30 (dd, J = 14.56, 7.78 Hz, 3H), 7.25-7.20 (m, 2H), 7.19-7.10 (m, 2H), 7.03 (br d, J = 7.78 Hz, 1H), 6.86 (s, 1H), 4.81-4.65 (m, 1H), 3.49-3.39 (m, 1H), 3.32-3.29 (m, 2H), 2.97-2.81 (m, 1H), 2.13 (br d, J = 12.05 Hz, 2H), 1.92 (br d, J = 10.04 Hz, 2H), 1.65-1.48 (m, 2H), 1.23 (br d, J = 6.02 Hz, 4H), 1.17 (d, J = 6.27 Hz, 6H), 1.04 (s, 9H).

Example 18: Synthesis of isopropyl trans-N-[4-[5-[2-(tert-butylsulfamoyl)-4-[(2-phenylcyclopropanecarbonyl)amino]phenyl]thiazol-2-yl]cyclohexyl]carbamate

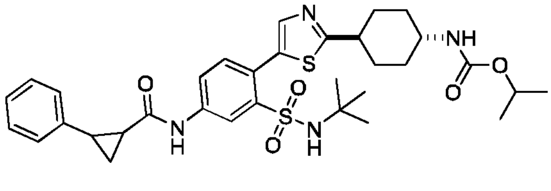

Synthetic Method

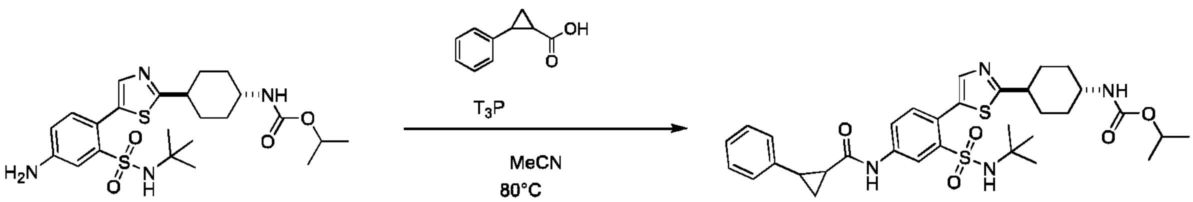


Isopropyl trans-N-[4-[5-[4-amino-2-(tert-butylsulfamoyl)phenyl]thiazol-2-yl]cyclohexyl] carbamate (40 mg, 80.86 μmol) and 2-phenylcyclopropanecarboxylic acid (15.74 mg, 97.03 μmol) were dissolved in acetonitrile (1 mL). Under the protection of nitrogen, tri-n-propylphosphoric anhydride (1.07 g, 3.36 mmol, 1 mL) was added. The reaction solution appeared as a yellow suspension, and was stirred and reacted at 80°C for 1 hour. The completion of the reaction was detected by LCMS. The reaction solution was added with water (15 mL) and extracted with dichloromethane (15 mL) twice. The organic layer was dried over anhydrous magnesium sulfate and subjected to suction filtration. The filtrate was concentrated to dryness under reduced pressure. The resultant was purified by preparative liquid chromatography (PhenomenexGeminiC18 column, 10 μm silica, 50 mm in diameter, 250 mm in length; using decreasingly polar mixtures of water (containing 0.05% aqueous ammonia) and methanol as eluents) to obtain the compound isopropyl trans-N-[4-[5-[2-(tert-butylsulfamoyl)-4-[(2-phenylcyclopropanecarbonyl)amino]phenyl]thiazol-2-yl]cyclohexyl ]carbamate (18.19 mg).

MS m/z (ESI): 639.1 [M+H]+;

$^1$HNMR(400 MHz, DMSO-$d_6$) δ 10.68 (s, 1H), 8.42 (d, J = 2.13 Hz, 1H), 7.82 (dd, J = 8.44, 2.06 Hz, 1H), 7.68 (s, 1H), 7.42 (d, J = 8.13 Hz, 1H), 7.34-7.28 (m, 2H), 7.24-7.19 (m, 3H), 7.02 (s, 1H), 6.93 (s, 1H), 4.82-4.71 (m, 1H), 3.29 (s, 2H), 2.91 (s, 1H), 2.17-2.07 (m, 3H), 1.93 (br d, J = 12.88 Hz, 2H), 1.63-1.51 (m, 3H), 1.45-1.31 (m, 3H), 1.17 (d, J = 6.25 Hz, 6H), 1.06 (s, 9H).

Example 19: Synthesis of isopropyl trans-N-[3-(tert-butylsulfonylamino)-4-[2-[4-(isopropoxycarbonylamino)cyclohexyl]thiazol-5-yl]phenyl]carbamate

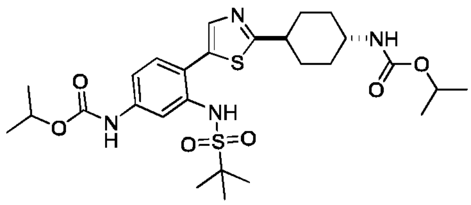

Synthetic Method

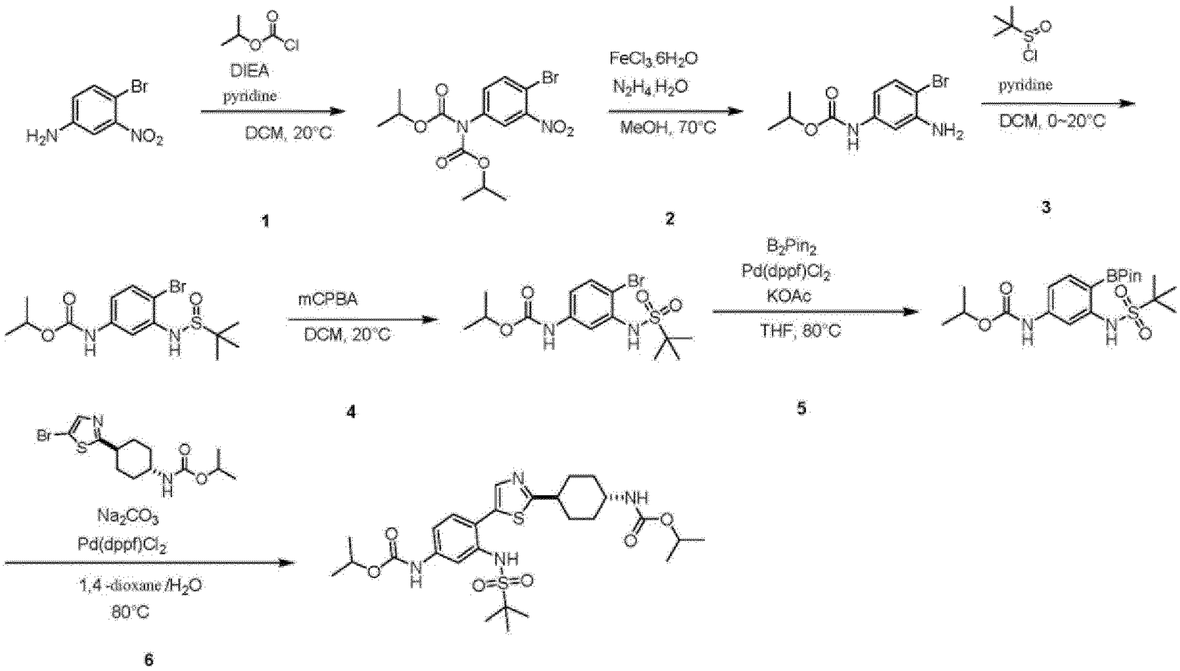


Step 1: Synthesis of isopropyl N-(4-bromo-3-nitro-phenyl)-N-isopropoxycarbonyl-carbamate

4-Bromo-3-nitroaniline (5 g, 23.04 mmol) and isopropyl chloroformate (5.65 g, 46.08 mmol, 6.40 mL) were dissolved in anhydrous dichloromethane (100 mL), and N,N-diisopropylethylamine (5.96 g, 46.08 mmol, 8.03 mL) and pyridine (9.11 g, 115.20 mmol, 9.30 mL) were added. The reaction solution appeared as a dark brown suspension, and was stirred and reacted at 20°C for 0.5 h. The completion of the reaction was detected by LCMS. The reaction solution was concentrated to dryness under reduced pressure and purified by column chromatography (silica, tetrahydrofuran:petroleum ether = 1:6) to obtain isopropyl N-(4-bromo-3-nitro-phenyl)-N-isopropoxycarbonyl-carbamate (5.6 g, 14.39 mmol).

MS m/z (ESI): 411/413 $[M+Na]^+$;

Step 2: Synthesis of isopropyl N-(3-amino-4-bromophenyl)carbamate

Isopropyl N-(4-bromo-3-nitro-phenyl)-N-isopropoxycarbonyl-carbamate (1 g, 2.57 mmol) was dissolved in anhydrous methanol (10 mL), and iron trichloride hexahydrate (891.75 mg, 3.30 mmol) was added. The reaction solution was stirred and reacted at 70°C for 12 min. Thereafter, hydrazine hydrate (1.98 g, 39.59 mmol) was added. The yellow reaction solution was stirred and reacted at 70°C for 3 h. The completion of the reaction was detected by LCMS. The reaction solution was filtered, evaporated to dryness via rotary evaporation, and extracted with ethyl acetate (30 mL), water (30 mL) and saturated saline (30 mL). The organic layer was dried over anhydrous magnesium sulfate and subjected to suction filtration. The filtrate was concentrated to dryness under reduced pressure and purified by column chromatography (silica, ethyl acetate/petroleum ether = 1:4) to obtain isopropyl N-(3-amino-4-bromophenyl)carbamate (300 mg, 1.10 mmol).

MS m/z (ESI): 273/275 $[M+H]^+$;

Step 3: Synthesis of isopropyl N-[4-bromo-3-(tert-butylsulfinylamino)phenyl]carbamate

Isopropyl N-(3-amino-4-bromophenyl)carbamate (300 mg, 1.10 mmol) was dissolved in anhydrous dichloromethane (3 mL), and pyridine (260.65 mg, 3.30 mmol, 265.97 μL) was added. Under the protection of nitrogen, tert-butylsulfinyl chloride (185.36 mg, 1.32 mmol, 162.60 μL) was added dropwise. The reaction solution was stirred and reacted at 20°C for 7 h. The completion of the reaction was detected by LCMS. The resultant was purified by column chromatography (silica, ethyl acetate/petroleum ether = 1:4) to obtain isopropyl N-[4-bromo-3-(tert-butylsulfinylamino)phenyl]carbamate (160 mg, 424.07 μmol).

MS m/z (ESI): 377/379 $[M+H]^+$;

Step 4: Synthesis of isopropyl N-[4-bromo-3-(tert-butylsulfonylamino)phenyl]carbamate

Isopropyl N-[4-bromo-3-(tert-butylsulfinylamino)phenyl]carbamate (150 mg, 397.57 μmol) was dissolved in anhydrous dichloromethane (1 mL). Under the protection of nitrogen, m-chloroperoxybenzoic acid (171.52 mg, 993.91 μmol) was added. The reaction solution was stirred and reacted at 20°C for 3 h. The completion of the reaction was detected by LCMS. The reaction solution was poured into ammonium chloride in ice-water bath, and extracted with dichloromethane (2 × 15 mL) and saturated saline (15 mL). The organic phase was dried over anhydrous magnesium sulfate, filtered, and evaporated to dryness via rotary evaporation. The resultant was purified by column chromatography (silica, ethyl acetate/petroleum ether = 1:4) to obtain isopropyl N-[4-bromo-3-(tert- butylsulfonylamino)phenyl]carbamate (100 mg, 254.26 μmol).

MS m/z (ESI): 410/412 $[M+H_2O]^+$;

Step 5: Synthesis of isopropyl N-[3-(tert-butylsulfonylamino)-4-(4,4,5,5-tetramethyl-1,3,2-dioxaborolan-2-yl)phenyl]carbamate

Isopropyl N-[4-bromo-3-(tert-butylsulfonylamino)phenyl]carbamate (50 mg, 127.13 μmol) and bis(pinacolato)diboron (129.13 mg, 508.52 μmol) were dissolved in anhydrous tetrahydrofuran (5 mL). Under the protection of nitrogen, potassium acetate (37.43 mg, 381.39 μmol) and 1,1-bis(diphenylphosphino)ferrocene dichloropalladium (II) - dichloromethane complex (10.38 mg, 12.71 μmol) were added. The red reaction solution was stirred and reacted at 80°C for 7 h. The completion of the reaction was detected by LCMS. The resultant was purified by thin-layer chromatography (silica, petroleum ether/tetrahydrofuran = 3/1) to obtain isopropyl N-[3-(tert-butylsulfonylamino)-4-(4,4,5,5-tetramethyl-1,3,2-dioxaborolan-2-yl) phenyl]carbamate (30 mg, 68.13 μmol).

MS m/z (ESI): 458 $[M+H_2Q]^+$;

Step 6: Synthesis of isopropyl trans-N-[3-(tert-butylsulfonylamino)-4-[2-[4-(isopropoxycarbonylamino)cyclohexyl]thiazol-5-yl]phenyl]carbamate

Isopropyl N-[3-(tert-butylsulfonylamino)-4-(4,4,5,5-tetramethyl-1,3,2-dioxaborolan-2-yl) phenyl]carbamate (30 mg, 68.13 μmol) and isopropyl trans-N-[4-(5-bromothiazol-2-yl)cyclohexyl]carbamate (23.66 mg, 68.13 μmol) were dissolved in 1,4-dioxane (4 mL) and water (0.4 mL). Under the protection of nitrogen, sodium carbonate (14.44 mg, 136.25 μmol) and 1,1-bis(diphenylphosphino)ferrocene dichloropalladium (II) - dichloromethane complex (5.56 mg, 6.81 μmol) were added. The reaction solution was stirred and reacted at 80°C for 7 h. The completion of the reaction was detected by LCMS. The reaction solution was concentrated to dryness under reduced pressure. The reaction solution was added with water (15 mL) and extracted with dichloromethane (15 mL) twice. The organic layer was dried over anhydrous magnesium sulfate and subjected to suction filtration. The filtrate was concentrated to dryness under reduced pressure. The resultant was purified by preparative liquid chromatography (Boston Prime C18 column; 5 μm silica, 30 mm in diameter, 150 mm in length; using decreasingly polar mixtures of water (containing 0.05% aqueous ammonia) and acetonitrile as eluents) to obtain isopropyl trans-N-[3-(tert-butylsulfonylamino)-4-[2-[4-(isopropoxycarbonylamino)cyclohexyl]thiazol-5-yl]phenyl]carbamate.

MS m/z (ESI): 581.1 $[M+H]^+$;

$^1H$ NMR (400 MHz, DMSO-$d_6$) δ ppm 9.81 (br s, 1 H) 8.80 - 8.91 (m, 1 H) 7.86 (s, 1 H) 7.73 (s, 1 H) 7.32 - 7.44 (m, 2 H) 7.04 (br d, J = 7.53 Hz, 1 H) 4.86 - 4.96 (m, 1 H) 4.69 - 4.81 (m, 1 H) 3.32 - 3.32 (m, 1 H) 2.90 (br t, J = 11.80 Hz, 1 H) 2.08 (br s, 2 H) 1.91 (br d, J = 9.54 Hz, 2 H) 1.48 - 1.61 (m, 2 H) 1.31 - 1.40 (m, 2 H) 1.26 (d, J = 6.27 Hz, 6 H) 1.13 - 1.20 (m, 15 H).

Example 20: Synthesis of isopropyl trans-N-[4-[2-[4-(isopropoxycarbonylamino) cyclohexyl]thiazol-5-yl]-3-(isopropyl-N-methyl-sulfonimidoyl)phenyl]carbamate

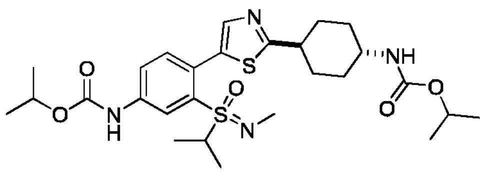

Synthetic Method

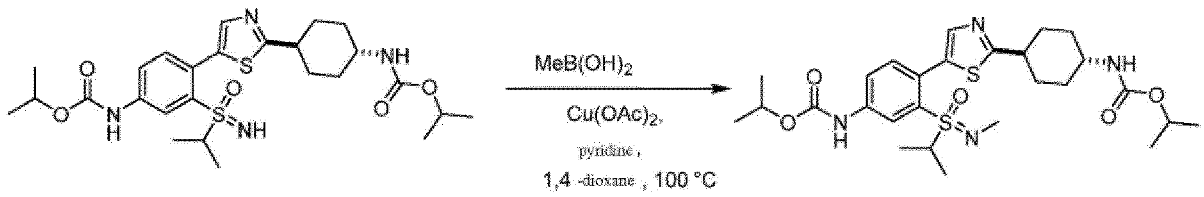


Isopropyl trans-N-[4-[2-[4-(isopropoxycarbonylamino)cyclohexyl]thiazol-5-yl]-3-(isopropylsulfonimidoyl)phenyl]carbamate (20 mg, 36.32 μmol) as a reactant was dissolved in 1,4-dioxane (2 mL). Anhydrous copper acetate (9.89 mg, 54.47 μmol) and pyridine (6.89 mg, 87.16 μmol, 7.03 μL) were added at 20°C. The reaction solution was stirred at 20°C for 5 minutes, and methylboronic acid (4.35 mg, 72.63 μmol) was added. The reaction solution was stirred at 100°C for 12 hours. The completion of the reaction was detected by LC-MS. The reaction solution was filtered under reduced pressure. The filtrate was concentrated to dryness under reduced pressure and purified by preparative high-performance liquid chromatography (Phenomenex Gemini-NX C18 column, 5 μm silica, 30 mm in diameter, 100 mm in length) (using decreasingly polar mixtures of water (containing 0.05% aqueous ammonia) and acetonitrile (50% to 70%) as eluents) to obtain isopropyl trans-N-[4-[2-[4-(isopropoxycarbonylamino)cyclohexyl]thiazol-5-yl]-3-(S-isopropyl-N-methyl -sulfonimidoyl)phenyl]carbamate (1.5 mg).

MS m/z (ESI): 565.3 $[M+H]^+$

$^1$H NMR: (400 MHz, METHANOL-$d_4$) δ ppm 8.23 (d, J = 2.26 Hz, 1H), 7.83-7.77 (m, 1H), 7.63 (s, 1H), 7.44 (d, J = 8.53 Hz, 1H), 5.05-5.00 (m, 1H), 4.92-4.80 (m, 1H), 3.55-3.45 (m, 1H), 3.17-3.09 (m, 1H), 3.06-2.96 (m, 1H), 2.26-2.21 (m, 2H), 2.15-2.02 (m, 2H), 1.74-1.63 (m, 2H), 1.49-1.40 (m, 2H), 1.35-1.32 (m, 12H), 1.24 (br d, J = 6.27 Hz, 6H), 1.11 (d, J = 6.78 Hz, 3H).

Example 21: Synthesis of isopropyl N-[3-(tert-butylsulfamoyl)-4-[2-[8-(isopropoxycarbonylamino)-3-bicyclo[3.2.1]octyl]thiazol-5-yl]phenyl]carbamate

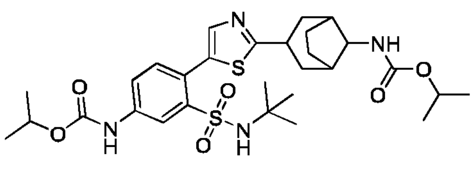

Synthetic Method

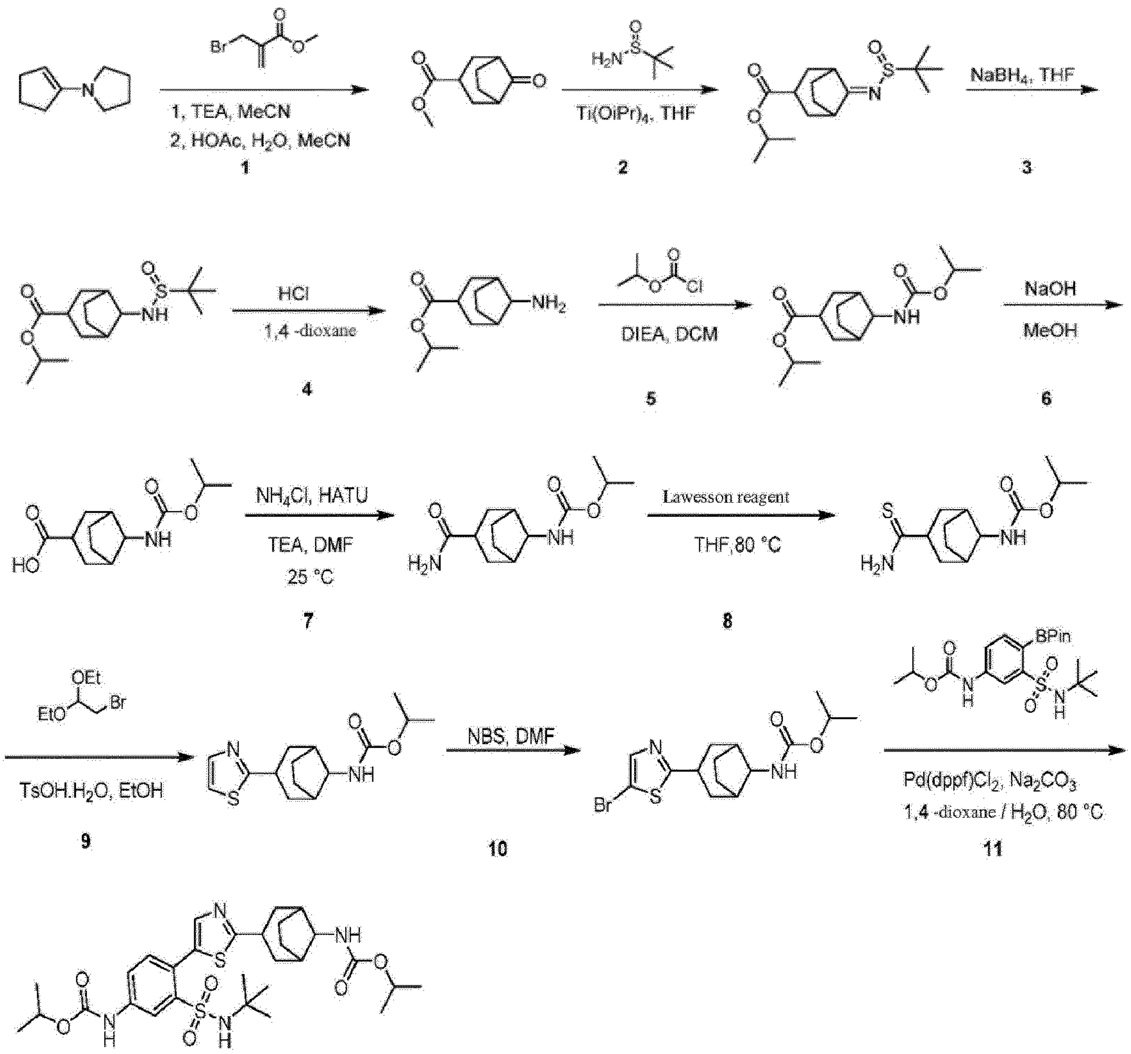


Step 1: Synthesis of methyl 8-oxobicyclo[3.2.1]octane-3-carboxylate

Methyl 2-(bromomethyl)acrylate (19.57 g, 109.31 mmol) was dissolved in acetonitrile (135 mL). At room temperature, a solution of 1-pyrrolidinyl-1-cyclopentene (15 g, 109.31 mmol) in acetonitrile (135 mL) was slowly added dropwise, and then triethylamine (17.81 g, 175.99 mmol) was added. The reaction solution was stirred and reacted at 90°C for 5 hours. Afterwards, acetic acid (77.46 g, 64.49 mmol) was added. The reaction solution was continued to be stirred and reacted at 90°C for 1 hour. The completion of the reaction was detected by LCMS. The reaction solution was concentrated to dryness under reduced pressure. The crude product was purified by column chromatography (silica, dichloromethane:methanol = 15:1) to obtain methyl 8-oxobicyclo[3.2.1]octane-3- carboxylate (12 g).

$^1$H NMR (400 MHz, DMSO-$d_6$): δ ppm 3.70 (s, 3H) 2.76 (t, J = 7.82Hz, 1H) 2.58-2.69 (m, 2H) 2.03-2.22 (m, 4H) 1.75-1.85 (m, 2H) 1.59-1.70 (m, 2H).

Step 2: Synthesis of isopropyl 8-((tert-butylsulfinyl)imino)bicyclo[3.2.1]octane-3-carboxylate

Methyl 8-oxobicyclo[3.2.1]octane-3-carboxylate (8 g, 43.90 mmol) and 2-methyl-2-propanesulfinamide (15.96 g, 131.71 mmol) were dissolved in tetrahydrofuran (160 mL), and then titanium tetraisopropoxide (37.43 g, 131.71 mmol) was added. The reaction solution was stirred and reacted at 60°C for 2 hours. The completion of the reaction was detected by LCMS. The reaction product undergone transesterification and became an isopropyl ester product. The reaction solution was used directly in the next reaction step without treatment.

MS m/z (ESI):314.3 [M+28]$^+$;

Step 3: Synthesis of isopropyl 8-(tert-butylsulfinylamino)bicyclo[3.2.1]octane-3-carboxylate

Sodium borohydride (4.98 g, 131.71 mmol) was slowly added into the reaction solution obtained in Step 2. The reaction solution was stirred and reacted at 30°C for 4 hours. The completion of the reaction was detected by LCMS. The reaction solution was added with a saturated sodium carbonate solution (100 mL), stirred at room temperature for 30 minutes, and then extracted with dichloromethane (100 mL) twice. The organic phases were combined, dried over anhydrous sodium sulfate, filtered, and concentrated to dryness under reduced pressure. The resultant was purified by column chromatography (silica, dichloromethane:methanol = 15:1) to obtain isopropyl 8-(tert-butylsulfinylamino)bicyclo[3.2.1] octane-3-carboxylate (7.7 g).

$^1$H NMR (400 MHz, DMSO-$d_6$): δ ppm 4.95-4.88 (m, 1H) 3.20-3.13 (m, 1H) 2.50-2.45 (m, 1H) 2.40-1.93 (m, 6H) 1.63-1.60 (m, 2H) 1.49-1.47 (m, 2H) 1.20-1.08 (m, 15H).

Step 4: Synthesis of isopropyl 8-aminobicyclo[3.2.1]octane-3-carboxylate

Isopropyl 8-(tert-butylsulfinylamino)bicyclo[3.2.1]octane-3-carboxylate (2.31 g, 8.05 mmol) was dissolved in methanol (18 mL), and hydrochloric acid/dioxane (22 mL) was added. The reaction solution was stirred and reacted at 30°C for 2 hours. The completion of the reaction was detected by LCMS. The reaction solution was concentrated to dryness under reduced pressure to obtain isopropyl 8-aminobicyclo[3.2.1]octane-3-carboxylate (1.2 g, crude product).

MS m/z (ESI): 212.2 $[M+H]^+$

Step 5: Synthesis of isopropyl 8-((isopropoxycarbonyl)amino)bicyclo[3.2.1]octane-3-carboxylate

Methyl 8-aminobicyclo[3.2.1]octane-3-carboxylate (547 mg, 2.99 mmol) and isopropyl chloroformate (438.98 mg, 3.58 mmol) were dissolved in dichloromethane (26 mL), and N,N-diisopropylethylamine (1.93 g, 14.93 mmol) was added. The reaction solution was stirred and reacted at 30°C for 1 hour. The reaction solution was added with water (10 mL), and then extracted with ethyl acetate (30 mL) twice. The organic phases were combined, dried over anhydrous sodium sulfate, filtered, and concentrated to dryness under reduced pressure. The resultant was purified by column chromatography (silica, petroleum ether:tetrahydrofuran = 3:1) to obtain isopropyl 8-((isopropoxycarbonyl)amino)bicyclo[3.2.1] octane-3-carboxylate (416 mg, 1.54 mmol).

MS m/z (ESI): 298.3 $[M+H]^+$

Step 6: Synthesis of 8-(isopropoxycarbonylamino)bicyclo[3.2.1]octane-3-carboxylic acid

Isopropyl 8-((isopropoxycarbonyl)amino)bicyclo[3.2.1]octane-3-carboxylate (416 mg, 1.54 mmol) was dissolved in methanol (4 mL), and sodium hydroxide (2 M, 2.32 mL) was added. The reaction solution was stirred and reacted at 30°C for 1 hour. The completion of the reaction was detected by LCMS. The reaction solution was concentrated to dryness under reduced pressure. Hydrochloric acid (2 M, 4 mL) was added and the pH of the mixture was adjusted to 3. The reaction solution was added with 3 mL of dichloromethane (1 mL × 3). The organic phase was dried over anhydrous sodium sulfate and filtered. The filtrate was concentrated to dryness under reduced pressure to obtain 8-(isopropoxycarbonylamino)bicyclo[3.2.1]octane-3-carboxylic acid (360 mg, crude product).

MS m/z (ESI): 256.2 $[M+H]^+$

$^1$H NMR (400 MHz, DMSO-$d_6$) δ ppm 11.95 (s, 1H) 7.12 (br d, J = 3.51Hz, 1H) 4.70-4.80 (m, 1H) 3.33-3.34 (m, 1H) 2.36-2.48 (m, 1H) 2.10-2.04 (m, 2H) 1.79 (br t, J = 12.80Hz, 2H) 1.63-1.72 (m, 2H) 1.42-1.52 (m, 2H) 1.33-1.41 (m, 2H) 1.13-1.22 (m, 6H).

Step 7: Synthesis of isopropyl (3-carbamoylbicyclo[3.2.1]oct-8-yl)carbamate

8-(Isopropoxycarbonylamino)bicyclo[3.2.1]octane-3-carboxylic acid (1.6 g, 6.27 mmol) and O-(7-azabenzotriazol-1-yl)-N,N,N,N-tetramethyluronium hexafluorophosphate (2.62 g, 6.89 mmol) were dissolved in N,N-dimethylformamide (100 mL), and ammonium chloride (1.68 g, 31.33 mmol) and N,N-diisopropylethylamine (4.05 g, 31.33 mmol) were added. The reaction solution was stirred and reacted at 30°C for 1 hour. The completion of the reaction was detected by LCMS. The reaction solution was concentrated to dryness under reduced pressure and purified by column chromatography (silica, petroleum ether:tetrahydrofuran = 3:1) to obtain isopropyl (3-carbamoylbicyclo[3.2.1]oct-8-yl)carbamate (500 mg, crude product).

MS m/z (ESI): 255.2 $[M+H]^+$

$^1$H NMR (400 MHz, DMSO-$d_6$) δ ppm 7.17 (br s, 1H) 7.02 (br d, J = 5.13Hz, 1H) 6.65 (br s, 1H) 4.81-4.72 (m, 1H) 3.40-3.30 (m, 1H) 2.30-2.45 (m, 1H) 2.15-2.06 (m, 2H) 1.63-1.81 (m, 4H) 1.41-1.50 (m, 2H) 1.23-1.32 (m, 2H) 1.18 (d, J = 6.25Hz, 6H).

Step 8: Synthesis of isopropyl (3-aminomethylthiobicyclo[3.2.1]oct-8-yl)carbamate

Isopropyl (3-carbamoylbicyclo[3.2.1]oct-8-yl)carbamate (500 mg, 1.97 mmol) was dissolved in tetrahydrofuran (25 mL), and Lawesson reagent (397.59 mg, 983.00 μmol) was added. The reaction solution was stirred and reacted at 80°C for 0.5 hours. The completion of the reaction was detected by LCMS. The reaction solution was added with a saturated aqueous sodium carbonate solution (50 mL) so that the pH of the mixture was adjusted to 8~9, and the resulting mixture was extracted with ethyl acetate (50 mL) three times. The organic layer was dried over anhydrous magnesium sulfate and subjected to suction filtration. The filtrate was concentrated to dryness under reduced pressure. The reaction solution was concentrated to dryness under reduced pressure and purified by column chromatography (silica, petroleum ether:tetrahydrofuran = 3:1) to obtain isopropyl (3-aminomethylthiobicyclo[3.2.1]oct-8-yl)carbamate (270 mg, 998.56 μmol).

MS m/z (ESI): 271.1 $[M+H]^+$

Step 9: Synthesis of isopropyl (3-(thiazol-2-yl)bicyclo[3.2.1]oct-8-yl)carbamate

Isopropyl (3-aminomethylthiobicyclo[3.2.1]oct-8-yl)carbamate (278 mg, 1.03 mmol) and bromoacetaldehyde diethyl acetal (607.85 mg, 3.08 mmol) were dissolved in ethanol (4 mL), and p-toluenesulfonic acid monohydrate (391.14 mg, 2.06 mmol) was added. The reaction solution was stirred and reacted at 80°C for 16 hours. The completion of the reaction was detected by LCMS. The reaction solution was concentrated to dryness under reduced pressure. The reaction solution was added with a saturated aqueous sodium carbonate solution (20 mL) so that the pH of the mixture was adjusted to 8~9, and the resulting mixture was extracted with dichloromethane (50 mL) three times. The organic layer was dried over anhydrous magnesium sulfate, subjected to suction filtration, and concentrated to dryness under reduced pressure. The resultant was purified by thin-layer chromatography (silica, petroleum ether:tetrahydrofuran = 2:1) to obtain isopropyl (3-(thiazol-2-yl)bicyclo[3.2.1]oct-8-yl)carbamate (100 mg).

MS m/z (ESI): 295.2 $[M+H]^+$

$^1H$ NMR (400 MHz, CHLOROFORM-d) δ ppm 7.69 (d, J = 3.26Hz, 1H) 7.20 (d, J = 3.51Hz, 1H) 5.05 (br s, 1H) 4.84-4.99 (m, 1H) 3.78 (br s, 1H) 3.30-3.46 (m, 1H) 2.30 (br s, 2H) 1.83-1.90 (m, 4H) 1.64-1.69 (m, 2H) 1.24 (d, J = 6.27Hz, 6H).

Step 10: Synthesis of isopropyl (3-(5-bromothiazol-2-yl)bicyclo[3.2.1]oct-8-yl)carbamate

Isopropyl (3-(thiazol-2-yl)bicyclo[3.2.1]oct-8-yl)carbamate (52 mg, 176.62 μmol) was dissolved in N,N-dimethylformamide (3 mL), and then the mixture was slowly added dropwise into a solution of N-bromosuccinimide (62.87 mg, 353.25 μmol) in N,N-dimethylformamide (3 mL). The reaction solution was stirred and reacted at 50°C for 1 hour. The completion of the reaction was detected by LCMS. The reaction solution was concentrated to dryness under reduced pressure and purified by column chromatography (silica, petroleum ether:tetrahydrofuran = 2:1) to obtain isopropyl (3-(5-bromothiazol-2-yl)bicyclo[3.2.1]oct-8-yl)carbamate (50 mg).

MS m/z (ESI): 373.1 & 375.1 $[M+H]^+$

$^1H$ NMR (400 MHz, CHLOROFORM-d) δ ppm 7.55 (s, 1H) 4.95-4.90 (m, 2H) 3.76 (br s, 1H) 3.23-3.37 (m, 1H) 2.30 (br s, 2H) 1.95-1.76 (m, 6H) 1.70-1.63 (m 2H) 1.24 (d, J = 6.27Hz, 6H).

Step 11: Synthesis of isopropyl N-[3-(tert-butylsulfamoyl)-4-[2-[8-(isopropoxycarbonylamino)-3-bicyclo[3.2.1]octyl]thiazol-5-yl]phenyl]carbamate

Isopropyl (3-(5-bromothiazol-2-yl)bicyclo[3.2.1]oct-8-yl)carbamate (30 mg, 80.36 μmol) and isopropyl (3-(N-(tert-butyl)sulfamoyl)-4-(4,4,5,5-tetramethyl-1,3,2-dioxaborolan-2-yl)phenyl)carbamate (42.47 mg, 96.44 μmol) were dissolved in water (240 μl) and dioxane (3 mL). Afterwards, [1,1-bis(diphenylphosphino)ferrocene]dichloropalladium - dichloromethane (6.56 mg, 8.04 μmol) and sodium carbonate (25.55 mg, 241.09 μmol) were added under the condition where nitrogen gas was introduced. The reaction solution was stirred and reacted at 80°C for 2 hours. The completion of the reaction was detected by LCMS. The reaction solution was added with water (2 mL) and extracted with ethyl acetate (6 mL) three times. The organic layer was dried over anhydrous magnesium sulfate and subjected to suction filtration. The filtrate was concentrated to dryness under reduced pressure and purified by preparative liquid chromatography (YMC Triart C18 column, 7 μm silica, 50 mm in diameter, 250 mm in length; using decreasingly polar mixtures of water (containing 0.05% aqueous ammonia) and acetonitrile as eluents) to obtain the compound isopropyl N-[3-(tert-butylsulfamoyl)-4-[2-[8-(isopropoxycarbonylamino)-3-bicyclo[3.2.1]octyl] thiazol-5-yl]phenyl]carbamate (30 mg).

MS m/z (ESI): 607.4 $[M+H]^+$

$^1H$ NMR (400 MHz, DMSO-d6) δ ppm 10.07 (s, 1H) 8.31 (d, J = 2.25Hz, 1H) 7.55-7.72 (m, 2H) 7.37 (d, J = 8.38Hz, 1H) 7.23 (br s, 1H) 6.98 (s, 1H) 4.96-4.89 (m, 1H) 4.80-4.75 (m, 1H) 3.40 (br s, 1H) 3.37-3.46 (m, 1H) 2.26 (br

s, 2H) 1.99 (br t, J = 12.07Hz, 2H) 1.82-1.55 (m, 6H) 1.27 (d, J = 6.25Hz, 6H) 1.17 (d, J = 6.25Hz, 6H) 1.09 (s, 9H).

Example 22: Synthesis of isopropyl trans-(4-(5-(4-((1H-pyrazol-3-yl)amino)-2-(N-(tert-butyl)sulfamoyl)phenyl)thiazol-2-yl)cyclohexyl)carbamate

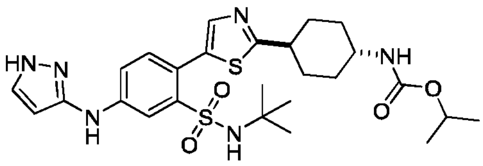

Synthetic Method

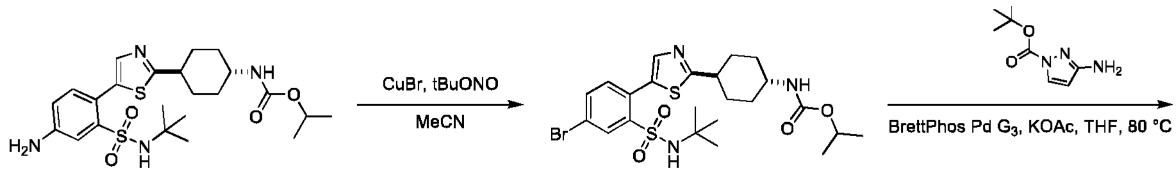


1 2

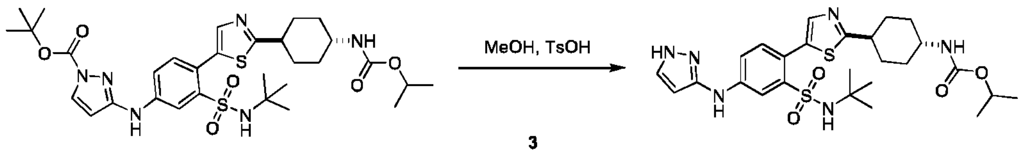


3

Step 1: Synthesis of isopropyl trans-(4-(5-(4-bromo-2-(N-(tert-butyl)sulfamoyl) phenyl)thiazol-2-yl)cyclohexyl)carbamate

Isopropyl trans-(4-(5-(4-amino-2-(N-(tert-butyl)sulfamoyl)phenyl)thiazol-2-yl)cyclohexyl) carbamate (1.4 g, 2.83 mmol) was dissolved in acetonitrile (30 mL). Under the protection of nitrogen, tert-butyl nitrite (437.77 mg, 4.25 mmol, 504.93 μL) was added. The reaction solution was stirred at 0°C for 1 hour. Cuprous bromide (487.19 mg, 3.40 mmol) was added into the reaction solution under the protection of nitrogen. The reaction solution was stirred at 25°C for 15 hours. LCMS showed that the reaction was complete. The reaction system was evaporated to dryness via rotary evaporation, and then the resultant was purified by column chromatography (silica, tetrahydrofuran/petroleum ether = 0% to 20%) to obtain isopropyl trans-(4-(5-(4-bromo-2-(N-(tert-butyl)sulfamoyl)phenyl)thiazol-2-yl)cyclohexyl)carbamate (0.9 g, 1.46 mmol).

MS m/z (ESI): 558.2, 560.2 $[M+H]^+$;

Step 2: Synthesis of tert-butyl trans-3-((3-(N-(tert-butyl)sulfamoyl) -4-(2-(4-((isopropoxycarbonyl)amino)cyclohexyl)thiazol-5-yl)phenyl)amino)-1H-pyrazole-1-carboxylate

Isopropyl trans-(4-(5-(4-bromo-2-(N-(tert-butyl)sulfamoyl)phenyl)thiazol-2-yl)cyclohexyl) carbamate (250 mg, 447.59 μmol) and tert-butyl 3-amino-1H-pyrazole-1-carboxylate (123.00 mg, 671.38 μmol) were dissolved in anhydrous tetrahydrofuran (6 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (40.57 mg, 44.76 μmol) and potassium acetate (131.78 mg, 1.34 mmol) were added. The reaction solution was stirred at 80°C for 1 hour. LCMS showed that the reaction was complete. The reaction system was evaporated to dryness via rotary evaporation, and then the resultant was extracted with ethyl acetate (40 mL × 3) to obtain tert-butyl trans-3-((3-(N-(tert-butyl)sulfamoyl)-4-(2-(4-((isopropoxycarbonyl) amino)cyclohexyl)thiazol-5-yl)phenyl)amino)-1H-pyrazole-1-carboxylate (235 mg, crude product).

MS m/z (ESI): 661.4 $[M+H]^+$;

Step 3: Synthesis of isopropyl trans-(4-(5-(4-((1H-pyrazol-3-yl)amino)-2-(N-(tert-butyl)sulfamoyl)phenyl)thiazol-2-yl)cyclohexyl)carbamate

Tert-butyl trans-3-((3-(N-(tert-butyl)sulfamoyl)-4-(2-(4-((isopropoxycarbonyl)amino) cyclohexyl)thiazol-5-yl)phenyl)amino)-1H-pyrazole-1-carboxylate (200 mg, 302.64 μmol) was dissolved in methanol (5 mL), and p-toluenesulfonic acid monohydrate (156.35 mg, 907.93 μmol) was added. The reaction solution was stirred at 30°C for 2 hours. LCMS showed that the reaction was complete. The reaction system was evaporated to dryness via rotary evaporation. The resultant was purified by preparative high-performance liquid chromatography (Phenomenex Gemini-NX C18 column, 5 μm silica, 30 mm in diameter, 150 mm in length) (using decreasingly polar mixtures of water (containing 0.05% aqueous ammonia and 10 mmol ammonium bicarbonate) and acetonitrile (45% to 65%) as eluents) and lyophilized to obtain isopropyl trans-(4-(5-(4-((1H-pyrazol-3-yl)amino)-2-(N-(tert-butyl)sulfamoyl)phenyl)thiazol-2-yl)cyclohexyl)carbamate (36.79 mg).

MS m/z (ESI): 561.3 $[M+H]^+$;

$^1$H NMR (400 MHz, DMSO-$d_6$) δ ppm 12.17 (s, 1H) 9.02 (s, 1H) 8.16 (d, J = 2.32 Hz, 1H) 7.69-7.57 (m, 2H) 7.53 (dd, J = 8.44, 2.32 Hz, 1H) 7.25 (d, J = 8.31 Hz, 1H) 7.01 (br d, J = 7.70 Hz, 1H) 6.71 (s, 1H) 5.86 (t, J = 2.08 Hz, 1H) 4.83-4.70 (m, 1H) 3.31-3.27 (m, 1H) 2.95-2.83 (m, 1H) 2.17-2.05 (m, 2H) 1.95-1.83 (m, 2H) 1.65-1.47 (m, 2H) 1.42-1.26 (m, 2H) 1.16 (d, J = 6.24 Hz, 6H) 1.07 (s, 9H).

Example 23: Synthesis of isopropyl trans-N-[3-diethylphosphoryl-4-[2-[4-(isopropoxycarbonylamino)cyclohexyl]thiazol-5-yl]phenyl]carbamate

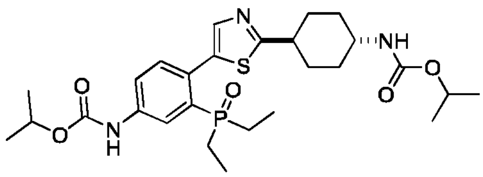

Synthetic Method

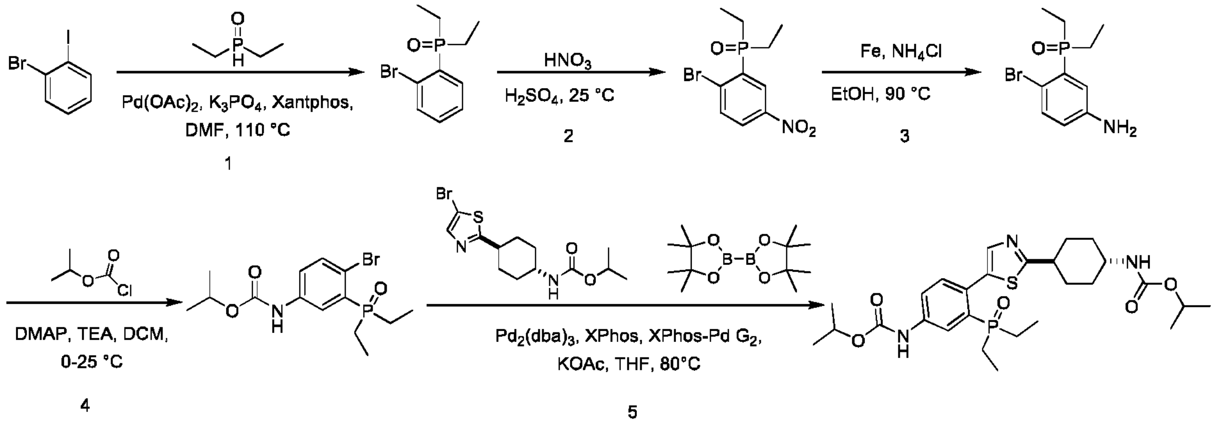


Step 1: Synthesis of (2-bromophenyl)diethylphosphine oxide

O-bromoiodobenzene (1 g, 3.53 mmol, 454.55 μL), potassium phosphate (1.50 g, 7.07 mmol) and 4,5-bis(diphenylphosphino)-9,9-dimethylxanthene (409.06 mg, 706.95 μmol) were dissolved in N,N-dimethylformamide (15 mL). Under the protection of nitrogen, diethylphosphine oxide (750.10 mg, 7.07 mmol) and palladium acetate (158.72 mg, 706.95 μmol) were added. The reaction solution appeared as a light yellow suspension, and was stirred and reacted at 100°C for 1 h. The completion of the reaction was detected by LCMS. The reaction solution was added with water (50 mL) and extracted with dichloromethane (100 mL). The organic layer was dried over anhydrous magnesium sulfate and subjected to suction filtration. The filtrate was concentrated to dryness under reduced pressure. The resultant was purified

by column chromatography (dichloromethane:methanol = 20:1) to obtain the compound (2-bromophenyl)diethylphosphine oxide (900 mg).

$^1$H NMR (400 MHz, DMSO-$d_6$) δ ppm 8.05-7.92 (m, 1H) 7.81-7.70 (m, 1H) 7.64-7.45 (m, 2H) 2.27-2.01 (m, 4H) 1.01-0.88 (m, 6H).

Step 2: Synthesis of (2-bromo-5-nitrophenyl)diethylphosphine oxide

(2-Bromophenyl)diethylphosphine oxide (900 mg, 3.45 mmol) was dissolved in concentrated sulfuric acid (10 mL), and fuming nitric acid (651.62 mg, 10.34 mmol, 465.44 μL) was added. The reaction solution appeared as a yellow suspension, and was stirred and reacted at 30°C for 1 h. The completion of the reaction was detected by LCMS. The reaction solution was added with water (20 mL), and then extracted with ethyl acetate (20 mL). The organic layer was dried over anhydrous magnesium sulfate and subjected to suction filtration. The filtrate was concentrated to dryness under reduced pressure to obtain crude (2-bromo-5-nitrophenyl)diethylphosphine oxide (840 mg).

MS m/z (ESI): 306.0, 308.0 $[M+H]^+$;

Step 3: Synthesis of (5-amino-2-bromophenyl)diethylphosphine oxide

(2-Bromo-5-nitrophenyl)diethylphosphine oxide (840 mg, 2.74 mmol) was dissolved in anhydrous ethanol (3 mL). Under the protection of nitrogen, reduced iron powder (919.52 mg, 16.47 mmol) and ammonium chloride (1.47 g, 27.44 mmol) were added. The reaction solution appeared as a yellow suspension, and was stirred and reacted at 80°C for 12 h. The completion of the reaction was detected by LCMS. The reaction solution was filtered while it was still hot. The filtrate was concentrated to dryness under reduced pressure. The resultant was purified by column chromatography (dichloromethane:methanol = 20:1) to obtain the compound (5-amino-2-bromophenyl)diethylphosphine oxide (360 mg).

MS m/z (ESI): 276.0, 278.0 $[M+H]^+$;

Step 4: Synthesis of isopropyl (4-bromo-3-(diethylphosphoryl)phenyl)carbamate

(5-Amino-2-bromophenyl)diethylphosphine oxide (180 mg, 651.92 μmol) was dissolved in anhydrous dichloromethane (3 mL). Under the protection of nitrogen, pyridine (309.40 mg, 3.91 mmol, 315.71 μL) and isopropyl chloroformate (239.68 mg, 1.96 mmol, 271.43 μL) were added. The reaction solution appeared as a yellow suspension, and was stirred and reacted at 25°C for 1 h. The completion of the reaction was detected by LCMS. The reaction solution was concentrated to dryness under reduced pressure. The resultant was purified by column chromatography (petroleum ether:ethyl acetate = 1:1) to obtain the compound isopropyl (4-bromo-3-(diethylphosphoryl)phenyl)carbamate (180 mg, 496.97 μmol).

MS m/z (ESI): 362.0, 364.0 $[M+H]^+$;

Step 5: Synthesis of isopropyl trans-N-[3-diethylphosphoryl-4-[2-[4-(isopropoxycarbonylamino)cyclohexyl]thiazol-5-yl]phenyl]carbamate

Isopropyl (4-bromo-3-(diethylphosphoryl)phenyl)carbamate (80 mg, 220.87 μmol), isopropyl trans-N-[4-(5-bromothiazol-2-yl)cyclohexyl]carbamate (76.70 mg, 220.87 μmol) and bis(pinacolato)diboron (224.35 mg, 883.49 μmol) were dissolved in anhydrous tetrahydrofuran (6 mL). Under the protection of nitrogen, tris(dibenzylideneacetone)dipalladium(0)-chloroform adduct (10.11 mg, 11.04 μmol), 2-dicyclohexylphosphino-2,4,6-triisopropylbiphenyl (5.26 mg, 11.04 μmol), chloro(2-dicyclohexylphosphino-2,4,6-triisopropyl-1,1-biphenyl)[2-(2-amino-1,1-biphenyl)]palladium (II) (8.69 mg, 11.04 μmol) and potassium acetate (43.35 mg, 441.75 μmol) were added. The reaction solution appeared as a yellow suspension, and was stirred and reacted at 80°C for 16 h. The completion of the reaction was detected by LCMS. The reaction solution was concentrated to dryness under reduced pressure. The resultant was added with water (5 mL) and the mixture was extracted with ethyl acetate (10 mL). The organic layer was dried over anhydrous magnesium sulfate and subjected to suction filtration. The filtrate was concentrated to dryness under reduced pressure. The resultant was purified by preparative liquid chromatography (PhenomenexGeminiC18 column, 10 μm silica, 50 mm in diameter, 250 mm in length; using decreasingly polar mixtures of water (containing 0.225% formic acid) and acetonitrile as eluents) to obtain the compound isopropyl trans-N-[3-diethylphosphoryl-4-[2-[4-(isopropoxycarbonylamino)cyclohexyl]thiazol-5-yl]phenyl]carbamate (10.35 mg).

MS m/z (ESI): 550.0 $[M+H]^+$;

$^1$H NMR (400 MHz, DMSO-$d_6$) δ ppm 9.92 (s, 1H) 8.14-8.00 (m, 1H) 7.75-7.68 (m, 2H) 7.40-7.30 (m, 1H) 7.08-6.97 (m, 1H) 4.98-4.86 (m, 1H) 4.80-4.67 (m, 1H) 3.32-3.27 (m, 1H) 3.00-2.86 (m, 1H) 2.19-2.06 (m, 2H) 1.97-1.85 (m, 2H) 1.76-1.63 (m, 2H) 1.63-1.50 (m, 4H) 1.40-1.30 (m, 2H) 1.27 (d, J = 6.27 Hz, 6H) 1.17 (d, J = 6.27 Hz, 6H) 0.95-0.83 (m, 6H).

Example 24: Synthesis of isopropyl trans-N-[3-(2,2-dimethylpropylsulfonyl)-4-[2-[4-(isopropoxycarbonylamino)cyclohexyl]thiazol-5-yl]phenyl]carbamate

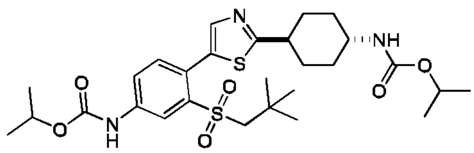

Synthetic Method

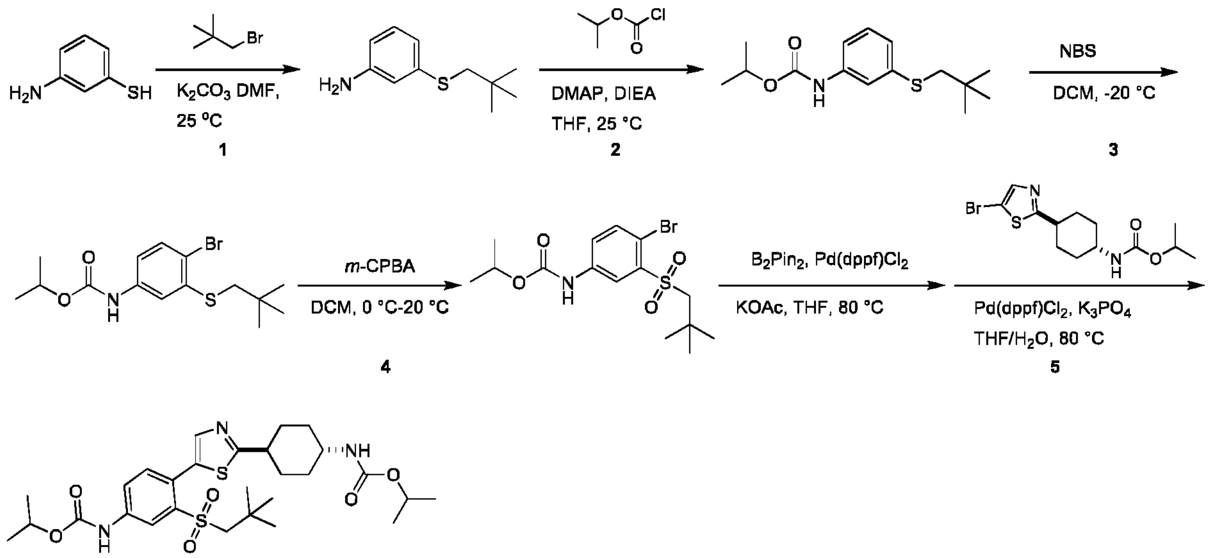


Step 1: Synthesis of 3-(neopentylthio)aniline

3-Aminobenzenethiol (400 mg, 3.20 mmol) was dissolved in N,N-dimethylformamide (10 mL), and 1-bromo-2,2-dimethylpropane (579.12 mg, 3.83 mmol) and potassium carbonate (662.38 mg, 4.79 mmol) were added. The reaction solution was stirred and reacted at 25°C for 3 hours. The reaction solution was diluted with water (50 mL) and extracted with ethyl acetate (15 mL) four times. The organic phase was dried, filtered, concentrated to dryness, and then purified by column chromatography (silica, petroleum ether/ethyl acetate = 5/1) to obtain 3-(neopentylthio)aniline as the product (230 mg).

MS m/z (ESI): 196.2 $[M+H]^+$;

$^1H$ NMR (400MHz, METHANOL-$d_4$) $\delta$ ppm 7.01 (t, J = 8.0 Hz, 1H), 6.75 (s, 1H), 6.78 (d, J = 6.8 Hz, 1H), 6.53 (d, J = 1.6 Hz, 1H), 2.88 (s, 2H), 1.03 (s, 9H).

Step 2: Synthesis of isopropyl (3-(neopentylthio)phenyl)carbamate

Diisopropylethylamine (880.04 mg, 6.81 mmol), 4-dimethylaminopyridine (207.97 mg, 1.70 mmol) and isopropyl chloroformate (677.28 mg, 5.53 mmol) were added into a solution of 3-(neopentylthio)aniline (665 mg, 3.40 mmol) in tetrahydrofuran (10 mL) at room temperature. The reaction solution was stirred overnight at room temperature. The reaction solution was poured into ice water (20 mL), and the resulting mixture was extracted with ethyl acetate (20 mL) three times. The organic phase was dried over anhydrous sodium sulfate, filtered, concentrated to dryness under reduced pressure, and then purified by column chromatography (silica, petroleum ether/ethyl acetate = 10/1) to obtain isopropyl (3-(neopentylthio)phenyl)carbamate as the product (794 mg).

MS m/z (ESI): 282.2 $[M+H]^+$;

Step 3: Synthesis of isopropyl (4-bromo-3-(neopentylthio)phenyl)carbamate

At -20°C, N-bromosuccinimide (126.49 mg, 710.7 μmol) was added into a solution of isopropyl (3-(neopentylthio)phenyl)carbamate (200 mg, 710.7 μmol) in dichloromethane (5 mL). The reaction solution was stirred at -20°C for 3 hours. The resulting mixture was diluted with dichloromethane (15 mL). The organic phase was washed with saturated saline (5 mL), dried, filtered, and concentrated to dryness. Thereafter, the resultant was purified by column chromatography (silica, petroleum ether/ethyl acetate = 20/1) to obtain isopropyl (4-bromo-3-(neopentylsulfonyl)phenyl)carbamate as the product (85 mg).

MS m/z (ESI): 361.1 $[M+H]^+$.

Step 4: Synthesis of isopropyl (4-bromo-3-(neopentylsulfonyl)phenyl)carbamate

At 0°C, m-chloroperoxybenzoic acid (95.79 mg, 471.82 μmol) was added into a solution of isopropyl (4-bromo-3-(neopentylthio)phenyl)carbamate (85 mg, 235.91 μmol) in dichloromethane (5 mL). The reaction solution was stirred overnight at room temperature. The resulting mixture was diluted with dichloromethane (20 mL), washed with a saturated sodium sulfite solution (5 mL), dried, filtered, and concentrated to obtain isopropyl (4-bromo-3-(neopentylsulfonyl)phenyl)carbamate as the product (85 mg).

MS m/z (ESI): 392.0, 394.0 $[M+H]^+$;

Step 5: Synthesis of isopropyl trans-N-[3-(2,2-dimethylpropylsulfonyl)-4-[2-[4-(isopropoxycarbonylamino)cyclohexyl]thiazol-5-yl]phenyl]carbamate

Under the nitrogen atmosphere, a solution of isopropyl (4-bromo-3-(neopentylsulfonyl)phenyl)carbamate (40 mg, 101.96 μmol), 4,4,5,5-tetramethyl-2-(4,4,5,5-tetramethyl-1,3,2-dioxaborolan-2-yl)-1,3,2-dioxaborolane (38.84 mg, 152.94 μmol), [1,1'-bis(diphenylphosphino)ferrocene]dichloropalladium (3.73 mg, 5.10 μmol) and potassium acetate (15.01 mg, 152.94 μmol) in tetrahydrofuran (2 mL) was heated to 80°C, and was stirred and reacted for 2 hours. Afterwards, isopropyl trans-N-[4-(5-bromothiazol-2-yl)cyclohexyl] carbamate (42.49 mg, 122.35 μmol), [1,1'-bis(diphenylphosphino)ferrocene]dichloropalladium (3.73 mg, 5.10 μmol), potassium phosphate (43.29 mg, 203.92 μmol) and water (0.5 mL) were added into the above reaction solution. The reaction solution was reacted overnight at 80°C. The reaction solution was filtered and concentrated. The resultant was purified by preparative liquid chromatography (Agela DuraShell C18 column: 5 μm silica, 25 mm in diameter, 150 mm in length; using decreasingly polar mixtures of water (containing 0.04% aqueous ammonia and 10 mm $NH_4CO_3$) and acetonitrile as eluents) to obtain the compound isopropyl trans-N -[3-(2,2-dimethylpropylsulfonyl)-4-[2-[4-(isopropoxycarbonylamino)cyclohexyl]thiazol-5-yl] phenyl]carbamate (3.25 mg).

MS m/z (ESI): 580.1 $[M+H]^+$;

$^1H$ NMR (400MHz, METHANOL-$d_4$) δ ppm 8.34 (s, 1H), 7.84 (d, J = 2.4 Hz, 1H), 7.67 (s, 1H), 7.47(d, J = 8.4 Hz, 1H), 5.06-5.00 (m, 1H), 4.99-4.83 (m, 1H), 3.51-3.47 (m, 1H), 3.08-3.02 (m, 1H), 2.91 (s, 2H), 2.23 (d, J = 12.0 Hz, 2H), 2.08 (d, J = 10.0 Hz, 2H), 1.74-1.69 (m, 2H), 1.48-1.41 (m, 2H), 1.34 (d, J = 6.0 Hz, 6H), 1.24 (d, J = 6.0 Hz, 6H), 1.02 (s, 9H).

Example 25: Synthesis of isopropyl trans-N-[4-[5-[4-(isopropoxycarbonylamino) cyclohexyl]-1,3,4-thiadiazol-2-yl]-3-isopropylsulfonyl-phenyl]carbamate

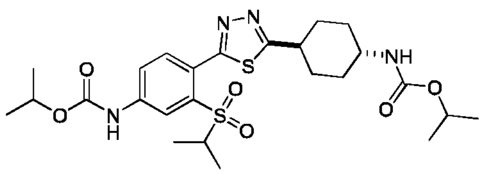

Synthetic Method

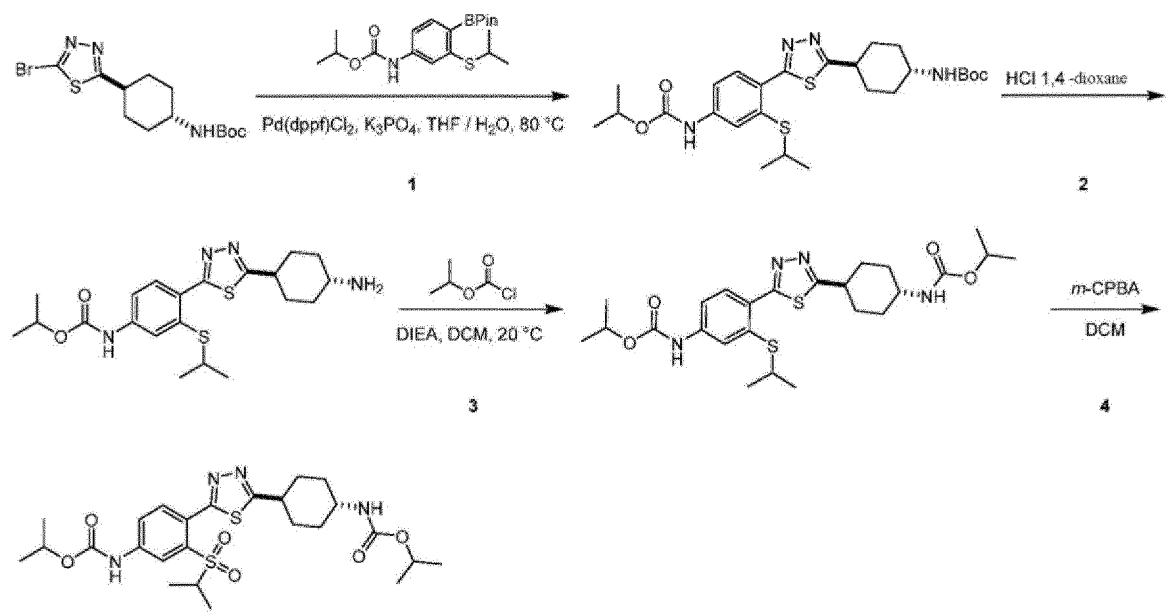


Step 1: Synthesis of isopropyl trans-N-[4-[5-[4-(tert-butoxycarbonylamino)cyclohexyl]-1,3,4-thiadiazol-2-yl]-3-isopropylmercapto-phenyl]carbamate

[1,1'-Bis(diphenylphosphino)ferrocene]dichloropalladium - dichloromethane complex (4.51 mg, 5.52 μmol) was added into a solution of isopropyl N-[3-isopropylmercapto-4-(4,4,5,5-tetramethyl-1,3,2-dioxaborolan-2-yl)phenyl]carbamate (50.26 mg, 132.49 μmol), tert-butyl trans-N-[4-(5-bromo-1,3,4-thiadiazol-2-yl)cyclohexyl]carbamate (40 mg, 110.41 μmol) and potassium phosphate (46.87 mg, 220.82 μmol) in tetrahydrofuran (2 mL) and water (0.5 mL). Under the protection of nitrogen, the reaction solution was stirred overnight at 80°C. The completion of the reaction was monitored by LCMS. The reaction solution was concentrated to dryness, and then the resultant was purified by column chromatography (silica, petroleum ether/ethyl acetate = 1/1) to obtain isopropyl trans-N-[4-[5-[4-(tert-butoxycarbonylamino)cyclohexyl]-1,3,4-thiadiazol-2-yl]-3-isopropylmercapto-phenyl] carbamate as the product (35 mg).

MS m/z (ESI): 535.2 $[M+H]^+$;

Step 2: Synthesis of trans-N-[4-[5-(4-aminocyclohexyl)-1,3,4-thiadiazol-2-yl]-3-isopropylmercapto-phenyl]carbamate

At room temperature, hydrochloric acid/dioxane (4M, 0.35 mL) was added into a solution of isopropyl trans-N-[4-[5-[4-(tert-butoxycarbonylamino)cyclohexyl]-1,3,4-thiadiazol-2-yl]-3-isopropylmercapto-phenyl]carbamate (35 mg, 65.45 μmol) in dioxane (1 mL). The reaction solution was stirred at room temperature for 4 hours. The completion of the reaction was monitored by LCMS. The reaction solution was concentrated to dryness to obtain isopropyl trans-N-[4-[5-(4-aminocyclohexyl)-1,3,4-thiadiazol-2-yl]-3-isopropylmercapto-phenyl]carbamate as the product (28 mg).

MS m/z (ESI): 435.1 $[M+H]^+$;

Step 3: Synthesis of isopropyl trans-N-[4-[5-[4-(isopropoxycarbonylamino)cyclohexyl]-1,3,4-thiadiazol-2-yl]-3-isopropylmercapto-phenyl]carbamate

Isopropyl chloroformate (9.47 mg, 77.31 μmol) and diisopropylethylamine (24.98 mg, 193.27 μmol) were added into a solution of isopropyl trans-N-[4-[5-(4-aminocyclohexyl)-1,3,4-thiadiazol-2-yl]-3-isopropylmercapto-phenyl]carbamate (28 mg, 64.42 μmol) in dichloromethane (3 mL). The reaction solution was stirred overnight at room temperature. The completion of the reaction was monitored by LCMS. The reaction solution was concentrated to dryness and purified by thin-layer chromatography (silica, petroleum ether/ethyl acetate = 1/1) to obtain isopropyl trans-N-[4-[5-[4-(isopropoxycarbonylamino) cyclohexyl]-1,3,4-thiadiazol-2-yl]-3-isopropylmercapto-phenyl]carbamate as the product (23.5 mg).

MS m/z (ESI): 521.2 $[M+H]^+$;

Step 4: Synthesis of isopropyl trans-N-[4-[5-[4-(isopropoxycarbonylamino)cyclohexyl]-1,3,4-thiadiazol-2-yl]-3-isopropylsulfonyl-phenyl]carbamate

At 0°C, m-chloroperoxybenzoic acid (10.99 mg, 54.16 μmol) was added into a solution of isopropyl trans-N-[4-[5-[4-(isopropoxycarbonylamino)cyclohexyl]-1,3,4-thiadiazol-2-yl]-3-isopropylmercapto-phenyl]carbamate (23.5 mg, 45.13 μmol) in dichloromethane (3 mL). The reaction solution was stirred at room temperature for 4 hours. The resulting mixture was diluted with dichloromethane (20 mL) and then washed with a saturated aqueous sodium sulfite solution (5 mL, twice). The organic phase was dried over anhydrous sodium sulfate, filtered, and concentrated to dryness. The resultant was purified by preparative liquid chromatography (Agela DuraShell C18 column: 5 μm silica, 25 mm in diameter, 150 mm in length; using decreasingly polar mixtures of water (containing 0.04% aqueous ammonia and 10 mM $NH_4CO_3$) and acetonitrile as eluents) to obtain the compound isopropyl trans-N-[4-[5 -[4-(isopropoxycarbonylamino)cyclohexyl]-1,3,4-thiadiazol-2-yl]-3-isopropylsulfonyl-phenyl] carbamate (1.52 mg).

MS m/z (ESI): 553.1 $[M+H]^+$;

$^1H$ NMR (400 MHz, MeOD) δ ppm 8.31 (d, J = 2.4 Hz, 1H), 7.93 (d, J = 8.4 Hz, 1H), 7.63 (d, J = 2.4 Hz, 1H), 5.06-5.01 (m, 2H), 4.06-4.02 (m, 1H), 3.50-3.49 (m, 1H), 3.25-3.22 (m, 1H), 2.36-2.25 (m, 2H), 2.20-2.07 (m, 2H), 1.79-1.72 (m, 2H), 1.48-1.44 (m, 2H), 1.35 (d, J = 6.4 Hz, 6H), 1.29 (d, J = 6.4 Hz, 6H), 1.25 (d, J = 6.4 Hz, 6H).

Example 26: Synthesis of isopropyl trans-(4-(5-(4-(3-benzylureido)-2-(dimethylphosphoryl)phenyl)thiazol-2-yl)cyclohexyl)carbamate

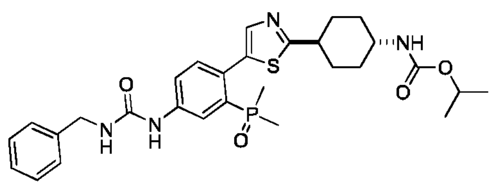

Synthetic Method

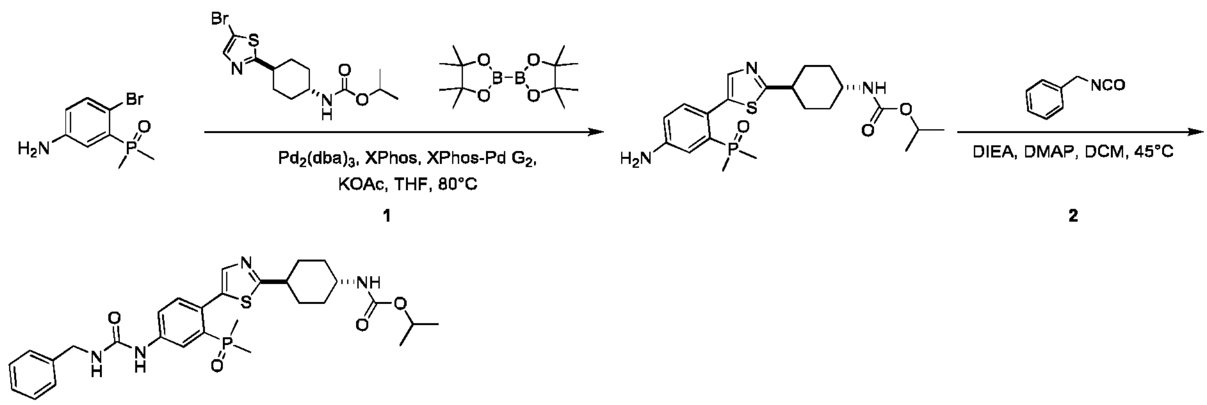


Step 1: Synthesis of isopropyl trans-(4-(5-(4-amino-2-(dimethylphosphoryl)phenyl) thiazol-2-yl)cyclohexyl)carbamate

(5-Amino-2-bromophenyl)dimethylphosphine oxide (60 mg, 241.88 μmol), isopropyl trans-N-[4-(5-bromothiazol-2-yl)cyclohexyl]carbamate (84 mg, 241.88 μmol) and 4,4,5,5-tetramethyl-2-(4,4,5,5-tetramethyl-1,3,2-dioxaborolan-2-yl)-1,3,2-dioxaborolane (245.69 mg, 967.52 μmol) were dissolved in tetrahydrofuran (6 mL). Under the protection of nitrogen, 2-dicyclohexylphosphino-2,4,6-triisopropylbiphenyl (5.97 mg, 12.09 μmol), chloro(2-dicyclohexylphosphino-2,4,6-triisopropyl-1,1-biphenyl)[2-(2-amino-1,1-biphenyl)] palladium (II) (9.52 mg, 12.09 μmol), tris(dibenzylideneacetone)dipalladium (11.07 mg, 12.09 μmol) and potassium acetate (47.48 mg, 483.76 μmol) were added. The reaction solution was stirred and reacted at 80°C for 16 hours. The reaction solution was filtered. The filtrate was concentrated to dryness under reduced pressure. The resultant was added with water (5 mL), and the mixture was extracted with 15 mL of ethyl acetate (5 mL × 3). The organic phases were combined, dried over anhydrous sodium sulfate, filtered, and concentrated to obtain a black oily compound, which was further purified by column chromatography (silica, petroleum ether:ethyl acetate = 1:2) to obtain isopropyl trans-(4-(5-(4-amino-2-(dimethylphosphoryl) phenyl)thiazol-2-yl)cyclohexyl)carbamate (23 mg).

MS m/z (ESI): 436.2 $[M+H]^+$.

Step 2: Synthesis of isopropyl trans-(4-(5-(4-(3-benzylureido)-2-(dimethylphosphoryl) phenyl)thiazol-2-yl)cyclohexyl)carbamate

Isopropyl trans-(4-(5-(4-amino-2-(dimethylphosphoryl)phenyl)thiazol-2-yl)cyclohexyl) carbamate (20 mg, 45.92 μmol) and benzyl isocyanate (12.23 mg, 91.84 μmol) were dissolved in dichloromethane (4 mL), and N,N-diisopropylethylamine (11.87 mg, 91.84 μmol, 16.00 μL) and 4-dimethylaminopyridine (2.81 mg, 22.96 μmol) were added. The reaction solution was stirred at 45°C for 2 hours. LCMS showed that the reaction was complete. The reaction system was evaporated to dryness via rotary evaporation, and then the resultant was purified by preparative liquid chromatography (Phenomenex Gemini column: 10 μm silica, 50 mm in diameter, 250 mm in length; using decreasingly polar mixtures of water (containing 0.05% aqueous ammonia) and acetonitrile (35% to 55%) as eluents) to obtain the compound isopropyl trans-(4-(5-(4-(3-benzylureido)-2-(dimethylphosphoryl)phenyl)thiazol-2-yl)cyclohexyl) carbamate as a white solid (4.75 mg).

MS m/z (ESI): 569.1 $[M+H]^+$;

$^1H$ NMR (400 MHz, DMSO-$d_6$) δ ppm 9.02 (s, 1H) 7.91 (dd, J = 14.31, 2.32 Hz, 1H) 7.80 (s, 1H) 7.71 (dd, J = 8.44, 1.47 Hz, 1H) 7.39-7.28 (m, 5H) 7.27-7.22 (m, 1H) 7.03 (br d, J = 7.70 Hz, 1H) 6.76 (t, J = 5.93 Hz, 1H) 4.82-4.70 (m, 1H) 4.32 (d, J = 5.99 Hz, 2H) 3.30-3.26 (m, 1H) 3.02-2.85 (m, 1H) 2.12 (br d, J = 11.25 Hz, 2H) 1.91 (br d, J = 9.66 Hz, 2H) 1.63-1.49 (m, 2H) 1.43 (d, J = 13.20 Hz, 6H) 1.39-1.26 (m, 2H) 1.16 (d, J = 6.24 Hz, 6H).

Example 27: Synthesis of isopropyl trans-N-[4-[2-[4-(isopropoxycarbonylamino) cyclohexyl]thiazol-5-yl]-3-(1-oxophospholan-1-yl)phenyl]carbamate

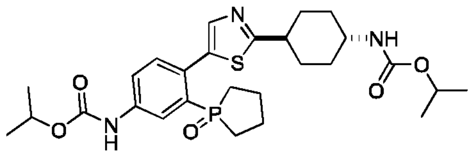

Synthetic Method

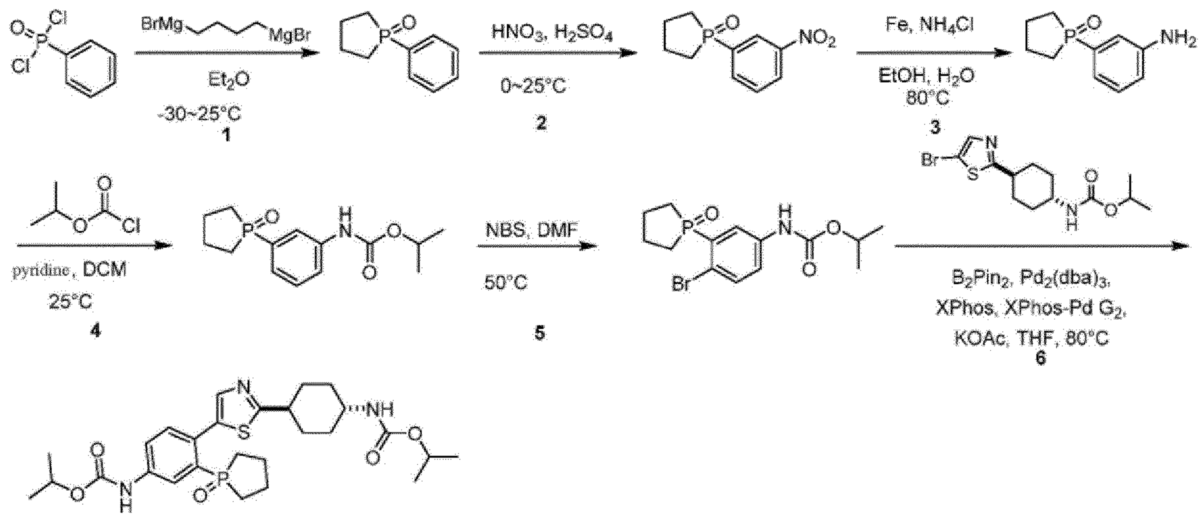


Step 1: Synthesis of 1-phenylphospholane-1-oxide

Phenylphosphonic dichloride (4 g, 20.51 mmol) was dissolved in diethyl ether (15 mL). Under the protection of nitrogen, butyl di(magnesium bromide) (5.43 g, 20.51 mmol) was added at -30°C. The reaction solution appeared as a gray suspension, and was stirred and reacted at 25°C for 16 hours. The completion of the reaction was detected by LCMS. The reaction solution was added with saturated ammonium chloride solution (30 mL), and extracted with ethyl acetate (60 mL). The organic layer was dried over anhydrous magnesium sulfate and subjected to suction filtration. The

filtrate was concentrated to dryness under reduced pressure. The resultant was purified by column chromatography (dichloromethane:methanol = 20:1) to obtain the compound 1-phenylphospholane-1-oxide (830 mg, 4.61 mmol) as a yellow oil.

MS m/z (ESI): 181.1 $[M+H]^+$

Step 2: Synthesis of 1-(3-nitrophenyl)phospholane-1-oxide

1-Phenylphospholane-1-oxide (700 mg, 3.88 mmol) was dissolved in concentrated sulfuric acid (10 mL), and fuming nitric acid (734.40 mg, 11.65 mmol, 524.57 μL) was added at 0°C. The reaction solution appeared as a yellow suspension, and was stirred and reacted at 25°C for 2 hours. The completion of the reaction was detected by LCMS. The reaction solution was added with water (30 mL), and then extracted with ethyl acetate (60 mL). The organic layer was dried over anhydrous magnesium sulfate and subjected to suction filtration. The filtrate was concentrated to dryness under reduced pressure to obtain crude 1-(3-nitrophenyl)phospholane-1-oxide (800mg) as a yellow oil.

MS m/z (ESI): 226.1 $[M+H]^+$

Step 3: Synthesis of 1-(3-aminophenyl)phospholane-1-oxide

1-(3-Nitrophenyl)phospholane-1-oxide (800 mg, 3.55 mmol) was dissolved in anhydrous ethanol (3 mL) and water (600 μL). Under the protection of nitrogen, reduced iron powder (1.19 g, 21.32 mmol) and ammonium chloride (1.90 g, 35.53 mmol) were added. The reaction solution appeared as a black suspension, and was stirred and reacted at 80°C for 3 hours. The completion of the reaction was detected by LCMS. The reaction solution was filtered while it was still hot. The filtrate was concentrated to dryness under reduced pressure. The resultant was purified by column chromatography (dichloromethane:methanol = 10:1) to obtain the compound 1-(3-aminophenyl)phospholane-1-oxide (230 mg, 1.18 mmol) as a yellow oil.

MS m/z (ESI): 196.1 $[M+H]^+$

Step 4: Synthesis of isopropyl (3-(1-oxidophospholan-1-yl)phenyl)carbamate

1-(3-Aminophenyl)phospholane-1-oxide (230 mg, 1.18 mmol) was dissolved in anhydrous dichloromethane (3 mL). Under the protection of nitrogen, pyridine (559.22 mg, 7.07 mmol, 570.63 μL) and isopropyl chloroformate (433.20 mg, 3.53 mmol, 490.60 μL) were added. The reaction solution appeared as a yellow suspension, and was stirred and reacted at 25°C for 1 hour. The completion of the reaction was detected by LCMS. The reaction solution was concentrated to dryness under reduced pressure. The resultant was purified by column chromatography (dichloromethane:methanol = 10:1) to obtain the compound isopropyl (3-(1-oxidophospholan-1-yl)phenyl)carbamate (260 mg, 924.32 μmol) as a yellow solid.

MS m/z (ESI): 282.2 $[M+H]^+$

Step 5: Synthesis of isopropyl (4-bromo-3-(1-oxidophospholan-1-yl)phenyl)carbamate

Isopropyl (3-(1-oxidophospholan-1-yl)phenyl)carbamate (260 mg, 924.32 μmol) was dissolved in N,N-dimethylformamide (5 mL), and N-bromosuccinimide (493.54 mg, 2.77 mmol) was added. The reaction solution appeared as a yellow suspension, and was stirred and reacted at 50°C for 16 hours. The completion of the reaction was detected by LCMS. The reaction solution was concentrated to dryness under reduced pressure. The resultant was purified by column chromatography (petroleum ether:tetrahydrofuran = 1:1) to obtain the compound isopropyl (4-bromo-3-(1-oxidophospholan-1-yl)phenyl)carbamate (300 mg, 832.91 μmol) as a white solid.

MS m/z (ESI): 360.1, 362.0 $[M+H]^+$

Step 6: Synthesis of isopropyl trans-N-[4-[2-[4-(isopropoxycarbonylamino)cyclohexyl] thiazol-5-yl]-3-(1-oxophospholan-1-yl)phenyl]carbamate

Isopropyl (4-bromo-3-(1-oxidophospholan-1-yl)phenyl)carbamate (200 mg, 555.27 μmol), isopropyl trans-N-[4-(5-bromothiazol-2-yl)cyclohexyl]carbamate (192.83 mg, 555.27 μmol) and bis(pinacolato)diboron (564.02 mg, 2.22 mmol) were dissolved in anhydrous tetrahydrofuran (5 mL). Under the protection of nitrogen, tris(dibenzylideneacetone)dipalladium(0) (25.42 mg, 27.76 μmol) and 2-dicyclohexylphosphino-2,4,6-triisopropylbiphenyl (13.24 mg, 27.76 μmol), chloro(2-dicyclohexylphosphino-2,4,6-triisopropyl-1,1-biphenyl)[2-(2-amino-1,1-biphenyl)] palladium (II) (21.84 mg, 27.76 μmol) and potassium acetate (108.99 mg, 1.11 mmol) were added. The reaction solution appeared as a black suspension, and was stirred and reacted at 80°C for 16 hours. The completion of the reaction was detected by LCMS. The reaction solution was concentrated to dryness under reduced pressure. The resultant was added with water (10

mL) and the mixture was extracted with ethyl acetate (20 mL). The organic layer was dried over anhydrous magnesium sulfate and subjected to suction filtration. The filtrate was concentrated to dryness under reduced pressure. The resultant was purified by preparative liquid chromatography (PhenomenexGeminiC18 column, 10 μm silica, 50 mm in diameter, 250 mm in length; using decreasingly polar mixtures of water (containing 0.05% aqueous ammonia) and acetonitrile as eluents) to obtain the compound isopropyl trans-N-[4-[2-[4-(isopropoxycarbonylamino)cyclohexyl]thiazol-5-yl]-3-(1-oxophospholan-1-yl) phenyl]carbamate (65.22 mg) as a white solid.

MS m/z (ESI): 548.1 $[M+H]^+$;

$^1H$ NMR (400MHz, DMSO-$d_6$) δ ppm 9.92 (s, 1H), 8.09-8.02 (m, 1H), 7.94 (s, 1H), 7.76-7.70 (m, 1H), 7.49-7.44 (m, 1H), 7.08-7.01 (m, 1H), 5.00-4.89 (m, 1H), 4.83-4.70 (m, 1H), 3.36-3.33 (m, 1H), 3.02-2.89 (m, 1H), 2.19-2.09 (m, 2H), 1.98-1.78 (m, 6H), 1.69-1.50 (m, 6H), 1.43-1.32 (m, 2H), 1.29 (d, J = 6.3 Hz, 6H), 1.18 (d, J = 6.3 Hz, 6H).

Example 28: Synthesis of isopropyl trans-(4-(5-(2-(N-(tert-butyl)sulfamoyl)-4-((5-methyl-1H-pyrazol-3-yl)amino)phenyl)thiazol-2-yl)cyclohexyl)carbamate

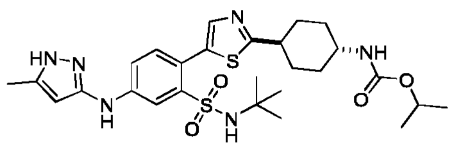

Synthetic Method

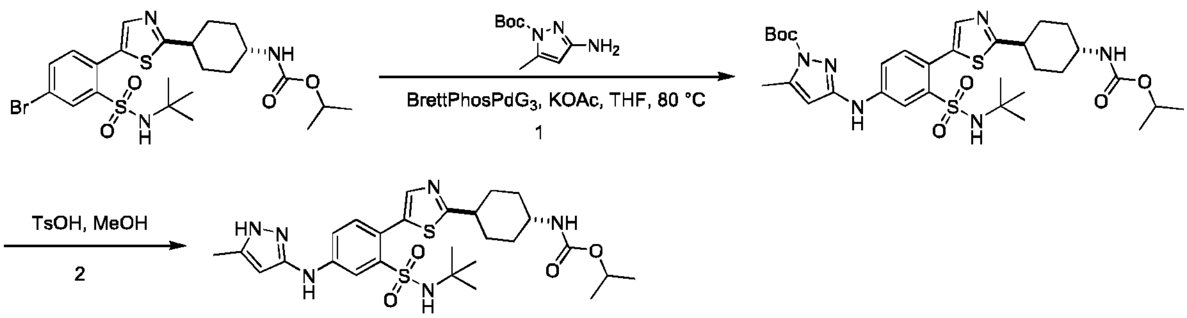


Step 1: Synthesis of tert-butyl trans-3-((3-(N-(tert-butyl)sulfamoyl)-4-(2-4-((isopropoxycarbonyl)amino)cyclohexyl)thiazol-5-yl)phenyl)amino)-5-methyl-1H-pyrazole-1-carboxylate

Isopropyl trans-N-[4-[5-[4-bromo-2-(tert-butylsulfamoyl)phenyl]thiazol-2-yl]cyclohexyl] carbamate (250 mg, 447.59 μmol) (an intermediate synthesized in Example 22) and tert-butyl 3-amino-5-methyl-pyrazole-1-carboxylate (264.84 mg, 1.34 mmol) were dissolved in anhydrous tetrahydrofuran (5 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (20.29 mg, 22.38 μmol) and potassium acetate (131.78 mg, 1.34 mmol) were added. The reaction solution was stirred at 80°C for 16 hours. LCMS showed that the reaction was complete. The reaction system was evaporated to dryness via rotary evaporation, and then the resultant was subjected to liquid-liquid extraction (extracted with 120 mL of ethyl acetate three times) to obtain tert-butyl trans-3-((3-(N-(tert-butyl)sulfamoyl)-4-(2-4-((isopropoxycarbonyl)amino)cyclohexyl)thiazol-5 -yl)phenyl)amino)-5-methyl-1H-pyrazole-1-carboxylate (280 mg, crude product).

MS m/z (ESI): 674.9 $[M+H]^+$;

Step 2: Synthesis of isopropyl trans-(4-(5-(2-(N-(tert-butyl)sulfamoyl)-4-((5-methyl-1H-pyrazol-3-yl)amino)phenyl)thiazol-2-yl)cyclohexyl)carbamate

Tert-butyl trans-3-((3-(N-(tert-butyl)sulfamoyl)-4-(2-4-((isopropoxycarbonyl)amino) cyclohexyl)thiazol-5-yl)phenyl)amino)-5-methyl-1H-pyrazole-1-carboxylate (235 mg, 348.21 μmol) was dissolved in methanol (5 mL), and p-toluenesulfonic acid (179.89 mg, 1.04 mmol) was added. The reaction solution was stirred at 50°C for 2 hours. LCMS showed that the reaction was complete. The reaction system was evaporated to dryness via rotary evaporation. The

resultant was added with 2M sodium hydroxide (10 mL), and then the mixture was extracted with dichloromethane (120 mL) three times. The resultant was purified by preparative high-performance liquid chromatography (Phenomenex Gemini-NX column, 5 μm silica, 30 mm in diameter, 150 mm in length) (using decreasingly polar mixtures of water (containing 0.05% aqueous ammonia and 10 mmol of ammonium bicarbonate) and acetonitrile (45% to 65%) as eluents) and lyophilized to obtain isopropyl trans-(4-(5-(2-(N-(tert-butyl)sulfamoyl)-4-((5-methyl-1H-pyrazol-3-yl)amino)phenyl)thiazol-2-yl)cyclohexyl)carbamate (32.73 mg).

MS m/z (ESI): 575.2 $[M+H]^+$;

$^1H$ NMR (400 MHz, DMSO-$d_6$) δ ppm 11.82 (s, 1H) 8.91 (s, 1H) 8.14 (d, J = 2.32 Hz, 1H) 7.60 (s, 1H) 7.50 (dd, J = 8.44, 2.32 Hz, 1H) 7.23 (d, J = 8.31 Hz, 1H) 7.01 (br d, J = 7.46 Hz, 1H) 6.70 (s, 1H) 5.63 (s, 1H) 4.81-4.69 (m, 1H) 3.35-3.25 (m, 1H) 2.92-2.80 (m, 1H) 2.19 (s, 3H) 2.12 (br d, J = 11.62 Hz, 2H) 1.91 (br d, J = 10.15 Hz, 2H) 1.63-1.49 (m, 2H) 1.41-1.26 (m, 2H) 1.16 (d, J = 6.24 Hz, 6H) 1.07 (s, 9H).

Example 29: Synthesis of isopropyl trans-(4-(5-(2-(N-(tert-butyl)sulfamoyl)-4-(isoxazol-5-ylamino)phenyl)thiazol-2-yl)cyclohexyl)carbamate

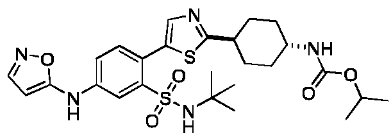

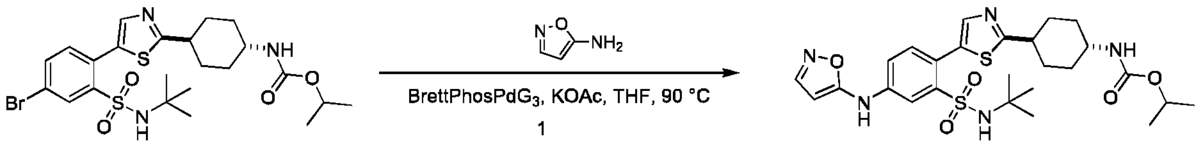


Isopropyl trans-N-[4-[5-[4-bromo-2-(tert-butylsulfamoyl)phenyl]thiazol-2-yl]cyclohexyl] carbamate (120 mg, 214.84 μmol) and isoxazol-5-amine (72.25 mg, 859.37 μmol) were dissolved in tetrahydrofuran (5 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (9.74 mg, 10.74 μmol) and potassium acetate (63.26 mg, 644.52 μmol) were added. The reaction solution was stirred at 90°C for 1 hour. TLC (dichloromethane:methanol = 15:1) showed that the reaction was complete. The reaction system was evaporated to dryness via rotary evaporation, and then the resultant was purified twice by thin-layer chromatography (silica, dichloromethane:methanol = 10:1) to obtain isopropyl trans-(4-(5-(2-(N-(tert-butyl)sulfamoyl)-4-(isoxazol-5-ylamino)phenyl)thiazol-2-yl)cyclohexyl)carbamate (2.35mg).

MS m/z (ESI): 562.1 $[M+H]^+$;

$^1H$NMR: (400MHz, DMSO-$d_6$) δ ppm 10.74 (s, 1H) 8.35 (d, J = 2.25 Hz, 1H) 7.78 (dd, J = 8.44, 2.19 Hz, 1H) 7.68 (s, 1H) 7.46 (d, J = 8.25 Hz, 1H) 7.17-6.95 (m, 2H) 4.80-4.70 (m, 1H) 3.97 (d, J = 4.13 Hz, 1H) 3.31-3.25 (m, 1H) 2.99-2.86 (m, 1H) 2.19-2.05 (m, 2H) 1.92-1.87 (m, 2H) 1.68-1.50 (m, 2H) 1.43-1.27 (m, 2H) 1.16 (d, J = 6.25 Hz, 6H) 1.05 (s, 9H).

Example 30: Synthesis of isopropyl trans-(4-(5-(2-(N-(tert-butyl)sulfamoyl)-4-(isoxazol-3-ylamino)phenyl)thiazol-2-yl)cyclohexyl)carbamate

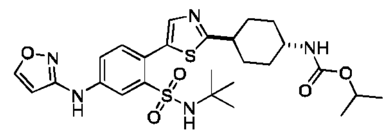

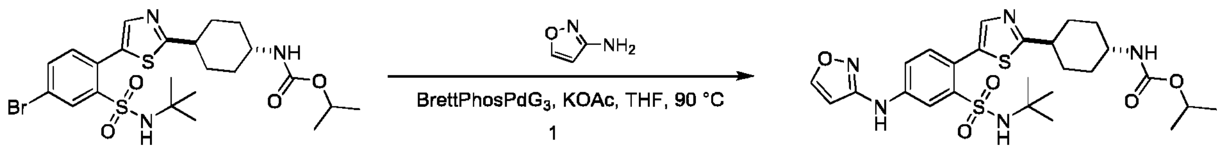


Isopropyl trans-N-[4-[5-[4-bromo-2-(tert-butylsulfamoyl)phenyl]thiazol-2-yl]cyclohexyl] carbamate (300 mg, 537.10 μmol) and isoxazol-3-amine (135.47 mg, 1.61 mmol) were dissolved in anhydrous tetrahydrofuran (15 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (24.34 mg, 26.86 μmol) and potassium acetate (158.14 mg, 1.61 mmol) were added. The reaction solution was stirred at 80°C for 0.5 hours. LCMS showed that the reaction was complete. The reaction system was evaporated to dryness via rotary evaporation, and then the resultant was purified by preparative high-performance liquid chromatography (Phenomenex Gemini-NX column, 5 μm silica, 30 mm in diameter, 150 mm in length) (using decreasingly polar mixtures of water (containing 0.05% aqueous ammonia and 10 mmol of ammonium bicarbonate) and acetonitrile (45% to 65%) as eluents) and lyophilized to obtain isopropyl trans-(4-(5-(2-(N-(tert-butyl)sulfamoyl)-4-(isoxazol-3-ylamino)phenyl)thiazol-2-yl)cyclohexyl)carbamate (32.79 mg).

MS m/z (ESI): 562.2 $[M+H]^+$;

$^1$H NMR (400 MHz, DMSO-$d_6$) δ ppm 9.76 (s, 1H) 8.67 (d, J = 1.59 Hz, 1H) 8.26 (d, J = 2.45 Hz, 1H) 7.71-7.57 (m, 2H) 7.40 (d, J = 8.44 Hz, 1H) 7.08-6.98 (m, 1H) 6.92 (br s, 1H) 6.25 (d, J = 1.71 Hz, 1H) 4.80-4.70 (m, 1H) 3.31-3.24 (m, 1H) 2.97-2.82 (m, 1H) 2.13 (br d, J = 11.74 Hz, 2H) 1.92 (br d, J = 10.15 Hz, 2H) 1.66-1.49 (m, 2H) 1.43-1.26 (m, 2H) 1.16 (d, J = 6.11 Hz, 6H) 1.08 (s, 9H).

Example 31: Synthesis of isopropyl trans-N-[4-[2-[4-(isopropoxycarbonylamino) cyclohexyl]thiazol-5-yl]-3-[isopropyl(methyl)phosphoryl]phenyl]carbamate

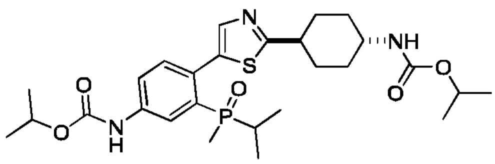

Synthetic Method

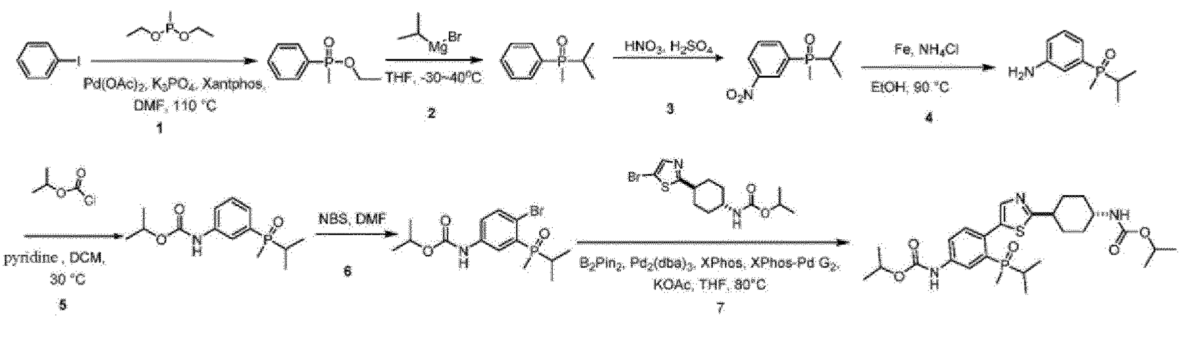


Step 1: Synthesis of ethyl methyl(phenyl)phosphinate

Iodobenzene (800 mg, 3.92 mmol, 437.16 μL), potassium phosphate (1.66 g, 7.84 mmol) and 4,5-bis(diphenylphosphino)-9,9-dimethylxanthene (453.80 mg, 784.28 μmol) were dissolved in N,N-dimethylformamide (2 mL). Under the protection of nitrogen, methyldiethoxyphosphine (1.07 g, 7.84 mmol) and palladium acetate (176.08 mg, 784.28 μmol) were added. The reaction solution appeared as a light yellow suspension, and was stirred and reacted at 100°C for 16 hours. The completion of the reaction was detected by LCMS. The reaction solution was concentrated to dryness under reduced pressure. The resultant was purified by column chromatography (petroleum ether:tetrahydrofuran = 3:1)

to obtain the compound ethyl methyl(phenyl)phosphinate (540 mg, 2.93 mmol).
MS m/z (ESI): 185.1 $[M+H]^+$;

Step 2: Synthesis of isopropyl(methyl)(phenyl)phosphine oxide

Ethyl methyl(phenyl)phosphinate (200 mg, 1.09 mmol) was dissolved in anhydrous tetrahydrofuran (3 mL). Under the protection of nitrogen, a solution (2M, 3.26 mL) of isopropylmagnesium chloride (2.0M) in tetrahydrofuran was added at -30°C. The reaction solution appeared as a brown suspension, and was stirred and reacted at 40°C for 16 h. The completion of the reaction was detected by LCMS. The reaction solution was concentrated to dryness under reduced pressure. The resultant was added with saturated ammonium chloride (10 mL), and the mixture was extracted with ethyl acetate (20 mL) twice. The organic layer was dried over anhydrous magnesium sulfate and subjected to suction filtration. The filtrate was concentrated to dryness under reduced pressure. The resultant was purified by column chromatography (petroleum ether:tetrahydrofuran = 1:2) to obtain the compound isopropyl(methyl)(phenyl)phosphine oxide (115 mg, 631.18 μmol).
MS m/z (ESI): 183.2 $[M+H]^+$;

Step 3: Synthesis of isopropyl(methyl)(3-nitrophenyl)phosphine oxide

Isopropyl(methyl)(phenyl)phosphine oxide (115 mg, 631.18 μmol) was dissolved in concentrated sulfuric acid (2 mL), and fuming nitric acid (119.32 mg, 1.89 mmol, 85.23 μL) was added at 0°C. The reaction solution appeared as a yellow suspension, and was stirred and reacted at 25°C for 2 h. The completion of the reaction was detected by LCMS. The reaction solution was added with water (10 mL) and then extracted with ethyl acetate (20 mL). The organic layer was dried over anhydrous magnesium sulfate and subjected to suction filtration. The filtrate was concentrated to dryness under reduced pressure to obtain crude isopropyl(methyl)(3-nitrophenyl)phosphine oxide (70 mg).
MS m/z (ESI): 228.1 $[M+H]^+$;

Step 4: Synthesis of (3-aminophenyl)(isopropyl)(methyl)phosphine oxide

Isopropyl(methyl)(3-nitrophenyl)phosphine oxide (70 mg, 308.10 μmol) was dissolved in anhydrous ethanol (3 mL) and water (600 μL). Under the protection of nitrogen, reduced iron powder (103.24 mg, 1.85 mmol) and ammonium chloride (164.80 mg, 3.08 mmol) were added. The reaction solution appeared as a black suspension, and was stirred and reacted at 90°C for 12 h. The completion of the reaction was detected by LCMS. The reaction solution was filtered while it was still hot. The filtrate was concentrated to dryness under reduced pressure. The resultant was purified by column chromatography (dichloromethane:methanol = 10:1) to obtain the compound (3-aminophenyl)(isopropyl)(methyl)phosphine oxide (45 mg, 228.18 μmol).
MS m/z (ESI): 198.2 $[M+H]^+$;

Step 5: Synthesis of isopropyl (3-(isopropyl(methyl)phosphoryl)phenyl)carbamate

(3-Aminophenyl)(isopropyl)(methyl)phosphine oxide was dissolved in anhydrous dichloromethane (3 mL). Under the protection of nitrogen, pyridine (108.29 mg, 1.37 mmol, 110.50 μL) and isopropyl chloroformate (83.89 mg, 684.54 μmol, 95.01 μL) were added. The reaction solution appeared as a yellow suspension, and was stirred and reacted at 30°C for 1 h. The completion of the reaction was detected by LCMS. The reaction solution was concentrated to dryness under reduced pressure. The resultant was purified by thin-layer chromatography (dichloromethane:methanol = 10:1) to obtain the compound isopropyl (3-(isopropyl(methyl)phosphoryl)phenyl)carbamate (30 mg, 105.89 μmol).
MS m/z (ESI): 284.2 $[M+H]^+$;

Step 6: Synthesis of isopropyl (4-bromo-3-(isopropyl(methyl)phosphoryl)phenyl) carbamate

Isopropyl (3-(isopropyl(methyl)phosphoryl)phenyl)carbamate (30 mg, 105.89 μmol) was dissolved in N,N-dimethylformamide (2 mL). N-bromosuccinimide (56.54 mg, 317.68 μmol) was added. The reaction solution appeared as a yellow suspension, and was stirred and reacted at 50°C for 16 h. The completion of the reaction was detected by LCMS. The reaction solution was concentrated to dryness under reduced pressure. The resultant was purified by column chromatography (petroleum ether:ethyl acetate = 1:1) to obtain the compound isopropyl (4-bromo-3-(isopropyl(methyl)phosphoryl)phenyl)carbamate (30 mg, 82.83 μmol).
MS m/z (ESI): 362.1, 364.1 $[M+H]^+$;

Step 7: Synthesis of isopropyl trans-N-[4-[2-[4-(isopropoxycarbonylamino) cyclohexyl]thiazol-5-yl]-3-[isopropyl(methyl)phosphoryl]phenyl]carbamate

Isopropyl (4-bromo-3-(isopropyl(methyl)phosphoryl)phenyl)carbamate (25 mg, 69.02 μmol), isopropyl trans-N-[4-(5-bromothiazol-2-yl)cyclohexyl]carbamate (23.97 mg, 69.02 μmol) and bis(pinacolato)diboron (70.11 mg, 276.09 μmol) were dissolved in anhydrous tetrahydrofuran (3 mL). Under the protection of nitrogen, tris(dibenzylideneacetone)dipalladium(0) (3.16 mg, 3.45 μmol) and 2-dicyclohexylphosphino-2,4,6-triisopropylbiphenyl (1.65 mg, 3.45 μmol), chloro(2-dicyclohexylphosphino-2,4,6-triisopropyl-1,1-biphenyl)[2-(2-amino-1,1-biphenyl)]palladium (II) (2.72 mg, 3.45 μmol) and potassium acetate (13.55 mg, 138.05 μmol) were added. The reaction solution appeared as a black suspension, and was stirred and reacted at 80°C for 16 h. The completion of the reaction was detected by LCMS. The reaction solution was concentrated to dryness under reduced pressure. The resultant was added with water (10 mL), and the mixture was extracted with ethyl acetate (20 mL). The organic layer was dried over anhydrous magnesium sulfate and subjected to suction filtration. The filtrate was concentrated to dryness under reduced pressure. The resultant was purified by preparative liquid chromatography (PhenomenexGeminiC18 column, 10 μm silica, 50 mm in diameter, 250 mm in length; using decreasingly polar mixtures of water (containing 0.225% formic acid) and acetonitrile as eluents) to obtain the compound isopropyl trans-N-[4-[2-[4-(isopropoxycarbonylamino)cyclohexyl]thiazol-5-yl]-3-[isopropyl (methyl)phosphoryl]phenyl]carbamate (5.44 mg, 9.89 μmol).

MS m/z (ESI): 550.1 $[M+H]^+$;

$^1H$ NMR (400MHz, DMSO-$d_6$) δ ppm 9.93 (s, 1H), 8.14 - 8.03 (m, 1H), 7.75 - 7.68 (s, 2H), 7.41 - 7.33 (m, 1H), 7.09 - 7.01 (m, 1H), 5.00 - 4.88 (m, 1H), 4.81 - 4.71 (m, 1H), 3.35 - 3.34 (m, 1H), 3.02 - 2.89 (m, 1H), 2.19 - 2.08 (m, 2H), 1.98 - 1.88 (m, 2H), 1.75 - 1.64 (m, 1H), 1.61 - 1.54 (m, 2H), 1.35 - 1.45 (m, 3H), 1.38 - 1.32 (m, 2H), 1.29 (d, J=6.3 Hz, 6H), 1.18 (d, J=6.3 Hz, 6H), 1.00 - 0.83 (m, 5H).

Example 32: Synthesis of isopropyl trans-N-[4-[2-[4-(isopropoxycarbonylamino) cyclohexyl]thiazol-5-yl]-3-isopropylsulfinyl-phenyl]carbamate

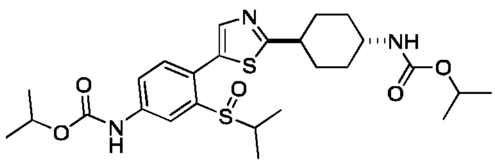

Synthetic Method

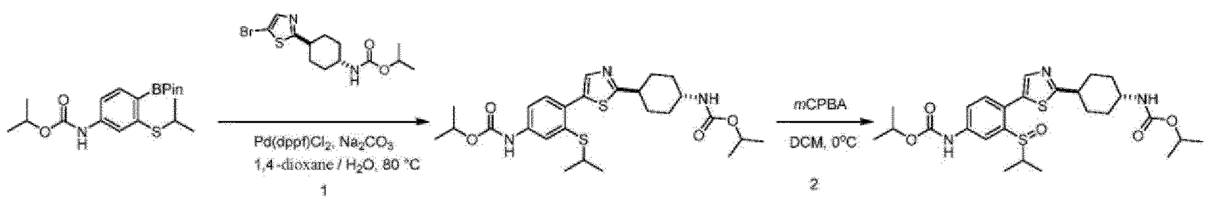


Step 1: Synthesis of isopropyl trans-N-[4-[2-[4-(isopropoxycarbonylamino) cyclohexyl]thiazol-5-yl]-3-isopropylsulfanyl-phenyl]carbamate

Isopropyl trans-N-[4-(5-bromothiazol-2-yl)cyclohexyl]carbamate (100 mg, 287.96 μmol), isopropyl N-[3-isopropylsulfanyl-4-(4,4,5,5-tetramethyl-1,3,2-dioxaborolan-2-yl)phenyl] carbamate (131.08 mg, 345.55 μmol) and sodium carbonate (61.04 mg, 575.92 μmol) were dissolved in water (600 μL) and 1,4-dioxane (6 mL). Under the protection of nitrogen, [1,1-bis(diphenylphosphino)ferrocene]dichloropalladium - dichloromethane (23.52 mg, 28.80 μmol) was added. The reaction solution was stirred at 80°C for 6 hours. LCMS showed that the reaction was complete. The reaction solution was filtered. The filtrate was evaporated to dryness via rotary evaporation to remove 1,4-dioxane. The resultant was added with water (10 mL), and the mixture was extracted three times with ethyl acetate (10 mL × 3). After dried over anhydrous sodium sulfate, the resultant was purified by thin-layer chromatography (silica, petroleum ether/ethyl acetate

= 2:1) to obtain isopropyl trans-N-[4-[2-[4-(isopropoxycarbonylamino)cyclohexyl]thiazol-5-yl]-3-isopropylsulfanyl-phenyl]carbamate (84.8 mg).

MS m/z (ESI): 520.3 $[M+H]^+$

Step 2: Synthesis of isopropyl trans-N-[4-[2-[4-(isopropoxycarbonylamino) cyclohexyl]thiazol-5-yl]-3-isopropylsulfinyl-phenyl]carbamate

Isopropyl trans-N-[4-[2-[4-(isopropoxycarbonylamino)cyclohexyl]thiazol-5-yl]-3-isopropylsulfanyl-phenyl]carbamate (75 mg, 144.31 μmol) was dissolved in dichloromethane (2 mL), and m-chloroperoxybenzoic acid (19.92 mg, 115.45 μmol) was added at 20°C. The reaction solution was reacted at 20°C for 2 hours. LCMS showed that the reaction was complete. The reaction solution was filtered. The filtrate was evaporated to dryness via rotary evaporation to remove dichloromethane. The resultant was added with water (2 mL), and the mixture was extracted three times with ethyl acetate (2 mL × 3). The organic phase was dried over anhydrous sodium sulfate and evaporated to dryness via rotary evaporation under reduced pressure. The resultant was purified by preparative high-performance liquid chromatography (Phenomenex Gemini-NX C18 column, 5 μm silica, 30 mm in diameter, 150 mm in length) (using decreasingly polar mixtures of water (containing 0.05% aqueous ammonia) and acetonitrile (0% to 70%) as eluents) to obtain isopropyl trans-N-[4-[2-[4-(isopropoxycarbonylamino)cyclohexyl]thiazol-5-yl]-3-isopropylsulfinyl-phenyl]carbamate (16.14 mg).

MS m/z (ESI): 536.0 $[M+H]^+$

$^1H$ NMR: (400 MHz, METHANOL-$d_4$) δ ppm 8.02 (d, J = 2.26 Hz, 1H) 7.77-7.71 (m, 2H) 7.48 (d, J = 8.53 Hz, 1H) 5.05-4.95 (m, 1H) 4.88-4.82 (m, 1H) 3.52-3.44 (m, 1H) 3.08-2.98 (m, 1H) 2.82-2.73 (m, 1H) 2.24 (br d, J = 11.80 Hz, 2H) 2.09 (br d, J = 10.04 Hz, 2H) 1.77-1.66 (m, 2H) 1.48-1.38 (m, 2H) 1.34 (d, J = 6.27 Hz, 6H) 1.26-0.98 (m, 12H).

Example 33: Synthesis of isopropyl trans-N-[3-cyclopropylsulfonyl-4-[2-[4-(isopropoxycarbonylamino)cyclohexyl]thiazol-5-yl]phenyl]carbamate

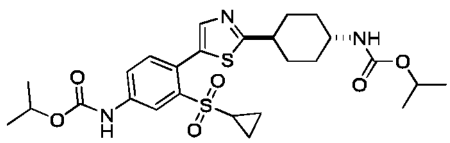

Synthetic Method

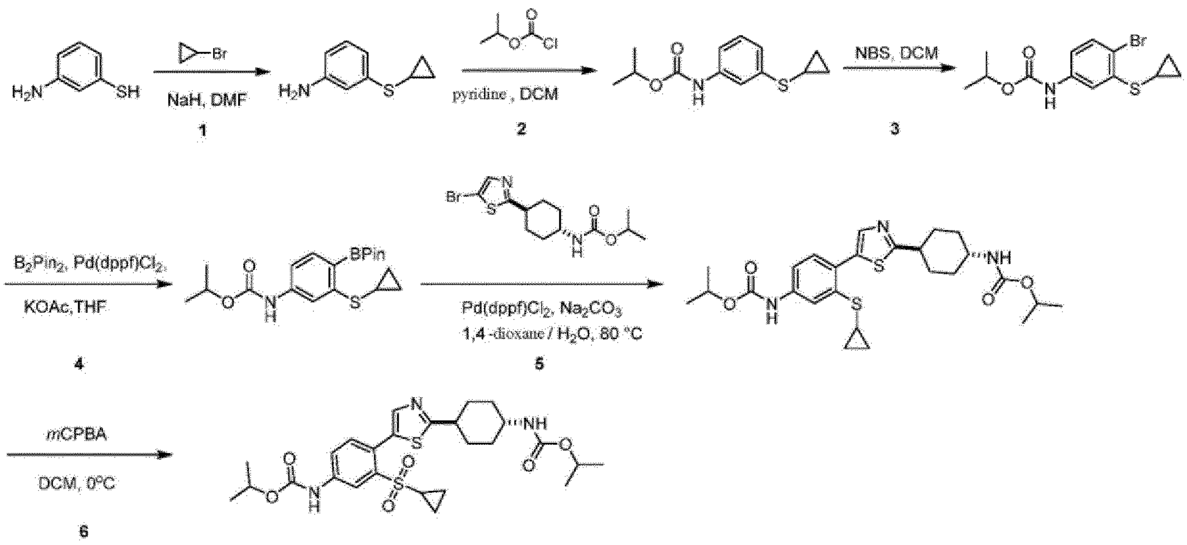

Step 1: Synthesis of 3-cyclopropylsulfanylaniline

3-Aminothiophenol (6 g, 47.93 mmol) was dissolved in N,N-dimethylformamide (60 mL). Under the protection of nitrogen, sodium hydride (2.01 g, 50.30 mmol) was added. The reaction solution was stirred and reacted at 30°C for 1 hour. Thereafter, cyclopropyl bromide (6.09 g, 50.30 mmol) was added into the reaction solution. The reaction solution was stirred and refluxed at 100°C for 5 hours. The completion of the reaction was detected by LCMS. The reaction solution was concentrated to dryness under reduced pressure and then purified by column chromatography (silica, dichloromethane/methanol = 20/1) to obtain 3-cyclopropylsulfanylaniline (1.35 g).
MS m/z (ESI): 166.0 $[M+H]^+$;

Step 2: Synthesis of isopropyl N-(3-cyclopropylsulfanylphenyl)carbamate

3-Cyclopropylsulfanylaniline (1.28 g, 7.75 mmol) and isopropyl chloroformate (1.42 g, 11.62 mmol) were dissolved in dichloromethane (400 mL), and pyridine (3.68 g, 46.47 mmol) was added. The reaction solution was stirred and reacted at 30°C for 1 hour. The completion of the reaction was detected by LCMS. The reaction solution was concentrated to dryness under reduced pressure, and then the resultant was purified by column chromatography (silica, dichloromethane/methanol = 20/1) to obtain isopropyl N-(3-cyclopropylsulfanylphenyl) carbamate (1.6 g).
MS m/z (ESI): 252 $[M+H]^+$;
$^1H$ NMR (400 MHz, DMSO-$d_6$) $\delta$ ppm 9.59 (s, 1H) 7.56 (s, 1H) 7.31-7.14 (m, 2H) 6.95 (dt, J = 7.47, 1.41Hz, 1H) 4.88 (spt, J = 6.27Hz, 1H) 2.20 (tt, J = 7.40, 4.27Hz, 1H) 1.27-1.22 (m, 1H) 1.24 (s, 3H) 1.11-1.02 (m, 2H) 0.66-0.50 (m, 2H).

Step 3: Synthesis of isopropyl N-(4-bromo-3-cyclopropylsulfanyl-phenyl)carbamate

Isopropyl N-(3-cyclopropylsulfanylphenyl)carbamate (300 mg, 1.19 mmol) was dissolved in dichloromethane (20 mL), and then a solution of N-bromosuccinimide (212.44 mg, 1.19 mmol) in dichloromethane (10 mL) was slowly added dropwise into the reaction solution. The reaction solution was stirred and reacted at -20°C for 3 hours. The completion of the reaction was detected by LCMS. The reaction solution was concentrated to dryness under reduced pressure and purified by column chromatography (silica, petroleum ether:dichloromethane = 1:2) to obtain isopropyl N-(4-bromo-3-cyclopropylsulfanyl-phenyl) carbamate (201 mg).
MS m/z (ESI): 330.1, 332.1 $[M+H]^+$.

Step 4: Synthesis of isopropyl N-[3-cyclopropylsulfanyl-4-(4,4,5,5-tetramethyl-1,3,2-dioxaborolan-2-yl)phenyl]carbamate

Isopropyl N-(4-bromo-3-cyclopropylsulfanyl-phenyl)carbamate (600 mg, 1.82 mmol) and bis(pinacolato)diboron (1.85 g, 7.27 mmol) were dissolved in tetrahydrofuran (6 mL). Under the protection of nitrogen, [1,1-bis(diphenylphosphino)ferrocene]dichloropalladium -dichloromethane (148.37 mg, 181.69 μmol) and potassium acetate (534.93 mg, 5.45 mmol) were added. The reaction solution was stirred and reacted at 80°C for 16 hours. The completion of the reaction was detected by LCMS. The reaction solution was added with 9 mL of dichloromethane (3 mL × 3) and 4 mL of water. The organic phase was dried over anhydrous sodium sulfate and filtered. The filtrate was concentrated to dryness under reduced pressure and purified by column chromatography (silica, petroleum ether:ethyl acetate = 5:1) to obtain isopropyl N-[3-cyclopropylsulfanyl-4-(4,4,5,5-tetramethyl-1,3,2-dioxaborolan-2-yl)phenyl] carbamate (400 mg).
MS m/z (ESI): 378 $[M+H]^+$;

Step 5: Synthesis of isopropyl trans-N-[3-cyclopropylsulfanyl-4-[2-[4-(isopropoxycarbonylamino)cyclohexyl]thiazol-5-yl]phenyl]carbamate

Isopropyl N-[3-cyclopropylsulfanyl-4-(4,4,5,5-tetramethyl-1,3,2-dioxaborolan-2-yl) phenyl]carbamate (400 mg, 1.06 mmol) and isopropyl trans-N-[4-(5-bromothiazol-2-yl)cyclohexyl]carbamate (306.80 mg, 883.46 μmol) were dissolved in water (2.3 mL) and dioxane (24 mL). Thereafter, [1,1-bis(diphenylphosphino)ferrocene]dichloropalladium-dichloromethane (72.15 mg, 88.35 μmol) and sodium carbonate (280.91 mg, 2.65 mmol) were added under the condition where nitrogen gas was introduced. The reaction solution was stirred and reacted at 80°C for 16 hours. The completion of the reaction was detected by LCMS. The reaction solution was concentrated to dryness under reduced pressure and purified by column chromatography (silica, petroleum ether:tetrahydrofuran = 2:1) to obtain isopropyl trans-N-[3-cyclopropylsulfanyl-4-[2-[4-(isopropoxycarbonylamino)cyclohexyl]thiazol-5-yl] phenyl]carbamate (300 mg).
MS m/z (ESI): 518.3 $[M+H]^+$;

Step 6: Synthesis of isopropyl trans-N-[3-cyclopropylsulfonyl-4-[2-[4-(isopropoxycarbonylamino)cyclohexyl]thiazol-5-yl]phenyl]carbamate

Isopropyl trans-N-[3-cyclopropylsulfanyl-4-[2-[4-(isopropoxycarbonylamino)cyclohexyl] thiazol-5-yl]phenyl]carbamate (150 mg, 289.74 μmol) was dissolved in dichloromethane (2 mL), and then a solution of m-chloroperoxybenzoic acid (117.65 mg, 579.48 μmol) in dichloromethane (3 mL) was slowly added dropwise into the reaction solution. The reaction solution was stirred and reacted at 30°C for 16 hours. The completion of the reaction was detected by LCMS. The reaction solution was concentrated to dryness under reduced pressure. A saturated sodium carbonate solution (3 mL) and 9 mL of dichloromethane (3 mL × 3) were added for extraction. The organic phase was dried over anhydrous sodium sulfate and filtered. The filtrate was concentrated to dryness under reduced pressure and purified by preparative liquid chromatography (Phenomenexgemini C18 column, 10 μm silica, 50 mm in diameter, 250 mm in length; using decreasingly polar mixtures of water (containing 0.05% aqueous ammonia) and acetonitrile as eluents) to obtain the compound isopropyl trans-N-[3-cyclopropylsulfonyl-4-[2-[4-(isopropoxycarbonylamino)cyclohexyl]thiazol-5-yl] phenyl]carbamate (33.3 mg).

MS m/z (ESI): 5503 $[M+H]^+$;

$^1H$ NMR (400 MHz, DMSO-$d_6$) δ ppm 10.14 (s, 1H) 8.29 (d, J = 2.32Hz, 1H) 7.84-7.66 (m, 2H) 7.48 (d, J = 8.44Hz, 1H) 7.04 (br d, J = 7.82Hz, 1H) 4.98-4.87 (m, 1H) 4.80-4.68 (m, 1H) 3.33-3.26 (m, 1H) 3.01-2.85 (m, 1H) 2.45-2.37 (m, 1H) 2.14 (br d, J = 11.86Hz, 2H) 1.92 (br d, J = 10.27Hz, 2H) 1.65-1.50 (m, 2H) 1.41-1.30 (m, 2H) 1.28 (d, J = 6.24Hz, 6H) 1.17 (d, J = 6.24Hz, 6H) 1.01-0.94 (m, 2H) 0.91-0.85 (m, 2H).

Example 34: Synthesis of isopropyl trans-N-[3-tert-butylsulfonyl-4-[2-[4-(isopropoxycarbonylamino)cyclohexyl]thiazol-5-yl]phenyl]carbamate

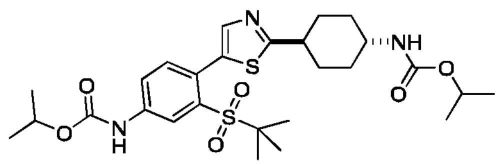

Synthetic Method

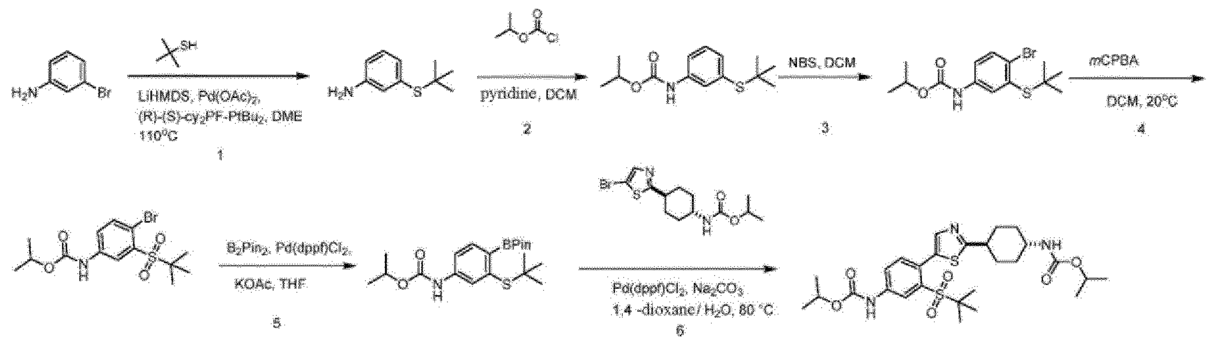


Step 1: Synthesis of 3-tert-butylsulfanylaniline

3-Bromoaniline (4 g, 23.25 mmol, 2.53 mL) and a solution (2M, 27.90 mL) of lithium bis(trimethylsilyl)amide (2M) in tetrahydrofuran were dissolved in ethylene glycol dimethyl ether (100 mL). Under the protection of nitrogen, palladium acetate (261.02 mg, 1.16 mmol), (R)-1-[(S)-2-(dicyclohexylphosphino)ferrocenyl]ethyl-tert-butylphosphine (644.74 mg, 1.16 mmol) and tert-butyl mercaptan (2.10 g, 23.25 mmol, 2.62 mL) were added. The reaction solution appeared as a black suspension, and was stirred and reacted at 110°C for 48 h. The completion of the reaction was detected by LCMS. The reaction solution was concentrated to dryness under reduced pressure. The resultant was purified by column chromatography (dichloromethane = 1) to obtain the compound 3-tert-butylsulfanylaniline (3.68 g, 20.30 mmol).

MS m/z (ESI): 182.1 $[M+H]^+$;

Step 2: Synthesis of isopropyl N-(3-tert-butylsulfanylphenyl)carbamate

3-Tert-butylsulfanylaniline (2 g, 11.03 mmol) was dissolved in anhydrous dichloromethane (30 mL). Under the protection of nitrogen, pyridine (5.24 g, 66.19 mmol, 5.34 mL) and isopropyl chloroformate (4.06 g, 33.09 mmol, 4.59 mL) were added. The reaction solution appeared as a yellow suspension, and was stirred and reacted at 25°C for 2 h. The completion of the reaction was detected by LCMS. The reaction solution was concentrated to dryness under reduced pressure. The resultant was purified by column chromatography (petroleum ether:dichloromethane = 1:2) to obtain the compound isopropyl N-(3-tert-butylsulfanylphenyl)carbamate (2 g, 7.48 mmol).

MS m/z (ESI): 268.2 $[M+H]^+$;

Step 3: Synthesis of isopropyl N-(4-bromo-3-tert-butylsulfanyl-phenyl)carbamate

Isopropyl N-(3-tert-butylsulfanylphenyl)carbamate (2 g, 7.48 mmol) was dissolved in anhydrous dichloromethane (30 mL), and a solution of N-bromosuccinimide (1.33 g, 7.48 mmol) in anhydrous dichloromethane (30 mL) was added dropwise. The reaction solution appeared as a yellow suspension, and was stirred and reacted at -30°C for 2 h. The completion of the reaction was detected by LCMS. The reaction solution was concentrated to dryness under reduced pressure. The resultant was purified by column chromatography (petroleum ether:ethyl acetate = 5:1) to obtain the compound isopropyl N-(4-bromo-3-tert-butylsulfanyl-phenyl)carbamate (360 mg, 1.04 mmol).

MS m/z (ESI): 347.0 $[M+H]^+$.

Step 4: Synthesis of isopropyl N-(4-bromo-3-tert-butylsulfonyl-phenyl)carbamate

Isopropyl N-(4-bromo-3-tert-butylsulfanyl-phenyl)carbamate (200 mg, 577.56 μmol) was dissolved in anhydrous dichloromethane (3 mL), and m-chloroperoxybenzoic acid (299.00 mg, 1.73 mmol) was added. The reaction solution appeared as a yellow suspension, and was stirred and reacted at 20°C for 3 h. The completion of the reaction was detected by LCMS. The reaction solution was added with saturated sodium carbonate solution (15 mL) and extracted with dichloromethane (30 mL). The organic layer was dried over anhydrous magnesium sulfate and subjected to suction filtration. The filtrate was concentrated to dryness under reduced pressure. The resultant was purified by column chromatography (petroleum ether:ethyl acetate = 5:1) to obtain the compound isopropyl N-(4-bromo-3- tert-butylsulfonyl-phenyl)carbamate (130 mg, 343.66 μmol).

MS m/z (ESI): 400.0, 402.0 $[M+Na]^+$;

Step 5: Synthesis of isopropyl N-[3-tert-butylsulfonyl-4-(4,4,5,5-tetramethyl-1,3,2-dioxaborolan-2-yl)phenyl]carbamate

Isopropyl N-(4-bromo-3-tert-butylsulfonyl-phenyl)carbamate (130 mg, 343.66 μmol) and bis(pinacolato)diboron (349.07 mg, 1.37 mmol) were dissolved in anhydrous tetrahydrofuran (4 mL). Under the protection of nitrogen, [1,1-bis(diphenylphosphino) ferrocene]dichloropalladium - dichloromethane (28.06 mg, 34.37 μmol) and potassium acetate (101.18 mg, 1.03 mmol) were added. The reaction solution appeared as a red suspension, and was stirred and reacted at 80°C for 7 h. The completion of the reaction was detected by LCMS. The reaction solution was concentrated to dryness under reduced pressure. The resultant was purified by column chromatography (petroleum ether:ethyl acetate = 5:1) to obtain the compound isopropyl N-[3-tert-butylsulfonyl-4-(4,4,5,5-tetramethyl-1,3,2-dioxaborolan-2-yl) phenyl]carbamate (100 mg, 235.10 μmol).

MS m/z (ESI): 426.2 $[M+H]^+$;

Step 6: Synthesis of isopropyl trans-N-[3-tert-butylsulfonyl-4-[2-[4-(isopropoxycarbonylamino)cyclohexyl]thiazol-5-yl]phenyl]carbamate

Isopropyl N-[3-tert-butylsulfonyl-4-(4,4,5,5-tetramethyl-1,3,2-dioxaborolan-2-yl)phenyl] carbamate (100 mg, 235.10 μmol) and isopropyl trans-N-[4-(5-bromothiazol-2-yl)cyclohexyl]carbamate (81.64 mg, 235.10 μmol) were dissolved in anhydrous dioxane (3 mL). Under the protection of nitrogen, [1,1-bis(diphenylphosphino) ferrocene]dichloropalladium - dichloromethane (19.20 mg, 23.51 μmol), sodium carbonate (49.84 mg, 470.21 μmol) and water (300 μL) were added. The reaction solution appeared as a black suspension, and was stirred and reacted at 80°C for 7 h. The completion of the reaction was detected by LCMS. The reaction solution was concentrated to dryness under reduced pressure. The resultant was added with water (10 mL), and the mixture was extracted with ethyl acetate (20 mL). The organic layer was dried over anhydrous magnesium sulfate and subjected to suction filtration. The filtrate was concentrated to dryness under reduced pressure. The resultant was purified by preparative liquid chromatography

(PhenomenexGeminiC18 column, 10 μm silica, 50 mm in diameter, 250 mm in length; using decreasingly polar mixtures of water (containing 0.05% aqueous ammonia) and acetonitrile as eluents) to obtain the compound isopropyl trans-N-[3-tert-butylsulfonyl-4-[2-[4-(isopropoxycarbonylamino) cyclohexyl]thiazol-5-yl]phenyl]carbamate (32.94 mg, 58.22 μmol).

MS m/z (ESI): 566.1 $[M+H]^+$;

1H NMR (400MHz, DMSO-d6) δ ppm 10.13 (s, 1H), 8.31 - 8.24 (m, 1H), 7.81 - 7.74 (m, 1H), 7.62 - 7.57 (m, 1H), 7.47 - 7.41 (m, 1H), 7.05 - 6.99 (m, 1H), 4.97 - 4.89 (m, 1H), 4.79 - 4.70 (m, 1H), 3.32 - 3.31 (m, 1H), 2.96 - 2.86 (m, 1H), 2.16 - 2.07 (m, 2H), 1.96 - 1.87 (m, 2H), 1.62 - 1.48 (m, 2H), 1.40 - 1.30 (m, 2H), 1.28 (d, J=6.3 Hz, 6H), 1.17 (d, J=6.3 Hz, 6H), 1.11 (s, 9H).

Example 35: Synthesis of isopropyl trans-N-[4-[5-[2-isopropylsulfonyl-4-[(2-oxo-1H-pyridin-3-yl)amino]phenyl]thiazol-2-yl]cyclohexyl]carbamate

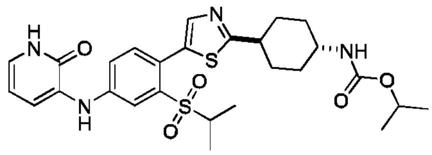

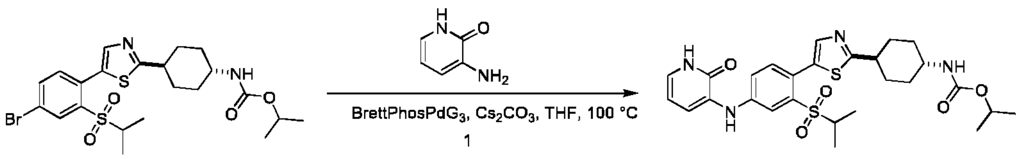


The intermediate isopropyl trans-N-[4-[5-(4-bromo-2-isopropylsulfonyl-phenyl)thiazol-2-yl]cyclohexyl]carbamate (50 mg, 94.43 μmol) in Example 36 was dissolved in tetrahydrofuran (1 mL). 3-Amino-1H-pyridin-2-one (12.48 mg, 113.31 μmol) was added. Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (4.28 mg, 4.72 μmol) and cesium carbonate (92.30 mg, 283.28 μmol) were added. The reaction solution was stirred at 100°C for 2 hours. LCMS showed that the reaction was complete. The reaction solution was filtered. The filtrate was evaporated to dryness via rotary evaporation to remove tetrahydrofuran. The resultant was added with water (15 mL), and the mixture was extracted three times with ethyl acetate (15 mL × 3). The organic phase was dried over anhydrous sodium sulfate, and evaporated to dryness via rotary evaporation under reduced pressure. The resultant was purified by preparative high-performance liquid chromatography (YMC-Actus Triart C18 column, 5 μm silica, 30 mm in diameter, 150 mm in length) (using decreasingly polar mixtures of water (containing 0.05% aqueous ammonia) and acetonitrile (38% to 58%) as eluents) to obtain isopropyl trans-N-[4-[2-[4-(isopropoxycarbonylamino)cyclohexyl]thiazol-5-yl]-3-isopropylsulfinyl-phenyl]carbamate (1.02 mg).

MS m/z (ESI): 559.0 $[M+H]^+$;

$^1H$ NMR: (400 MHz, METHANOL-$d_4$) δ ppm 7.91 (d, J = 1.96 Hz, 1H) 7.74 (s, 1H) 7.50-7.44 (m, 3H) 7.07 (d, J = 6.60 Hz, 1H) 6.42 (t, J = 6.97 Hz, 1H) 5.42-5.35 (m, 1H) 3.49-3.40 (m, 1H) 3.12-2.90 (m, 2H) 2.28-2.23 (m, 2H) 2.15-2.05 (m, 2H) 1.78-1.68 (m, 2H) 1.50-1.42 (m, 2H) 1.35-1.20 (m, 6H) 1.19 (d, J = 12.00 Hz, 6H).

Example 36: Synthesis of isopropyl trans-(4-(5-(4-((1,5-dimethyl-1H-pyrazol-3-yl)amino)-2-(isopropylsulfonyl)phenyl)thiazol-2-yl)cyclohexyl)carbamate

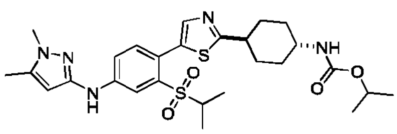

Synthetic Method

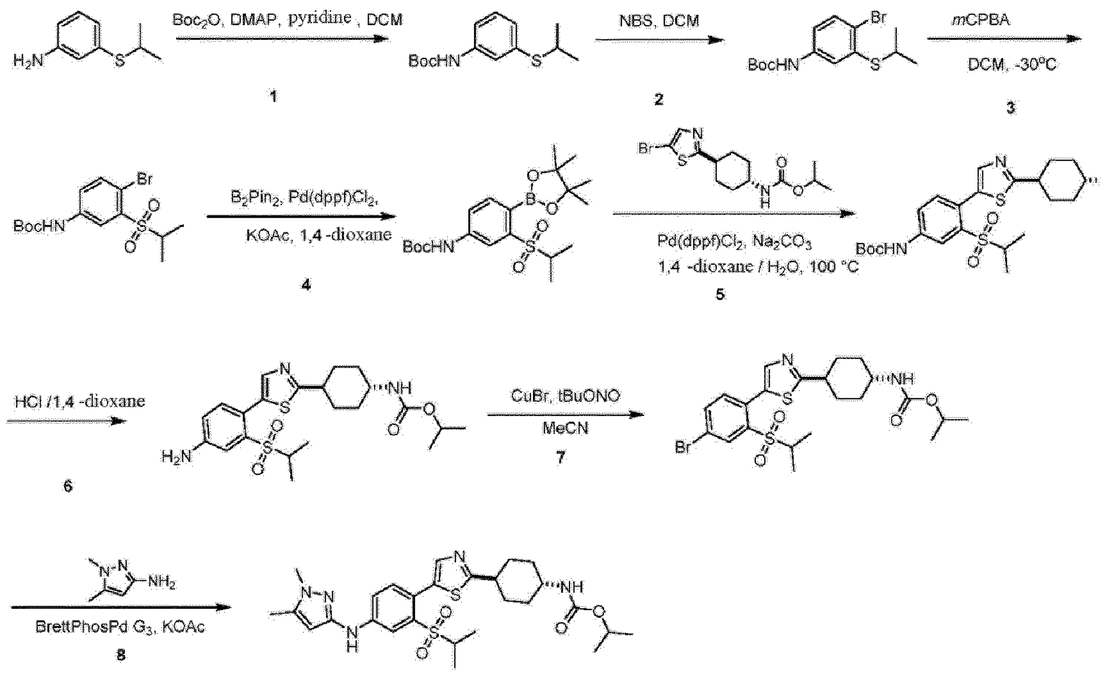


Step 1: Synthesis of tert-butyl (3-(isopropylthio)phenyl)carbamate

3-Isopropylsulfanylaniline (15 g, 44.88 mmol) was dissolved in anhydrous dichloromethane (500 mL), and di-tert-butyl dicarbonate (30.81 g, 141.18 mmol, 32.43 mL), pyridine (18.85 g, 235.31 mmol, 18.48 mL) and 4-dimethylaminopyridine (14.37 g, 117.65 mmol) were added at 0°C. The reaction solution was stirred and reacted at 25°C for 5 hours. The completion of the reaction was detected by LCMS. The reaction solution was concentrated under reduced pressure to remove the solvent dichloromethane, and purified by column chromatography (silica, petroleum ether: ethyl acetate = 10:1) to obtain tert-butyl (3-(isopropylthio)phenyl)carbamate (15 g).

MS m/z (ESI): 212.1 $[M+H-t-Bu]^+$;

Step 2: Synthesis of tert-butyl (4-bromo-3-(isopropylthio)phenyl)carbamate

Tert-butyl (3-(isopropylthio)phenyl)carbamate (5.0 g, 18.70 mmol) was dissolved in dichloromethane (200 mL), and N-bromosuccinimide (3.33 g, 18.70 mmol) was added into the reaction system. The reaction solution was stirred at -30°C for 3 hours. The completion of the reaction was detected by LCMS. The fraction was concentrated to dryness under reduced pressure, and the resultant was purified by column chromatography (silica, petroleum ether:ethyl acetate = 20:1) to obtain tert-butyl (4-bromo-3-(isopropylthio)phenyl)carbamate (2.3 g).

MS m/z (ESI): 290.0, 292.0 $[M+H-t-Bu]^+$;

$^1$H NMR (400MHz, DMSO-$d_6$) $\delta$ ppm 9.51 (s, 1H), 7.58 (d, J = 2.3 Hz, 1H), 7.47 (d, J = 8.8 Hz, 1H), 7.25 (dd, J = 2.3, 8.5 Hz, 1H), 3.43 (spt, J = 6.5 Hz, 1H), 1.47 (s, 9H), 1.31 (d, J = 6.8 Hz, 6H).

Step 3: Synthesis of tert-butyl (4-bromo-3-(isopropylsulfonyl)phenyl)carbamate

Tert-butyl (4-bromo-3-(isopropylthio)phenyl)carbamate (1.0 g, 2.89 mmol) was dissolved in anhydrous dichloromethane (25 mL), and a solution of m-chloroperoxybenzoic acid (996.68 mg, 5.78 mmol, purity: 85%) in anhydrous dichloromethane (25 mL) was added at -30°C. The reaction solution was reacted at -30°C for 3 hours. LCMS showed that the reaction was complete. A saturated aqueous sodium carbonate solution (50 mL) was added into the reaction solution, and then the reaction solution was extracted with dichloromethane (100 mL) twice. The dichloromethane phases were combined, dried over anhydrous sodium sulfate and subjected to suction filtration. The resultant was purified by column chromatography (silica, petroleum ether: ethyl acetate = 20:1) to obtain tert-butyl (4-bromo-3-(isopropylsulfonyl)phenyl)carbamate (0.8 g).

MS m/z (ESI): 322.0, 324.0 $[M+H-t-Bu]^+$;

$^1$H NMR (400MHz, DMSO-$d_6$) $\delta$ ppm 9.90 (s, 1H), 8.31 (d, J = 2.6 Hz, 1H), 7.78 (d, J = 8.8 Hz, 1H), 7.61 (dd, J = 2.6, 8.7 Hz, 1H), 3.82-3.75 (m, 1H), 1.48 (s, 9H), 1.18 (d, J = 6.9 Hz, 6H).

Step 4: Synthesis of tert-butyl (3-(isopropylsulfonyl)-4-(4,4,5,5-tetramethyl-1,3,2-dioxaborolan-2-yl)phenyl)carbamate

Tert-butyl (4-bromo-3-(isopropylsulfonyl)phenyl)carbamate (800 mg, 2.11 mmol) and bis(pinacolato)diboron (2.69 g, 10.57 mmol) were dissolved in anhydrous tetrahydrofuran (25 mL). Under the protection of nitrogen, potassium acetate (207.55 mg, 2.11 mmol) and [1,1-bis(diphenylphosphino)ferrocene]dichloropalladium - dichloromethane (863.52 mg, 1.06 mmol) were added. The reaction solution was stirred at 80°C for 16 hours. LCMS showed that the reaction was complete. The reaction system was evaporated to dryness via rotary evaporation, and then the resultant was purified by column chromatography (silica, petroleum ether:ethyl acetate = 10:1) to obtain tert-butyl (3-(isopropylsulfonyl)-4-(4,4,5,5-tetramethyl-1,3,2-dioxaborolan-2-yl)phenyl)carbamate (560 mg).

MS m/z (ESI): 443.3 $[M+H+NH_3]^+$;

Step 5: Synthesis of isopropyl trans-N-[4-[2-[4-(tert-butoxycarbonylamino)-2-isopropylsulfonyl-phenyl]thiazol-5-yl]cyclohexyl]carbamate

Isopropyl trans-N-[4-(5-bromothiazol-2-yl)cyclohexyl]carbamate (961.18 mg, 2.77 mmol) and tert-butyl (3-(isopropylsulfonyl)-4-(4,4,5,5-tetramethyl-1,3,2-dioxaborolan-2-yl) phenyl)carbamate (1.18 g, 2.77 mmol) were dissolved in an aqueous sodium carbonate solution (2M, 4.15 mL, 8.30 mmol) and 1,4-dioxane (30 mL). Under the protection of nitrogen, [1,1-bis(diphenylphosphino)ferrocene]dichloropalladium - dichloromethane (113.01 mg, 138.39 μmol) was added. The reaction solution was stirred at 100°C for 16 hours. LCMS showed that the reaction was complete. The reaction solution was filtered. The filtrate was evaporated to dryness via rotary evaporation to remove 1,4-dioxane, added with water (10 mL), and extracted three times with dichloromethane (10 mL × 3). The resultant was dried over anhydrous sodium sulfate and then purified by thin-layer chromatography (silica, petroleum ether/ethyl acetate = 5:1) to obtain isopropyl trans-N-[4-[2-[4-(tert-butoxycarbonylamino)-2-isopropylsulfonyl-phenyl]thiazol-5-yl]cyclohexyl]carbamate (0.8 g).

MS m/z (ESI): 566.3 $[M+H]^+$;

Step 6: Synthesis of isopropyl trans-(4-(5-(4-amino-2-(isopropylsulfonyl)phenyl)thiazol-2-yl)cyclohexyl)carbamate

Isopropyl trans-N-[4-[2-[4-(tert-butoxycarbonylamino)-2-isopropylsulfonyl-phenyl] thiazol-5-yl]cyclohexyl]carbamate (0.8 g, 1.41 mmol) was dissolved in anhydrous methanol (20 mL). Hydrochloric acid/dioxane (4M, 3.54 mL, 14.14 mmol) was added. The reaction solution was stirred at 25°C for 1 hour. LCMS showed that the reaction was complete. The reaction system was evaporated to dryness via rotary evaporation, and then the resultant was added with 1M aqueous sodium hydroxide solution (10 mL), followed by liquid-liquid extraction (extraction with 120 mL of ethyl acetate three times) to obtain the compound isopropyl trans-(4-(5-(4-amino-2-(isopropylsulfonyl)phenyl)thiazol-2-yl)cyclohexyl)carbamate (560 mg).

MS m/z (ESI): 466.2 $[M+H]^+$;

Step 7: Synthesis of isopropyl trans-(4-(5-(4-bromo-2-(isopropylsulfonyl)phenyl)thiazol-2-yl)cyclohexyl)carbamate

Isopropyl trans-(4-(5-(4-amino-2-(isopropylsulfonyl)phenyl)thiazol-2-yl)cyclohexyl) carbamate (0.5 g, 1.07 mmol) was dissolved in anhydrous acetonitrile (50 mL). Under the protection of nitrogen, tert-butyl nitrite (166.1 mg, 1.61 mmol) was slowly added at 0°C. The reaction solution was stirred at 25°C for 30 minutes. Under the protection of nitrogen, cuprous bromide (184.85 mg, 1.29 mmol) was added. The reaction solution was continued to be stirred at 25°C for 16 hours. LCMS showed that the reaction was complete. The reaction system was evaporated to dryness via rotary evaporation, and then the resultant was added with a saturated ammonium chloride solution, followed by liquid-liquid extraction (extraction with 120 mL of dichloromethane three times). The dichloromethane phases were combined, dried over anhydrous sodium sulfate, and subjected to suction filtration. The resultant was purified by column chromatography (silica, dichloromethane/methanol = 20/1) to obtain isopropyl trans-(4-(5-(4-bromo-2-(isopropylsulfonyl)phenyl)thiazol-2-yl)cyclohexyl)carbamate (350 mg).

MS m/z (ESI): 529.2, 531.2 $[M+H]^+$;

Step 8: Isopropyl trans-(4-(5-(4-((1,5-dimethyl-1H-pyrazol-3-yl)amino)-2-(isopropylsulfonyl)phenyl)thiazol-2-yl)cyclohexyl)carbamate

Isopropyl trans-(4-(5-(4-bromo-2-(isopropylsulfonyl)phenyl)thiazol-2-yl)cyclohexyl) carbamate (50 mg, 94.43 μmol) and 1,5-dimethyl-1H-pyrazol-3-amine (26.24 mg, 236.07 μmol) were dissolved in anhydrous tetrahydrofuran (4 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (4.28 mg, 4.72 μmol) and potassium acetate (27.8 mg, 283.28 μmol)

were added. The reaction solution was stirred at 85°C for 16 hours. LCMS showed that the reaction was complete. The reaction system was evaporated to dryness via rotary evaporation, and then the resultant was purified by preparative high-performance liquid chromatography (Phenomenex Gemini-NX column, 10 μm silica, 50 mm in diameter, 250 mm in length) (using decreasingly polar mixtures of water (containing 0.05% aqueous ammonia) and acetonitrile (50% to 70%) as eluents) and lyophilized to obtain isopropyl trans-(4-(5-(2-(isopropylsulfonyl)-4-((5-methyl-1H-pyrazol-3-yl)amino)phenyl)thiazol-2-yl) cyclohexyl)carbamate (4.34 mg).

MS m/z (ESI): 560.1 $[M+H]^+$;

$^1$H NMR (400MHz, DMSO-$d_6$) δ ppm 9.07 (s, 1H), 7.95 (d, J = 2.6 Hz, 1H), 7.74 (dd, J = 2.4, 8.5 Hz, 1H), 7.64 (s, 1H), 7.35 (d, J = 8.4 Hz, 1H), 7.02 (br d, J = 7.9 Hz, 1H), 5.66 (s, 1H), 4.80-4.68 (m, 1H), 3.64 (s, 3H), 3.31 - 3.20 (m, 1H), 2.97 - 2.81 (m, 2H), 2.21 (s, 3H), 2.12 (br d, J = 12.7 Hz, 2H), 1.91 (br d, J = 10.5 Hz, 2H), 1.62 - 1.48 (m, 2H), 1.39 - 1.27 (m, 2H), 1.16 (d, J = 6.2 Hz, 6H), 1.05 (d, J = 6.7 Hz, 6H).

Example 37: Synthesis of isopropyl trans-(4-(5-(2-(isopropylsulfonyl)-4-((5-methylisoxazol-3-yl)amino)phenyl)thiazol-2-yl)cyclohexyl)carbamate

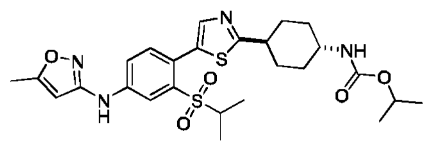

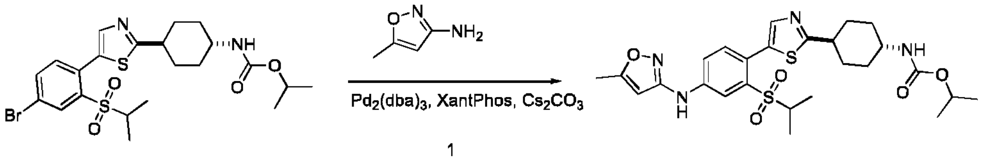


Isopropyl trans-(4-(5-(4-bromo-2-(isopropylsulfonyl)phenyl)thiazol-2-yl)cyclohexyl) carbamate (50 mg, 94.43 μmol) and 3-amino-5-methylisoxazole (23.16 mg, 236.07 μmol) were dissolved in anhydrous 1,4-dioxane (4 mL). Under the protection of nitrogen, tris(dibenzylideneacetone)dipalladium (4.32 mg, 4.72 μmol), 4,5-bis(diphenylphosphino)-9,9-dimethylxanthene (2.73 mg, 4.72 μmol) and cesium carbonate (92.3 mg, 283.28 μmol) were added. The reaction solution was stirred at 85°C for 1 hour. LCMS showed that the reaction was complete. The reaction system was evaporated to dryness via rotary evaporation, and then the resultant was subjected to liquid-liquid extraction (extraction with 12 mL of ethyl acetate three times) to obtain a yellow solid compound. The resultant was purified by preparative high-performance liquid chromatography (Phenomenex Gemini-NX column, 10 μm silica, 50 mm in diameter, 250 mm in length) (using decreasingly polar mixtures of water (containing 0.05% aqueous ammonia) and acetonitrile (45% to 65%) as eluents) and lyophilized to obtain isopropyl trans-(4-(5-(2-(isopropylsulfonyl)-4-((5-methylisoxazol-3-yl)amino)phenyl)thiazol-2-yl)cyclohexyl)carbamate (15.85 mg).

MS m/z (ESI): 547.1 $[M+H]^+$;

$^1$H NMR (400MHz, DMSO-$d_6$) δ ppm 9.77 (s, 1H), 8.14 (d, J = 2.4 Hz, 1H), 7.80 (dd, J = 2.5, 8.5 Hz, 1H), 7.72-7.65 (m, 1H), 7.49 (d, J = 8.3 Hz, 1H), 7.02 (br d, J = 7.7 Hz, 1H), 5.96 (d, J = 0.7 Hz, 1H), 4.80-4.70 (m, 1H), 3.32-3.25 (m, 1H), 3.01-2.81 (m, 2H), 2.37 (s, 3H), 2.13 (br d, J = 11.7 Hz, 2H), 1.91 (br d, J = 10.0 Hz, 2H), 1.65-1.50 (m, 2H), 1.41-1.26 (m, 2H), 1.16 (d, J = 6.2 Hz, 6H), 1.06 (d, J = 6.7 Hz, 6H).

Example 38: Synthesis of isopropyl trans-N-[4-[5-[2-isopropylsulfonyl]-4-[(3-methylisothiazol-5-yl)amino]phenyl]thiazol-2-yl]cyclohexyl]carbamate

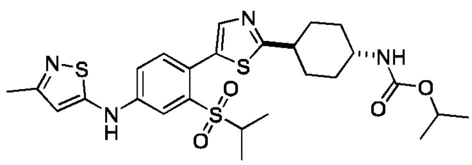

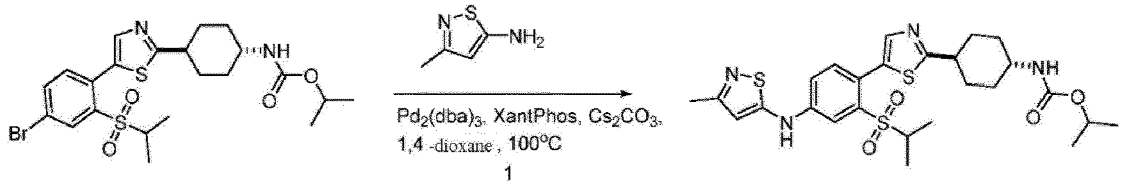


Isopropyl trans-N-[4-[5-(4-bromo-2-isopropylsulfonyl-phenyl)thiazol-2-yl]cyclohexyl] carbamate (20 mg, 37.77 μmol) and 3-methyl-5-aminoisothiazole (10.78 mg, 94.43 μmol) were dissolved in anhydrous dioxane (4 mL). Under the protection of nitrogen, tris(dibenzylideneacetone)dipalladium (1.73 mg, 1.89 μmol), 4,5-bis(diphenylphosphino)-9,9-dimethylxanthene (1.09 mg, 1.89 μmol) and cesium carbonate (36.92 mg, 113.31 μmol) were added. The reaction solution appeared as a black suspension, and was stirred and reacted at 100°C for 16 h. The completion of the reaction was detected by LCMS. The reaction solution was concentrated to dryness under reduced pressure, added with water (10 mL), and extracted with ethyl acetate (20 mL). The organic layer was dried over anhydrous magnesium sulfate and subjected to suction filtration. The filtrate was concentrated to dryness under reduced pressure. The resultant was purified by preparative liquid chromatography (PhenomenexGeminiC18 column, 10 μm silica, 50 mm in diameter, 250 mm in length; using decreasingly polar mixtures of water (containing 0.225% formic acid) and acetonitrile as eluents) to obtain the compound isopropyl trans-N-[4-[5-[2-isopropylsulfonyl]-4-[(3-methylisothiazol-5-yl)amino]phenyl] thiazol-2-yl]cyclohexyl]carbamate (2.62 mg, 4.66 μmol).

MS m/z (ESI): 563.1 [M+H]$^+$;

$^1$H NMR (400MHz, DMSO-d$_6$) δ ppm 10.50 (s, 1H), 7.81-7.76 (m, 1H), 7.72 (s, 1H), 7.57-7.53 (m, 1H), 7.45-7.40 (m, 1H), 7.08-7.01 (m, 1H), 6.74 (s, 1H), 4.81-4.69 (m, 1H), 3.33-3.31 (m, 1H), 3.00-2.85 (m, 2H), 2.33 (s, 3H), 2.20-2.09 (m, 2H), 1.97-1.87 (m, 2H), 1.64-1.50 (m, 2H), 1.41-1.27 (m, 2H), 1.17 (d, J = 6.2 Hz, 6H), 1.08 (d, J = 6.7 Hz, 6H).

Example 39: Synthesis of isopropyl trans-(4-(5-(2-(isopropylsulfonyl)-4-((1-methyl-1H-1,2,3-triazol-4-yl)amino)phenyl)thiazol-2-yl)cyclohexyl)carbamate

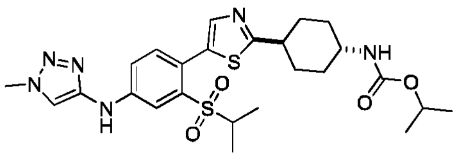

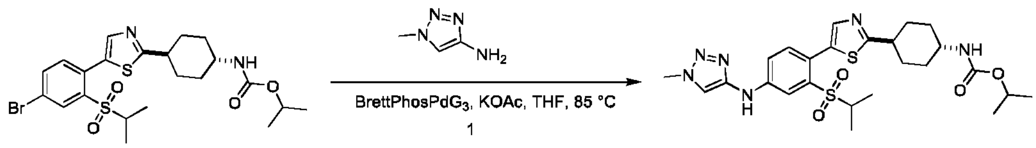


Isopropyl trans-(4-(5-(4-bromo-2-(isopropylsulfonyl)phenyl)thiazol-2-yl)cyclohexyl) carbamate (50 mg, 94.43 μmol) and 1-methyl-1H-1,2,3-triazol-4-amine (236.07 mg, 236.07 μmol) were dissolved in anhydrous tetrahydrofuran (4 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (4.28 mg, 4.72 μmol) and potassium acetate (27.8 mg, 283.28 μmol) were added. The reaction solution was stirred at 85°C for 16 hours. LCMS showed that the reaction was complete. The reaction system was evaporated to dryness via rotary evaporation, and then the resultant was added with water (3 mL), followed by liquid-liquid extraction (extraction with 12 mL of ethyl acetate three times) to obtain a yellow solid compound. The resultant was purified by preparative high-performance liquid chromatography (Phenomenex Gemini-NX column, 10 μm silica, 30 mm in diameter, 100 mm in length) (using decreasingly polar mixtures of water (containing 0.225% formic acid) and acetonitrile (26% to 66%) as eluents) and lyophilized to obtain isopropyl trans-(4-(5-(2-(isopropylsulfonyl)-4-((1-methyl-1H-1,2,3-triazol-4-yl)amino)phenyl)thiazol-2-yl)cyclohexyl)carbamate (8.4 mg).

MS m/z (ESI): 547.1 [M+H]$^+$;

$^1$H NMR (400MHz, DMSO-d$_6$) δ ppm 9.22 (s, 1H), 7.87-7.80 (m, 2H), 7.65 (s, 1H), 7.47 (dd, J = 2.5, 8.5 Hz, 1H), 7.38 (d, J = 8.4 Hz, 1H), 7.02 (br d, J = 7.7 Hz, 1H), 4.80-4.70 (m, 1H), 4.03 (s, 3H), 3.35-3.28 (m, 1H), 2.98-2.80 (m, 2H), 2.12 (br d, J = 12.0 Hz, 2H), 1.91 (br d, J = 9.9 Hz, 2H), 1.62-1.49 (m, 2H), 1.41-1.27 (m, 2H), 1.16 (d, J = 6.2 Hz, 6H), 1.05 (d, J = 6.7 Hz, 6H).

Example 40: Synthesis of isopropyl trans-(4-(5-(2-(isopropylsulfonyl)-4-((5-methyl-1H-pyrazol-3-yl)amino)phenyl)thiazol-2-yl)cyclohexyl)carbamate

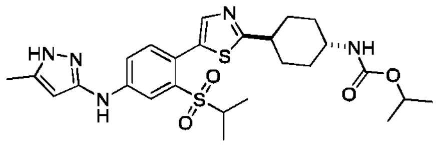

Synthetic Method

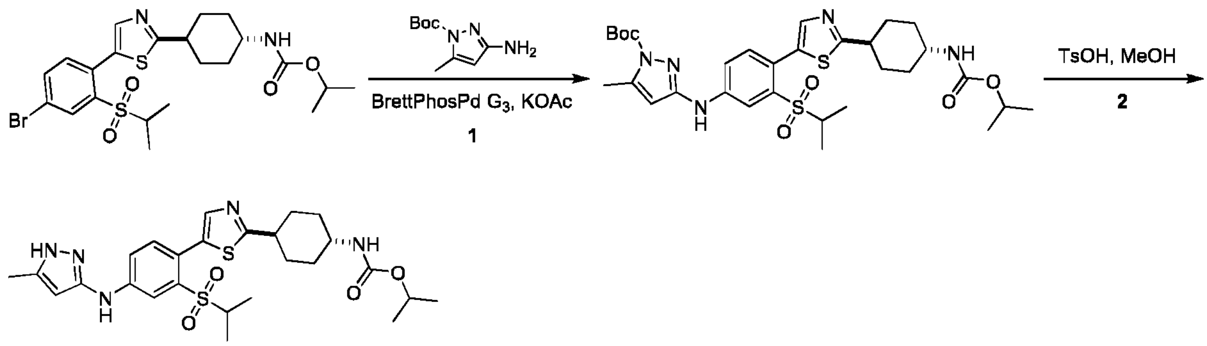


Step 1: Synthesis of tert-butyl trans-3-((4-(2-(4-((isopropoxycarbonyl)amino)cyclohexyl) thiazol-5-yl)-3-(isopropylsulfonyl)phenyl)amino)-5-methyl-1H-pyrazole-1-carboxylate

Isopropyl trans-(4-(5-(4-bromo-2-(isopropylsulfonyl)phenyl)thiazol-2-yl)cyclohexyl) carbamate (50 mg, 94.43 μmol) and tert-butyl 3-amino-5-methyl-pyrazole-1-carboxylate (46.56 mg, 236.07 μmol) were dissolved in anhydrous tetrahydrofuran (4 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (4.28 mg, 4.72 μmol) and potassium acetate (27.8 mg, 283.28 μmol) were added. The reaction solution was stirred at 85°C for 16 hours. LCMS showed that the reaction was complete. The reaction system was evaporated to dryness via rotary evaporation, and then the resultant was subjected to liquid-liquid extraction (extraction with 12 mL of ethyl acetate three times) to obtain tert-butyl trans-3-((4-(2-(4-((isopropoxycarbonyl)amino)cyclohexyl)thiazol-5-yl)-3-(isopropylsulfonyl) phenyl)amino)-5-methyl-1H-pyrazole-1-carboxylate (50 mg, crude product).

MS m/z (ESI): 646.3 $[M+H]^+$;

Step 2: Synthesis of isopropyl trans-(4-(5-(2-(isopropylsulfonyl)-4-((5-methyl-1H-pyrazol-3-yl)amino)phenyl)thiazol-2-yl)cyclohexyl)carbamate

Tert-butyl trans-3-((4-(2-(4-((isopropoxycarbonyl)amino)cyclohexyl)thiazol-5-yl)-3-(isopropylsulfonyl)phenyl)amino)-5-methyl-1H-pyrazole-1-carboxylate (50 mg, 77.42 μmol) was dissolved in methanol (3 mL), and p-toluenesulfonic acid (73.63 mg, 387.10 μmol) was added. The reaction solution was stirred at 50°C for 2 hours. LCMS showed that the reaction was complete. The reaction system was evaporated to dryness via rotary evaporation. The resultant was added with saturated sodium bicarbonate solution (5 mL), and then the mixture was extracted with dichloromethane (15 mL) three times. The dichloromethane phases were combined, dried over anhydrous sodium sulfate, and subjected to suction filtration. The resultant was purified by preparative high-performance liquid chromatography (Phenomenex Gemini-NX column, 5 μm silica, 30 mm in diameter, 150 mm in length) (using decreasingly polar mixtures of water (containing 0.05% aqueous ammonia and 10 mmol of ammonium bicarbonate) and acetonitrile (45% to 65%) as eluents) and lyophilized to obtain isopropyl trans-(4-(5-(2-(isopropylsulfonyl)-4-((5-methyl-1H-pyrazol-3-yl)amino)phenyl)thiazol-2-yl) cyclohexyl)carbamate (5.47 mg).

MS m/z (ESI): 546.1 $[M+H]^+$;

$^1H$ NMR (400MHz, DMSO-$d_6$) δ ppm 11.88 (s, 1H), 9.08 (s, 1H), 8.10 (s, 1H), 7.71-7.62 (m, 2H), 7.34 (d, J = 8.5 Hz, 1H), 7.03 (br d, J = 7.5 Hz, 1H), 5.63 (s, 1H), 4.81-4.68 (m, 1H), 3.32-3.26 (m, 1H), 2.98-2.78 (m, 2H), 2.20 (s,

3H), 2.12 (br d, J = 11.8 Hz, 2H), 1.91 (br d, J = 9.8 Hz, 2H), 1.62-1.47 (m, 2H), 1.40-1.27 (m, 2H), 1.16 (d, J = 6.3 Hz, 6H), 1.05 (d, J = 6.8 Hz, 6H).

Example 41: Synthesis of isopropyl trans-N-[4-[5-[4-(isopropoxycarbonylamino) cyclohexyl]-1,3,4-thiadiazol-2-yl]-3-(isopropylsulfonimidoyl)phenyl]carbamate

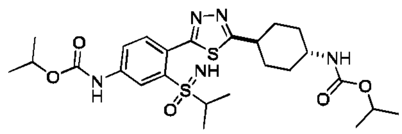

Synthetic Method

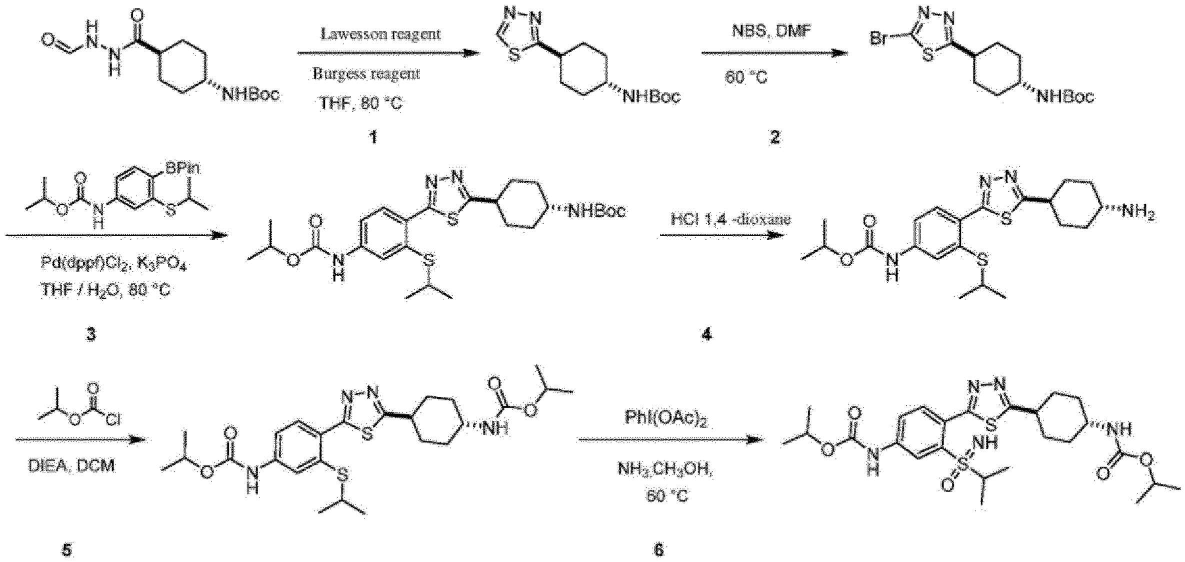


Step 1: Synthesis of tert-butyl trans-N-[4-(1,3,4-thiadiazol-2-yl)cyclohexyl]carbamate

Tert-butyl trans-N-[4-(formamidocarbamoyl)cyclohexyl]carbamate (10 g, 35.05 mmol) was dissolved in anhydrous tetrahydrofuran (250 mL), and then Lawesson reagent (17.01 g, 42.06 mmol) and methyl N-(triethylammoniosulfonyl)carbamate (8.35 g, 35.05 mmol) were added. The reaction solution was stirred and reacted at 80°C for 16 hours. The complete reaction of the raw materials was detected. The reaction solution was concentrated to dryness under reduced pressure and then purified by column chromatography (silica, petroleum ether:tetrahydrofuran = 2:1) to obtain tert-butyl trans-N-[4-(1,3,4-thiadiazol-2-yl)cyclohexyl]carbamate as the product (5.5 g).

MS m/z (ESI): 228.1 $[M+H-tBu]^+$;

$^1H$ NMR (400MHz, DMSO-$d_6$) $\delta$ ppm 9.49 (s, 1H), 6.83 (br d, J = 7.6 Hz, 1H), 3.29 (br dd, J = 3.7, 8.1 Hz, 1H), 3.11 (tt, J = 3.5, 12.0 Hz, 1H), 2.14-2.06 (m, 2H), 1.89 (br d, J = 10.1 Hz, 2H), 1.63-1.51 (m, 2H), 1.39 (s, 9H), 1.36-1.26 (m, 2H).

Step 2: Synthesis of tert-butyl trans-N-[4-(5-bromo-1,3,4-thiadiazol-2-yl)cyclohexyl]carbamate

Tert-butyl trans-N-[4-(1,3,4-thiadiazol-2-yl)cyclohexyl]carbamate (2 g, 7.06 mmol) was dissolved in anhydrous tetrahydrofuran (200 mL), and then N-bromosuccinimide (3.77 g, 21.17 mmol) was added. The reaction solution was stirred and reacted at 60°C for 16 hours. The complete reaction of the raw materials was detected. The reaction solution was concentrated to dryness under reduced pressure and then purified by column chromatography (silica, petroleum ether:tetrahydrofuran = 2:1) to obtain tert-butyl trans-N-[4-(5-bromo-1,3,4-thiadiazol-2-yl) cyclohexyl]carbamate as the product (1.5 g).

MS m/z (ESI): 305.8, 307.8 $[M+H-tBu]^+$;

$^1H$ NMR (400MHz, DMSO-$d_6$) δ ppm 6.88-6.76 (m, 1H), 3.58-3.58 (m, 1H), 3.55 (t, J = 6.8 Hz, 1H), 3.42 (t, J = 6.3 Hz, 1H), 2.08 (br d, J = 12.0 Hz, 2H), 1.92-1.85 (m, 2H), 1.62-1.53 (m, 2H), 1.39 (s, 9H), 1.42-1.21 (m, 2H).

Step 3: Synthesis of isopropyl trans-N-[4-[5-[4-(tert-butoxycarbonylamino)cyclohexyl]-1,3,4-thiadiazol-2-yl]-3-isopropylsulfanyl-phenyl]carbamate

Tert-butyl trans-N-[4-(5-bromo-1,3,4-thiadiazol-2-yl)cyclohexyl]carbamate (150 mg, 414.04 μmol) and isopropyl N-[3-isopropylsulfanyl-4-(4,4,5,5-tetramethyl-1,3,2-dioxaborolan-2-yl)phenyl]carbamate (188.46 mg, 496.85 μmol) were dissolved in tetrahydrofuran (7 mL). Under the protection of nitrogen, [1,1-bis(diphenylphosphino)ferrocene]dichloropalladium - dichloromethane (33.81 mg, 41.40 μmol) and an aqueous potassium phosphate solution (2M, 621.06 μL) were added. The reaction solution was stirred and reacted at 80°C for 16 hours. The completion of the reaction was detected by LCMS. The reaction solution was concentrated to dryness under reduced pressure and purified by column chromatography (silica, petroleum ether:tetrahydrofuran = 2:1) to obtain isopropyl trans-N-[4-[5-[4-(tert-butoxycarbonylamino) cyclohexyl]-1,3,4-thiadiazol-2-yl]-3-isopropylsulfanyl-phenyl]carbamate (120 mg).

MS m/z (ESI): 535 $[M+H]^+$;

Step 4: Synthesis of isopropyl trans-N-[4-[5-(4-aminocyclohexyl)-1,3,4-thiadiazol-2-yl]-3-isopropylsulfanyl-phenyl]carbamate

Isopropyl trans-N-[4-[5-[4-(tert-butoxycarbonylamino)cyclohexyl]-1,3,4-thiadiazol-2-yl]-3-isopropylsulfanyl-phenyl]carbamate (220 mg, 411.42 μmol) was dissolved in methanol (2 mL), and hydrochloric acid/dioxane (1.13 mL) was added. The reaction solution was stirred and reacted at 30°C for 3 hours. The completion of the reaction was detected by LCMS. The reaction solution was concentrated to dryness under reduced pressure to obtain isopropyl trans-N-[4-[5-(4-aminocyclohexyl)-1,3,4-thiadiazol-2-yl]-3-isopropylsulfanyl-phenyl]carbamate (178 mg).

MS m/z (ESI): 435.3 $[M+H]^+$;

Step 5: Synthesis of isopropyl trans-N-[4-[5-[4-(isopropoxycarbonylamino)cyclohexyl]-1,3,4-thiadiazol-2-yl]-3-isopropylsulfanyl-phenyl]carbamate

Isopropyl trans-N-[4-[5-(4-aminocyclohexyl)-1,3,4-thiadiazol-2-yl]-3-isopropylsulfanyl-phenyl]carbamate (178 mg, 409.56 μmol) and isopropyl chloroformate (60.23 mg, 491.47 μmol) were dissolved in dichloromethane (4 mL), and N,N-diisopropylethylamine (264.66 mg, 2.05 mmol) was added. The reaction solution was stirred and reacted at 30°C for 2 hours. The reaction solution was concentrated to dryness under reduced pressure and purified by column chromatography (silica, petroleum ether:ethyl acetate = 1:1) to obtain isopropyl trans-N-[4-[5-[4-(isopropoxycarbonylamino)cyclohexyl]-1,3,4-thiadiazol-2-yl]-3-isopropylsulfanyl-phenyl]carbamate (120 mg).

MS m/z (ESI): 521.3 $[M+H]^+$;

Step 6: Synthesis of isopropyl trans-N-[4-[5-[4-(isopropoxycarbonylamino)cyclohexyl]-1,3,4-thiadiazol-2-yl]-3-(isopropylsulfonimidoyl)phenyl]carbamate

Isopropyl trans-N-[4-[5-[4-(isopropoxycarbonylamino)cyclohexyl]-1,3,4-thiadiazol-2-yl]-3-isopropylsulfanyl-phenyl]carbamate (38 mg, 72.98 μmol) was dissolved in a solution of ammonia in methanol (2 mL), and iodobenzene diacetate (352.59 mg, 1.09 mmol) was added. The reaction solution was stirred and reacted at 60°C for 16 hours. The completion of the reaction was detected by LCMS. The reaction solution was concentrated to dryness under reduced pressure. The resultant was purified by preparative liquid chromatography (Boston Prime C18 column: 5 μm silica, 25 mm in diameter, 150 mm in length; using decreasingly polar mixtures of water (containing 0.05% aqueous ammonia) and acetonitrile as eluents) to obtain isopropyl trans-N-[4-[5-[4-(isopropoxycarbonylamino)cyclohexyl]-1,3,4-thiadiazol-2-yl]-3-(isopropylsulfonimidoyl)phenyl]carbamate (20 mg).

MS m/z (ESI): 552.1 $[M+H]^+$;

$^1H$ NMR (400MHz, DMSO-$d_6$) δ ppm 10.18 (s, 1H), 8.33 (d, J = 2.1 Hz, 1H), 7.76 (br d, J = 8.5 Hz, 1H), 7.53 (d, J = 8.4 Hz, 1H), 7.04 (br d, J = 8.0 Hz, 1H), 4.98-4.90 (m, 1H), 4.80-4.68 (m, 1H), 4.11 (s, 1H), 3.74-3.64 (m, 1H), 3.28-3.22 (m, 1H), 3.16-3.06 (m, 1H), 2.15 (br d, J = 11.0 Hz, 2H), 1.92 (br d, J = 15.0 Hz, 2H), 1.68-1.53 (m, 2H), 1.42-1.32 (m, 2H), 1.28 (d, J = 6.3 Hz, 6H), 1.19-1.09 (m, 12H).

Example 42: Synthesis of isopropyl trans-N-[4-[2-[2-(isopropylsulfonimidoyl)]-4-[(5-methyl-1H-pyrazol-3-yl)amino]phenyl]thiazol-5-yl]cyclohexyl]carbamate

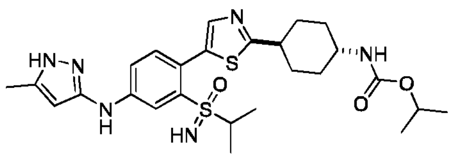

Synthetic Method

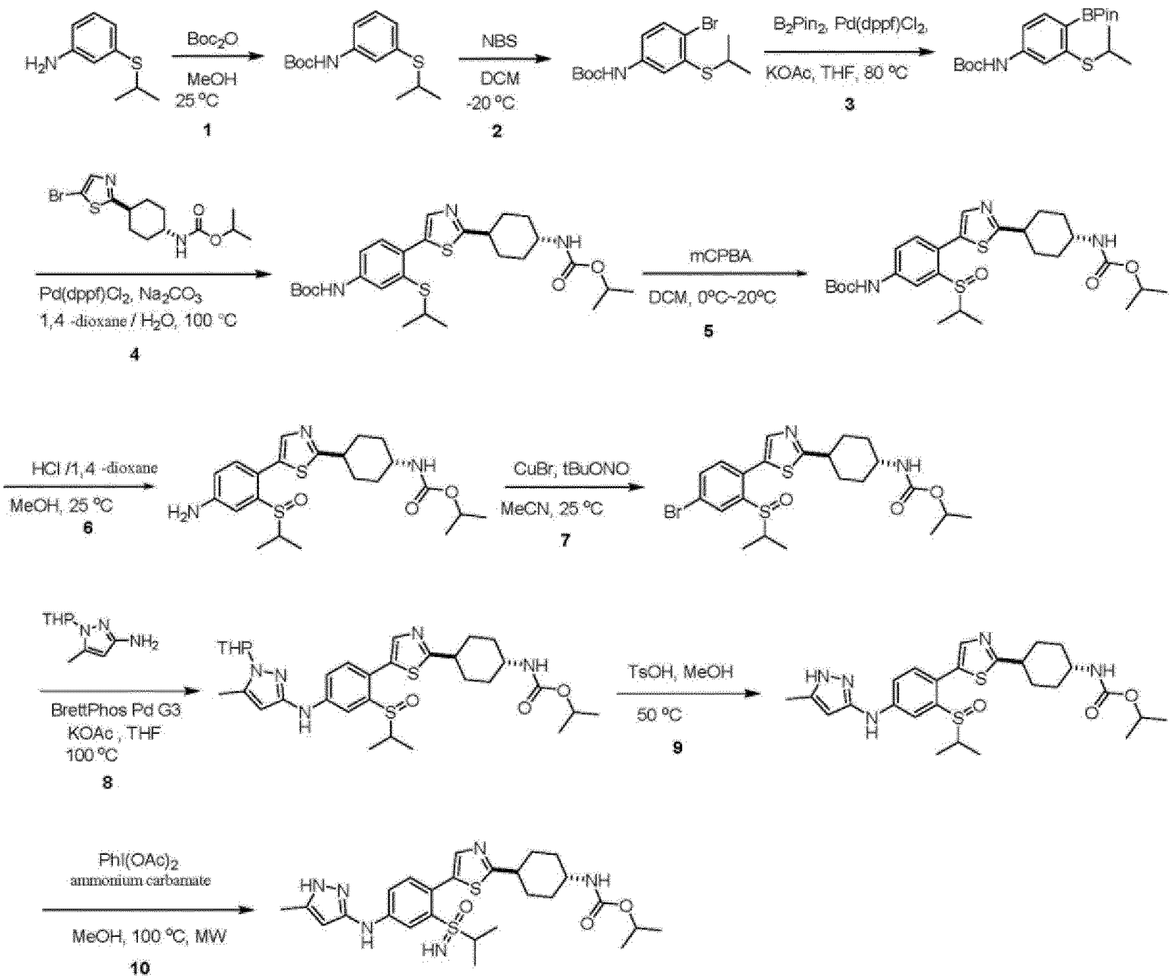


Step 1: Synthesis of tert-butyl N-(3-isopropylsulfanylphenyl)carbamate

3-Isopropylsulfanylaniline (10 g, 59.78 mmol) as a reactant was dissolved in anhydrous methanol (400 mL), and di-tert-butyl dicarbonate (195.71 g, 896.75 mmol, 206.01 mL) was added at 25°C. The reaction solution was stirred and reacted at 25°C for 12 hours. The completion of the reaction was detected by LCMS. The reaction solution was concentrated under reduced pressure to remove the solvent methanol, added with 400 mL of aqueous ammonia, and extracted three times with dichloromethane (300 mL × 3) to separate the organic phase. The organic phase was dried over anhydrous sodium sulfate and concentrated to dryness under reduced pressure. The resultant was purified by column chromatography (silica, petroleum ether: ethyl acetate = 10:1) to obtain tert-butyl N-(3-isopropylsulfanylphenyl)carbamate as a white solid (13.38 g, 50.04 mmol).

MS m/z (ESI): 212.1 [M-tBu+H]$^+$

Step 2: Synthesis of tert-butyl N-(4-bromo-3-isopropylsulfanyl-phenyl)carbamate

Tert-butyl N-(3-isopropylsulfanylphenyl)carbamate (13.38 g, 50.04 mmol) as a reactant was dissolved in dichloromethane (500 mL). N-bromosuccinimide (8.91 g, 50.04 mmol) was added into the reaction system. The reaction solution was stirred at -20°C for 3 hours. The completion of the reaction was detected by LCMS. The reaction solution was concentrated to dryness under reduced pressure and purified by column chromatography (silica, petroleum ether:ethyl acetate = 10:1) to obtain tert-butyl N-(4-bromo-3-isopropylsulfanyl-phenyl)carbamate as the product (5.32 g, 15.36 mmol), which was a white solid.
MS m/z (ESI): 290.0 [M-tBu+H]$^+$

Step 3: Synthesis of tert-butyl N-[3-isopropylsulfanyl-4-(4,4,5,5-tetramethyl-1,3,2-dioxaborolan-2-yl)phenyl]carbamate

Tert-butyl N-(4-bromo-3-isopropylsulfanyl-phenyl)carbamate (2 g, 5.78 mmol) and bis(pinacolato)diboron (5.87 g, 23.10 mmol) as reactants were dissolved in anhydrous tetrahydrofuran (50 mL). Under the protection of nitrogen, potassium acetate (1.70 g, 17.33 mmol) and [1,1-bis(diphenylphosphino)ferrocene]dichloropalladium - dichloromethane (235.83 mg, 288.78 μmol) were added. The reaction solution was stirred at 80°C for 2 hours. LCMS showed that the reaction was complete. The reaction solution was concentrated under reduced pressure to remove the solvent tetrahydrofuran, added with 50 mL of dichloromethane, and extracted three times with water (50 mL × 3) to separate the organic phase. The organic phase was dried over anhydrous sodium sulfate and concentrated to dryness under reduced pressure. The resultant was purified by column chromatography (silica, petroleum ether:ethyl acetate = 10:1) to obtain tert-butyl N-[3-isopropylsulfanyl-4-(4,4,5,5-tetramethyl-1,3,2-dioxaborolan-2-yl)phenyl]carbamate as a white solid (2.2 g, 5.59 mmol).
MS m/z (ESI): 394.3 [M+H]$^+$

Step 4: Synthesis of isopropyl trans-N-[4-[5-[4-(tert-butoxycarbonylamino)-2-isopropylsulfanyl-phenyl]thiazol-2-yl]cyclohexyl]carbamate

Tert-butyl N-[3-isopropylsulfanyl-4-(4,4,5,5-tetramethyl-1,3,2-dioxaborolan-2-yl) phenyl]carbamate (2 g, 5.08 mmol), isopropyl trans-N-[4-(5-bromothiazol-2-yl) cyclohexyl]carbamate (1.68 g, 4.84 mmol) and sodium carbonate (1.54 g, 14.53 mmol) were dissolved in water (600 μL) and 1,4-dioxane (6 mL). Under the protection of nitrogen, [1,1-bis(diphenylphosphino)ferrocene]dichloropalladium - dichloromethane (395.45 mg, 484.24 μmol) was added. The reaction solution was stirred at 100°C for 16 hours. The reaction solution was filtered. The filtrate was evaporated to dryness via rotary evaporation to remove 1,4-dioxane. The resultant was added with water (30 mL), and the mixture was extracted three times with dichloromethane (30 mL × 3). The resultant was dried over anhydrous sodium sulfate and then purified by column chromatography (silica, petroleum ether/tetrahydrofuran = 2/1) to obtain isopropyl trans-N-[4-[5-[4-(tert-butoxycarbonylamino)-2-isopropylsulfanyl-phenyl]thiazol-2-yl]cyclohexyl]carbamate as a yellow solid (1 g, 1.87 mmol).
MS m/z (ESI): 534.1 [M+H]$^+$

Step 5: Synthesis of isopropyl trans-N-[4-[5-[4-(tert-butoxycarbonylamino)-2-isopropylsulfinyl-phenyl]thiazol-2-yl]cyclohexyl]carbamate

Isopropyl trans-N-[4-[5-[4-(tert-butoxycarbonylamino)-2-isopropylsulfanyl-phenyl] thiazol-2-yl]cyclohexyl]carbamate (1.03 g, 1.93 mmol) was dissolved in dichloromethane (50 mL). m-Chloroperoxybenzoic acid (333.01 mg, 1.93 mmol) was added at 0°C. The reaction was carried out at 100°C for 3 hours. LCMS showed that the reaction was complete. The reaction solution was concentrated to dryness under reduced pressure and purified by column chromatography (silica, petroleum ether:ethyl acetate = 2:1) to obtain isopropyl trans-N-[4-[5-[4-(tert-butoxycarbonylamino)-2-isopropylsulfinyl-phenyl]thiazol-2-yl]cyclohexyl]carbamate as the product (725 mg, 1.32 mmol), which was a white solid with a yield of 68.34%.
MS m/z (ESI): 550.3 [M+H]$^+$

Step 6: Synthesis of isopropyl trans-N-[4-[5-(4-amino-2-isopropylsulfinyl-phenyl)thiazol-2-yl]cyclohexyl]carbamate

Isopropyl trans-N-[4-[5-[4-(tert-butoxycarbonylamino)-2-isopropylsulfinyl-phenyl] thiazol-2-yl]cyclohexyl]carbamate (200 mg, 363.81 μmol) was dissolved in methanol (4 mL). Hydrochloric acid/1,4-dioxane (4M, 909.51 μL) was added at 25°C. The reaction was carried out at 25°C for 12 hours. LCMS showed that the reaction was complete. The reaction solution was evaporated to dryness via rotary evaporation to remove methanol, added with water (5 mL), and

extracted three times with dichloromethane (5 mL × 3). The resultant was dried over anhydrous sodium sulfate and purified by column chromatography (silica, dichloromethane:methanol = 15:1) to obtain isopropyl trans-N-[4-[5-(4-amino-2-isopropylsulfinyl-phenyl)thiazol-2-yl]cyclohexyl]carbamate as the product (116 mg, 257.99 μmol), which was a white solid.

MS m/z (ESI): 450.1 $[M+H]^+$

Step 7: Synthesis of isopropyl trans-N-[4-[5-(4-bromo-2-isopropylsulfinyl-phenyl)thiazol-2-yl]cyclohexyl]carbamate

Isopropyl trans-N-[4-[5-(4-amino-2-isopropylsulfinyl-phenyl)thiazol-2-yl]cyclohexyl] carbamate (238 mg, 529.33 μmol) was dissolved in acetonitrile (6 mL). At 0°C, tert-butyl nitrite (163.75 mg, 1.59 mmol, 188.87 μL) was added and the mixture was stirred for 1 hour, and cuprous bromide (189.83 mg, 1.32 mmol) was added at 25°C. The reaction solution was reacted at 25°C for 15 hours. LCMS showed that the reaction was complete. The reaction solution was evaporated to dryness via rotary evaporation to remove acetonitrile, added with saturated ammonium chloride solution (20 mL), and extracted three times with dichloromethane (20 mL × 3). The resultant was dried over anhydrous sodium sulfate and purified by column chromatography (silica, dichloromethane:methanol = 15:1) to obtain isopropyl trans-N-[4-[5-(4-bromo-2-isopropylsulfinyl-phenyl)thiazol-2-yl]cyclohexyl] carbamate as the product (198 mg, 385.58 μmol), which was a yellow solid.

MS m/z (ESI): 515.1 $[M+H]^+$

Step 8: Synthesis of isopropyl trans-N-[4-[5-[2-isopropylsulfinyl]-4-[(5-methyl-1-tetrahydropyran-2-yl-pyrazol-3-yl)amino]phenyl]thiazol-2-yl]cyclohexyl]carbamate

Isopropyl trans-N-[4-[5-(4-bromo-2-isopropylsulfinyl-phenyl)thiazol-2-yl]cyclohexyl] carbamate (212 mg, 412.84 μmol) was dissolved in tetrahydrofuran (6 mL). 5-Methyl-1-tetrahydropyran-2-yl-pyrazol-3-amine (82.30 mg, 454.13 μmol) was added at 25°C. Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (37.42 mg, 41.28 μmol) and potassium acetate (40.52 mg, 412.84 μmol) were added. The reaction was carried out at 100°C for 1 hour. LCMS showed that the reaction was complete. The reaction solution was concentrated to dryness and purified by column chromatography (silica, dichloromethane:methanol = 15:1) to obtain isopropyl trans-N-[4-[5-[2-isopropylsulfinyl]-4-[(5-methyl-1-tetrahydropyran-2-yl-pyrazol-3-yl)amino]phenyl]thiazol-2-yl ]cyclohexyl]carbamate as the product (226 mg, 368.18 μmol), which was a yellow solid.

MS m/z (ESI): 614.4 $[M+H]^+$

Step 9: Synthesis of isopropyl trans-N-[4-[2-[2-isopropylsulfinyl]-4-[(5-methyl-1H-pyrazol-3-yl)amino]phenyl]thiazol-5-yl]cyclohexyl]carbamate

Isopropyl trans-N-[4-[5-[2-isopropylsulfinyl]-4-[(5-methyl-1-tetrahydropyran-2-yl-pyrazol-3-yl)amino]phenyl]thiazol-2-yl]cyclohexyl]carbamate (90 mg, 146.62 μmol) was dissolved in methanol (2 mL). p-Toluenesulfonic acid (126.24 mg, 733.10 μmol) was added at 25°C. The reaction was carried out at 50°C for 2 hours. LCMS showed that the reaction was complete. The reaction solution was evaporated to dryness via rotary evaporation to remove methanol, added with saturated sodium bicarbonate solution (5 mL), and stirred for 5 minutes. The aqueous phase was extracted three times with dichloromethane (5 mL × 3). The resultant was dried over anhydrous sodium sulfate and purified by thin-layer chromatography (silica, dichloromethane:methanol = 15:1) to obtain isopropyl trans-N-[4-[2-[2-isopropylsulfinyl]-4-[(5-methyl-1H-pyrazol-3-yl)amino]phenyl]thiazol-5-yl]cyclohexyl]carbamate as the product (30 mg, 56.63 μmol), which was a white solid.

MS m/z (ESI): 530.3 $[M+H]^+$

Step 10: Synthesis of isopropyl trans-N-[4-[2-[2-(isopropylsulfonimidoyl)]-4-][(5-methyl-1H-pyrazol-3-yl)amino]phenyl]thiazol-5-yl]cyclohexyl]carbamate

Isopropyl trans-N-[4-[2-[2-isopropylsulfinyl]-4-[(5-methyl-1H-pyrazol-3-yl)amino]phenyl] thiazol-5-yl]cyclohexyl]carbamate (30 mg, 56.63 μmol), ammonium carbamate (88.43 mg, 1.13 mmol) and iodobenzene diacetate (182.42 mg, 566.34 μmol) were charged into a microwave vial containing methanol (0.5 mL). The reaction was carried out under microwave irradiation at 100°C for 2 hours. LCMS showed that the reaction was complete. The reaction solution was concentrated to dryness under reduced pressure and purified by thin-layer chromatography (silica, dichloromethane:methanol = 15:1) to obtain a crude product. Then, the crude product was further purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile as eluents) to obtain isopropyl trans-N-[4-[2-[2-(isopropylsulfonimidoyl)]-4-[(5-methyl-1H-pyrazol-3-yl)amino] phenyl]thiazol-5-yl]cyclohexyl]carbamate as the product (1.21mg), which was a white solid.

MS m/z (ESI): 545.3 [M+H]+

$^1$H NMR: (400 MHz, METHANOL-$d_4$) δ ppm 8.01 (s, 1H) 7.67 (s, 1H) 7.49 (br d, J = 7.28 Hz, 1H) 7.31 (d, J = 8.53 Hz, 1H) 5.82 (s, 1H) 4.82-4.70 (m, 1H) 3.55-3.42 (m, 1H) 2.98-3.13 (m, 2H) 2.30 (s, 3H) 2.25 (br d, J = 9.03 Hz, 2H) 2.09 (br d, J = 10.54 Hz, 2H) 1.78-1.65 (m, 2H) 1.38-1.54 (m, 2H) 1.32 (d, J = 6.2 Hz, 6H) 1.24 (d, J = 6.2 Hz, 6H).

Example 43: Synthesis of isopropyl trans-N-[3-(N-cyclopropyl-S-methyl-sulfonimidoyl)-4-[2-[4-(isopropoxycarbonylamino)cyclohexyl]thiazol-5-yl]phenyl]carbamate

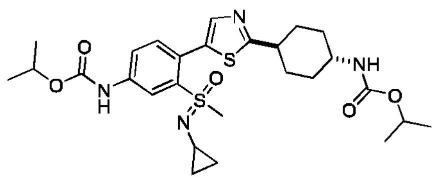

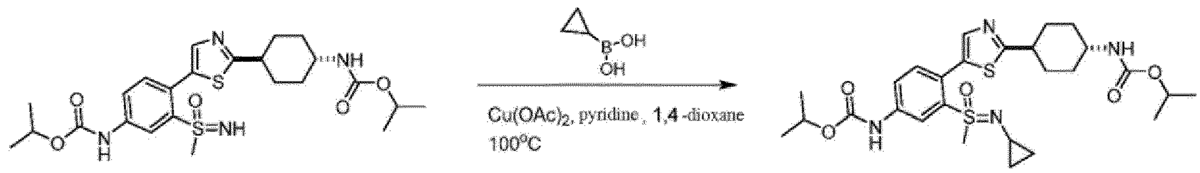


Isopropyl trans-N-[4-[2-[4-(isopropoxycarbonylamino)cyclohexyl]thiazol-5-yl]-3-(methylsulfonimidoyl)phenyl]carbamate (40 mg, 76.53 μmol) was dissolved in 1,4-dioxane (1 mL), and copper acetate (20.85 mg, 114.79 μmol) and pyridine (14.53 mg, 183.67 μmol, 14.82 μL) were added. The reaction mixture was stirred at 20°C for 5 minutes, and cyclopropylboronic acid (13.15 mg, 153.06 μmol) was added. The reaction solution was stirred at 100°C for 12 hours. LCMS showed that the reaction was complete. The reaction solution was filtered. The filtrate was evaporated to dryness via rotary evaporation to remove 1,4-dioxane, added with water (15 mL), and extracted three times with ethyl acetate (15 mL × 3). The organic phase was dried over anhydrous sodium sulfate and evaporated to dryness via rotary evaporation under reduced pressure. The resultant was purified by preparative high-performance liquid chromatography (YMC-Actus Triart C18 column, 5 μm silica, 30 mm in diameter, 150 mm in length) (using decreasingly polar mixtures of water (containing 0.05% aqueous ammonia) and acetonitrile (48% to 68%) as eluents) to obtain isopropyl trans-N-[3-(N-cyclopropyl-S-methyl-sulfonimidoyl)-4-[2-[4-(isopropoxycarbonylamino)cyclohexyl] thiazol-5-yl]phenyl]carbamate (14.12 mg, 25.09 μmol).

MS m/z (ESI): 563.3 [M+H]+

$^1$H NMR: (400 MHz, DMSO-$d_6$) δ ppm 10.10 (s, 1H) 8.37 (d, J = 2.32Hz, 1H) 7.74-7.68 (m, 2H) 7.43 (d, J = 8.31Hz, 1H) 7.03 (br d, J = 7.70Hz, 1H) 4.97-4.88 (m, 1H) 4.80-4.71 (m, 1H) 2.97-2.89 (m, 1H) 2.87 (s, 3H) 2.55-2.51 (m, 1H) 2.32-2.26 (m, 1H) 2.12 (br d, J = 12.35Hz, 2H) 1.91 (br d, J = 10.15Hz, 2H) 1.62-1.51 (m, 2H) 1.39-1.31 (m, 2H) 1.28 (d, J = 6.24Hz, 6H) 1.17 (d, J = 6.24Hz, 6H) 0.44-0.33 (m, 2H) 0.33-0.20 (m, 2H).

Example 44: Synthesis of isopropyl trans-N-[4-[5-[2-isopropylsulfonyl-4-(pyridazin-3-ylamino)phenyl]thiazol-2-yl]cyclohexyl]carbamate

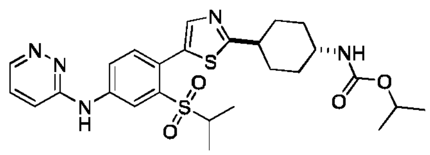

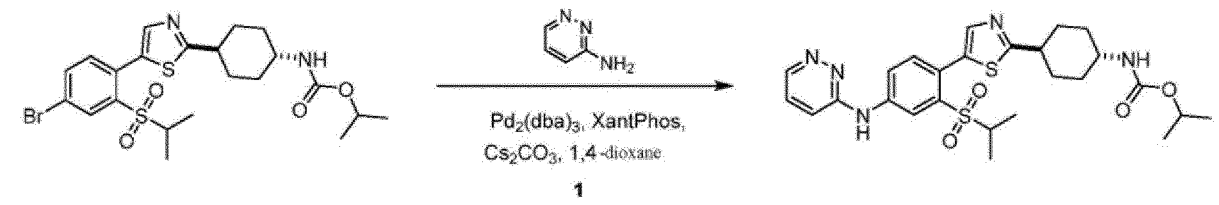


Isopropyl trans-N-[4-[5-(4-bromo-2-isopropylsulfonyl-phenyl)thiazol-2-yl]cyclohexyl] carbamate (20 mg, 37.77 μmol) and 3-aminopyridazine (8.98 mg, 94.43 μmol) were dissolved in dioxane (2 mL). Afterwards, tris(dibenzylideneacetone)dipalladium (1.73 mg, 1.89 μmol) and 4,5-bis(diphenylphosphino)-9,9-dimethylxanthene (1.09 mg, 1.89 μmol) and cesium carbonate (36.92 mg, 113.31 μmol) were added under the condition where nitrogen gas was introduced. The reaction solution was stirred and reacted at 100°C for 16 hours. The completion of the reaction was detected by LCMS. The reaction solution was added with 9 mL of ethyl acetate (3 mL × 3) and 4 mL of water. The organic phase was dried over anhydrous sodium sulfate and filtered. The filtrate was concentrated to dryness under reduced pressure and purified by preparative liquid chromatography (Phenomenex Gemini C18 column: 10 μm silica, 50 mm in diameter, 250 mm in length; using decreasingly polar mixtures of water (containing 0.05% aqueous ammonia) and acetonitrile as eluents) to obtain isopropyl trans-N-[4-[5-[2-isopropylsulfonyl-4-(pyridazin-3-ylamino)phenyl]thiazol-2-yl]cyclohexyl]carbamate (7 mg).

MS m/z (ESI): 544.1 $[M+H]^+$;

$^1H$ NMR (400MHz, DMSO-$d_6$) δ ppm 9.91 (s, 1H), 8.77 (d, J = 4.4 Hz, 1H), 8.45-8.26 (m, 2H), 7.72 (s, 1H), 7.61-7.46 (m, 2H), 7.19 (d, J = 8.8 Hz, 1H), 7.03 (br d, J = 7.6 Hz, 1H), 4.77-4.66 (m, 1H), 3.30-3.24 (m, 1H), 3.00-2.84 (m, 2H), 2.13 (br d, J = 12.0 Hz, 2H), 1.92 (br d, J = 9.6 Hz, 2H), 1.64-1.50 (m, 2H), 1.41-1.28 (m, 2H), 1.16 (d, J = 6.3 Hz, 6H), 1.08 (d, J = 6.8 Hz, 6H).

Example 45: Synthesis of isopropyl trans-N-[4-[5-[4-[(5-cyclopropyl-1H-pyrazol-3-yl)amino]-2-isopropylsulfonyl-phenyl]thiazol-2-yl]cyclohexyl]carbamate

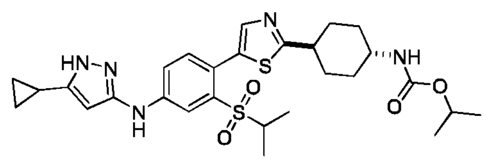

Synthetic Method

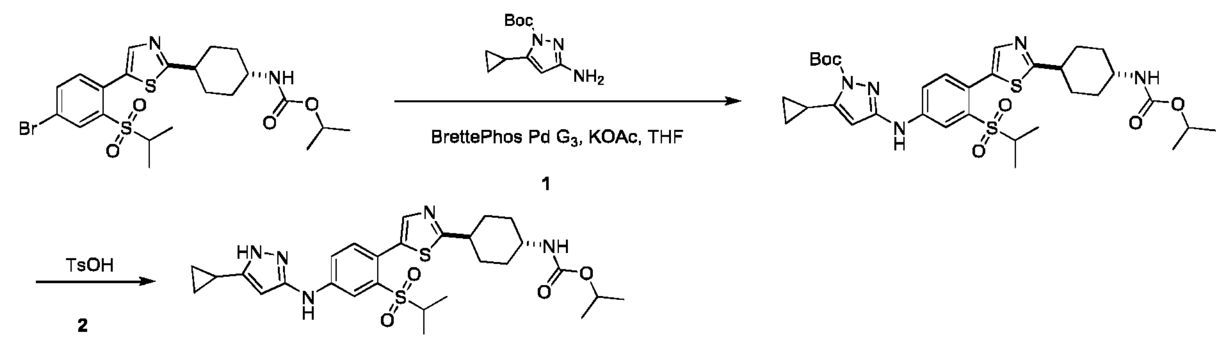


Step 1: Synthesis of tert-butyl trans-5-cyclopropyl-3-[4-[2-[4-(isopropoxycarbonylamino) cyclohexyl]thiazol-5-yl]-3-isopropylsulfonyl-anilino]pyrazole-1-carboxylate

Isopropyl trans-N-[4-[5-(4-bromo-2-isopropylsulfonyl-phenyl)thiazol-2-yl]cyclohexyl] carbamate (40 mg, 75.54 μmol) and tert-butyl 3-amino-5-cyclopropyl-pyrazole-1-carboxylate (18.55 mg, 83.10 μmol) were dissolved in tetrahydrofuran (5 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (6.85 mg, 7.55 μmol) and potassium acetate (7.41 mg, 75.54 μmol) were added. The reaction solution was stirred and reacted at 100°C for 16 hours. The completion of the reaction was detected by LCMS. The reaction solution was added with 9 mL of ethyl acetate (3 mL × 3) and 4 mL of water. The organic phase was dried over anhydrous sodium sulfate and filtered. The filtrate was concentrated to dryness under reduced pressure and purified by column chromatography (silica, dichloromethane:methanol = 15:1) to obtain tert-butyl trans-5-cyclopropyl-3-[4-[2-[4-(isopropoxycarbonylamino)cyclohexyl]thiazol-5-yl]-3-isopropylsulfonyl-anilino]pyrazole-1-carboxylate as a yellow substance (30 mg).

MS m/z (ESI): 672.3 $[M+H]^+$;

Step 2: Synthesis of isopropyl trans-N-[4-[5-[4-[(5-cyclopropyl-1H-pyrazol-3-yl)amino]-2-isopropylsulfonyl-phenyl]thiazol-2-yl]cyclohexyl]carbamate

Tert-butyl trans-5-cyclopropyl-3-[4-[2-[4-(isopropoxycarbonylamino)cyclohexyl]thiazol-5-yl]-3-isopropylsulfonyl-anilino]pyrazole-1-carboxylate (63 mg, 93.77 μmol) was dissolved in methanol (5 mL), and then p-toluenesulfonic acid monohydrate (80.74 mg, 468.84 μmol) was added into the reaction solution. The reaction solution was stirred and reacted at 50°C for 2 hours. The completion of the reaction was detected by LCMS. The resultant was concentrated to dryness under reduced pressure and purified by preparative liquid chromatography (Phenomenex Gemini-NX column: 5 μm silica, 30 mm in diameter, 150 mm in length; using decreasingly polar mixtures of water (containing 0.225% formic acid) and acetonitrile as eluents) to obtain isopropyl trans-N-[4-[5-[4-[(5-cyclopropyl-1H-pyrazol-3-yl)amino]-2-isopropylsulfonyl-phenyl]thiazol-2-yl]cyclohexyl]carbamate as a white substance (2.9 mg).

MS m/z (ESI): 572.1 $[M+H]^+$;

1H NMR (400MHz, DMSO-d6) δ ppm 11.93 (s, 1H), 9.06 (s, 1H), 8.13 (s, 1H), 7.70-7.58 (m, 2H), 7.34 (d, J=8.4 Hz, 1H), 7.02 (br d, J = 7.8 Hz, 1H), 5.54 (s, 1H), 4.82-4.70 (m, 1H), 3.31-3.27 (m, 1H), 2.98-2.79 (m, 2H), 2.12 (br d, J = 11.5 Hz, 2H), 1.96-1.88 (m, 2H), 1.88-1.82 (m, 1H), 1.63-1.47 (m, 2H), 1.41-1.25 (m, 2H), 1.16 (d, J = 6.2 Hz, 6H), 1.05 (d, J = 6.8 Hz, 6H), 0.96-0.88 (m, 2H), 0.74-0.64 (m, 2H).

Example 46: Synthesis of isopropyl trans-N-[4-[5-[2-cyclopropylsulfinyl]-4-[(5-methyl-1H-pyrazol-3-yl)amino]phenyl]thiazol-2-yl]cyclohexyl]carbamate

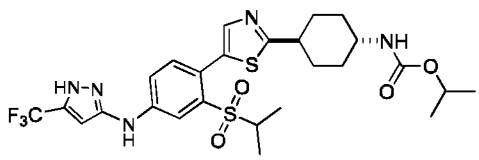

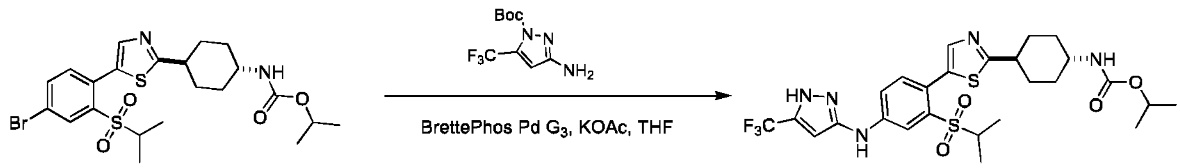


1

Isopropyl trans-N-[4-[5-(4-bromo-2-isopropylsulfonyl-phenyl)thiazol-2-yl]cyclohexyl] carbamate (50 mg, 94.43 μmol) and tert-butyl 3-amino-5-(trifluoromethyl)pyrazole-1-carboxylate (28.46 mg, 113.31 μmol) were dissolved in tetrahydrofuran (5 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (8.56 mg, 9.44 μmol) and potassium acetate (9.27 mg, 94.43 μmol) were added. The reaction solution was stirred and reacted at 100°C for 16 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and purified by thin-layer chromatography (silica, dichloromethane:methanol = 10:1) to obtain isopropyl trans-N-[4-[5-[2-cyclopropylsulfinyl]-4-[(5-methyl-1H-pyrazol-3-yl)amino]phenyl]thiazol-2-yl] cyclohexyl]carbamate as a white substance (2.3 mg).

MS m/z (ESI): 600.2 $[M+H]^+$;

$^1$H NMR (400MHz, DMSO-$d_6$) δ ppm 13.80-13.39 (m, 1H), 9.58-9.12 (m, 1H), 8.24-8.04 (m, 1H), 7.72-7.58 (m, 2H), 7.41 (d, J = 8.3 Hz, 1H), 7.21 (br s, 1H), 7.02 (br d, J = 7.5 Hz, 1H), 6.49 (br s, 1H), 4.82-4.70 (m, 1H), 3.40-3.33 (m, 1H), 3.00-2.80 (m, 2H), 2.12 (br d, J = 11.8 Hz, 2H), 1.91 (br d, J = 9.8 Hz, 2H), 1.63-1.47 (m, 2H), 1.40-1.27 (m,

2H), 1.16 (d, J = 6.3 Hz, 6H), 1.05 (d, J = 6.8 Hz, 6H).

Example 47: Synthesis of isopropyl trans-N-[4-[5-[4-[(5-methyl-1H-pyrazol-3-yl)amino]-2-(1-oxidophospholan-1-yl)phenyl]thiazol-2-yl]cyclohexyl]carbamate

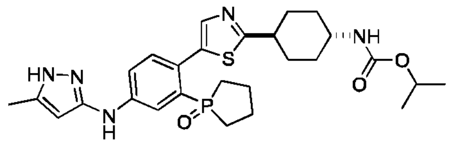

Synthetic Method

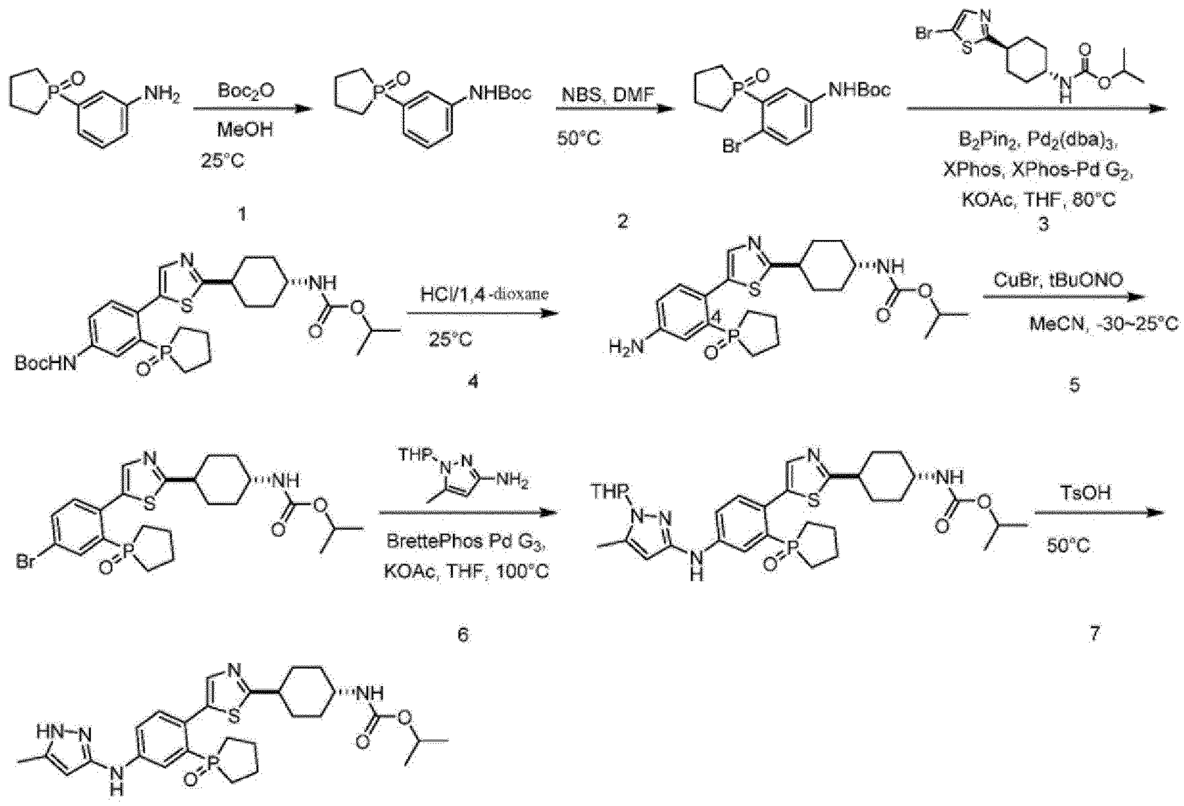


Step 1: Synthesis of tert-butyl (3-(1-oxidophospholan-1-yl)phenyl)carbamate

1-(3-Aminophenyl)phospholane-1-oxide (340 mg, 1.74 mmol) was dissolved in methanol (10 mL), and BOC anhydride (3.80 g, 17.42 mmol, 4.00 mL) was added at 25°C. The reaction solution appeared as a yellow suspension, and was stirred and reacted at 25°C for 16 h. The completion of the reaction was detected by LCMS. The reaction solution was concentrated to dryness under reduced pressure. Aqueous ammonia (10 mL) was added into the reaction solution. The reaction solution was stirred and reacted at 25°C for 2 h, and then extracted with dichloromethane (20 mL). The organic layer was dried over anhydrous magnesium sulfate and subjected to suction filtration. The filtrate was concentrated to dryness under reduced pressure. The resultant was purified by column chromatography (dichloromethane:methanol = 10:1) to obtain the compound tert-butyl (3-(1-oxidophospholan-1-yl)phenyl)carbamate (400 mg, 1.35 mmol) as a white solid.
MS m/z (ESI): 296.2 $[M+H]^+$;

Step 2: Synthesis of tert-butyl N-[4-bromo-3-(1-oxidophospholan-1-yl)phenyl]carbamate

Tert-butyl (3-(1-oxidophospholan-1-yl)phenyl)carbamate (360 mg, 1.22 mmol) was dissolved in N,N-dimethylformamide (5 mL), and N-bromosuccinimide (650.91 mg, 3.66 mmol) was added. The reaction solution appeared as a yellow suspension, and was stirred and reacted at 50°C for 16 h. The completion of the reaction was detected by LCMS. The reaction solution was concentrated to dryness under reduced pressure and purified by column chromatography (dichloromethane:methanol = 10:1) to obtain the compound tert-butyl N-[4-bromo-3-(1-oxidophospholan-1-yl)phenyl]carbamate (320 mg, 855.14 μmol) as a yellow oil.
MS m/z (ESI): 374.0, 376.0 $[M+H]^+$;
$^1H$ NMR (400MHz, DMSO-$d_6$) δ ppm 9.68 (s, 1H), 8.13-8.05 (m, 1H), 7.65 (d, J = 4.0 Hz, 1H), 7.63-7.58 (m, 1H), 2.30-2.17 (m, 2H), 2.05-1.86 (m, 6H), 1.50 (s, 9H).

Step 3: Synthesis of isopropyl trans-N-[4-[5-[4-(tert-butoxycarbonylamino)-2-(1-oxidophospholan-1-yl)phenyl]thiazol-2-yl]cyclohexyl]carbamate

Tert-butyl N-[4-bromo-3-(1-oxidophospholan-1-yl)phenyl]carbamate (220 mg, 587.91 μmol), isopropyl trans-N-[4-(5-bromothiazol-2-yl)cyclohexyl]carbamate (204.16 mg, 587.91 μmol) and bis(pinacolato)diboron (597.17 mg, 2.35 mmol) were dissolved in anhydrous tetrahydrofuran (4 mL). Under the protection of nitrogen, tris(dibenzylideneacetone)dipalladium(0) (26.92 mg, 29.40 μmol) and 2-dicyclohexylphosphino-2,4,6-triisopropylbiphenyl (14.01 mg, 29.40 μmol), chloro(2-dicyclohexylphosphino-2,4,6-triisopropyl-1,1-biphenyl)[2-(2-amino-1,1-biphenyl)] palladium (II) (23.13 mg, 29.40 μmol) and potassium acetate (115.40 mg, 1.18 mmol) were added. The reaction solution appeared as a dark yellow suspension, and was stirred and reacted at 80°C for 16 h. The completion of the reaction was detected by LCMS. The reaction solution was concentrated to dryness under reduced pressure, added with water (10 mL), and extracted with ethyl acetate (20 mL). The organic layer was dried over anhydrous magnesium sulfate and subjected to suction filtration. The filtrate was concentrated to dryness under reduced pressure. The resultant was purified by column chromatography (dichloromethane:methanol = 10:1) to obtain the compound isopropyl trans-N-[4-[5-[4-(tert-butoxycarbonylamino)-2-(1-oxidophospholan-1-yl)phenyl]thiazol-2-yl]cyclohexyl] carbamate (160 mg, 284.86 μmol) as a yellow oil.
MS m/z (ESI): 562.2 $[M+H]^+$;

Step 4: Synthesis of isopropyl trans-N-[4-[5-[4-amino-2-(1-oxidophospholan-1-yl)phenyl]thiazol-2-yl]cyclohexyl]carbamate

Isopropyl trans-N-[4-[5-[4-(tert-butoxycarbonylamino)-2-(1-oxidophospholan-1-yl)phenyl] thiazol-2-yl]cyclohexyl]carbamate (160 mg, 284.86 μmol) was dissolved in anhydrous methanol (2.5 mL), and hydrochloric acid/dioxane (4M, 2.5 mL) was added. The reaction solution appeared as a yellow suspension, and was stirred and reacted at 25°C for 2 h. The completion of the reaction was detected by LCMS. The reaction solution was concentrated to dryness under reduced pressure, added with a saturated sodium carbonate solution (10 mL), and extracted with dichloromethane (20 mL) twice. The organic layer was dried over anhydrous magnesium sulfate and subjected to suction filtration. The filtrate was concentrated to dryness under reduced pressure to obtain crude isopropyl trans-N-[4-[5-[4-amino-2-(1-oxidophospholan-1-yl)phenyl]thiazol-2-yl]cyclohexyl]carbamate (110 mg) as a white solid.
MS m/z (ESI): 462.2 $[M+H]^+$;

Step 5: Synthesis of isopropyl trans-N-[4-[5-[4-bromo-2-(1-oxidophospholan-1-yl)phenyl]thiazol-2-yl]cyclohexyl]carbamate

Isopropyl trans-N-[4-[5-[4-amino-2-(1-oxidophospholan-1-yl)phenyl]thiazol-2-yl] cyclohexyl]carbamate (110 mg, 238.32 μmol) was dissolved in acetonitrile (3 mL). Under the protection of nitrogen, tert-butyl nitrite (73.73 mg, 714.97 μmol, 85.04 μL) was added at -30°C. The reaction solution was stirred and reacted for 1 h. Under the protection of nitrogen, cuprous bromide (85.47 mg, 595.81 μmol, 18.15 μL) was added at -30°C. The reaction solution appeared as a yellow suspension, and was stirred and reacted at 25°C for 16 h. The completion of the reaction was detected by LCMS. The reaction solution was concentrated to dryness under reduced pressure, added with saturated ammonium chloride solution (10 mL), and extracted with dichloromethane (20 mL). The organic layer was dried over anhydrous magnesium sulfate and subjected to suction filtration. The filtrate was concentrated to dryness under reduced pressure. The resultant was purified by column chromatography (dichloromethane:methanol = 10:1) to obtain the compound isopropyl trans-N-[4-[5-[4-bromo-2-(1-oxidophospholan-1-yl)phenyl]thiazol-2-yl]cyclohexyl]carbamate (70 mg, 133.22 μmol) as a white solid.
MS m/z (ESI): 525.1, 527.1 $[M+H]^+$;

Step 6: Synthesis of isopropyl trans-N-[4-[5-[4-[(5-methyl-1-tetrahydropyran-2-yl-pyrazol-3-yl)amino]-2-(1-oxidophospholan-1-yl)phenyl]thiazol-2-yl]cyclohexyl]carbamate

Isopropyl trans-N-[4-[5-[4-bromo-2-(l-oxidophospholan-1-yl)phenyl]thiazol-2-yl] cyclohexyl]carbamate (35 mg, 66.61 μmol) and 1-Boc-3-amino-5-methylpyrazole (13.28 mg, 73.27 μmol) were dissolved in anhydrous tetrahydrofuran (3 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (6.04 mg, 6.66 μmol) and potassium acetate (6.54 mg, 66.61 μmol) were added. The reaction solution appeared as a black suspension, and was stirred and reacted at 100°C for 1 h. The completion of the reaction was detected by LCMS. The reaction solution was concentrated to dryness under reduced pressure. The resultant was purified by thin-layer chromatography (dichloromethane:methanol = 10:1) to obtain the compound isopropyl trans-N-[4-[5-[4-[(5-methyl-1-tetrahydropyran-2-yl-pyrazol-3-yl)amino]-2-(1-oxidophospholan-1-yl)phenyl]thiazol-2-yl]cyclohexyl]carbamate (20 mg, 31.96 μmol) as a white solid.
MS m/z (ESI): 626.3 $[M+H]^+$;

Step 7: Synthesis of isopropyl trans-N-[4-[5-[4-[(5-methyl-1H-pyrazol-3-yl)amino]-2-(1-oxidophospholan-1-yl)phenyl]thiazol-2-yl]cyclohexyl]carbamate

Isopropyl trans-N-[4-[5-[4-[(5-methyl-1-tetrahydropyran-2-yl-pyrazol-3-yl)amino]-2-(1-oxidophospholan-1-yl)phenyl]thiazol-2-yl]cyclohexyl]carbamate (20 mg, 31.96 μmol) was dissolved in anhydrous methanol (3 mL), and p-toluenesulfonic acid monohydrate (27.52 mg, 159.81 μmol) was added. The reaction solution appeared as a black suspension, and was stirred and reacted at 50°C for 1 h. The completion of the reaction was detected by LCMS. The reaction solution was concentrated to dryness under reduced pressure, added with saturated sodium carbonate solution (5 mL), and extracted with dichloromethane (10 mL). The organic layer was dried over anhydrous magnesium sulfate and subjected to suction filtration. The filtrate was concentrated to dryness under reduced pressure. The resultant was purified by preparative liquid chromatography (PhenomenexGeminiC18 column, 10 μm silica, 50 mm in diameter, 250 mm in length; using decreasingly polar mixtures of water (containing 0.05% aqueous ammonia) and acetonitrile as eluents) to obtain the compound isopropyl trans-N-[4-[5-[4-[(5-methyl-1H-pyrazol-3-yl)amino]-2-(1-oxidophospholan-1-yl)phenyl]thiazol-2-yl ]cyclohexyl]carbamate (1.12 mg, 2.07 μmol) as a white solid.
MS m/z (ESI): 542.2 $[M+H]^+$;
$^1H$ NMR (400MHz, DMSO-d6) δ ppm 11.81 (s, 1H), 8.86-8.78 (m, 1H), 7.99-7.91 (m, 1H), 7.86 (s, 1H), 7.59-7.53 (m, 1H), 7.34-7.28 (m, 1H), 7.06-7.00 (m, 1H), 5.69 (s, 1H), 4.83-4.66 (m, 1H), 3.35-3.29 (m, 1H), 2.97-2.85 (m, 1H), 2.20 (s, 3H), 2.16-2.07 (m, 2H), 1.96-1.78 (m, 6H), 1.67-1.48 (m, 6H), 1.40-1.29 (m, 2H), 1.17 (d, J = 6.3 Hz, 6H).

Example 48: Synthesis of isopropyl trans-(4-(5-(2-(cyclopropylsulfonyl)-4-((5-methyl-1H-pyrazol-3-yl)amino)phenyl)thiazol-2-yl)cyclohexyl)carbamate

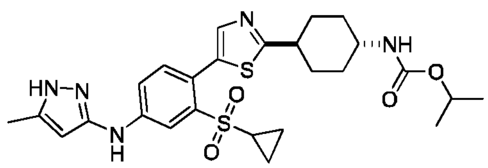

Synthetic Method

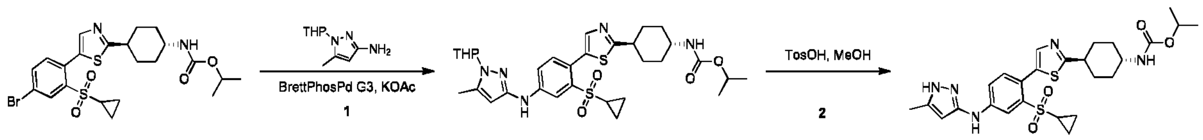

Step 1: Synthesis of isopropyl trans-(4-(5-(2-(cyclopropylsulfonyl)-4-((5-methyl-1-(tetrahydro-2H-pyran-2-yl)-1H-pyrazol-3-yl)amino)phenyl)thiazol-2-yl)cyclohexyl)carbamate

The intermediate isopropyl trans-(4-(5-(4-bromo-2-(cyclopropylsulfonyl)phenyl)thiazol-2-yl)cyclohexyl)carbamate (150 mg, 284.36 μmol) in Example 55 and 5-methyl-1-(tetrahydro-2H-pyran-2-yl)-1H-pyrazol-3-amine (56.69 mg, 312.8 μmol) were dissolved in anhydrous tetrahydrofuran (6 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (25.78 mg, 428.44 μmol) and potassium acetate (83.72 mg, 853.09 μmol) were added. The reaction solution was stirred at 100°C for 1 hour. LCMS showed that the reaction was complete. The reaction system was evaporated to dryness via rotary evaporation, and then the resultant was subjected to liquid-liquid extraction (extraction with 12 mL of ethyl acetate three times) to obtain isopropyl trans-(4-(5-(2-(cyclopropylsulfonyl)-4-((5-methyl-1-(tetrahydro-2H-pyran-2-yl)-1H-pyrazol-3-yl)amino) phenyl)thiazol-2-yl)cyclohexyl)carbamate as a yellow solid compound (150 mg, crude product).

MS m/z (ESI): 628.3 $[M+H]^+$;

Step 2: Synthesis of isopropyl trans-(4-(5-(2-(cyclopropylsulfonyl)-4-((5-methyl-1H-pyrazol-3-yl)amino)phenyl)thiazol-2-yl)cyclohexyl)carbamate

Isopropyl trans-(4-(5-(2-(cyclopropylsulfonyl)-4-((5-methyl-1-(tetrahydro-2H-pyran-2-yl)-1H-pyrazol-3-yl)amino)phenyl)thiazol-2-yl)cyclohexyl)carbamate (150 mg, 238.92 μmol) was dissolved in methanol (5 mL), and p-toluenesulfonic acid (205.72 mg, 1.19 mmol) was added. The reaction solution was stirred at 50°C for 30 minutes. LCMS showed that the reaction was complete. The reaction system was evaporated to dryness via rotary evaporation. The resultant was added with saturated sodium bicarbonate solution (20 mL), and then the mixture was extracted with dichloromethane (60 mL) three times. The dichloromethane phases were combined, dried over anhydrous sodium sulfate, and subjected to suction filtration. The resultant was purified by preparative high-performance liquid chromatography (Phenomenex Gemini-NX column, 10 μm silica, 30 mm in diameter, 100 mm in length) (using decreasingly polar mixtures of water (containing 0.05% aqueous ammonia) and acetonitrile (35% to 55%) as eluents) and lyophilized to obtain isopropyl trans-(4-(5-(2-(cyclopropylsulfonyl)-4-((5-methyl-1H-pyrazol-3-yl)amino)phenyl)thiazol-2-yl)cyclohexyl)carbamate as a white solid compound (55.59 mg).

MS m/z (ESI): 544.2 $[M+H]^+$;

$^1H$ NMR (400MHz, DMSO-$d_6$) δ ppm 11.86 (s, 1H), 9.04 (s, 1H), 8.09 (d, J = 2.1 Hz, 1H), 7.67-7.58 (m, 2H), 7.32 (d, J = 8.4 Hz, 1H), 7.02 (br d, J = 7.6 Hz, 1H), 5.64 (s, 1H), 4.80-4.69 (m, 1H), 3.31-3.23 (m, 1H), 2.98-2.88 (m, 1H), 2.42-2.32 (m, 1H), 2.20 (s, 3H), 2.13 (br d, J = 12.3 Hz, 2H), 1.91 (br d, J = 10.1 Hz, 2H), 1.65-1.47 (m, 2H), 1.42-1.25 (m, 2H), 1.16 (d, J = 6.1 Hz, 6H), 1.00-0.90 (m, 2H), 0.90-0.83 (m, 2H).

Example 49: Synthesis of isopropyl trans-(4-(5-(2-(cyclopropylsulfonyl)-4-((1,5-dimethyl-1H-pyrazol-3-yl)amino)phenyl)thiazol-2-yl)cyclohexyl)carbamate

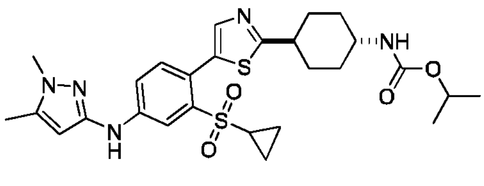

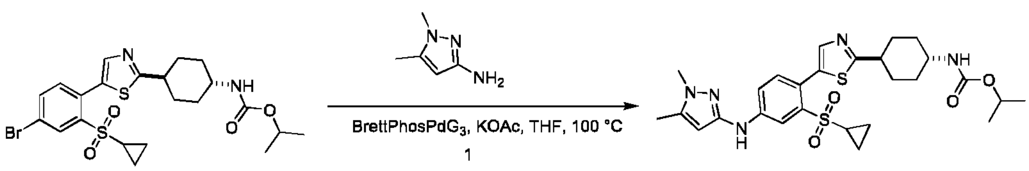


Isopropyl trans-(4-(5-(4-bromo-2-(cyclopropylsulfonyl)phenyl)thiazol-2-yl)cyclohexyl) carbamate (40 mg, 75.83 μmol) and 1,5-dimethyl-1H-pyrazol-3-amine (10.11 mg, 91.0 μmol) were dissolved in anhydrous tetrahydrofuran (5 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (6.87 mg, 7.58 μmol) and potassium acetate (22.33 mg, 227.49 μmol)

were added. The reaction solution was stirred at 100°C for 1 hour. LCMS showed that the reaction was complete. The reaction system was evaporated to dryness via rotary evaporation, and then the resultant was subjected to liquid-liquid extraction (extraction with 12 mL of ethyl acetate three times) to obtain a yellow solid compound. The compound was purified by preparative high-performance liquid chromatography (Phenomenex Gemini-NX column, 10 μm silica, 30 mm in diameter, 100 mm in length) (using decreasingly polar mixtures of water (containing 0.05% aqueous ammonia) and acetonitrile (40% to 60%) as eluents) and lyophilized to obtain isopropyl trans-(4-(5-(2-(cyclopropylsulfonyl)-4-((1,5-dimethyl-1H-pyrazol-3-yl)amino)phenyl)thiazol-2-yl)cyclohexyl)carbamate as a white solid compound (4.55 mg).

MS m/z (ESI): 558.2 $[M+H]^+$;

$^1H$ NMR (400MHz, DMSO-$d_6$) δ ppm 9.04 (s, 1H), 7.97 (br s, 1H), 7.75-7.58 (m, 2H), 7.33 (br d, J = 8.4 Hz, 1H), 7.02 (br d, J = 6.1 Hz, 1H), 5.66 (s, 1H), 4.85-4.64 (m, 1H), 3.63 (s, 3H), 3.28-3.22 (m, 1H), 3.00-2.84 (m, 1H), 2.46-2.35 (m, 1H), 2.21 (s, 3H), 2.13 (br d, J = 11.3 Hz, 2H), 1.91 (br d, J = 10.4 Hz, 2H), 1.66-1.47 (m, 2H), 1.42-1.26 (m, 2H), 1.16 (br d, J = 6.0 Hz, 6H), 1.02-0.76 (m, 4H).

Example 50: Synthesis of isopropyl trans-N-[4-[5-[2-(cyclopropylsulfonimidoyl)-4-]((5-methyl-1H-pyrazol-3-yl)amino]phenyl]thiazol-2-yl]cyclohexyl]carbamate

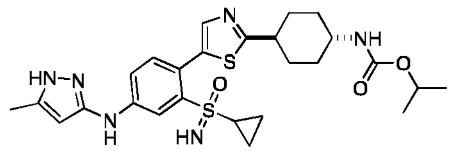

Synthetic Method

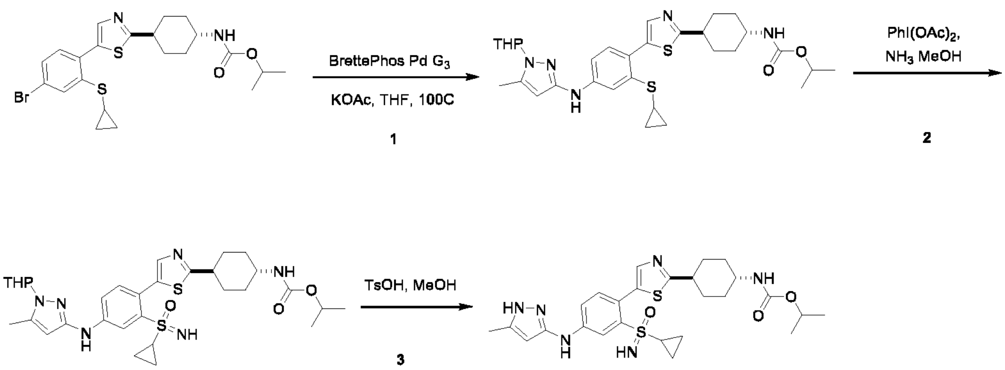


Step 1: Synthesis of isopropyl trans-N-[4-[5-[2-cyclopropylsulfanyl]-4-[(3-methyl-2-tetrahydropyran-2-yl-2H-pyrrol-5-yl)amino]phenyl]thiazol-2-yl]cyclohexyl]carbamate

Isopropyl trans-N-[4-[5-(4-bromo-2-cyclopropylsulfanyl-phenyl)thiazol-2-yl]cyclohexyl] carbamate (20 mg, 40.36 μmol) and 5-methyl-1-tetrahydropyran-2-yl-pyrazol-3-amine (8.05 mg, 44.40 μmol) were dissolved in tetrahydrofuran (4 mL), and methanesulfonato(2-dicyclohexylphosphino)-3,6-dimethoxy-2,4,6-triisopropyl-1,1-biphenyl)(2 -amino-1,1-biphenyl-2-yl)palladium (II) (3.66 mg, 4.04 μmol) and potassium acetate (3.96 mg, 40.36 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by thin-layer chromatography (silica, dichloromethane:methanol = 15:1) to obtain isopropyl trans-N-[4-[5-[2-cyclopropylsulfanyl]-4-[(3-methyl-2-tetrahydropyran-2-yl-2H-pyrrol-5-yl)amino]phenyl]thiazol-2-yl]cyclohexyl]carbamate as the product (20 mg).

MS m/z (ESI): 596.3 $[M+H]^+$;

Step 2: Synthesis of isopropyl trans-N-[4-[5-[2-(cyclopropylsulfonimidoyl)-4-]((5-methyl-1-tetrahydropyran-2-ylpyrazol-3-yl)amino]phenyl]thiazol-2-yl]cyclohexyl]carbamate

Isopropyl trans-N-[4-[5-[2-cyclopropylsulfanyl]-4-[(3-methyl-2-tetrahydropyran-2-yl-2H-pyrrol-5-yl)amino]phenyl]thiazol-2-yl]cyclohexyl]carbamate (33 mg, 55.39 μmol) was dissolved in a solution of ammonia in methanol (3 mL), and diacetoxyiodobenzene (267.59 mg, 830.79 μmol) was added. The reaction solution was stirred and reacted at 60°C for 16 hours. The completion of the reaction was detected by LCMS. The reaction solution was concentrated to dryness under reduced pressure and then purified by thin-layer chromatography (silica, dichloromethane:methanol = 15:1) to obtain isopropyl trans-N-[4-[5-[2-(cyclopropylsulfonimidoyl)-4-]((5-methyl-1-tetrahydropyran-2-ylpyrazol-3-yl)amino]phenyl] thiazol-2-yl]cyclohexyl]carbamate as the product (20 mg).

MS m/z (ESI): 627.3 $[M+H]^+$;

Step 3: Synthesis of isopropyl trans-N-[4-[5-[2-(cyclopropylsulfonimidoyl)-4-]((5-methyl-1H-pyrazol-3-yl)amino]phenyl]thiazol-2-yl]cyclohexyl]carbamate

Isopropyl trans-N-[4-[5-[2-(cyclopropylsulfonimidoyl)-4-]((5-methyl-1-tetrahydropyran-2-ylpyrazol-3-yl)amino]phenyl]thiazol-2-yl]cyclohexyl]carbamate (20 mg, 31.91 μmol) was dissolved in methanol (2 mL), and p-toluenesulfonic acid monohydrate (27.47 mg, 159.53 μmol) was added. The reaction solution was stirred and reacted at 50°C for 2 hours. The completion of the reaction was detected by LCMS. The reaction solution was concentrated to dryness under reduced pressure. The reaction solution was added with 9 mL of dichloromethane (3 mL × 3) and 5 mL of a saturated sodium bicarbonate solution. The organic phase was dried over anhydrous sodium sulfate and filtered. The filtrate was concentrated to dryness under reduced pressure and purified by preparative liquid chromatography (Phenomenex Gemini C18 column: 10 μm silica, 50 mm in diameter, 250 mm in length; using decreasingly polar mixtures of water (containing 0.225% formic acid) and acetonitrile as eluents) to obtain the compound isopropyl trans-N-[4-[5-[2-(cyclopropylsulfonimidoyl)-4-]((5-methyl-1H-pyrazol-3-yl)amino]phenyl]thiazol-2-yl]cyclohexyl]carbamate (2.46 mg).

MS m/z (ESI): 543.3 $[M+H]^+$;

$^1$H NMR (400MHz, DMSO-d6) δ 11.81 (br s, 1H), 8.93 (s, 1H), 8.03 (s, 1H), 7.70-7.55 (m, 2H), 7.24 (d, *J*= 8.5 Hz, 1H), 7.01 (br d, *J* = 7.8 Hz, 1H), 5.63 (s, 1H), 4.80-4.69 (m, 1H), 4.20 (s, 1H), 3.32-3.31 (m, 1H), 2.95-2.83 (m, 1H), 2.31-2.23 (m, 1H), 2.19 (s, 3H), 2.17-2.06 (m, 2H), 1.95-1.83 (m, 2H), 1.63-1.47 (m, 2H), 1.41-1.25 (m, 2H), 1.16 (d, *J* = 6.3 Hz, 6H), 0.94-0.73 (m, 4H).

Example 51: Synthesis of isopropyl trans-N-[4-[5-[4-[(5-methyl-1H-pyrazol-3-yl)amino]-2-(trifluoromethylsulfonyl)phenyl]thiazol-2-yl]cyclohexyl]carbamate

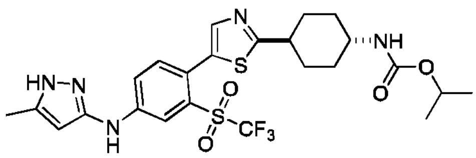

Synthetic Method

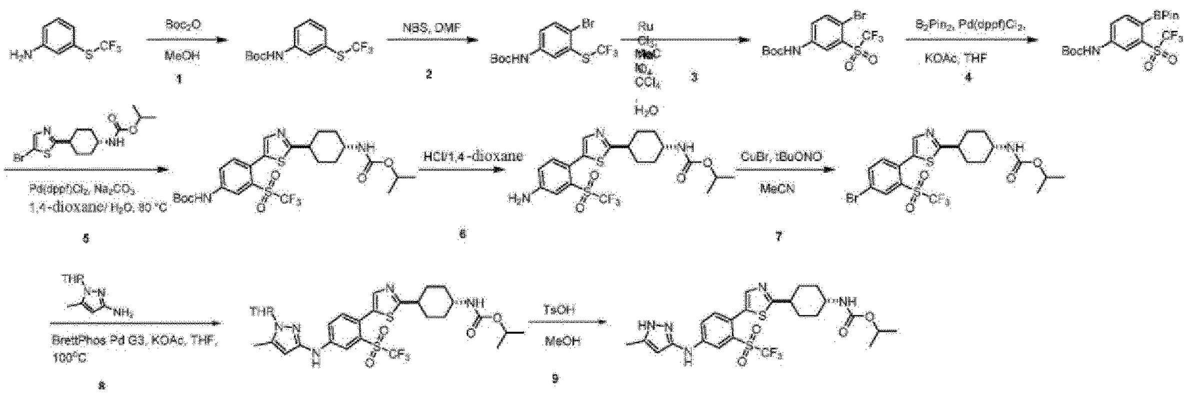


Step 1: Synthesis of tert-butyl (3-((trifluoromethyl)thio)phenyl)carbamate

3-(Trifluoromethylthio)aniline (1 g, 5.18 mmol) was dissolved in methanol (20 mL), and BOC anhydride (16.95 g, 77.64 mmol, 17.84 mL) was added at 25°C. The reaction solution appeared as a yellow suspension, and was stirred and reacted at 25°C for 16 h. The completion of the reaction was detected by LCMS. The reaction solution was concentrated to dryness under reduced pressure. Aqueous ammonia (60 mL) was added into the reaction solution. The reaction solution was stirred and reacted at 25°C for 2h, and then extracted with ethyl acetate (60 mL). The organic layer was dried over anhydrous magnesium sulfate and subjected to suction filtration. The filtrate was concentrated to dryness under reduced pressure. The resultant was purified by column chromatography (petroleum ether:ethyl acetate = 10:1) to obtain the compound tert-butyl (3-((trifluoromethyl)thio)phenyl)carbamate (600 mg, 2.05 mmol) as a white solid.
MS m/z (ESI): 238.0 $[M+H\text{-tert-butyl}]^+$;

Step 2: Synthesis of tert-butyl N-[4-bromo-3-(trifluoromethylsulfanyl)phenyl]carbamate

Tert-butyl (3-((trifluoromethyl)thio)phenyl)carbamate (560 mg, 1.91 mmol) was dissolved in N,N-dimethylformamide (5 mL), and N-bromosuccinimide (679.64 mg, 3.82 mmol) was added. The reaction solution appeared as a yellow suspension, and was stirred and reacted at 25°C for 16 h. The completion of the reaction was detected by LCMS. The reaction solution was concentrated to dryness under reduced pressure and purified by column chromatography (petroleum ether: ethyl acetate = 10:1) to obtain the compound tert-butyl N-[4-bromo-3-(trifluoromethylsulfanyl)phenyl]carbamate (470 mg, 1.26 mmol) as a yellow oil.
MS m/z (ESI): 318.0, 316.0 $[M+H\text{-tert-butyl}]^+$;

Step 3: Synthesis of tert-butyl N-[4-bromo-3-(trifluoromethylsulfonyl)phenyl]carbamate

Tert-butyl N-[4-bromo-3-(trifluoromethylsulfanyl)phenyl]carbamate (450 mg, 1.21 mmol) and anhydrous ruthenium trichloride (2.51 mg, 12.09 μmol) were dissolved in acetonitrile (2 mL), tetrachloromethane (2 mL) and water (4 mL), and sodium periodate (775.80 mg, 3.63 mmol, 200.98 μL) was added. The reaction solution appeared as a black suspension, and was stirred and reacted at 25°C for 16 h. The completion of the reaction was detected by LCMS. The reaction solution was concentrated to dryness under reduced pressure, added with water (10 mL), and extracted with dichloromethane (20 mL). The organic layer was washed with saturated saline (10 mL), dried over anhydrous magnesium sulfate, and subjected to suction filtration. The filtrate was concentrated to dryness under reduced pressure. The resultant was purified by column chromatography (petroleum ether:ethyl acetate = 5:1) to obtain the compound tert-butyl N-[4-bromo-3-(trifluoromethylsulfonyl)phenyl]carbamate (360 mg, 890.65 μmol) as a yellow oil.
MS m/z (ESI): 426.0, 428.0 $[M+Na]^+$;

Step 4: Synthesis of tert-butyl N-[4-(4,4,5,5-tetramethyl-1,3,2-dioxaborolan-2-yl)-3-(trifluoromethylsulfonyl)phenyl]carbamate

Tert-butyl N-[4-bromo-3-(trifluoromethylsulfonyl)phenyl]carbamate (360 mg, 890.65 μmol) and bis(pinacolato)diboron (904.68 mg, 3.56 mmol) were dissolved in anhydrous tetrahydrofuran (3 mL). Under the protection of nitrogen, [1,1-bis(diphenylphosphino)ferrocene]dichloropalladium - dichloromethane (72.73 mg, 89.06 μmol) and potassium acetate (262.23 mg, 2.67 mmol) were added. The reaction solution appeared as a yellow suspension, and was stirred and reacted at 80°C for 16 h. The completion of the reaction was detected by LCMS. The reaction solution was concentrated to dryness under reduced pressure. The resultant was purified by column chromatography (petroleum ether:ethyl acetate

= 5:1) to obtain the compound tert-butyl N-[4-(4,4,5,5-tetramethyl-1,3,2-dioxaborolan-2-yl)-3-(trifluoromethylsulfonyl)phenyl]carbamate (300 mg, 664.80 μmol) as a white solid.

MS m/z (ESI): 469.2 $[M+H_2O]^+$;

Step 5: Synthesis of isopropyl trans-N-[4-[5-[4-(tert-butoxycarbonylamino)-2-(trifluoromethylsulfonyl)phenyl]thiazol-2-yl]cyclohexyl]carbamate

Tert-butyl N-[4-(4,4,5,5-tetramethyl-1,3,2-dioxaborolan-2-yl)-3-(trifluoromethylsulfonyl) phenyl]carbamate (300 mg, 664.80 μmol) and isopropyl trans-N-[4-(5-bromothiazol-2-yl)cyclohexyl]carbamate (230.86 mg, 664.80 μmol) were dissolved in anhydrous dioxane (3 mL). Under the protection of nitrogen, [1,1-bis(diphenylphosphino) ferrocene]dichloropalladium - dichloromethane (54.29 mg, 66.48 μmol), sodium carbonate (140.92 mg, 1.33 mmol) and water (400 μL) were added. The reaction solution appeared as a yellow suspension, and was stirred and reacted at 80°C for 16 h. The completion of the reaction was detected by LCMS. The reaction solution was concentrated to dryness under reduced pressure. The resultant was purified by column chromatography (dichloromethane:methanol = 10:1) to obtain the compound isopropyl trans-N-[4-[5-[4-(tert-butoxycarbonylamino)-2-(trifluoromethylsulfonyl)phenyl]thiazol-2-yl]cyclohexyl]carbamate (300 mg, 507.05 μmol) as a white solid.

MS m/z (ESI): 592.3 $[M+H]^+$;

Step 6: Synthesis of isopropyl trans-N-[4-[5-[4-amino-2-(trifluoromethylsulfonyl) phenyl]thiazol-2-yl]cyclohexyl]carbamate

Isopropyl trans-N-[4-[5-[4-(tert-butoxycarbonylamino)-2-(trifluoromethylsulfonyl)phenyl] thiazol-2-yl]cyclohexyl]carbamate (300 mg, 507.05 μmol) was dissolved in anhydrous methanol (3 mL), and hydrochloric acid/dioxane (4M, 3 mL) was added. The reaction solution appeared as a yellow suspension, and was stirred and reacted at 25°C for 2 h. The completion of the reaction was detected by LCMS. The reaction solution was concentrated to dryness under reduced pressure, added with a saturated sodium carbonate solution (10 mL), and extracted with dichloromethane (20 mL) twice. The organic layer was dried over anhydrous magnesium sulfate and subjected to suction filtration. The filtrate was concentrated to dryness under reduced pressure to obtain crude isopropyl trans-N-[4-[5-[4-amino-2-(trifluoromethylsulfonyl)phenyl]thiazol-2-yl]cyclohexyl]carbamate (230mg) as a white solid.

MS m/z (ESI): 492.2 $[M+H]^+$;

Step 7: Synthesis of isopropyl trans-N-[4-[5-[4-bromo-2-(trifluoromethylsulfonyl) phenyl]thiazol-2-yl]cyclohexyl]carbamate

Isopropyl trans-N-[4-[5-[4-amino-2-(trifluoromethylsulfonyl)phenyl]thiazol-2-yl] cyclohexyl]carbamate (230 mg, 467.91 μmol) was dissolved in acetonitrile (5 mL). Under the protection of nitrogen, tert-butyl nitrite (144.75 mg, 1.40 mmol, 166.96 μL) was added at -30°C. The reaction solution was stirred and reacted for 1 h. Under the protection of nitrogen, cuprous bromide (167.80 mg, 1.17 mmol, 35.63 μL) was added at -30°C. The reaction solution appeared as a yellow suspension, and was stirred and reacted at 25°C for 16 h. The completion of the reaction was detected by LCMS. The reaction solution was concentrated to dryness under reduced pressure, added with saturated ammonium chloride solution (10 mL), and extracted with dichloromethane (20 mL). The organic layer was dried over anhydrous magnesium sulfate and subjected to suction filtration. The filtrate was concentrated to dryness under reduced pressure. The resultant was purified by column chromatography (petroleum ether:ethyl acetate = 1:1) to obtain the compound isopropyl trans-N-[4-[5-[4-bromo-2-(trifluoromethylsulfonyl)phenyl]thiazol-2-yl]cyclohexyl]carbamate (20 mg, 36.01 μmol) as a white solid.

MS m/z (ESI): 555.0, 557.0 $[M+H]^+$;

Step 8: Synthesis of isopropyl trans-N-[4-[5-[4-[(5-methyl-1-tetrahydropyran-2-yl-pyrazol-3-yl)amino]-2-(trifluoromethylsulfonyl)phenyl]thiazol-2-yl]cyclohexyl]carbamate

Isopropyl trans-N-[4-[5-[4-bromo-2-(trifluoromethylsulfonyl)phenyl]thiazol-2-yl] cyclohexyl]carbamate (10 mg, 18.00 μmol) and 5-methyl-1-(tetrahydro-2H-pyran-2-yl)-1H-pyrazol-3-amine (3.59 mg, 19.80 μmol) were dissolved in anhydrous tetrahydrofuran (2 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (1.63 mg, 1.80 μmol) and potassium acetate (1.77 mg, 18.00 μmol) were added. The reaction solution appeared as a black suspension, and was stirred and reacted at 100°C for 1 h. The completion of the reaction was detected by LCMS. The reaction solution was concentrated to dryness under reduced pressure. The resultant was purified by thin-layer chromatography (dichloromethane:methanol = 10:1) to obtain the compound isopropyl trans-N-[4-[5-[4-[(5-methyl-1-tetrahydropyran-2-yl-pyrazol-3-yl)amino]-2-(trifluoromethylsulfonyl)phenyl]thiazol-2-yl]cyclohexyl]carbamate (10 mg, 15.25 μmol) as a white solid.

MS m/z (ESI): 656.3 $[M+H]^+$;

Step 9: Synthesis of isopropyl trans-N-[4-[5-[4-[(5-methyl-1H-pyrazol-3-yl)amino]-2-(trifluoromethylsulfonyl)phenyl]thiazol-2-yl]cyclohexyl]carbamate

Isopropyl trans-N-[4-[5-[4-[(5-methyl-1-tetrahydropyran-2-yl-pyrazol-3-yl)amino]-2-(trifluoromethylsulfonyl)phenyl]thiazol-2-yl]cyclohexyl]carbamate (20 mg, 30.50 μmol) was dissolved in anhydrous methanol (3 mL), and p-toluenesulfonic acid monohydrate (26.26 mg, 152.50 μmol) was added. The reaction solution appeared as a black suspension, and was stirred and reacted at 50°C for 1 h. The completion of the reaction was detected by LCMS. The reaction solution was concentrated to dryness under reduced pressure, added with saturated sodium carbonate solution (5 mL), and extracted with dichloromethane (10 mL). The organic layer was dried over anhydrous magnesium sulfate and subjected to suction filtration. The filtrate was concentrated to dryness under reduced pressure. The resultant was purified by preparative liquid chromatography (PhenomenexGeminiC18 column, 10 μm silica, 50 mm in diameter, 250 mm in length; using decreasingly polar mixtures of water (containing 0.05% aqueous ammonia) and acetonitrile as eluents) to obtain the compound isopropyl trans-N-[4-[5-[4-[(5-methyl-1H-pyrazol-3-yl)amino]-2-(trifluoromethylsulfonyl)phenyl]thiazol -2-yl]cyclohexyl]carbamate (1.08 mg, 1.89 μmol) as a white solid.

MS m/z (ESI): 572.1 $[M+H]^+$;

$^1$H NMR (400MHz, METHANOL-$d_4$) δ ppm 8.14 (br s, 1H), 7.70 (br d, J = 8.5 Hz, 1H), 7.55 (s, 1H), 7.43 (d, J = 8.5 Hz, 1H), 5.79 (s, 1H), 4.82-4.70 (m, 1H), 3.55-3.42 (m, 1H), 3.07-2.96 (m, 1H), 2.31 (s, 3H), 2.24 (br d, J = 12.0 Hz, 2H), 2.12-2.05 (m, 2H), 1.76-1.63 (m, 2H), 1.48-1.34 (m, 2H), 1.24 (br d, J = 6.3 Hz, 6H).

Example 52: Synthesis of isopropyl trans-N-[4-[5-[2-cyclopropylsulfinyl]-4-[(5-methyl-1H-pyrazol-3-yl)amino]phenyl]thiazol-2-yl]cyclohexyl]carbamate

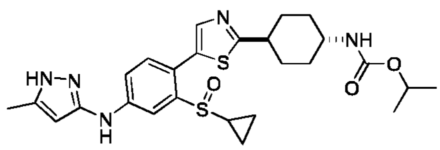

Synthetic Method

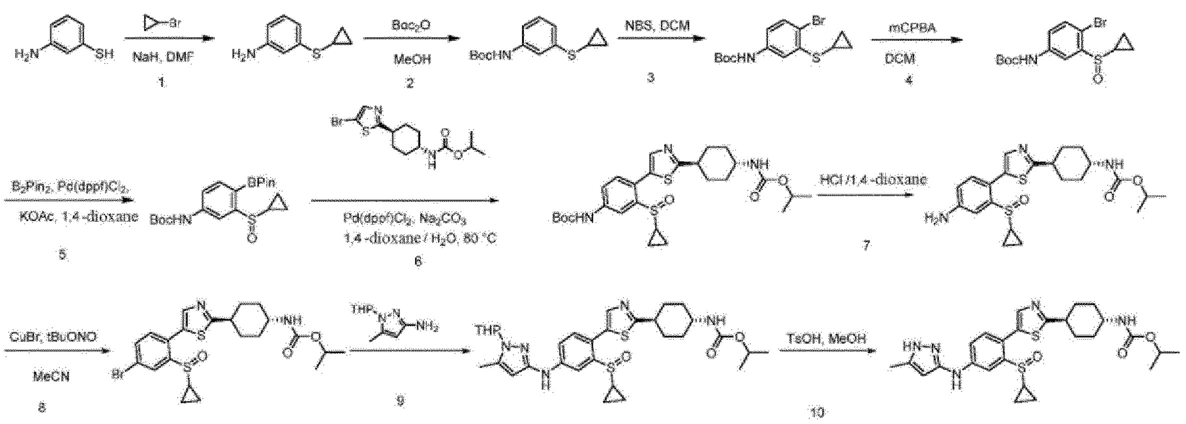


Step 1: Synthesis of 3-cyclopropylsulfanylaniline

3-Aminothiophenol (10 g, 79.88 mmol) was dissolved in N,N-dimethylformamide (60 mL). Under the protection of nitrogen, sodium hydride (3.19 g, 79.88 mmol) was added. The reaction solution was stirred and reacted at 30°C for 1 hour. Thereafter, cyclopropyl bromide (9.66 g, 79.88 mmol) was added into the reaction solution. The reaction solution was stirred under reflux at 100°C for 16 hours. The completion of the reaction was detected by LCMS. The reaction

solution was concentrated to dryness under reduced pressure and then purified by column chromatography (silica, dichloromethane/methanol = 20/1) to obtain 3-cyclopropylsulfanylaniline (10 g).

MS m/z (ESI):165.9 $[M+H]^+$;

Step 2: Synthesis of tert-butyl N-(3-cyclopropylsulfanylphenyl)carbamate

3-Cyclopropylsulfanylaniline (21 g, 127.08 mmol) was dissolved in methanol (315 mL), and Boc anhydride (416.01 g, 1.91 mol) was added. The reaction solution was stirred and reacted at 30°C for 16 hours. Aqueous ammonia (250 mL) was added into the reaction solution. The reaction solution was stirred and reacted at 0°C for 2 hours. The reaction solution was extracted with dichloromethane (300 mL) three times. The organic phase was concentrated to dryness under reduced pressure and then purified by column chromatography (silica, petroleum ether/ethyl acetate = 20/1) to obtain tert-butyl N-(3-cyclopropylsulfanylphenyl)carbamate as the product (25 g).

MS m/z (ESI):210.1 $[M+H\text{-tert-butyl}]^+$;

$^1H$ NMR (400MHz, DMSO-$d_6$) $\delta$ ppm 9.37 (s, 1H), 7.52 (s, 1H), 7.25-7.13 (m, 2H), 6.94 (dd, J = 1.5, 7.3 Hz, 1H), 2.26-2.14 (m, 1H), 1.47 (s, 9H), 1.14-1.01 (m, 2H), 0.62-0.50 (m, 2H).

Step 3: Synthesis of tert-butyl N-(4-bromo-3-cyclopropylsulfanyl-phenyl)carbamate

Tert-butyl N-(3-cyclopropylsulfanylphenyl)carbamate (25 g, 94.21 mmol) was dissolved in dichloromethane (1L), and then a solution of N-bromosuccinimide (16.77 g, 94.21 mmol) in dichloromethane (1L) was slowly added dropwise into the reaction solution. The reaction solution was stirred and reacted at -20°C for 3 hours. The completion of the reaction was detected by LCMS. The reaction solution was concentrated to dryness under reduced pressure and purified by column chromatography (silica, petroleum ether:ethyl acetate = 10:1) to obtain tert-butyl N-(4-bromo-3-cyclopropylsulfanyl-phenyl)carbamate as a white solid (10 g).

MS m/z (ESI): 289.9 $[M+H\text{-tert-butyl}]^+$;

$^1H$ NMR (400MHz, DMSO-$d_6$) $\delta$ ppm 9.55 (s, 1H), 7.80 (d, J = 2.3 Hz, 1H), 7.41 (d, J = 8.5 Hz, 1H), 7.24 (dd, J = 2.3, 8.8 Hz, 1H), 2.25-2.01 (m, 1H), 1.47 (s, 9H), 1.21-1.12 (m, 2H), 0.66-0.59 (m, 2H).

Step 4: Synthesis of tert-butyl N-(4-bromo-3-cyclopropylsulfinyl-phenyl)carbamate

Tert-butyl N-(4-bromo-3-cyclopropylsulfanyl-phenyl)carbamate (5 g, 14.52 mmol) was dissolved in dichloromethane (85 mL), and then a solution of m-chloroperoxybenzoic acid (2.95 g, 14.52 mmol) in dichloromethane (80 mL) was slowly added dropwise into the reaction solution. The reaction solution was stirred and reacted at 30°C for 16 hours. The completion of the reaction was detected by LCMS. The reaction solution was concentrated to dryness under reduced pressure, added with saturated sodium carbonate solution (30 mL), and extracted with 90 mL of dichloromethane (30 mL $\times$ 3). The organic phase was dried over anhydrous sodium sulfate and filtered. The filtrate was concentrated to dryness under reduced pressure and then purified by column chromatography (silica, petroleum ether:ethyl acetate = 5:1) to obtain tert-butyl N-(4-bromo-3-cyclopropylsulfinyl-phenyl)carbamate as the product (700 mg).

MS m/z (ESI): 305.9 $[M+H\text{-tert-butyl}]^+$;

Step 5: Synthesis of tert-butyl N-[3-cyclopropylsulfinyl-4-(4,4,5,5-tetramethyl-1,3,2-dioxaborolan-2-yl)phenyl]carbamate

Tert-butyl N-(4-bromo-3-cyclopropylsulfinyl-phenyl)carbamate (600 mg, 1.67 mmol) and bis(pinacolato)diboron (1.69 g, 6.66 mmol) were dissolved in tetrahydrofuran (15 mL). Under the protection of nitrogen, [1,1-bis(diphenylphosphino)ferrocene]dichloropalladium -dichloromethane (954.97 mg, 1.17 mmol) and potassium acetate (490.35 mg, 5.00 mmol) were added. The reaction solution was stirred and reacted at 100°C for 16 hours. The completion of the reaction was detected by LCMS. The reaction solution was added with 30 mL of dichloromethane (100 mL $\times$ 3) and 5 mL of water. The organic phase was dried over anhydrous sodium sulfate and filtered. The filtrate was concentrated to dryness under reduced pressure and purified by column chromatography (silica, petroleum ether:tetrahydrofuran = 3:1) to obtain tert-butyl N-[3-cyclopropylsulfinyl-4-(4,4,5,5-tetramethyl-1,3,2-dioxaborolan-2-yl)phenyl]carbamate as a white substance (360 mg).

MS m/z (ESI): 408.2 $[M+H]^+$;

Step 6: Synthesis of isopropyl trans-N-[4-[5-[4-(tert-butoxycarbonylamino)-2-cyclopropylsulfinyl-phenyl]thiazol-2-yl]cyclohexyl]carbamate

Tert-butyl N-[3-cyclopropylsulfinyl-4-(4,4,5,5-tetramethyl-1,3,2-dioxaborolan-2-yl) phenyl]carbamate (360 mg, 883.80 $\mu$mol) and isopropyl trans-N-[4-(5-bromothiazol-2-yl)cyclohexyl]carbamate (306.92 mg, 883.80 $\mu$mol) were dis-

solved in water (0.5 mL) and dioxane (5 mL). Thereafter, [1,1-bis(diphenylphosphino)ferrocene]dichloropalladium - dichloromethane (72.17 mg, 88.38 μmol) and sodium carbonate (281.02 mg, 2.65 mmol) were added under the condition where nitrogen gas was introduced. The reaction solution was stirred and reacted at 100°C for 16 hours. The completion of the reaction was detected by LCMS. The reaction solution was concentrated to dryness under reduced pressure and purified by column chromatography (silica, petroleum ether:tetrahydrofuran = 3:1) to obtain isopropyl trans-N-[4-[5-[4-(tert-butoxycarbonylamino)-2-cyclopropylsulfinyl-phenyl]thiazol-2-yl]cyclohexyl] carbamate as a yellow substance (230 mg).

MS m/z (ESI): 548.3 $[M+H]^+$;

Step 7: Synthesis of isopropyl trans-N-[4-[5-(4-amino-2-cyclopropylsulfinyl-phenyl)thiazol-2-yl]cyclohexyl]carbamate

Isopropyl trans-N-[4-[5-[4-(tert-butoxycarbonylamino)-2-cyclopropylsulfinyl-phenyl] thiazol-2-yl]cyclohexyl]carbamate (230 mg, 419.92 μmol) was dissolved in methanol (2 mL), and hydrochloric acid/dioxane (2.10 mL) was added. The reaction solution was stirred and reacted at 30°C for 2 hours. The completion of the reaction was detected by LCMS. The reaction solution was concentrated to dryness under reduced pressure and added with 5 mL of saturated sodium carbonate solution. The reaction solution was added with 9 mL of dichloromethane (3 mL × 3). The organic phase was dried over anhydrous sodium sulfate and filtered. The filtrate was concentrated to dryness under reduced pressure to obtain isopropyl trans-N-[4-[5-(4-amino-2-cyclopropylsulfinyl-phenyl)thiazol-2-yl]cyclohexyl]carbamate (100 mg).

MS m/z (ESI): 448.2 $[M+H]^+$;

Step 8: Synthesis of isopropyl trans-N-[4-[5-(4-bromo-2-cyclopropylsulfinyl-phenyl)thiazol-2-yl]cyclohexyl]carbamate

Isopropyl trans-N-[4-[5-(4-amino-2-cyclopropylsulfinyl-phenyl)thiazol-2-yl]cyclohexyl] carbamate (180 mg, 402.13 μmol) was dissolved in acetonitrile (2 mL), and tert-butyl nitrite (124.40 mg, 1.21 mmol) was added at 0°C. The reaction solution was stirred and reacted at 30°C for 1 hour. The cuprous bromide (144.22 mg, 1.01 mmol) was added into the reaction solution at 0°C. The reaction solution was stirred and reacted at 30°C for 15 hours. The completion of the reaction was detected by LCMS. The reaction solution was concentrated to dryness under reduced pressure and added with 50 mL of saturated ammonium chloride solution. The reaction solution was added with 90 mL of dichloromethane (30 mL × 3). The organic phase was dried over anhydrous sodium sulfate and filtered. The filtrate was concentrated to dryness under reduced pressure and purified by column chromatography (silica, petroleum ether:ethyl acetate = 3:2) to obtain isopropyl trans-N-[4-[5-(4-bromo-2-cyclopropylsulfinyl-phenyl)thiazol-2-yl]cyclohexyl]carbamate as a yellow substance (27 mg).

MS m/z (ESI): 511.1 $[M+H]^+$;

Step 9: Synthesis of isopropyl trans-N-[4-[5-[2-cyclopropylsulfinyl]-4-[(5-methyl-1-tetrahydropyran-2-yl-pyrazol-3-yl)amino]phenyl]thiazol-2-yl]cyclohexyl]carbamate

Isopropyl trans-N-[4-[5-(4-bromo-2-cyclopropylsulfinyl-phenyl)thiazol-2-yl]cyclohexyl] carbamate (17 mg, 33.24 μmol) and 5-methyl-1-tetrahydropyran-2-yl-pyrazol-3-amine (6.63 mg, 36.56 μmol) were dissolved in tetrahydrofuran (4 mL), and methanesulfonato(2-dicyclohexylphosphino)-3,6-dimethoxy-2,4,6-triisopropyl-1,1-biphenyl)(2 -amino-1,1-biphenyl-2-yl)palladium (II) (3.01 mg, 3.32 μmol) and potassium acetate (3.26 mg, 33.24 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by thin-layer chromatography (silica, dichloromethane/methanol = 15/1) to obtain isopropyl trans-N-[4-[5-[2-cyclopropylsulfinyl]-4-[(5-methyl-1-tetrahydropyran-2-yl-pyrazol-3-yl)amino]phenyl]thiazol-2-yl]cyclohexyl]carbamate as the product (10 mg).

MS m/z (ESI):612.3 $[M+H]^+$;

Step 10: Synthesis of isopropyl trans-N-[4-[5-[2-cyclopropylsulfinyl]-4-[(5-methyl-1H-pyrazol-3-yl)amino]phenyl]thiazol-2-yl]cyclohexyl]carbamate

Isopropyl trans-N-[4-[5-[2-cyclopropylsulfinyl]-4-[(5-methyl-1-tetrahydropyran-2-yl-pyrazol-3-yl)amino]phenyl]thiazol-2-yl]cyclohexyl]carbamate (32 mg, 52.30 μmol) was dissolved in methanol (3 mL), and p-toluenesulfonic acid monohydrate (45.03 mg, 261.52 μmol) was added. The reaction solution was stirred and reacted at 50°C for 2 hours. The completion of the reaction was detected by LCMS. The reaction solution was concentrated to dryness under reduced pressure. The reaction solution was added with 9 mL of dichloromethane (3 mL × 3) and 5 mL of a saturated sodium bicarbonate solution. The organic phase was dried over anhydrous sodium sulfate and filtered. The filtrate was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile as eluents) to obtain the compound isopropyl trans-N-[4-[5-[2-cyclopropylsulfinyl]-4-[(5-methyl-1H-pyrazol-3-yl)amino]phenyl]thiazol-2-yl]cyclohexyl]carbamate (2.36 mg).

MS m/z (ESI): 528.3 $[M+H]^+$;

$^1H$ NMR (400MHz, DMSO-d6) δ ppm 11.81 (s, 1H), 8.93 (s, 1H), 7.83 (d, J = 2.3 Hz, 1H), 7.68 (s, 1H), 7.54 (dd, J = 2.3, 8.5 Hz, 1H), 7.32 (d, J = 8.5 Hz, 1H), 7.02 (br d, J = 7.5 Hz, 1H), 5.63 (s, 1H), 4.82-4.70 (m, 1H), 3.29-3.26 (m, 1H), 2.98-2.88 (m 1H), 2.52-2.45 (m, 1H), 2.19 (s, 3H), 2.15-2.08 (m, 2H), 1.91 (br d, J = 10.4 Hz, 2H), 1.62-1.49 (m, 2H), 1.40-1.26 (m, 2H), 1.16 (d, J = 6.3 Hz, 6H), 0.90-0.75 (m, 2H), 0.72-0.60 (m, 1H), 0.57-0.49 (m, 1H).

Example 53: Synthesis of isopropyl trans-N-[4-[5-[4-[(1-methyltriazol-4-yl)amino]-2-(1-oxidophospholan-1-yl)phenyl]thiazol-2-yl]cyclohexyl]carbamate

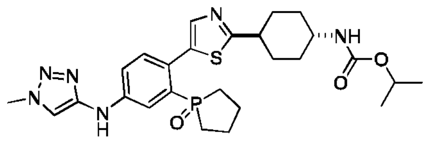

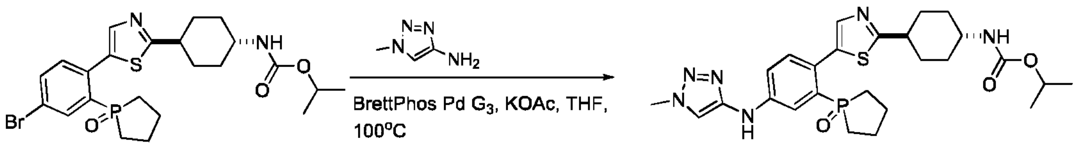


Isopropyl trans-N-[4-[5-[4-bromo-2-(1-oxidophospholan-1-yl)phenyl]thiazol-2-yl] cyclohexyl]carbamate (35 mg, 66.61 μmol) and 1-methyl-1H-1,2,3-triazol-4-amine (7.19 mg, 73.27 μmol) were dissolved in anhydrous tetrahydrofuran (3 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (6.04 mg, 6.66 μmol) and potassium acetate (6.54 mg, 66.61 μmol) were added. The reaction solution appeared as a black suspension, and was stirred and reacted at 100°C for 1 h. The completion of the reaction was detected by LCMS. The reaction solution was concentrated to dryness under reduced pressure, added with water (10 mL), and extracted with dichloromethane (20 mL). The organic layer was dried over anhydrous magnesium sulfate and subjected to suction filtration. The filtrate was concentrated to dryness under reduced pressure. The resultant was purified by preparative liquid chromatography (PhenomenexGeminiC18 column, 10 μm silica, 50 mm in diameter, 250 mm in length; using decreasingly polar mixtures of water (containing 0.05% aqueous ammonia) and acetonitrile as eluents) to obtain the compound isopropyl trans-N-[4-[5-[4-[(1-methyltriazol-4-yl)amino]-2-(1-oxidophospholan-1-yl)phenyl]thiazol-2-yl ]cyclohexyl]carbamate (10.49 mg, 19.33 μmol) as a white solid with a yield of 29.02%.

MS m/z (ESI): 543.3 $[M+H]^+$;

$^1H$ NMR (400MHz, DMSO-d6) δ ppm 8.99 (s, 1H), 7.85 (d, J = 7.5 Hz, 2H), 7.73-7.66 (m, 1H), 7.37-7.32 (m, 2H), 7.06-6.99 (m, 1H), 4.81-4.69 (m, 1H), 4.03 (s, 3H), 3.33-3.32 (m, 1H), 2.99-2.87 (m, 1H), 2.17-2.08 (m, 2H), 1.96-1.79 (m, 6H), 1.67-1.49 (m, 6H), 1.40-1.28 (m, 2H), 1.17 (d, J = 6.0 Hz, 6H).

Example 54: Synthesis of isopropyl trans-N-[4-[5-[2-(cyclopropylsulfonimidoyl)]-4-[(1-methyltriazol-4-yl)amino]phenyl]thiazol-2-yl]cyclohexyl]carbamate

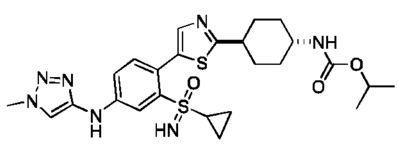

Synthetic Method

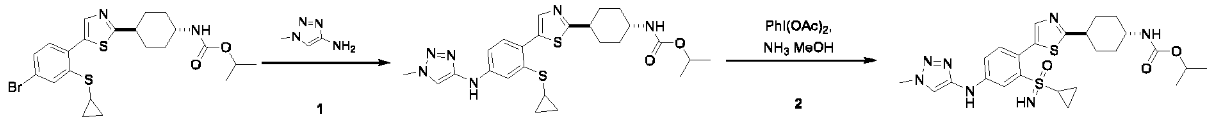


Step 1: Synthesis of isopropyl trans-N-[4-[5-[2-cyclopropylsulfanyl]-4-[(1-methyltriazol-4-yl)amino]phenyl]thiazol-2-yl]cyclohexyl]carbamate

Isopropyl trans-N-[4-[5-(4-bromo-2-cyclopropylsulfanyl-phenyl)thiazol-2-yl]cyclohexyl] carbamate (20 mg, 40.36 μmol) and 1-methyl-1H-1,2,3-triazol-4-amine (4.36 mg, 44.40 μmol) were dissolved in THF (2 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (3.66 mg, 4.04 μmol) and potassium acetate (3.96 mg, 40.36 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by thin-layer chromatography (silica, dichloromethane/methanol = 15/1) to obtain isopropyl trans-N-[4-[5-[2-cyclopropylsulfanyl]-4-[(1-methyltriazol-4-yl)amino]phenyl]thiazol-2-yl]cyclohexyl] carbamate as the product (20 mg).

MS m/z (ESI): 513.2 $[M+H]^+$;

Step 2: Synthesis of isopropyl trans-N-[4-[5-[2-(cyclopropylsulfonimidoyl)]-4-[(1-methyltriazol-4-yl)amino]phenyl]thiazol-2-yl]cyclohexyl]carbamate

Isopropyl trans-N-[4-[5-[2-cyclopropylsulfanyl]-4-[(1-methyltriazol-4-yl)amino]phenyl] thiazol-2-yl]cyclohexyl]carbamate (19 mg, 37.06 μmol) was dissolved in a solution of ammonia in methanol (3 mL), and diacetoxyiodobenzene (179.05 mg, 555.89 μmol) was added. The reaction solution was stirred and reacted at 60°C for 16 hours. The completion of the reaction was detected by LCMS. The reaction solution was concentrated to dryness under reduced pressure and then purified by preparative liquid chromatography (YMC-Actus Triart C18 column: 5μm silica, 30 mm in diameter, 150 mm in length; using decreasingly polar mixtures of water (containing 0.05% aqueous ammonia) and acetonitrile as eluents) to obtain the compound isopropyl trans-N-[4-[5-[2-(cyclopropylsulfonimidoyl)]-4-[(1-methyltriazol-4-yl)amino]phenyl]thiazol-2-yl]cyclohexyl]carbamate (1.02 mg).

MS m/z (ESI): 544.2 $[M+H]^+$;

$^1H$ NMR (400MHz, DMSO-d6) δ ppm 9.10 (s, 1H), 7.84-7.78 (m, 2H), 7.67 (s, 1H), 7.37-7.32 (m, 1H), 7.29-7.25 (m, 1H), 7.01 (br d, J = 7.9 Hz, 1H), 4.80-4.69 (m, 1H), 4.29 (s, 1H), 4.02 (s, 3H), 3.33-3.32 (m, 1H), 2.95-2.84 (m, 1H), 2.31-2.23 (m, 1H), 2.12 (br d, J = 12.2 Hz, 2H), 1.90 (br d, J = 10.0 Hz, 2H), 1.62-1.48 (m, 2H), 1.39-1.26 (m, 2H), 1.16 (d, J = 6.2 Hz, 6H), 0.91-0.76 (m, 4H).

Example 55: Synthesis of isopropyl trans-N-[4-[5-[2-cyclopropylsulfonyl]-4-[(1-methyltriazol-4-yl)amino]phenyl]thiazol-2-yl]cyclohexyl]carbamate

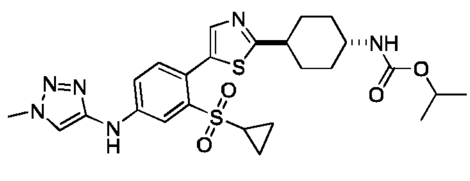

Synthetic Method

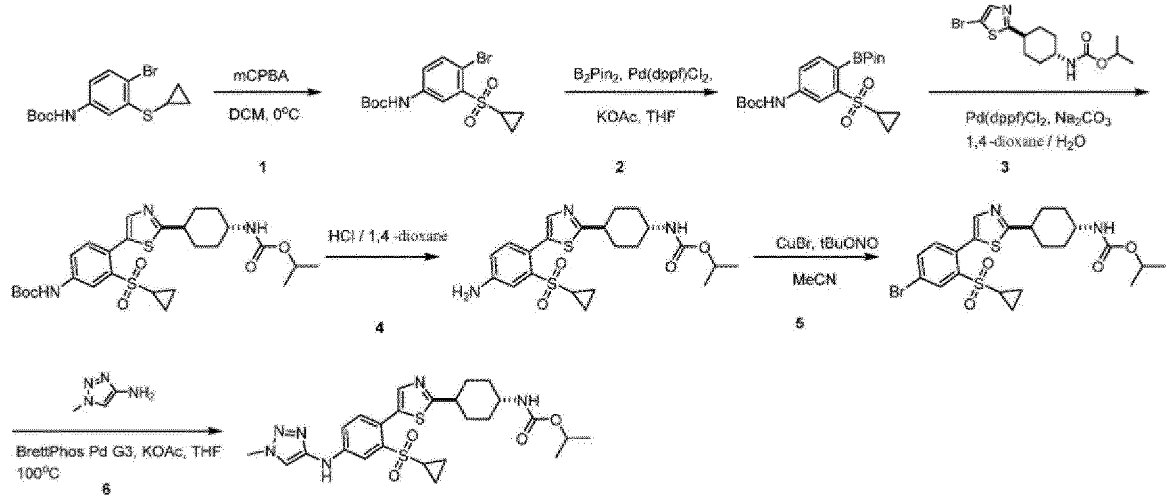


Step 1: Synthesis of tert-butyl N-(4-bromo-3-cyclopropylsulfonyl-phenyl)carbamate

Tert-butyl N-(4-bromo-3-cyclopropylsulfanyl-phenyl)carbamate (5.2 g, 15.10 mmol) was dissolved in dichloromethane (80 mL), and then a solution of m-chloroperoxybenzoic acid (12.27 g, 60.42 mmol) in dichloromethane (85 mL) was slowly added dropwise into the reaction solution. The reaction solution was stirred and reacted at 25°C for 16 hours. The completion of the reaction was detected by LCMS. The reaction solution was concentrated to dryness under reduced pressure, added with saturated sodium carbonate solution (30 mL), and extracted with 90 mL of dichloromethane (30 mL × 3). The organic phase was dried over anhydrous sodium sulfate and filtered. The filtrate was concentrated to dryness under reduced pressure and then purified by column chromatography (silica, petroleum ether:ethyl acetate = 5:1) to obtain tert-butyl N-(4-bromo-3-cyclopropylsulfonyl-phenyl)carbamate as the product (4.4 g).

MS m/z (ESI): 320.0 $[M+H\text{-tert-butyl}]^+$;

$^1$H NMR (400MHz, DMSO-d6) δ ppm 9.79 (s, 1H), 7.91 (d, J = 2.6 Hz, 1H), 7.59 (d, J = 8.7 Hz, 1H), 7.48 (dd, J = 2.6, 8.7 Hz, 1H), 2.63-2.58 (m, 1H), 1.48 (s, 9H), 1.02-0.79 (m, 3H), 0.72-0.60 (m, 1H).

Step 2: Synthesis of tert-butyl N-[3-cyclopropylsulfonyl-4-(4,4,5,5-tetramethyl-1,3,2-dioxaborolan-2-yl)phenyl]carbamate

Tert-butyl N-(4-bromo-3-cyclopropylsulfonyl-phenyl)carbamate (4.4 g, 11.69 mmol) and bis(pinacolato)diboron (11.88 g, 46.78 mmol) were dissolved in tetrahydrofuran (130 mL). Under the protection of nitrogen, [1,1-bis(diphenylphosphino)ferrocene]dichloropalladium - dichloromethane (954.97 mg, 1.17 mmol) and potassium acetate (3.44 g, 35.08 mmol) were added. The reaction solution was stirred and reacted at 100°C for 16 hours. The completion of the reaction was detected by LCMS. The reaction solution was added with 300 mL of dichloromethane (100 mL × 3) and 50 mL of water. The organic phase was dried over anhydrous sodium sulfate and filtered. The filtrate was concentrated to dryness under reduced pressure and purified by column chromatography (silica, petroleum ether:tetrahydrofuran = 2:1) to obtain tert-butyl N-[3-cyclopropylsulfonyl-4-(4,4,5,5-tetramethyl-1,3,2-dioxaborolan-2-yl)phenyl]carbamate as a white substance (4.4 g).

MS m/z (ESI): 268.1 $[M+H]^+$;

$^1$H NMR (400MHz, DMSO-$d_6$) δ ppm 9.81 (s, 1H), 8.05 (d, J = 1.8 Hz, 1H), 7.67 (dd, J = 2.0, 8.0 Hz, 1H), 7.48 (d, J = 8.0 Hz, 1H), 2.98-2.87 (m, 1H), 1.48 (s, 9H), 1.33 (s, 12H), 1.16-1.06 (m, 4H).

Step 3: Synthesis of isopropyl trans-N-[4-[5-[4-(tert-butoxycarbonylamino)-2-cyclopropylsulfonyl-phenyl]thiazol-2-yl]cyclohexyl]carbamate

Tert-butyl N-[3-cyclopropylsulfonyl-4-(4,4,5,5-tetramethyl-1,3,2-dioxaborolan-2-yl) phenyl]carbamate (1.3 g, 3.07 mmol) and isopropyl trans-N-[4-(5-bromothiazol-2-yl) cyclohexyl]carbamate (969.48 mg, 2.79 mmol) were dissolved in water (3 mL) and dioxane (30 mL). Thereafter, [1,1-bis(diphenylphosphino)ferrocene]dichloropalladium - dichloromethane (227.98 mg, 279.17 μmol) and sodium carbonate (887.67 mg, 8.38 mmol) were added under the condition where nitrogen gas was introduced. The reaction solution was stirred and reacted at 100°C for 16 hours. The completion of the reaction was detected by LCMS. The reaction solution was concentrated to dryness under reduced pressure and purified by column chromatography (silica, petroleum ether:tetrahydrofuran = 3:1) to obtain isopropyl trans-N-[4-[5-[4-(tert-butoxycarbonylamino)-2-cyclopropylsulfonyl-phenyl]thiazol-2-yl] cyclohexyl]carbamate as a yellow substance (1.25 g).

MS m/z (ESI): 564.3 $[M+H]^+$;

Step 4: Synthesis of isopropyl trans-N-[4-[5-(4-amino-2-cyclopropylsulfonyl-phenyl)thiazol-2-yl]cyclohexyl]carbamate

Isopropyl trans-N-[4-[5-[4-(tert-butoxycarbonylamino)-2-cyclopropylsulfonyl-phenyl] thiazol-2-yl]cyclohexyl]carbamate (1.25 g, 2.22 mmol) was dissolved in methanol (12 mL), and hydrochloric acid/dioxane (11.09 mL) was added. The reaction solution was stirred and reacted at 30°C for 2 hours. The completion of the reaction was detected by LCMS. The reaction solution was concentrated to dryness under reduced pressure and added with 5 mL of saturated sodium carbonate solution. The reaction solution was added with 9 mL of dichloromethane (3 mL × 3). The organic phase was dried over anhydrous sodium sulfate and filtered. The filtrate was concentrated to dryness under reduced pressure to obtain isopropyl trans-N-[4-[5-(4-amino-2-cyclopropylsulfonyl-phenyl)thiazol-2-yl]cyclohexyl]carbamate (900 mg).

MS m/z (ESI): 464.2 $[M+H]^+$;

Step 5: Synthesis of isopropyl trans-N-[4-[5-(4-bromo-2-cyclopropylsulfonyl-phenyl)thiazol-2-yl]cyclohexyl]carbamate

Isopropyl trans-N-[4-[5-(4-amino-2-cyclopropylsulfonyl-phenyl)thiazol-2-yl]cyclohexyl] carbamate (900 mg, 1.94 mmol) was dissolved in acetonitrile (30 mL), and tert-butyl nitrite (600.55 mg, 5.82 mmol) was added at 0°C. The reaction solution was stirred and reacted at 30°C for 1 hour. Cuprous bromide was added into the reaction solution at 0°C. The reaction solution was stirred and reacted at 30°C for 15 hours. The completion of the reaction was detected by LCMS. The reaction solution was concentrated to dryness under reduced pressure and added with 50 mL of saturated ammonium chloride solution. The reaction solution was added with 90 mL of dichloromethane (30 mL × 3). The organic phase was dried over anhydrous sodium sulfate and filtered. The filtrate was concentrated to dryness under reduced pressure and purified by column chromatography (silica, petroleum ether:ethyl acetate = 3:2) to obtain isopropyl trans-N-[4-[5-(4-bromo-2-cyclopropylsulfonyl-phenyl)thiazol-2-yl]cyclohexyl] carbamate as a yellow substance (578 mg).

MS m/z (ESI): 529.1 $[M+H]^+$;

Step 6: Synthesis of isopropyl trans-N-[4-[5-[2-cyclopropylsulfonyl]-4-[(1-methyltriazol-4-yl)amino]phenyl]thiazol-2-yl]cyclohexyl]carbamate

Isopropyl trans-N-[4-[5-(4-bromo-2-cyclopropylsulfonyl-phenyl)thiazol-2-yl]cyclohexyl] carbamate (150 mg, 284.36 μmol) and 1-methyl-1H-1,2,3-triazol-4-amine (30.69 mg, 312.80 μmol) were dissolved in tetrahydrofuran (15 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (25.78 mg, 28.44 μmol) and potassium acetate (27.91 mg, 284.36 μmol) were added. The reaction solution was stirred and reacted at 100°C for 2 hours. The completion of the reaction was detected by LCMS. The reaction solution was added with 18 mL of ethyl acetate (6 mL × 3) and 5 mL of water. The organic phase was dried over anhydrous sodium sulfate and filtered. The filtrate was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.225% formic acid) and acetonitrile as eluents) to obtain the compound isopropyl trans-N-[4-[5-[2-cyclopropylsulfonyl]-4-[(1-methyltriazol-4-yl)amino]phenyl]thiazol-2-yl] cyclohexyl]carbamate (44.83 mg).

MS m/z (ESI): 545.2 $[M+H]^+$;

$^1H$ NMR (400MHz, DMSO-d6) δ ppm 9.21 (s, 1H), 7.87-7.78 (m, 2H), 7.65 (s, 1H), 7.48-7.40 (m, 1H), 7.39-7.33 (m, 1H), 7.04 (br d, J = 7.7 Hz, 1H), 4.80-4.70 (m, 1H), 4.04 (s, 3H), 3.34-3.30 (m, 1H), 2.98-2.86 (m, 1H), 2.44-2.35 (m, 1H), 2.14 (br d, J = 12.0 Hz, 2H), 1.92 (br d, J = 10.1 Hz, 2H), 1.65-1.48 (m, 2H), 1.42-1.26 (m, 2H), 1.17 (d, J = 6.2 Hz, 6H), 1.01-0.92 (m, 2H), 0.91-0.86 (m, 2H).

Example 56: Synthesis of isopropyl trans-(4-(5-(4-((1H-indazol-3-yl)amino)-2-(cyclopropylsulfonyl)phenyl)thiazol-2-yl)cyclohexyl)carbamate

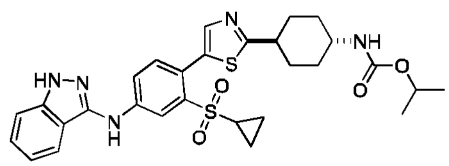

Synthetic Method

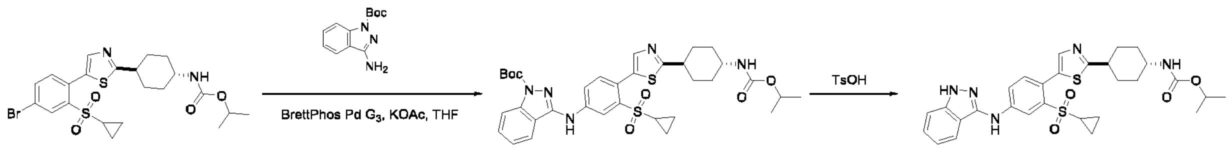


Step 1: Synthesis of tert-butyl trans-3-((3-(cyclopropylsulfonyl)-4-(2-(4-((isopropoxycarbonyl)amino)cyclohexyl)thiazol-5-yl)phenyl)amino)-1H-indazole-1-carboxylate

Isopropyl trans-(4-(5-(4-bromo-2-(cyclopropylsulfonyl)phenyl)thiazol-2-yl)cyclohexyl) carbamate (50 mg, 94.79 μmol) and tert-butyl 3-amino-1H-indazole-1-carboxylate (26.53 mg, 113.75 μmol) were dissolved in anhydrous tetrahydrofuran (5 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (8.59 mg, 9.48 μmol) and potassium acetate (27.91 mg, 284.36 μmol) were added. The reaction solution was stirred at 100°C for 1 hour. LCMS showed that the reaction was complete. The reaction system was evaporated to dryness via rotary evaporation, and the resultant was subjected to liquid-liquid extraction (extraction with 15 mL of ethyl acetate three times) to obtain tert-butyl trans-3-((3-(cyclopropylsulfonyl)-4-(2-(4-((isopropoxycarbonyl)amino)cyclohexyl)thiazol-5-yl)phenyl) amino)-1H-indazole-1-carboxylate as a yellow solid compound (50 mg, crude product).

MS m/z (ESI): 680.3 $[M+H]^+$;

Step 2: Synthesis of isopropyl trans-(4-(5-(4-((1H-indazol-3-yl)amino)-2-(cyclopropylsulfonyl)phenyl)thiazol-2-yl)cyclohexyl)carbamate

Tert-butyl trans-3-((3-(cyclopropylsulfonyl)-4-(2-(4-((isopropoxycarbonyl)amino) cyclohexyl)thiazol-5-yl)phenyl)amino)-1H-indazole-1-carboxylate (40 mg, 58.84 μmol) was dissolved in methanol (5 mL), and p-toluenesulfonic acid (50.66 mg, 294.18 μmol) was added. The reaction solution was stirred at 50°C for 30 minutes. LCMS showed that the reaction was complete. The reaction system was evaporated to dryness via rotary evaporation. The resultant was added with saturated sodium bicarbonate solution (20 mL), and then the mixture was extracted with dichloromethane (60 mL) three times. The dichloromethane phases were combined, dried over anhydrous sodium sulfate, and subjected to suction filtration. The resultant was purified by preparative high-performance liquid chromatography (Phenomenex Gemini-NX column, 10 μm silica, 30 mm in diameter, 100 mm in length) (using decreasingly polar mixtures of water (containing 0.05% aqueous ammonia) and acetonitrile (40% to 60%) as eluents) and lyophilized to obtain isopropyl trans-(4-(5-(4-((1H-indazol-3-yl)amino)-2-(cyclopropylsulfonyl)phenyl)thiazol-2-yl)cyclohexyl)carbamate as a white solid compound (10.55 mg).

MS m/z (ESI): 580.2 $[M+H]^+$;

$^1H$ NMR (400MHz, DMSO-d6) δ ppm 12.27 (br s, 1H), 9.60 (br s, 1H), 8.38 (br s, 1H), 8.19-7.90 (m, 2H), 7.69 (br s, 1H), 7.51-7.32 (m, 3H), 7.25-6.93 (m, 2H), 4.80-4.65 (m, 1H), 2.94-2.85 (m, 2H), 2.52-2.38 (m, 1H), 2.17-2.05 (m, 2H), 1.98-1.80 (m, 2H), 1.65-1.51 (m, 2H), 1.42-1.28 (m, 2H), 1.16 (d, J = 6.3 Hz, 6H), 1.10-0.83 (m, 4H).

Example 57: Synthesis of isopropyl trans-(4-(5-(2-(cyclopropylsulfonyl)-4-(pyrazolo[1,5-a]pyridin-2-ylamino)phenyl)thiazol-2-yl)cyclohexyl)carbamate

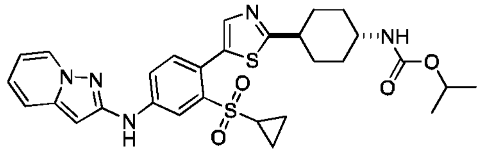

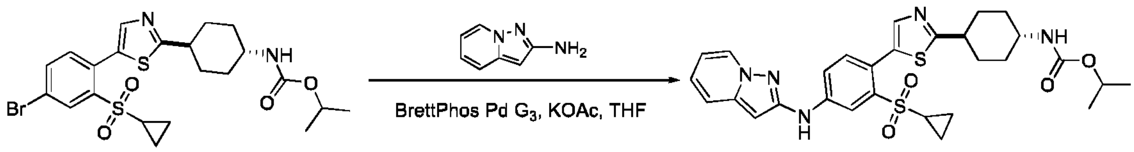


Isopropyl trans-(4-(5-(4-bromo-2-(cyclopropylsulfonyl)phenyl)thiazol-2-yl)cyclohexyl) carbamate (50 mg, 94.79 μmol) and 2-aminopyrazolo[1,5-a]pyridine (13.88 mg, 104.27 μmol) were dissolved in anhydrous tetrahydrofuran (5 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (8.59 mg, 9.48 μmol) and potassium acetate (27.91 mg, 284.36 μmol) were added. The reaction solution was stirred at 100°C for 1 hour. LCMS showed that the reaction was complete. The reaction system was evaporated to dryness via rotary evaporation, and the resultant was subjected to liquid-liquid extraction (extraction with 15 mL of ethyl acetate three times) to obtain a yellow solid compound. The compound was purified by preparative high-performance liquid chromatography (Phenomenex Gemini-NX column, 10 μm silica, 30 mm in diameter, 100 mm in length) (using decreasingly polar mixtures of water (containing 0.05% aqueous ammonia) and acetonitrile (43% to 63%) as eluents) and lyophilized to obtain isopropyl trans-(4-(5-(2-(cyclopropylsulfonyl)-4-(pyrazolo[1,5-a]pyridin-2-ylamino)phenyl)thiazol-2-yl)cyclohexyl)carbamate as a white solid compound (16.72 mg).

MS m/z (ESI): 580.2 $[M+H]^+$;

$^1$H NMR (400MHz, DMSO-d6) δ ppm 9.65 (s, 1H), 8.52 (d, J = 6.9 Hz, 1H), 8.17 (d, J = 2.4 Hz, 1H), 7.93 (dd, J = 2.4, 8.5 Hz, 1H), 7.69 (s, 1H), 7.52-7.40 (m, 2H), 7.20-7.14 (m, 1H), 7.02 (br d, J = 7.6 Hz, 1H), 6.72 (t, J=6.9 Hz, 1H), 6.12 (s, 1H), 4.80-4.68 (m, 1H), 2.98-2.85 (m, 1H), 2.46-2.41 (m, 1H), 2.14 (br d, J = 12.0 Hz, 2H), 1.92 (br d, J = 10.3 Hz, 2H), 1.64-1.50 (m, 2H), 1.41-1.27 (m, 2H), 1.16 (d, J = 6.1 Hz, 6H), 1.03-0.95 (m, 2H), 0.95-0.88 (m, 2H).

Example 58: Synthesis of isopropyl N-[4-[5-[2-cyclopropylsulfonyl]-4-[(1-methyltriazol-4-yl)amino]phenyl]thiazol-2-yl]-1-bicyclo[2.2.2]octyl]carbamate

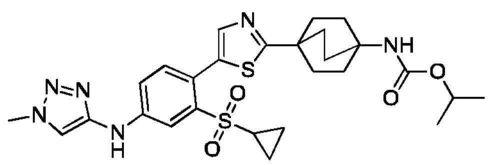

Synthetic Method

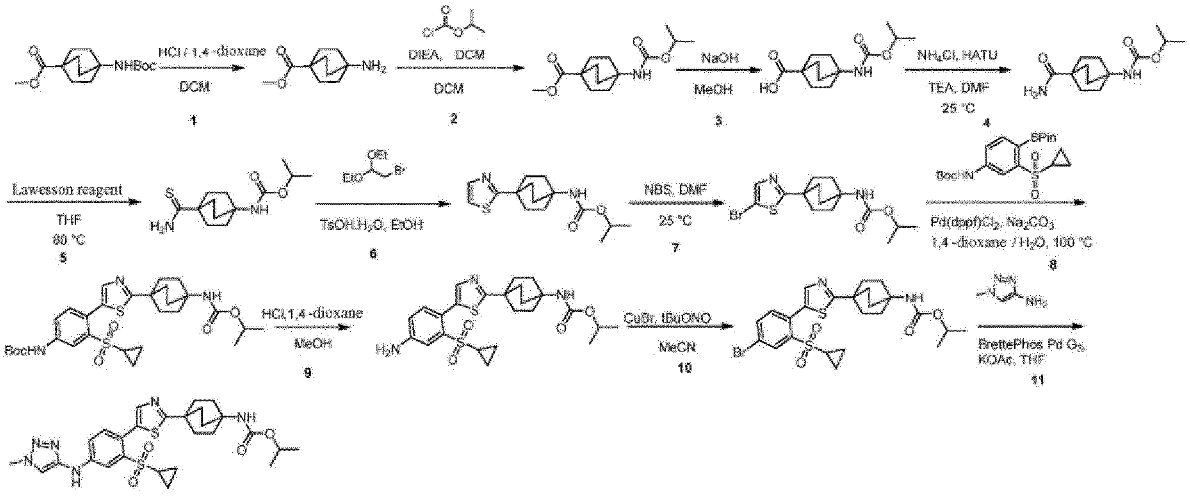

Step 1: Synthesis of methyl 4-aminobicyclo[2.2.2]octane-1-carboxylate

4-(Tert-butoxycarbonylamino)methylbicyclo[2.2.2]octane-1-carboxylate (1 g, 3.53 mmol) was dissolved in methanol (15 mL), and hydrochloric acid/dioxane (4M, 17.65 mL) was added. The reaction solution appeared as a yellow suspension, and was stirred and reacted at 25°C for 1 hour. TLC (dichloromethane:methanol = 10:1) detection showed that the reaction was complete. The reaction solution was concentrated to dryness under reduced pressure, added with a saturated sodium carbonate solution (50 mL), and extracted with dichloromethane (100 mL). The organic layer was dried over anhydrous magnesium sulfate and subjected to suction filtration. The filtrate was concentrated to dryness under reduced pressure to obtain methyl 4-aminobicyclo[2.2.2]octane-1-carboxylate as a white solid compound (700 mg, crude product).

Step 2: Synthesis of methyl 4-(isopropoxycarbonylamino)bicyclo[2.2.2]octane-1-carboxyl

Methyl 4-aminobicyclo[2.2.2]octane-1-carboxylate (700 mg, 3.82 mmol) and isopropyl chloroformate (1.40 g, 11.46 mmol, 1.59 mL) were dissolved in anhydrous dichloromethane (5 mL). Under the protection of nitrogen, N,N-diisopropylethylamine (1.48 g, 11.46 mmol, 2.00 mL) was added. The reaction solution appeared as a yellow suspension, and was stirred and reacted at 25°C for 1 hour. TLC (petroleum ether:ethyl acetate = 3:1) detection showed that the reaction was complete. The reaction solution was concentrated to dryness under reduced pressure and purified by column chromatography (petroleum ether:ethyl acetate = 3:1) to obtain the compound methyl 4-(isopropoxycarbonylamino)bicyclo[2.2.2]octane-1-carboxyl (900 mg, 3.34 mmol) as a while solid.

$^1$H NMR (400MHz, DMSO-d6) $\delta$ = 6.72 (br s, 1H), 4.76 - 4.60 (m, 1H), 3.56 (s, 3H), 1.75 (s, 12H), 1.13 (d, J = 6.3 Hz, 6H).

Step 3: Synthesis of 4-(isopropoxycarbonylamino)bicyclo[2.2.2]octane-1-carboxylic acid

Methyl 4-(isopropoxycarbonylamino)bicyclo[2.2.2]octane-1-carboxyl (850 mg, 3.16 mmol) was dissolved in anhydrous methanol (15 mL), and sodium hydroxide (2M, 6.31 mL) was added. The reaction solution appeared as a colorless suspension, and was stirred and reacted at 25°C for 2 hours. TLC (dichloromethane:methanol = 10:1) detection showed that the reaction was complete. The reaction solution was concentrated to dryness under reduced pressure and diluted with dichloromethane (10 mL). The pH of the mixture was adjusted to 3 with 2 mol of hydrochloric acid (10 mL), and then the resultant was extracted with dichloromethane (10 mL) twice. The organic layer was dried over anhydrous magnesium sulfate and subjected to suction filtration. The filtrate was concentrated to dryness under reduced pressure to obtain 4-(isopropoxycarbonylamino)bicyclo[2.2.2]octane-1-carboxylic acid (430 mg, crude product) as a white solid.

Step 4: Synthesis of isopropyl N-(4-carbamoyl-1-bicyclo[2.2.2]octyl)carbamate

4-(Isopropoxycarbonylamino)bicyclo[2.2.2]octane-1-carboxylic acid (400 mg, 1.57 mmol) was dissolved in N,N-dimethylformamide (15 mL), and O-(7-azabenzotriazol-1-yl)-N,N,N,N-tetramethyluronium hexafluorophosphate (893.58 mg, 2.35 mmol), ammonium chloride (251.42 mg, 4.70 mmol) and triethylamine (475.61 mg, 4.70 mmol, 654.21 $\mu$L) were added. The reaction solution appeared as a yellow suspension, and was stirred and reacted at 25°C for 2 hours. The completion of the reaction was detected by LCMS. The reaction solution was concentrated to dryness under reduced pressure. The resultant was purified by column chromatography (dichloromethane:methanol = 10:1) to obtain the compound isopropyl N-(4-carbamoyl-1-bicyclo[2.2.2]octyl)carbamate (350 mg, 1.38 mmol) as a white solid.

MS m/z (ESI): 255.2 $[M+H]^+$;

Step 5: Synthesis of isopropyl N-(4-carbamothioyl-1-bicyclo[2.2.2]octyl)carbamate

Isopropyl N-(4-carbamoyl-1-bicyclo[2.2.2]octyl)carbamate (350 mg, 1.38 mmol) was dissolved in anhydrous tetrahydrofuran (4 mL), and Lawesson reagent (278.31 mg, 688.1 $\mu$mol) was added. The reaction solution appeared as a yellow suspension, and was stirred and reacted at 80°C for 1 hour. The completion of the reaction was detected by LCMS. The reaction solution was concentrated to dryness under reduced pressure. The resultant was purified by column chromatography (dichloromethane:methanol = 10:1) to obtain the compound isopropyl N-(4-carbamothioyl-1-bicyclo[2.2.2]octyl)carbamate (300 mg, 1.11 mmol) as a yellow solid.

MS m/z (ESI): 271.2 $[M+H]^+$;

Step 6: Synthesis of isopropyl N-(4-thiazol-2-yl-1-bicyclo[2.2.2]octyl)carbamate

Isopropyl N-(4-carbamothioyl-1-bicyclo[2.2.2]octyl)carbamate (290 mg, 1.07 mmol) was dissolved in anhydrous

ethanol (5 mL), and p-toluenesulfonic acid monohydrate (408.02 mg, 2.15 mmol) and bromoacetaldehyde diethyl acetal (634.09 mg, 3.22 mmol, 484.04 μL) were added. The reaction solution appeared as a light yellow suspension, and was stirred and reacted at 80°C for 2 hours. The completion of the reaction was detected by LCMS. The reaction solution was concentrated to dryness under reduced pressure. The resultant was purified by column chromatography (dichloromethane:methanol = 15:1) to obtain the compound isopropyl N-(4-thiazol-2-yl-1-bicyclo[2.2.2]octyl)carbamate (300mg) as a light orange solid.

MS m/z (ESI): 295.1 $[M+H]^+$;

Step 7: Synthesis of isopropyl N-[4-(5-bromothiazol-2-yl)-1-bicyclo[2.2.2]octyl] carbamate

Isopropyl N-(4-thiazol-2-yl-1-bicyclo[2.2.2]octyl)carbamate (170 mg, 577.42 μmol) was dissolved in N,N-dimethylformamide (3 mL), and N-bromosuccinimide (308.32 mg, 1.73 mmol) was added. The reaction solution appeared as a yellow suspension, and was stirred and reacted at 50°C for 2 h. The completion of the reaction was detected by LCMS. The reaction solution was concentrated to dryness under reduced pressure. The resultant was purified by thin-layer chromatography (petroleum ether:tetrahydrofuran = 4:1) to obtain the compound isopropyl N-[4-(5-bromothiazol-2-yl)-1-bicyclo[2.2.2]octyl]carbamate (90 mg, 241.09 μmol) as a white solid.

MS m/z (ESI): 372.9, 374.9 $[M+H]^+$;

$^1$H NMR (400MHz, DMSO-d6) δ = 7.74 (s, 1H), 6.86 - 6.72 (m, 1H), 4.77 - 4.65 (m, 1H), 1.88 (br d, J = 5.0 Hz, 12H), 1.15 (d, J=6.3 Hz, 6H).

Step 8: Synthesis of isopropyl N-[4-[5-[4-(tert-butoxycarbonylamino)-2-cyclopropylsulfonyl-phenyl]thiazol-2-yl]-1-bicyclo[2.2.2]octyl]carbamate

Isopropyl N-[4-(5-bromothiazol-2-yl)-1-bicyclo[2.2.2]octyl]carbamate (120 mg, 321.45 μmol) and tert-butyl N-[3-cyclopropylsulfonyl-4-(4,4,5,5-tetramethyl-1,3,2-dioxaborolan-2-yl)phenyl]carbamate (149.69 mg, 353.60 μmol) were dissolved in anhydrous dioxane (4 mL). Under the protection of nitrogen, [1,1-bis(diphenylphosphino)ferrocene]dichloropalladium - dichloromethane (26.25 mg, 32.15 μmol), sodium carbonate (68.14 mg, 642.90 μmol) and water (400 μL) were added. The reaction solution appeared as a yellow suspension, and was stirred and reacted at 100°C for 16 hours. The completion of the reaction was detected by LCMS. The reaction solution was concentrated to dryness under reduced pressure. The resultant was purified by thin-layer chromatography (dichloromethane:methanol = 10:1) to obtain the compound isopropyl N-[4-[5-[4-(tert-butoxycarbonylamino)-2-cyclopropylsulfonyl-phenyl]thiazol-2-yl]-1-bicyclo[2.2.2]octyl]carbamate (100 mg, 169.56 μmol) as a white solid.

MS m/z (ESI): 590.2 $[M+H]^+$;

Step 9: Synthesis of isopropyl N-[4-[5-(4-amino-2-cyclopropylsulfonyl-phenyl)thiazol-2-yl]-1-bicyclo[2.2.2]octyl]carbamate

Isopropyl N-[4-[5-[4-(tert-butoxycarbonylamino)-2-cyclopropylsulfonyl-phenyl]thiazol-2-yl]-1-bicyclo[2.2.2]octyl]carbamate (100 mg, 169.56 μmol) was dissolved in anhydrous methanol (2 mL), and hydrochloric acid/dioxane (4M, 847.80 μL) was added. The reaction solution appeared as a yellow suspension, and was stirred and reacted at 25°C for 2 hours. The completion of the reaction was detected by LCMS. The reaction solution was concentrated to dryness under reduced pressure, added with a saturated sodium carbonate solution (10 mL), and extracted with dichloromethane (20 mL) twice. The organic layer was dried over anhydrous magnesium sulfate and subjected to suction filtration. The filtrate was concentrated to dryness under reduced pressure to obtain crude isopropyl N-[4-[5-(4-amino-2-cyclopropylsulfonyl-phenyl)thiazol-2-yl]-1-bicyclo[2.2.2]octyl]carbamate (80 mg) as a white solid.

MS m/z (ESI): 490.2 $[M+H]^+$;

Step 10: Synthesis of isopropyl N-[4-[5-(4-bromo-2-cyclopropylsulfonyl-phenyl)thiazol-2-yl]-1-bicyclo[2.2.2]octyl]carbamate

Isopropyl N-[4-[5-(4-amino-2-cyclopropylsulfonyl-phenyl)thiazol-2-yl]-1-bicyclo[2.2.2] octyl]carbamate (80 mg, 163.38 μmol) was dissolved in acetonitrile (5 mL). Under the protection of nitrogen, tert-butyl nitrite (50.54 mg, 490.15 μmol, 58.30 μL) was added at -30°C. The reaction solution was stirred and reacted for 1 hour. Under the protection of nitrogen, cuprous bromide (58.59 mg, 408.46 μmol) was added at -30°C. The reaction solution appeared as a yellow suspension, and was stirred and reacted at 25°C for 16 hours. The completion of the reaction was detected by LCMS. The reaction solution was concentrated to dryness under reduced pressure, added with saturated ammonium chloride solution (10 mL), and extracted with dichloromethane (20 mL). The organic layer was dried over anhydrous magnesium sulfate and subjected to suction filtration. The filtrate was concentrated to dryness under reduced pressure. The resultant

was purified by thin-layer chromatography (petroleum ether:tetrahydrofuran = 3:1) to obtain the compound isopropyl N-[4-[5-(4-bromo-2-cyclopropylsulfonyl-phenyl)thiazol-2-yl]-1-bicyclo[2.2.2]octyl]carbamate (60 mg, 108.40 μmol) as a white solid.

MS m/z (ESI): 553.2, 555.1 $[M+H]^+$;

Step 11: Synthesis of isopropyl N-[4-[5-[2-cyclopropylsulfonyl-4-[(1-methyltriazol-4-yl)amino]phenyl]thiazol-2-yl]-1-bicyclo[2.2.2]octyl]carbamate

Isopropyl N-[4-[5-(4-bromo-2-cyclopropylsulfonyl-phenyl)thiazol-2-yl]-1-bicyclo[2.2.2] octyl]carbamate (20 mg, 36.13 μmol) and 1-methyl-1H-1,2,3-triazol-4-amine (3.90 mg, 39.74 μmol) were dissolved in anhydrous tetrahydrofuran (2 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (3.28 mg, 3.61 μmol) and potassium acetate (3.55 mg, 36.13 μmol) were added. The reaction solution appeared as a black suspension, and was stirred and reacted at 100°C for 1 hour. The completion of the reaction was detected by LCMS. The reaction solution was concentrated to dryness under reduced pressure. The resultant was purified by preparative liquid chromatography (YMC-ActusTriartC18 column, 5 μm silica, 30 mm in diameter, 150 mm in length; using decreasingly polar mixtures of water (containing 0.05% aqueous ammonia) and acetonitrile as eluents) to obtain the compound isopropyl N-[4-[5-[2-cyclopropylsulfonyl-4-[(1-methyltriazol-4-yl)amino]phenyl]thiazol-2-yl]-1-bicyclo[ 2.2.2]octyl]carbamate (1.39 mg) as a white solid.

MS m/z (ESI): 571.1 $[M+H]^+$;

$^1$H NMR (400MHz, DMSO-d6) δ 9.20 (s, 1H), 7.84 (s, 1H), 7.81 (d, J = 2.5 Hz, 1H), 7.64 (s, 1H), 7.42 (d, J = 2.5 Hz, 1H), 7.37 (s, 1H), 6.83 - 6.74 (m, 1H), 4.77 - 4.67 (m, 1H), 4.04 (s, 3H), 2.41 - 2.35 (m, 1H), 1.96 (br s, 6H), 1.90 (br d, J = 9.0 Hz, 6H), 1.16 (d, J = 6.0 Hz, 6H), 0.99 - 0.92 (m, 2H), 0.89 - 0.82 (m, 2H).

Example 59: Synthesis of isopropyl N-[4-[5-[2-cyclopropylsulfonyl]-4-[(1-methyltriazol-4-yl)amino]phenyl]thiazol-2-yl]phenyl]carbamate

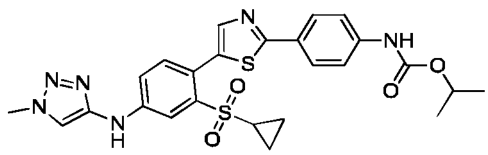

Synthetic Method

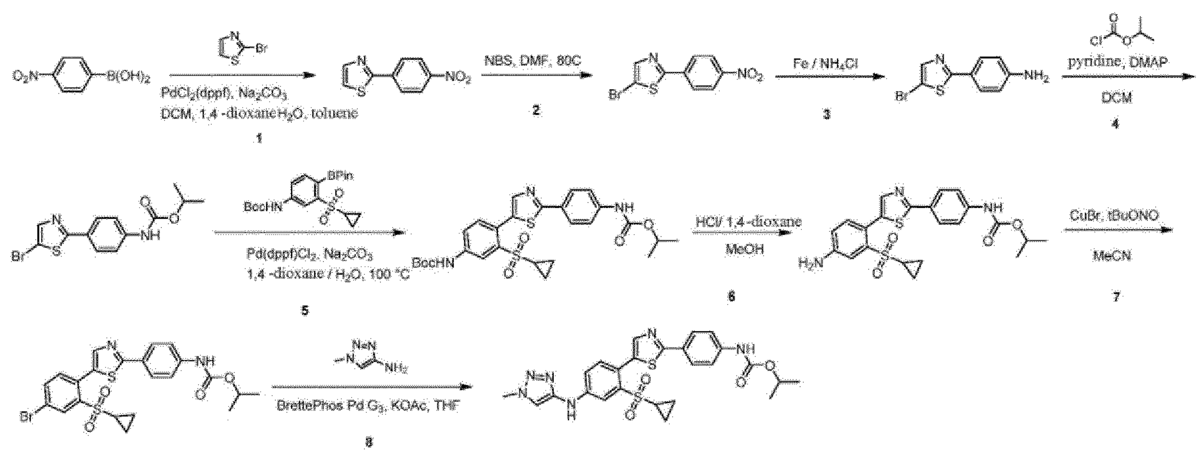


Step 1: Synthesis of (4-nitrophenyl)thiazole

4-Nitrophenylboronic acid (10 g, 59.91 mmol) and 2-bromothiazole (11.10 g, 67.70 mmol) were dissolved in toluene (40 mL), water (16 mL) and dioxane (40 mL). Under the protection of nitrogen, [1,1-bis(diphenylphosphino)ferrocene]dichloropalladium - dichloromethane (2.93 g, 3.59 mmol) and sodium carbonate (15.87 g, 149.77 mmol) were added. The reaction solution was stirred and reacted at 80°C for 16 hours. The completion of the reaction was detected by LCMS. The reaction solution was concentrated to dryness under reduced pressure and purified by column chromatography (silica, petroleum ether:ethyl acetate = 5:1) to obtain (4-nitrophenyl)thiazole as a white solid compound (4.44 g).

MS m/z (ESI): 206.9 $[M+H]^+$;

$^1H$ NMR (400MHz, DMSO-d6) δ 8.39 - 8.27 (m, 2H), 8.26 - 8.16 (m, 2H), 8.06 (d, J = 3.1 Hz, 1H), 7.99 (d, J = 3.1 Hz, 1H).

Step 2: Synthesis of 5-bromo-2-(4-nitrophenyl)thiazole

(4-Nitrophenyl)thiazole (4.2 g, 20.37 mmol) was dissolved in N,N-dimethylformamide (15 mL), and then a solution of N-bromosuccinimide (10.88 g, 61.12 mmol) in N,N-dimethylformamide (25 mL) was slowly added dropwise into the reaction solution. The reaction solution was stirred and reacted at 80°C for 15 minutes. The completion of the reaction was detected by LCMS. The reaction solution was added with 1L of water and then subjected to suction filtration. The filter cake was washed with 50 mL of methanol to obtain 5-bromo-2-(4-nitrophenyl)thiazole as a white solid compound (5.38 g).

MS m/z (ESI): 285.0, 287.0 $[M+H]^+$;

$^1H$ NMR (400MHz, DMSO-d6) δ 8.35 - 8.27 (m, 2H), 8.17 - 8.10 (m, 3H).

Step 3: Synthesis of 4-(5-bromothiazol-2-yl)aniline

5-Bromo-2-(4-nitrophenyl)thiazole (5.38 g, 18.87 mmol) was dissolved in ethanol (125 mL), tetrahydrofuran (125 mL) and water (62.5 mL), and iron powder (5.27 g, 94.35 mmol) and ammonium chloride (5.05 g, 94.35 mmol) were added. The reaction solution was stirred and reacted at 80°C for 2 hours. The completion of the reaction was detected by LCMS. The reaction solution was filtered. The filtrate was concentrated to dryness under reduced pressure and then purified by column chromatography (silica, petroleum ether: ethyl acetate = 5:1) to obtain 4-(5-bromothiazol-2-yl)aniline as a yellow solid compound (2.8 g).

MS m/z (ESI): 255.0, 257.0 $[M+H]^+$;

$^1H$ NMR (400MHz, DMSO-d6) δ 7.86 - 7.69 (m, 1H), 7.62 - 7.44 (m, 2H), 6.71 - 6.53 (m, 2H), 5.77 (s, 2H).

Step 4: Synthesis of isopropyl N-[4-(5-bromothiazol-2-yl)phenyl]carbamate

4-(5-Bromothiazol-2-yl)aniline (2.0 g, 7.84 mmol) was dissolved in dichloromethane (300 mL). At 0°C, a solution of isopropyl chloroformate (1.44 g, 11.76 mmol) in dichloromethane (200 mL) was added, and pyridine (3.72 g, 47.03 mmol) was added. The reaction solution was stirred and reacted at 30°C for 16 hours. The completion of the reaction was detected by LCMS. The reaction solution was concentrated to dryness under reduced pressure and then purified by column chromatography (silica, petroleum ether:ethyl acetate = 5:1) to obtain isopropyl N-[4-(5-bromothiazol-2-yl)phenyl]carbamate as a white solid compound (2.55 g).

MS m/z (ESI): 341.0, 343.0 $[M+H]^+$;

$^1H$ NMR (400MHz, DMSO-d6) δ 9.90 (s, 1H), 7.94-7.90 (m, 1H), 7.84-7.78 (m, 2H), 7.59 (d, J = 8.8 Hz, 2H), 4.91 (spt, J = 6.2 Hz, 1H), 1.26 (d, J = 6.3 Hz, 6H).

Step 5: Synthesis of isopropyl N-[4-[5-[4-(tert-butoxycarbonylamino)-2-cyclopropylsulfonyl-phenyl]thiazol-2-yl]phenyl]carbamate

Isopropyl N-[4-(5-bromothiazol-2-yl)phenyl]carbamate (850 mg, 2.49 mmol) and tert-butyl N-[3-cyclopropylsulfonyl-4-(4,4,5,5-tetramethyl-1,3,2-dioxaborolan-2-yl)phenyl] carbamate (1.11 g, 2.62 mmol) were dissolved in 1,4-dioxane (30 mL) and water (3 mL). Under the protection of nitrogen, [1,1-bis(diphenylphosphino)ferrocene]dichloropalladium - dichloromethane (203.43 mg, 249.10 μmol) and sodium carbonate (792.07 mg, 7.47 mmol) were added. The reaction solution was stirred and reacted at 100°C for 16 hours. The completion of the reaction was detected by LCMS. The reaction solution was concentrated to dryness under reduced pressure and then purified by column chromatography (silica, petroleum ether:tetrahydrofuran = 2:1) to obtain isopropyl N-[4-[5-[4-(tert-butoxycarbonylamino)-2-cyclopropylsulfonyl-phenyl]thiazol-2-yl]phenyl]carbamate as a white solid compound (670 mg).

MS m/z (ESI): 558.2 $[M+H]^+$;

Step 6: Synthesis of isopropyl N-[4-[5-(4-amino-2-cyclopropylsulfonyl-phenyl)thiazol-2-yl]phenyl]carbamate

Isopropyl N-[4-[5-[4-(tert-butoxycarbonylamino)-2-cyclopropylsulfonyl-phenyl]thiazol-2-yl]phenyl]carbamate

(670 mg, 1.22 mmol) was dissolved in methanol (6 mL), and hydrochloric acid-dioxane (4M, 6.10 mL) was added. The reaction solution was stirred and reacted at 30°C for 2 hours. The completion of the reaction was detected by LCMS. The reaction solution was concentrated to dryness under reduced pressure, and the resultant was added with 5 mL of saturated sodium carbonate solution. The reaction solution was extracted with 9 mL of dichloromethane (3 mL × 3). The organic phase was dried over anhydrous sodium sulfate and filtered. The filtrate was concentrated to dryness under reduced pressure to obtain isopropyl N-[4-[5-(4-amino-2-cyclopropylsulfonyl-phenyl)thiazol-2-yl]phenyl]carbamate as a white solid compound (590 mg).

MS m/z (ESI):458.1 $[M+H]^+$;

Step 7: Synthesis of isopropyl N-[4-[5-(4-bromo-2-cyclopropylsulfonyl-phenyl)thiazol-2-yl]phenyl]carbamate

Isopropyl N-[4-[5-(4-amino-2-cyclopropylsulfonyl-phenyl)thiazol-2-yl]phenyl]carbamate (350 mg, 764.92 μmol) was dissolved in acetonitrile (60 mL), and tert-butyl nitrite (236.64 mg, 2.29 mmol) was added at 0°C. The reaction solution was stirred and reacted at 30°C for 1 hour. Cuprous bromide (274.32 mg, 1.91 mmol) was added into the reaction solution at 0°C. The reaction solution was stirred and reacted at 30°C for 15 hours. The completion of the reaction was detected by LCMS. The reaction solution was concentrated to dryness under reduced pressure, and the resultant was added with saturated ammonium chloride solution (50 mL). The reaction solution was extracted with 90 mL of dichloromethane (30 mL × 3). The organic phase was dried over anhydrous sodium sulfate and filtered. The filtrate was concentrated to dryness under reduced pressure and purified by thin-layer chromatography (silica, petroleum ether:ethyl acetate = 3:2) to obtain isopropyl N-[4-[5-(4-bromo-2-cyclopropylsulfonyl-phenyl)thiazol-2-yl]phenyl]carbamate as a yellow solid compound (100 mg).

MS m/z (ESI):521.0, 523.0 $[M+H]^+$;

Step 8: Synthesis of isopropyl N-[4-[5-[2-cyclopropylsulfonyl]-4-[(1-methyltriazol-4-yl)amino]phenyl]thiazol-2-yl]phenyl]carbamate

Isopropyl N-[4-[5-(4-bromo-2-cyclopropylsulfonyl-phenyl)thiazol-2-yl]phenyl]carbamate (50 mg, 95.89 μmol) and 1-methyl-1H-1,2,3-triazol-4-amine (10.35 mg, 105.48 μmol) were dissolved in tetrahydrofuran (5 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (8.69 mg, 9.59 μmol) and potassium acetate (9.41 mg, 95.89 μmol) were added. The reaction solution was stirred and reacted at 100°C for 2 hours. The completion of the reaction was detected by LCMS. The reaction solution was added with water (3 mL) and extracted with ethyl acetate (9 mL) three times. The organic phase was dried over anhydrous sodium sulfate and filtered. The filtrate was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (45% to 65%) as eluents) to obtain isopropyl N-[4-[5-[2-cyclopropylsulfonyl]-4-[(1-methyltriazol-4-yl)amino]phenyl]thiazol-2-yl]phenyl]carbamate as a white solid compound (10 mg).

MS m/z (ESI):539.0$[M+H]^+$;

$^1H$ NMR (400MHz, DMSO-d6) δ 9.88 (s, 1H), 9.24 (s, 1H), 7.92-7.78 (m, 5H), 7.61 (d, J = 8.8 Hz, 2H), 7.51-7.40 (m, 2H), 4.92 (quin, J = 6.3 Hz, 1H), 4.04 (s, 3H), 3.32 (br s, 1H), 1.27 (d, J = 6.3 Hz, 6H), 1.05-0.88 (m, 4H).

Example 60: Synthesis of isopropyl N-[4-[5-[2-cyclopropylsulfonyl]-4-[(5-methyl-1H-pyrazol-3-yl)amino]phenyl]thiazol-2-yl]-1-bicyclo[2.2.2]octylcarbamate

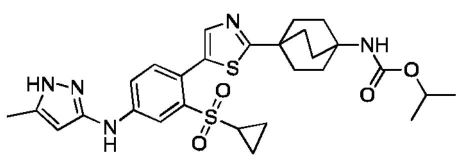

Synthetic Method

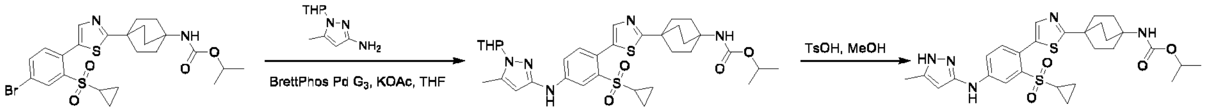


Step 1: Synthesis of isopropyl N-[4-[5-[2-cyclopropylsulfonyl]-4-[(5-methyl-1-tetrahydropyran-2-yl-pyrazol-3-yl)amino]phenyl]thiazol-2-yl]-1-bicyclo[2.2.2]octyl]carbamate

Isopropyl N-[4-[5-(4-bromo-2-cyclopropylsulfonyl-phenyl)thiazol-2-yl]-1-bicyclo[2.2.2] octyl]carbamate (40 mg, 72.26 μmol) and 1-methyl-1H-1,2,3-triazol-4-amine (14.41 mg, 79.49 μmol) were dissolved in anhydrous tetrahydrofuran (3 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (6.55 mg, 7.23 μmol) and potassium acetate (7.09 mg, 72.26 μmol) were added. The reaction solution appeared as a black suspension, and was stirred and reacted at 100°C for 1 hour. The completion of the reaction was detected by LCMS. The reaction solution was concentrated to dryness under reduced pressure. The resultant was purified by thin-layer chromatography (petroleum ether:tetrahydrofuran = 2:1) to obtain the compound isopropyl N-[4-[5-[2-cyclopropylsulfonyl]-4-[(5-methyl-1-tetrahydropyran-2-yl-pyrazol-3-yl)amino]phenyl]thiazol-2-yl]-1-bicyclo[2.2.2]octyl]carbamate (35 mg, 53.53 μmol) as a white solid.
MS m/z (ESI): 654.3 $[M+H]^+$;

Step 2: Synthesis of isopropyl N-[4-[5-[2-cyclopropylsulfonyl]-4-[(5-methyl-1H-pyrazol-3-yl)amino]phenyl]thiazol-2-yl]-1-bicyclo[2.2.2]octylcarbamate

Isopropyl N-[4-[5-[2-cyclopropylsulfonyl]-4-[(5-methyl-1-tetrahydropyran-2-yl-pyrazol-3-yl)amino]phenyl]thiazol-2-yl]-1-bicyclo[2.2.2]octyl]carbamate (35 mg, 53.53 μmol) was dissolved in anhydrous methanol (2 mL), and p-toluenesulfonic acid monohydrate (46.09 mg, 267.64 μmol) was added. The reaction solution appeared as a black suspension, and was stirred and reacted at 50°C for 1 hour. The completion of the reaction was detected by LCMS. The reaction solution was concentrated to dryness under reduced pressure, added with saturated sodium carbonate solution (10 mL), and extracted with dichloromethane (10 mL) twice. The organic layer was dried over anhydrous magnesium sulfate and subjected to suction filtration. The filtrate was concentrated to dryness under reduced pressure. The resultant was purified by preparative liquid chromatography (YMCCHIRALAmylose-C column, 5 μm silica, 30 mm in diameter, 250 mm in length; using decreasingly polar mixtures of water (containing 0.05% aqueous ammonia) and acetonitrile as eluents) to obtain the compound isopropyl N-[4-[5-[2-cyclopropylsulfonyl]-4-[(5-methyl-1H-pyrazol-3-yl)amino]phenyl]thiazol-2-yl]-1-bicyclo[2.2.2]octylcarbamate (1.24 mg, 2.18 μmol) as a white solid.
MS m/z (ESI): 570.1 $[M+H]^+$;
$^1H$ NMR (400MHz, DMSO-d6) δ12.02 - 11.74 (m, 1H), 9.04 (s, 1H), 8.12 - 8.06 (m, 1H), 7.63 (s, 2H), 7.35 - 7.29 (m, 1H), 6.84 - 6.73 (m, 1H), 5.64 (s, 1H), 4.77 - 4.66 (m, 1H), 2.40 - 2.31 (m, 1H), 2.20 (s, 3H), 1.97 (br d, J = 9.3 Hz, 6H), 1.89 (br d, J = 8.5 Hz, 6H), 1.15 (d, J = 6.3 Hz, 6H), 0.95 (dd, J = 2.5, 7.5 Hz, 2H), 0.89 - 0.81 (m, 2H).

Example 61: Synthesis of isopropyl N-[4-[5-[2-cyclopropylsulfonyl]-4-[(5-methyl-1H-pyrazol-3-yl)amino]phenyl]thiazol-2-yl]phenyl]carbamate

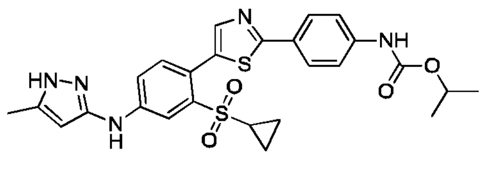

Synthetic Method

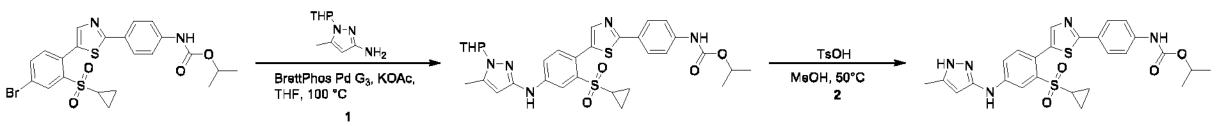

Step 1: Synthesis of isopropyl N-[4-[5-[2-cyclopropylsulfonyl]-4-[(5-methyl-1-tetrahydropyran-2-yl-pyrazol-3-yl)amino]phenyl]thiazol-2-yl]phenyl]carbamate

Isopropyl N-[4-[5-(4-bromo-2-cyclopropylsulfonyl-phenyl)thiazol-2-yl]phenyl]carbamate (50 mg, 95.89 μmol) and 5-methyl-1-tetrahydropyran-2-yl-pyrazol-3-amine (17.38 mg, 95.89 μmol) were dissolved in tetrahydrofuran (5 mL), and methanesulfonato(2-dicyclohexylphosphino)-3,6-dimethoxy-2,4,6-triisopropyl-1,1-biphenyl)(2-amino-1,1-biphenyl -2-yl)palladium (II) (8.69 mg, 9.59 μmol) and potassium acetate (9.41 mg, 95.89 μmol) were added. The reaction solution was stirred and reacted at 100°C for 2 hours. The completion of the reaction was detected by LCMS. The reaction solution was added with water (3 mL) and extracted with ethyl acetate (9 mL) three times. The organic phase was dried over anhydrous sodium sulfate and filtered. The filtrate was concentrated to dryness under reduced pressure and then purified by thin-layer chromatography (silica, dichloromethane/methanol = 15/1) to obtain isopropyl N-[4-[5-[2-cyclopropylsulfonyl]-4-[(5-methyl-1-tetrahydropyran-2-yl-pyrazol-3-yl)amino]phenyl]thiazol-2-yl]phenyl]carbamate as a yellow solid compound (50 mg).

MS m/z (ESI):622.2$[M+H]^+$;

Step 2: Synthesis of isopropyl N-[4-[5-[2-cyclopropylsulfonyl]-4-[(5-methyl-1H-pyrazol-3-yl)amino]phenyl]thiazol-2-yl]phenyl]carbamate

Isopropyl N-[4-[5-[2-cyclopropylsulfonyl]-4-[(5-methyl-1-tetrahydropyran-2-yl-pyrazol-3-yl)amino]phenyl]thiazol-2-yl]phenyl]carbamate (50 mg, 80.42 μmol) was dissolved in methanol (5 mL), and p-toluenesulfonic acid monohydrate (69.24 mg, 402.08 μmol) was added. The reaction solution was stirred and reacted at 50°C for 2 hours. The completion of the reaction was detected by LCMS. The reaction solution was concentrated to dryness under reduced pressure. The reaction solution was added with a saturated sodium bicarbonate solution (5 mL) and extracted with dichloromethane (9 mL) three times. The organic phase was dried over anhydrous sodium sulfate and filtered. The filtrate was concentrated to dryness under reduced pressure and purified by preparative liquid chromatography (Phenomenex Gemini-NX column: 5 μm silica, 30 mm in diameter, 150 mm in length; using decreasingly polar mixtures of water (containing 0.05% aqueous ammonia) and acetonitrile (50% to 70%) as eluents) to obtain isopropyl N-[4-[5-[2-cyclopropylsulfonyl]-4-[(5-methyl-1H-pyrazol-3-yl)amino]phenyl]thiazol-2-yl]phenyl]carbamate as a yellow solid compound (2.08 mg).

MS m/z (ESI): 538.0 $[M+H]^+$;

$^1$H NMR (400MHz, DMSO-d6) δ 11.89 (s, 1H), 9.88 (s, 1H), 9.09 (s, 1H), 8.13 (d, J = 2.3 Hz, 1H), 7.92-7.81 (m, 3H), 7.69-7.57 (m, 3H), 7.42 (d, J = 8.5 Hz, 1H), 5.65 (s, 1H), 4.92 (quin, J = 6.3 Hz, 1H), 3.31 (s, 1H), 2.20 (s, 3H), 1.27 (d, J = 6.3 Hz, 6H), 1.05-0.87 (m, 4H).

Example 62: Synthesis of isopropyl trans-N-[4-[5-[4-[(5-cyclopropyl-1H-pyrazol-3-yl)amino]-2-cyclopropylsulfonyl-phenyl]thiazol-2-yl]cyclohexyl]carbamate

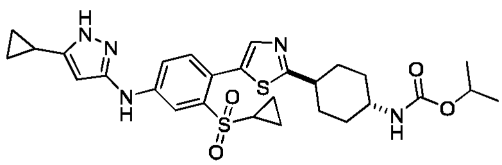

Synthetic Method

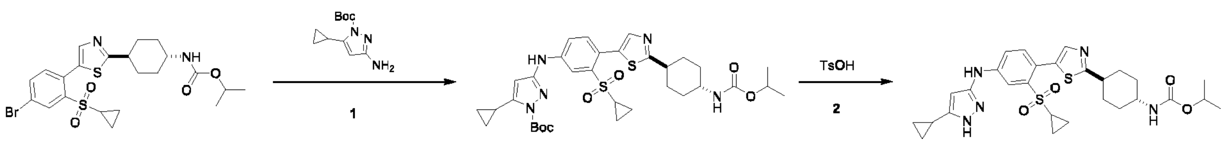

Step 1: Synthesis of tert-butyl trans-5-cyclopropyl-3-[3-cyclopropylsulfonyl-4-[2-[4-(isopropoxycarbonylamino)cyclohexyl]thiazol-5-yl]anilino]pyrazole-1-carboxylate

Isopropyl trans-N-[4-[5-(4-bromo-2-cyclopropylsulfonyl-phenyl)thiazol-2-yl]cyclohexyl] carbamate (40 mg, 75.83 μmol) and tert-butyl 3-amino-5-cyclopropyl-pyrazole-1-carboxylate (16.93 mg, 75.83 μmol) were dissolved in tetrahydrofuran (5 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (6.87 mg, 7.58 μmol) and potassium acetate (7.44 mg, 75.83 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and purified by thin-layer chromatography (silica, dichloromethane/methanol = 15/1) to obtain tert-butyl trans-5-cyclopropyl-3-[3-cyclopropylsulfonyl-4-[2-[4-(isopropoxycarbonylamino)cyclohexyl]thiazol-5-yl]anilino] pyrazole-1-carboxylate as the product (40 mg).

MS m/z (ESI): 670.3 $[M+H]^+$;

Step 2: Synthesis of isopropyl trans-N-[4-[5-[4-[(5-cyclopropyl-1H-pyrazol-3-yl)amino]-2-cyclopropylsulfonyl-phenyl]thiazol-2-yl]cyclohexyl]carbamate

Tert-butyl trans-5-cyclopropyl-3-[3-cyclopropylsulfonyl-4-[2-[4-(isopropoxycarbonyl amino)cyclohexyl]thiazol-5-yl]anilino]pyrazole-1-carboxylate (40 mg, 59.71 μmol) was dissolved in methanol (3 mL), and p-toluenesulfonic acid monohydrate (51.41 mg, 298.57 μmol) was added. The reaction solution was stirred and reacted at 50°C for 2 hours. The completion of the reaction was detected by LCMS. The reaction solution was concentrated to dryness under reduced pressure. The reaction solution was added with 9 mL of dichloromethane (3 mL × 3) and 5 mL of a saturated sodium bicarbonate solution. The organic phase was dried over anhydrous sodium sulfate and filtered. The filtrate was concentrated to dryness under reduced pressure and purified by preparative liquid chromatography (Phenomenex Gemini C18 column: 10 μm silica, 50 mm in diameter, 250 mm in length; using decreasingly polar mixtures of water (containing 0.05% aqueous ammonia) and acetonitrile as eluents) to obtain the compound isopropyl trans-N-[4-[5-[4-[(5-cyclopropyl-1H-pyrazol-3-yl)amino]-2-cyclopropylsulfonyl-phenyl]thiazol-2-yl]cyclohexyl]carbamate (6.01 mg).

MS m/z (ESI): 570.1 $[M+H]^+$;

$^1$H NMR (400MHz, DMSO-d6) δ 11.92 (s, 1H), 9.03 (s, 1H), 8.12 (d, J=2.3 Hz, 1H), 7.66-7.57 (m, 2H), 7.32 (d, J = 8.4 Hz, 1H), 7.02 (br d, J = 7.8 Hz, 1H), 5.54 (d, J = 2.1 Hz, 1H), 4.81-4.70 (m, 1H), 3.35-3.31 (m, 1H), 2.96-2.86 (m, 1H), 2.40-2.34 (m, 1H), 2.13 (br d, J = 11.6 Hz, 2H), 1.97-1.81 (m, 3H), 1.63-1.48 (m, 2H), 1.42-1.27 (m, 2H), 1.16 (d, J = 6.2 Hz, 6H), 1.01-0.82 (m, 6H), 0.73-0.63 (m, 2H).

Example 63: Synthesis of isopropyl trans-N-[4-[5-[4-[(5-cyclopropyl-1H-pyrazol-3-yl)amino]-2-(cyclopropylsulfonimidoyl)phenyl]thiazol-2-yl]cyclohexyl]carbamate

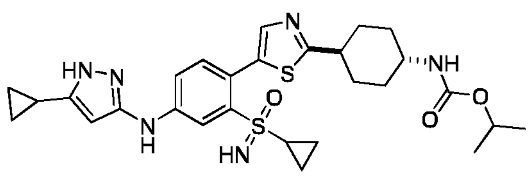

Synthetic Method

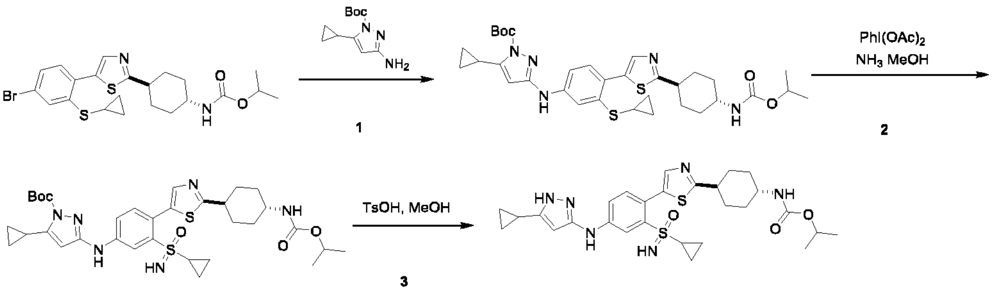


Step 1: Synthesis of tert-butyl trans-5-cyclopropyl-3-[3-cyclopropylsulfanyl-4-[2-[4-(isopropoxycarbonylamino)cyclohexyl]thiazol-5-yl]anilino]pyrazole-1-carboxylate

Isopropyl trans-N-[4-[5-(4-bromo-2-cyclopropylsulfanyl-phenyl)thiazol-2-yl]cyclohexyl] carbamate (20 mg, 40.36 μmol) and tert-butyl 3-amino-5-cyclopropyl-pyrazole-1-carboxylate (9.91 mg, 44.40 μmol) were dissolved in tetrahydrofuran (5 mL), and methanesulfonato(2-dicyclohexylphosphino)-3,6-dimethoxy-2,4,6-triisopropyl-1,1-biphenyl)(2-amino-1,1-biphenyl -2-yl)palladium (II) (3.66 mg, 4.04 μmol) and potassium acetate (3.96 mg, 40.36 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by thin-layer chromatography (silica, dichloromethane/methanol = 15/1) to obtain tert-butyl trans-5-cyclopropyl-3-[3-cyclopropylsulfanyl-4-[2-[4-(isopropoxycarbonylamino) cyclohexyl]thiazol-5-yl]anilino]pyrazole-1-carboxylate as the product (20 mg).

MS m/z (ESI): 638.3 $[M+H]^+$;

Step 2: Synthesis of tert-butyl trans-5-cyclopropyl-3-[3-(cyclopropylsulfonimidoyl)-4-[2-[4-(isopropoxycarbonylamino)cyclohexyl]thiazol-5-yl]anilino]pyrazole-1-carboxylate

Tert-butyl trans-5-cyclopropyl-3-[3-cyclopropylsulfanyl-4-[2-[4-(isopropoxycarbonyl amino)cyclohexyl]thiazol-5-yl]anilino]pyrazole-1-carboxylate (20 mg, 31.36 μmol) was dissolved in a solution of ammonia in methanol (3 mL), and diacetoxyiodobenzene (151.49 mg, 470.33 μmol) was added. The reaction solution was stirred and reacted at 60°C for 16 hours. The completion of the reaction was detected by LCMS. The reaction solution was concentrated to dryness under reduced pressure and then purified by thin-layer chromatography (silica, dichloromethane/methanol = 15/1) to obtain tert-butyl trans-5-cyclopropyl-3-[3-(cyclopropylsulfonimidoyl)-4-[2-[4-(isopropoxycarbonylamino)cyclohexyl]thiazol-5-yl]anilino ]pyrazole-1-carboxylate as the product (19 mg).

MS m/z (ESI): 569.3 $[M+H-Boc]^+$;

Step 3: Synthesis of isopropyl trans-N-[4-[5-[4-[(5-cyclopropyl-1H-pyrazol-3-yl)amino]-2-(cyclopropylsulfonimidoyl)phenyl]thiazol-2-yl]cyclohexyl]carbamate

Tert-butyl trans-5-cyclopropyl-3-[3-(cyclopropylsulfonimidoyl)-4-[2-[4-(isopropoxy carbonylamino)cyclohexyl]thiazol-5-yl]anilino]pyrazole-1-carboxylate (15 mg, 22.43 μmol) was dissolved in methanol (2 mL), and p-toluenesulfonic acid monohydrate (19.31 mg, 112.13 μmol) was added. The reaction solution was stirred and reacted at 50°C for 2 hours. The completion of the reaction was detected by LCMS. The reaction solution was concentrated to dryness under reduced pressure. The reaction solution was added with 9 mL of dichloromethane (3 mL × 3) and 5 mL of a saturated sodium bicarbonate solution. The organic phase was dried over anhydrous sodium sulfate and filtered. The filtrate was concentrated to dryness under reduced pressure and purified by preparative liquid chromatography (Phenomenex Gemini C18 column: 10 μm silica, 50 mm in diameter, 250 mm in length; using decreasingly polar mixtures of water (containing 0.05% aqueous ammonia) and acetonitrile as eluents) to obtain the compound isopropyl trans-N-[4-[5-[4-[(5-cyclopropyl-1H-pyrazol-3-yl)amino]-2-(cyclopropylsulfonimidoyl)phenyl]thiazol-2-yl]cyclohexyl]carbamate (1.10 mg).

MS m/z (ESI): 569.4 $[M+H]^+$;

$^1$H NMR (400MHz, DMSO-d6) δ 11.87 (s, 1H), 8.91 (s, 1H), 8.05 (s, 1H), 7.70-7.51 (m, 2H), 7.23 (d, J = 8.4 Hz, 1H), 7.02 (br d, J = 7.5 Hz, 1H), 5.54 (s, 1H), 4.80-4.70 (m, 1H), 4.20 (s, 1H), 4.06 (s, 1H), 2.95-2.83 (m, 1H), 2.32-2.27 (m, 1H), 2.12 (br d, J = 13.6 Hz, 2H), 1.95-1.84 (m, 3H), 1.62-1.48 (m, 2H), 1.40-1.27 (m, 2H), 1.16 (d, J = 6.2 Hz, 6H), 0.96-0.75 (m, 6H), 0.73-0.62 (m, 2H).

Example 64: Synthesis of isopropyl trans-(4-(5-(2-(cyclopropanesulfonimidoyl)-4-(isoxazolidin-2-yl)phenyl)thiazol-2-yl)cyclohexyl)carbamate

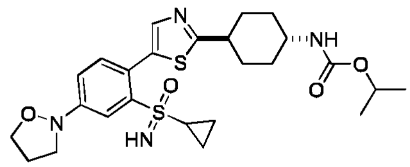

Synthetic Method

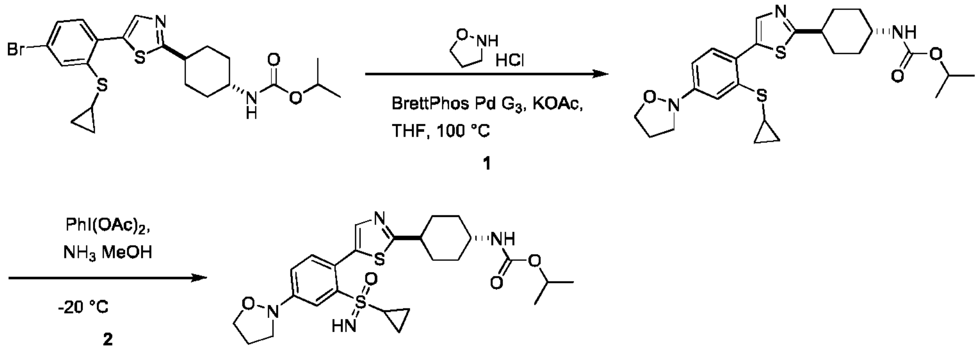


Step 1: Synthesis of isopropyl trans-(4-(5-(2-(cyclopropylthio)-4-(isoxazolidin-2-yl)phenyl)thiazol-2-yl)cyclohexyl)carbamate

Isopropyl trans-(4-(5-(4-bromo-2-(cyclopropylthio)phenyl)thiazol-2-yl)cyclohexyl) carbamate (30.0 mg, 60.6 μmol) and isoxazolidine hydrochloride (13.3 mg, 121 μmol) were dissolved in tetrahydrofuran (1 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (10.3 mg, 11.4 μmol) and potassium acetate (2.74 mg, 3.03 μmol) were added. The reaction solution was stirred and reacted at 100°C for 2 hours. The completion of the reaction was detected by LCMS. The reaction solution was concentrated to dryness under reduced pressure. The reaction solution was added with water (1 mL) and extracted with ethyl acetate (3 mL) three times. The organic phase was dried over anhydrous sodium sulfate and filtered. The filtrate was concentrated to dryness under reduced pressure and purified by thin-layer chromatography (silica, dichloromethane:methanol = 50:1) to obtain isopropyl trans-(4-(5-(2-(cyclopropylthio)-4-(isoxazolidin-2-yl)phenyl)thiazol-2-yl)cyclohexyl) carbamate as a yellow solid compound (24.0 mg).

MS m/z (ESI): 488.3 $[M+H]^+$;

Step 2: Synthesis of isopropyl trans-(4-(5-(2-(cyclopropanesulfonimidoyl)-4-(isoxazolidin-2-yl)phenyl)thiazol-2-yl)cyclohexyl)carbamate

Isopropyl trans-(4-(5-(2-(cyclopropylthio)-4-(isoxazolidin-2-yl)phenyl)thiazol-2-yl)cyclohexyl)carbamate (24.0 mg, 49.2 μmol) was dissolved in a solution of ammonia in methanol (3 mL), and diacetoxyiodobenzene (238 mg, 738 μmol) was added. The reaction solution was stirred and reacted at -20°C for 2 hours. The completion of the reaction was detected by LCMS. The reaction solution was concentrated to dryness under reduced pressure and then purified by preparative liquid chromatography (Phenomenex Gemini-NX column: 5 μm silica, 30 mm in diameter, 150 mm in length; using decreasingly polar mixtures of water (containing 0.225% aqueous ammonia) and acetonitrile (35% to 55%) as eluents) to obtain isopropyl trans-(4-(5-(2-(cyclopropanesulfonimidoyl)-4-(isoxazolidin-2-yl)phenyl)thiazol-2-yl)cyclohexyl)carbamate as a white solid compound (1.01 mg).

MS m/z (ESI): 519.0 $[M+H]^+$;

$^1$H NMR (400MHz, METHANOL-$d_4$) δ 8.71-8.31 (m, 1H), 7.80 (d, J = 2.4 Hz, 1H), 7.70 (s, 1H), 7.39 (d, J = 8.3 Hz, 1H), 7.28 (dd, J = 2.4, 8.4 Hz, 1H), 5.19 (s, 1H), 4.75-4.73 (m, 1H), 4.02 (t, J = 7.3 Hz, 2H), 3.62 (t, J = 7.1 Hz, 2H),

3.43 (br d, J = 11.4 Hz, 1H), 3.06-2.94 (m, 1H), 2.44-2.36 (m, 1H), 2.35-2.27 (m, 2H), 2.27-2.17 (m, 2H), 2.12-2.00 (m, 2H), 1.76-1.61 (m, 2H), 1.48-1.34 (m, 2H), 1.31-1.15 (m, 7H), 1.08-0.97 (m, 1H), 0.94-0.81 (m, 2H).

Example 65: Isopropyl trans-(4-(5-(4-(3-benzylureido)-2-(cyclopropanesulfonimidoyl) phenyl)thiazol-2-yl)cyclohexyl)carbamate

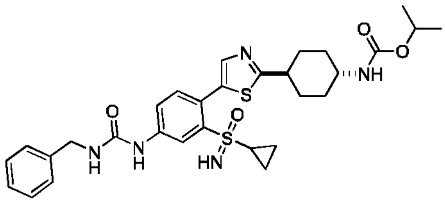

Synthetic Method

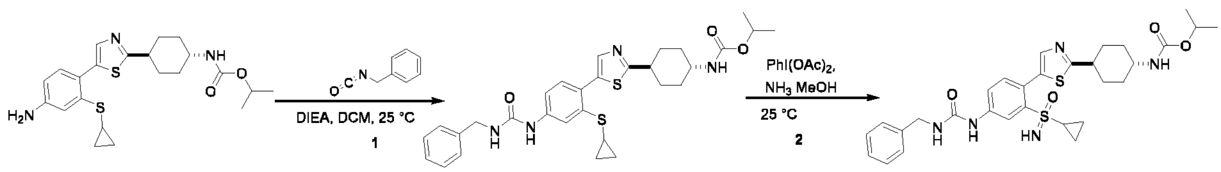


Step 1: Synthesis of isopropyl trans-N-[3-cyclopropylsulfanyl-4-[2-[4-(isopropoxycarbonylamino)cyclohexyl]thiazol-5-yl]phenyl]carbamate

Isopropyl trans-N-[4-[5-(4-amino-2-cyclopropylsulfanyl-phenyl)thiazol-2-yl] cyclohexyl]carbamate (500 mg, 1.16 mmol) was dissolved in dichloromethane (50 mL). At 0°C, benzyl isocyanate (463 mg, 3.48 mmol) was added, and N,N-diisopropylethylamine (606 μl, 3.48 mmol) was added. The reaction solution was stirred and reacted at 25°C for 2 hours. The completion of the reaction was detected by LCMS. The reaction solution was concentrated to dryness under reduced pressure and then purified by column chromatography (silica, petroleum ether:ethyl acetate = 1:5) to obtain isopropyl trans-N-[3-cyclopropylsulfanyl-4-[2-[4-(isopropoxycarbonylamino)cyclohexyl]thiazol-5-yl]phenyl]carbamate as a white solid compound (503 mg).
MS m/z (ESI): 565.3 $[M+H]^+$;

Step 2: Isopropyl trans-(4-(5-(4-(3-benzylureido)-2-(cyclopropanesulfonimidoyl)phenyl) thiazol-2-yl)cyclohexyl)carbamate

Isopropyl trans-N-[3-cyclopropylsulfanyl-4-[2-[4-(isopropoxycarbonylamino)cyclohexyl] thiazol-5-yl]phenyl]carbamate (500 mg, 885 μmol) was dissolved in a solution of ammonia in methanol (7M, 80 mL), and diacetoxyiodobenzene (4.28 g, 13.3 mmol) was added. The reaction solution was stirred and reacted at 25°C for 2 hours. The completion of the reaction was detected by LCMS. The reaction solution was concentrated to dryness under reduced pressure and then purified by column chromatography (silica, petroleum ether:ethyl acetate = 70% to 100%) to obtain isopropyl trans-(4-(5-(4-(3-benzylureido)-2-(cyclopropanesulfonimidoyl)phenyl)thiazol-2-yl)cyclohexyl)carbamate as a white solid compound (141.31 mg).
MS m/z (ESI): 596.2 $[M+H]^+$;
$^1$H NMR (400MHz, DMSO-d6) δ 9.14 (s, 1H), 8.11 (d, J = 2.3 Hz, 1H), 7.74 (dd, J = 2.3, 8.5 Hz, 1H), 7.70 (s, 1H), 7.37 - 7.20 (m, 6H), 7.01 (br d, J = 7.8 Hz, 1H), 6.73 (t, J = 6.0 Hz, 1H), 4.77-4.71 (m, 1H), 4.32 (d, J = 5.8 Hz, 2H), 3.22 (br s, 1H), 2.97-2.80 (m, 1H), 2.30-2.22 (m, 1H), 2.12 (br d, J = 12.0 Hz, 2H), 1.94-1.88 (m, 2H), 1.63-1.46 (m, 2H), 1.41-1.25 (m, 2H), 1.25-1.10 (m, 6H), 0.94-0.74 (m, 4H).

Example 66: Synthesis of isopropyl trans-N-[4-[5-[2-cyclopropylsulfonyl-4-(2-pyridylamino)phenyl]thiazol-2-yl]cyclohexyl]carbamate

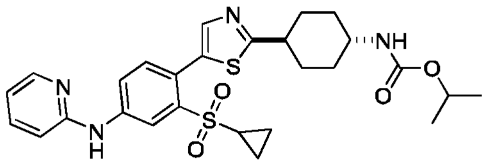

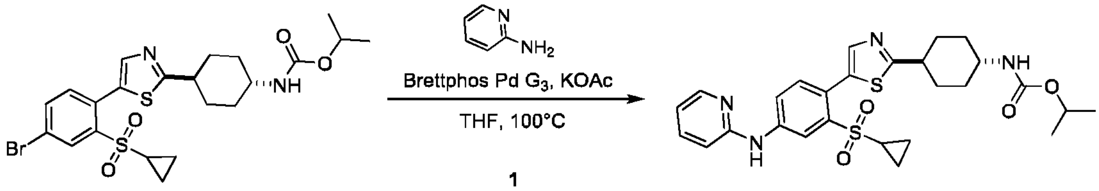


Isopropyl trans-N-[4-[5-(4-bromo-2-cyclopropylsulfonyl-phenyl)thiazol-2-yl] cyclohexyl]carbamate (30 mg, 56.87 μmol) and 2-aminopyridine (5.89 mg, 62.56 μmol) were dissolved in anhydrous tetrahydrofuran (2 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (5.16 mg, 5.69 μmol) and potassium acetate (5.58 mg, 56.87 μmol) were added. The reaction solution appeared as a black suspension, and was stirred and reacted at 100°C for 2 hours. The completion of the reaction was detected by LCMS. The reaction solution was concentrated to dryness under reduced pressure. The resultant was purified by preparative liquid chromatography (YMC-ActusTriartC18 column, 5 μm silica, 30 mm in diameter, 150 mm in length; using decreasingly polar mixtures of water (containing 0.05% aqueous ammonia) and acetonitrile as eluents) to obtain the compound isopropyl trans-N-[4-[5-[2-cyclopropylsulfonyl-4-(2-pyridylamino)phenyl]thiazol-2-yl]cyclohexyl]carbamate (13.29 mg, 24.58 μmol) as a white solid.

MS m/z (ESI): 541.1 $[M+H]^+$;

$^1$H NMR (400MHz, DMSO-d6) δ = 9.68 (s, 1H), 8.36 (d, J = 2.3 Hz, 1H), 8.24 - 8.17 (m, 2H), 7.69 (s, 1H), 7.65 (t, J = 7.8 Hz, 1H), 7.45 (d, J = 8.3 Hz, 1H), 7.02 (br d, J = 8.0 Hz, 1H), 6.89 (d, J = 8.0 Hz, 1H), 6.86 (t, J = 6.0 Hz, 1H), 4.78 - 4.70 (m, 1H), 3.35 (br s, 1H), 3.31 - 3.27 (m, 1H), 2.97 - 2.89 (m, 1H), 2.56 - 2.52 (m, 1H), 2.47 - 2.39 (m, 1H), 2.17 - 2.10 (m, 2H), 1.92 (br d, J = 10.3 Hz, 2H), 1.63 - 1.51 (m, 2H), 1.40 - 1.24 (m, 2H), 1.16 (d, J = 6.3 Hz, 6H), 1.01 - 0.94 (m, 2H), 0.94 - 0.87 (m, 2H).

Example 67: Synthesis of isopropyl trans-(4-(5-(4-acetylamino-2-(cyclopropanesulfonimidoyl)phenyl]thiazol-2-yl)cyclohexyl)carbamate

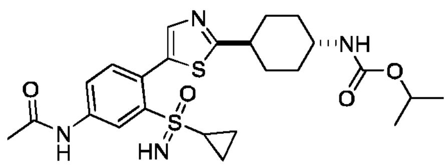

Synthetic Method

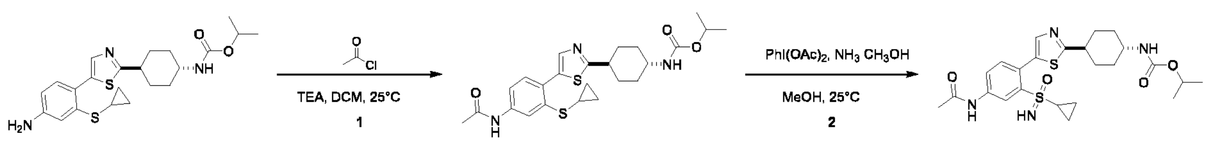

Step 1: Synthesis of isopropyl trans-(4-(5-(4-acetylamino-2-(cyclopropylthio)phenyl) thiazol-2-yl)cyclohexyl)carbamate

Isopropyl trans-(4-(5-(4-amino-2-(cyclopropylthio)phenyl)thiazol-2-yl)cyclohexyl) carbamate (50 mg, 115.84 μmol) was dissolved in dichloromethane (2 mL), and chloroacetyl (10.91 mg, 139.01 μmol, 9.92 μL) and triethylamine (35.17 mg, 347.53 μmol, 48.37 μL) were added. The reaction solution was stirred and reacted at 25°C for 1 hour. The completion of the reaction was detected by LCMS. Thereafter, the resultant was purified by thin-layer chromatography (silica, dichloromethane/methanol = 10/1) to obtain isopropyl trans-(4-(5-(4-acetylamino-2-(cyclopropylthio)phenyl)thiazol-2-yl)cyclohexyl)carbamate as the product (20 mg).

MS m/z (ESI): 474.2 $[M+H]^+$;

Step 2: Synthesis of isopropyl trans-(4-(5-(4-acetylamino-2-(cyclopropanesulfonimidoyl)phenyl]thiazol-2-yl)cyclohexyl)carbamate

Isopropyl trans-(4-(5-(4-acetylamino-2-(cyclopropylthio)phenyl)thiazol-2-yl)cyclohexyl) carbamate (20 mg, 42.23 μmol) was dissolved in a methanol solution (2 mL), and a solution of ammonia in methanol (7M, 30.16 μL) and diacetoxyiodobenzene (34.00 mg, 105.56 μmol) were added. The reaction solution was stirred and reacted at 25°C for 0.5 hours. The completion of the reaction was detected by LCMS. The reaction solution was concentrated to dryness under reduced pressure and purified by preparative liquid chromatography (Phenomenex Gemini C18 column: 10 μm silica, 50 mm in diameter, 250 mm in length; using decreasingly polar mixtures of water (containing 0.05% aqueous ammonia) and acetonitrile (30% to 50%) as eluents) to obtain isopropyl trans-(4-(5-(4-acetylamino-2-(cyclopropanesulfonimidoyl)phenyl]thiazol-2-yl)cyclohexyl)carbamate as a white solid compound (4.5 mg).

MS m/z (ESI): 505.1 $[M+H]^+$;

$^1$H NMR (400MHz, DMSO-d6) δ = 10.39 (s, 1H), 8.25 (d, J = 2.3 Hz, 1H), 7.93 (dd, J = 2.3, 8.3 Hz, 1H), 7.72 (s, 1H), 7.40 (d, J = 8.5 Hz, 1H), 7.01 (br d, J = 7.8 Hz, 1H), 4.74 (td, J = 6.3, 12.4 Hz, 1H), 4.46-4.32 (m, 1H), 3.31 (br s, 1H), 3.00-2.82 (m, 1H), 2.31-2.23 (m, 1H), 2.16-2.09 (m, 2H), 2.08 (s, 3H), 1.91 (br d, J = 10.0 Hz, 2H), 1.63-1.48 (m, 2H), 1.40-1.26 (m, 2H), 1.16 (d, J = 6.0 Hz, 6H), 0.94-0.75 (m, 4H).

Example 68: Synthesis of isopropyl trans-N-[3-(cyclopropylsulfonimidoyl)-4-[2-[4-(isopropoxycarbonylamino)cyclohexyl]thiazol-5-yl]phenyl]carbamate

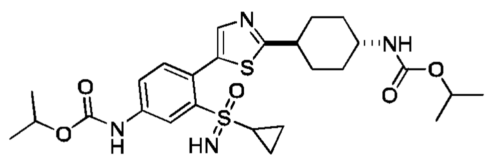

Synthetic Method

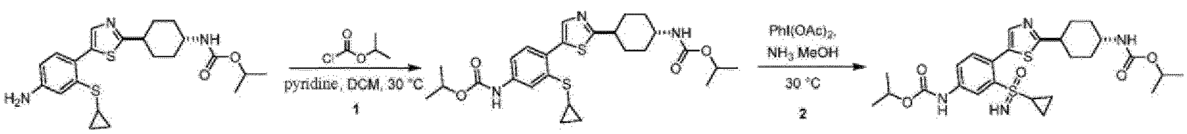


Step 1: Synthesis of isopropyl trans-N-[3-cyclopropylsulfanyl-4-[2-[4-(isopropoxycarbonylamino)cyclohexyl]thiazol-5-yl]phenyl]carbamate

Isopropyl trans-N-[4-[5-(4-amino-2-cyclopropylsulfanyl-phenyl)thiazol-2-yl]cyclohexyl] carbamate (2.5 g, 5.79 mmol) was dissolved in dichloromethane (800 mL). At 0°C, isopropyl chloroformate (1.06 g, 8.69 mmol) was added, and pyridine (2.75 g, 34.75 mmol) was added. The reaction solution was stirred and reacted at 30°C for 3 hours. The reaction solution was concentrated to dryness under reduced pressure and then purified by column chromatography (silica, petroleum ether/ethyl acetate=3/1) to obtain isopropyl trans-N-[3-cyclopropylsulfanyl-4-[2-[4-(isopropoxycarbonylamino)cyclohexyl]thiazol-5-yl]phenyl]carbamate as a yellow oil (1.32 g).

MS m/z (ESI): 518.1 $[M+H]^+$;

Step 2: Synthesis of isopropyl trans-N-[3-(cyclopropylsulfonimidoyl)-4-[2-[4-(isopropoxycarbonylamino)cyclohexyl]thiazol-5-yl]phenyl]carbamate

Isopropyl trans-N-[3-cyclopropylsulfanyl-4-[2-[4-(isopropoxycarbonylamino)cyclohexyl] thiazol-5-yl]phenyl]carbamate (1.3 g, 2.51 mmol) was dissolved in a solution of ammonia in methanol (150 mL), and diacetoxyiodobenzene (12.13 g, 37.67 mmol) was added. The reaction solution was stirred and reacted at 30°C for 16 hours. The completion of the reaction was detected by LCMS. The reaction solution was concentrated to dryness under reduced pressure and then purified by column chromatography (silica, petroleum ether:ethyl acetate = 0:1) to obtain isopropyl trans-N-[3-(cyclopropylsulfonimidoyl)-4-[2-[4-(isopropoxycarbonylamino) cyclohexyl]thiazol-5-yl]phenyl]carbamate (527.8 mg) as a white solid compound.
MS m/z (ESI): 549.1 $[M+H]^+$;
$^1H$ NMR (400MHz, DMSO-d6) $\delta$ 10.03 (s, 1H), 8.30 (d, J = 2.3 Hz, 1H), 7.76-7.58 (m, 2H), 7.38 (d, J = 8.3 Hz, 1H), 7.02 (br d, J = 7.8 Hz, 1H), 4.91 (quin, J = 6.3 Hz, 1H), 4.74 (td, J = 6.1, 12.4 Hz, 1H), 4.34 (s, 1H), 3.32-3.31 (m, 1H), 3.02-2.80 (m, 1H), 2.30-2.21 (m, 1H), 2.12 (br d, J = 12.0 Hz, 2H), 1.96-1.85 (m, 2H), 1.62-1.48 (m, 2H), 1.40-1.29 (m, 2H), 1.27 (d, J = 6.3 Hz, 6H), 1.16 (d, J = 6.3 Hz, 6H), 0.95-0.75 (m, 4H).

Example 69: Synthesis of isopropyl trans-(4-(5-(2-(cyclopropanesulfonimidoyl)-4-((1-methyl-1H-pyrazol-4-yl)amino)phenyl]thiazol-2-yl)cyclohexyl)carbamate

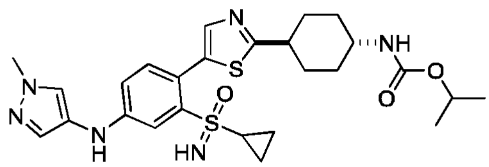

Synthetic Method

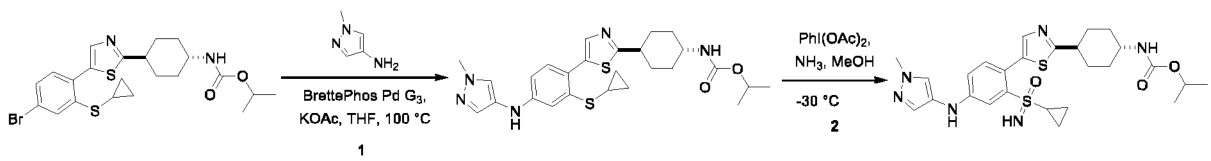


Step 1: Synthesis of isopropyl trans-(4-(5-(2-(cyclopropylthio)-4-((1-methyl-1H-pyrazol-4-yl)amino)phenyl)thiazol-2-yl)cyclohexyl)carbamate

Isopropyl trans-(4-(5-(4-bromo-2-(cyclopropylthio)phenyl)thiazol-2-yl)cyclohexyl) carbamate (200 mg, 403 μmol) and 1-methyl-1H-pyrazol-4-amine (78.4 mg, 807 μmol) were dissolved in tetrahydrofuran (10 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (36.6 mg, 40.4 μmol) and potassium acetate (118 mg, 1.21 mmol) were added. The reaction solution was stirred and reacted at 100°C for 3 hours. The completion of the reaction was detected by LCMS. The reaction solution was added with water (10 mL) and extracted with 21 mL of ethyl acetate (7 mL × 3). The organic phase was dried over anhydrous sodium sulfate and filtered. The filtrate was concentrated to dryness under reduced pressure and then purified by column chromatography (petroleum ether:ethyl acetate = 1:4) to obtain isopropyl trans-(4-(5-(2-(cyclopropylthio)-4-((1-methyl-1H-pyrazol-4-yl)amino)phenyl)thiazol-2-yl)cyclohexyl) carbamate as a brown oily product (175 mg).
MS m/z (ESI): 512.3 $[M+H]^+$;

Step 2: Synthesis of isopropyl trans-(4-(5-(2-(cyclopropanesulfonimidoyl)-4-((1-methyl-1H-pyrazol-4-yl)amino)phenyl]thiazol-2-yl)cyclohexyl)carbamate

Isopropyl trans-(4-(5-(2-(cyclopropylthio)-4-((1-methyl-1H-pyrazol-4-yl)amino)phenyl) thiazol-2-yl)cyclohexyl)carbamate (175 mg, 342 μmol) was dissolved in a solution of ammonia in methanol (7M, 5 mL), and diacetoxyiodobenzene (1.65 g, 5.13 mmol) was added. The reaction solution was stirred and reacted at -30°C for 2 hours. The

completion of the reaction was detected by LCMS. The reaction solution was concentrated to dryness under reduced pressure and then purified by preparative liquid chromatography (YMC-Actus Triart C18 column: 7 μm silica, 50 mm in diameter, 250 mm in length; using decreasingly polar mixtures of water (containing 0.23% formic acid) and acetonitrile as eluents) to obtain isopropyl trans-(4-(5-(2-(cyclopropanesulfonimidoyl)-4-((1-methyl-1H-pyrazol-4-yl)amino)phenyl]thiazol-2-yl)cyclohexyl)carbamate as a white solid compound (2.96 mg).

MS m/z (ESI): 543.1 $[M+H]^+$;

$^1$H NMR (400MHz, DMSO-d6) δ = 8.17 (s, 1H), 7.70 (s, 1H), 7.64 (s, 1H), 7.50 (d, J = 2.5 Hz, 1H), 7.37 (s, 1H), 7.19 (d, J = 8.3 Hz, 1H), 7.02 (br d, J = 6.8 Hz, 1H), 6.90 (dd, J = 2.5, 8.3 Hz, 1H), 4.77-4.64 (m, 1H), 4.26-4.19 (m, 1H), 3.77 (s, 3H), 3.43-3.33 (m, 1H), 2.89 (br t, J = 11.8 Hz, 1H), 2.30-2.24 (m, 1H), 2.12 (br d, J = 11.0 Hz, 2H), 1.91 (br d, J = 10.5 Hz, 2H), 1.66-1.46 (m, 2H), 1.43-1.26 (m, 2H), 1.17 (d, J = 6.4 Hz, 6H), 0.91-0.73 (m, 4H).

Example 70: Synthesis of isopropyl trans-N-[4-[5-[2-cyclopropylsulfonyl]-4-[(1-methylpyrazol-4-yl)amino]phenyl]thiazol-2-yl]cyclohexyl]carbamate

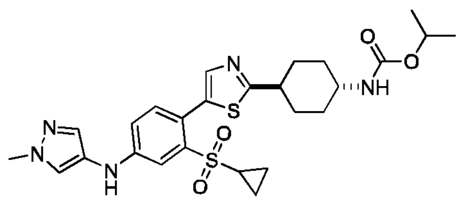

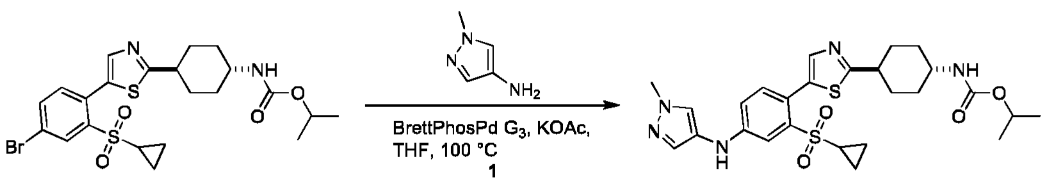


Isopropyl trans-N-[4-[5-(4-bromo-2-cyclopropylsulfonyl-phenyl)thiazol-2-yl]cyclohexyl] carbamate (100 mg, 189.58 μmol) and 1-methylpyrazol-4-amine (22.09 mg, 227.49 μmol) were dissolved in tetrahydrofuran (6 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (17.19 mg, 18.96 μmol) and potassium acetate (18.61 mg, 189.58 μmol) were added. The reaction solution was stirred and reacted at 100°C for 2 hours. The completion of the reaction was detected by LCMS. The reaction solution was added with water (3 mL) and extracted with ethyl acetate (9 mL) three times. The organic phase was dried over anhydrous sodium sulfate and filtered. The filtrate was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.225% formic acid) and acetonitrile (40% to 60%) as eluents) to obtain isopropyl trans-N-[4-[5-[2-cyclopropylsulfonyl]-4-[(1-methylpyrazol-4-yl)amino]phenyl] thiazol-2-yl]cyclohexyl]carbamate as a white solid compound (18.57 mg).

MS m/z (ESI): 544.1 $[M+H]^+$;

$^1$H NMR (400MHz, DMSO-d6) δ 8.25 (s, 1H), 7.72 (s, 1H), 7.65-7.57 (m, 1H), 7.42-7.35 (m, 2H), 7.26 (d, J = 8.5 Hz, 1H), 7.06-6.93 (m, 2H), 4.74 (td, J = 6.2, 12.4 Hz, 1H), 3.82 (s, 3H), 3.34-3.33 (m, 1H), 2.90 (tt, J = 3.5, 11.9 Hz, 1H), 2.40-2.31 (m, 1H), 2.12 (br d, J = 11.8 Hz, 2H), 1.90 (br d, J = 10.3 Hz, 2H), 1.63-1.47 (m, 2H), 1.41-1.25 (m, 2H), 1.16 (d, J = 6.3 Hz, 6H), 0.99-0.78 (m, 4H).

Example 71: Synthesis of isopropyl trans-N-[4-[5-[2-cyclopropylsulfonyl]-4-(1H-pyrazol-4-ylamino)phenyl]thiazol-2-yl]cyclohexyl]carbamate

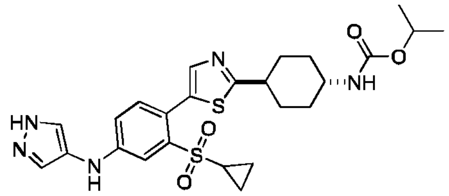

Synthetic Method

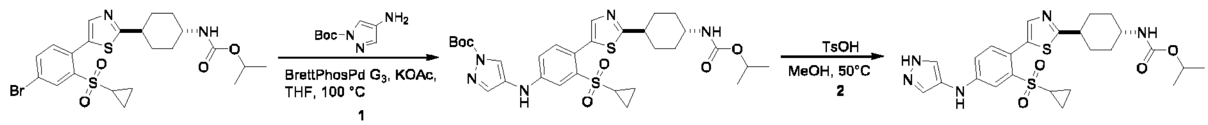


Step 1: Synthesis of tert-butyl trans-4-((3-(cyclopropylsulfonyl)-4-(2-((4-((isopropoxycarbonyl)amino)cyclohexyl)thiazol-5-yl)phenyl)amino)-1H-pyrazole-1-carboxylate

Isopropyl trans-N-[4-[5-(4-bromo-2-cyclopropylsulfonyl-phenyl)thiazol-2-yl]cyclohexyl] carbamate (100 mg, 189.58 μmol) and tert-butyl 4-aminopyrazole-1-carboxylate (41.68 mg, 227.49 μmol) were dissolved in tetrahydrofuran (5 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (17.19 mg, 18.96 μmol) and potassium acetate (18.61 mg, 189.58 μmol) were added. The reaction solution was stirred and reacted at 100°C for 2 hours. The completion of the reaction was detected by LCMS. The reaction solution was added with water (6 mL) and extracted with ethyl acetate (18 mL) three times. The organic phase was dried over anhydrous sodium sulfate and filtered. The filtrate was concentrated to dryness under reduced pressure and then purified by thin-layer chromatography (silica, dichloromethane:methanol = 10:1) to obtain tert-butyl trans-4-((3-(cyclopropylsulfonyl)-4-(2-((4-((isopropoxycarbonyl)amino)cyclohexyl)thiazol-5-yl)phenyl)amino)-1H-pyrazole-1-carboxylate as a yellow solid compound (90 mg).

MS m/z (ESI): 630.2 $[M+H]^+$;

Step 2: Synthesis of isopropyl trans-N-[4-[5-[2-cyclopropylsulfonyl]-4-(1H-pyrazol-4-ylamino)phenyl]thiazol-2-yl]cyclohexyl]carbamate

Tert-butyl trans-4-((3-(cyclopropylsulfonyl)-4-(2-((4-((isopropoxycarbonyl)amino) cyclohexyl)thiazol-5-yl)phenyl)amino)-1H-pyrazole-1-carboxylate (90 mg, 142.91 μmol) was dissolved in methanol (5 mL), and then p-toluenesulfonic acid monohydrate (123.04 mg, 714.53 μmol) was added into the reaction solution. The reaction solution was stirred and reacted at 50°C for 2 hours. The completion of the reaction was detected by LCMS. The reaction solution was concentrated to dryness under reduced pressure. The reaction solution was added with a saturated aqueous sodium carbonate solution (3 mL) and extracted with ethyl acetate (9 mL) three times. The organic phase was dried over anhydrous sodium sulfate and filtered. The filtrate was concentrated to dryness under reduced pressure and purified by preparative liquid chromatography (YMC-Actus Triart C18: 5 μm silica, 30 mm in diameter, 150 mm in length; using decreasingly polar mixtures of water (containing 0.05% aqueous ammonia) and acetonitrile (40% to 60%) as eluents) to obtain isopropyl trans-N-[4-[5-[2-cyclopropylsulfonyl]-4-(1H-pyrazol-4-ylamino)phenyl]thiazol-2-yl]cyclohexyl]carbamate as a white solid compound (24.54 mg).

MS m/z (ESI): 530.0 $[M+H]^+$;

$^1H$ NMR (400MHz, DMSO-d6) δ 12.77 (br s, 1H), 8.23 (s, 1H), 7.75 (br s, 1H), 7.62 (s, 1H), 7.46 (br s, 1H), 7.38 (d, J = 2.6 Hz, 1H), 7.26 (d, J = 8.4 Hz, 1H), 7.08-6.88 (m, 2H), 4.74 (td, J = 6.1, 12.4 Hz, 1H), 3.33-3.27 (m, 1H), 2.99-2.82 (m, 1H), 2.42-2.28 (m, 1H), 2.12 (br d, J = 11.6 Hz, 2H), 1.90 (br d, J = 10.4 Hz, 2H), 1.63-1.46 (m, 2H), 1.42-1.24 (m, 2H), 1.16 (d, J = 6.2 Hz, 6H), 1.01-0.74 (m, 4H).

Example 72: Synthesis of isopropyl trans-(4-(5-(2-(cyclopropylsulfonyl)-4-((5-(hydroxymethyl)-1H-pyrazol-3-yl)amino)phenyl)thiazol-2-yl)cyclohexyl)carbamate

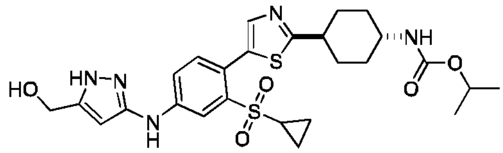

Synthetic Method

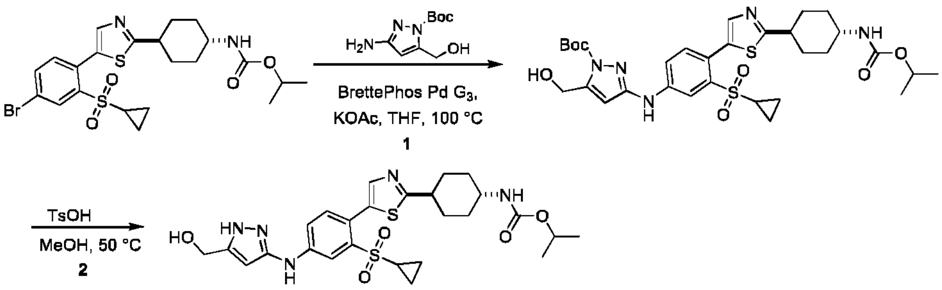


Step 1: Synthesis of tert-butyl trans-3-((3-(cyclopropylsulfonyl)-4-(2-((4-((isopropoxycarbonyl)amino)cyclohexyl)thiazol-5-yl)phenyl)amino)-5-(hydroxymethyl)-1H-pyrazole-1-carboxylate

Isopropyl trans-(4-(5-(4-bromo-2-(cyclopropylsulfonyl)phenyl)thiazol-2-yl)cyclohexyl) carbamate (49.5 mg, 93.8 μmol) and tert-butyl 3-amino-5-(hydroxymethyl)-1H-pyrazole-1-carboxylate (20.0 mg, 93.8 μmol) were dissolved in tetrahydrofuran (4 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (85.0 mg, 93.8 μmol) and potassium acetate (9.21 mg, 93.8 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and purified by thin-layer chromatography (silica, dichloromethane:methanol = 15:1) to obtain the compound tert-butyl trans-3-((3-(cyclopropylsulfonyl)-4-(2-((4-((isopropoxycarbonyl)amino)cyclohexyl)thiazol-5-yl)phenyl) amino)-5-(hydroxymethyl)-1H-pyrazole-1-carboxylate as a yellow product (33.0 mg).

MS m/z (ESI): 660.3$[M+H]^+$;

Step 2: Synthesis of isopropyl trans-(4-(5-(2-(cyclopropylsulfonyl)-4-((5-(hydroxymethyl)-1H-pyrazol-3-yl)amino)phenyl)thiazol-2-yl)cyclohexyl)carbamate

Tert-butyl trans-3-((3-(cyclopropylsulfonyl)-4-(2-((4-((isopropoxycarbonyl)amino) cyclohexyl)thiazol-5-yl)phenyl)amino)-5-(hydroxymethyl)-1H-pyrazole-1-carboxylate (33.0 mg, 50.0 μmol) was dissolved in methanol (3 mL), and p-toluenesulfonic acid monohydrate (43.1 mg, 250 μmol) was added. The reaction solution was stirred and reacted at 50°C for 2 hours. The completion of the reaction was detected by LCMS. The reaction solution was concentrated to dryness under reduced pressure. The reaction solution was added with 9 mL of dichloromethane (3 mL × 3) and 5 mL of a saturated sodium bicarbonate solution. The organic phase was dried over anhydrous sodium sulfate and filtered. The filtrate was concentrated to dryness under reduced pressure and purified by preparative liquid chromatography (Phenomenex Gemini C18 column: 5 μm silica, 30 mm in diameter, 150 mm in length; using decreasingly polar mixtures of water (containing 0.05% aqueous ammonia) and acetonitrile (33% to 53%) as eluents) to obtain isopropyl trans-(4-(5-(2-(cyclopropylsulfonyl)-4-((5-(hydroxymethyl)-1H-pyrazol-3-yl)amino)phenyl)thiazol-2-yl)cyclohexyl)carbamate (4.66 mg) as a white compound.

MS m/z (ESI): 560.1 $[M+H]^+$;

$^1$H NMR (400MHz, DMSO-d6) δ 12.33-11.79 (m, 1H), 9.44-8.76 (m, 1H), 8.08 (d, J = 2.2 Hz, 1H), 7.58 (s, 1H), 7.72-7.57 (m, 1H), 7.34 (d, J = 8.4 Hz, 1H), 7.02 (br d, J = 7.7 Hz, 1H), 5.77 (d, J = 1.8 Hz, 1H), 5.27 (t, J = 5.7 Hz, 1H), 4.74 (quin, J = 6.2 Hz, 1H), 4.44 (d, J = 5.6 Hz, 2H), 3.31-3.19 (m, 1H), 3.01-2.83 (m, 1H), 2.43-2.33 (m, 1H), 2.13 (br d, J = 11.6 Hz, 2H), 1.91 (br d, J = 10.3 Hz, 2H), 1.67-1.48 (m, 2H), 1.41-1.25 (m, 2H), 1.12 - 1.05 (m, 6H), 1.02-0.80 (m, 4H)

Example 73: Synthesis of isopropyl trans-N-[4-[5-(2-cyclopropylsulfonyl-4-isoxazolidin-2-yl-phenyl)thiazol-2-yl]cyclohexyl]carbamate

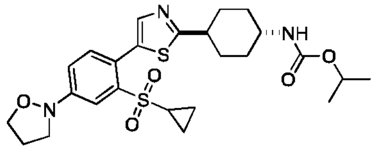

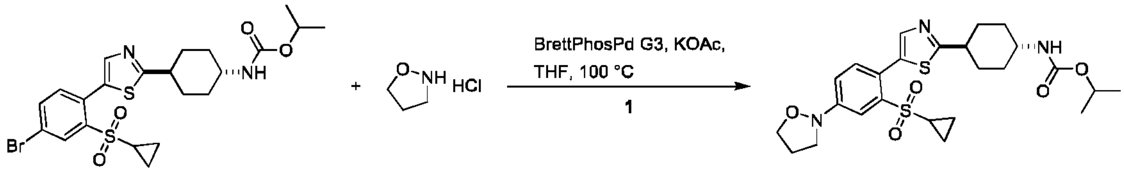


Isopropyl trans-N-[4-[5-(4-bromo-2-cyclopropylsulfonyl-phenyl)thiazol-2-yl]cyclohexyl] carbamate (70 mg, 133 μmol) and isoxazolidine hydrochloride (18.9 mg, 173 μmol) were dissolved in tetrahydrofuran (3 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (12.0 mg, 13.3 μmol) and potassium acetate (13.0 mg, 133 μmol) were added. The reaction solution was stirred and reacted at 100°C for 2 hours. The completion of the reaction was detected by LCMS. The reaction solution was added with water (3 mL) and extracted with ethyl acetate (9 mL) three times. The organic phase was dried over anhydrous sodium sulfate and filtered. The filtrate was concentrated to dryness under reduced pressure and then purified by preparative liquid chromatography (YMC-Actus Triart C18 column: 5 μm silica, 30 mm in diameter, 150 mm in length; using decreasingly polar mixtures of water (containing 0.225% formic acid) and acetonitrile (43% to 63%) as eluents) to obtain isopropyl trans-N-[4-[5-(2-cyclopropylsulfonyl-4-isoxazolidin-2-yl-phenyl)thiazol-2-yl] cyclohexyl]carbamate as a white solid compound (2.01 mg).

MS m/z (ESI): 520.0 $[M+H]^+$;

$^1H$ NMR (400MHz, DMSO-d6) δ 7.68 (s, 1H), 7.60 (d, J = 2.5 Hz, 1H), 7.44 (d, J = 8.3 Hz, 1H), 7.29 (dd, J = 2.4, 8.4 Hz, 1H), 7.02 (br d, J = 8.0 Hz, 1H), 4.74 (td, J = 6.1, 12.3 Hz, 1H), 3.94 (t, J = 7.3 Hz, 2H), 3.59 (t, J = 7.0 Hz, 2H), 3.30-3.27 (m, 1H), 2.99-2.87 (m, 1H), 2.47-2.40 (m, 1H), 2.25 (quin, J = 7.2 Hz, 2H), 2.13 (br d, J = 11.8 Hz, 2H), 1.91 (br d, J = 10.3 Hz, 2H), 1.63-1.49 (m, 2H), 1.41-1.25 (m, 2H), 1.16 (d, J = 6.3 Hz, 6H), 1.02-0.84 (m, 4H).

Example 74: Synthesis of isopropyl trans-(4-(5-(4-(cyclopropanecarboxamido)-2-(cyclopropylsulfonyl)phenyl)thiazol-2-yl)cyclohexyl)carbamate

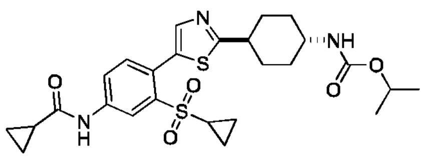

Synthetic Method

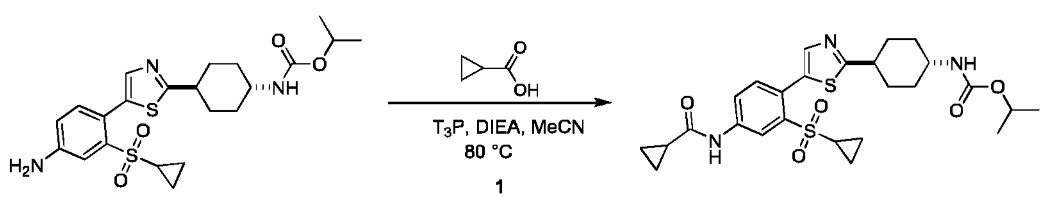

Isopropyl trans-(4-(5-(4-amino-2-(cyclopropylsulfonyl)phenyl)thiazol-2-yl)cyclohexyl) carbamate (80.0 mg, 173 μmol) and cyclopropylcarboxylic acid (44.6 mg, 518 μmol, 41 μL) were dissolved in anhydrous acetonitrile (3 mL), and tri-n-propylphosphonic anhydride (50% solution in ethyl acetate) (329 mg, 518 μmol, 308 μL) and N,N-diisopropylethylamine (66.9 mg, 518 μmol, 90.2 μL) were added. The reaction solution was stirred and reacted at 80°C for 2 hours. The completion of the reaction was detected by LCMS. The reaction solution was concentrated to dryness under reduced pressure and purified by preparative liquid chromatography (YMC-Actus Triart C18 column: 7 μm silica, 30 mm in diameter, 150 mm in length; using decreasingly polar mixtures of water (containing 0.05% aqueous ammonia) and acetonitrile (50% to 75%) as eluents) to obtain isopropyl trans-(4-(5-(4-(cyclopropanecarboxamido)-2-(cyclopropylsulfonyl)phenyl)thiazol-2-yl)cyclohexyl)carbamate as a white solid compound (28.34 mg).

MS m/z (ESI): 532.1 $[M+H]^+$;

$^1$H NMR (400MHz, DMSO-d6) δ = 10.72 (s, 1H), 8.33 (d, J = 2.3 Hz, 1H), 7.94 (dd, J = 2.3, 8.3 Hz, 1H), 7.71 (s, 1H), 7.50 (d, J = 8.5 Hz, 1H), 7.02 (br d, J = 7.8 Hz, 1H), 4.74 (td, J = 6.2, 12.5 Hz, 1H), 3.31-3.27 (m, 1H), 2.94 (tt, J = 3.4, 11.8 Hz, 1H), 2.44-2.36 (m, 1H), 2.13 (br d, J = 11.8 Hz, 2H), 1.91 (br d, J = 10.0 Hz, 2H), 1.80 (quin, J = 6.1 Hz, 1H), 1.64-1.49 (m, 2H), 1.40-1.27 (m, 2H), 1.16 (d, J = 6.3 Hz, 6H), 1.01-0.93 (m, 2H), 0.90-0.81 (m, 6H).

Example 75: Synthesis of isopropyl trans-N-[4-[5-[2-cyclopropylsulfonyl]-4-[(5-fluoro-2-pyridyl)amino]phenyl]thiazol-2-yl]cyclohexyl]carbamate

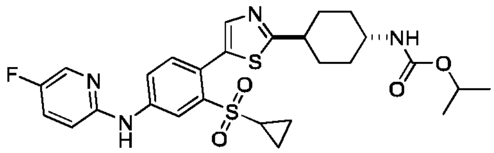

Synthetic Method

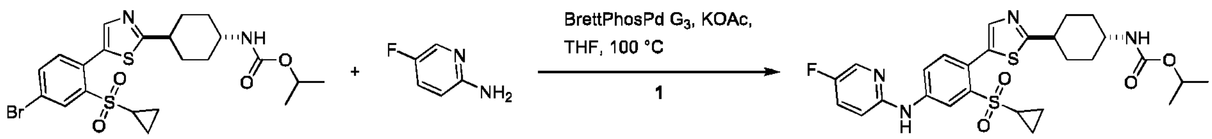


Isopropyl trans-N-[4-[5-(4-bromo-2-cyclopropylsulfonyl-phenyl)thiazol-2-yl]cyclohexyl] carbamate (250 mg, 474 μmol) and 2-amino-5-fluoropyridine (53.1 mg, 474 μmol) were dissolved in tetrahydrofuran (18 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (42.9 mg, 47.4 μmol) and potassium acetate (140 mg, 1.42 mmol) were added. The reaction solution was stirred and reacted at 100°C for 2 hours. The completion of the reaction was detected by LCMS. The reaction solution was concentrated to dryness under reduced pressure. Thereafter, the reaction solution was added with water (3 mL) and extracted with ethyl acetate (9 mL) three times. The organic phase was dried over anhydrous sodium sulfate and filtered. The filtrate was concentrated to dryness under reduced pressure and then purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (50% to 70%) as eluents) to obtain isopropyl trans-N-[4-[5-[2-cyclopropylsulfonyl]-4-[(5-fluoro-2-pyridyl)amino]phenyl] thiazol-2-yl]cyclohexyl]carbamate as a white solid compound (74.96 mg).

MS m/z (ESI): 559.0 $[M+H]^+$;

$^1$H NMR (400MHz, DMSO-d6) δ 9.76 (s, 1H), 8.30 (d, J = 2.3 Hz, 1H), 8.21 (d, J = 3.1 Hz, 1H), 8.10 (dd, J = 2.3, 8.4 Hz, 1H), 7.71-7.59 (m, 2H), 7.44 (d, J = 8.4 Hz, 1H), 7.05-6.90 (m, 2H), 4.74 (td, J = 6.1, 12.4 Hz, 1H), 3.29-3.23 (m, 1H), 2.93 (tt, J = 3.6, 11.9 Hz, 1H), 2.47-2.37 (m, 1H), 2.13 (br d, J = 11.4 Hz, 2H), 1.91 (br d, J = 9.9 Hz, 2H), 1.64-1.50 (m, 2H), 1.41-1.26 (m, 2H), 1.16 (d, J = 6.2 Hz, 6H), 1.04-0.83 (m, 4H).

Example 76: Synthesis of isopropyl trans-N-[4-[5-[2-cyclopropylsulfonyl-4-(3-pyridylamino)phenyl]thiazol-2-yl]cyclohexyl]carbamate

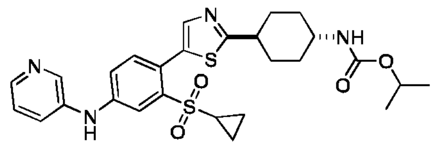

Synthetic Method

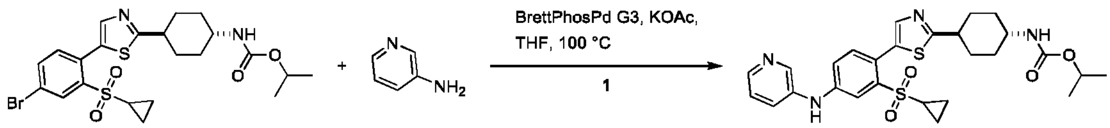


Isopropyl trans-N-[4-[5-(4-bromo-2-cyclopropylsulfonyl-phenyl)thiazol-2-yl]cyclohexyl] carbamate (50.0 mg, 94.8 μmol) and 3-aminopyridine (10.7 mg, 114 μmol) were dissolved in tetrahydrofuran (6 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (8.59 mg, 9.48 μmol) and potassium acetate (27.9 mg, 284 μmol) were added. The reaction solution was stirred and reacted at 100°C for 2 hours. The completion of the reaction was detected by LCMS. The reaction solution was concentrated to dryness under reduced pressure. Thereafter, the reaction solution was added with water (3 mL) and extracted with ethyl acetate (9 mL) three times. The organic phase was dried over anhydrous sodium sulfate and filtered. The filtrate was concentrated to dryness under reduced pressure and then purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (53% to 73%) as eluents) to obtain isopropyl trans-N-[4-[5-[2-cyclopropylsulfonyl-4-(3-pyridylamino)phenyl]thiazol-2-yl]cyclohexyl]carbamate as a white solid compound (10.65 mg).

MS m/z (ESI):541.1 $[M+H]^+$;

$^1H$ NMR (400MHz, DMSO-d6) δ 9.03 (s, 1H), 8.44 (d, J = 2.6 Hz, 1H), 8.19 (dd, J = 1.3, 4.7 Hz, 1H), 7.71-7.56 (m, 3H), 7.45-7.31 (m, 3H), 7.02 (br d, J = 7.8 Hz, 1H), 4.77-4.70 (m, 1H), 3.30-3.19 (m, 1H), 3.02-2.82 (m, 1H), 2.46-2.38 (m, 1H), 2.13 (br d, J = 11.5 Hz, 2H), 1.91 (br d, J = 10.3 Hz, 2H), 1.68-1.47 (m, 2H), 1.42-1.26 (m, 2H), 1.16 (d, J = 6.2 Hz, 6H), 1.03-0.80 (m, 4H).

Example 77: Synthesis of isopropyl trans-(4-(5-(2-(cyclopropylsulfonyl)-4-((5-methylpyridin-2-ylamino)phenyl)thiazol-2-yl)cyclohexyl)carbamate

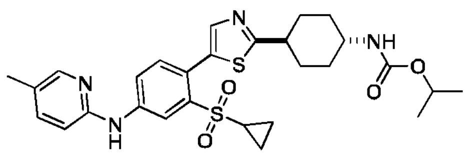

Synthetic Method

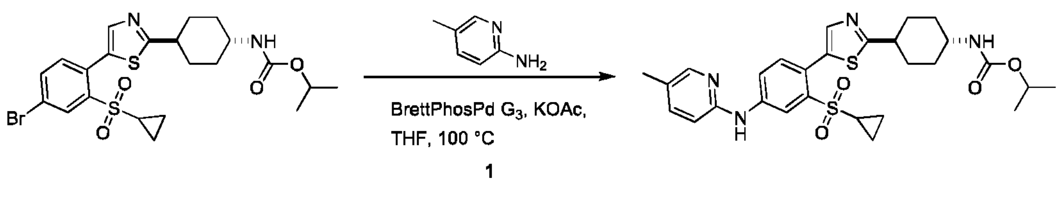

Step 1: Synthesis of isopropyl trans-(4-(5-(2-(cyclopropylsulfonyl)-4-((5-methylpyridin-2-ylamino)phenyl)thiazol-2-yl)cyclohexyl)carbamate

Isopropyl trans-(4-(5-(4-bromo-2-(cyclopropylsulfonyl)phenyl)thiazol-2-yl)cyclohexyl) carbamate (50.0 mg, 94.8 μmol) and 2-amino-5-methylpyridine (15.4 mg, 142 μmol) were dissolved in anhydrous tetrahydrofuran (3 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (8.59 mg, 9.48 μmol) and potassium acetate (27.9 mg, 284 μmol) were added. The reaction solution was stirred and reacted at 100°C for 1 hour. The completion of the reaction was detected by LCMS. The reaction solution was added with 5 mL of water and extracted with ethyl acetate (9 mL) three times. The organic phase was dried over anhydrous sodium sulfate and filtered. The filtrate was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.225% formic acid) and acetonitrile (45% to 65%) as eluents) to obtain isopropyl trans-(4-(5-(2-(cyclopropylsulfonyl)-4-((5-methylpyridin-2-ylamino)phenyl)thiazol-2-yl) cyclohexyl)carbamate as a white solid compound (4.64 mg).

MS m/z (ESI): 555.0 $[M+H]^+$;

$^1H$ NMR (400MHz, DMSO-d6) δ = 9.56 (s, 1H), 8.34 (d, J = 2.3 Hz, 1H), 8.11 (dd, J = 2.3, 8.5 Hz, 1H), 8.06 (s, 1H), 7.68 (s, 1H), 7.49 (dd, J = 2.1, 8.4 Hz, 1H), 7.42 (d, J = 8.5 Hz, 1H), 7.01 (br d, J = 7.5 Hz, 1H), 6.82 (d, J = 8.5 Hz, 1H), 4.81-4.69 (m, 1H), 3.30-3.28 (m, 1H), 2.92 (br t, J = 11.8 Hz, 1H), 2.44-2.38 (m, 1H), 2.20 (s, 3H), 2.13 (br d, J = 12.0 Hz, 2H), 1.91 (br d, J = 10.3 Hz, 2H), 1.64-1.48 (m, 2H), 1.41-1.27 (m, 2H), 1.16 (d, J = 6.3 Hz, 6H), 1.02-0.85 (m, 4H).

Example 78: Synthesis of isopropyl trans-(4-(5-(2-(cyclopropylsulfonyl)-4-((5-methoxypyridin-2-yl)amino)phenyl)thiazol-2-yl)cyclohexyl)carbamate

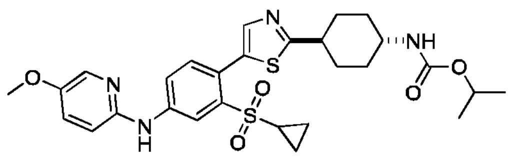

Synthetic Method

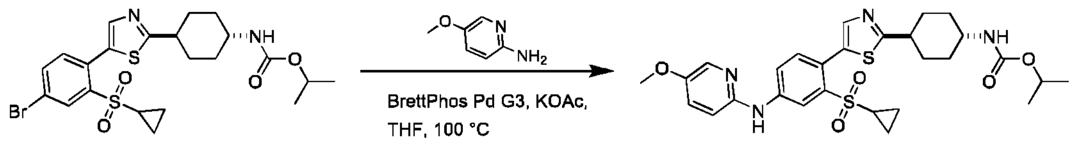


1

Isopropyl trans-(4-(5-(4-bromo-2-(cyclopropylsulfonyl)phenyl)thiazol-2-yl)cyclohexyl) carbamate (60.0 mg, 114 μmol) and 2-amino-5-methoxypyridine (28.2 mg, 227 μmol) were dissolved in tetrahydrofuran (4 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (10.3 mg, 11.4 μmol) and potassium acetate (33.4 mg, 341 μmol) were added. The reaction solution was stirred and reacted at 100°C for 2 hours. The completion of the reaction was detected by LCMS. The reaction solution was filtered, concentrated to dryness under reduced pressure, and purified by preparative liquid chromatography (YMC-ActusTriartC18 column: 5 μm silica, 30 mm in diameter, 150 mm in length; using decreasingly polar mixtures of water (containing 0.05% aqueous ammonia) and acetonitrile (53% to 73%) as eluents) to obtain isopropyl trans-(4-(5-(2-(cyclopropylsulfonyl)-4-((5-methoxypyridin-2-yl)amino)phenyl)thiazol-2-yl) cyclohexyl)carbamate as a white solid compound (27.90 mg).

MS m/z (ESI): 571.1 $[M+H]^+$;

$^1H$ NMR (400MHz, DMSO-d6) δ = 9.52 (s, 1H), 8.30 (d, J = 2.3 Hz, 1H), 8.07 (dd, J = 2.5, 8.5 Hz, 1H), 7.97 (d, J = 3.0 Hz, 1H), 7.68 (s, 1H), 7.44-7.33 (m, 2H), 7.02 (br d, J = 7.8 Hz, 1H), 6.90 (d, J = 9.0 Hz, 1H), 4.81 - 4.70 (m, 1H), 3.79 (s, 3H), 3.33-3.32 (m, 1H), 2.93 (s, 1H), 2.46-2.38 (m, 1H), 2.14 (br d, J = 12.0 Hz, 2H), 1.92 (br d, J = 10.3 Hz, 2H), 1.65-1.50 (m, 2H), 1.42-1.28 (m, 2H), 1.16 - 1.08 (m, 6H), 1.02-0.88 (m, 4H).

Example 79: Synthesis of isopropyl trans-(4-(5-(2-(cyclopropylsulfonyl)-4-(pyrimidin-2-ylamino)phenyl)thiazol-2-yl)cyclohexyl)carbamate

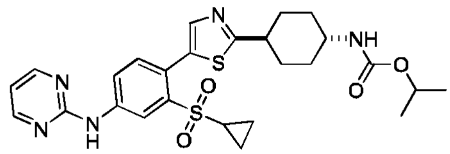

Synthetic Method

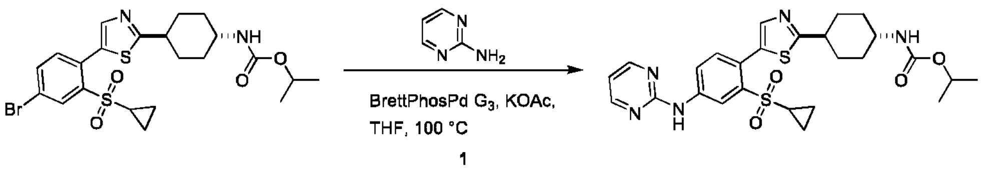


Isopropyl trans-(4-(5-(4-bromo-2-(cyclopropylsulfonyl)phenyl)thiazol-2-yl)cyclohexyl) carbamate (50.0 mg, 94.8 μmol) and 2-aminopyrimidine (13.5 mg, 142 μmol) were dissolved in anhydrous tetrahydrofuran (3 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (8.59 mg, 9.48 μmol) and potassium acetate (27.9 mg, 284 μmol) were added. The reaction solution was stirred and reacted at 100°C for 1 hour. The completion of the reaction was detected by LCMS. The reaction solution was added with water (3 mL) and extracted with ethyl acetate (9 mL) three times. The organic phase was dried over anhydrous sodium sulfate and filtered. The filtrate was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (48% to 68%) as eluents) to obtain isopropyl trans-(4-(5-(2-(cyclopropylsulfonyl)-4-(pyrimidin-2-ylamino)phenyl)thiazol-2-yl)cyclohexyl)carbamate as a white solid (12.32 mg).

MS m/z (ESI): 542.1 $[M+H]^+$;

$^1H$ NMR (400MHz, DMSO-d6) δ = 10.19 (s, 1H), 8.60-8.53 (m, 3H), 8.11 (dd, J = 2.5, 8.5 Hz, 1H), 7.70 (s, 1H), 7.48 (d, J = 8.3 Hz, 1H), 7.08-6.91 (m, 2H), 4.78-4.71 (m, 1H), 3.31 (s, 1H), 3.01-2.87 (m, 1H), 2.47-2.41 (m, 1H), 2.14 (br d, J = 11.5 Hz, 2H), 1.91 (br d, J = 10.8 Hz, 2H), 1.65-1.50 (m, 2H), 1.42-1.27 (m, 2H), 1.16 (d, J = 6.3 Hz, 6H), 1.04-0.95 (m, 2H), 0.94-0.86 (m, 2H).

Example 80: Synthesis of isopropyl trans-N-[4-[5-[2-cyclopropylsulfonyl]-4-(1,3,5-triazin-2-ylamino)phenyl]thiazol-2-yl]cyclohexyl]carbamate

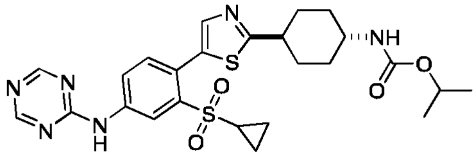

Synthetic Method

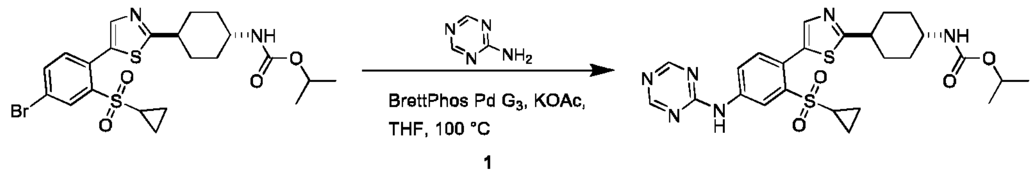


Isopropyl trans-(4-(5-(4-bromo-2-(cyclopropylsulfonyl)phenyl)thiazol-2-yl)cyclohexyl) carbamate (60.0 mg, 114 μmol) and 2-amino-1,3,5-triazine (21.8 mg, 2274 μmol) were dissolved in tetrahydrofuran (4 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (10.3 mg, 11.4 μmol) and potassium acetate (33.4 mg, 3414 μmol) were added. The reaction solution was stirred and reacted at 100°C for 2 hours. The completion of the reaction was detected by LCMS. The reaction solution was filtered, concentrated to dryness under reduced pressure, and purified by preparative liquid chromatography (YMC-ActusTriartC18 column: 5 μm silica, 30 mm in diameter, 150 mm in length; using decreasingly polar mixtures of water (containing 0.05% aqueous ammonia) and acetonitrile (44% to 64%) as eluents) to obtain isopropyl trans-N-[4-[5-[2-cyclopropylsulfonyl]-4-(1,3,5-triazin-2-ylamino)phenyl]thiazol-2-yl]cyclohexyl] carbamate as a white solid compound (18.7 mg).

MS m/z (ESI): 543.0 $[M+H]^+$;

$^1$H NMR (400MHz, DMSO-d6) δ = 10.78 (s, 1H), 8.78 (s, 2H), 8.47 (s, 1H), 8.04 (br d, J = 8.3 Hz, 1H), 7.73 (s, 1H), 7.54 (d, J = 8.5 Hz, 1H), 7.03 (br d, J = 8.0 Hz, 1H), 4.81-4.67 (m, 1H), 3.29-3.25 (m, 1H), 3.04-2.86 (m, 1H), 2.47-2.40 (m, 1H), 2.15 (br d, J = 12.5 Hz, 2H), 1.92 (br d, J = 9.8 Hz, 2H), 1.64-1.51 (m, 2H), 1.42-1.28 (m, 2H), 1.17 - 1.05 (m, 6H), 1.03-0.89 (m, 4H).

Example 81: Synthesis of isopropyl trans-N-[4-[5-[2-(cyclopropylsulfonimidoyl)]-4-[(5-fluoro-2-pyridyl)amino]phenyl]thiazol-2-yl]cyclohexyl]carbamate

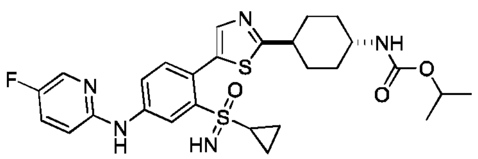

Synthetic Method

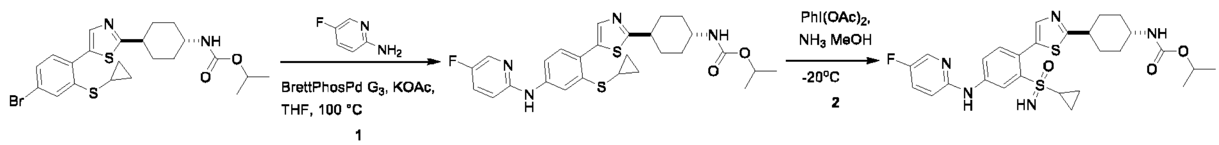


Step 1: Synthesis of isopropyl trans-N-[4-[5-[2-cyclopropylsulfanyl]-4-[(5-fluoro-2-pyridyl)amino]phenyl]thiazol-2-yl]cyclohexyl]carbamate

Isopropyl trans-N-[4-[5-(4-bromo-2-cyclopropylsulfanyl-phenyl)thiazol-2-yl]cyclohexyl] carbamate (190 mg, 383 μmol) and 2-amino-5-fluoropyridine (51.6 mg, 460 μmol) were dissolved in tetrahydrofuran (12 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (34.8 mg, 38.4 μmol) and potassium acetate (113 mg, 1.15 mmol) were added. The reaction solution was stirred and reacted at 100°C for 2 hours. The completion of the reaction was detected by LCMS. The reaction solution was added with water (3 mL) and extracted with ethyl acetate (9 mL) three times. The organic phase was dried over anhydrous sodium sulfate and filtered. The filtrate was concentrated to dryness under reduced pressure and then purified by thin-layer chromatography (silica, dichloromethane:methanol = 15:1) to obtain isopropyl trans-N-[4-[5-[2-cyclopropylsulfanyl]-4-[(5-fluoro-2-pyridyl)amino]phenyl]thiazol-2-yl]cyclohexyl] carbamate as a white solid compound (80.0 mg).

MS m/z (ESI): 527.2 [M+H]+;

Step 2: Synthesis of isopropyl trans-N-[4-[5-[2-(cyclopropylsulfonimidoyl)]-4-[(5-fluoro-2-pyridyl)amino]phenyl]thiazol-2-yl]cyclohexyl]carbamate

Isopropyl trans-N-[4-[5-[2-cyclopropylsulfanyl]-4-[(5-fluoro-2-pyridyl)amino]phenyl] thiazol-2-yl]cyclohexyl]carbamate (80 mg, 152 μmol) was dissolved in a solution of ammonia in methanol (6 mL), and diacetoxyiodobenzene (734 mg, 2.28 mmol) was added. The reaction solution was stirred and reacted at -20°C for 1 hour. The completion of the reaction was detected by LCMS. The reaction solution was concentrated to dryness under reduced pressure and then purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (48% to 68%) as eluents) to obtain isopropyl trans-N-[4-[5-[2-(cyclopropylsulfonimidoyl)]-4-[(5-fluoro-2-pyridyl)amino]phenyl] thiazol-2-yl]cyclohexyl]carbamate as a white solid compound (20.0 mg).
MS m/z (ESI): 558.0 $[M+H]^+$;
$^1H$ NMR (400MHz, DMSO-$d_6$) δ 9.67 (s, 1H), 8.21 (dd, J = 2.7, 18.1 Hz, 2H), 8.11 (dd, J = 2.4, 8.5 Hz, 1H), 7.71 (s, 1H), 7.62 (dt, J = 3.1, 8.7 Hz, 1H), 7.36 (d, J = 8.4 Hz, 1H), 7.04-6.90 (m, 2H), 4.78-4.71 (m, 1H), 4.32 (s, 1H), 3.29-3.24 (m, 1H), 2.97-2.84 (m, 1H), 2.31-2.26 (m, 1H), 2.13 (br d, J = 10.8 Hz, 2H), 1.91 (br d, J = 10.8 Hz, 2H), 1.63-1.49 (m, 2H), 1.41-1.26 (m, 2H), 1.16 (d, J = 6.1 Hz, 6H), 0.94-0.76 (m, 4H).

Example 82: Synthesis of isopropyl trans-N-[4-[5-[4-[(5-cyclopropyl-1H-pyrazol-3-yl)amino]-2-(cyclopropylsulfonimidoyl)phenyl]thiazol-2-yl]cyclohexyl]carbamate

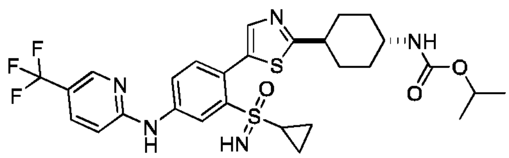

Synthetic Method

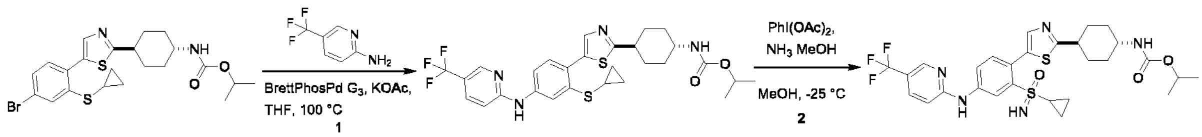


Step 1: Synthesis of isopropyl trans-(4-(5-(2-(cyclopropylthio)-4-((5-(trifluoromethyl)pyridin-2-yl)amino)phenyl)thiazol-2-yl)cyclohexyl)carbamate

Isopropyl trans-(4-(5-(4-bromo-2-(cyclopropylthio)phenyl)thiazol-2-yl)cyclohexyl) carbamate (200 mg, 404 μmol) and 2-amino-6-(trifluoromethyl)pyridine (131 mg, 807 μmol) were dissolved in anhydrous tetrahydrofuran (5 mL), and methanesulfonato(2-dicyclohexylphosphino)-3,6-dimethoxy-2,4,6-triisopropyl-1,1-biphenyl)(2-amino-1,1-biphenyl -2-yl)palladium (II) (36.6 mg, 40.4 μmol) and potassium acetate (118.8 mg, 1.21 mmol) were added. The reaction solution was stirred and reacted at 100°C for 1 hour. The completion of the reaction was detected by LCMS. The reaction solution was added with water (3 mL) and extracted with ethyl acetate (9 mL) three times. The organic phase was dried over anhydrous sodium sulfate and filtered. The filtrate was concentrated to dryness under reduced pressure. The resultant was purified by column chromatography (petroleum ether:ethyl acetate = 3:1) to obtain isopropyl trans-(4-(5-(2-(cyclopropylthio)-4-((5-(trifluoromethyl)pyridin-2-yl)amino) phenyl)thiazol-2-yl)cyclohexyl)carbamate as a white solid compound (120 mg).
MS m/z (ESI): 577.1 $[M+H]^+$;

Step 2: Synthesis of isopropyl trans-N-[4-[5-[4-[(5-cyclopropyl-1H-pyrazol-3-yl)amino]-2-(cyclopropylsulfonimidoyl)phenyl]thiazol-2-yl]cyclohexyl]carbamate

Isopropyl trans-(4-(5-(2-(cyclopropylthio)-4-((5-(trifluoromethyl)pyridin-2-yl)amino) phenyl)thiazol-2-yl)cyclohexyl)carbamate (120 mg, 208 μmol) was dissolved in a solution of ammonia in methanol (7M, 3 mL), and diacetoxyiodobenzene (1.01 g, 3.12 mmol) was added. The reaction solution was stirred and reacted at -25°C for 1 hour. The completion of the reaction was detected by LCMS. The reaction solution was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (53% to 73%) as eluents) to obtain isopropyl trans-N-[4-[5-[4-[(5-cyclopropyl-1H-pyrazol-3-yl)amino]-2-(cyclopropylsulfonimidoyl)phenyl]thiazol-2-yl]cyclohexyl]carbamate as a white solid compound (35.43 mg).

MS m/z (ESI): 608.0 $[M+H]^+$;

$^1H$ NMR (400MHz, DMSO-d6) δ = 10.13 (s, 1H), 8.56 (s, 1H), 8.34 (d, J = 2.3 Hz, 1H), 8.20 (dd, J = 2.4, 8.4 Hz, 1H), 7.95 (dd, J = 2.5, 8.9 Hz, 1H), 7.74 (s, 1H), 7.43 (d, J = 8.4 Hz, 1H), 7.06-6.98 (m, 2H), 4.78-4.71 (m, 1H), 4.39 (br s, 1H), 2.92 (tt, J = 3.5, 11.8 Hz, 1H), 2.38-2.28 (m, 1H), 2.14 (br d, J = 11.9 Hz, 2H), 1.92 (br d, J = 10.1 Hz, 2H), 1.65-1.50 (m, 2H), 1.42-1.28 (m, 2H), 1.17 (d, J = 6.3 Hz, 6H), 0.96-0.79 (m, 4H).

Example 83: Synthesis of isopropyl trans-(4-(5-(2-(cyclopropylsulfonyl)-4-(pyridin-2-yloxy)phenyl)thiazol-2-yl)cyclohexyl)carbamate

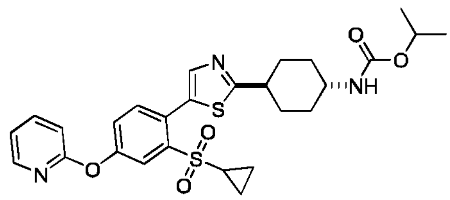

Synthetic Method

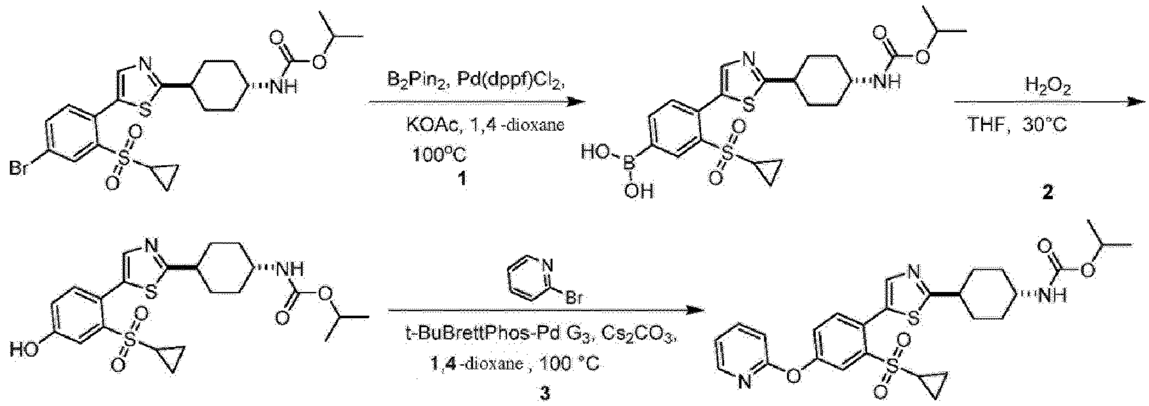


Step 1: Synthesis of trans-(3-(cyclopropylsulfonyl)-4-(2-((4-((isopropoxycarbonyl) amino)cyclohexyl)thiazol-5-yl)phenyl)boronic acid

Isopropyl trans-(4-(5-(4-bromo-2-(cyclopropylsulfonyl)phenyl)thiazol-2-yl)cyclohexyl) carbamate (200 mg, 379 μmol) and bis(pinacolato)diboron (481 mg, 1.90 mmol) were dissolved in anhydrous tetrahydrofuran (5 mL). Under the protection of nitrogen, dichlorobis[di-tert-butyl-(4-dimethylaminophenyl)phosphine]palladium (II) (31.0 mg, 37.9 μmol) and potassium acetate (112 mg, 1.14 mmol) were added. The reaction solution was stirred and reacted at 100°C for 16 hours. The completion of the reaction was detected by LCMS. The reaction solution was concentrated to dryness under reduced pressure and purified by thin-layer chromatography (silica, dichloromethane:methanol = 15:1) to obtain trans-(3-(cyclopropylsulfonyl)-4-(2-((4-((isopropoxycarbonyl)amino)cyclohexyl)thiazol-5-yl) phenyl)boronic acid as a yellow solid compound (120 mg).

MS m/z (ESI): 493.0 $[M+H]^+$;

Step 2: Synthesis of isopropyl trans-(4-(5-(2-(cyclopropylsulfonyl)-4-hydroxyphenyl) thiazol-2-yl)cyclohexyl)carbamate

Trans-(3-(cyclopropylsulfonyl)-4-(2-((4-((isopropoxycarbonyl)amino)cyclohexyl)thiazol-5-yl)phenyl)boronic acid (120.0 mg, 244 μmol) was dissolved in a tetrahydrofuran solution (600 μL), and 30% hydrogen peroxide (3.54, 31.2 mmol) was added. The reaction solution was stirred and reacted at 30°C for 0.5 hours. The completion of the reaction was detected by LCMS. The reaction solution was concentrated to dryness under reduced pressure. The reaction solution was added with 9 mL of ethyl acetate (3 mL × 3) and 5 mL of an aqueous sodium sulfite solution. The organic phase was dried over anhydrous sodium sulfate and filtered. The filtrate was concentrated to dryness under reduced pressure to obtain the compound isopropyl trans-(4-(5-(2-(cyclopropylsulfonyl)-4-hydroxyphenyl)thiazol-2-yl)cyclohexyl)carbamate (60.0 mg).
MS m/z (ESI): 465.2 $[M+H]^+$;

Step 3: Synthesis of isopropyl trans-(4-(5-(2-(cyclopropylsulfonyl)-4-(pyridin-2-yloxy)phenyl)thiazol-2-yl)cyclohexyl)carbamate

Isopropyl trans-(4-(5-(2-(cyclopropylsulfonyl)-4-hydroxyphenyl)thiazol-2-yl)cyclohexyl) carbamate (55 mg, 118.38 μmol) and 2-bromopyridine (28.06 mg, 177.57 μmol) were dissolved in dioxane (2.5 mL), and methanesulfonato(2-(di-tert-butylphosphino)-3,6-dimethoxy-2,4,6-triisopropyl-1,1-biphenyl)(2-amino-1,1-biphenyl-2-yl)palladium (II) (10.11 mg, 11.84 μmol) and cesium carbonate (115.71 mg, 355.15 μmol) were added. The reaction solution was stirred and reacted at 100°C for 16 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by preparative liquid chromatography (Phenomenex Gemini C18 column: 3 μm silica, 40 mm in diameter, 75 mm in length; using decreasingly polar mixtures of water (containing 0.05% aqueous ammonia) and acetonitrile (44% to 84%) as eluents) to obtain isopropyl trans-(4-(5-(2-(cyclopropylsulfonyl)-4-(pyridin-2-yloxy)phenyl)thiazol-2-yl)cyclohexyl)carbamate as a white solid compound (14.65 mg).
MS m/z (ESI): 442.0 $[M+H]^+$;
$^1H$ NMR (400MHz, DMSO-d6) δ 8.21 (dd, J = 1.3, 5.0 Hz, 1H), 7.98-7.91 (m, 1H), 7.78 (s, 1H), 7.74 (d, J = 2.5 Hz, 1H), 7.65-7.59 (m, 1H), 7.58-7.50 (m, 1H), 7.26-7.18 (m, 1H), 7.27-7.17 (m, 1H), 7.03 (br d, J = 7.5 Hz, 1H), 4.74 (td, J = 6.3, 12.5 Hz, 1H), 3.31 (br s, 1H), 3.31-3.26 (m, 1H), 3.02-2.90 (m, 1H), 2.54 (s, 1H), 2.15 (br d, J = 12.3 Hz, 2H), 1.92 (br d, J = 10.3 Hz, 2H), 1.65-1.51 (m, 2H), 1.42-1.28 (m, 2H), 1.17 - 1.11 (m, 6H), 1.03-0.85 (m, 4H).

Example 84: Synthesis of isopropyl trans-N-[4-[5-[2-cyclopropylsulfonyl]-4-[[1-(2-(hydroxyethyl)pyrazol-4-yl]amino]phenyl]thiazol-2-yl]cyclohexyl]carbamate

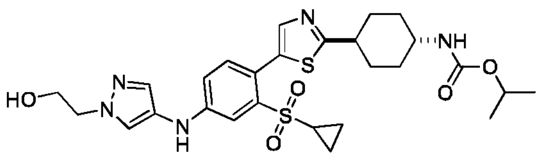

Synthetic Method

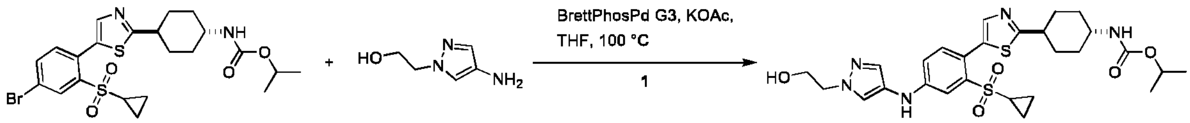


Isopropyl trans-N-[4-[5-(4-bromo-2-cyclopropylsulfonyl-phenyl)thiazol-2-yl]cyclohexyl] carbamate (60 mg, 114 μmol) and 2-(4-amino-1H-pyrazol-1-yl)ethanol (17.4 mg, 137 μmol) were dissolved in tetrahydrofuran (4 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (10.3 mg, 11.4 μmol) and potassium acetate (22.3 mg, 227 μmol) were added. The reaction solution was stirred and reacted at 100°C for 2 hours. The completion of the reaction was detected by LCMS. The reaction solution was concentrated to dryness under reduced pressure. The reaction solution was added

with water (3 mL) and extracted with ethyl acetate (9 mL) three times. The organic phase was dried over anhydrous sodium sulfate and filtered. The filtrate was concentrated to dryness under reduced pressure and then purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (42% to 62%) as eluents) to obtain isopropyl trans-N-[4-[5-[2-cyclopropylsulfonyl]-4-[[1-(2-(hydroxyethyl)pyrazol-4-yl]amino] phenyl]thiazol-2-yl]cyclohexyl]carbamate as a white solid compound (30.0 mg).

MS m/z (ESI):574.1 $[M+H]^+$;

$^1H$ NMR (400MHz, DMSO-d6) δ 8.25 (s, 1H), 7.72 (s, 1H), 7.65-7.60 (m, 1H), 7.62 (s, 1H), 7.45-7.38 (m, 2H), 7.27 (d, J = 8.5 Hz, 1H), 7.06-6.93 (m, 2H), 4.90 (t, J = 5.3 Hz, 1H), 4.78-4.71 (m, 1H), 4.11 (t, J = 5.6 Hz, 2H), 3.73 (q, J = 5.8 Hz, 2H), 3.28 (br d, J = 2.3 Hz, 1H), 2.98-2.83 (m, 1H), 2.41-2.32 (m, 1H), 2.12 (br d, J = 11.8 Hz, 2H), 1.90 (br d, J = 10.5 Hz, 2H), 1.65-1.46 (m, 2H), 1.41-1.25 (m, 2H), 1.16 (d, J = 6.3 Hz, 6H), 1.00-0.78 (m, 4H).

Example 85: Synthesis of isopropyl trans-N-[4-[5-[2-cyclopropylsulfonyl]-4-[[1-(2-fluoroethyl)pyrazol-4-yl]amino]phenyl]thiazol-2-yl]cyclohexyl]carbamate

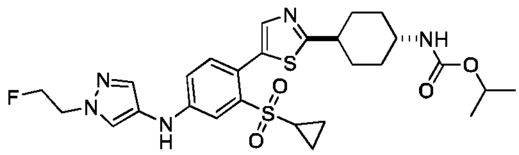

Synthetic Method

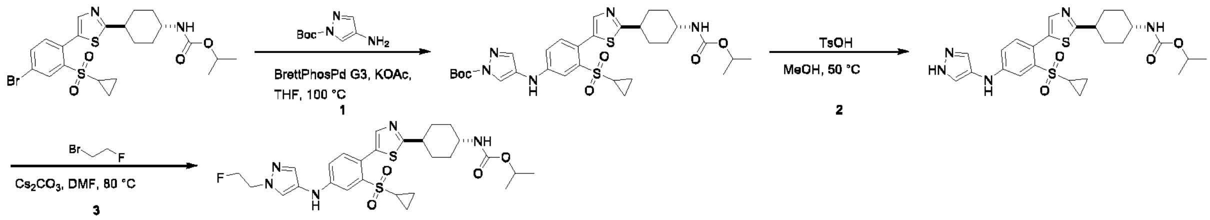


Step 1: Synthesis of tert-butyl trans-4-[3-3-cyclopropylsulfonyl-4-[2-[4-(isopropoxycarbonylamino)cyclohexyl]thiazol-5-yl]anilino]pyrazole-1-carboxylate

Isopropyl trans-N-[4-[5-(4-bromo-2-cyclopropylsulfonyl-phenyl)thiazol-2-yl]cyclohexyl] carbamate (230 mg, 436 μmol) and tert-butyl 4-aminopyrazole-1-carboxylate (95.9 mg, 523 μmol) were dissolved in tetrahydrofuran (10 mL), and methanesulfonato(2-dicyclohexylphosphino)-3,6-dimethoxy-2,4,6-triisopropyl-1,1-biphenyl)(2-amino-1,1-biphenyl -2-yl)palladium (II) (39.5 mg, 43.6 μmol) and potassium acetate (128.4 mg, 1.31 mmol) were added. The reaction solution was stirred and reacted at 100°C for 2 hours. The completion of the reaction was detected by LCMS. The reaction solution was added with water (3 mL) and extracted with ethyl acetate (9 mL) three times. The organic phase was dried over anhydrous sodium sulfate and filtered. The filtrate was concentrated to dryness under reduced pressure and then purified by thin-layer chromatography (silica, dichloromethane:methanol = 10:1) to obtain tert-butyl trans-4-[3-3-cyclopropylsulfonyl-4-[2-[4-(isopropoxycarbonylamino) cyclohexyl]thiazol-5-yl]anilino]pyrazole-1-carboxylate as a yellow solid compound (158 mg).

MS m/z (ESI):630.3 $[M+H]^+$;

Step 2: Synthesis of isopropyl trans-N-[4-[5-[2-cyclopropylsulfonyl]-4-(1H-pyrazol-4-ylamino)phenyl]thiazol-2-yl]cyclohexyl]carbamate

Tert-butyl trans-4-[3-3-cyclopropylsulfonyl-4-[2-[4-(isopropoxycarbonylamino) cyclohexyl]thiazol-5-yl]anilino]pyrazole-1-carboxylate (158 mg, 251 μmol) was dissolved in methanol (6 mL), and p-toluenesulfonic acid monohydrate (216 mg, 1.25 mmol) was added. The reaction solution was stirred and reacted at 50°C for 2 hours. The completion of the reaction was detected by LCMS. The reaction solution was concentrated to dryness under reduced pressure. The

reaction solution was added with a saturated aqueous sodium carbonate solution (3 mL) and extracted with ethyl acetate (9 mL) three times. The organic phase was dried over anhydrous sodium sulfate and filtered. The filtrate was concentrated to dryness under reduced pressure to obtain isopropyl trans-N-[4-[5-[2-cyclopropylsulfonyl]-4-(1H-pyrazol-4-ylamino)phenyl]thiazol-2-yl]cyclohexyl]carbamate as a yellow solid compound (127 mg).

MS m/z (ESI):530.2 $[M+H]^+$;

Step 3: Synthesis of isopropyl trans-N-[4-[5-[2-cyclopropylsulfonyl]-4-[[1-(2-fluoroethyl)pyrazol-4-yl]amino]phenyl]thiazol-2-yl]cyclohexyl]carbamate

Isopropyl trans-N-[4-[5-[2-cyclopropylsulfonyl]-4-(1H-pyrazol-4-ylamino)phenyl]thiazol-2-yl]cyclohexyl]carbamate (98 mg, 185 μmol) and 1-bromo-2-fluoroethane (70.5 mg, 555 μmol) were dissolved in N,N-dimethylformamide (2 mL), and then cesium carbonate (181 mg, 555 μmol) was added. The reaction solution was stirred and reacted at 80°C for 16 hours. The completion of the reaction was detected by LCMS. The reaction solution was filtered, concentrated to dryness under reduced pressure, and purified by preparative liquid chromatography (YMC-Actus Triart C18 column: 5 μm silica, 25 mm in diameter, 150 mm in length; using decreasingly polar mixtures of water (containing 0.05% aqueous ammonia) and acetonitrile (40% to 60%) as eluents) to obtain the compound isopropyl trans-N-[4-[5-[2-cyclopropylsulfonyl]-4-[[1-(2-fluoroethyl)pyrazol-4-yl]amino]phenyl]thiazol-2-yl]cyclohexyl]carbamate (6.35 mg).

MS m/z (ESI): 576.1 $[M+H]^+$;

$^1$H NMR (400MHz, DMSO-d6) δ 8.29 (s, 1H), 7.80 (s, 1H), 7.62 (s, 1H), 7.47 (s, 1H), 7.41 (d, J = 2.5 Hz, 1H), 7.28 (d, J = 8.5 Hz, 1H), 7.06 - 6.95 (m, 2H), 4.83 (t, J = 4.8 Hz, 1H), 4.78-4.67 (m, 2H), 4.44 (t, J = 4.8 Hz, 1H), 4.37 (t, J = 4.8 Hz, 1H), 3.28-3.26 (m, 1H), 2.96-2.84 (m, 1H), 2.42-2.32 (m, 1H), 2.12 (br d, J = 12.0 Hz, 2H), 1.91 (br d, J = 10.0 Hz, 2H), 1.63-1.48 (m, 2H), 1.40-1.26 (m, 2H), 1.16 (d, J = 6.3 Hz, 6H), 0.99-0.78 (m, 4H).

Example 86: Synthesis of isopropyl trans-N-[4-[5-[2-cyclopropylsulfonyl]-4-[[1-(oxetan-3-yl)pyrazol-4-yl]amino]phenyl]thiazol-2-yl]cyclohexyl]carbamate

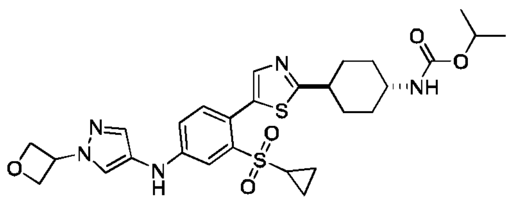

Synthetic Method

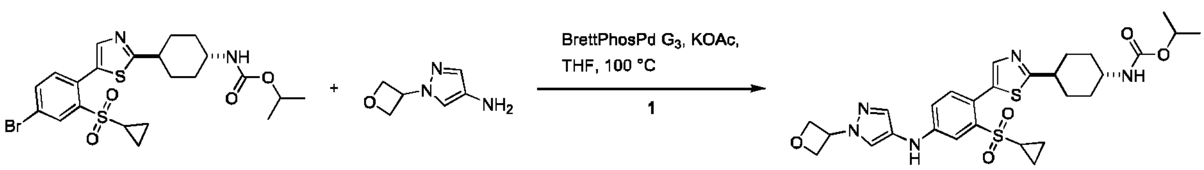


Isopropyl trans-N-[4-[5-(4-bromo-2-cyclopropylsulfonyl-phenyl)thiazol-2-yl]cyclohexyl] carbamate (60 mg, 114 μmol) and 1-(oxetan-3-yl)-1H-pyrazol-4-amine (19.0 mg, 137 μmol) were dissolved in tetrahydrofuran (4 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (10.3 mg, 11.4 μmol) and potassium acetate (22.3 mg, 227 μmol) were added. The reaction solution was stirred and reacted at 100°C for 2 hours. The completion of the reaction was detected by LCMS. The reaction solution was concentrated to dryness under reduced pressure. The reaction solution was added with water (3 mL) and extracted with ethyl acetate (9 mL) three times. The organic phase was dried over anhydrous sodium sulfate and filtered. The filtrate was concentrated to dryness under reduced pressure and then purified by thin-layer chromatography (silica, dichloromethane:methanol = 15:1)) to obtain isopropyl trans-N-[4-[5-[2-cyclopropylsulfonyl]-4-[[1-(oxetan-3-yl)pyrazol-4-yl]amino] phenyl]thiazol-2-yl]cyclohexyl]carbamate as a yellow solid compound (31.52 mg).

MS m/z (ESI):586.1 [M+H]+;

$^1$H NMR (400MHz, DMSO-d6) δ 8.35 (s, 1H), 8.07-7.78 (m, 1H), 7.63 (s, 1H), 7.56 (s, 1H), 7.42 (d, J = 2.5 Hz, 1H), 7.28 (d, J = 8.5 Hz, 1H), 7.02 (dd, J = 2.5, 8.5 Hz, 2H), 5.55 (quin, J = 7.0 Hz, 1H), 4.91 (d, J = 6.8 Hz, 3H), 4.93 (s, 1H), 4.78-4.71 (m, 1H), 3.31 (br s, 1H), 2.98-2.83 (m, 1H), 2.42-2.34 (m, 1H), 2.12 (br d, J = 12.0 Hz, 2H), 1.98-1.84 (m, 2H), 1.67-1.45 (m, 2H), 1.42-1.26 (m, 2H), 1.16 (d, J = 6.3 Hz, 6H), 1.00-0.76 (m, 4H).

Example 87: Synthesis of isopropyl trans-N-[4-[5-(2-cyclopropylsulfonyl-4-pyrazol-1-yl-phenyl)thiazol-2-yl]cyclohexyl]carbamate

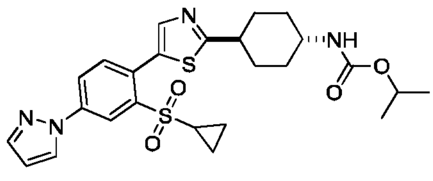

Synthetic Method

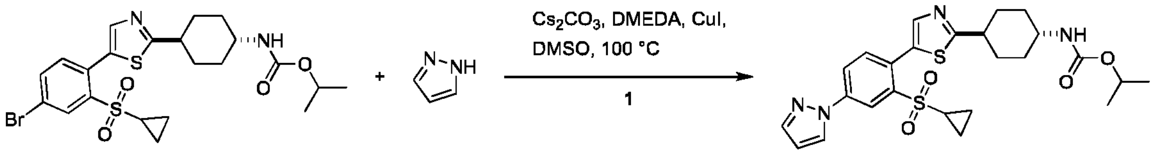


Isopropyl trans-N-[4-[5-(4-bromo-2-cyclopropylsulfonyl-phenyl)thiazol-2-yl]cyclohexyl] carbamate (150 mg, 284 μmol) and pyrazole (23.2 mg, 341 μmol) were dissolved in dimethyl sulfoxide (800 μL). Under the protection of nitrogen, cuprous iodide (27.1 mg, 142 μmol), N,N-dimethylethylenediamine (25.1 mg, 284 μmol) and cesium carbonate (278 mg, 853 μmol) were added. The reaction solution was stirred and reacted at 100°C for 16 hours. The completion of the reaction was detected by LCMS. The reaction solution was concentrated to dryness under reduced pressure. Thereafter, the reaction solution was added with a saturated aqueous ammonium chloride solution (5 mL) and extracted with ethyl acetate (15 mL) three times. The organic phase was dried over anhydrous sodium sulfate and filtered. The filtrate was concentrated to dryness under reduced pressure and then purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (43% to 63%) as eluents) to obtain isopropyl trans-N-[4-[5-(2-cyclopropylsulfonyl-4-pyrazol-1-yl-phenyl)thiazol-2-yl]cyclohexyl]carbamate as a white solid compound (8.55 mg).

MS m/z (ESI): 515.0 [M+H]+;

$^1$H NMR (400MHz, DMSO-d6) δ 8.72 (d, J = 2.5 Hz, 1H), 8.49 (d, J = 2.5 Hz, 1H), 8.21 (dd, J = 2.4, 8.4 Hz, 1H), 7.86 (d, J = 1.5 Hz, 1H), 7.80 (s, 1H), 7.72 (d, J = 8.3 Hz, 1H), 7.04 (br d, J = 8.0 Hz, 1H), 6.67-6.61 (m, 1H), 4.78-4.71 (m, 1H), 3.20 (br s, 1H), 3.02-2.91 (m, 1H), 2.46-2.43 (m, 1H), 2.15 (br d, J = 12.5 Hz, 2H), 1.92 (br d, J = 10.5 Hz, 2H), 1.66-1.52 (m, 2H), 1.42-1.27 (m, 2H), 1.16 (d, J = 6.0 Hz, 6H), 1.05-0.92 (m, 4H).

Example 88: Synthesis of isopropyl trans-(4-(5-(2-(cyclobutylsulfonyl)-4-((5-methyl-1H-pyrazol-3-yl)amino)phenyl)thiazol-2-yl)cyclohexyl)carbamate

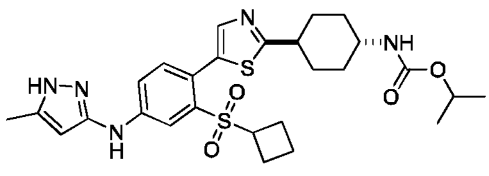

Synthetic Method

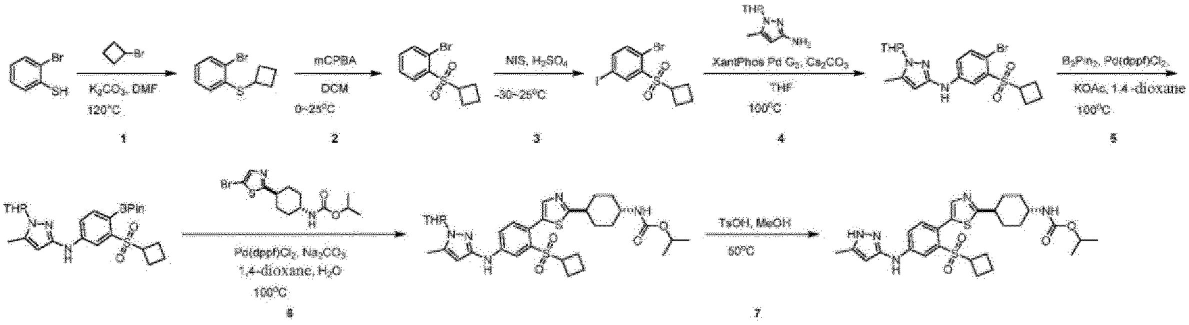


Step 1: Synthesis of (2-bromophenyl)(cyclobutyl)sulfane

o-Bromothiophenol (5 g, 26.4 mmol, 3.1 mL) was dissolved in N,N-dimethylformamide (50 mL), and potassium carbonate (5.48 g, 39.7 mmol) and cyclobutyl bromide (4.64 g, 34.4 mmol, 3.3 mL) were added. The reaction solution was stirred and reacted at 120°C for 24 hours. TLC (petroleum ether:ethyl acetate = 1:0) detection showed that the reaction was complete. The reaction solution was concentrated to dryness under reduced pressure and then purified by column chromatography (silica, petroleum ether: ethyl acetate = 1:0) to obtain (2-bromophenyl)(cyclobutyl)sulfane (4.5 g).

$^1$H NMR (400MHz, DMSO-d6) $\delta$ = 7.59 (dd, J = 1.1, 7.9 Hz, 1H), 7.36 (t, J = 7.1 Hz, 1H), 7.16 (d, J = 7.5 Hz, 1H), 7.08 (t, J = 7.3 Hz, 1H), 4.07 - 3.98 (m, 1H), 2.60 - 2.53 (m, 2H), 2.09 - 1.94 (m, 4H).

Step 2: Synthesis of 1-bromo-2-(cyclobutylsulfonyl)benzene

(2-Bromophenyl)(cyclobutyl)sulfane (2.0 g, 8.22 mmol) was dissolved in dichloromethane (20 mL), and m-chloroperoxybenzoic acid (5.68 g, 32.9 mmol) was added at 0ºC. The reaction solution was stirred and reacted at 25°C for 16 hours. The completion of the reaction was detected by LCMS. The reaction solution was concentrated to dryness under reduced pressure, added with a saturated sodium carbonate solution (30 mL), and extracted with 90 mL of dichloromethane (30 mL $\times$ 3). The organic phase was dried over anhydrous sodium sulfate and filtered. The filtrate was concentrated to dryness under reduced pressure and then purified by thin-layer chromatography (silica, petroleum ether:ethyl acetate = 10:1) to obtain 1-bromo-2-(cyclobutylsulfonyl)benzene as a colorless product (2.0 g, 7.27 mmol).

MS m/z (ESI): 275.0, 277.0 $[M+H]^+$;

Step 3: Synthesis of 1-bromo-2-(cyclobutylsulfonyl)-4-iodobenzene

1-Bromo-2-(cyclobutylsulfonyl)benzene (2.2 g, 8.00 mmol) was dissolved in sulfuric acid (20 mL), and then N-iodosuccinimide (5.40 g, 24.0 mmol) was added at -30°C. The reaction solution was stirred and reacted at 25°C for 1 hour. The completion of the reaction was detected by LCMS. The reaction solution was quenched with water (100 mL), and then extracted with dichloromethane (200 mL) twice. The organic phase was dried over anhydrous sodium sulfate and filtered. The filtrate was concentrated to dryness under reduced pressure. The resultant was slurried with an ethyl acetate solution to obtain 1-bromo-2-(cyclobutylsulfonyl)-4-iodobenzene as a white solid compound (1.5 g, 3.74 mmol).

MS m/z (ESI): 417.9, 419.9 $[M+NH_3]^+$;

Step 4: Synthesis of N-(4-bromo-3-(cyclobutylsulfonyl)phenyl)-5-methyl-1-(tetrahydro-2H-pyran-2-yl)-1H-pyrazol-3-amine

1-Bromo-2-(cyclobutylsulfonyl)-4-iodobenzene (900 mg, 2.24 mmol) and 5-methyl-1-tetrahydropyran-2-yl-pyrazol-3-amine (447 mg, 2.47 mmol) were dissolved in tetrahydrofuran (5 mL). Under the protection of nitrogen, methanesulfonato[9,9-dimethyl-4,5-bis(diphenylphosphino)xanthene][2-amino-1,1-diphenyl]palladium (II) dichloromethane adduct (213 mg, 224 $\mu$mol) and cesium carbonate (1.46 g, 4.49 mmol) were added. The reaction solution was stirred and reacted at 100°C for 1 hour. The completion of the reaction was detected by LCMS. The reaction solution was concentrated to dryness under reduced pressure and purified by column chromatography (silica, petroleum ether:ethyl acetate = 4:1) to obtain N-(4-bromo-3-(cyclobutylsulfonyl)phenyl)-5-methyl-1-(tetrahydro-2H-pyran-2-yl)-1H-pyrazol -3-amine as a white solid compound (500 mg, 1.10 mmol).

MS m/z (ESI):454.1, 456.1 $[M+H]^+$;

Step 5: Synthesis of N-(3-(cyclobutylsulfonyl)-4-(4,4,5,5-tetramethyl-1,3,2-dioxaborolan-2-yl)phenyl)-5-methyl-1-(tetrahydro-2H-pyran-2-yl)-1H-pyrazol-3-amine

N-(4-bromo-3-(cyclobutylsulfonyl)phenyl)-5-methyl-1-(tetrahydro-2H-pyran-2-yl)-1H-pyrazol-3-amine (300 mg, 660.24 μmol) and bis(pinacolato)diboron (670.64 mg, 2.64 mmol) were dissolved in dioxane (5 mL). Under the protection of nitrogen, dichlorobis[di-tert-butyl-(4-dimethylaminophenyl)phosphine]palladium (II) (46.75 mg, 66.02 μmol) and potassium acetate (129.59 mg, 1.32 mmol) were added. The reaction solution was stirred and reacted at 100°C for 16 hours. The completion of the reaction was detected by LCMS. The reaction solution was concentrated to dryness under reduced pressure and purified by column chromatography (silica, petroleum ether:ethyl acetate = 3:1) to obtain N-(3-(cyclobutylsulfonyl)-4-(4,4,5,5-tetramethyl-1,3,2-dioxaborolan-2-yl)phenyl)-5-methyl-1-( tetrahydro-2H-pyran-2-yl)-1H-pyrazol-3-amine as a white substance (150 mg).

MS m/z (ESI): 502.3 $[M+H]^+$;

Step 6: Synthesis of isopropyl trans-(4-(5-(2-(cyclobutylsulfonyl)-4-((5-methyl-1-(tetrahydro-2H-pyran-2-yl)-1H-pyrazol-3-yl)amino)phenyl)thiazol-2-yl)cyclohexyl)carbamate

N-(3-(cyclobutylsulfonyl)-4-(4,4,5,5-tetramethyl-1,3,2-dioxaborolan-2-yl)phenyl)-5-methyl-1-(tetrahydro-2H-pyran-2-yl)-1H-pyrazol-3-amine (150 mg, 299 μmol) and isopropyl trans-N-[4-(5-bromothiazol-2-yl)cyclohexyl]carbamate (94.4 mg, 272 μmol) were dissolved in water (0.4 mL) and dioxane (4 mL). Thereafter, [1,1-bis(diphenylphosphino)ferrocene]dichloropalladium - dichloromethane (22.2 mg, 27.2 μmol) and sodium carbonate (57.7 mg, 544 μmol) were added under the condition where nitrogen gas was introduced. The reaction solution was stirred and reacted at 100°C for 16 hours. The completion of the reaction was detected by LCMS. The reaction solution was concentrated to dryness under reduced pressure and purified by thin-layer chromatography (silica, petroleum ether:tetrahydrofuran = 1:1) to obtain isopropyl trans-(4-(5-(2-(cyclobutylsulfonyl)-4-((5-methyl-1-(tetrahydro-2H-pyran-2-yl)-1H-pyrazol-3-yl )amino)phenyl)thiazol-2-yl)cyclohexyl)carbamate as a yellow substance (70.0 mg, 109 μmol).

MS m/z (ESI): 642.4 $[M+H]^+$;

Step 7: Synthesis of isopropyl trans-(4-(5-(2-(cyclobutylsulfonyl)-4-((5-methyl-1H-pyrazol-3-yl)amino)phenyl)thiazol-2-yl)cyclohexyl)carbamate

Isopropyl trans-(4-(5-(2-(cyclobutylsulfonyl)-4-((5-methyl-1-(tetrahydro-2H-pyran-2-yl)-1H-pyrazol-3-yl)amino)phenyl)thiazol-2-yl)cyclohexyl)carbamate (70.0 mg, 109 μmol) was dissolved in methanol (3 mL), and p-toluenesulfonic acid monohydrate (93.9 mg, 545 μmol) was added. The reaction solution was stirred and reacted at 50°C for 1 hour. The completion of the reaction was detected by LCMS. The reaction solution was concentrated to dryness under reduced pressure. The reaction solution was added with 60 mL of dichloromethane (20 mL × 3) and 20 mL of saturated sodium bicarbonate solution. The organic phase was dried over anhydrous sodium sulfate and filtered. The filtrate was concentrated to dryness under reduced pressure and purified by preparative liquid chromatography (YMC-ActusTriartC18 column, 5 μm silica, 30 mm in diameter, 150 mm in length; using decreasingly polar mixtures of water (containing 0.05% aqueous ammonia) and acetonitrile as eluents) to obtain isopropyl trans-(4-(5-(2-(cyclobutylsulfonyl)-4-((5-methyl-1H-pyrazol-3-yl)amino)phenyl)thiazol-2-yl) cyclohexyl)carbamate as a white solid compound (23.57 mg).

MS m/z (ESI): 558.0 $[M+H]^+$;

$^1H$ NMR (400MHz, DMSO-d6) δ = 11.90-11.87 (m, 1H), 9.06 (s, 1H), 8.13 (d, J = 2.5 Hz, 1H), 7.64-7.61 (m, 2H), 7.31 (d, J = 8.5 Hz, 1H), 7.03 (br d, J = 7.5 Hz, 1H), 5.65-5.63 (m, 1H), 4.78-4.71 (m, 1H), 3.65 (t, J = 8.2 Hz, 1H), 3.33-3.30 (m, 1H), 2.96-2.88 (m, 1H), 2.24-2.11 (m, 6H), 2.06-1.97 (m, 2H), 1.95-1.80 (m, 4H), 1.62-1.51 (m, 2H), 1.40-1.29 (m, 2H), 1.17 (d, J = 6.3 Hz, 6H).

Examples 89 and 89-1: Synthesis of isopropyl trans-(4-(5-(2-(cyclopropylsulfonyl)-4-((5-methyl-1H-pyrazol-3-yl)amino)phenyl)thiophen-2-yl)cyclohexyl)carbamate and isopropyl cis-(4-(5-(2-(cyclopropylsulfonyl)-4-((5-methyl-1H-pyrazol-3-yl)amino)phenyl)thiophen-2-yl) cyclohexyl)carbamate

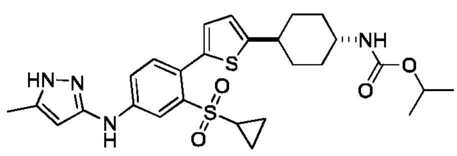

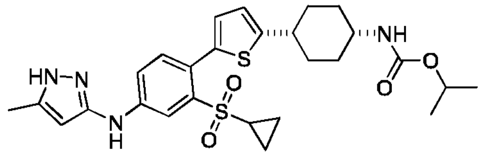

Synthetic Method

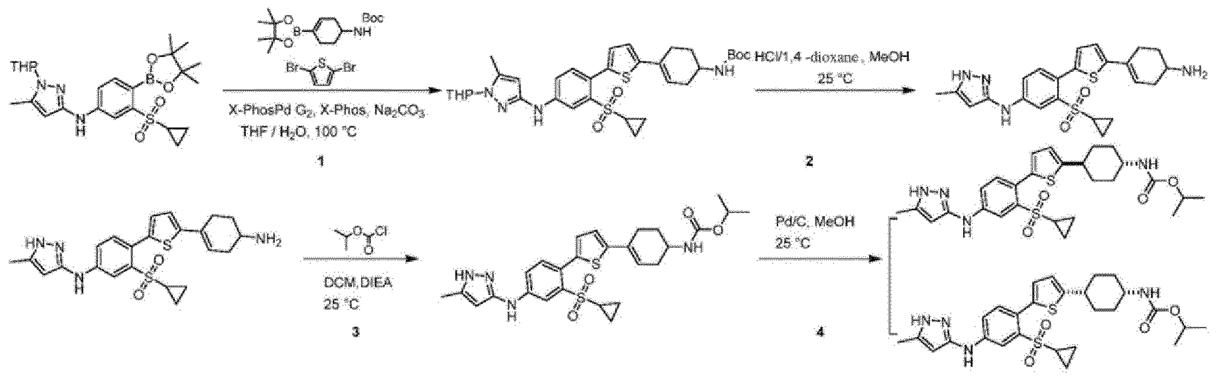


Step 1: Synthesis of tert-butyl (4-(5-(2-(cyclopropylsulfonyl)-4-((5-methyl-1-(tetrahydro-2H-pyran-2-yl)-1H-pyrazol-3-yl)amino)phenyl)thiophen-2-yl)cyclohex-3-en-1-ylcarbamate

N-(3-(cyclopropylsulfonyl)-4-(4,4,5,5-tetramethyl-1,3,2-dioxaborolan-2-yl)phenyl)-5-methyl-1-(tetrahydro-2H-pyran-2-yl)-1H-pyrazol-3-amine (210 mg, 431 μmol), 2,5-dibromothiophene (130 mg, 538 μmol), and tert-butyl (4-(4,4,5,5-tetramethyl-1,3,2-dioxaborolan-2-yl)cyclohextrien-3-en-1-yl)carbamate (174 mg, 538 μmol) were dissolved in THF (5 mL). Under the protection of nitrogen, 2-dicyclohexylphosphino-2,4,6-triisopropylbiphenyl (25 mg, 53 μmol), chloro(2-dicyclohexylphosphino-2,4,6-triisopropyl-1,1-biphenyl)[2-(2-amino-1,1-biphenyl)]palladium (II) (42 mg, 53 μmol), tris(dibenzylideneacetone)dipalladium (49 mg, 53 μmol) and sodium carbonate (2M, 1.05 mL) were added. The reaction solution was stirred at 100°C for 3 hours. LCMS showed that the reaction was complete. The reaction system was subjected to liquid-liquid extraction (extraction with 10 mL of ethyl acetate three times) and then evaporated to dryness via rotary evaporation. The resultant was purified by column chromatography (silica, ethyl acetate/petroleum ether = 0% to 25%) to obtain tert-butyl (4-(5-(2-(cyclopropylsulfonyl)-4-((5-methyl-1-(tetrahydro-2H-pyran-2-yl)-1H-pyrazol-3-yl)amino)phenyl)thiophen-2-yl)cyclohex-3 -en-1-ylcarbamate as a yellow solid compound (330 mg).

MS m/z (ESI): 639.2 $[M+H]^+$;

Step 2: Synthesis of N-(4-(5-(4-(4-aminocyclohex-1-en-1-yl)thiophen-2-yl)-3-(cyclopropylsulfonyl)phenyl)-5-methyl-1H-pyrazol-3-amine

Tert-butyl (4-(5-(2-(cyclopropylsulfonyl)-4-((5-methyl-1-(tetrahydro-2H-pyran-2-yl)-1H-pyrazol-3-yl)amino)phenyl)thiophen-2-yl)cyclohex-3-en-1-ylcarbamate (105 mg, 188 μmol) was dissolved in anhydrous methanol (6 mL), and then hydrochloric acid/dioxane (12 mL) was added. The reaction solution was stirred at 25°C for 2 hours. LCMS showed that the reaction was complete. The reaction system was evaporated to dryness via rotary evaporation, and then the resultant was purified by column chromatography (silica, dichloromethane/methanol = 0% to 10%) to obtain N-(4-(5-(4-(4-aminocyclohex-1-en-1-yl)thiophen-2-yl)-3-(cyclopropylsulfonyl)phenyl)-5-methyl-1H-pyrazol-3-amine as a yellow solid (223 mg).

MS m/z (ESI): 455.2 $[M+H]^+$;

Step 3: Synthesis of isopropyl (4-(5-(2-(cyclopropylsulfonyl)-4-((5-methyl-1H-pyrazol-3-yl)amino)phenyl)thiophen-2-yl)cyclohex-3-en-1-yl)carbamate

N-(4-(5-(4-(4-aminocyclohex-1-en-1-yl)thiophen-2-yl)-3-(cyclopropylsulfonyl)phenyl)-5-methyl-1H-pyrazol-3-amine (200 mg, 352 μmol) and N,N-diisopropylethylamine (136 mg, 1 mmol) were dissolved in dichloromethane (15 mL), and then isopropyl chloroformate (30 mg, 246 μmol) was slowly added dropwise. The reaction solution was stirred and reacted at 25°C for 2 hours. The completion of the reaction was detected by LCMS. The reaction solution was

concentrated to dryness under reduced pressure and then purified by column chromatography (silica, dichloromethane/methanol = 10/1) to obtain isopropyl (4-(5-(2-(cyclopropylsulfonyl)-4-((5-methyl-1H-pyrazol-3-yl)amino)phenyl)thiophen-2-yl)cyclohex-3-en-1-yl)carbamate as a yellow solid product (120 mg).

MS m/z (ESI): 541.2 $[M+H]^+$;

Step 4: Synthesis of isopropyl trans-(4-(5-(2-(cyclopropylsulfonyl)-4-((5-methyl-1H-pyrazol-3-yl)amino)phenyl)thiophen-2-yl)cyclohexyl)carbamate and isopropyl cis-(4-(5-(2-(cyclopropylsulfonyl)-4-((5-methyl-1H-pyrazol-3-yl)amino)phenyl)thiophen-2-yl)cyclohexyl) carbamate

Compound isopropyl (4-(5-(2-(cyclopropylsulfonyl)-4-((5-methyl-1H-pyrazol-3-yl)amino) phenyl)thiophen-2-yl)cyclohex-3-en-1-yl)carbamate (100 mg, 185 μmol) and wet Pd/C (36 mg, 1.11 mmol) were dissolved in methanol (10 mL). The reaction solution was stirred and reacted at 25°C for 18 hours. The completion of the reaction was detected by LCMS. The reaction solution was concentrated to dryness under reduced pressure and purified by preparative liquid chromatography (Boston Prime C18 column, 5 μm silica, 30 mm in diameter, 150 mm in length; using decreasingly polar mixtures of water (containing 0.05% aqueous ammonia) and acetonitrile (35% to 65%) as eluents) to obtain a pair of enantiomers (retention time on chromatogram: 4.590 minutes, named as Compound of Example 89) and another pair of enantiomers (retention time on chromatogram: 4.189 minutes, named as Compound of Example 89-1).

Example 89: MS m/z (ESI): 543.2 $[M+H]^+$;

$^1$H NMR (400MHz, DMSO-d6) δ = 11.84 (s, 1H), 8.96 (s, 1H), 8.06 (s, 1H), 7.70-7.53 (m, 1H), 7.31 (d, J = 8.5 Hz, 1H), 7.04 (d, J = 3.5 Hz, 1H), 6.99 (br d, J = 7.6 Hz, 1H), 6.84 (d, J = 3.5 Hz, 1H), 5.63 (s, 1H), 4.77-4.60 (m, 1H), 3.30-3.22 (m, 1H), 2.75 (br t, J = 11.7 Hz, 1H), 2.29-2.22 (m, 1H), 2.20 (s, 3H), 2.03 (br d, J = 12.3 Hz, 2H), 1.89 (br d, J = 10.6 Hz, 2H), 1.54-1.41 (m, 2H), 1.38-1.29 (m, 2H), 1.21-1.16 (d, J = 6.4 Hz, 6H), 0.95-0.84 (m, 4H).

Example 89-1: MS m/z (ESI): 543.2 $[M+H]^+$;

$^1$H NMR (400MHz, DMSO-d6) δ = 11.84 (br s, 1H), 8.96 (s, 1H), 8.07 (br s, 1H), 7.61 (br d, J = 8.0 Hz, 1H), 7.32 (d, J = 8.4 Hz, 1H), 7.13 (br d, J = 5.0 Hz, 1H), 7.08-7.03 (m, 1H), 6.89-6.83 (m, 1H), 5.64 (s, 1H), 4.75-4.60 (m, 1H), 3.65-3.55 (m, 1H), 2.95-2.85 (m, 1H), 2.28-2.22 (m, 1H), 2.20 (s, 3H), 1.94-1.81 (m, 2H), 1.80-1.72 (m, 2H), 1.71-1.55 (m, 4H), 1.17 (d, J = 6.0 Hz, 6H), 0.94-0.84 (m, 4H).

Example 90: Synthesis of isopropyl trans-(4-(5-(2-(cyclopropylsulfonyl)-4-((5-methyl-1H-pyrazol-3-yl)amino)phenyl)-1H-imidazol-2-yl)cyclohexylcarbamate

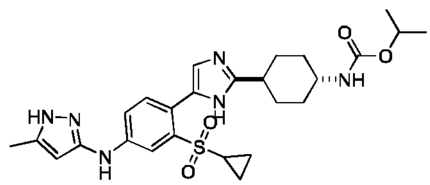

Synthetic Method

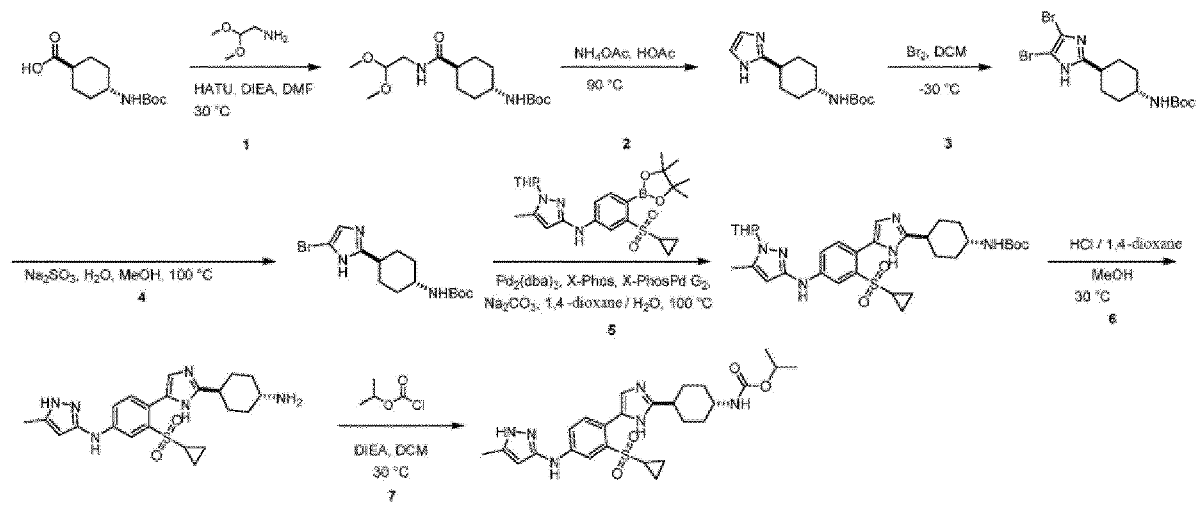


Step 1: Synthesis of tert-butyl trans-(4-(((2,2-dimethoxyethyl)carbamoyl) cyclohexyl)carbamate

Trans-(4-((tert-butoxycarbonyl)amino)cyclohexanecarboxylic acid (4.88 g, 20.1 mmol) was dissolved in N,N-dimethylformamide (50 mL), and O-(7-azabenzotriazol-1-yl)-N,N,N,N-tetramethyluronium hexafluorophosphate (11.4 g, 30.1 mmol), 2,2-dimethoxyethylamine (6.33 g, 60.2 mmol) and triethylamine (8.38 mL, 60.2 mmol) were added. The reaction solution was stirred and reacted at 30°C for 1 hour. The completion of the reaction was detected by LCMS. The reaction solution was concentrated to dryness under reduced pressure. Methanol (50 mL) and water (450 mL) were added, and a white solid precipitated. Thereafter, the reaction solution was further stirred at room temperature for 30 minutes. The resultant was filtered and then washed with 150 mL of water (50 mL × 3) to obtain tert-butyl trans-(4-(((2,2-dimethoxyethyl)carbamoyl)cyclohexyl)carbamate as a white solid compound (5.1 g).
MS m/z (ESI): 353.3 $[M+H]^+$;

Step 2: Synthesis of tert-butyl trans-(4-(1H-imidazol-2-yl)cyclohexyl)carbamate

Tert-butyl trans-(4-(((2,2-dimethoxyethyl)carbamoyl)cyclohexyl)carbamate (2.60 g, 7.87 mmol) was dissolved in glacial acetic acid (40 mL), and ammonium acetate (30.3 g, 393 μmol) was added. The reaction solution was stirred and reacted at 90°C for 16 hours. The completion of the reaction was detected by LCMS. The reaction solution was concentrated to dryness under reduced pressure. The resultant was purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (12% to 52%) as eluents) to obtain tert-butyl trans-((4-(1H-imidazol-2-yl)cyclohexyl)carbamate as a white solid compound (0.37 g).
MS m/z (ESI): 266.1 $[M+H]^+$;

Step 3: Synthesis of tert-butyl trans-(4-(4,5-dibromo-1H-imidazol-2-yl) cyclohexyl)carbamate

Tert-butyl trans-(4-(1H-imidazol-2-yl)cyclohexyl)carbamate (266 mg, 501 μmol) was dissolved in anhydrous dichloromethane (10 mL), and liquid bromine (25.8 μl, 501 μmol) was slowly added at -30°C. The reaction solution was stirred and reacted at -30°C for 10 hours. The completion of the reaction was detected by LCMS. A saturated sodium sulfite solution (10 mL) was added into the reaction solution to quench the reaction. The reaction solution was extracted with DCM (30 mL) three times. The organic phases were combined, dried over anhydrous sodium sulfate, filtered, and concentrated under reduced pressure to obtain tert-butyl trans-(4-(4,5-dibromo-1H-imidazol-2-yl)cyclohexyl)carbamate as a white solid compound (150 mg, crude product).
MS m/z (ESI): 422.0; 424.0; 426.0 $[M+H]^+$;

Step 4: Synthesis of tert-butyl trans-(4-(5-bromo-1H-imidazol-2-yl)cyclohexyl)carbamate

Tert-butyl trans-(4-(4,5-dibromo-1H-imidazol-2-yl)cyclohexyl)carbamate (100 mg, 118 μmol) was dissolved in anhydrous methanol (5 mL) and water (5 mL), and sodium sulfite (1.49 g, 11.8 mmol) was added. The reaction solution was stirred and reacted at 100°C for 48 hours. The completion of the reaction was detected by LCMS. The reaction solution was concentrated to dryness under reduced pressure, added with water (10 mL), and extracted with DCM (30

mL) three times. The organic phases were combined, dried over anhydrous sodium sulfate, filtered, and concentrated under reduced pressure to obtain tert-butyl trans-(4-(5-bromo-1H-imidazol-2-yl)cyclohexyl)carbamate as a white solid compound (50.0 mg, crude product).

MS m/z (ESI): 344.1; 346.2 $[M+H]^+$;

Step 5: Synthesis of tert-butyl trans-(4-(5-(2-(cyclopropylsulfonyl)-4-((5-methyl-1-(tetrahydro-2H-pyran-2-yl)-1H-pyrazol-3-amino)phenyl)-1H-imidazol-2-yl) cyclohexyl)carbamate

Tert-butyl trans-(4-(5-bromo-1H-imidazol-2-yl)cyclohexyl)carbamate (50.0 mg, 145 μmol) and N-(3-(cyclopropylsulfonyl)-4-(4,4,5,5-tetramethyl-1,3,2-dioxaborolan-2-yl)phenyl)-5-methyl-1-(tetrahydro-2H-pyran-2-yl)-1H-pyrazol-3-amine (70.8 mg, 145 μmol) were dissolved in tetrahydrofuran (5 mL). Thereafter, tris(dibenzylideneacetone)dipalladium (13.3 mg, 14.5 μmol), sodium carbonate (2M, 1 mL), chloro(2-dicyclohexylphosphino-2,4,6-triisopropyl-1,1-biphenyl)[2-(2-amino-1,1-biphenyl)]palladium (II) (11.4 mg, 14.5 μmol) and 2-dicyclohexylphosphino-2,4,6-triisopropylbiphenyl (6.92 mg, 14.5 μmol) were added under the condition where nitrogen gas was introduced. The reaction solution was stirred and reacted at 100°C for 16 hours. The completion of the reaction was detected by LCMS. The reaction solution was concentrated to dryness under reduced pressure and purified by column chromatography (silica, dichloromethane:methanol = 15:1) to obtain tert-butyl trans-(4-(5-(2-(cyclopropylsulfonyl)-4-((5-methyl-1-(tetrahydro-2H-pyran-2-yl)-1H-pyrazol-3-amino)phenyl)-1H-imidazol-2-yl)cyclohexyl)carbamate as a yellow solid compound (40.0 mg).

MS m/z (ESI): 625.3 $[M+H]^+$;

Step 6: Synthesis of N-[4-[2-(4-aminocyclohexyl)-1H-imidazol-5-yl]-3-cyclopropylsulfonyl-phenyl]-5-methyl-1H-pyrazol-3-amine

Tert-butyl trans-(4-(5-(2-(cyclopropylsulfonyl)-4-((5-methyl-1-(tetrahydro-2H-pyran-2-yl)-1H-pyrazol-3-amino)phenyl)-1H-imidazol-2-yl)cyclohexyl)carbamate (40.0 mg, 64.0 μmol) was dissolved in methanol (1 mL), and hydrochloric acid/dioxane (4M, 800 μL) was added. The reaction solution was stirred and reacted at 30°C for 2 hours. The completion of the reaction was detected by LCMS. The reaction solution was concentrated to dryness under reduced pressure to obtain N-[4-[2-(4-aminocyclohexyl)-1H-imidazol-5-yl]-3-cyclopropylsulfonyl-phenyl]-5-methyl-1H-pyrazol-3-amine as a yellow solid (30.0 mg).

MS m/z (ESI): 441.1 $[M+H]^+$;

Step 7: Synthesis of isopropyl trans-(4-(5-(2-(cyclopropylsulfonyl)-4-((5-methyl-1H-pyrazol-3-yl)amino)phenyl)-1H-imidazol-2-yl)cyclohexylcarbamate

N-[4-[2-(4-aminocyclohexyl)-1H-imidazol-5-yl]-3-cyclopropylsulfonyl-phenyl]-5-methyl -1H-pyrazol-3-amine (24.0 mg, 54.5 μmol) was dissolved in dichloromethane (1 mL), and then a solution of isopropyl chloroformate (4.01 mg, 32.7 μmol) and N,N-diisopropylethylamine (35.2 mg, 272 μmol) in dichloromethane (2 mL) was added. The reaction solution was stirred and reacted at 30°C for 1 hour. The completion of the reaction was detected by LCMS. The reaction solution was filtered and concentrated to dryness under reduced pressure, and purified by preparative liquid chromatography (YMC-Actus Triart C18 column: 5 μm silica, 30 mm in diameter, 150 mm in length; using decreasingly polar mixtures of water (containing 0.225% formic acid) and acetonitrile (20% to 40%) as eluents) to obtain isopropyl trans-(4-(5-(2-(cyclopropylsulfonyl)-4-((5-methyl-1H-pyrazol-3-yl)amino)phenyl)-1H-imidazol-2-yl )cyclohexylcarbamate as a white solid compound (2.11 mg).

MS m/z (ESI): 527.0 $[M+H]^+$;

$^1$H NMR (400MHz, DMSO-d6) δ 12.25-11.05 (m, 2H), 8.88-8.65 (m, 1H), 8.76 (br s, 1H), 8.15 (s, 1H), 8.21-8.11 (m, 1H), 7.97 (br s, 1H), 8.02-7.90 (m, 1H), 7.58 (br d, J = 7.0 Hz, 1H), 7.46 (br s, 1H), 7.51-7.36 (m, 1H), 7.19 (br s, 1H), 6.97 (br d, J = 7.9 Hz, 1H), 5.60 (s, 1H), 4.73 (td, J = 6.2, 12.5 Hz, 1H), 3.29-3.28 (m, 1H), 2.99 (br s, 1H), 2.70-2.52 (m, 2H), 2.19 (s, 3H), 2.09-1.80 (m, 4H), 1.61-1.45 (m, 2H), 1.37-1.20 (m, 2H), 1.16 - 1.05 (m, 6H), 1.04-0.79 (m, 4H).

Examples 91 and 91-1: Synthesis of isopropyl trans-(4-(2-(2-(cyclopropylsulfonyl)-4-((5-methyl-1H-pyrazol-3-yl)amino)phenyl)thiazol-5-yl)cyclohexyl)carbamate and isopropyl cis-(4-(2-(2-(cyclopropylsulfonyl)-4-((5-methyl-1H-pyrazol-3-yl)amino)phenyl)thiazol-5-yl) cyclohexyl)carbamate

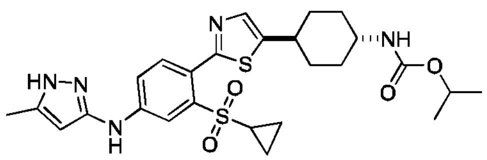 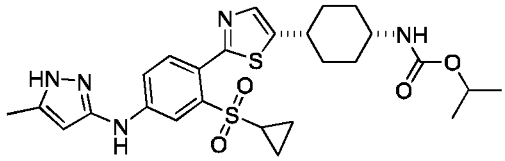

Synthetic Method

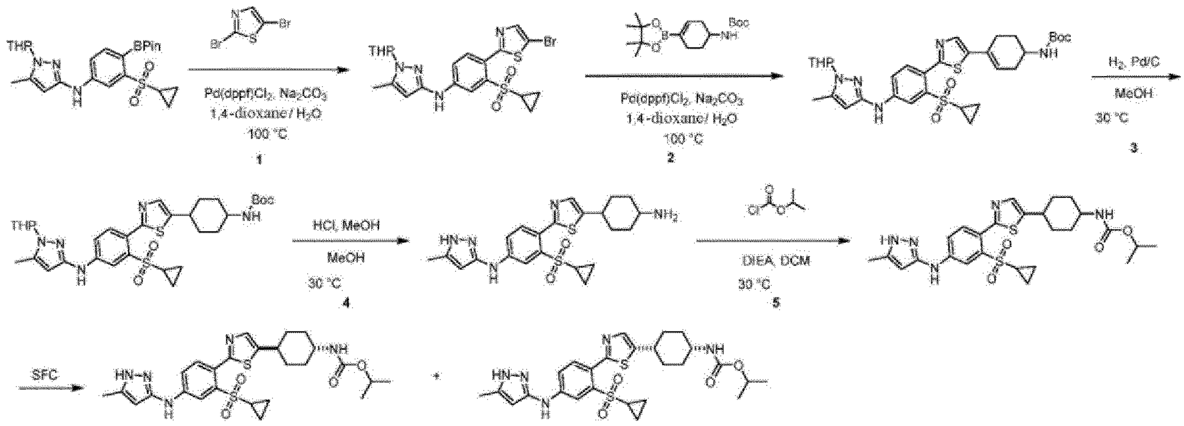


Step 1: Synthesis of N-(4-(5-bromothiazol-2-yl)-3-(cyclopropylsulfonyl)phenyl)-5-methyl-1-(tetrahydro-2H-pyran-2-yl)-1H-pyrazol-3-amine

N-[3-cyclopropylsulfonyl-4-(4,4,5,5-tetramethyl-1,3,2-dioxaborolan-2-yl)phenyl]-5-methyl-1-tetrahydropyran-2-yl-pyrazol-3-amine (1.00 g, 2.05 mmol) and 2,5-dibromothiazole (1.25 g, 5.13 mmol) were dissolved in dioxane (30 mL). Thereafter, [1,1-bis(diphenylphosphino)ferrocene]dichloropalladium - dichloromethane (167 mg, 205 μmol) and sodium carbonate (2M, 6 mL) were added under the condition where nitrogen gas was introduced. The reaction solution was stirred and reacted at 100°C for 16 hours. The completion of the reaction was detected by LCMS. The reaction solution was concentrated to dryness under reduced pressure and purified by column chromatography (silica, petroleum ether:tetrahydrofuran = 2:1) to obtain N-(4-(5-bromothiazol-2-yl)-3-(cyclopropylsulfonyl) phenyl)-5-methyl-1-(tetrahydro-2H-pyran-2-yl)-1H-pyrazol-3-amine as a yellow solid compound (220 mg).

MS m/z (ESI):523.0; 525.0 $[M+H]^+$;

Step 2: Synthesis of tert-butyl (4-(2-(2-(cyclopropylsulfonyl)-4-((5-methyl-1-(tetrahydro-2H-pyran-2-yl)-1H-pyrazol-3-yl)amino)phenyl)thiazol-5-yl)cyclohex-3-en-1-yl)carbamate

N-(4-(5-bromothiazol-2-yl)-3-(cyclopropylsulfonyl)phenyl)-5-methyl-1-(tetrahydro-2H-pyran-2-yl)-1H-pyrazol-3-amine (150 mg, 286 μmol) and tert-butyl (4-(4,4,5,5-tetramethyl-1,3,2-dioxaborolan-2-yl)cyclohex-3-en-1-yl)carbamate (111 mg, 344 μmol) were dissolved in dioxane (5 mL). Thereafter, [1,1-bis(diphenylphosphino)ferrocene]dichloropalladium - dichloromethane (23.4 mg, 28.7 μmol) and sodium carbonate (2M, 1 mL) were added under the condition where nitrogen gas was introduced. The reaction solution was stirred and reacted at 100°C for 16 hours. The completion of the reaction was detected by LCMS. The reaction solution was concentrated to dryness under reduced pressure and purified by thin-layer chromatography (silica, petroleum ether:tetrahydrofuran = 1:1) to obtain tert-butyl (4-(2-(2-(cyclopropylsulfonyl)-4-((5-methyl-1-(tetrahydro-2H-pyran-2-yl)-1H-pyrazol-3-yl) amino)phenyl)thiazol-5-yl)cyclohex-3-en-1-yl)carbamate as a yellow solid compound (126 mg).

MS m/z (ESI):640.5 $[M+H]^+$;

Step 3: Synthesis of tert-butyl (4-(2-(2-(cyclopropylsulfonyl)-4-((5-methyl-1-(tetrahydro-2H-pyran-2-yl)-1H-pyrazol-3-yl)amino)phenyl)thiazol-5-yl)cyclohexyl)carbamate

Tert-butyl (4-(2-(2-(cyclopropylsulfonyl)-4-((5-methyl-1-(tetrahydro-2H-pyran-2-yl)-1H-pyrazol-3-yl)amino)phenyl)thiazol-5-yl)cyclohex-3-en-1-yl)carbamate (100 mg, 156 μmol) was dissolved in methanol (4 mL), and then

Pd/C (20 mg, 156 μmol) was added into the reaction solution. The reaction solution was purged three times with hydrogen gas. The reaction solution was pressurized by using a hydrogen balloon (15 psi), and was stirred and reacted at 30°C for 16 hours. The completion of the reaction was detected by LCMS. The resultant was filtered and concentrated to dryness under reduced pressure to obtain tert-butyl (4-(2-(2-(cyclopropylsulfonyl)-4-((5-methyl-1-(tetrahydro-2H-pyran-2-yl)-1H-pyrazol-3-yl) amino)phenyl)thiazol-5-yl)cyclohexyl)carbamate as a yellow solid product (75.0 mg).

MS m/z (ESI): 642.3 $[M+H]^+$;

Step 4: Synthesis of N-(4-(5-(4-aminocyclohexyl)thiazol-2-yl)-3-(cyclopropylsulfonyl) phenyl)-5-methyl-1H-pyrazol-3-amine

Tert-butyl (4-(2-(2-(cyclopropylsulfonyl)-4-((5-methyl-1-(tetrahydro-2H-pyran-2-yl)-1H-pyrazol-3-yl)amino)phenyl)thiazol-5-yl)cyclohexyl)carbamate (70.0 mg, 109 μmol) was dissolved in methanol (3 mL), and then hydrochloric acid/methanol (4M, 600 μL) was added into the reaction solution. The reaction solution was stirred and reacted at 30°C for 16 hours. The completion of the reaction was detected by LCMS. The resultant was filtered and concentrated to dryness under reduced pressure to obtain N-(4-(5-(4-aminocyclohexyl)thiazol-2-yl)-3-(cyclopropylsulfonyl)phenyl)-5-methyl-1H-pyrazol-3-amine as a yellow oily product (53.0 mg).

MS m/z (ESI):458.1 $[M+H]^+$;

Step 5: Synthesis of isopropyl (4-(2-(2-(2-(cyclopropylsulfonyl))-4-((5-methyl-1H-pyrazol-3-yl)amino)phenyl)thiazol-5-yl)cyclohexyl)carbamate

N-(4-(5-(4-aminocyclohexyl)thiazol-2-yl)-3-(cyclopropylsulfonyl)phenyl)-5-methyl-1H-pyrazol-3-amine (53.0 mg, 116 μmol) was dissolved in dichloromethane (2 mL), and then a solution of isopropyl chloroformate (9.94 mg, 81.1 μmol) and N,N-diisopropylethylamine (74.8 mg, 579 μmol) in dichloromethane (2 mL) was added. The reaction solution was stirred and reacted at 30°C for 16 hours. The completion of the reaction was detected by LCMS. The resultant was filtered, concentrated to dryness under reduced pressure, and purified by preparative liquid chromatography (Phenomenex Gemini C18 column: 5 μm silica, 30 mm in diameter, 150 mm in length; using decreasingly polar mixtures of water (containing 0.225% formic acid) and acetonitrile (40% to 60%) as eluents) to obtain isopropyl (4-(2-(2-(2-(cyclopropylsulfonyl))-4-((5-methyl-1H-pyrazol-3-yl)amino)phenyl)thiazol-5-yl) cyclohexyl)carbamate as a white solid compound (23.0 mg).

MS m/z (ESI):544.2 $[M+H]^+$;

Step 6: Synthesis of isopropyl trans-(4-(2-(2-(cyclopropylsulfonyl)-4-((5-methyl-1H-pyrazol-3-yl)amino)phenyl)thiazol-5-yl)cyclohexyl)carbamate and isopropyl cis-(4-(2-(2-(cyclopropylsulfonyl)-4-((5-methyl-1H-pyrazol-3-yl)amino)phenyl)thiazol-5-yl)cyclohexyl) carbamate

Isopropyl (4-(2-(2-(2-(cyclopropylsulfonyl))-4-((5-methyl-1H-pyrazol-3-yl)amino)phenyl) thiazol-5-yl)cyclohexyl)carbamate (23.0mg) as a racemic compound was separated by supercritical fluid chromatography (DAICEL CHIRALPAK AS column, 10 μm silica, 30 mm in diameter, 250 mm in length) (using polar mixtures of water (containing 0.1% aqueous ammonia) and ethanol (35% to 35%) as eluents) to obtain a pair of enantiomers (4.52 mg, retention time on chromatogram: 4.396 minutes, named as Compound of Example 91) and another pair of enantiomers (5.17 mg, retention time on chromatogram: 4.092 minutes, named as Compound of Example 91-1).

MS m/z (ESI): 544.0 $[M+H]^+$;

$^1$H NMR (400MHz, DMSO-d6) δ 11.90 (s, 1H), 9.12 (s, 1H), 8.01 (d, J = 2.3 Hz, 1H), 7.69 (dd, J = 2.3, 8.5 Hz, 1H), 7.62 (s, 1H), 7.48 (d, J = 8.5 Hz, 1H), 7.18 (br d, J = 6.8 Hz, 1H), 5.65 (s, 1H), 4.77-4.71 (m, 1H), 3.75-3.57 (m, 2H), 3.09-2.94 (m, 1H), 2.20 (s, 3H), 2.05-1.94 (m, 1H), 1.92-1.73 (m, 4H), 1.73-1.55 (m, 4H), 1.17-1.06 (m, 6H), 1.11-0.99 (m, 4H).

$^1$H NMR (400MHz, DMSO-d6) δ 11.90 (s, 1H), 9.12 (s, 1H), 8.01 (d, J = 1.8 Hz, 1H), 7.71-7.65 (m, 1H), 7.61 (s, 1H), 7.47 (d, J = 8.5 Hz, 1H), 7.01 (br d, J = 7.8 Hz, 1H), 5.64 (s, 1H), 4.77-4.71 (m, 1H), 3.74-3.62 (m, 1H), 3.26 (s, 1H), 2.86 (br t, J = 12.2 Hz, 1H), 2.20 (s, 3H), 2.05 (br d, J = 11.3 Hz, 2H), 1.90 (br d, J = 10.5 Hz, 2H), 1.60-1.43 (m, 2H), 1.40-1.27 (m, 2H), 1.16-1.05 (m, 6H), 1.11-0.99 (m, 4H).

Example 92: Synthesis of isopropyl trans-N-[4-[5-[4-[(5-methyl-1H-pyrazol-3-yl)amino]-2-(oxetan-3-ylsulfonyl)phenyl]thiazol-2-yl]cyclohexyl]carbamate

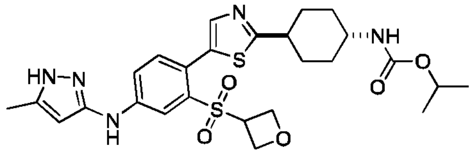

Synthetic Method

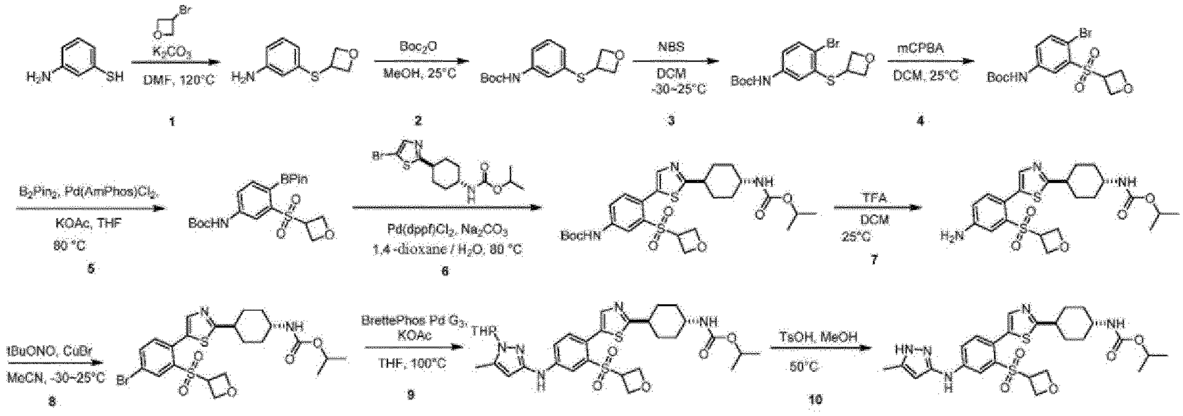


Step 1: Synthesis of 3-(oxetan-3-ylsulfanyl)aniline

3-Aminothiophenol (1 g, 7.99 mmol) was dissolved in N,N-dimethylformamide (20 mL), and potassium carbonate (1.10 g, 7.99 mmol) and 3-bromooxetane (1.42 g, 10.4 mmol) were added. The reaction solution was stirred and reacted at 120°C for 24 hours. The completion of the reaction was detected by LCMS. The reaction solution was concentrated to dryness under reduced pressure and then purified by column chromatography (silica, petroleum ether:ethyl acetate = 1:1) to obtain 3-(oxetan-3-ylsulfanyl)aniline (400 mg).

MS m/z (ESI): 182.1 $[M+H]^+$;

Step 2: Synthesis of tert-butyl N-(3-cyclopropylsulfanylphenyl)carbamate

3-(Oxetan-3-ylsulfanyl)aniline (400 mg, 2.21 mmol) was dissolved in methanol (5 mL), and di-tert-butyl dicarbonate (4.82 g, 22.1 mmol, 5.07 mL) was added. The reaction solution was stirred and reacted at 25°C for 16 hours. The completion of the reaction was detected by LCMS. Aqueous ammonia (20 mL) was added into the reaction solution. The reaction solution was stirred and reacted at 0ºC for 2 hours. The reaction solution was extracted with dichloromethane (20 mL) three times. The organic phase was concentrated to dryness under reduced pressure and then purified by column chromatography (silica, petroleum ether:ethyl acetate = 20:1) to obtain tert-butyl N-(3-cyclopropylsulfanylphenyl)carbamate (400 mg).

MS m/z (ESI):226.0 $[M+H\text{-}t\text{-}Bu]^+$;

Step 3: Synthesis of tert-butyl N-[4-bromo-3-(oxetan-3-ylsulfanyl)phenyl]carbamate

Tert-butyl N-(3-cyclopropylsulfanylphenyl)carbamate (350 mg, 1.24 mmol) was dissolved in dichloromethane (5 mL), and then a solution of N-bromosuccinimide (243.54 mg, 1.37 mmol) in dichloromethane (5 mL) was slowly added dropwise into the reaction solution at -30°C. The reaction solution was stirred and reacted at 25°C for 16 hours. The completion of the reaction was detected by LCMS. The reaction solution was concentrated to dryness under reduced pressure and purified by column chromatography (silica, petroleum ether:ethyl acetate = 10:1) to obtain tert-butyl N-[4-bromo-3-(oxetan-3-ylsulfanyl)phenyl]carbamate as a white solid (250 mg).

MS m/z (ESI): 304.0, 306.0 $[M+H\text{-}t\text{-}Bu]^+$;

$^1H$ NMR (400MHz, DMSO-d6) $\delta$ = 9.53 (s, 1H), 7.48 (d, J = 8.8 Hz, 1H), 7.30 (dd, J = 2.3, 8.5 Hz, 1H), 7.05 (d, J = 2.0 Hz, 1H), 5.09 (t, J = 6.7 Hz, 2H), 4.57 - 4.45 (m, 3H), 1.48 (s, 9H).

Step 4: Synthesis of tert-butyl N-[4-bromo-3-(oxetan-3-ylsulfonyl)phenyl]carbamate

Tert-butyl N-[4-bromo-3-(oxetan-3-ylsulfanyl)phenyl]carbamate (250 mg, 694 μmol) was dissolved in dichloromethane (5 mL), and m-chloroperoxybenzoic acid (479 mg, 2.78 mmol) was added. The reaction solution was stirred and reacted at 25°C for 16 hours. The completion of the reaction was detected by LCMS. The reaction solution was concentrated to dryness under reduced pressure, added with saturated sodium carbonate solution (30 mL), and extracted with 90 mL of dichloromethane (30 mL × 3). The organic phase was dried over anhydrous sodium sulfate and filtered. The filtrate was concentrated to dryness under reduced pressure and then purified by thin-layer chromatography (silica, petroleum ether:ethyl acetate = 3:1) to obtain tert-butyl N-[4-bromo-3-(oxetan-3-ylsulfonyl)phenyl]carbamate as the product (150 mg).

MS m/z (ESI): 336.0, 338.0 $[M+H\text{-}t\text{-}Bu]^+$;

Step 5: Synthesis of tert-butyl N-[3-(oxetan-3-ylsulfonyl)-4-(4,4,5,5-tetramethyl-1,3,2-dioxaborolan-2-yl)phenyl]carbamate

Tert-butyl N-[4-bromo-3-(oxetan-3-ylsulfonyl)phenyl]carbamate (150 mg, 382 μmol) and bis(pinacolato)diboron (388 mg, 1.53 mmol) were dissolved in tetrahydrofuran (2 mL). Under the protection of nitrogen, dichlorobis[di-tert-butyl-(4-dimethylaminophenyl) phosphine]palladium (II) (27.1 mg, 38.2 μmol) and potassium acetate (75.1 mg, 765 μmol) were added. The reaction solution was stirred and reacted at 80°C for 16 hours. The completion of the reaction was detected by LCMS. The reaction solution was concentrated to dryness under reduced pressure and purified by thin-layer chromatography (silica, petroleum ether:ethyl acetate = 3:1) to obtain tert-butyl N-[3-(oxetan-3-ylsulfonyl)-4-(4,4,5,5-tetramethyl-1,3,2-dioxaborolan-2-yl)phenyl]carbamate as a yellow substance (130 mg).

MS m/z (ESI): 438.1 $[M+H]^+$;

Step 6: Synthesis of isopropyl trans-N-[4-[5-[4-(tert-butoxycarbonylamino)-2-(oxetan-3-ylsulfonyl)phenyl]thiazol-2-yl]cyclohexyl]carbamate

Tert-butyl N-[3-(oxetan-3-ylsulfonyl)-4-(4,4,5,5-tetramethyl-1,3,2-dioxaborolan-2-yl) phenyl]carbamate (127 mg, 288 μmol) and isopropyl trans-N-[4-(5-bromothiazol-2-yl)cyclohexyl]carbamate (100 mg, 288 μmol) were dissolved in water (0.5 mL) and dioxane (5 mL). Thereafter, [1,1-bis(diphenylphosphino)ferrocene]dichloropalladium - dichloromethane (23.5 mg, 28.8 μmol) and sodium carbonate (61.1 mg, 576 μmol) were added under the condition where nitrogen gas was introduced. The reaction solution was stirred and reacted at 80°C for 16 hours. The completion of the reaction was detected by LCMS. The reaction solution was concentrated to dryness under reduced pressure and purified by thin-layer chromatography (silica, petroleum ether:tetrahydrofuran = 3:1) to obtain isopropyl trans-N-[4-[5-[4-(tert-butoxycarbonylamino)-2-(oxetan-3-ylsulfonyl)phenyl]thiazol-2-yl]cyclohexyl] carbamate as a white substance (80 mg).

MS m/z (ESI): 580.2 $[M+H]^+$;

Step 7: Synthesis of isopropyl trans-N-[4-[5-[4-amino-2-(oxetan-3-ylsulfonyl)phenyl] thiazol-2-yl]cyclohexyl]carbamate

Isopropyl trans-N-[4-[5-[4-(tert-butoxycarbonylamino)-2-(oxetan-3-ylsulfonyl)phenyl] thiazol-2-yl]cyclohexyl]carbamate (80mg) was dissolved in dichloromethane (4 mL), and trifluoroacetic acid (616 mg, 5.40 mmol, 400 μL) was added. The reaction solution was stirred and reacted at 25°C for 3 hours. The completion of the reaction was detected by LCMS. The reaction solution was concentrated to dryness under reduced pressure. The reaction solution was added with 5 mL of saturated sodium carbonate solution and 9 mL of dichloromethane (3 mL × 3). The organic phase was dried over anhydrous sodium sulfate and filtered. The filtrate was concentrated to dryness under reduced pressure to obtain isopropyl trans-N-[4-[5-[4-amino-2-(oxetan-3-ylsulfonyl)phenyl]thiazol-2-yl]cyclohexyl]carbamate as a white solid compound (60.0 mg).

MS m/z (ESI): 480.2 $[M+H]^+$;

Step 8: Synthesis of isopropyl trans-N-[4-[5-[4-bromo-2-(oxetan-3-ylsulfonyl) phenyl]thiazol-2-yl]cyclohexyl]carbamate

Isopropyl trans-N-[4-[5-[4-amino-2-(oxetan-3-ylsulfonyl)phenyl]thiazol-2-yl]cyclohexyl] carbamate (50 mg, 104 μmol) was dissolved in acetonitrile (2 mL), and tert-butyl nitrite (32.3 mg, 313 μmol, 37 μL) was added at -30°C. The reaction solution was stirred and reacted at 25°C for 1 hour. Cuprous bromide (37.39 mg, 260.63 μmol) was added into the reaction solution at -30°C. The reaction solution was stirred and reacted at 25°C for 15 hours. The completion of the reaction was detected by LCMS. The reaction solution was concentrated to dryness under reduced pressure. The reaction solution was added with 50 mL of saturated ammonium chloride solution and 90 mL of dichloromethane (30 mL × 3). The organic phase was dried over anhydrous sodium sulfate and filtered. The filtrate was concentrated to

dryness under reduced pressure and purified by thin-layer chromatography (silica, petroleum ether:tetrahydrofuran = 2:1) to obtain isopropyl trans-N-[4-[5-[4-bromo-2-(oxetan-3-ylsulfonyl) phenyl]thiazol-2-yl]cyclohexyl]carbamate as a yellow substance (30.0 mg).

MS m/z (ESI): 543.1, 545.1 $[M+H]^+$;

Step 9: Synthesis of isopropyl trans-N-[4-[5-[4-[(5-methyl-1-tetrahydropyran-2-yl-pyrazol-3-yl)amino]-2-(oxetan-3-ylsulfonyl)phenyl]thiazol-2-yl]cyclohexyl]carbamate

Isopropyl trans-N-[4-[5-[4-bromo-2-(oxetan-3-ylsulfonyl)phenyl]thiazol-2-yl]cyclohexyl] carbamate (25.0 mg, 46.0 μmol) and 5-methyl-1-tetrahydropyran-2-yl-pyrazol-3-amine (9.17 mg, 50.6 μmol) were dissolved in tetrahydrofuran (2 mL), and methanesulfonato(2-dicyclohexylphosphino)-3,6-dimethoxy-2,4,6-triisopropyl-1,1-biphenyl)(2 -amino-1,1-biphenyl-2-yl)palladium (II) (4.17 mg, 4.60 μmol) and potassium acetate (9.03 mg, 92.0 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by thin-layer chromatography (silica, petroleum ether:tetrahydrofuran = 2:1) to obtain isopropyl trans-N-[4-[5-[4-[(5-methyl-1-tetrahydropyran-2-yl-pyrazol-3-yl)amino]-2-(oxetan-3-ylsulfonyl)phenyl]thiazol-2-yl]cyclohexyl]carbamate as a white solid compound (25.0 mg).

MS m/z (ESI):644.4 $[M+H]^+$;

Step 10: Synthesis of isopropyl trans-N-[4-[5-[4-[(5-methyl-1H-pyrazol-3-yl)amino]-2-(oxetan-3-ylsulfonyl)phenyl]thiazol-2-yl]cyclohexyl]carbamate

Isopropyl trans-N-[4-[5-[4-[(5-methyl-1-tetrahydropyran-2-yl-pyrazol-3-yl)amino]-2-(oxetan-3-ylsulfonyl)phenyl]thiazol-2-yl]cyclohexyl]carbamate (25.0 mg, 38.8 μmol) was dissolved in methanol (1 mL), and p-toluenesulfonic acid monohydrate (33.4 mg, 194 μmol) was added. The reaction solution was stirred and reacted at 50°C for 1 hour. The completion of the reaction was detected by LCMS. The reaction solution was concentrated to dryness under reduced pressure. The reaction solution was added with 9 mL of dichloromethane (3 mL × 3) and 5 mL of a saturated sodium bicarbonate solution. The organic phase was dried over anhydrous sodium sulfate and filtered. The filtrate was concentrated to dryness under reduced pressure and purified by thin-layer chromatography (silica, dichloromethane/methanol = 15/1) to obtain the compound isopropyl trans-N-[4-[5-[4-[(5-methyl-1H-pyrazol-3-yl)amino]-2-(oxetan-3-ylsulfonyl)phenyl]thiazol-2-yl]cyclohexyl]carbamate (2.36 mg).

MS m/z (ESI): 560.0 $[M+H]^+$;

$^1H$ NMR (400MHz, DMSO-d6) δ = 11.92 (br s, 1H), 9.14 (s, 1H), 8.19 (br s, 1H), 7.63 (d, J = 5.4 Hz, 2H), 7.33 (d, J = 8.3 Hz, 1H), 7.04 (br d, J = 8.0 Hz, 1H), 5.65 (s, 1H), 4.78-4.70 (m, 1H), 4.67-4.60 (m, 2H), 4.55-4.48 (m, 3H), 3.33 (br s, 1H), 2.92 (s, 1H), 2.21 (s, 3H), 2.13 (br d, J = 11.8 Hz, 2H), 1.92 (br d, J = 10.8 Hz, 2H), 1.56 (br dd, J = 2.3, 12.5 Hz, 2H), 1.40-1.28 (m, 2H), 1.17 (d, J = 6.3 Hz, 6H).

Example 93: Synthesis of isopropyl trans-(4-(5-(2-(cyclopropylsulfonyl)-4-((6-methoxypyridin-3-yl)amino)phenyl)thiazol-2-yl)cyclohexyl)carbamate

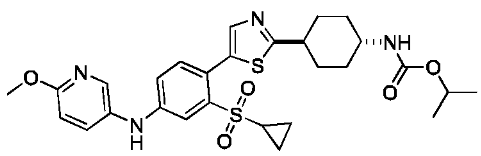

Synthetic Method

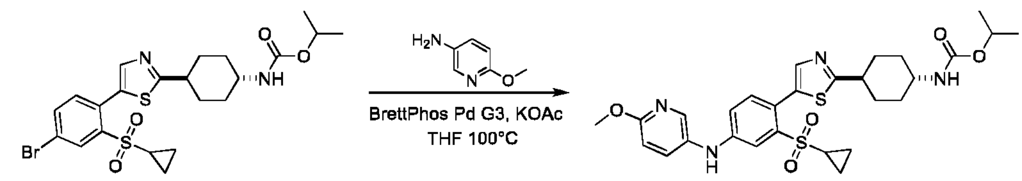


Isopropyl trans-(4-(5-(4-bromo-2-(cyclopropylsulfonyl)phenyl)thiazol-2-yl)cyclohexyl) carbamate (30 mg, 56.9 μmol) and 2-methoxy-5-aminopyridine (8.47 mg, 68.3 μmol) were dissolved in tetrahydrofuran (3 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (5.16 mg, 5.69 μmol) and potassium acetate (5.58 mg, 56.9 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by preparative liquid chromatography (Boston Prime column, 5 μm silica, 30 mm in diameter, 150 mm in length; using decreasingly polar mixtures of water (containing 0.05% aqueous ammonia) and acetonitrile as eluents) to obtain isopropyl trans-(4-(5-(2-(cyclopropylsulfonyl)-4-((6-methoxypyridin-3-yl)amino)phenyl)thiazol-2-yl)cyclohexyl)carbamate as a white solid product (10.95 mg).

MS m/z (ESI): 571.2 [M+H]$^+$;

$^1$H NMR (400MHz, DMSO-d6) δ = 8.66 (s, 1H), 8.07 (d, J = 2.6 Hz, 1H), 7.65 (s, 1H), 7.62 (dd, J = 2.8, 8.8 Hz, 1H), 7.49 (d, J = 2.5 Hz, 1H), 7.33 (d, J = 8.4 Hz, 1H), 7.10 (dd, J = 2.6, 8.4 Hz, 1H), 7.05-6.99 (m, 1H), 6.87 (d, J = 8.8 Hz, 1H), 4.81-4.68 (m, 1H), 3.85 (s, 3H), 2.97-2.87 (m, 1H), 2.45-2.35 (m, 1H), 2.13 (br d, J = 12.8 Hz, 2H), 1.92 (br d, J = 10.0 Hz, 2H), 1.63-1.50 (m, 2H), 1.40-1.27 (m, 2H), 1.17 (d, J = 6.3 Hz, 6H), 1.00-0.92 (m, 2H), 0.90-0.83 (m, 2H).

Examples 94 and 94-1: Synthesis of isopropyl trans-(4-(5-(2-((S)-cyclopropanesulfonimidoyl))-4-(6-methoxypyridin-3-yl)phenyl)thiazol-2-yl)cyclohexyl )carbamate and isopropyl trans-((4-(5-(2-((R)-cyclopropanesulfonimidoyl))-4-(6-methoxypyridin-3-yl)phenyl)thiazol-2-yl)cyclohexyl)carbamate

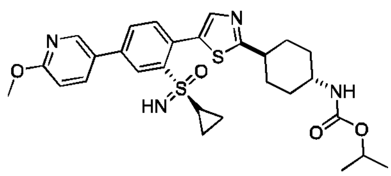

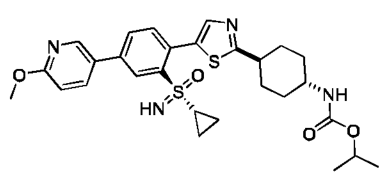

Synthetic Method

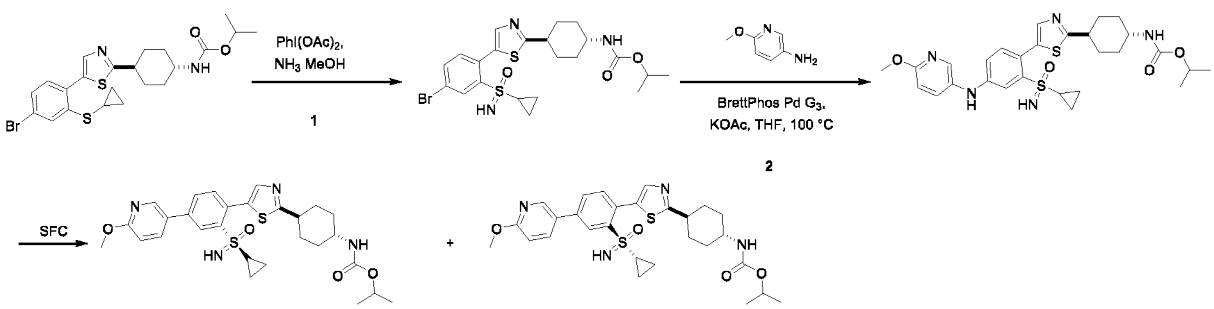


Step 1: Synthesis of isopropyl trans-(4-(5-(4-bromo-2-(cyclopropanesulfonimidoyl) phenyl)thiazol-2-yl)cyclohexyl)carbamate

Isopropyl trans-((4-(5-(4-bromo-2-(cyclopropylthio)phenyl)thiazol-2-yl)cyclohexyl) carbamate (850 mg, 1.72 mmol) was dissolved in a solution of ammonia in methanol (61 mL), and diacetoxyiodobenzene (8.29 g, 25.7 mmol) was added. The reaction solution was stirred and reacted at -20°C for 0.5 hours, and then slowly warmed to 30°C at which

the reaction solution was stirred and reacted for 0.5 hours. The completion of the reaction was detected by LCMS. The reaction solution was concentrated to dryness under reduced pressure and then purified by column chromatography (silica, petroleum ether:ethyl acetate = 0:1) to obtain isopropyl trans-(4-(5-(4-bromo-2-(cyclopropanesulfonimidoyl)phenyl)thiazol-2-yl) cyclohexyl)carbamate as a white solid product (547 mg).

MS m/z (ESI) : 526.2; 528.2 $[M+H]^+$;

Step 2: Synthesis of isopropyl trans-(4-(5-(2-(cyclopropanesulfonimidoyl)-4-((6-methoxypyridin-3-yl)amino)phenyl]thiazol-2-yl)cyclohexyl)carbamate

Isopropyl trans-(4-(5-(4-bromo-2-(cyclopropanesulfonimidoyl)phenyl)thiazol-2-yl) cyclohexyl)carbamate (547 mg, 1.04 mmol) and 6-methoxypyridin-3-amine (194 mg, 1.56 mmol) were dissolved in a tetrahydrofuran solution (20 mL), and methanesulfonato(2-dicyclohexylphosphino)-3,6-dimethoxy-2,4,6-triisopropyl-1,1-biphenyl)(2 -amino-1,1-biphenyl-2-yl)palladium (II) (94.2 mg, 104 μmol) and potassium acetate (305 mg, 3.12 mmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by column chromatography (silica, petroleum ether:ethyl acetate = 0:1) to obtain isopropyl trans-(4-(5-(2-(cyclopropanesulfonimidoyl)-4-((6-methoxypyridin-3-yl)amino) phenyl]thiazol-2-yl)cyclohexyl)carbamate as a yellow solid product (450 mg).

MS m/z (ESI) : 569.9 $[M+H]^+$;

Step 3: Synthesis of isopropyl trans-(4-(5-(2-((S)-cyclopropanesulfonimidoyl))-4-(6-methoxypyridin-3-yl)phenyl)thiazol-2-yl)cyclohexyl)carbamate and isopropyl trans-((4-(5-(2-((R)-cyclopropanesulfonimidoyl))-4-(6-methoxypyridin-3-yl)phenyl)thiazol-2-yl)cyclohexyl )carbamate

Isopropyl trans-(4-(5-(2-(cyclopropanesulfonimidoyl)-4-((6-methoxypyridin-3-yl)amino) phenyl]thiazol-2-yl)cyclohexyl)carbamate (450mg) as a racemic compound was separated by supercritical fluid chromatography (DAICEL CHIRALCEL OD-H column, 5 μm silica, 30 mm in diameter, 250 mm in length) (using polar mixtures of water (containing 0.1% aqueous ammonia) and ethanol (50% to 50%) as eluents) to obtain a pair of enantiomers (207.40 mg, retention time on chromatogram: 5.708 minutes, named as Compound of Example 94) and another pair of enantiomers (210.80 mg, retention time on chromatogram: 6.506 minutes, named as Compound of Example 94-1).

Example 94

MS m/z (ESI): 570.1 $[M+H]^+$;

$^1$H NMR (400MHz, DMSO-d6) δ 8.56 (s, 1H), 8.05 (d, J = 2.8 Hz, 1H), 7.66 (s, 1H), 7.61-7.56 (m, 2H), 7.24 (d, J = 8.3 Hz, 1H), 7.08-6.96 (m, 2H), 6.84 (d, J = 8.8 Hz, 1H), 4.74 (td, J = 6.2, 12.4 Hz, 1H), 4.29 (br s, 1H), 3.83 (s, 3H), 3.31-3.25 (m, 1H), 2.89 (tt, J = 3.5, 11.9 Hz, 1H), 2.31-2.24 (m, 1H), 2.12 (br d, J = 11.7 Hz, 2H), 1.96-1.86 (m, 2H), 1.62-1.48 (m, 2H), 1.40-1.26 (m, 2H), 1.16 - 1.05 (m, 6H), 0.91-0.75 (m, 4H).

Example 94-1

MS m/z (ESI): 570.1 $[M+H]^+$;

$^1$H NMR (400MHz, DMSO-d6) δ 8.56 (s, 1H), 8.05 (d, J = 2.8 Hz, 1H), 7.66 (s, 1H), 7.62-7.55 (m, 2H), 7.24 (d, J = 8.3 Hz, 1H), 7.07-6.97 (m, 2H), 6.84 (d, J = 8.8 Hz, 1H), 4.74 (td, J = 6.2, 12.5 Hz, 1H), 4.28 (br s, 1H), 3.83 (s, 3H), 3.24 (br s, 1H), 2.89 (tt, J = 3.4, 11.9 Hz, 1H), 2.30-2.22 (m, 1H), 2.12 (br d, J = 11.6 Hz, 2H), 1.98-1.84 (m, 2H), 1.62-1.48 (m, 2H), 1.39-1.26 (m, 2H), 1.16 - 1.05 (m, 6H), 0.92-0.72 (m, 4H).

Example 95: Synthesis of isopropyl trans-(4-(5-(2-(cyclopropanesulfonimidoyl)-4-((6-oxo-1,6-dihydropyridin-3-yl)amino)phenyl)thiazol-2-yl)cyclohexyl)carbamate

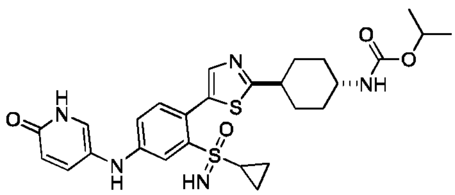

Synthetic Method

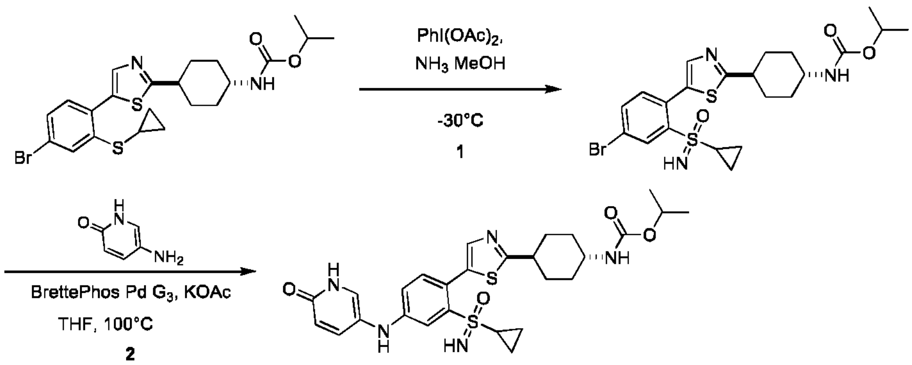


Step 1: Synthesis of isopropyl trans-(4-(5-(4-bromo-2-(cyclopropanesulfonimidoyl) phenyl)thiazol-2-yl)cyclohexyl)carbamate

Isopropyl trans-(4-(5-(4-bromo-2-(cyclopropylthio)phenyl)thiazol-2-yl)cyclohexyl) carbamate (200 mg, 403 μmol) was dissolved in a solution of ammonia in methanol (9 mL), and diacetoxyiodobenzene (1.95 g, 6.05 mmol) was added. The reaction solution was stirred and reacted at -30°C for 2 hours. The completion of the reaction was detected by LCMS. The reaction solution was concentrated to dryness under reduced pressure and then purified by column chromatography (silica, petroleum ether:ethyl acetate = 1:1) to obtain isopropyl trans-(4-(5-(4-bromo-2-(cyclopropanesulfonimidoyl)phenyl)thiazol-2-yl)cyclohexyl)carbamate as a yellow solid compound (140 mg).

MS m/z (ESI): 527.8 $[M+H]^+$;

Step 2: Synthesis of isopropyl trans-(4-(5-(2-(cyclopropanesulfonimidoyl)-4-((6-oxo-1,6-dihydropyridin-3-yl)amino)phenyl)thiazol-2-yl)cyclohexyl)carbamate

Isopropyl trans-(4-(5-(4-bromo-2-(cyclopropanesulfonimidoyl)phenyl)thiazol-2-yl) cyclohexyl)carbamate and 5-amino-2-hydroxypyridine (14.6 mg, 133 μmol) were dissolved in tetrahydrofuran (3 mL), and potassium acetate (39.1 mg, 399 μmol) and methanesulfonato(2-dicyclohexylphosphino)-3,6-dimethoxy-2,4,6-triisopropyl-1,1-biphenyl)(2 -amino-1,1-biphenyl-2-yl)palladium (II) (12.0 mg, 13.3 μmol) were added. Under the protection of nitrogen, the reaction solution was stirred and reacted at 100°C for 2 hours. The completion of the reaction was detected by LCMS. The reaction solution was concentrated to dryness under reduced pressure and purified by preparative liquid chromatography (Phenomenex Gemini-NX column: 5 μm silica, 30 mm in diameter, 150 mm in length; using decreasingly polar mixtures of water (containing 0.05% aqueous ammonia) and acetonitrile (33% to 53%) as eluents) to obtain isopropyl trans-(4-(5-(2-(cyclopropanesulfonimidoyl)-4-((6-oxo-1,6-dihydropyridin-3-yl)amino)phenyl)thiazol-2-yl)cyclohexyl)carbamate as a yellow solid compound (23.6 mg).

MS m/z (ESI): 556.0 $[M+H]^+$;

$^1H$ NMR (400MHz, DMSO-d6) δ = 8.11 (s, 1H), 7.64 (s, 1H), 7.45-7.35 (m, 2H), 7.29 (br s, 1H), 7.20 (d, J = 8.3 Hz, 1H), 7.03 (br d, J = 7.5 Hz, 1H), 6.81 (dd, J = 2.5, 8.3 Hz, 1H), 6.40 (d, J = 9.5 Hz, 1H), 4.74 (td, J = 6.2, 12.5 Hz, 1H), 4.26 (br s, 1H), 3.31-3.29 (m, 1H), 2.88 (br t, J = 12.0 Hz, 1H), 2.28-2.18 (m, 1H), 2.11 (br d, J = 11.5 Hz, 2H), 1.90 (br d, J = 10.8 Hz, 2H), 1.61-1.47 (m, 2H), 1.40-1.24 (m, 2H), 1.16 - 1.05 (m, 6H), 0.89-0.73 (m, 4H).

Example 96: Synthesis of isopropyl trans-(4-(5-(2-(cyclopropylsulfonyl)-4-((6-oxo-1,6-dihydropyridin-3-yl)amino)phenyl)thiazol-2-yl)cyclohexyl)carbamate

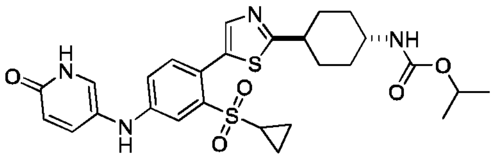

Synthetic Method

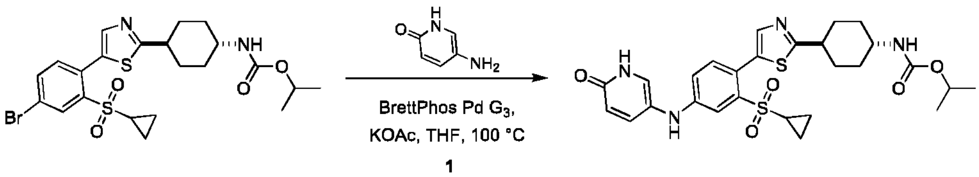


Isopropyl trans-(4-(5-(4-bromo-2-(cyclopropylsulfonyl)phenyl)thiazol-2-yl)cyclohexyl) carbamate (50.0 mg, 94.8 μmol) and 5-amino-2-hydroxypyridine (15.7 mg, 142 μmol) were dissolved in tetrahydrofuran (5 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (8.59 mg, 9.48 μmol) and cesium carbonate (92.7 mg, 284 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by preparative liquid chromatography (YMC-Actus Triart C18 column: 5 μm silica, 30 mm in diameter, 150 mm in length; using decreasingly polar mixtures of water (containing 0.225% formic acid) and acetonitrile (33% to 53%) as eluents) to obtain isopropyl trans-(4-(5-(2-(cyclopropylsulfonyl)-4-((6-oxo-1,6-dihydropyridin-3-yl)amino)phenyl)thiazol-2-yl)cyclohexyl)carbamate as a white solid compound (2.50 mg).

MS m/z (ESI): 557.0 $[M+H]^+$;

$^1$H NMR (400MHz, DMSO-d6) δ 11.83-11.16 (m, 1H), 11.83-11.16 (m, 1H), 8.20 (s, 1H), 7.69-7.56 (m, 1H), 7.41 (dd, J = 2.9, 9.6 Hz, 1H), 7.35-7.26 (m, 3H), 7.02 (br d, J = 7.7 Hz, 1H), 6.90 (dd, J = 2.6, 8.4 Hz, 1H), 6.41 (d, J = 9.5 Hz, 1H), 4.74 (td, J = 6.3, 12.4 Hz, 1H), 3.32 (d, J = 1.7 Hz, 1H), 2.91 (tt, J = 3.3, 11.8 Hz, 1H), 2.40-2.31 (m, 1H), 2.12 (br d, J = 11.6 Hz, 2H), 1.90 (br d, J = 10.3 Hz, 2H), 1.63-1.48 (m, 2H), 1.41-1.26 (m, 2H), 1.16 - 1.05 (m, 6H), 0.99-0.80 (m, 4H).

Example 97: Synthesis of isopropyl trans-(4-(5-(4-((5-methyl-1H-pyrazol-3-yl)amino)-2-(N-methylcyclopropanesulfonimidoyl)phenyl)thiazol-2-yl)cyclohexyl)carbamate

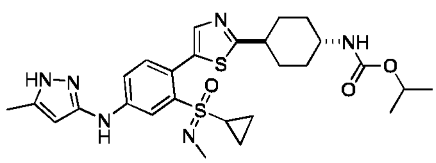

Synthetic Method

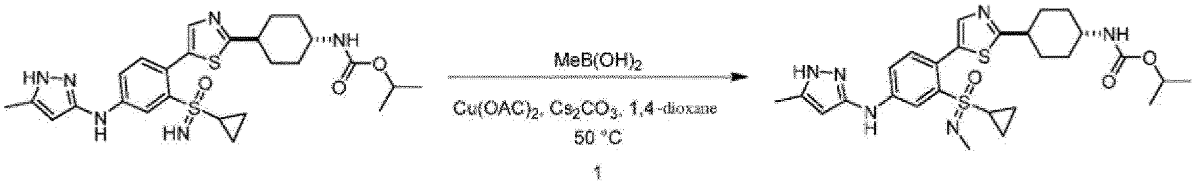


Isopropyl trans-(4-(5-(2-(cyclopropanesulfonimidoyl)-4-((5-methyl-1H-pyrazol-3-yl) amino)phenyl]thiazol-2-yl)cyclohexyl)carbamate (50.0 mg, 92.2 μmol) was dissolved in anhydrous dioxane (3 mL), and anhydrous copper acetate (1.67 mg, 9.35 μmol) and cesium carbonate (60.1 mg, 184 μmol) were added. The reaction solution was stirred and reacted at 25°C for 5 minutes. Methylboronic acid (6.6 mg, 111 μmol) was added into the reaction solution. The reaction solution was stirred and reacted at 50°C for 16 hours. The completion of the reaction was detected by LCMS. The reaction solution was filtered, concentrated to dryness, added with a saturated ammonium chloride solution (10 mL), and extracted with ethyl acetate (30 mL) three times. The organic phase was dried over anhydrous sodium sulfate and filtered. The filtrate was concentrated to dryness under reduced pressure and purified by preparative high-performance liquid chromatography (YMC-Actus Triart column, 5 μm silica, 30 mm in diameter, 150 mm in length) (using decreasingly polar mixtures of water (containing 0.05% aqueous ammonia and 10 mmol of ammonium bicarbonate) and acetonitrile (40% to 60%) as eluents) and lyophilized to obtain isopropyl trans-(4-(5-(4-((5-methyl-1H-pyrazol-3-yl)amino)-2-(N-methylcyclopropanesulfonimidoyl)phenyl)thiazol-2-yl)cyclohexyl)carbamate as a white solid compound (2.01 mg).

MS m/z (ESI): 557.0 [M+H]$^+$;

$^1$H NMR (400MHz, DMSO-d6) δ = 11.80 (br s, 1H), 8.93 (s, 1H), 7.91 (s, 1H), 7.70 - 7.48 (m, 2H), 7.24 (d, J = 8.6 Hz, 1H), 7.00 (br d, J = 7.8 Hz, 1H), 5.58 (s, 1H), 4.70 (td, J = 6.3, 12.3 Hz, 1H), 3.30 - 3.29 (m, 3H), 3.28 - 3.24 (m, 1H), 2.86 (br t, J = 12.0 Hz, 1H), 2.16 (s, 4H), 2.13 - 2.03 (m, 2H), 1.87 (br d, J = 11.0 Hz, 2H), 1.60 - 1.43 (m, 2H), 1.38 - 1.22 (m, 2H), 1.13 - 1.11 (m, 6H), 1.06 (br dd, J = 6.5, 10.6 Hz, 1H), 1.00 - 0.89 (m, 1H), 0.79 - 0.69 (m, 1H), 0.65 - 0.51 (m, 1H).

Example 98: Synthesis of isopropyl trans-(4-(5-(2-(cyclopropanesulfonimidoyl)-4-((1,3-dimethyl-1H-pyrazol-5-yl)amino)phenyl)thiazol-2-yl)cyclohexyl)carbamate

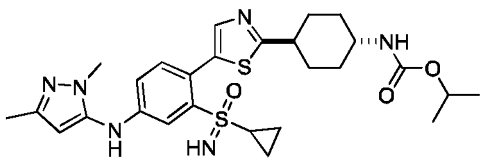

Synthetic Method

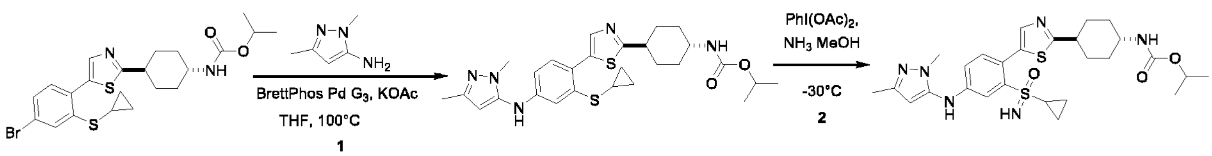


Step 1: Synthesis of isopropyl trans-(4-(5-(2-(cyclopropylthio)-4-((1,3-dimethyl-1H-pyrazol-5-yl)amino)phenyl)thiazol-2-yl)cyclohexyl)carbamate

Isopropyl trans-(4-(5-(4-bromo-2-(cyclopropylthio)phenyl)thiazol-2-yl)cyclohexyl) carbamate (200 mg, 404 μmol) and 1,3-dimethyl-1H-pyrazol-5-amine (67.3 mg, 605 μmol) were dissolved in tetrahydrofuran (6 mL), and potassium acetate (119 mg, 1.21 mmol) and methanesulfonato(2-dicyclohexylphosphino)-3,6-dimethoxy-2,4,6-triisopropyl-1,1-biphenyl)(2 -amino-1,1-biphenyl-2-yl)palladium (II) (36.6 mg, 40.4 μmol) were added. Under the protection of nitrogen, the reaction solution was stirred and reacted at 100°C for 2 hours. The completion of the reaction was detected by LCMS. The reaction solution was concentrated to dryness under reduced pressure and purified by column chromatography (silica, petroleum ether:ethyl acetate = 1:2) to obtain isopropyl trans-(4-(5-(2-(cyclopropylthio)-4-((1,3-dimethyl-1H-pyrazol-5-yl)amino)phenyl)thiazol-2-yl)cyclohexyl)carbamate as a yellow solid compound (120 mg).

Step 2: Synthesis of isopropyl trans-(4-(5-(2-(cyclopropanesulfonimidoyl)-4-((1,3-dimethyl-1H-pyrazol-5-yl)amino)phenyl)thiazol-2-yl)cyclohexyl)carbamate

Isopropyl trans-(4-(5-(2-(cyclopropylthio)-4-((1,3-dimethyl-1H-pyrazol-5-yl)amino) phenyl)thiazol-2-yl)cyclohexyl)carbamate (120 mg, 228 μmol) was dissolved in a solution of ammonia in methanol (7M, 6 mL), and diacetoxyiodobenzene (1.10 g, 3.42 mmol) was added. The reaction solution was stirred and reacted at -30°C for 2 hours. The completion of the reaction was detected by LCMS. The reaction solution was concentrated to dryness under reduced pressure and then purified by column chromatography (silica, petroleum ether:ethyl acetate = 1:2) to obtain a yellow solid, which was purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (30% to 50%) as eluents) to obtain isopropyl trans-(4-(5-(2-(cyclopropanesulfonimidoyl)-4-((1,3-dimethyl-1H-pyrazol-5-yl)amino)phenyl)thiazol-2-yl) cyclohexyl)carbamate as a yellow solid compound (1.29 mg).
MS m/z (ESI): 557.0 $[M+H]^+$;
$^1$H NMR (400MHz, DMSO-d6) δ = 8.57 (s, 1H), 7.66 (s, 1H), 7.59 (d, J = 2.5 Hz, 1H), 7.26 (d, J = 8.4 Hz, 1H), 7.05-6.94 (m, 2H), 5.88 (s, 1H), 4.82-4.65 (m, 1H), 4.32 (s, 1H), 3.62 (s, 3H), 3.21 (br s, 1H), 2.91-2.86 (m, 1H), 2.28-2.22 (m, 1H), 2.16-2.05 (m, 5H), 1.90 (br d, J = 10.1 Hz, 2H), 1.61-1.48 (m, 2H), 1.38-1.28 (m, 2H), 1.16-1.05 (m, 6H), 0.89-0.76 (m, 4H).

Example 99: Synthesis of isopropyl trans-(4-(5-(2-(cyclopropanesulfonimidoyl)-4-(oxazol-2-ylamino)phenyl)thiazol-2-yl)cyclohexyl)carbamate

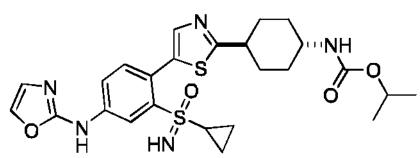

Synthetic Method

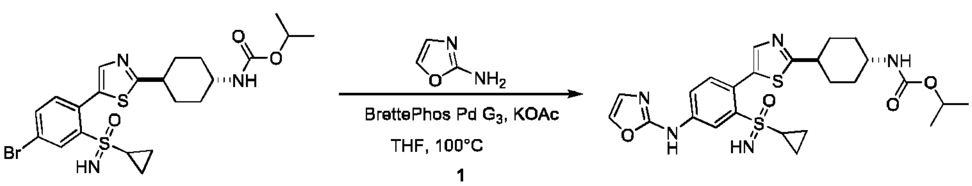


Isopropyl trans-(4-(5-(4-bromo-2-(cyclopropanesulfonimidoyl)phenyl)thiazol-2-yl) cyclohexyl)carbamate (70.0 mg, 132 μmol) and oxazol-2-amine (12.3 mg, 146 μmol) were dissolved in tetrahydrofuran (3 mL), and potassium acetate (39.1 mg, 398 μmol) and methanesulfonato(2-dicyclohexylphosphino)-3,6-dimethoxy-2,4,6-triisopropyl-1,1-biphenyl)(2 -amino-1,1-biphenyl-2-yl)palladium (II) (12.0 mg, 13.3 μmol) were added. Under the protection of nitrogen, the reaction solution was stirred and reacted at 100°C for 2 hours. The completion of the reaction was detected by LCMS. The reaction solution was concentrated to dryness under reduced pressure and purified by preparative liquid chromatography (Phenomenex Gemini-NX column: 5 μm silica, 30 mm in diameter, 150 mm in length; using decreasingly polar mixtures of water (containing 0.05% aqueous ammonia) and acetonitrile (33% to 53%) as eluents) to obtain isopropyl trans-(4-(5-(2-(cyclopropanesulfonimidoyl)-4-(oxazol-2-ylamino)phenyl)thiazol-2-yl)cyclohexyl)carbamate as a white solid (12.8 mg).
MS m/z (ESI): 530.0 $[M+H]^+$;
$^1$H NMR (400MHz, DMSO-d6) δ = 10.62 (s, 1H), 8.34 (d, J = 2.3 Hz, 1H), 7.91 (dd, J = 2.4, 8.4 Hz, 1H), 7.74-7.69 (m, 2H), 7.41 (d, J = 8.5 Hz, 1H), 7.09-6.99 (m, 2H), 4.74 (td, J = 6.1, 12.4 Hz, 1H), 4.38 (s, 1H), 3.28 (br s, 1H), 2.96-2.86 (m, 1H), 2.32-2.23 (m, 1H), 2.13 (br d, J = 12.3 Hz, 2H), 1.91 (br d, J = 9.8 Hz, 2H), 1.81 (d, J = 13.6 Hz, 1H), 1.63-1.48 (m, 2H), 1.40-1.26 (m, 2H), 1.16 - 1.05 (d, J = 6.0 Hz, 6H), 0.96-0.75 (m, 4H).

Example 100: Synthesis of isopropyl trans-(4-(5-(4-((1,3,4-oxadiazol-2-yl)amino)-2-(cyclopropanesulfonimidoyl)phenyl)thiazol-2-yl)cyclohexyl)carbamate

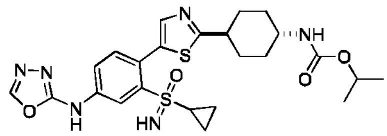

Synthetic Method

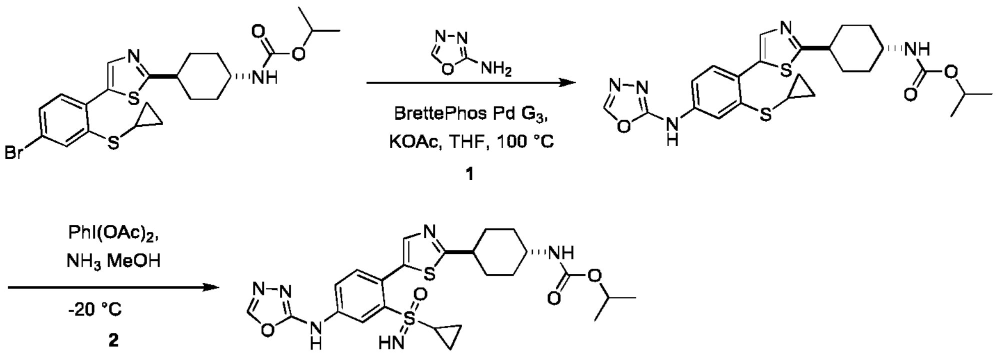


Step 1: Synthesis of isopropyl trans-(4-(5-(4-((1,3,4-oxadiazol-2-yl)amino)-2-(cyclopropylthio)phenyl)thiazol-2-yl)cyclohexyl)carbamate

Isopropyl trans-(4-(5-(4-bromo-2-(cyclopropylthio)phenyl)thiazol-2-yl)cyclohexyl) carbamate (90.0 mg, 182 μmol) and 1,3,4-oxadiazol-2-amine (23.2 mg, 272 μmol) were dissolved in tetrahydrofuran (5 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (16.5 mg, 18.2 μmol) and potassium acetate (53.5 mg, 545 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by thin-layer chromatography (silica, dichloromethane:methanol = 15:1) to obtain isopropyl trans-(4-(5-(4-((1,3,4-oxadiazol-2-yl)amino)-2-(cyclopropylthio)phenyl)thiazol-2-yl)cyclohexyl)carbamate as a white solid product (51.0 mg).
MS m/z (ESI): 537.4 $[M+H]^+$;

Step 2: Synthesis of isopropyl trans-(4-(5-(4-((1,3,4-oxadiazol-2-yl)amino)-2-(cyclopropanesulfonimidoyl)phenyl)thiazol-2-yl)cyclohexyl)carbamate

Isopropyl trans-(4-(5-(4-((1,3,4-oxadiazol-2-yl)amino)-2-(cyclopropylthio)phenyl)thiazol-2-yl)cyclohexyl)carbamate (25.0 mg, 50.0 μmol) was dissolved in a solution of ammonia in methanol (1.25 mL), and diacetoxyiodobenzene (242 mg, 751 μmol) was added. The reaction solution was stirred and reacted at -20°C for 1 hour. The completion of the reaction was detected by LCMS. The reaction solution was concentrated to dryness under reduced pressure and then purified by preparative liquid chromatography (Phenomenex Gemini-NX column: 5 μm silica, 30 mm in diameter, 150 mm in length; using decreasingly polar mixtures of water (containing 0.225% formic acid) and acetonitrile as eluents) to obtain isopropyl trans-(4-(5-(4-((1,3,4-oxadiazol-2-yl)amino)-2-(cyclopropanesulfonimidoyl)phenyl)thiazol-2-yl)cyclohexyl)carbamate as a white solid compound (2.18 mg).
MS m/z (ESI): 531.0 $[M+H]^+$;
$^1H$ NMR (400MHz, DMSO-d6) δ 11.02 (s, 1H), 8.81 (s, 1H), 8.33 (d, J = 2.4 Hz, 1H), 7.88 (dd, J = 2.4, 8.4 Hz, 1H), 7.73 (s, 1H), 7.47 (d, J = 8.4 Hz, 1H), 7.01 (br d, J = 7.8 Hz, 1H), 4.74 (td, J = 6.2, 12.5 Hz, 1H), 4.43 (br s, 1H), 3.22-3.09 (m, 1H), 2.92 (ddd, J = 3.3, 8.4, 11.9 Hz, 1H), 2.34-2.25 (m, 1H), 2.13 (br d, J = 11.6 Hz, 2H), 1.91 (br d, J = 10.8 Hz, 2H), 1.66-1.47 (m, 2H), 1.43-1.26 (m, 2H), 1.16 - 1.05 (m, 6H), 0.98-0.71 (m, 4H).

Example 101: Synthesis of isopropyl trans-(4-(5-(2-(cyclobutanesulfonimidoyl)-4-((5-methyl-1H-pyrazol-3-yl)amino)phenyl]thiazol-2-yl)cyclohexyl)carbamate

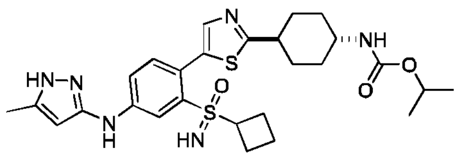

Synthetic Method

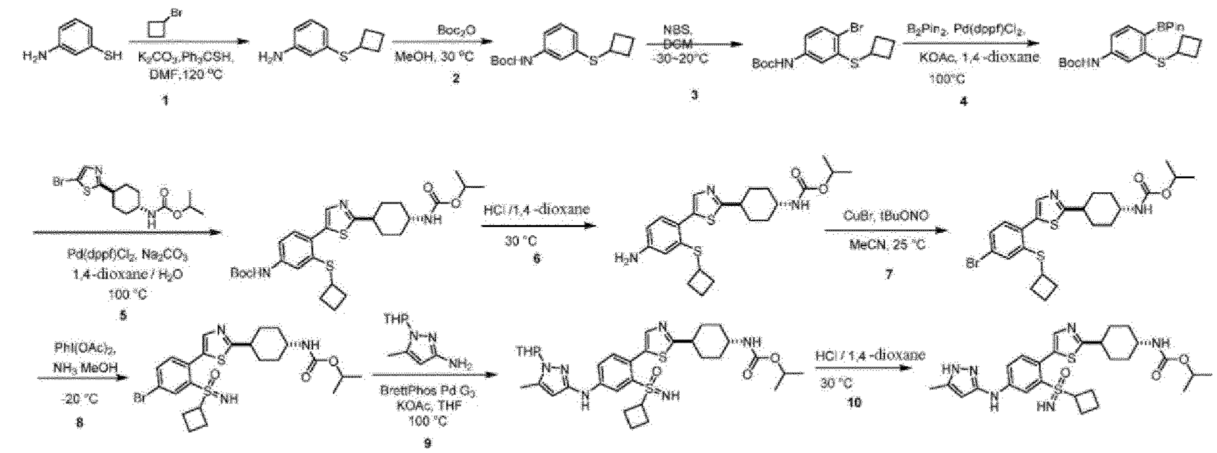


Step 1: Synthesis of 3-(cyclobutylthio)aniline

3-Aminothiophenol (15.0 g, 119 mmol), triphenylmethanethiol (993 mg, 3.59 mmol) and potassium carbonate (49.6 g, 359 mmol) were dissolved in N,N-dimethylformamide (150 mL), and cyclobutyl bromide (24.2 g, 179 mmol) was added. The reaction solution appeared as a yellow suspension, and was stirred and reacted at 120°C for 16 hours. The completion of the reaction was detected by LCMS. The reaction solution was concentrated to dryness under reduced pressure, added with water (500 mL), and extracted with ethyl acetate (300 mL) three times. The organic layer was dried over anhydrous magnesium sulfate and subjected to suction filtration. The filtrate was concentrated to dryness under reduced pressure. Thereafter, the resultant was purified by column chromatography (petroleum ether:ethyl acetate = 4:1) to obtain the compound 3-(cyclobutylthio)aniline (18.0 g) as a red oily liquid.
MS m/z (ESI): 180.1 $[M+H]^+$;

Step 2: Synthesis of tert-butyl (3-(cyclobutylthio)phenyl)carbamate

3-(Cyclobutylthio)aniline (32.0 g, 178 mmol) was dissolved in anhydrous methanol (500 mL), and BOC anhydride (389 g, 1.78 mol) was added. The reaction solution appeared as a yellow solution, and was stirred and reacted at 30°C for 16 hours. The completion of the reaction was detected by LCMS. The reaction solution was concentrated to dryness under reduced pressure. The resultant was added with dichloromethane (1L), and the pH of the resulting mixture was adjusted to 10 with aqueous ammonia. The organic layer was washed with saturated saline (1L), dried over anhydrous magnesium sulfate, and subjected to suction filtration. The filtrate was concentrated to dryness under reduced pressure to obtain the compound tert-butyl (3-(cyclobutylthio)phenyl)carbamate (120 g, crude product) as a yellow oily liquid.
MS m/z (ESI): 224.1 $[M+H-t-Bu]^+$;

Step 3: Synthesis of tert-butyl (4-bromo-3-(cyclobutylthio)phenyl)carbamate

Tert-butyl (3-(cyclobutylthio)phenyl)carbamate (120 g, 171 mmol, 40% purity) was dissolved in anhydrous dichloromethane (1L), and a suspension of N-bromosuccinimide (389 g, 1.78 mol) in dichloromethane (500 mL) was

added at -30°C. The reaction solution appeared as a yellow solution, and was stirred and reacted at 20°C for 16 hours. The completion of the reaction was detected by LCMS. The reaction solution was concentrated to dryness under reduced pressure. The resultant was purified by column chromatography (petroleum ether:ethyl acetate = 100:1) to obtain the compound tert-butyl (4-bromo-3-(cyclobutylthio) phenyl)carbamate (120 g, crude product) as a white solid.

MS m/z (ESI): 358.1; 360.1 $[M+H]^+$;

Step 4: Synthesis of tert-butyl N-[3-cyclobutylsulfanyl-4-(4,4,5,5-tetramethyl-1,3,2-dioxaborolan-2-yl)phenyl]carbamate

Tert-butyl (4-bromo-3-(cyclobutylthio)phenyl)carbamate (10.0 g, 27.9 mmol) and bis(pinacolato)diboron (28.3 g, 112 mmol) were dissolved in N,N-dimethylformamide (300 mL). Under nitrogen atmosphere, potassium acetate (8.22 g, 83.7 mmol) and [1,1-bis(diphenylphosphino)ferrocene] dichloropalladium - dichloromethane (2.28 g, 2.79 mmol) were added. The reaction solution was stirred and reacted at 100°C for 3 hours. The completion of the reaction was detected by LCMS. The reaction solution was filtered. The filtrate was concentrated to dryness under reduced pressure and then purified by column chromatography (silica, petroleum ether: ethyl acetate = 10:1) to obtain tert-butyl N-[3-cyclobutylsulfanyl-4-(4,4,5,5-tetramethyl-1,3,2-dioxaborolan-2-yl)phenyl]carbamate as a yellow solid product (8.70 g).

MS m/z (ESI): 406.2 $[M+H]^+$;

$^1$H NMR (400MHz, DMSO-d6) $\delta$ = 9.52 (s, 1H), 7.45 (d, J = 8.3 Hz, 1H), 7.32-7.24 (m, 1H), 7.20-7.15 (m, 1H), 3.85-3.71 (m, 1H), 2.03-1.94 (m, 4H), 1.47 (s, 9H), 1.29 (s, 2H), 1.26 (s, 12H).

Step 5: Synthesis of isopropyl trans-N-[4-[5-[4-(tert-butoxycarbonylamino)-2-cyclobutylsulfanyl-phenyl]thiazol-2-yl]cyclohexyl]carbamate

Tert-butyl N-[3-cyclobutylsulfanyl-4-(4,4,5,5-tetramethyl-1,3,2-dioxaborolan-2-yl) phenyl]carbamate (8.60 g, 22.0 mmol) and isopropyl trans-(4-(5-bromothiazol-2-yl) cyclohexyl)carbamate (7.63 g, 22.0 mmol) were dissolved in anhydrous dioxane (300 mL), and a saturated sodium carbonate solution (40 mL) was added. Under nitrogen atmosphere, [1,1-bis(diphenylphosphino)ferrocene]dichloropalladium - dichloromethane (1.79 g, 2.20 mmol) was added. The reaction solution was stirred and reacted at 100°C for 10 hours. The completion of the reaction was detected by LCMS. The reaction solution was filtered. The filtrate was concentrated to dryness under reduced pressure and then purified by column chromatography (silica, petroleum ether:ethyl acetate = 1:3) to obtain isopropyl trans-N-[4-[5-[4-(tert-butoxycarbonylamino)-2-cyclobutylsulfanyl-phenyl]thiazol-2-yl]cyclohexyl]carbamate as a yellow solid product (6.50 g).

MS m/z (ESI): 546.3$[M+H]^+$;

Step 6: Synthesis of isopropyl trans-N-[4-[5-(4-amino-2-cyclobutylsulfanyl-phenyl) thiazol-2-yl]cyclohexyl]carbamate

Isopropyl trans-N-[4-[5-[4-(tert-butoxycarbonylamino)-2-cyclobutylsulfanyl-phenyl] thiazol-2-yl]cyclohexyl]carbamate (6.50 g, 11.91 mmol) was dissolved in anhydrous methanol (100 mL), and hydrochloric acid/dioxane (4M, 100 mL) was added. The reaction solution was stirred and reacted at 30°C for 2 hours. The completion of the reaction was detected by LCMS. The reaction solution was concentrated to dryness under reduced pressure and then extracted with dichloromethane (300 mL $\times$ 3) and a saturated sodium carbonate solution. The organic phases were combined, dried, and filtered. The filtrate was concentrated to dryness under reduced pressure to obtain isopropyl trans-N-[4-[5-(4-amino-2-cyclobutylsulfanyl-phenyl)thiazol-2-yl]cyclohexyl]carbamate as a brown oily product (4.00 g).

MS m/z (ESI): 446.2 $[M+H]^+$;

Step 7: Synthesis of isopropyl trans-N-[4-[5-(4-bromo-2-cyclobutylsulfanyl-phenyl) thiazol-2-yl]cyclohexyl]carbamate

Isopropyl trans-N-[4-[5-(4-amino-2-cyclobutylsulfanyl-phenyl)thiazol-2-yl]cyclohexyl] carbamate (3.90 g, 8.75 mmol) and tert-butyl nitrite (3.91 g, 37.89 mmol) were dissolved in acetonitrile (100 mL). Under nitrogen atmosphere, cuprous bromide (2.54 g, 17.7 mmol) was added. The reaction solution was stirred and reacted at 25°C for 16 hours. The completion of the reaction was detected by LCMS. The reaction solution was filtered. The filtrate was concentrated to dryness under reduced pressure and then extracted with ethyl acetate (400 mL $\times$ 3) and a saturated aqueous ammonium chloride solution (400 mL). The organic phase was dried over anhydrous sodium sulfate, filtered, evaporated to dryness via rotary evaporation, and then purified by column chromatography (silica, petroleum ether:ethyl acetate = 1:1) to obtain isopropyl trans-N-[4-[5-(4-bromo-2-cyclobutylsulfanyl-phenyl)thiazol-2-yl]cyclohexyl] carbamate as a brown solid product (1.00 g).

MS m/z (ESI):509.1; 511.1 $[M+H]^+$;

Step 8: Synthesis of isopropyl trans-N-[4-[5-[4-bromo-2-(cyclobutylsulfonimidoyl) phenyl]thiazol-2-yl]cyclohexyl]carbamate

Isopropyl trans-N-[4-[5-(4-bromo-2-cyclobutylsulfanyl-phenyl)thiazol-2-yl]cyclohexyl] carbamate (950 mg, 1.86 mmol) was dissolved in a solution of ammonia in methanol (7M, 66.6 mL), and diacetoxyiodobenzene (9.01 g, 27.97 mmol) was added. The reaction solution was stirred and reacted at -20°C for 1 hour. The completion of the reaction was detected by LCMS. The reaction solution was filtered. The filtrate was concentrated to dryness under reduced pressure and then purified by column chromatography (silica, petroleum ether:ethyl acetate = 1:2) to obtain isopropyl trans-N-[4-[5-[4-bromo-2-(cyclobutylsulfonimidoyl)phenyl]thiazol-2-yl]cyclohexyl]carbamate as a brown oily product (1.00 g).
MS m/z (ESI): 540.0; 542.0 $[M+H]^+$;

Step 9: Synthesis of isopropyl N-[4-[5-[2-(cyclobutylsulfonimidoyl)]-4-((5-methyl-1-tetrahydropyran-2-ylpyrazol-3-yl)amino]phenyl]thiazol-2-yl]cyclohexyl]carbamate

Isopropyl trans-N-[4-[5-[4-bromo-2-(cyclobutylsulfonimidoyl)phenyl]thiazol-2-yl] cyclohexyl]carbamate (100 mg, 555 μmol) was dissolved in anhydrous tetrahydrofuran (6 mL). Under nitrogen atmosphere, potassium acetate (108 mg, 1.11 mmol) and methanesulfonato(2-dicyclohexylphosphino)-3,6-dimethoxy-2,4,6-triisopropyl-1,1-biphenyl)(2 -amino-1,1-biphenyl-2-yl)palladium (II) (33.5 mg, 37.0 μmol) were added. The reaction solution was stirred and reacted at 100°C for 5 hours. The completion of the reaction was detected by LCMS. The reaction solution was filtered. The filtrate was concentrated to dryness under reduced pressure and then purified by column chromatography (silica, petroleum ether:ethyl acetate = 1:3) to obtain isopropyl N-[4-[5-[2-(cyclobutylsulfonimidoyl)]-4-((5-methyl-1-tetrahydropyran-2-ylpyrazol-3-yl)amino]phenyl]thiazol-2-yl]cyclohexyl]carbamate as a brown solid product (130 mg).
MS m/z (ESI): 641.1$[M+H]^+$;

Step 10: Synthesis of isopropyl trans-(4-(5-(2-(cyclobutanesulfonimidoyl)-4-((5-methyl-1H-pyrazol-3-yl)amino)phenyl]thiazol-2-yl)cyclohexyl)carbamate

Isopropyl N-[4-[5-[2-(cyclobutylsulfonimidoyl)]-4-((5-methyl-1-tetrahydropyran-2-ylpyrazol-3-yl)amino]phenyl]thiazol-2-yl]cyclohexyl]carbamate (130 mg, 203 μmol) was dissolved in anhydrous methanol (2 mL), and then hydrochloric acid/dioxane (4M, 2 mL) was added. The reaction solution was stirred and reacted at 30°C for 5 hours. The completion of the reaction was detected by LCMS. The reaction solution was filtered. The filtrate was concentrated to dryness under reduced pressure and purified by preparative liquid chromatography (YMC-ActusTriartC18 column: 5 μm silica, 30 mm in diameter, 150 mm in length; using decreasingly polar mixtures of water (containing 0.05% aqueous ammonia) and acetonitrile (40% to 60%) as eluents) to obtain isopropyl trans-(4-(5-(2-(cyclobutanesulfonimidoyl)-4-((5-methyl-1H-pyrazol-3-yl)amino)phenyl]thiazol-2-yl) cyclohexyl)carbamate as a white solid (43.8 mg).
MS m/z (ESI): 557.1 $[M+H]^+$;
$^1H$ NMR (400MHz, DMSO-d6) δ = 11.93-11.69 (m, 1H), 8.95 (s, 1H), 8.06 (d, J = 2.1 Hz, 1H), 7.70-7.50 (m, 2H), 7.21 (d, J = 8.4 Hz, 1H), 7.02 (br d, J = 7.8 Hz, 1H), 5.64 (s, 1H), 4.75 (s, 1H), 4.16 (s, 1H), 3.60 (t, J = 8.2 Hz, 1H), 3.32-3.26 (m, 1H), 2.90 (tt, J = 3.4, 11.8 Hz, 1H), 2.35-2.26 (m, 1H), 2.20 (s, 3H), 2.18-2.09 (m, 3H), 2.01-1.74 (m, 6H), 1.63-1.48 (m, 2H), 1.41-1.27 (m, 2H), 1.17 - 1.06 (m, 6H).

Example 102: Synthesis of isopropyl trans-N-[4-[5-[2-(cyclobutylsulfonimidoyl)]-4-[[1-(2-fluoroethyl)pyrazol-4-yl]amino]phenyl]thiazol-2-yl]cyclohexyl]carbamate

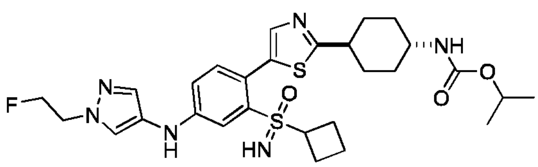

Synthetic Method

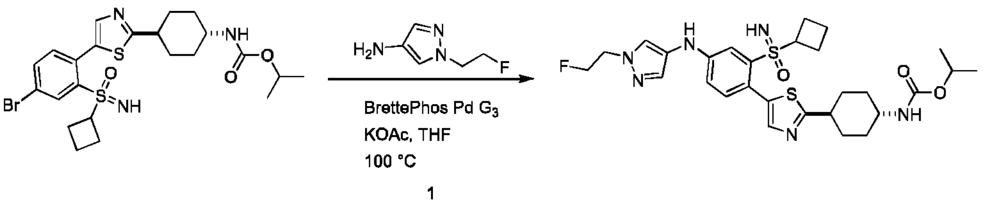


Isopropyl trans-N-[4-[5-[4-bromo-2-(cyclobutylsulfonimidoyl)phenyl]thiazol-2-yl] cyclohexyl]carbamate (100 mg, 185 μmol) and 1-(2-fluoroethyl)pyrazol-4-amine (35.8 mg, 277 μmol) were dissolved in anhydrous tetrahydrofuran (3 mL). Under nitrogen atmosphere, potassium acetate (KOAc) (54.4 mg, 555 μmol) and methanesulfonato(2-dicyclohexylphosphino-3,6-dimethoxy-2,4,6-triisopropyl-1,1-biphenyl)(2-amino-1,1-biphenyl-2-yl)palladium (II) (16.7 mg, 18.5 μmol) were added. The reaction solution was stirred and reacted at 100°C for 3 hours. The completion of the reaction was detected by LCMS. The reaction solution was concentrated to dryness under reduced pressure and filtered. The filtrate was concentrated to dryness under reduced pressure and purified by preparative liquid chromatography (YMC-ActusTriartC18 column: 5 μm silica, 30 mm in diameter, 150 mm in length; using decreasingly polar mixtures of water (containing 0.05% aqueous ammonia) and acetonitrile (30% to 50%) as eluents) to obtain isopropyl trans-N-[4-[5-[2-(cyclobutylsulfonimidoyl)]-4-[[1-(2-fluoroethyl)pyrazol-4-yl]amino]phenyl]thiazol-2-yl] cyclohexyl]carbamate as a white solid (10.3 mg).

MS m/z (ESI): 589.1 $[M+H]^+$;

$^1H$ NMR (400MHz, DMSO-d6) δ = 8.23 (s, 1H), 7.78 (s, 1H), 7.62 (s, 1H), 7.54 (br s, 1H), 7.46 (s, 1H), 7.17 (br d, J = 8.3 Hz, 1H), 7.02 (br d, J = 7.3 Hz, 1H), 6.90 (br d, J = 8.0 Hz, 1H), 4.89-4.66 (m, 3H), 4.48-4.32 (m, 2H), 4.23 (s, 1H), 3.63-3.51 (m, 1H), 3.30-3.24 (m, 1H), 2.89 (br t, J = 11.7 Hz, 1H), 2.32-2.07 (m, 4H), 1.99-1.69 (m, 6H), 1.62-1.46 (m, 2H), 1.33 (q, J = 11.4 Hz, 2H), 1.16 - 1.05 (m, 6H).

Example 103: Synthesis of isopropyl trans-(4-(5-(4-((5-methyl-1H-pyrazol-3-yl)amino)-2-(1-oxophosphetan-1-yl)phenyl)thiazol-2-yl)cyclohexyl)carbamate

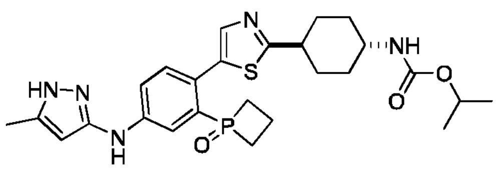

Synthetic Method

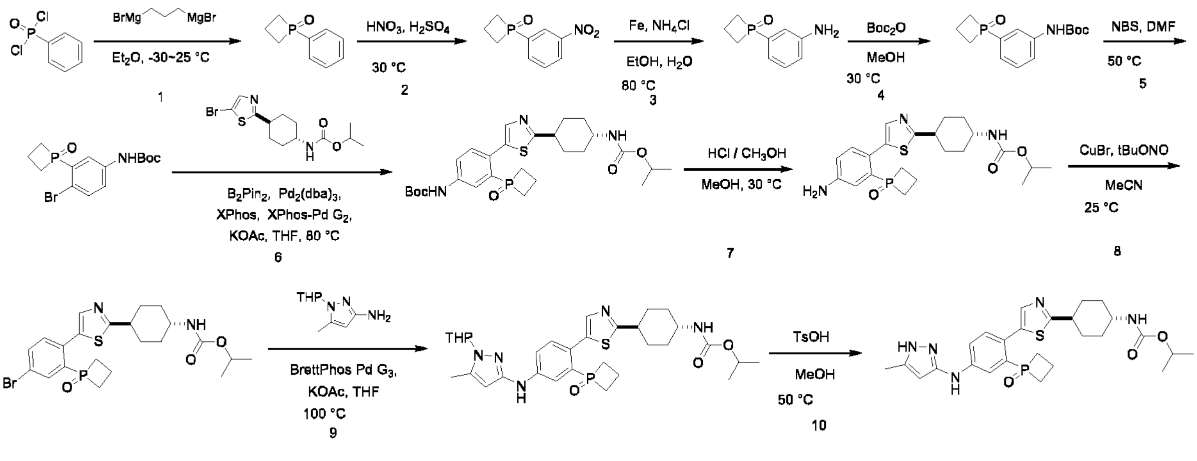

Step 1: Synthesis of 1-phenylphosphetane-1-oxide

Phenylphosphonic dichloride (28.9 g, 148 mmol) was dissolved in diethyl ether (300 mL). Under the protection of nitrogen, bromo-[3-(bromomagnesium)propyl]magnesium (37.2 g, 148 mmol) was added at -30°C. The reaction solution appeared as a gray suspension, and was stirred and reacted at 25°C for 16 hours. The completion of the reaction was detected by LCMS. The reaction solution was added with a saturated ammonium chloride solution (1L), and extracted with ethyl acetate (3L) three times. The organic phase was dried over anhydrous magnesium sulfate and subjected to suction filtration. The filtrate was concentrated to dryness under reduced pressure. The resultant was purified by column chromatography (dichloromethane:methanol = 10:1) to obtain the compound 1-phenylphosphetane-1-oxide (3.90 g, crude product) as a yellow oil.
MS m/z (ESI): 166.8 $[M+H]^+$;

Step 2: Synthesis of 1-(3-nitrophenyl)phosphine-1-oxide

1-Phenylphosphetane-1-oxide (3.90 g, 7.04 mmol) was dissolved in concentrated sulfuric acid (30 mL), and concentrated nitric acid (1.33 g, 21.1 mmol) was added at 0°C. The reaction solution appeared as a yellow suspension, and was stirred and reacted at 30°C for 2 hours. The completion of the reaction was detected by LCMS. The reaction solution was added with water (30 mL), and then extracted with dichloromethane (150 mL). The organic phase was dried over anhydrous magnesium sulfate and subjected to suction filtration. The filtrate was concentrated to dryness under reduced pressure to obtain crude 1-(3-nitrophenyl)phosphine-1-oxide (3.00g) as a yellow oil.
MS m/z (ESI): 211.9 $[M+H]^+$;

Step 3: Synthesis of 1-(3-aminophenyl)phosphine-1-oxide

1-(3-Nitrophenyl)phosphine-1-oxide (3.00 g, 4.26 mmol) was dissolved in anhydrous ethanol (40 mL) and water (8 mL). Under the protection of nitrogen, reduced iron powder (1.43 g, 25.6 mmol) and ammonium chloride (2.28 g, 42.6 mmol) were added. The reaction solution appeared as a black suspension, and was stirred and reacted at 80°C for 2 hours. The completion of the reaction was detected by LCMS. The reaction solution was filtered while it was still hot. The filtrate was concentrated to dryness under reduced pressure. The resultant was purified by column chromatography (dichloromethane:methanol = 10:1) to obtain the compound 1-(3-aminophenyl)phosphine-1-oxide (646 mg) as a yellow solid.
MS m/z (ESI): 181.9 $[M+H]^+$;

Step 4: Synthesis of tert-butyl (3-(1-oxophosphino-1-yl)phenyl)carbamate

1-(3-Aminophenyl)phosphine-1-oxide (646 mg, 3.57 mmol) was dissolved in methanol (35 mL), and BOC anhydride (11.7 g, 53.5 mmol) was added at 30°C. The reaction solution appeared as a yellow suspension, and was stirred and reacted at 30°C for 16 hours. The completion of the reaction was detected by LCMS. The reaction solution was concentrated to dryness under reduced pressure. Aqueous ammonia (10 mL) was added into the reaction solution. The reaction solution was stirred and reacted at 30°C for 2 hours, and then extracted with dichloromethane (20 mL). The organic layer was dried over anhydrous magnesium sulfate and subjected to suction filtration. The filtrate was concentrated to dryness under reduced pressure. The resultant was purified by column chromatography (dichloromethane:methanol = 10:1) to obtain the compound tert-butyl (3-(1-oxophosphino-1-yl)phenyl)carbamate(1.50 g) as a white solid.
MS m/z (ESI):282.1 [M+H]+;

Step 5: Synthesis of tert-butyl 4-bromo-3-(1-oxophosphino-1-yl)phenyl)carbamate

Tert-butyl (3-(1-oxophosphino-1-yl)phenyl)carbamate(1.40 g, 4.98 mmol) was dissolved in N,N-dimethylformamide (40 mL), and N-bromosuccinimide (1.77 g, 9.95 mmol) was added. The reaction solution appeared as a yellow suspension, and was stirred and reacted at 50°C for 3 hours. The completion of the reaction was detected by LCMS. The reaction solution was concentrated to dryness under reduced pressure and purified by column chromatography (dichloromethane:methanol = 10:1) to obtain the compound tert-butyl 4-bromo-3-(1-oxophosphino-1-yl)phenyl)carbamate (1.10 g) as a white solid.
MS m/z (ESI): 360.0 $[M+H]^+$;

Step 6: Synthesis of isopropyl trans-(4-(5-(4-tert-butoxycarbonylamino)-2-(1-oxophosphino-1-yl)phenyl)thiazol-2-yl)cyclohexyl)carbamate

Tert-butyl 4-bromo-3-(1-oxophosphino-1-yl)phenyl)carbamate (482 mg, 1.39 mmol) and bis(pinacolato)diboron (597 mg, 2.35 mmol) were dissolved in anhydrous tetrahydrofuran (18 mL). Under the protection of nitrogen, tris(dibenzylideneacetone)dipalladium (0) (127 mg, 138 μmol), 2-dicyclohexylphosphino-2,4,6-triisopropylbiphenyl (66.2 mg, 139 μmol), chloro(2-dicyclohexylphosphino-2,4,6-triisopropyl-1,1-biphenyl)[2-(2-amino-1,1-biphenyl)] palladium (II) (109 mg, 139 μmol) and potassium acetate (409 mg, 4.16 mmol) were added. The reaction solution appeared as a dark yellow suspension, and was stirred and reacted at 80°C for 16 hours. The completion of the reaction was detected by LCMS. The reaction solution was concentrated to dryness under reduced pressure, added with water (10 mL), and extracted with ethyl acetate (30 mL). The organic layer was dried over anhydrous magnesium sulfate and subjected to suction filtration. The filtrate was concentrated to dryness under reduced pressure. The resultant was purified by column chromatography (dichloromethane:methanol = 10:1) to obtain the compound isopropyl trans-(4-(5-(4-tert-butoxycarbonylamino)-2-(1-oxophosphino-1-yl)phenyl)thiazol-2-yl)cyclohexyl)carbamate (649 mg) as a yellow solid product.

MS m/z (ESI): 548.4 $[M+H]^+$;

Step 7: Synthesis of isopropyl trans-(4-(5-(4-amino-2-(1-oxophosphino-1-yl)phenyl) thiazol-2-yl)cyclohexyl)carbamate

Isopropyl trans-(4-(5-(4-tert-butoxycarbonylamino)-2-(1-oxophosphino-1-yl)phenyl) thiazol-2-yl)cyclohexyl)carbamate (600 mg, 1.10 mmol) was dissolved in anhydrous methanol (3 mL), hydrochloric acid/methanol (4M, 8.22 mL) was added. The reaction solution appeared as a yellow suspension, and was stirred and reacted at 30°C for 16 hours. The completion of the reaction was detected by LCMS. The reaction solution was concentrated to dryness under reduced pressure, added with a saturated sodium carbonate solution (30 mL), and extracted with dichloromethane (90 mL) three times. The organic layer was dried over anhydrous magnesium sulfate and subjected to suction filtration. The filtrate was concentrated to dryness under reduced pressure to obtain isopropyl trans-(4-(5-(4-amino-2-(1-oxophosphino-1-yl) phenyl)thiazol-2-yl)cyclohexyl)carbamate (410 mg, crude product) as a white solid.

MS m/z (ESI): 448.1 $[M+H]^+$;

Step 8: Synthesis of isopropyl trans-(4-(5-(4-bromo-2-(1-oxophosphino-1-yl) phenyl)thiazol-2-yl)cyclohexyl)carbamate

Isopropyl trans-(4-(5-(4-amino-2-(1-oxophosphino-1-yl)phenyl)thiazol-2-yl)cyclohexyl) carbamate (300 mg, 670 μmol) was dissolved in acetonitrile (360 mL). Under the protection of nitrogen, tert-butyl nitrite (1.38 g, 13.4 mmol, 1.59 mL) was added. The reaction solution was stirred and reacted for 1 hour. Under the protection of nitrogen, cuprous bromide (1.92 g, 13.4 mmol) was added. The reaction solution appeared as a yellow suspension, and was stirred and reacted at 25°C for 15 hours. The completion of the reaction was detected by LCMS. The reaction solution was concentrated to dryness under reduced pressure, added with saturated ammonium chloride solution (150 mL), and extracted with dichloromethane (90 mL) three times. The organic layer was dried over anhydrous magnesium sulfate and subjected to suction filtration. The filtrate was concentrated to dryness under reduced pressure. The resultant was purified by column chromatography (dichloromethane:methanol = 6:1) to obtain the compound isopropyl trans-(4-(5-(4-bromo-2-(1-oxophosphino-1-yl)phenyl)thiazol-2-yl)cyclohexyl) carbamate (245 mg) as a white solid.

MS m/z (ESI): 512.9 $[M+H]^+$;

Step 9: Synthesis of isopropyl trans-(4-(5-(4-((5-methyl-1-(tetrahydro-2H-pyran-2-yl)-1H-pyrazol-3-yl)amino)-2-(1-oxophosphetan-1-yl)phenyl)thiazol-2-yl)cyclohexyl)carbamate

Isopropyl trans-(4-(5-(4-bromo-2-(1-oxophosphino-1-yl)phenyl)thiazol-2-yl)cyclohexyl) carbamate (100 mg, 196 μmol) and 5-methyl-1-tetrahydropyran-2-yl-pyrazol-3-amine (70.8 mg, 392 μmol) were dissolved in anhydrous tetrahydrofuran (4 mL). Under the protection of nitrogen, methanesulfonato(2-dicyclohexylphosphino)-3,6-dimethoxy-2,4,6-triisopropyl-1,1-biphenyl)(2-amino-1,1-biphenyl-2-yl)palladium (II) (17.7 mg, 19.6 μmol) and potassium acetate (57.6 mg, 586 μmol) were added. The reaction solution appeared as a black suspension, and was stirred and reacted at 100°C for 2 hours. The completion of the reaction was detected by LCMS. The reaction solution was concentrated to dryness under reduced pressure, added with water (5 mL), and extracted with ethyl acetate (9 mL) three times. The organic layer was dried over anhydrous magnesium sulfate and subjected to suction filtration. The filtrate was concentrated to dryness under reduced pressure. The resultant was purified by thin-layer chromatography (dichloromethane:methanol = 8:1) to obtain the compound isopropyl trans-(4-(5-(4-((5-methyl-1-(tetrahydro-2H-pyran-2-yl)-1H-pyrazol-3-yl)amino)-2-(1-oxophosphetan-1-yl)phenyl)thiazol-2-yl)cyclohexyl)carbamate (80.0 mg) as a white solid.

MS m/z (ESI): 612.1 $[M+H]^+$;

Step 10: Synthesis of isopropyl trans-(4-(5-(4-((5-methyl-1H-pyrazol-3-yl)amino)-2-(1-oxophosphetan-1-yl)phenyl)thiazol-2-yl)cyclohexyl)carbamate

Isopropyl trans-(4-(5-(4-((5-methyl-1-(tetrahydro-2H-pyran-2-yl)-1H-pyrazol-3-yl) amino)-2-(1-oxophosphetan-1-yl)phenyl)thiazol-2-yl)cyclohexyl)carbamate (80.0 mg, 131 μmol) was dissolved in anhydrous methanol (2 mL), and p-toluenesulfonic acid monohydrate (113 mg, 654 μmol) was added. The reaction solution appeared as a black suspension, and was stirred and reacted at 50°C for 2 hours. The completion of the reaction was detected by LCMS. The reaction solution was concentrated to dryness under reduced pressure, added with saturated sodium carbonate solution (5 mL), and extracted with dichloromethane (9 mL). The organic layer was dried over anhydrous magnesium sulfate and subjected to suction filtration. The filtrate was concentrated to dryness under reduced pressure. The resultant was purified by preparative liquid chromatography (YMC-Actus Triart C18 column, 5 μm silica, 30 mm in diameter, 150 mm in length; using decreasingly polar mixtures of water (containing 0.225% formic acid) and acetonitrile (30% to 50%) as eluents) to obtain the compound isopropyl trans-(4-(5-(4-((5-methyl-1H-pyrazol-3-yl)amino)-2-(1-oxophosphetan-1-yl)phenyl)thiazol-2-y l)cyclohexyl)carbamate (36.77 mg) as a white solid.

MS m/z (ESI): 528.1 $[M+H]^+$;

$^1$H NMR (400MHz, DMSO-d6) δ 11.82 (br s, 1H), 8.84 (s, 1H), 8.03 (br d, J = 14.6 Hz, 1H), 7.85 (s, 1H), 7.55 (br d, J = 8.5 Hz, 1H), 7.36 (dd, J = 4.6, 8.4 Hz, 1H), 7.02 (br d, J = 7.8 Hz, 1H), 6.84-6.46 (m, 1H), 5.64 (s, 1H), 4.74 (td, J = 6.3, 12.5 Hz, 1H), 3.32-3.25 (m, 1H), 2.98-2.84 (m, 1H), 2.46-2.40 (m, 1H), 2.49-2.39 (m, 1H), 2.31-2.22 (m, 2H), 2.20 (s, 3H), 2.11 (br d, J = 11.3 Hz, 2H), 2.02-1.77 (m, 3H), 1.64-1.47 (m, 2H), 1.41-1.26 (m, 2H), 1.16 - 1.05 (m, 6H), 1.23-1.06 (m, 1H).

Example 104: Synthesis of isopropyl trans-(4-(5-(4-((1-(2-fluoroethyl)-1H-pyrazol-4-yl)amino)-2-(1-oxophosphetan-1-yl)phenyl)thiazol-2-yl)cyclohexyl)carbamate

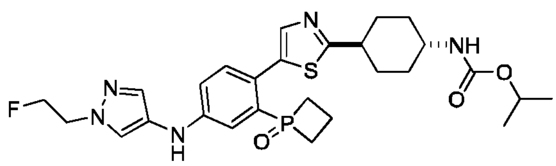

Synthetic Method

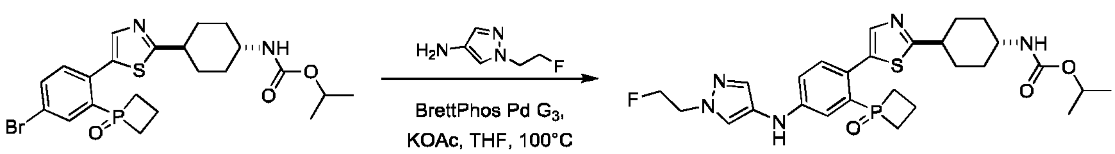


1

Isopropyl trans-(4-(5-(4-bromo-2-(1-oxophosphetan-1-yl)phenyl)thiazol-2-yl)cyclohexyl) carbamate (50 mg, 97.8 μmol) and 1-(2-fluoroethyl)-1H-pyrazol-4-amine (25.3 mg, 195 μmol) were dissolved in tetrahydrofuran (4 mL). Under the protection of nitrogen, methanesulfonato(2-dicyclohexylphosphino)-3,6-dimethoxy-2,4,6-triisopropyl-1,1-biphenyl)(2 -amino-1,1-biphenyl-2-yl)palladium (II) (8.86 mg, 9.78 μmol) and potassium acetate (28.8 mg, 293 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.225% formic acid) and acetonitrile (38% to 58%) as eluents) to obtain the compound isopropyl trans-(4-(5-(4-((1-(2-fluoroethyl)-1H-pyrazol-4-yl)amino)-2-(1-oxophosphetan-1-yl)phenyl)thiazol-2-yl)cyclohexyl)carbamate (44.04 mg).

MS m/z (ESI):560.0 $[M+H]^+$;

$^1$H NMR (400MHz, DMSO-d6) δ 8.08 (s, 1H), 7.87-7.78 (m, 2H), 7.46 (s, 1H), 7.37-7.21 (m, 2H), 7.08-6.92 (m, 2H), 4.83 (t, J = 4.8 Hz, 1H), 4.78-4.69 (m, 2H), 4.48-4.33 (m, 2H), 3.30 (br s, 1H), 2.97-2.83 (m, 1H), 2.45-2.34 (m, 2H),

2.29-2.16 (m, 2H), 2.10 (br d, J = 11.6 Hz, 2H), 1.90 (br d, J = 10.1 Hz, 1H), 2.01-1.77 (m, 1H), 1.62-1.47 (m, 2H), 1.40-1.25 (m, 2H), 1.16 - 1.05 (m, 7H)

Example 105: Synthesis of isopropyl trans-N-[4-[5-[4-[(5-methyl-1H-pyrazol-3-yl) amino]-2-(oxetan-3-ylsulfonimidoyl)phenyl]thiazol-2-yl]cyclohexyl]carbamate

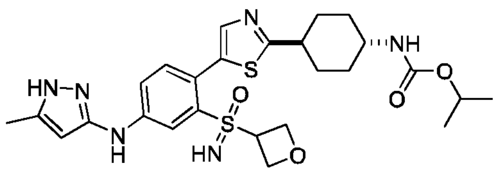

Synthetic Method

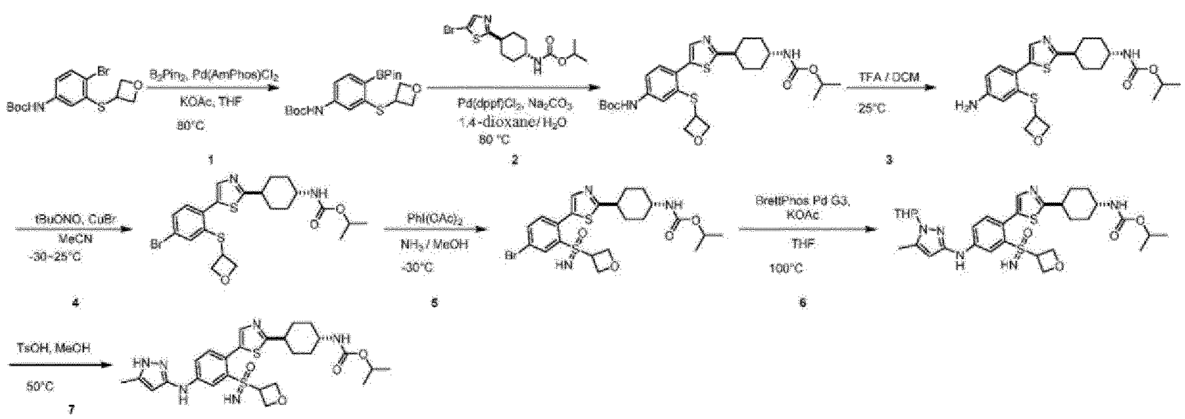


Step 1: Synthesis of tert-butyl N-[3-(oxetan-3-ylsulfanyl)-4-(4,4,5,5-tetramethyl-1,3,2-dioxaborolan-2-yl)phenyl]carbamate

Tert-butyl N-[4-bromo-3-(oxetan-3-ylsulfanyl)phenyl]carbamate (10.0 g, 27.8 mmol) and bis(pinacolato)diboron (28.2 g, 111 mmol) were dissolved in tetrahydrofuran (200 mL). Under the protection of nitrogen, dichlorobis[di-tert-butyl-(4-dimethylaminophenyl)phosphine] palladium (II) (1.97 g, 2.78 mmol) and potassium acetate (5.45 g, 55.5 mmol) were added. The reaction solution was stirred and reacted at 80°C for 16 hours. The completion of the reaction was detected by LCMS. The reaction solution was concentrated to dryness under reduced pressure and purified by column chromatography (silica, petroleum ether:ethyl acetate = 10:1) to obtain tert-butyl N-[3-(oxetan-3-ylsulfanyl)-4-(4,4,5,5-tetramethyl-1,3,2-dioxaborolan-2-yl)phenyl]carbamate as a yellow substance (130 mg).

MS m/z (ESI): 430.1 $[M+Na]^+$;

Step 2: Synthesis of isopropyl trans-N-[4-[5-[4-(tert-butoxycarbonylamino)-2-(oxetan-3-ylsulfanyl)phenyl]thiazol-2-yl]cyclohexyl]carbamate

Tert-butyl N-[3-(oxetan-3-ylsulfanyl)-4-(4,4,5,5-tetramethyl-1,3,2-dioxaborolan-2-yl) phenyl]carbamate (5.50 g, 13.5 mmol) and isopropyl trans-N-[4-(5-bromothiazol-2-yl) cyclohexyl]carbamate (4.26 g, 12.3 mmol) were dissolved in water (10 mL) and dioxane (100 mL). Thereafter, [1,1-bis(diphenylphosphino)ferrocene]dichloropalladium - dichloromethane (1.00 g, 1.23 mmol) and sodium carbonate (2.60 g, 24.6 mmol) were added under the condition where nitrogen gas was introduced. The reaction solution was stirred and reacted at 80°C for 16 hours. The completion of the reaction was detected by LCMS. The reaction solution was concentrated to dryness under reduced pressure and purified by column chromatography (silica, dichloromethane:methanol = 10:1) to obtain isopropyl trans-N-[4-[5-[4-(tert-butoxycarbonylamino)-2-(oxetan-3-ylsulfanyl)phenyl]thiazol-2-yl]cyclohexyl]carbamate as a white substance (2.0 g).

MS m/z (ESI): 548.2 $[M+H]^+$;

Step 3: Synthesis of isopropyl trans-N-[4-[5-[4-amino-2-(oxetan-3-ylsulfanyl)phenyl] thiazol-2-yl]cyclohexyl]carbamate

Isopropyl trans-N-[4-[5-[4-(tert-butoxycarbonylamino)-2-(oxetan-3-ylsulfanyl)phenyl] thiazol-2-yl]cyclohexyl]carbamate (80.0 mg, 138 μmol) was dissolved in dichloromethane (30 mL), and trifluoroacetic acid (15.4 g, 135 mmol, 10 mL) was added. The reaction solution was stirred and reacted at 25°C for 2 hours. The completion of the reaction was detected by LCMS. The reaction solution was concentrated to dryness under reduced pressure. The resultant was added with 30 mL of saturated sodium carbonate solution, and 90 mL of dichloromethane (30 mL × 3) was added to the reaction solution. The organic phase was dried over anhydrous sodium sulfate and filtered. The filtrate was concentrated to dryness under reduced pressure to obtain isopropyl trans-N-[4-[5-[4-amino-2-(oxetan-3-ylsulfanyl)phenyl]thiazol-2-yl] cyclohexyl]carbamate as a yellow solid compound (1.80 g, crude product).

MS m/z (ESI): 448.2 $[M+H]^+$;

Step 4: Synthesis of isopropyl trans-N-[4-[5-[4-bromo-2-(oxetan-3-ylsulfanyl)phenyl] thiazol-2-yl]cyclohexyl]carbamate

Isopropyl trans-N-[4-[5-[4-amino-2-(oxetan-3-ylsulfanyl)phenyl]thiazol-2-yl]cyclohexyl] carbamate (1.0 g, 2.23 mmol) was dissolved in acetonitrile (20 mL), and tert-butyl nitrite (691 mg, 6.70 mmol, 797 μL) was added at -30°C. The reaction solution was stirred and reacted at 25°C for 1 hour. Cuprous bromide (801.19 mg, 5.59 mmol) was added into the reaction solution at -30°C. The reaction solution was stirred and reacted at 25°C for 15 hours. The completion of the reaction was detected by LCMS. The reaction solution was concentrated to dryness under reduced pressure. The resultant was added with 100 mL of saturated ammonium chloride solution, and 300 mL of dichloromethane (100 mL × 3) was added to the reaction solution. The organic phase was dried over anhydrous sodium sulfate and filtered. The filtrate was concentrated to dryness under reduced pressure and purified by column chromatography (silica, dichloromethane:methanol = 10:1) to obtain isopropyl trans-N-[4-[5-[4-bromo-2-(oxetan-3-ylsulfanyl)phenyl]thiazol-2-yl]cyclohexyl]carbamate as a white substance (120 mg).

MS m/z (ESI): 511.1 $[M+H]^+$;

Step 5: Synthesis of isopropyl trans-N-[4-[5-[4-bromo-2-(oxetan-3-ylsulfonimidoyl) phenyl]thiazol-2-yl]cyclohexyl]carbamate

Isopropyl trans-N-[4-[5-[4-bromo-2-(oxetan-3-ylsulfanyl)phenyl]thiazol-2-yl]cyclohexyl] carbamate (50 mg, 97.8 μmol) was dissolved in a solution of ammonia in methanol (7M, 3 mL), and iodobenzene diacetate (472 mg, 1.47 mmol) was added. The reaction solution was stirred and reacted at -30°C for 2 hours. The completion of the reaction was detected by LCMS. The reaction solution was concentrated to dryness under reduced pressure. The resultant was purified by thin-layer chromatography (silica, dichloromethane:methanol = 10:1) to obtain isopropyl trans-N-[4-[5-[4-bromo-2-(oxetan-3-ylsulfonimidoyl)phenyl]thiazol-2-yl]cyclohexyl] carbamate as a yellow solid (20.0 mg).

MS m/z (ESI): 542.1 $[M+H]^+$;

Step 6: Synthesis of isopropyl trans-N-[4-[5-[4-[(5-methyl-1-tetrahydropyran-2-yl-pyrazol-3-yl)amino]-2-(oxetan-3-ylsulfonimidoyl)phenyl]thiazol-2-yl]cyclohexyl]carbamate

Isopropyl trans-N-[4-[5-[4-bromo-2-(oxetan-3-ylsulfonimidoyl)phenyl]thiazol-2-yl] cyclohexyl]carbamate (20.0 mg, 36.9 μmol) and 5-methyl-1-tetrahydropyran-2-yl-pyrazol-3-amine (7.35 mg, 40.6 μmol) were dissolved in tetrahydrofuran (2 mL), and methanesulfonato(2-dicyclohexylphosphino)-3,6-dimethoxy-2,4,6-triisopropyl-1,1-biphenyl)(2 -amino-1,1-biphenyl-2-yl)palladium (II) (3.34 mg, 3.69 μmol) and potassium acetate (7.24 mg, 73.7 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure and then purified by thin-layer chromatography (silica, dichloromethane:methanol = 10:1) to obtain isopropyl trans-N-[4-[5-[4-[(5-methyl-1-tetrahydropyran-2-yl-pyrazol-3-yl)amino]-2-(oxetan-3-ylsulfonimidoyl)phenyl]thiazol-2-yl]cyclohexyl]carbamate as a yellow product (20.0 mg).

MS m/z (ESI):643.3 $[M+H]^+$;

Step 7: Synthesis of isopropyl trans-N-[4-[5-[4-[(5-methyl-1H-pyrazol-3-yl)amino]-2-(oxetan-3-ylsulfonimidoyl)phenyl]thiazol-2-yl]cyclohexyl]carbamate

Isopropyl trans-N-[4-[5-[4-[(5-methyl-1-tetrahydropyran-2-yl-pyrazol-3-yl)amino]-2-(oxetan-3-ylsulfonimidoyl)phenyl]thiazol-2-yl]cyclohexyl]carbamate (20.0 mg, 31.1 μmol) was dissolved in methanol (2 mL), and p-toluenesulfonic acid monohydrate (26.8 mg, 156 μmol) was added. The reaction solution was stirred and reacted at 50°C for 1

hour. The completion of the reaction was detected by LCMS. The reaction solution was concentrated to dryness under reduced pressure. The reaction solution was added with 9 mL of dichloromethane (3 mL × 3) and 5 mL of a saturated sodium bicarbonate solution. The organic phase was dried over anhydrous sodium sulfate and filtered. The filtrate was concentrated to dryness under reduced pressure. The resultant was purified by preparative liquid chromatography (PhenomenexGemini-NX column, 5 μm silica, 30 mm in diameter, 150 mm in length; using decreasingly polar mixtures of water (containing 0.05% aqueous ammonia and 10 mM ammonium carbonate) and acetonitrile as eluents) to obtain the compound isopropyl trans-N-[4-[5-[4-[(5-methyl-1H-pyrazol-3-yl)amino]-2-(oxetan-3-ylsulfonimidoyl)phenyl]thiazol-2-yl]cyclohexyl]carbamate (2.53 mg) as a white solid.

MS m/z (ESI): 559.0 $[M+H]^+$;

$^1H$ NMR (400MHz, DMSO-d6) δ = 11.85 (s, 1H), 9.00 (s, 1H), 8.09 (d, J = 1.8 Hz, 1H), 7.63-7.58 (m, 2H), 7.22 (d, J = 8.5 Hz, 1H), 7.02 (br d, J = 7.5 Hz, 1H), 5.64 (s, 1H), 4.77-4.71 (m, 1H), 4.69 (s, 1H), 4.64-4.60 (m, 1H), 4.57-4.51 (m, 3H), 4.39-4.32 (m, 1H), 3.31-3.27 (m, 1H), 2.94-2.86 (m, 1H), 2.19 (s, 3H), 2.12 (br d, J = 11.5 Hz, 2H), 1.91 (br d, J = 10.3 Hz, 2H), 1.61-1.50 (m, 2H), 1.39-1.28 (m, 2H), 1.16 (d, J = 6.3 Hz, 6H).

Example 106: Synthesis of isopropyl trans-N-[4-[5-[4-[[1-(2-fluoroethyl)pyrazol-4-yl] amino]-2-(oxetan-3-ylsulfonimidoyl)phenyl]thiazol-2-yl]cyclohexyl]carbamate

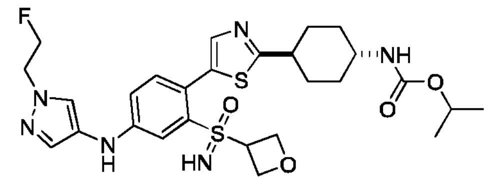

Synthetic Method

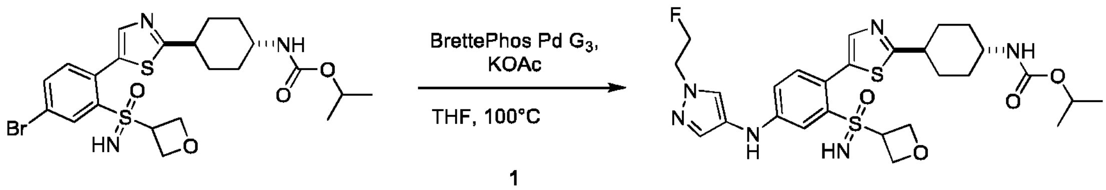


1

Isopropyl trans-N-[4-[5-[4-bromo-2-(oxetan-3-ylsulfonimidoyl)phenyl]thiazol-2-yl] cyclohexyl]carbamate (30 mg, 55.3 μmol) and 1-(2-fluoroethyl)pyrazol-4-amine (7.86 mg, 60.8 μmol) was dissolved in tetrahydrofuran (2 mL), and methanesulfonato(2-dicyclohexylphosphino)-3,6-dimethoxy-2,4,6-triisopropyl-1,1-biphenyl)(2-amino-1,1-biphenyl -2-yl)palladium (II) (5.01 mg, 5.53 μmol) and potassium acetate (5.43 mg, 55.3 μmol) were added. The reaction solution was stirred and reacted at 100°C for 2 hours. The completion of the reaction was detected by LCMS. The reaction solution was added with 9 mL of ethyl acetate (3 mL × 3) and 5 mL of water. The organic phase was dried over anhydrous sodium sulfate and filtered. The filtrate was concentrated to dryness under reduced pressure. The resultant was purified by preparative liquid chromatography (YMC-ActusTriartC18 column, 5 μm silica, 30 mm in diameter, 150 mm in length; using decreasingly polar mixtures of water (containing 0.05% aqueous ammonia) and acetonitrile as eluents) to obtain isopropyl trans-N-[4-[5-[4-[[1-(2-fluoroethyl)pyrazol-4-yl]amino]-2-(oxetan-3-ylsulfonimidoyl)phenyl] thiazol-2-yl]cyclohexyl]carbamate as a white solid compound (1.02 mg).

MS m/z (ESI): 591.0 $[M+H]^+$;

$^1H$ NMR (400MHz, DMSO-d6) δ = 8.29 (s, 1H), 7.80 (s, 1H), 7.60 (s, 1H), 7.55 (d, J = 2.5 Hz, 1H), 7.47 (s, 1H), 7.18 (d, J = 8.5 Hz, 1H), 7.03 (br d, J = 7.5 Hz, 1H), 6.93 (dd, J = 2.6, 8.4 Hz, 1H), 4.84 (t, J = 4.6 Hz, 1H), 4.77 (s, 1H), 4.76-4.70 (m, 2H), 4.61-4.56 (m, 1H), 4.53-4.47 (m, 3H), 4.45 (t, J = 4.8 Hz, 1H), 4.38 (t, J = 4.9 Hz, 1H), 4.35-4.30 (m, 1H), 3.31-3.29 (m, 1H), 2.94-2.86 (m, 1H), 2.18-2.07 (m, 2H), 1.97-1.87 (m, 2H), 1.61-1.50 (m, 2H), 1.40-1.28 (m, 2H), 1.17 (d, J = 6.0 Hz, 6H).

Example 107: Synthesis of isopropyl (2-(2-(cyclopropylsulfonyl)-4-((5-methyl-1H-pyrazol-3-yl)amino)phenyl)-4,5,6,7-tetrahydrobenzo[d]thiazol-6-yl)carbamate

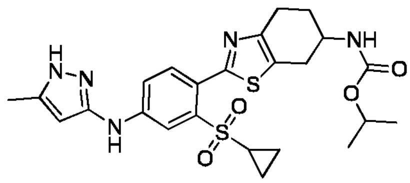

Synthetic Method

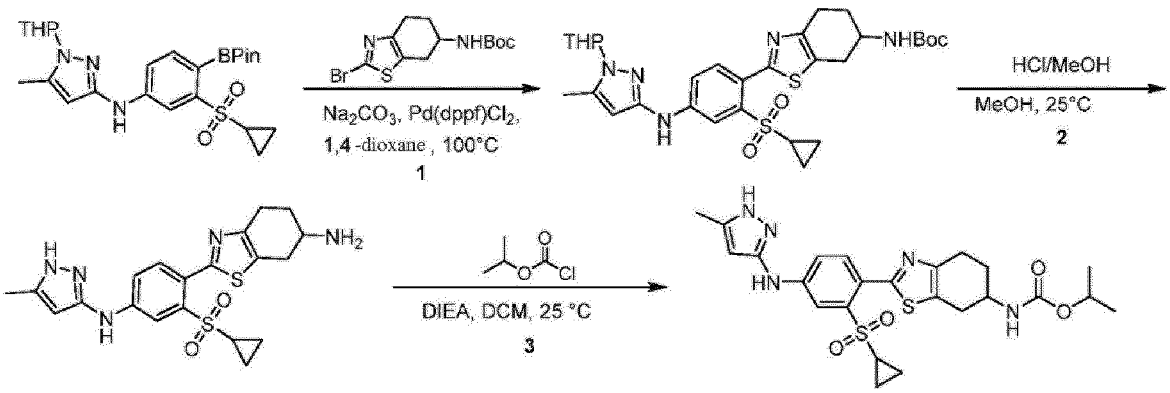


Step 1: Synthesis of tert-butyl (2-(2-(cyclopropylsulfonyl)-4-((5-methyl-1-(tetrahydro-2H-pyran-2-yl)-1H-pyrazol-3-yl)amino)phenyl)-4,5,6,7-tetrahydrobenzo[d]thiazol-6-yl)carbamate

N-(3-(cyclopropylsulfonyl)-4-(4,4,5,5-tetramethyl-1,3,2-dioxaborolan-2-yl)phenyl)-5-methyl-1-(tetrahydro-2H-pyran-2-yl)-1H-pyrazol-3-amine (402 mg, 825 μmol) and tert-butyl (2-bromo-4,5,6,7-tetrahydrobenzo[d]thiazol-6-yl)carbamate (250 mg, 750 μmol) were dissolved in dioxane (6 mL), and [1,1-bis(diphenylphosphino)ferrocene]dichloropalladium - dichloromethane (61.3 mg, 75.0 μmol) and an aqueous sodium carbonate solution (2M, 1 mL) were added. The reaction solution was stirred and reacted at 100°C for 2 hours. The completion of the reaction was detected by LCMS. The reaction solution was concentrated to dryness under reduced pressure and then purified by column chromatography (silica, petroleum ether:tetrahydrofuran = 2:1) to obtain the compound tert-butyl (2-(2-(cyclopropylsulfonyl)-4-((5-methyl-1-(tetrahydro-2H-pyran-2-yl)-1H-pyrazol-3-yl)amino )phenyl)-4,5,6,7-tetrahydrobenzo[d]thiazol-6-yl)carbamate (420 mg) as a white solid.

MS m/z (ESI): 614.5 $[M+H]^+$;

Step 2: Synthesis of 2-(2-(cyclopropylsulfonyl)-4-((5-methyl-1H-pyrazol-3-yl)amino) phenyl)-4,5,6,7-tetrahydrobenzo[d]thiazol-6-amine

Tert-butyl (2-(2-(cyclopropylsulfonyl)-4-((5-methyl-1-(tetrahydro-2H-pyran-2-yl)-1H-pyrazol-3-yl)amino)phenyl)-4,5,6,7-tetrahydrobenzo[d]thiazol-6-yl)carbamate (420 mg, 534 μmol) was dissolved in a methanol solution (3 mL), and hydrochloric acid/methanol (4M, 3 mL) was added. The reaction solution was stirred and reacted at 25°C for 2 hours. The completion of the reaction was detected by LCMS. The reaction solution was concentrated to dryness under reduced pressure, and aqueous ammonia was added to adjust the pH of the mixture to 7, so as to obtain the compound 2-(2-(cyclopropylsulfonyl)-4-((5-methyl-1H-pyrazol-3-yl)amino)phenyl)-4,5,6,7-tetrahydrobenzo[d]thiazol-6-amine (250 mg, crude product) as a white solid.

MS m/z (ESI): 430.1 $[M+H]^+$;

Step 3: Synthesis of isopropyl (2-(2-(cyclopropylsulfonyl)-4-((5-methyl-1H-pyrazol-3-yl)amino)phenyl)-4,5,6,7-tetrahydrobenzo[d]thiazol-6-yl)carbamate

2-(2-(Cyclopropylsulfonyl)-4-((5-methyl-1H-pyrazol-3-yl)amino)phenyl)-4,5,6,7-tetrahydrobenzo[d]thiazol-6-amine (250 mg, 582 μmol) was dissolved in dichloromethane (3 mL), and isopropyl chloroformate (71.3 mg, 582 μmol, 80.8 μL) and N,N-diisopropylethylamine (226 mg, 1.75 mmol, 304 μL) were added. The reaction solution was stirred and reacted at 25°C for 1 hour. The completion of the reaction was detected by LCMS. The reaction solution was concentrated to dryness under reduced pressure, and aqueous ammonia was added to adjust the pH of the mixture to 8. The reaction solution was added with 9 mL of dichloromethane (3 mL × 3) and 5 mL of water. The organic phase was dried over anhydrous sodium sulfate and filtered. The filtrate was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (38% to 58%) as eluents) to obtain isopropyl (2-(2-(cyclopropylsulfonyl)-4-((5-methyl-1H-pyrazol-3-yl)amino)phenyl)-4,5,6,7-tetrahydrobenzo[d]thiazol-6-yl)carbamate as a white solid compound (71.0 mg).

MS m/z (ESI): 516.0 $[M+H]^+$;

$^1H$ NMR (400MHz, DMSO-d6) δ = 11.91 (br s, 1H), 9.13 (s, 1H), 8.01 (br s, 1H), 7.66 (br d, J = 8.8 Hz, 1H), 7.45 (d, J = 8.5 Hz, 1H), 7.31 (br d, J = 7.0 Hz, 1H), 5.64 (s, 1H), 4.77 (td, J = 6.1, 12.4 Hz, 1H), 3.80 (br s, 1H), 3.69-3.59 (m, 1H), 3.09 (br dd, J = 4.6, 16.2 Hz, 1H), 2.91-2.81 (m, 1H), 2.80-2.71 (m, 1H), 2.71-2.63 (m, 1H), 2.19 (s, 3H), 2.01 (br d, J = 14.3 Hz, 1H), 1.85-1.70 (m, 1H), 1.19 - 1.08 (m, 6H), 1.11-0.98 (m, 4H).

Example 108: Synthesis of isopropyl 2-(2-(cyclopropylsulfonyl)-4-((5-methyl-1H-pyrazol-3-yl)amino)phenyl)-7,8-dihydro-4H-thiazolo[4,5-d]aza-6(5H)-carboxylate

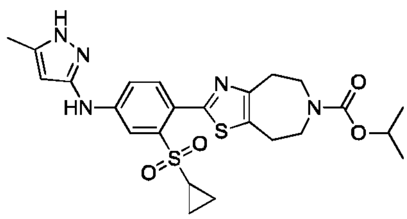

Synthetic Method

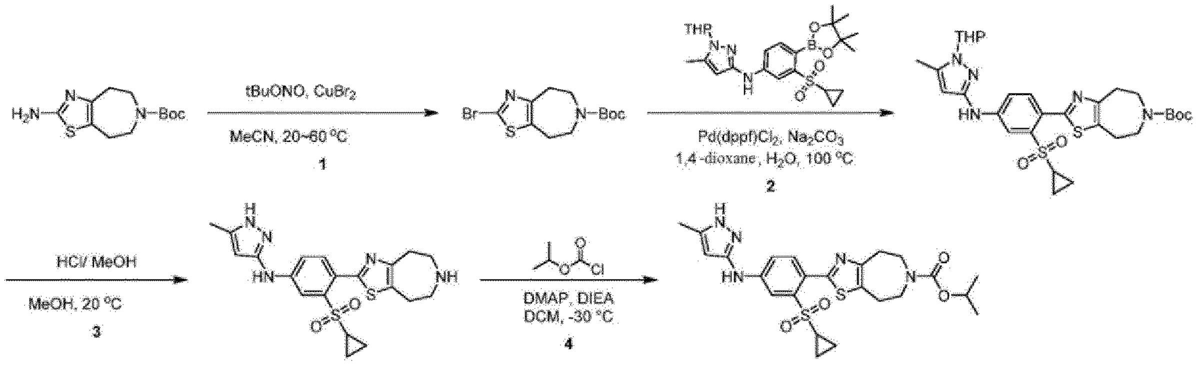


Step 1: Synthesis of tert-butyl 2-bromo-4,5,7,8-tetrahydrothiazolo[4,5-d]aza-6-carboxylate

Tert-butyl nitrite (191 mg, 1.86 mmol) and cupric bromide (414 mg, 1.86 mmol) were dissolved in anhydrous acetonitrile (6 mL). The reaction solution was stirred at 20°C for 0.5 hours. Thereafter, tert-butyl 2-amino-4,5,7,8-tetrahydrothiazolo(4,5-d)aza-6-carboxylate (0.25 g, 928 μmol) was added at 60°C. The reaction solution appeared as a blue suspension, and was stirred and reacted at 60°C for 1 hour. The completion of the reaction was detected by LCMS. The reaction solution was concentrated to dryness under reduced pressure, added with water (5 mL), and extracted with ethyl acetate (3 mL) three times. The organic layer was dried over anhydrous magnesium sulfate and subjected to

suction filtration. The filtrate was concentrated to dryness under reduced pressure. The resultant was purified by thin-layer chromatography (silica, petroleum ether: ethyl acetate = 4:1) to obtain the compound tert-butyl 2-bromo-4,5,7,8-tetrahydrothiazolo[4,5-d]aza-6-carboxylate as a yellow oily liquid.

MS m/z (ESI): 333.0; 335.0 $[M+H]^+$;

Step 2: Synthesis of tert-butyl 2-(2-(cyclopropylsulfonyl)-4-((5-methyl-1-(tetrahydro-2H-pyran-2-yl)-1H-pyrazol-3-yl)amino)phenyl)-7,8-dihydro-4H-thiazolo[4,5-d]azepine-6(5H)-carboxylate

Tert-butyl 2-bromo-4,5,7,8-tetrahydrothiazolo[4,5-d]aza-6-carboxylate (0.13 g, 390 μmol), N-(3-(cyclopropylsulfonyl)-4-(4,4,5,5-tetramethyl-1,3,2-dioxaborolan-2-yl)phenyl)-5-methyl-1 -(tetrahydro-2H-pyran-2-yl)-1H-pyrazol-3-amine (228 mg, 468 μmol) and sodium carbonate (82.6 mg, 780 μmol) were dissolved in water (0.5 mL) and 1,4-dioxane (2.5 mL). Under the protection of nitrogen, [1,1-bis(diphenylphosphino)ferrocene]dichloropalladium - dichloromethane (31.8 mg, 39.0 μmol) was added. The reaction solution appeared as a brown suspension, and was stirred and reacted at 100°C for 16 hours. The completion of the reaction was detected by LCMS. The reaction solution was filtered and concentrated to dryness under reduced pressure. The resultant was added with water (10 mL), and the mixture was extracted with dichloromethane (10 mL) three times. The organic layer was dried over anhydrous magnesium sulfate and subjected to suction filtration. The filtrate was concentrated to dryness under reduced pressure. The resultant was purified by thin-layer chromatography (silica, petroleum ether:ethyl acetate = 4:1) to obtain tert-butyl 2-(2-(cyclopropylsulfonyl)-4-((5-methyl-1-(tetrahydro-2H-pyran-2-yl)-1H-pyrazol-3-yl)amino)phenyl)-7,8-dihydro-4H-thiazolo [4,5-d]azepine-6(5H)-carboxylate as the product (0.19 g), which was a yellow oily liquid.

MS m/z (ESI): 614.1 $[M+H]^+$;

Step 3: Synthesis of N-(3-(cyclopropylsulfonyl)-4-(5,6,7,8-tetrahydro-4H-thiazolo[4,5-d]aza-2-yl)phenyl)-5-methyl-1H-pyrazol-3-amine

Tert-butyl 2-(2-(cyclopropylsulfonyl)-4-((5-methyl-1-(tetrahydro-2H-pyran-2-yl)-1H-pyrazol-3-yl)amino)phenyl)-7,8-dihydro-4H-thiazolo[4,5-d]azepine-6(5H)-carboxylate (0.18 g, 293 μmol) was dissolved in methanol (1 mL). Hydrochloric acid/methanol (4M, 2 mL) was added at 20°C. The reaction solution appeared as a yellow solution, and was stirred and reacted at 20°C for 1 hour. The completion of the reaction was detected by LCMS. The reaction solution was concentrated to dryness under reduced pressure to obtain N-(3-(cyclopropylsulfonyl)-4-(5,6,7,8-tetrahydro-4H-thiazolo[4,5-d]aza-2-yl)phenyl)-5-methyl -1H-pyrazol-3-amine as the product (0.12 g, crude product), which was a white solid.

MS m/z (ESI): 430.1 $[M+H]^+$;

Step 4: Synthesis of isopropyl 2-(2-(cyclopropylsulfonyl)-4-((5-methyl-1H-pyrazol-3-yl)amino)phenyl)-7,8-dihydro-4H-thiazolo[4,5-d]aza-6(5H)-carboxylate

N-(3-(cyclopropylsulfonyl)-4-(5,6,7,8-tetrahydro-4H-thiazolo[4,5-d]aza-2-yl)phenyl)-5-methyl-1H-pyrazol-3-amine (0.12 g, 257 μmol), 4-dimethylaminopyridine (62.9 mg, 515 μmol) and N,N-diisopropylethylamine (332 mg, 2.58 mmol) were dissolved in anhydrous dichloromethane (3 mL). Isopropyl chloroformate (63.1 mg, 515 μmol) was added at -30°C. The reaction solution appeared as a yellow solution, and was stirred and reacted at -30°C for 1 hour. The completion of the reaction was detected by LCMS. The reaction solution was concentrated to dryness under reduced pressure. The resultant was dissolved in methanol (0.1 mL), and then the pH of the mixture was adjusted to 10 with a solution of ammonia in methanol (7M). The resultant was purified by preparative liquid chromatography (PhenomenexGeminiC18 column: 5 μm silica, 30 mm in diameter, 150 mm in length; using decreasingly polar mixtures of water (containing 0.05% aqueous ammonia) and acetonitrile as eluents) to obtain the compound isopropyl 2-(2-(cyclopropylsulfonyl)-4-((5-methyl-1H-pyrazol-3-yl)amino)phenyl)-7,8-dihydro-4H-thiazolo[4,5-d]aza-6(5H)-carboxylate (40.00 mg) as a white solid.

MS m/z (ESI): 516.1 $[M+H]^+$;

$^1$H NMR (400MHz, DMSO-d6) δ = 11.92 (br s, 1H), 9.13 (s, 1H), 8.01 (d, J = 1.6 Hz, 1H), 7.67 (dd, J = 1.8, 8.4 Hz, 1H), 7.47 (d, J = 8.5 Hz, 1H), 5.65 (s, 1H), 4.85 (spt, J = 6.2 Hz, 1H), 3.73 - 3.57 (m, 5H), 3.02 (td, J = 4.8, 15.8 Hz, 4H), 2.20 (s, 3H), 1.23 - 1.13 (m, 6H), 1.12 - 0.97 (m, 3H), 1.12 - 0.97 (m, 1H).

Example 109: Synthesis of isopropyl (3-(5-(2-(cyclopropylsulfonyl)-4-((5-methyl-1H-pyrazol-3-yl)amino)phenyl)thiazol-2-yl)-3-azabicyclo[3.1,0]hexanone-6-yl)carbamate

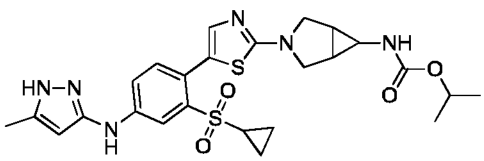

Synthetic Method

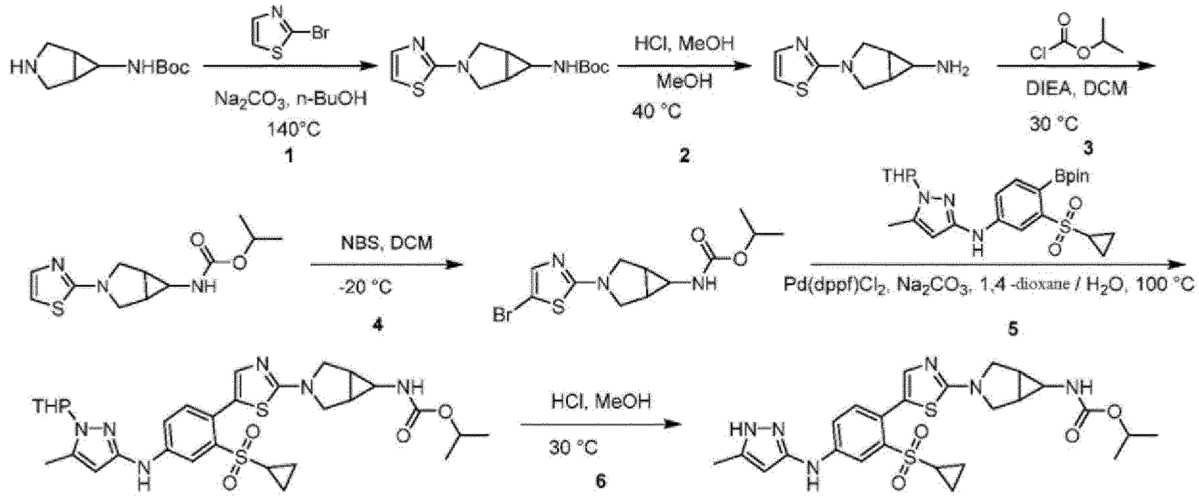


Step 1: Synthesis of tert-butyl (3-(thiazol-2-yl)-3-azabicyclo[3.1.0]hex-6-yl)carbamate

Tert-butyl 3-azabicyclo[3.1.0]hex-6-carbamate (220 mg, 1.11 mmol) and 2-bromothiazole (546 mg, 3.33 mmol) were dissolved in n-butanol (4 mL). The reaction solution was stirred and reacted at 140°C for 5 hours. The completion of the reaction was detected by LCMS. The reaction solution was concentrated to dryness under reduced pressure and then purified by column chromatography (petroleum ether:ethyl acetate = 1:3) to obtain tert-butyl (3-(thiazol-2-yl)-3-azabicyclo[3.1.0]hex-6-yl)carbamate as a white solid product (120 mg).

MS m/z (ESI): 282.1 $[M+H]^+$;

Step 2: Synthesis of 3-(thiazol-2-yl)-3-azabicyclo[3.1.0]hex-6-amine

Tert-butyl (3-(thiazol-2-yl)-3-azabicyclo[3.1.0]hex-6-yl)carbamate (78.0 mg, 277 μmol) was dissolved in methanol (3 mL), and then hydrochloric acid/methanol (4M, 2.08 mL) was added into the reaction solution. The reaction solution was stirred and reacted at 40°C for 16 hours. The completion of the reaction was detected by LCMS. The resultant was filtered and concentrated to dryness under reduced pressure to obtain crude 3-(thiazol-2-yl)-3-azabicyclo[3.1.0]hex-6-amine as a yellow oily product (50.0 mg).

MS m/z (ESI):182.0 $[M+H]^+$;

Step 3: Synthesis of isopropyl (3-(thiazol-2-yl)-3-azabicyclo[3.1.0]hex-6-yl)carbamate

3-(Thiazol-2-yl)-3-azabicyclo[3.1.0]hex-6-amine (50 mg, 276 μmol) was dissolved in dichloromethane (2 mL), and then a solution of isopropyl chloroformate in dichloromethane (2 mL, 50.7 mg, 414 μmol) and N,N-diisopropylethylamine (357 mg, 2.76 mmol) were added. The reaction solution was stirred and reacted at 30°C for 1 hour. The completion of the reaction was detected by LCMS. The reaction solution was concentrated to dryness under reduced pressure and purified by thin-layer chromatography (silica, petroleum ether:ethyl acetate = 1:1) to obtain isopropyl (3-(thiazol-2-yl)-3-azabicyclo[3.1.0]hex-6-yl)carbamate as a white solid substance (50.0 mg).

MS m/z (ESI):268.0 $[M+H]^+$;

Step 4: Synthesis of isopropyl (3-(5-bromothiazol-2-yl)-3-azabicyclo[3.1.0]hex-6-yl)carbamate

Isopropyl (3-(thiazol-2-yl)-3-azabicyclo[3.1.0]hex-6-yl)carbamate (45.0 mg, 168 μmol) was dissolved in dichlo-

romethane (1 mL), and then a solution of N-bromosuccinimide (29.9 mg, 168 μmol) in dichloromethane (1 mL) was added at -20°C. The reaction solution was stirred and reacted at -20°C for 1 hour. The completion of the reaction was detected by LCMS. The reaction solution was concentrated to dryness under reduced pressure and purified by thin-layer chromatography (silica, petroleum ether:ethyl acetate = 1:1) to obtain isopropyl (3-(5-bromothiazol-2-yl)-3-azabicyclo[3.1.0]hex-6-yl)carbamate as a white solid substance (50.0 mg).

MS m/z (ESI): 347.9 [M+H]+;

Step 5: Synthesis of isopropyl (3-(5-(2-(cyclopropylsulfonyl))-4-((5-methyl-1-(tetrahydro-2H-pyran-2-yl)-1H-pyrazol-3-yl)amino)phenyl)thiazol-2-yl)-3-azabicyclo[3.1.0] hex-6-yl)carbamate

Isopropyl (3-(5-bromothiazol-2-yl)-3-azabicyclo[3.1.0]hex-6-yl)carbamate (50.0 mg, 144 μmol) and N-[3-cyclopropylsulfonyl-4-(4,4,5,5-tetramethyl-1,3,2-dioxaborolan-2-yl)phenyl]-5-methyl-1-tetrahydropyran-2-yl-pyrazol-3-amine (84.5 mg, 173 μmol) were dissolved in dioxane (3 mL). Thereafter, [1,1-bis(diphenylphosphino)ferrocene]dichloropalladium - dichloromethane (5.90 mg, 7.22 μmol) and sodium carbonate (2M, 600 μL) were added under the condition where nitrogen gas was introduced. The reaction solution was stirred and reacted at 100°C for 16 hours. The completion of the reaction was detected by LCMS. The reaction solution was concentrated to dryness under reduced pressure. The reaction solution was added with 9 mL of dichloromethane (3 mL × 3) and 5 mL of water. The organic phase was dried over anhydrous sodium sulfate and filtered. The filtrate was concentrated to dryness under reduced pressure and purified by thin-layer chromatography (silica, petroleum ether:ethyl acetate = 1:4) to obtain isopropyl (3-(5-(2-(cyclopropylsulfonyl))-4-((5-methyl-1-(tetrahydro-2H-pyran-2-yl)-1H-pyrazol-3-yl)amino)phenyl)thiazol-2-yl)-3-azabicyclo[3.1.0]hex-6-yl)carbamate as a yellow solid substance (60.0 mg).

MS m/z (ESI):627.1 $[M+H]^+$;

Step 6: Synthesis of isopropyl (3-(5-(2-(cyclopropylsulfonyl)-4-((5-methyl-1H-pyrazol-3-yl)amino)phenyl)thiazol-2-yl)-3-azabicyclo[3.1.0]hexanone-6-yl)carbamate

Isopropyl (3-(5-(2-(cyclopropylsulfonyl))-4-((5-methyl-1-(tetrahydro-2H-pyran-2-yl)-1H-pyrazol-3-yl)amino)phenyl)thiazol-2-yl)-3-azabicyclo[3.1.0]hex-6-yl)carbamate (60.0 mg, 76.6 μmol) was dissolved in methanol (1 mL), and then hydrochloric acid/methanol (4M, 1.15 mL) was added into the reaction solution. The reaction solution was stirred and reacted at 30°C for 2 hours. The completion of the reaction was detected by LCMS. The reaction solution was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.225% formic acid) and acetonitrile (25% to 45%) as eluents) to obtain isopropyl (3-(5-(2-(cyclopropylsulfonyl)-4-((5-methyl-1H-pyrazol-3-yl)amino)phenyl)thiazol-2-yl)-3-azabicyclo[3.1.0]hexanone-6-yl) carbamate as a white solid (4.78 mg).

MS m/z (ESI):543.0 $[M+H]^+$;

$^1H$ NMR (400MHz, DMSO-d6) δ 11.84 (br s, 1H), 8.97 (s, 1H), 8.04 (br s, 1H), 7.58 (br d, J = 7.5 Hz, 1H), 7.36 (br s, 1H), 7.29 (d, J = 8.5 Hz, 1H), 7.14 (s, 1H), 5.62 (s, 1H), 4.92-4.75 (m, 1H), 3.61-3.39 (m, 4H), 2.54 (s, 1H), 2.30 (br s, 1H), 2.19 (s, 3H), 1.82 (br s, 2H), 1.17-1.05 (m, 6H), 1.02-0.83 (m, 4H).

Example 110: Synthesis of isopropyl 4-(5-(2-(cyclopropylsulfonyl)-4-((5-methyl-1H-pyrazol-3-yl)amino)phenyl)thiazol-2-yl)-1,4-diaza-1-carboxylate

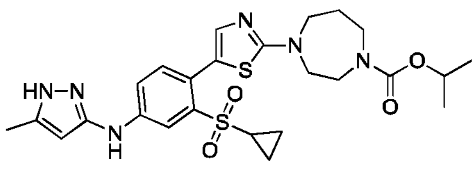

Synthetic Method

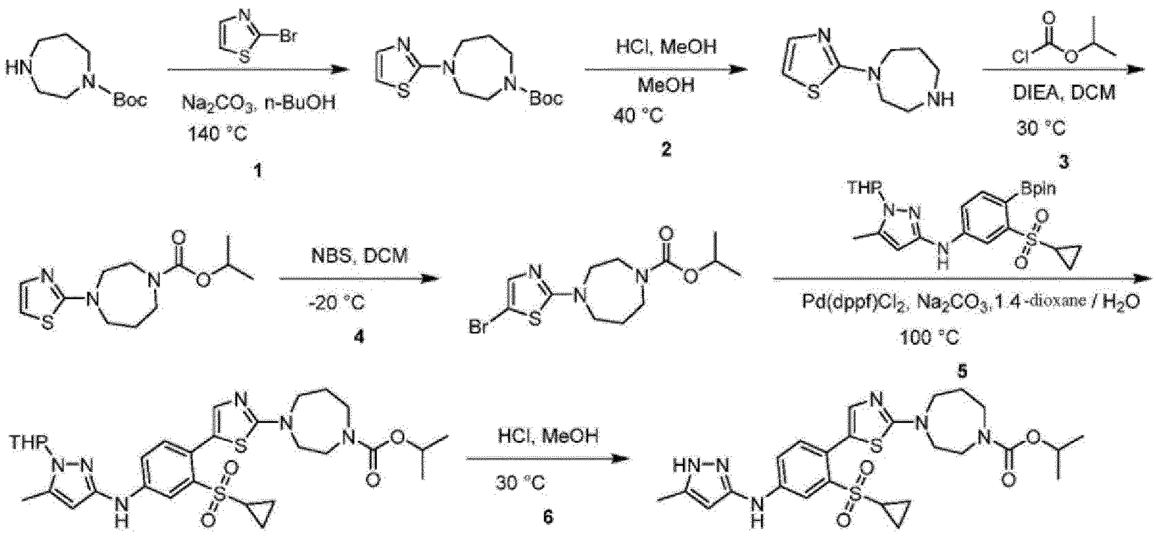


Step 1: Synthesis of tert-butyl 4-(thiazol-2-yl)-1,4-diaza-1-carboxylate

Tert-butyl 1,4-diaza-1-carboxylate (4.09 g, 24.9 mmol) and 2-bromothiazole (5 g, 24.97 mmol) were dissolved in n-butanol (50 mL). The reaction solution was stirred and reacted at 140°C for 5 hours. The completion of the reaction was detected by LCMS. The reaction solution was filtered, concentrated to dryness under reduced pressure, and then purified by column chromatography (petroleum ether:ethyl acetate = 1:3) to obtain tert-butyl 4-(thiazol-2-yl)-1,4-diaza-1-carboxylate as a yellow oily product (2.50 g).

MS m/z (ESI): 284.2 $[M+H]^+$;

Step 2: Synthesis of 2-(1,4-diaza-1-yl)thiazole

Tert-butyl 4-(thiazol-2-yl)-1,4-diaza-1-carboxylate(438 mg, 1.55 mmol) was dissolved in methanol (6 mL), and then hydrochloric acid/methanol (4M, 11.6 mL) was added into the reaction solution. The reaction solution was stirred and reacted at 40°C for 2 hours. The completion of the reaction was detected by LCMS. The resultant was filtered and concentrated to dryness under reduced pressure to obtain 2-(1,4-diaza-1-yl)thiazole as a white solid product (283 mg, crude product).

MS m/z (ESI): 184.1 $[M+H]^+$;

Step 3: Synthesis of isopropyl 4-(thiazol-2-yl)-1,4-diaza-1-carboxylate

2-(1,4-Diaza-1-yl)thiazole (283 mg, 1.55 mmol) was dissolved in dichloromethane (4 mL), and then a solution of isopropyl chloroformate (284 mg, 2.32 mmol) and N,N-diisopropylethylamine (2.00 g, 15.5 mmol) in dichloromethane (4 mL) was added. The reaction solution was stirred and reacted at 30°C for 1 hour. The completion of the reaction was detected by LCMS. The reaction solution was concentrated to dryness under reduced pressure and purified by column chromatography (silica, petroleum ether:ethyl acetate = 1:1) to obtain isopropyl 4-(thiazol-2-yl)-1,4-diaza-1-carboxylateas a white solid substance (200 mg).

MS m/z (ESI):270.1 $[M+H]^+$;

Step 4: Synthesis of isopropyl 4-(5-bromothiazol-2-yl)-1,4-diaza-1-carboxylate

Isopropyl 4-(thiazol-2-yl)-1,4-diaza-1-carboxylate(100 mg, 371 μmol) was dissolved in dichloromethane (3 mL), and then a solution of N-bromosuccinimide (66.1 mg, 371 μmol) in dichloromethane (3 mL) was added at -20°C. The reaction solution was stirred and reacted at -20°C for 1 hour. The completion of the reaction was detected by LCMS. The reaction solution was concentrated to dryness under reduced pressure and purified by thin-layer chromatography (silica, petroleum ether:ethyl acetate = 1:1) to obtain isopropyl 4-(5-bromothiazol-2-yl)-1,4-diaza-1-carboxylate as a yellow solid substance (260 mg).

MS m/z (ESI): 349.8 $[M+H]^+$;

Step 5: Synthesis of isopropyl 4-(5-(2-(cyclopropylsulfonyl))-4-((5-methyl-1-(tetrahydro-2H-pyran-2-yl)-1H-pyrazol-3-yl)amino)phenyl)thiazol-2-yl)-1,4-diaza-1-carboxylate

Isopropyl 4-(5-bromothiazol-2-yl)-1,4-diaza-1-carboxylate (130 mg, 373 μmol) and N-[3-cyclopropylsulfonyl-4-(4,4,5,5-tetramethyl-1,3,2-dioxaborolan-2-yl)phenyl]-5-methyl-1-tetrahydropyran-2-yl-pyrazol-3-amine (218 mg, 448 μmol) were dissolved in dioxane (5 mL). Thereafter, [1,1-bis(diphenylphosphino)ferrocene]dichloropalladium - dichloromethane (15.2 mg, 18.7 μmol) and sodium carbonate (2M, 1 mL) were added under the condition where nitrogen gas was introduced. The reaction solution was stirred and reacted at 100°C for 16 hours. The completion of the reaction was detected by LCMS. The reaction solution was concentrated to dryness under reduced pressure. The reaction solution was added with 30 mL of dichloromethane (10 mL × 3) and 5 mL of water. The organic phase was dried over anhydrous sodium sulfate and filtered. The filtrate was concentrated to dryness under reduced pressure and purified by column chromatography (silica, petroleum ether:tetrahydrofuran = 1:2) to obtain isopropyl 4-(5-(2-(cyclopropylsulfonyl))-4-((5-methyl-1-(tetrahydro-2H-pyran-2-yl)-1H-pyrazol-3-yl)amino)phenyl)thiazol-2-yl)-1,4-diaza-1-carboxylate as a yellow solid substance (162 mg).

MS m/z (ESI):629.1 $[M+H]^+$;

Step 6: Synthesis of isopropyl 4-(5-(2-(cyclopropylsulfonyl)-4-((5-methyl-1H-pyrazol-3-yl)amino)phenyl)thiazol-2-yl)-1,4-diaza-1-carboxylate

Isopropyl 4-(5-(2-(cyclopropylsulfonyl))-4-((5-methyl-1-(tetrahydro-2H-pyran-2-yl)-1H-pyrazol-3-yl)amino)phenyl)thiazol-2-yl)-1,4-diaza-1-carboxylate (81.0 mg, 129 μmol) was dissolved in methanol (1 mL), and then hydrochloric acid/methanol (4M, 1.93 mL) was added into the reaction solution. The reaction solution was stirred and reacted at 30°C for 2 hours. The completion of the reaction was detected by LCMS. The reaction solution was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.225% formic acid) and acetonitrile (30% to 50%) as eluents) to obtain isopropyl 4-(5-(2-(cyclopropylsulfonyl)-4-((5-methyl-1H-pyrazol-3-yl)amino)phenyl)thiazol-2-yl)-1,4-diaza-1-carboxylate as a white solid compound (4.65 mg).

MS m/z (ESI):545.0 $[M+H]^+$;

$^1H$ NMR (400MHz, DMSO-d6) δ 11.82 (br s, 1H), 8.96 (s, 1H), 8.16 (s, 1H), 8.04 (d, J = 2.0 Hz, 1H), 7.67-7.45 (m, 1H), 7.29 (d, J = 8.5 Hz, 1H), 7.12 (s, 1H), 5.62 (s, 1H), 4.81-4.58 (m, 1H), 3.63 (br s, 5H), 3.30-3.20 (m, 3H), 2.46-2.41 (m, 1H), 2.19 (s, 3H), 1.90-1.74 (m, 2H), 1.16-1.05 (m, 6H), 1.00-0.87 (m, 4H).

Example 111: Synthesis of isopropyl trans-(4-(5-(2-(cyclopropylsulfonyl)-4-(ethylcarbamoyl)phenyl)thiazol-2-yl)cyclohexyl)carbamate

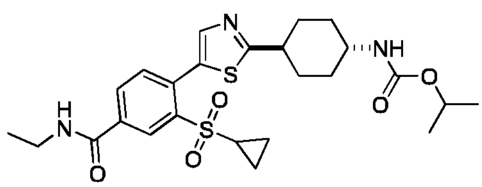

Synthetic Method

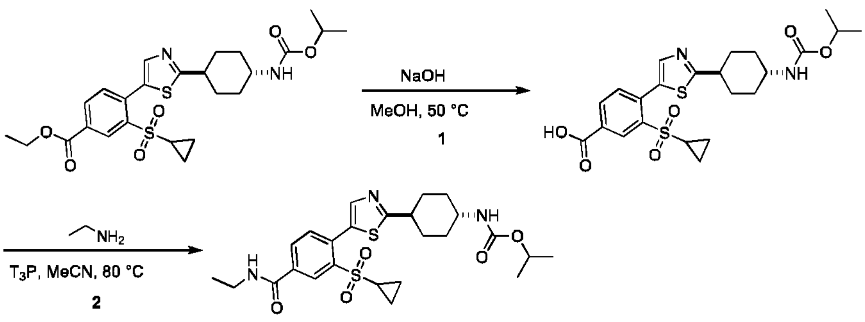


Step 1: Synthesis of trans-3-(cyclopropylsulfonyl)-4-(2-((4-((isopropoxycarbonyl) amino)cyclohexyl)thiazol-5-yl)benzoic acid

Ethyl trans-3-(cyclopropylsulfonyl)-4-(2-((4-((isopropoxycarbonyl)amino)cyclohexyl) thiazol-5-yl)benzoate (80.0 mg, 154 μmol) was dissolved in anhydrous methanol (3 mL), and an aqueous sodium hydroxide solution (2M, 230 μL) was added. The reaction solution was stirred and reacted at 50°C for 2 hours. The completion of the reaction was detected by LCMS. The reaction solution was concentrated to dryness under reduced pressure, added with water (5 mL), and extracted with ethyl acetate (9 mL) three times. Thereafter, the aqueous phase was added with dilute hydrochloric acid so that the pH of the mixture was adjusted to 3. The aqueous phase was extracted with ethyl acetate (9 mL) three times. The organic phase was dried over anhydrous sodium sulfate and filtered. The filtrate was concentrated to dryness under reduced pressure to obtain trans-3-(cyclopropylsulfonyl)-4-(2-((4-((isopropoxycarbonyl) amino)cyclohexyl)thiazol-5-yl)benzoic acid as a white solid compound (60.0 mg).

MS m/z (ESI): 493.0 $[M+H]^+$;

Step 2: Synthesis of isopropyl trans-(4-(5-(2-(cyclopropylsulfonyl)-4-(ethylcarbamoyl) phenyl)thiazol-2-yl)cyclohexyl)carbamate

Trans-3-(cyclopropylsulfonyl)-4-(2-((4-((isopropoxycarbonyl)amino)cyclohexyl)thiazol-5 -yl)benzoic acid (60.0 mg, 122 μmol) and anhydrous ethylamine (54.9 mg, 1.22 mmol) were dissolved in acetonitrile (2 mL), and tri-n-propylphosphonic anhydride (50% solution in ethyl acetate) (116 mg, 365 μmol, 108 μL) was added. The reaction solution was stirred and reacted at 80°C for 2 hours. The completion of the reaction was detected by LCMS. The reaction solution was concentrated to dryness under reduced pressure, added with a saturated sodium carbonate solution (5 mL), and extracted with dichloromethane (15 mL) three times. The organic phase was dried over anhydrous sodium sulfate and filtered. The filtrate was concentrated to dryness under reduced pressure and purified by preparative liquid chromatography (YMC-Actus Triart C18 column: 5 μm silica, 30 mm in diameter, 150 mm in length; using decreasingly polar mixtures of water (containing 0.05% aqueous ammonia) and acetonitrile (40% to 60%) as eluents) to obtain isopropyl trans-(4-(5-(2-(cyclopropylsulfonyl)-4-(ethylcarbamoyl)phenyl)thiazol-2-yl)cyclohexyl)carbamate as a white solid compound (17.5 mg).

MS m/z (ESI): 520.0 $[M+H]^+$;

$^1$H NMR (400MHz, DMSO-d6) δ = 8.85 (t, J = 5.5 Hz, 1H), 8.46 (d, J = 1.8 Hz, 1H), 8.16 (dd, J = 1.8, 8.0 Hz, 1H), 7.81 (s, 1H), 7.69 (d, J = 8.0 Hz, 1H), 7.02 (br d, J = 7.6 Hz, 1H), 4.74 (td, J = 6.3, 12.4 Hz, 1H), 3.43-3.37 (m, 1H), 3.31-3.23 (m, 2H), 3.04-2.91 (m, 1H), 2.48-2.40 (m, 1H), 2.15 (br d, J = 12.0 Hz, 2H), 1.92 (br d, J = 10.1 Hz, 2H), 1.68-1.49 (m, 2H), 1.45-1.26 (m, 2H), 1.21-1.09 (m, 9H), 1.05-0.85 (m, 4H).

Example 112: Synthesis of isopropyl trans-N-[4-[5-[4-[(5-fluoro-2-pyridyl)amino]-2-(oxetan-3-ylsulfonyl)phenyl]thiazol-2-yl]cyclohexyl]carbamate

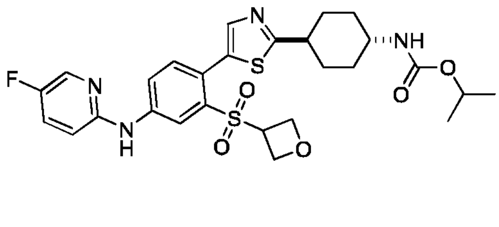

Synthetic Method

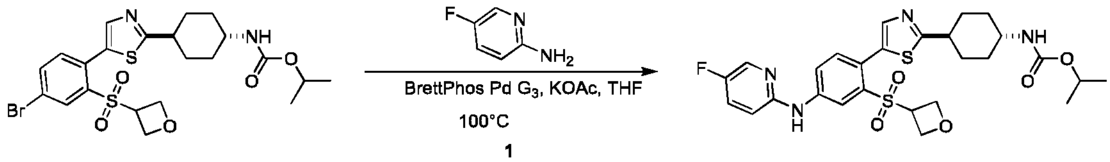


Isopropyl trans-N-[4-[5-[4-bromo-2-(oxetan-3-ylsulfonyl)phenyl]thiazol-2-yl]cyclohexyl] carbamate (60.0 mg, 110.4 μmol) and 2-amino-5-fluoropyridine (13.6 mg, 121 μmol) were dissolved in tetrahydrofuran (3 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (10.0 mg, 11.1 μmol) and potassium acetate (21.7 mg, 221 μmol) were added. The reaction solution was stirred and reacted at 100°C for 1 hour. The completion of the reaction was detected by LCMS. The reaction solution was added with 30 mL of ethyl acetate (10 mL × 3) and 10 mL of water. The organic phase was dried over anhydrous sodium sulfate and filtered. The filtrate was concentrated to dryness under reduced pressure. The resultant was purified by preparative liquid chromatography (YMC-ActusTriartC18 column, 5 μm silica, 30 mm in diameter, 150 mm in length; using decreasingly polar mixtures of water (containing 0.05% aqueous ammonia) and acetonitrile as eluents) to obtain the compound isopropyl trans-N-[4-[5-[4-[(5-fluoro-2-pyridyl)amino]-2-(oxetan-3-ylsulfonyl)phenyl]thiazol-2-yl]cyclohexyl]carbamate (8.18 mg) as a white solid.

MS m/z (ESI): 575.0 $[M+H]^+$;

$^1$H NMR (400MHz, DMSO-d6) δ = 9.82 (s, 1H), 8.37 (d, J = 2.3 Hz, 1H), 8.24 (d, J = 3.0 Hz, 1H), 8.13 (dd, J = 2.3, 8.5 Hz, 1H), 7.69-7.63 (m, 2H), 7.45 (d, J = 8.5 Hz, 1H), 7.04 (br d, J = 7.5 Hz, 1H), 6.96 (dd, J = 3.8, 9.3 Hz, 1H), 4.80-4.70 (m, 1H), 4.67-4.60 (m, 2H), 4.59-4.52 (m, 3H), 3.33-3.27 (m, 1H), 2.98-2.90 (m, 1H), 2.14 (br d, J = 12.5 Hz, 2H), 1.93 (br d, J = 10.0 Hz, 2H), 1.63-1.52 (m, 2H), 1.40-1.29 (m, 2H), 1.17 (d, J = 6.3 Hz, 6H).

Example 113: Synthesis of isopropyl trans-N-[4-[5-[4-[(6-methoxy-3-pyridyl)amino]-2-(oxetan-3-ylsulfonyl)phenyl]thiazol-2-yl]cyclohexyl]carbamate

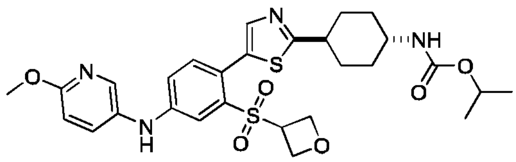

Synthetic Method

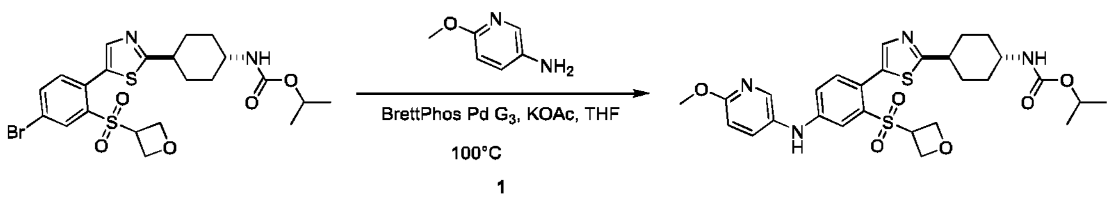


Isopropyl trans-N-[4-[5-[4-bromo-2-(oxetan-3-ylsulfonyl)phenyl]thiazol-2-yl]cyclohexyl] carbamate (60.0 mg, 110 μmol) and 6-methoxypyridin-3-amine (15.1 mg, 121 μmol) were dissolved in tetrahydrofuran (3 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (10.0 mg, 11.1 μmol) and potassium acetate (21.8 mg, 221 μmol) were added. The reaction solution was stirred and reacted at 100°C for 1 hour. The completion of the reaction was detected by LCMS. The reaction solution was added with 30 mL of ethyl acetate (10 mL × 3) and 10 mL of water. The organic phase was dried over anhydrous

sodium sulfate and filtered. The filtrate was concentrated to dryness under reduced pressure. The resultant was purified by preparative liquid chromatography (YMC-ActusTriartC18 column, 5 μm silica, 30 mm in diameter, 150 mm in length; using decreasingly polar mixtures of water (containing 0.05% aqueous ammonia) and acetonitrile as eluents) to obtain the compound isopropyl trans-N-[4-[5-[4-[(6-methoxy-3-pyridyl)amino]-2-(oxetan-3-ylsulfonyl)phenyl]thiazol-2-yl]cyclohexyl]carbamate (11.67 mg) as a white solid.

MS m/z (ESI): 587.0 $[M+H]^+$;

$^1H$ NMR (400MHz, DMSO-d6) δ = 8.72 (s, 1H), 8.08 (d, J = 2.8 Hz, 1H), 7.65-7.59 (m, 2H), 7.53 (d, J = 2.5 Hz, 1H), 7.31 (d, J = 8.3 Hz, 1H), 7.12 (dd, J = 2.6, 8.4 Hz, 1H), 7.03 (br d, J = 7.8 Hz, 1H), 6.88 (d, J = 8.8 Hz, 1H), 4.79-4.70 (m, 1H), 4.65-4.57 (m, 2H), 4.54-4.44 (m, 3H), 3.85 (s, 3H), 3.31-3.23 (m, 1H), 2.93 (s, 1H), 2.18-2.08 (m, 2H), 1.92 (br d, J = 9.8 Hz, 2H), 1.62-1.51 (m, 2H), 1.40-1.27 (m, 2H), 1.17 (d, J = 6.3 Hz, 6H).

Example 114: Synthesis of isopropyl trans-(4-(5-(4-((5-methyl-1H-pyrazol-3-yl)amino)-2-((1-methylazetidin-3-yl)sulfonyl)phenyl)thiazol-2-yl)cyclohexyl)carbamate

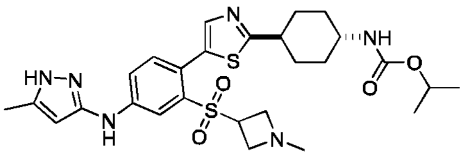

Synthetic Method

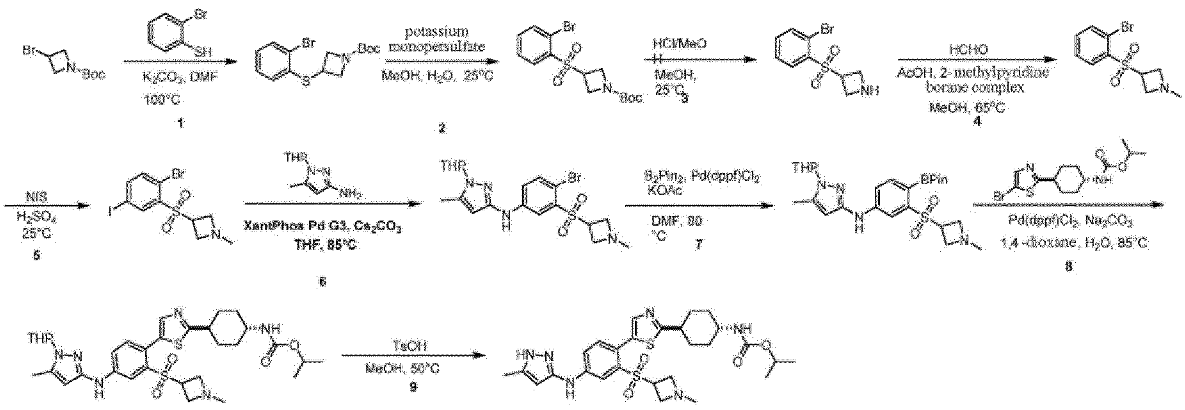


Step 1: Synthesis of tert-butyl 3-((2-bromophenyl)thio)azetidine-1-carboxylate

o-Bromothiophenol (5.07 g, 26.8 mmol, 3.15 mL) and tert-butyl 3-bromoazetidine-1-carboxylate (9.50 g, 40.2 mmol) were dissolved in N,N-dimethylformamide (100 mL), and potassium carbonate (11.1 g, 80.5 mmol) was added. The reaction solution was stirred and reacted at 100°C for 24 hours. The completion of the reaction was detected by LCMS. The reaction solution was filtered, concentrated to dryness under reduced pressure, and then purified by column chromatography (silica, petroleum ether:ethyl acetate = 10:1) to obtain tert-butyl 3-((2-bromophenyl)thio)azetidine-1-carboxylate as a white solid compound (8.74 g).

MS m/z (ESI): 287.9, 288.0 $[M+H]^+$;

Step 2: Synthesis of tert-butyl 3-((2-bromophenyl)sulfonyl)azetidine-1-carboxylate

Tert-butyl 3-((2-bromophenyl)thio)azetidine-1-carboxylate (8.70 g, 25.3 mmol) and potassium monopersulfate (109 g, 177 mmol) were dissolved in methanol (100 mL) and water (100 mL). The reaction solution was stirred and reacted at 25°C for 16 hours. The completion of the reaction was detected by LCMS. The reaction solution was filtered, concentrated to dryness under reduced pressure, and extracted with 2.4L of dichloromethane (800 mL × 3) and an aqueous sodium sulfite solution (800 mL). The organic phase was dried over anhydrous magnesium sulfate and filtered. The filtrate was concentrated to dryness under reduced pressure to obtain the compound tert-butyl 3-((2-bromophenyl)sulfonyl)azetidine-1-carboxylate as a white oily product (6.00 g, crude product).
MS m/z (ESI): 320.0; 322.0 $[M+H-t-Bu]^+$;

Step 3: Synthesis of 3-((2-bromophenyl)sulfonyl)azetidine

Tert-butyl 3-((2-bromophenyl)sulfonyl)azetidine-1-carboxylate (5.30 g, 12.0 mmol, purity: 85%) was dissolved in methanol (20 mL), and hydrochloric acid/methanol (4M, 29.9 mL) was added. The reaction solution was stirred and reacted at 25°C for 2 hours. The completion of the reaction was detected by LCMS. The reaction solution was filtered. The filtrate was concentrated to dryness under reduced pressure to obtain 3-((2-bromophenyl)sulfonyl)azetidine as a white solid compound (3.20 g, crude product).
MS m/z (ESI): 276.0, 278.0 $[M+H]^+$;

Step 4: Synthesis of 3-((2-bromophenyl)sulfonyl)-1-methylazetidine

3-((2-Bromophenyl)sulfonyl)azetidine (3.20 g, 11.6 mmol) and 2-methylpyridine borane complex (2.48 g, 23.2 mmol) were dissolved in methanol (60 mL), and formaldehyde (9.40 g, 116 mmol, 8.63 mL, purity: 37%) and glacial acetic acid (39.2 mg, 2.32 mmol, 133 μL) were added. The reaction solution was stirred and reacted at 65°C for 2 hours. The completion of the reaction was detected by LCMS. The reaction solution was concentrated to dryness under reduced pressure and then purified by column chromatography (silica, dichloromethane:methanol = 5:1) to obtain 3-((2-bromophenyl)sulfonyl)-1-methylazetidine as a white solid compound (3.00 g).
MS m/z (ESI): 290.0 $[M+H]^+$;

Step 5: Synthesis of 3-((2-bromo-5-iodophenyl)sulfonyl)-1-methylazetidine

3-((2-Bromophenyl)sulfonyl)-1-methylazetidine (2.00 g, 6.89 mmol) was dissolved in sulfuric acid (30 mL), and N-iodosuccinimide (2.79 g, 12.4 mmol) was added. The reaction solution was stirred and reacted at 25°C for 16 hours. The completion of the reaction was detected by LCMS. At 0°C, the reaction solution was added with a saturated aqueous sodium hydroxide solution so that the pH of the mixture was adjusted to 7. 80 mL of water and 300 mL of dichloromethane (100 mL × 3) were added for extraction. The organic phase was dried over anhydrous sodium sulfate and filtered. The filtrate was concentrated to dryness under reduced pressure and the resultant was slurried with ethyl acetate (8 mL) to obtain 3-((2-bromo-5-iodophenyl)sulfonyl)-1-methylazetidine as a white solid compound (800 mg).
MS m/z (ESI): 415.9, 417.9 $[M+H]^+$.

Step 6: Synthesis of N-(4-bromo-3-((1-methylazetidin-3-yl)sulfonyl)phenyl)-5-methyl-1-(tetrahydro-2H-pyran-2-yl)-1H-pyrazol-3-amine

3-((2-Bromo-5-iodophenyl)sulfonyl)-1-methylazetidine (1.30 g, 3.12 mmol) and 5-methyl-1-(tetrahydro-2H-pyran-2-yl)-1H-pyrazol-3-amine (566 mg, 3.12 mmol) were dissolved in tetrahydrofuran (40 mL), and cesium carbonate (2.04 g, 6.25 mmol) and methanesulfonato[9,9-dimethyl-4,5-bis(diphenylphosphino)xanthene][2-amino-1,1-diphenyl] palladium (II) - dichloromethane adduct (148 mg, 156 μmol) were added. Under the protection of nitrogen, the reaction solution was stirred and reacted at 85°C for 1.5 hours. The completion of the reaction was detected by LCMS. The reaction solution was filtered. The filtrate was concentrated to dryness under reduced pressure and then purified by column chromatography (silica, dichloromethane:methanol = 10:1) to obtain N-(4-bromo-3-((1-methylazetidin-3-yl) sulfonyl)phenyl)-5-methyl-1-(tetrahydro-2H-pyran-2-yl)-1H-pyrazol-3-amine as a white solid compound (900 mg).
MS m/z (ESI): 469.1 $[M+H]^+$.

Step 7: Synthesis of 5-methyl-N-(3-(((1-methylazetidin-3-yl)sulfonyl)-4-(4,4,5,5-tetramethyl-1,3,2-dioxaborolan-2-yl)phenyl)-1-(tetrahydro-2H-pyran-2-yl)-1H-pyrazol -3-amine

N-(4-bromo-3-((1-methylazetidin-3-yl)sulfonyl)phenyl)-5-methyl-1-(tetrahydro-2H-pyran -2-yl)-1H-pyrazol-3-amine (300 mg, 511 μmol, purity: 80%) and bis(pinacolato)diboron (649 mg, 2.56 mmol) were dissolved in N,N-dimethylformamide (6 mL), and potassium acetate (151 mg, 1.53 mmol) and [1,1-bis(diphenylphosphino)ferrocene]dichloropalladium - dichloromethane (41.8 mg, 51.1 μmol) were added. Under the protection of nitrogen, the reaction solution was stirred and reacted at 80°C for 2 hours. The completion of the reaction was detected by LCMS. The reaction solution was concentrated to dryness under reduced pressure and then purified by column chromatography (silica, dichloromethane:methanol = 10:1) to obtain 5-methyl-N-(3-(((1-methylazetidin-3-yl)sulfonyl)-4-(4,4,5,5-tetramethyl-1,3,2-dioxaborolan-2-yl)phenyl)-1-(tetrahydro-2H-pyran-2-yl)-1H-pyrazol-3-amine as a brown solid compound (230 mg, crude product).
MS m/z (ESI): 517.3 $[M+H]^+$;

Step 8: Synthesis of isopropyl trans-(4-(5-(4-((5-methyl-1-(tetrahydro-2H-pyran-2-yl)-1H-pyrazol-3-yl)amino)-2-((1-methylazetidin-3-yl)sulfonyl)phenyl)thiazol-2-yl)cyclohexyl )carbamate

5-Methyl-N-(3-(((1-methylazetidin-3-yl)sulfonyl)-4-(4,4,5,5-tetramethyl-1,3,2-dioxaborolan-2-yl)phenyl)-1-(tetrahydro-2H-pyran-2-yl)-1H-pyrazol-3-amine (230 mg, 134 μmol, purity: 30%) and isopropyl trans-(4-(5-bromothiazol-2-yl)cyclohexyl)carbamate (51.0 mg, 147 μmol) were dissolved in dioxane (3 mL), and an aqueous sodium carbonate solution (2M, 0.5 mL) and [1,1-bis(diphenylphosphino)ferrocene]dichloropalladium - dichloromethane (10.9 mg, 13.4 μmol) were added. Under the protection of nitrogen, the reaction solution was stirred and reacted at 85°C for 2 hours. The completion of the reaction was detected by LCMS. The reaction solution was concentrated to dryness under reduced pressure and then purified by column chromatography (silica, dichloromethane:methanol = 10:1) to obtain isopropyl trans-(4-(5-(4-((5-methyl-1-(tetrahydro-2H-pyran-2-yl)-1H-pyrazol-3-yl)amino)-2-((1-methylazetidin-3-yl)sulfonyl)phenyl)thiazol-2-yl)cyclohexyl)carbamate as a brown solid compound (80.0 mg).

MS m/z (ESI): 657.3 $[M+H]^+$;

Step 9: Synthesis of isopropyl trans-(4-(5-(4-((5-methyl-1H-pyrazol-3-yl)amino)-2-((1-methylazetidin-3-yl)sulfonyl)phenyl)thiazol-2-yl)cyclohexyl)carbamate

Isopropyl trans-(4-(5-(4-((5-methyl-1-(tetrahydro-2H-pyran-2-yl)-1H-pyrazol-3-yl) amino)-2-((1-methylazetidin-3-yl)sulfonyl)phenyl)thiazol-2-yl)cyclohexyl)carbamate (80.0 mg, 122 μmol) was dissolved in methanol (3 mL), and p-toluenesulfonic acid monohydrate (105 mg, 609 μmol) was added. The reaction solution was stirred and reacted at 50°C for 2 hours. The completion of the reaction was detected by LCMS. The reaction solution was concentrated to dryness under reduced pressure. Aqueous ammonia was added so that the pH of the mixture was adjusted to 8. The resulting mixture was extracted with water (5 mL) and dichloromethane (9 mL) three times. The organic phase was dried over anhydrous sodium sulfate and filtered. The filtrate was concentrated to dryness under reduced pressure and purified by preparative liquid chromatography (YMC-Actus Triart C18: 5 μm silica, 30 mm in diameter, 150 mm in length; using decreasingly polar mixtures of water (containing 0.05% aqueous ammonia) and acetonitrile (40% to 60%) as eluents) to obtain isopropyl trans-(4-(5-(4-((5-methyl-1H-pyrazol-3-yl)amino)-2-((1-methylazetidin-3-yl)sulfonyl)phenyl)thiazol-2-yl)cyclohexyl)carbamate as a white solid compound (6.39mg).

MS m/z (ESI): 572.9 $[M+H]^+$;

$^1$H NMR (400MHz, DMSO-d6) δ = 11.90 (br s, 1H), 9.09 (s, 1H), 8.14 (s, 1H), 7.66-7.59 (m, 2H), 7.31 (d, J = 8.5 Hz, 1H), 7.03 (br d, J = 7.8 Hz, 1H), 5.64 (s, 1H), 4.81-4.64 (m, 1H), 4.04 (s, 1H), 3.91-3.79 (m, 1H), 3.31 (br s, 2H), 3.21-3.09 (m, 2H), 2.98-2.86 (m, 1H), 2.20 (s, 3H), 2.17 (s, 3H), 2.13 (br d, J = 14.3 Hz, 2H), 1.91 (br d, J = 10.3 Hz, 2H), 1.64-1.49 (m, 2H), 1.40-1.25 (m, 2H), 1.17-1.05 (m, 6H).

Example 115: Synthesis of isopropyl trans-(4-(5-(4-((5-methyl-1H-pyrazol-3-yl) amino)-2-((tetrahydrofuran-3-yl)sulfonyl)phenyl)thiazol-2-yl)cyclohexyl)carbamate

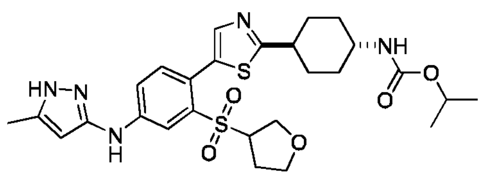

Synthetic Method

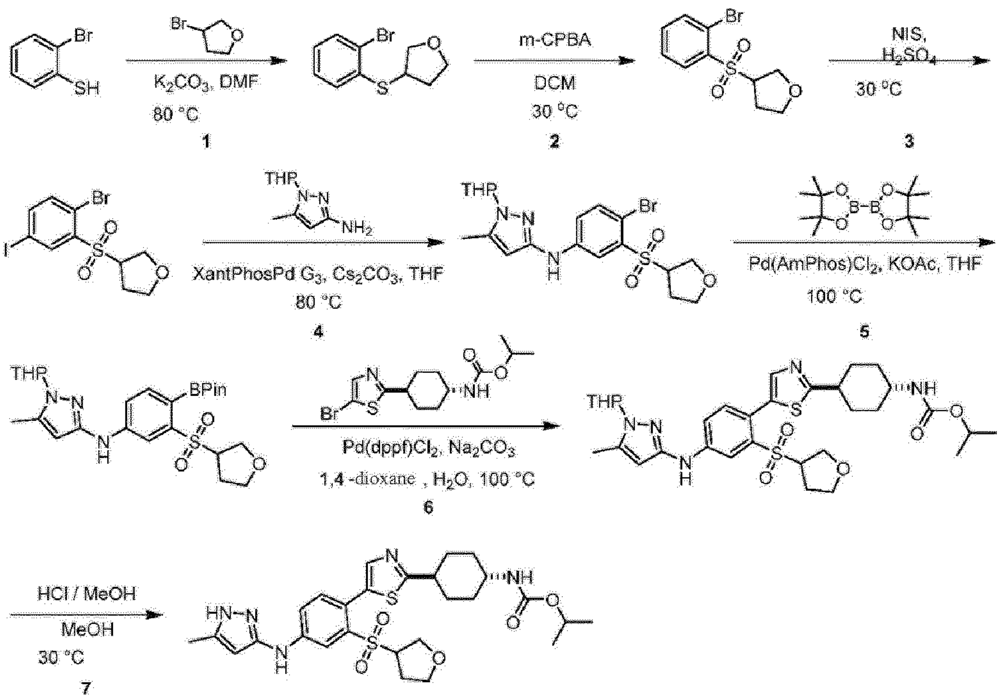


Step 1: Synthesis of 3-((2-bromophenyl)thio)tetrahydrofuran

o-Bromothiophenol (4.20 g, 22.2 mmol) and 3-bromotetrahydrofuran (5.03 g, 33.3 mmol) were dissolved in N,N-dimethylformamide (40 mL), and then potassium carbonate (9.21 g, 66.6 mmol) was added into the reaction solution. The reaction solution was stirred and reacted at 80°C for 16 hours. TLC detection showed that the reaction was complete. The reaction solution was filtered, concentrated to dryness under reduced pressure, and purified by column chromatography (silica, petroleum ether: ethyl acetate = 5:1) to obtain 3-((2-bromophenyl)thio)tetrahydrofuran as a yellow oily substance (5.30 g).

$^1$H NMR (400MHz, DMSO-d6) δ 7.62 (dd, J = 1.1, 7.9 Hz, 1H), 7.46-7.34 (m, 2H), 7.23-7.11 (m, 1H), 4.18-3.98 (m, 2H), 3.89-3.71 (m, 2H), 3.56 (dd, J = 4.4, 9.1 Hz, 1H), 2.46-2.36 (m, 1H), 1.85-1.75 (m, 1H).

Step 2: Synthesis of 3-((2-bromophenyl)sulfonyl)tetrahydrofuran

3-((2-Bromophenyl)thio)tetrahydrofuran (5.30 g, 20.4 mmol) was dissolved in dichloromethane (100 mL), and then m-chloroperoxybenzoic acid (10.7 g, 52.6 mmol) was added into the reaction solution. The reaction solution was stirred and reacted at 30°C for 16 hours. TLC detection showed that the reaction was complete. The reaction solution was concentrated to dryness under reduced pressure. A saturated sodium carbonate solution (30 mL) and dichloromethane (90 mL, 30 mL × 3) were added for extraction. The organic phase was dried over anhydrous sodium sulfate and filtered. The filtrate was concentrated to dryness under reduced pressure and then purified by column chromatography (silica, petroleum ether: ethyl acetate = 5:1) to obtain 3-((2-bromophenyl)sulfonyl)tetrahydrofuran as a white solid product (4.44 g).

$^1$H NMR (400MHz, DMSO-d6) δ 8.13-8.05 (m, 1H), 7.99-7.93 (m, 1H), 7.74-7.63 (m, 2H), 4.54-4.43 (m, 1H), 4.04-3.94 (m, 1H), 3.92-3.80 (m, 2H), 3.73-3.63 (m, 1H), 2.21-2.04 (m, 2H).

Step 3: Synthesis of 3-((2-bromo-5-iodophenyl)sulfonyl)tetrahydrofuran

3-((2-Bromophenyl)sulfonyl)tetrahydrofuran (4.44 g, 15.5 mmol) was dissolved in concentrated sulfuric acid (70 mL), and then N-iodosuccinimide (6.18 g, 27.5 mmol) was added into the reaction solution. The reaction solution was stirred and reacted at 30°C for 16 hours. TLC detection showed that the reaction was complete. The reaction solution was slowly poured into ice water (100 mL) to quench the reaction. The reaction solution was added with a saturated aqueous sodium sulfite solution (60 mL), and extracted with dichloromethane (600 mL) three times. The organic layer was dried over anhydrous magnesium sulfate and subjected to suction filtration. The filtrate was concentrated to dryness under reduced pressure. The resultant was purified by column chromatography (silica, petroleum ether:ethyl acetate = 1:1) to obtain 3-((2-bromo-5-iodophenyl)sulfonyl)tetrahydrofuran as a yellow oily substance (2.80 g).

$^1$H NMR (400MHz, DMSO-d6) δ 8.26 (d, J = 2.1 Hz, 1H), 8.05-7.95 (m, 1H), 7.71 (d, J = 8.3 Hz, 1H), 4.56-4.44 (m, 1H), 4.04-3.99 (m, 1H), 3.93-3.81 (m, 2H), 3.74-3.63 (m, 1H), 2.22-2.06 (m, 2H).

Step 4: Synthesis of N-(4-bromo-3-((tetrahydrofuran-3-yl)sulfonyl)phenyl)-5-methyl-1-(tetrahydro-2H-pyran-2-yl)-1H-pyrazol-3-amine

3-((2-Bromo-5-iodophenyl)sulfonyl)tetrahydrofuran (2.79 g, 6.68 mmol) and 5-methyl-1-tetrahydropyran-2-yl-pyrazol-3-amine (1.45 g, 8.02 mmol) were dissolved in tetrahydrofuran (60 mL). Thereafter, methanesulfonato[9,9-dimethyl-4,5-bis(diphenylphosphino)xanthene][2-amino-1,1-biphenyl]palladium (II) dichloromethane adduct (317 mg, 335 μmol) and cesium carbonate (6.53 g, 20.1 mmol) were added under the protection of nitrogen. The reaction solution was stirred and reacted at 80°C for 4 hours. The completion of the reaction was detected by LCMS. The reaction solution was concentrated to dryness under reduced pressure, added with water (10 mL), and extracted with ethyl acetate (30 mL). The organic layer was dried over anhydrous magnesium sulfate and subjected to suction filtration. The filtrate was concentrated to dryness under reduced pressure. The resultant was purified by column chromatography (silica, petroleum ether:ethyl acetate = 1:1) to obtain N-(4-bromo-3-((tetrahydrofuran-3-yl)sulfonyl)phenyl)-5-methyl-1-(tetrahydro-2H-pyran-2-yl)-1H-pyrazol-3-amine as a yellow solid substance (2.39 g).
MS m/z (ESI):470.0, 472.0 $[M+H]^+$;

Step 5: Synthesis of 5-methyl-1-(tetrahydro-2H-pyran-2-yl)-N-(3-((tetrahydrofuran-3-yl)sulfonyl)-4-(4,4,5,5-tetramethyl-1,3,2-dioxaborolan-2-yl)phenyl)-1H-pyrazol-3-amine

N-(4-Bromo-3-((tetrahydrofuran-3-yl)sulfonyl)phenyl)-5-methyl-1-(tetrahydro-2H-pyran-2-yl)-1H-pyrazol-3-amine (1.00 g, 2.13 mmol) and bis(pinacolato)diboron (1.62 g, 6.38 mmol) were dissolved in dioxane (5 mL). Thereafter, dichlorobis[di-tert-butyl-(4-dimethylaminophenyl)phosphine]palladium (II) (75.3 mg, 106 μmol) and potassium acetate (625 mg, 6.38 mmol) were added under the protection of nitrogen. The reaction solution was stirred and reacted at 100°C for 16 hours. The completion of the reaction was detected by LCMS. The reaction solution was concentrated to dryness under reduced pressure, added with water (5 mL), and extracted with ethyl acetate (18 mL). The organic phase was dried over anhydrous magnesium sulfate and subjected to suction filtration. The filtrate was concentrated to dryness under reduced pressure. The resultant was purified by column chromatography (silica, petroleum ether:ethyl acetate = 1:1) to obtain 5-methyl-1-(tetrahydro-2H-pyran-2-yl)-N-(3-((tetrahydrofuran-3-yl)sulfonyl)-4-(4,4,5,5-tetramethyl-1,3,2-dioxaborolan-2-yl)phenyl)-1H-pyrazol-3-amine as a yellow oily substance (150 mg).
MS m/z (ESI): 540.1 $[M+Na]^+$;

Step 6: Synthesis of isopropyl trans-(4-(5-(2-(2-hydroxy-2-methylpropoxy)-4-((5-methyl-1-(tetrahydro-2H-pyran-2-yl)-1H-pyrazol-3-yl)amino)phenyl)thiazol-2-yl)cyclohexyl) carbamate

5-Methyl-1-(tetrahydro-2H-pyran-2-yl)-N-(3-((tetrahydrofuran-3-yl)sulfonyl)-4-(4,4,5,5-tetramethyl-1,3,2-dioxaborolan-2-yl)phenyl)-1H-pyrazol-3-amine (75.0 mg, 145 μmol) and isopropyl trans-N-[4-(5-bromothiazol-2-yl)cyclohexyl]carbamate (50.3 mg, 145 μmol) were dissolved in dioxane (3 mL). Thereafter, [1,1-bis(diphenylphosphino)ferrocene] dichloropalladium - dichloromethane (5.92 mg, 7.25 μmol) and sodium carbonate (2M, 750 μL) were added under the condition where nitrogen gas was introduced. The reaction solution was stirred and reacted at 100°C for 16 hours (two parallel reactions of the same scale were carried out and the subsequent treatment was combined). The completion of the reaction was detected by LCMS. The reaction solution was concentrated to dryness under reduced pressure, added with water (5 mL), and extracted with ethyl acetate (9 mL). The organic layer was dried over anhydrous magnesium sulfate and subjected to suction filtration. The filtrate was concentrated to dryness under reduced pressure. The resultant was purified by column chromatography (silica, petroleum ether:ethyl acetate = 1:1) to obtain isopropyl trans-(4-(5-(2-(2-hydroxy-2-methylpropoxy)-4-((5-methyl-1-(tetrahydro-2H-pyran-2-yl)-1H-pyrazol-3-yl)amino)phenyl) thiazol-2-yl)cyclohexyl)carbamate as a yellow solid substance (190 mg, crude product).
MS m/z (ESI):658.2 $[M+H]^+$;

Step 7: Synthesis of isopropyl trans-(4-(5-(4-((5-methyl-1H-pyrazol-3-yl)amino)-2-((tetrahydrofuran-3-yl)sulfonyl)phenyl)thiazol-2-yl)cyclohexyl)carbamate

Isopropyl trans-(4-(5-(2-(2-hydroxy-2-methylpropoxy)-4-((5-methyl-1-(tetrahydro-2H-pyran-2-yl)-1H-pyrazol-3-yl)amino)phenyl)thiazol-2-yl)cyclohexyl)carbamate (100 mg, 152 μmol) was dissolved in methanol (1.5 mL), and then hydrochloric acid/methanol (4M, 2.1 mL) was added into the reaction solution. The reaction solution was stirred and reacted at 30°C for 2 hours. The completion of the reaction was detected by LCMS. The reaction solution was concentrated to dryness under reduced pressure and purified by preparative liquid chromatography (Agela DuraShell C18 column: 5

μm silica, 25 mm in diameter, 150 mm in length; using decreasingly polar mixtures of water (containing 0.225% formic acid) and acetonitrile (35% to 55%) as eluents) to obtain isopropyl trans-(4-(5-(4-((5-methyl-1H-pyrazol-3-yl)amino)-2-((tetrahydrofuran-3-yl)sulfonyl)phenyl)thiazol-2-yl)cyclohexyl)carbamate as a white solid compound (40.20 mg).

MS m/z (ESI):574.0 $[M+H]^+$;

$^1H$ NMR (400MHz, DMSO-d6) δ 11.90 (br s, 1H), 9.11 (s, 1H), 8.17 (s, 1H), 7.77-7.58 (m, 2H), 7.35 (d, J = 8.4 Hz, 1H), 7.02 (br d, J = 7.6 Hz, 1H), 5.64 (s, 1H), 4.85-4.62 (m, 1H), 3.87-3.80 (m, 1H), 3.80-3.68 (m, 2H), 3.65-3.52 (m, 2H), 3.31 (br s, 1H), 3.01-2.89 (m, 1H), 2.20 (s, 3H), 2.13 (br d, J = 11.9 Hz, 2H), 2.11-1.91 (m, 2H), 1.90-1.81 (m, 2H), 1.65-1.48 (m, 2H), 1.41-1.26 (m, 2H), 1.17-1.05 (m, 6H).

Example 116: Synthesis of isopropyl trans-(4-(5-(2-(cyclopropylsulfonyl)-4-((1,3-dimethyl-1H-pyrazol-5-yl)amino)phenyl)thiazol-2-yl)cyclohexyl)carbamate

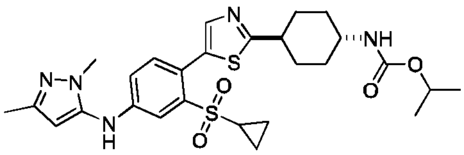

Synthetic Method

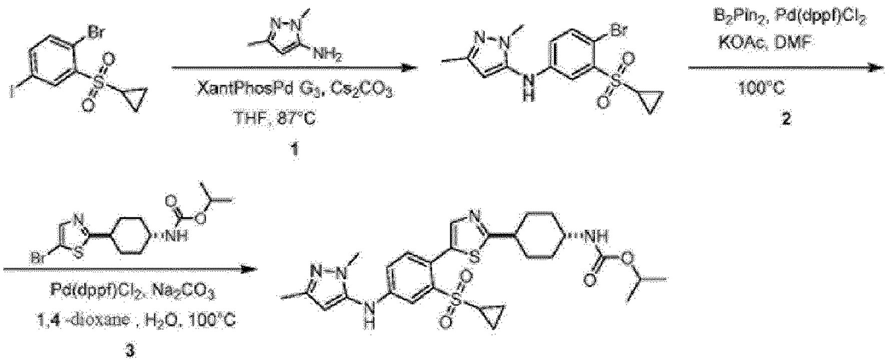


Step 1: Synthesis of N-(4-bromo-3-(cyclopropylsulfonyl)phenyl)-1,3-dimethyl-1H-pyrazol-5-amine

1-Bromo-2-(cyclopropylsulfonyl)-4-iodobenzene (5.00 g, 12.9 mmol) and 5-amino-1,3-dimethylpyrazole (1.72 g, 15.5 mmol) were dissolved in tetrahydrofuran (150 mL), and cesium carbonate (12.6 g, 38.8 mmol) and methanesulfonato[9,9-dimethyl-4,5-bis(diphenylphosphino) xanthene][2-amino-1,1-diphenyl]palladium (II) - dichloromethane adduct (612 mg, 646 μmol) were added. Under the protection of nitrogen, the reaction solution was stirred and reacted at 87°C for 5 hours. The completion of the reaction was detected by LCMS. The reaction solution was concentrated to dryness under reduced pressure and then purified by column chromatography (silica, petroleum ether: ethyl acetate = 2:1) to obtain N-(4-bromo-3-(cyclopropylsulfonyl)phenyl)-1,3-dimethyl-1H-pyrazol-5-amine as a yellow solid compound (4.00 g).

MS m/z (ESI): 371.9 $[M+H]^+$;

Step 2: Synthesis of N-(3-(cyclopropylsulfonyl)-4-(4,4,5,5-tetramethyl-1,3,2-dioxaborolan-2-yl)phenyl)-1,3-dimethyl-1H-pyrazol-5-amine

1-Bromo-2-(cyclopropylsulfonyl)-4-iodobenzene (600 mg, 1.62 mmol) and bis(pinacolato)diboron (2.06 g, 8.10 mmol) were dissolved in N,N-dimethylformamide (10 mL), and potassium acetate (795 mg, 8.10 mmol) and [1,1-bis(diphenylphosphino)ferrocene] dichloropalladium - dichloromethane (132 mg, 162 μmol) were added. The reaction solution was stirred and reacted at 100°C for 2 hours. The completion of the reaction was detected by LCMS. The reaction solution was concentrated to dryness under reduced pressure and then purified by column chromatography (silica, petroleum ether:ethyl acetate = 1:1) to obtain N-(3-(cyclopropylsulfonyl)-4-(4,4,5,5-tetramethyl-1,3,2-dioxaborolan-2-yl)phenyl)-1,3-dimethyl-1H-pyrazol-5-amine as a yellow solid compound (400 mg).

MS m/z (ESI): 418.2 $[M+H]^+$;

Step 3: Synthesis of isopropyl trans-(4-(5-(2-(cyclopropylsulfonyl)-4-((1,3-dimethyl-1H-pyrazol-5-yl)amino)phenyl)thiazol-2-yl)cyclohexyl)carbamate

N-(3-(cyclopropylsulfonyl)-4-(4,4,5,5-tetramethyl-1,3,2-dioxaborolan-2-yl)phenyl)-1,3-dimethyl-1H-pyrazol-5-amine (400 mg, 958 μmol) and isopropyl trans-(4-(5-bromothiazol-2-yl)cyclohexyl)carbamate (366 mg, 1.05 mmol) were dissolved in dioxane (10 mL), and an aqueous sodium carbonate solution (2M, 2 mL) and [1,1-bis(diphenylphosphino)ferrocene] dichloropalladium - dichloromethane (78.2 mg, 95.8 μmol) were added. The reaction solution was stirred and reacted at 100°C for 2 hours. The completion of the reaction was detected by LCMS. The reaction solution was concentrated to dryness under reduced pressure, added with water (10 mL), and extracted with dichloromethane (30 mL, 10 mL × 3) three times. The organic phase was dried over anhydrous sodium sulfate and filtered. The filtrate was concentrated to dryness under reduced pressure and purified by preparative liquid chromatography (YMC-Actus Triart C18: 5 μm silica, 30 mm in diameter, 150 mm in length; using decreasingly polar mixtures of water (containing 0.05% aqueous ammonia) and acetonitrile (44% to 64%) as eluents) to obtain isopropyl trans-(4-(5-(2-(cyclopropylsulfonyl)-4-((1,3-dimethyl-1H-pyrazol-5-yl)amino)phenyl)thiazol-2-yl)cyclohexyl)carbamate as a white solid compound (225 mg).

MS m/z (ESI): 558.1 $[M+H]^+$;

$^1$H NMR (400MHz, DMSO-d6) δ = 8.67 (s, 1H), 7.65 (s, 1H), 7.50 (d, J = 2.5 Hz, 1H), 7.35 (d, J = 8.4 Hz, 1H), 7.08-6.99 (m, 2H), 5.90 (s, 1H), 4.81-4.68 (m, 1H), 3.58 (s, 3H), 3.32-3.30 (m, 1H), 2.98-2.87 (m, 1H), 2.41-2.34 (m, 1H), 2.12 (s, 5H), 1.91 (br d, J = 10.5 Hz, 2H), 1.62-1.48 (m, 2H), 1.40-1.27 (m, 2H), 1.16 (d, J = 6.3 Hz, 6H), 0.99-0.91 (m, 2H), 0.89-0.82 (m, 2H).

Example 117: Synthesis of isopropyl trans-(4-(5-(4-((1,3-dimethyl-1H-pyrazol-5-yl)amino)-2-(oxetan-3-ylsulfonyl)phenyl)thiazol-2-yl)cyclohexyl)carbamate

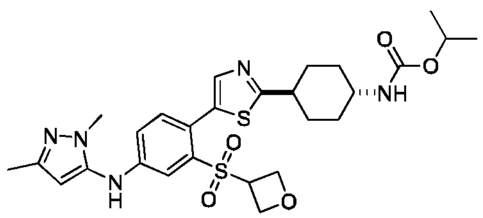

Synthetic Method

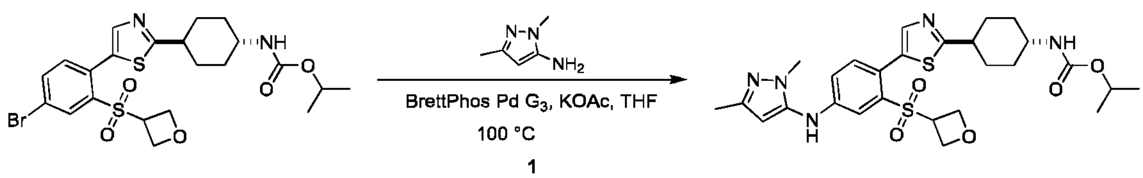


Isopropyl trans-N-[4-[5-[4-bromo-2-(oxetan-3-ylsulfonyl)phenyl]thiazol-2-yl]cyclohexyl] carbamate (60.0 mg, 110 μmol) and 5-amino-1,3-dimethylpyrazole (13.5 mg, 121 μmol) were dissolved in tetrahydrofuran (3 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (10.0 mg, 11.0 μmol) and potassium acetate (21.7 mg, 220 μmol) were added. The reaction solution was stirred and reacted at 100°C for 1 hour. The completion of the reaction was detected by LCMS. The reaction solution was added with 30 mL of ethyl acetate (10 mL × 3) and 10 mL of water. The organic phase was dried over anhydrous sodium sulfate and filtered. The filtrate was concentrated to dryness under reduced pressure. The resultant was purified by preparative liquid chromatography (YMC-ActusTriartC18 column, 5 μm silica, 30 mm in diameter, 150 mm in length; using decreasingly polar mixtures of water (containing 0.05% aqueous ammonia) and acetonitrile as eluents) to obtain the compound isopropyl trans-(4-(5-(4-((1,3-dimethyl-1H-pyrazol-5-yl)amino)-2-(oxetan-3-ylsulfonyl)phenyl)thiazol-2-yl)cyclohexyl)carbamate (12.20 mg) as a white solid.

MS m/z (ESI): 573.9 $[M+H]^+$;

$^1H$ NMR (400MHz, DMSO-d6) δ = 8.74 (s, 1H), 7.63 (s, 1H), 7.56 (d, J = 2.5 Hz, 1H), 7.35 (d, J = 8.3 Hz, 1H), 7.10-7.02 (m, 2H), 5.92 (s, 1H), 4.86-4.72 (m, 1H), 4.64-4.58 (m, 2H), 4.53-4.46 (m, 3H), 3.59 (s, 3H), 3.32-3.27 (m, 1H), 2.97-2.89 (m, 1H), 2.14 (s, 5H), 1.92 (br d, J = 9.5 Hz, 2H), 1.62-1.51 (m, 2H), 1.39-1.28 (m, 2H), 1.17-1.05 (m, 6H).

Example 118: Synthesis of isopropyl trans-(4-(5-(4-((1-(2-fluoroethyl)-1H-pyrazol-4-yl)amino)-2-(oxetan-3-ylsulfonyl)phenyl)thiazol-2-yl)cyclohexyl)carbamate

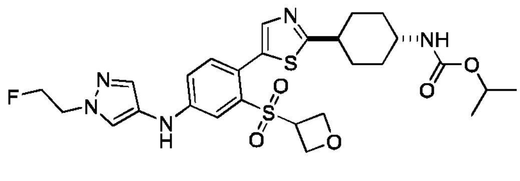

Synthetic Method

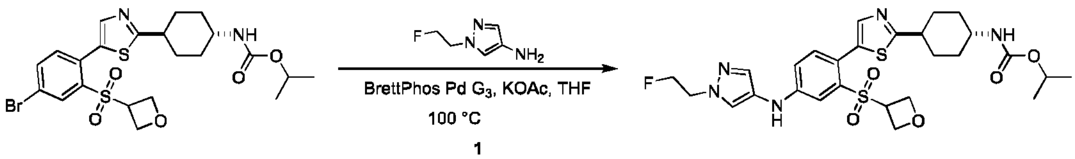


Isopropyl trans-N-[4-[5-[4-bromo-2-(oxetan-3-ylsulfonyl)phenyl]thiazol-2-yl]cyclohexyl] carbamate (60.0 mg, 110 μmol) and 1-(2-fluoroethyl)-1H-pyrazol-4-amine (15.7 mg, 121 μmol) were dissolved in tetrahydrofuran (3 mL), and methanesulfonato(2-dicyclohexylphosphino)-3,6-dimethoxy-2,4,6-triisopropyl-1,1-biphenyl)(2-amino-1,1-biphenyl-2-yl)palladium (II) (10.0 mg, 11.0 μmol) and potassium acetate (21.7 mg, 220 μmol) were added. The reaction solution was stirred and reacted at 100°C for 1 hour. The completion of the reaction was detected by LCMS. The reaction solution was added with 30 mL of ethyl acetate (10 mL × 3) and 10 mL of water. The organic phase was dried over anhydrous sodium sulfate and filtered. The filtrate was concentrated to dryness under reduced pressure. The resultant was purified by preparative liquid chromatography (YMC-ActusTriartC18 column, 5 μm silica, 30 mm in diameter, 150 mm in length; using decreasingly polar mixtures of water (containing 0.05% aqueous ammonia) and acetonitrile as eluents) to obtain the compound isopropyl trans-(4-(5-(4-((1-(2-fluoroethyl)-1H-pyrazol-4-yl)amino)-2-(oxetan-3-ylsulfonyl)phenyl)thiazol-2-yl)cyclohexyl) carbamate (13.85 mg) as a white solid.

MS m/z (ESI): 591.9 $[M+H]^+$;

$^1H$ NMR (400MHz, DMSO-d6) δ = 8.36 (s, 1H), 7.82 (s, 1H), 7.60 (s, 1H), 7.49 (s, 1H), 7.46 (s, 1H), 7.28 (d, J = 8.3 Hz, 1H), 7.05-6.99 (m, 2H), 4.86-4.80 (m, 1H), 4.78-4.70 (m, 2H), 4.64-4.58 (m, 2H), 4.52-4.44 (m, 4H), 4.46-4.33 (m, 1H), 3.33-3.25 (m, 1H), 2.96-2.88 (m, 1H), 2.13 (br d, J = 11.8 Hz, 2H), 1.95-1.88 (m, 2H), 1.62-1.50 (m, 2H), 1.39-1.28 (m, 2H), 1.19-1.05 (m, 6H).

Example 119: Synthesis of isopropyl trans-N-[4-[2-[4-(isopropoxycarbonylamino) cyclohexyl]thiazol-5-yl]-3-(1-phenylethylsulfonyl)phenyl]carbamate

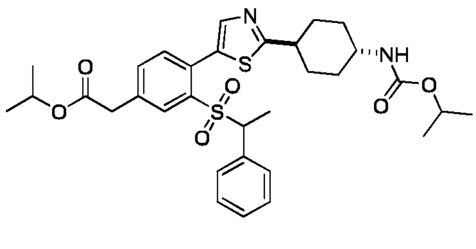

Synthetic Method

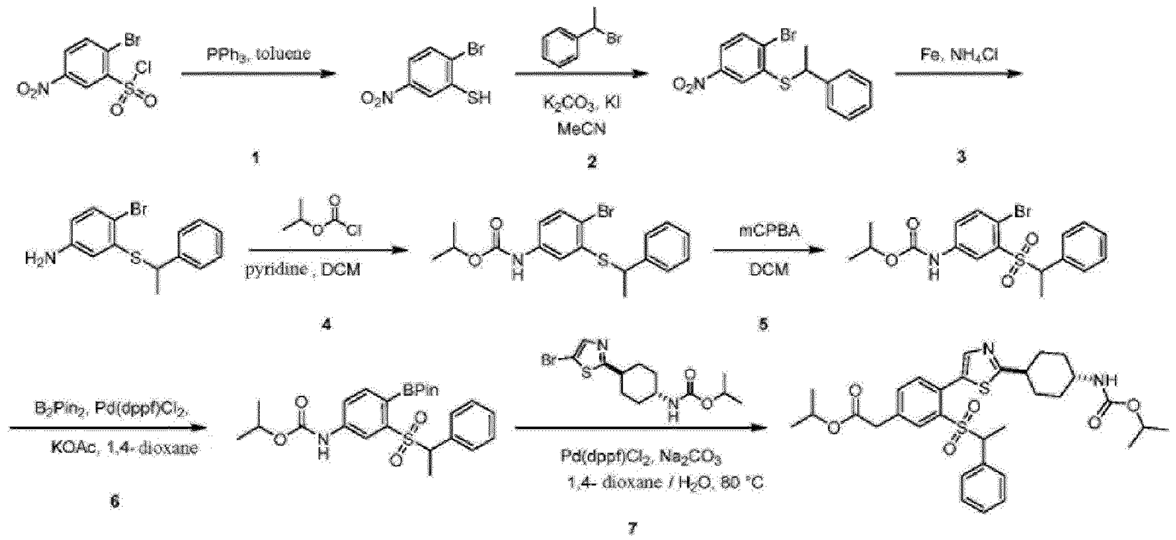


Step 1: Synthesis of 2-bromo-5-nitro-benzenethiol

2-Bromo-5-nitro-benzenesulfonyl chloride (1.5 g, 4.99 mmol) was dissolved in toluene (20 mL), and triphenylphosphine (3.93 g, 14.97 mmol) was added in two batches. The reaction solution appeared as a yellow suspension, and was stirred and reacted at 110°C for 1.2 h. TLC (petroleum ether:ethyl acetate = 5:1) detection showed that the reaction was complete. The reaction solution was concentrated to dryness under reduced pressure. The resultant was purified by column chromatography (petroleum ether:ethyl acetate = 5:1) to obtain the compound 2-bromo-5-nitro-benzenethiol (200 mg) as a white solid.

$^1$H NMR (400 MHz, DMSO-d6) $\delta$ ppm 8.52 (d, J = 2.26 Hz, 1H) 7.93-7.75 (m, 2H).

Step 2: Synthesis of 1-bromo-4-nitro-2-(1-phenylethylmercapto)benzene

2-Bromo-5-nitro-benzenethiol (175 mg, 747.64 $\mu$mol) and (1-bromoethyl)benzene (207.54 mg, 1.12 mmol) were dissolved in acetonitrile (900 $\mu$L). Under the protection of nitrogen, potassium carbonate (309.98 mg, 2.24 mmol) and potassium iodide (124.11 mg, 747.64 $\mu$mol) were added. The reaction solution appeared as a red suspension, and was stirred and reacted at 80°C for 1.5 h. TLC detection showed that the reaction was complete. The reaction solution was concentrated to dryness under reduced pressure. The resultant was purified by column chromatography (petroleum ether:ethyl acetate = 5:1) to obtain 1-bromo-4-nitro-2-(1-phenylethylmercapto)benzene as the product (205 mg), which was a yellow oil.

$^1$H NMR (400 MHz, DMSO-d6) $\delta$ ppm 8.09 (d, J = 2.25 Hz, 1H) 7.93-7.83 (m, 2H) 7.52 (d, J = 7.25 Hz, 2H) 7.39-7.32 (m, 2H) 7.29-7.22 (m, 1H) 5.01-4.90 (m, 1H) 1.65 (d, J = 7.00 Hz, 3H).

Step 3: Synthesis of 4-bromo-3-(1-phenylethylmercapto)aniline

1-Bromo-4-nitro-2-(1-phenylethylmercapto)benzene (240 mg, 709.60 $\mu$mol) was dissolved in anhydrous ethanol (2 mL). Under the protection of nitrogen, reduced iron powder (237.77 mg, 4.26 mmol) and ammonium chloride (379.57 mg, 7.10 mmol) were added. The reaction solution appeared as a yellow suspension, and was stirred and reacted at 80°C for 2 h. The completion of the reaction was detected by LCMS. The reaction solution was filtered while it was still hot, added with water (5 mL), and extracted with dichloromethane (10 mL). The organic layer was dried over anhydrous magnesium sulfate and subjected to suction filtration. The filtrate was concentrated to dryness under reduced pressure to obtain the crude compound 4-bromo-3-(1-phenylethylmercapto)aniline (175 mg) as a white solid.

MS m/z (ESI): 308.1, 310.1 $[M+H]^+$;

Step 4: Synthesis of isopropyl N-[4-bromo-3-(1-phenylethylmercapto)phenyl]carbamate

4-Bromo-3-(1-phenylethylmercapto)aniline (175 mg, 567.75 μmol) was dissolved in anhydrous dichloromethane (2 mL). Under the protection of nitrogen, pyridine (269.45 mg, 3.41 mmol) and isopropyl chloroformate (208.73 mg, 1.70 mmol, 236.39 μL) were added. The reaction solution appeared as a yellow suspension, and was stirred and reacted at 25°C for 1 h. The completion of the reaction was detected by LCMS. The reaction solution was concentrated to dryness under reduced pressure. The resultant was purified by a thin-layer chromatography plate (petroleum ether:ethyl acetate = 3:1) to obtain the compound isopropyl N-[4-bromo-3-(1-phenylethylmercapto)phenyl]carbamate (185 mg) as a white solid.

MS m/z (ESI): 394.1, 396.1 $[M+H]^+$;

Step 5: Synthesis of isopropyl N-[4-bromo-3-(1-phenylethylsulfonyl)phenyl]carbamate

Isopropyl N-[4-bromo-3-(1-phenylethylmercapto)phenyl]carbamate (185 mg, 469.16 μmol) was dissolved in anhydrous dichloromethane (3 mL), and m-chloroperoxybenzoic acid (242.88 mg, 1.41 mmol) was added. The reaction solution appeared as a yellow suspension, and was stirred and reacted at 25°C for 1 h. The completion of the reaction was detected by LCMS. The reaction solution was added with saturated ammonium chloride solution (10 mL) and extracted with dichloromethane (20 mL). The organic layer was dried over anhydrous magnesium sulfate and subjected to suction filtration. The filtrate was concentrated to dryness under reduced pressure. The resultant was purified by thin-layer chromatography (petroleum ether:ethyl acetate = 3:1) to obtain the compound isopropyl N-[4-bromo-3-(1-phenylethylsulfonyl)phenyl]carbamate (200 mg) as a white solid.

MS m/z (ESI): 443.1, 445.1 $[M+H_2O]^+$;

Step 6: Synthesis of isopropyl N-[3-(1-phenylethylsulfonyl)-4-(4,4,5,5-tetramethyl-1,3,2-dioxaborolan-2-yl)phenyl]carbamate

Isopropyl N-[4-bromo-3-(1-phenylethylsulfonyl)phenyl]carbamate (200 mg, 469.13 μmol) and bis(pinacolato)diboron (476.52 mg, 1.88 mmol) were dissolved in anhydrous tetrahydrofuran (4 mL). Under the protection of nitrogen, [1,1-bis(diphenylphosphino) ferrocene]dichloropalladium - dichloromethane complex (38.31 mg, 46.91 μmol) and potassium acetate (138.12 mg, 1.41 mmol) were added. The reaction solution appeared as a black suspension, and was stirred and reacted at 80°C for 7 h. The completion of the reaction was detected by LCMS. The reaction solution was concentrated to dryness under reduced pressure. The resultant was purified by thin-layer chromatography (petroleum ether:ethyl acetate = 3:1) to obtain the compound isopropyl N-[3-(1-phenylethylsulfonyl)-4-(4,4,5,5-tetramethyl-1,3,2-dioxaborolan-2-yl)phenyl]carbamate (100 mg) as a white solid.

MS m/z (ESI): 491.2 $[M+H_2O]^+$;

Step 7: Synthesis of isopropyl trans-N-[4-[2-[4-(isopropoxycarbonylamino)cyclohexyl] thiazol-5-yl]-3-(1-phenylethylsulfonyl)phenyl]carbamate

Isopropyl N-[3-(1-phenylethylsulfonyl)-4-(4,4,5,5-tetramethyl-1,3,2-dioxaborolan-2-yl) phenyl]carbamate (50 mg, 105.62 μmol) and isopropyl trans-N-[4-(5-bromothiazol-2-yl) cyclohexyl]carbamate (36.68 mg, 105.62 μmol) were dissolved in 1,4-dioxane (2 mL). Under the protection of nitrogen, [1,1-bis(diphenylphosphino)ferrocene]dichloropalladium - dichloromethane complex (8.63 mg, 10.56 μmol), sodium carbonate (22.39 mg, 211.24 μmol) and water (0.2 mL) were added. The reaction solution appeared as a yellow suspension, and was stirred and reacted at 80°C for 7 h. The completion of the reaction was detected by LCMS. The reaction solution was concentrated to dryness under reduced pressure, diluted with water (5 mL), and then extracted with dichloromethane (20 mL). The organic phase was evaporated to dryness via rotary evaporation and purified by preparative liquid chromatography (PhenomenexGeminiC18 column, 10 μm silica, 50 mm in diameter, 250 mm in length; using decreasingly polar mixtures of water (containing 0.05% aqueous ammonia) and acetonitrile as eluents) to obtain the compound isopropyl trans-N-[4-[2-[4-(isopropoxycarbonylamino) cyclohexyl]thiazol-5-yl]-3-(1-phenylethylsulfonyl)phenyl]carbamate (8.53 mg) as a white solid.

MS m/z (ESI): 613.0 $[M+H]^+$;

$^1H$ NMR (400 MHz, DMSO-d6) δ ppm 10.11 (s, 1H) 8.21 (d, J = 2.26 Hz, 1H) 7.78-7.72 (m, 2H) 7.51 (d, J = 8.53 Hz, 1H) 7.34-7.28 (m, 3H) 7.09-7.03 (m, 3H) 4.98-4.87 (m, 1H) 4.81-4.70 (m, 1H) 4.10-4.00 (m, 1H) 3.33-3.28 (m, 1H) 3.06-2.93 (m, 1H) 2.21-2.12 (m, 2H) 1.99-1.89 (m, 2H) 1.68-1.55 (m, 2H) 1.43 (d, J = 7.03 Hz, 3H) 1.40-1.30 (m, 2H) 1.27 (d, J = 6.27 Hz, 6H) 1.17 (d, J = 6.27 Hz, 6H).

Test Examples for Bioactivities and Related Properties

Test Example 1: Experiment on the inhibition of human Daudi lymphoma cell proliferation

Introduction of the experimental principle: After the RAD51 inhibitor to be tested was co-incubated with cancer cells for a period of time, the influence of the test compound on cell proliferation was measured by a method for counting cell proliferation based on the quantitative measurement of intracellular ATP content.

Materials and Cells: Daudi cells were purchased from ATCC; fetal bovine serum, 1640 medium and penicillin-streptomycin were purchased from Gibco Inc. (U.S.), 96-well plates were purchased from Corning Inc. (U.S.), and Cell-Titer Glo reagent was purchased from Promega Corporation (U.S.).

Cell culture: Daudi cells were cultured in 1640 culture medium containing 10% fetal bovine serum and 1% penicillin-streptomycin at 37°C in an atmosphere containing 5% $CO_2$. Cells in logarithmic growth phase could be used for experiments.

Assay of cell proliferation and viability: Cell-Titer Glo reagent was used to measure the inhibitory activities of the compounds on the proliferation of Daudi cell strain. The concentration of cells was adjusted, and the cells were seeded in a 96-well plate and cultured at 37°C in an atmosphere containing 5% $CO_2$ for 24 hours. The powder of the compound was dissolved in 100% DMSO to form a 10 mM solution. 10 μl of the solution of the compound was aspirated and transferred into a 96-well compound plate containing 20 μl of DMSO solution for 3-fold dilution and was then formulated into 10 serial dilutions. Thereafter, 2 μl of the solution of the compound was aspirated from the corresponding well and added correspondingly into a 96-well intermediate compound dilution plate containing 78 μl of complete culture medium in each well. The resultant was mixed well, and the plate was centrifuged at 1000 rpm for 1 minute. Finally, 10 μl of the solution of the compound was aspirated from the corresponding well of the 96-well intermediate compound dilution plate, and transferred into the corresponding well of the cell culture plate containing 90 μl of complete culture medium. The final concentration of DMSO was 0.25%. After the addition of the compound, the cells were continued to be incubated for 3 days at 37°C in an atmosphere containing 5% $CO_2$. Cell-Titer Glo reagent was added to measure cell viability.

Data Analysis

% Compound inhibition was calculated and $IC_{50}$ of the compound was fitted;

$$\%\ \text{Compound inhibition} = 1\text{-}100\% \times (\text{Signal-Bottom}) / (\text{Top-Bottom})$$

Experimental Results:

The corresponding anti-proliferative activities of the test compounds against Daudi cell were listed in Table 1.

Table 1 Inhibitory activities of the compounds of the present disclosure against the proliferation of Daudi cells

| Compounds | Anti-Proliferative Activity Against Daudi cells $IC_{50}$ (μM) | Compounds | Anti-Proliferative Activity Against Daudi cells $IC_{50}$ (μM) |
|---|---|---|---|
| Example 1 | 1.62 | Example 62 | 0.01 |
| Example 2 | 0.39 | Example 63 | 0.07 |
| Example 3 | 1.08 | Example 64 | 5.2 |
| Example 4 | 1.01 | Example 65 | 0.037 |
| Example 5 | 1.16 | Example 66 | 0.018 |
| Example 6 | 3.72 | Example 67 | 2.6 |
| Example 7 | 2.59 | Example 68 | 0.1 |
| Example 8 | 3.95 | Example 69 | 0.03 |
| Example 9 | 2.06 | Example 70 | 0.008 |
| Example 10 | 9.72 | Example 71 | 0.016 |
| Example 11 | 2.54 | Example 72 | 0.004 |

(continued)

| Compounds | Anti-Proliferative Activity Against Daudi cells $IC_{50}$ ($\mu$M) | Compounds | Anti-Proliferative Activity Against Daudi cells $IC_{50}$ ($\mu$M) |
|---|---|---|---|
| Example 12 | 0.02 | Example 73 | 0.2 |
| Example 13 | 0.41 | Example 74 | 0.09 |
| Example 14 | 0.45 | Example 75 | 0.022 |
| Example 15 | 0.28 | Example 76 | 0.009 |
| Example 16 | 0.49 | Example 77 | 0.004 |
| Example 17 | 0.11 | Example 78 | 0.004 |
| Example 18 | 0.09 | Example 79 | 0.051 |
| Example 19 | 0.96 | Example 80 | 0.33 |
| Example 20 | 2.75 | Example 81 | 0.04 |
| Example 21 | 0.44 | Example 82 | 0.25 |
| Example 22 | 0.035 | Example 83 | 0.2 |
| Example 23 | 3.93 | Example 84 | 0.007 |
| Example 24 | 2.27 | Example 85 | 0.0006 |
| Example 25 | 0.74 | Example 86 | 0.003 |
| Example 26 | 0.13 | Example 87 | 0.29 |
| Example 27 | 0.68 | Example 88 | 0.004 |
| Example 28 | 0.048 | Example 89 | 0.007 |
| Example 29 | 0.34 | Example 89-1 | 0.127 |
| Example 30 | 2.65 | Example 90 | 8.8 |
| Example 32 | 0.42 | Example 91 | 1.5 |
| Example 33 | 0.1 | Example 91-1 | 0.06 |
| Example 34 | 0.7 | Example 92 | 0.004 |
| Example 35 | 4.29 | Example 93 | 0.01 |
| Example 36 | 0.077 | Example 94 | 0.02 |
| Example 37 | 0.33 | Example 94-1 | 0.06 |
| Example 38 | 0.15 | Example 95 | 0.132 |
| Example 39 | 0.23 | Example 96 | 0.14 |
| Example 40 | 0.051 | Example 97 | 0.29 |
| Example 41 | 0.9 | Example 98 | 0.032 |
| Example 42 | 0.42 | Example 99 | 0.036 |
| Example 43 | 1.58 | Example 100 | 0.68 |
| Example 44 | 0.68 | Example 101 | 0.028 |
| Example 45 | 0.062 | Example 102 | 0.017 |
| Example 46 | 0.39 | Example 103 | 0.047 |
| Example 47 | 0.03 | Example 104 | 0.023 |
| Example 48 | 0.006 | Example 105 | 0.026 |
| Example 49 | 0.006 | Example 106 | 0.04 |

(continued)

| Compounds | Anti-Proliferative Activity Against Daudi cells $IC_{50}$ (μM) | Compounds | Anti-Proliferative Activity Against Daudi cells $IC_{50}$ (μM) |
|---|---|---|---|
| Example 50 | 0.026 | Example 107 | 0.275 |
| Example 51 | 0.065 | Example 108 | 0.468 |
| Example 52 | 0.08 | Example 109 | 0.076 |
| Example 53 | 0.1 | Example 111 | 0.7 |
| Example 54 | 0.055 | Example 112 | 0.017 |
| Example 56 | 0.007 | Example 113 | 0.016 |
| Example 57 | 0.007 | Example 114 | 0.014 |
| Example 58 | 0.027 | Example 115 | 0.016 |
| Example 59 | 0.125 | Example 116 | 0.004 |
| Example 60 | 0.004 | Example 117 | 0.007 |
| Example 61 | 0.104 | | |

Test Example 2: Experiment on the inhibition of human WI-38 embryonic lung cell proliferation

Introduction of the experimental principle: After the RAD51 inhibitor to be tested was co-incubated with human embryonic lung cells for a period of time, the influence of the test compound on cell proliferation was measured by a method for counting cell proliferation based on the quantitative measurement of intracellular ATP content.

Materials and Cells: WI-38 cells were purchased from ATCC; fetal bovine serum, 1640 medium and penicillin-streptomycin were purchased from Gibco Inc. (U.S.), 96-well plates were purchased from Corning Inc. (U.S.), and Cell-Titer Glo reagent was purchased from Promega Corporation (U.S.).

Cell culture: WI-38 cells were cultured in an EMEM culture medium containing 10% fetal bovine serum and 1% penicillin-streptomycin at 37°C in an atmosphere containing 5% $CO_2$. Cells in logarithmic growth phase could be used for experiments.

Assay of cell proliferation and viability: Cell-Titer Glo reagent was used to measure the inhibitory activities of the compounds on the proliferation of WI-38 cell strain. The concentration of cells was adjusted, and the cells were seeded in a 96-well plate and cultured at 37°C in an atmosphere containing 5% $CO_2$ for 24 hours. The powder of the compound was dissolved in 100% DMSO to form a 10 mM solution. 10 μl of the solution of the compound was aspirated and transferred into a 96-well compound plate containing 20 μl of DMSO solution in each well for 3-fold dilution and was then formulated to 10 serial dilutions. Thereafter, 2 μl of the solution of the compound was aspirated from the corresponding well and added correspondingly into a 96-well intermediate compound dilution plate containing 78 μl of complete culture medium in each well. The resultant was mixed well, and the plate was centrifuged at 1000 rpm for 1 minute. Finally, 10 μl of the solution of the compound was aspirated from the corresponding well of the 96-well intermediate compound dilution plate, and transferred into the corresponding well of the cell culture plate containing 90 μl of complete culture medium. The final concentration of DMSO was 0.25%. After the addition of the compound, the cells were continued to be incubated for 3 days at 37°C in an atmosphere containing 5% $CO_2$. Cell-Titer Glo reagent was added to measure cell viability.

Data Analysis:

% Compound inhibition was calculated and $IC_{50}$ of the compound was fitted;

$$\%\ \text{Compound inhibition} = 1\text{-}100\% \times (\text{Signal-Bottom}) / (\text{Top-Bottom})$$

Experimental Results:

The corresponding anti-proliferative activities of the test compounds against WI-38 cell were listed in Table 2.

Table 2 Inhibitory activities of the compounds of the present disclosure against the proliferation of WI-38 cells

| Compounds | Anti-Proliferative Activity Against WI-38 cells $IC_{50}$ (μM) | Compounds | Anti-Proliferative Activity Against WI-38 cells $IC_{50}$ (μM) |
|---|---|---|---|
| Example 1 | >25 | Example 45 | >25 |
| Example 2 | >25 | Example 47 | >25 |
| Example 3 | >25 | Example 48 | 15.6 |
| Example 4 | 7.22 | Example 49 | >25 |
| Example 5 | >25 | Example 50 | >25 |
| Example 6 | >25 | Example 53 | >25 |
| Example 7 | >25 | Example 54 | >25 |
| Example 9 | >25 | Example 56 | >25 |
| Example 12 | >25 | Example 57 | >25 |
| Example 13 | >25 | Example 62 | >25 |
| Example 14 | >25 | Example 65 | >25 |
| Example 15 | >25 | Example 68 | >25 |
| Example 16 | >25 | Example 69 | >25 |
| Example 17 | >25 | Example 70 | 21 |
| Example 18 | >25 | Example 72 | 20 |
| Example 19 | >25 | Example 81 | 14 |
| Example 21 | >25 | Example 84 | >25 |
| Example 22 | 23.3 | Example 85 | 17 |
| Example 25 | >25 | Example 86 | >25 |
| Example 26 | >25 | Example 88 | >25 |
| Example 28 | 15 | Example 92 | 19.5 |
| Example 29 | 18.4 | Example 98 | >25 |
| Example 32 | 23 | Example 101 | >25 |
| Example 33 | >25 | Example 102 | >25 |
| Example 34 | >25 | Example 103 | >25 |
| Example 36 | 24.6 | Example 104 | >25 |
| Example 37 | >25 | Example 114 | >25 |
| Example 39 | >25 | Example 115 | >25 |
| Example 40 | 22.9 | Example 117 | 20 |

Under the conditions of Test Example 1 and Test Example 2, the test compounds exhibited relatively strong inhibitory activity on the proliferation of Daudi cells and relatively weak inhibitory activity on the proliferation of WI-38 cells, and the compounds also exhibited certain selectivity in terms of the inhibitory activities on the proliferation of Daudi cells and WI-38 cells.

Test Example 3: Metabolic stability assay of the compounds of the present disclosure in liver microsomes

I. Test Materials and Instruments

1. Source of liver microsomes: human liver microsomes (Corning 452117), CD-1 mouse liver microsomes (XENOTECH M1000)
2. $Na_2HPO_4$ (Tianjin Guangfu Fine Chemical Research Institute,20180130)

3. $KH_2PO_4$ (Tianjin Guangfu Fine Chemical Research Institute, 20180920)
4. $MgCl_2$ (Tianjin Guangfu Fine Chemical Research Institute, 20191216)
5. NADPH (Solarbio 1216C022)
6. Verapamil as a positive control compound (Sigma MKBV4993V)
7. AB Sciex Triple Quad 4000 liquid chromatography-mass spectrometer

II. Experimental Steps

1. Formulation of 100 mM phosphate buffer solution (PBS): 7.098 g of $Na_2HPO_4$ was weighed and ultrasonically dissolved in 500 mL of pure water to form Solution A. 3.400 g of $KH_2PO_4$ was weighed and ultrasonically dissolved in 250 mL of pure water to form Solution B. Solution A was placed in a stirrer and Solution B was slowly added until the pH value reached 7.4, thus formulating 100 mM PBS buffer solution.

2. Formulation of the reaction system

The reaction system was formulated according to Table 3 below.

Table 3 Formulation information of reaction system

| Reagent | Conc. of Stock Solution | Volume | Final Conc. |
|---|---|---|---|
| Liver microsome | 20 mg/mL | 10 μL | 0.5 mg/mL |
| Phosphate buffer solution | 100 mM | 346 μL | 100 mM |

3. The reaction system was placed in a water bath at 37°C and pre-incubated for 10 minutes. 40 μL of 10 mM NADPH solution (NADPH was dissolved in 100 mM phosphate buffer solution) was added into the reaction system, and the final concentration of NADPH was 1 mM. 40 μL of Phosphate buffer solution was used instead of the NADPH solution as a negative control. The negative control was used to rule out the influence caused by the chemical stability of the compounds per se.

4. The reaction system was added with 4 μL of 100 μM the compound of the present disclosure and Verapamil (the positive control compound) (DMSO as the vehicle), so that the reaction was initiated. The final concentration of the compound was 1 μM.

5. At 0.5, 15, 30, 45 and 60 minutes, after being well mixed in a vortex oscillator, 50 μL of the incubated samples were taken out respectively, and the reaction was terminated with ice acetonitrile (4-fold) containing an internal standard. The samples were centrifuged at 3220 g for 45 minutes. After the centrifugation, 90 μL of the supernatants were transferred to a sample plate and mixed well with 90 μL of ultrapure water for LC-MS/MS analysis.

All data were calculated by Microsoft Excel software. Peak areas were determined from the extracted ion chromatograms. The *in-vitro* half-life ($t_{1/2}$) of the parent drug was determined by the linear fitting of the natural logarithm of the elimination percentage of the parent drug versus time.

The *in-vitro* half-life ($t_{1/2}$) was calculated from the slope:

$$\textit{In-vitro}\ t_{1/2} = 0.693 / k.$$

The $t_{1/2}$ values calculated by the above formula were as listed in Table 4.

Table 4 Half-life values and intrinsic clearance values of the compounds of the present disclosure in liver microsomes

| Example No. | Species | $t_{1/2}$ (min) | Example No. | Species | $t_{1/2}$ (min) |
|---|---|---|---|---|---|
| Example 1 | Human being | >500 | Example 65 | Human being | 111 |
| | CD-1 mouse | >500 | | CD-1 mouse | 80 |
| Example 2 | Human being | 145 | Example 72 | Human being | 422 |
| | CD-1 mouse | 256 | | CD-1 mouse | 271 |
| Example 3 | Human being | 477 | Example 68 | Human being | 187 |
| | CD-1 mouse | >500 | | CD-1 mouse | 169 |
| Example 32 | Human being | 251 | Example 81 | Human being | 319 |
| | CD-1 mouse | 162 | | CD-1 mouse | 210 |

(continued)

| Example No. | Species | $t_{1/2}$ (min) | Example No. | Species | $t_{1/2}$ (min) |
|---|---|---|---|---|---|
| Example 33 | Human being | >500 | Example 84 | Human being | 67 |
| | CD-1 mouse | 248 | | CD-1 mouse | 112 |
| Example 47 | Human being | 303 | Example 85 | Human being | 82 |
| | CD-1 mouse | 437 | | CD-1 mouse | 51 |
| Example 48 | Human being | >500 | Example 86 | Human being | 107 |
| | CD-1 mouse | 432 | | CD-1 mouse | 84 |
| Example 49 | Human being | 56 | Example 92 | Human being | 382 |
| | CD-1 mouse | 125 | | CD-1 mouse | 346 |
| Example 50 | Human being | 475 | Example 94 | Human being | 231 |
| | CD-1 mouse | 376 | | CD-1 mouse | 204 |
| Example 54 | Human being | >500 | Example 98 | Human being | 137 |
| | CD-1 mouse | >500 | | CD-1 mouse | >500 |
| Example 56 | Human being | 179 | Example 103 | Human being | >500 |
| | CD-1 mouse | 109 | | CD-1 mouse | >500 |
| Example 57 | Human being | 56 | | | |
| | CD-1 mouse | 126 | | | |

Test Example 4: Plasma protein binding rate assay of the compounds of the present disclosure

I. Test Materials and Instruments

1. CD-1 mouse plasma (BioIVT)
2. $Na_2HPO_4$ (Sigma S5136-500G)
3. $NaH_2PO_4$ (Sigma S3139-500G)
4. NaCl (Sigma S5886-IKG)
5. 96-well equilibrium dialysis plate (HTDialysis LLC, Gales Ferry, CT, HTD96B), equilibrium dialysis membrane (MWCO 12-14K, #1101)
6. Warfarin as a positive control compound
7. ABI QTrap 5500 liquid chromatography-mass spectrometer

II. Experimental Steps

1. Formulation of a buffer solution containing 100 mM sodium phosphate and 150 mM NaCl: An alkaline solution containing $Na_2HPO_4$ (14.2 g/L) and NaCl (8.77 g/L) was formulated with ultrapure water. An acidic solution containing $NaH_2PO_4$ (12.0 g/L) and NaCl (8.77 g/L) was formulated with ultrapure water. The alkaline solution was titrated with the acidic solution until the pH value was 7.4, thus formulating the buffer solution containing 100 mM sodium phosphate and 150 mM NaCl.
2. Preparation of dialysis membrane: The dialysis membrane was immersed in ultrapure water for 60 minutes to separate the membrane into two pieces, then immersed in 20% ethanol for 20 minutes, and finally immersed in a buffer for dialysis for 20 minutes.
3. Preparation of plasma: Frozen plasma was thawed rapidly at room temperature. Thereafter, the plasma was centrifuged at 3220 g for 10 minutes at 4°C to remove clots, and the supernatant was collected into a new centrifuge tube. The pH values of plasma were measured and recorded. The plasma with a pH value of 7 to 8 was used.
4. Formulation of compound-containing plasma samples: A 10 mM stock solution of the compound of the present

disclosure or the positive control compound was diluted with DMSO to obtain a 200 μM working solution. 3 μl of the 200 μM working solution of the compound was added into 597 μl of mouse plasma to obtain a plasma sample with a final concentration of 1 μM.

5. Steps of equilibrium dialysis: The dialysis device was assembled according to the operating instructions. 120 μL of a plasma sample containing 1 μM compound was added on one side of the dialysis membrane, and an equal volume of dialysate (phosphate buffer solution) was added on the other side. Duplicate samples were set in the experiment. The dialysis plate was sealed and placed in an incubation device where the samples were incubated at 37°C in an atmosphere containing 5% $CO_2$ for 6 hours with a rotational speed of about 100 rpm. After the incubation was complete, the sealing membrane was removed., 50 μl of samples were respectively aspirated from the buffer solution side and the plasma side of each well and transferred into different wells of a new plate. 50 μl of blank plasma was added into the sample obtained from the phosphate buffer solution side, and an equal volume of blank phosphate buffer solution was added into the plasma sample, followed by the addition of 300 μl of acetonitrile containing the internal standard to precipitate protein. The sample was vortexed for 5 minutes and centrifuged at 3220 g for 30 minutes at 4°C. 100 μl of the supernatant was aspirated and transferredto the sample plate, mixed well with 100 μL of ultrapure water, and used for LC-MS/MS analysis.

The peak areas of the compounds on the buffer solution side and the plasma side were determined. The formula for calculating the plasma protein binding rate of the compound was as follows:

% Free rate = (the ratio of the peak area of the compound to the peak area of the internal standard on the buffer solution side / the ratio of the peak area of the compound to the peak area of the internal standard on the plasma side) × 100

% Binding rate = 100 - % free rate

All data were calculated by Microsoft Excel software. The calculated plasma protein binding rates of the compounds of the present disclosure were listed in Table 5.

Table 5 Protein binding rates of the compounds of the present disclosure in CD-1 mouse plasma

| Example No. | % Binding Rate | Example No. | % Binding Rate |
|---|---|---|---|
| Example 1 | 94% | Example 56 | 97% |
| Example 2 | 99.4% | Example 57 | 99.9% |
| Example 3 | 97% | Example 65 | 97% |
| Example 32 | 99% | Example 68 | 97% |
| Example 33 | 99% | Example 81 | 99.7% |
| Example 47 | 99% | Example 92 | 98.8% |
| Example 48 | 99% | Example 94 | 99.8% |
| Example 49 | 97% | Example 98 | 99% |
| Example 50 | 96% | Example 103 | 98% |
| Example 54 | 95% | | |

Test Example 5: Pharmacokinetics Evaluation

Pharmacokinetic experiment in mouse

Experimental Materials:

CB17-SCID mice were purchased from Beijing Vital River Laboratory Animal Technology Co., Ltd.
DMSO, HP-β-CD (hydroxypropyl-β-cyclodextrin), PEG400 (polyethylene glycol 400), and Vitamin E TPGS (D-α-tocopherol polyethylene glycol 1000 succinate) were purchased from Sigma.

Acetonitrile was purchased from Merck (USA).

Experimental Method:

Six female CB17-SCID mice (20 g to 30 g, aged 4 to 6 weeks) were divided randomly into two groups, with 3 mice in each group. In Group 1, the compound in a vehicle of an aqueous solution containing 5% dimethyl sulfoxide and 95% hydroxypropyl-β-cyclodextrin (10%, w/v) was administered by intravenous injection via tail vein. In Group 2, a corresponding dose of the compound in a vehicle of an aqueous solution containing 30% polyethylene glycol 400 and 70% D-α-tocopherol polyethylene glycol 1000 succinate (10%, w/v) was orally administered. The animals were fed normally with food and water before experiment. The mice in each group underwent intravenous blood collection before administration and at 0.083 h (intravenous injection group only), 0.25 h, 0.5 h, 1 h, 2 h, 4 h, 6 h, 8 h, and 24 h after administration. The collected whole blood samples were placed in K2EDTA anticoagulation tubes and centrifuged for 5 min (4000 rpm, 4°C) to collect the plasma for testing.

10 μL of the mouse plasma sample was taken, and 150 μL of acetonitrile solvent (containing the internal standard compound) was added to precipitate protein. After the resultant was vortexed for 0.5 min and centrifuged (4700 rpm, 4°C) for 15 min, the supernatant was subjected to 2-fold dilution with water containing 0.05% (v/v) FA and was then subjected to quantitative measurement in LC-MS/MS system (AB Sciex Triple Quad 6500+). A calibration standard curve was prepared in CB17-SCID mouse plasma along with quality control samples to determine the plasma concentration. As for a 10 × diluted sample, 2 μL of sample was taken and 18 μL of blank plasma was added. After being vortexed for 0.5 min, the mixture was added with 300 μL of acetonitrile solvent (containing the internal standard compound) so as to precipitate protein. The rest of the processing steps were the same as those for the undiluted samples.

PK test results were as shown in Table 6 below.

Table 6: PK of the compounds of the present disclosure in mouse

| Compound No. | Oral Dose mg/kg | Max. Drug Conc. ng/ml | Peak Time of Drug Conc. (h) | Drug Elimination Half-life (h) | Drug Exposure ng × h/ml |
|---|---|---|---|---|---|
| Example 1 | 80 | 14640 | 2.3 | 3.7 | 211819 |
| Example 3 | 80 | 15438 | 2.8 | 32 | 271377 |
| Example 48 | 30 | 17657 | 2 | 7.6 | 203497 |
| Example 50 | 25 | 4076 | 1 | 4.7 | 18187 |
| Example 54 | 25 | 6698 | 1 | 3.1 | 47683 |
| Example 68 | 25 | 14798 | 1.3 | 5 | 156699 |
| Example 70 | 25 | 32840 | 0.75 | 5 | 137892 |
| Example 81 | 25 | 13563 | 5 | 10 | 223309 |
| Example 92 | 25 | 8038 | 1 | 3.6 | 40080 |
| Example 94 | 25 | 19257 | 1.3 | 3.5 | 125779 |

The embodiments of the present disclosure have been described above, but the present disclosure is not limited to the embodiments described above. Any modifications, equivalent substitutions, improvements and the like made within the spirit and principle of the present disclosure shall be encompassed in the protection scope of the present disclosure.

## Claims

**1.** A compound represented by formula (I) or a pharmaceutically acceptable salt thereof,

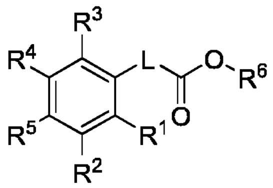

(I)

wherein

$R^1$ is

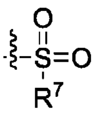, 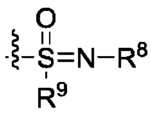, 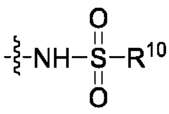, 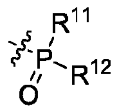, or 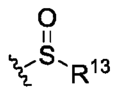;

$R^7$ is

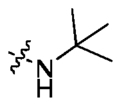,

$C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{7a}$;

$R^{7a}$ is F, Cl, Br, I, OH, CN, =O, $NO_2$, or the following group optionally substituted with one or more $R^{7b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{7b}$ is F, Cl, Br, I, OH, CN, =O, $NO_2$, or the following group optionally substituted with one or more $R^{7c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{7c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^8$ is H, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{8a}$;

$R^{8a}$ is F, Cl, Br, I, OH, CN, =O, $NO_2$, or the following group optionally substituted with one or more $R^{8b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{8b}$ is F, Cl, Br, I, OH, CN, =O, $NO_2$, or the following group optionally substituted with one or more $R^{8c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{8c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^9$ is $C_1$-$C_{10}$ alkyl, $C_3$-$C_{10}$ cycloalkyl, 3- to 10-membered heterocyclyl, $C_6$-$C_{10}$ aryl, or 5-to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{9a}$;

$R^{9a}$ is F, Cl, Br, I, OH, CN, =O, $NO_2$, or the following group optionally substituted with one or more $R^{9b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{9b}$ is F, Cl, Br, I, OH, CN, =O, $NO_2$, or the following group optionally substituted with one or more $R^{9c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{9c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^{10}$ is $C_1$-$C_{10}$ alkyl, $C_3$-$C_{10}$ cycloalkyl, 3- to 10-membered heterocyclyl, $C_6$-$C_{10}$ aryl, or 5-to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{10a}$;

$R^{10A}$ is F, Cl, Br, I, OH, CN, =O, $NO_2$, or the following group optionally substituted with one or more $R^{10b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{10b}$ is F, Cl, Br, I, OH, CN, =O, $NO_2$, or the following group optionally substituted with one or more $R^{10c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{10c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^{11}$, $R^{12}$, and $R^{13}$ are independently $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{11a}$; or

$R^{11}$ and $R^{12}$, together with the atoms to which they are attached, form 3- to 10-membered heterocyclyl, said 3- to 10-membered heterocyclyl is optionally substituted with one or more $R^{11a}$;

$R^{11a}$ is F, Cl, Br, I, OH, CN, =O, $NO_2$, or the following group optionally substituted with one or more $R^{11b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{11b}$ is F, Cl, Br, I, OH, CN, =O, $NO_2$, or the following group optionally substituted with one or more $R^{11c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{11c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$R^2$, $R^3$, and $R^4$ are independently H, F, Cl, Br, I, OH, CN, $NO_2$, or the following group optionally substituted with one or more $R^a$: $NH_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^a$ is F, Cl, Br, I, OH, CN, =O, $NO_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

Lis

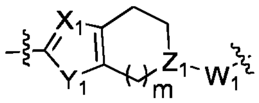 , 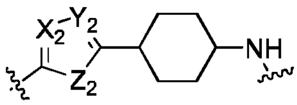 , or 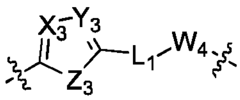 ;

$X_1$, $X_2$, $X_3$, $Y_2$, $Y_3$, and $Z_1$ are independently $CR^{15}$ or N;

$Y_1$, $Z_2$, and $Z_3$ are independently O, NH, or S;

m is 0, 1, or 2;

$R^{15}$ is H, F, Cl, Br, I, OH, CN, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{15a}$;

said $R^{15a}$ is F, Cl, Br, I, OH, CN, =O, $NO_2$, $NH_2$, SH, $C_1$-$C_{10}$ alkyl, $C_3$-$C_{10}$ cycloalkyl, 3-to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$L_1$ is phenyl, $C_7$-$C_{12}$ spirocyclyl, $C_6$-$C_{12}$ fused cyclyl, $C_5$-$C_{12}$ bridged cyclyl, 7- to 12-membered spiroheterocyclyl,

6- to 12-membered fused heterocyclyl, 3- to 10-membered monocyclic heterocyclyl, or 5- to 12-membered bridged heterocyclyl, said 7- to 12-membered spiroheterocyclyl, 6- to 12-membered fused heterocyclyl, 3- to 10-membered monocyclic heterocyclyl, or 5- to 12-membered bridged heterocyclyl contains 1 to 3 heteroatoms selected from O, N, or S; said $C_7$-$C_{12}$ spirocyclyl, $C_6$-$C_{12}$ fused cyclyl, $C_5$-$C_{12}$ bridged cyclyl, 7- to 12-membered spiroheterocyclyl, 6- to 12-membered fused heterocyclyl, 3- to 10-membered monocyclic heterocyclyl, or 5- to 12-membered bridged heterocyclyl is optionally substituted with one or more $R^{28}$;

said $R^{28}$ is F, Cl, Br, I, OH, CN, $NO_2$, or the following group optionally substituted with one or more $R^{28a}$: $NH_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{28a}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;

$W_1$ and $W_4$ are independently a chemical bond, $NR^{16}$, or O;

$R^{16}$ is H, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{16a}$;

said $R^{16a}$ is F, Cl, Br, I, OH, CN, =O, $NO_2$, $NH_2$, SH, $C_1$-$C_{10}$ alkyl, $C_3$-$C_{10}$ cycloalkyl, 3-to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^5$ is

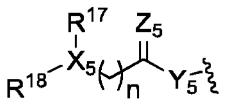, 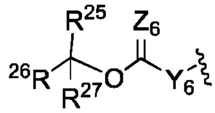, 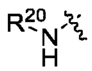, 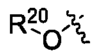,

3- to 10-membered heterocyclyl, or 5- to 10-membered heteroaryl; said 3- to 10-membered heterocyclyl or 5- to 10-membered heteroaryl is optionally substituted with one or more $R^{20a}$;

n is 0 or 1;

$X_5$ is N or $CR^{21}$;

$Y_5$ and $Y_6$ are independently a chemical bond, NH, or $CHR^{22}$;

$Z_5$ and $Z_6$ are independently O or $NR^{23}$;

$R^{17}$, $R^{18}$, $R^{25}$, and $R^{26}$ are independently H, $C_1$-$C_{10}$ alkyl, $C_3$-$C_{10}$ cycloalkyl, 3- to 10-membered heterocyclyl, $C_6$-$C_{10}$ aryl, 5- to 10-membered heteroaryl, $C_6$-$C_{10}$ aryloxy, or 5- to 10-membered heteroaryloxy, said $C_1$-$C_{10}$ alkyl, $C_3$-$C_{10}$ cycloalkyl, 3- to 10-membered heterocyclyl, $C_6$-$C_{10}$ aryl, 5- to 10-membered heteroaryl, $C_6$-$C_{10}$ aryloxy, or 5- to 10-membered heteroaryloxy is optionally substituted with one or more $R^{17a}$;

$R^{17a}$ is F, Cl, Br, I, OH, CN, =O, $NO_2$, or the following group optionally substituted with one or more $R^{17b}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^{17b}$ is F, Cl, Br, I, OH, CN, or the following group optionally substituted with one or more $R^{17c}$: $C_1$-$C_{10}$ alkyl, $C_3$-$C_{10}$ cycloalkyl, 3- to 10-membered heterocyclyl, or $C_6$-$C_{10}$ aryl;

$R^{17c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl; or

$R^{17}$ and $R^{18}$, together with the atom $X_5$ to which they are attached, form 3- to 10-membered heterocyclyl or $C_3$-$C_{10}$ cycloalkyl, said 3- to 10-membered heterocyclyl or $C_3$-$C_{10}$ cycloalkyl is optionally substituted with one or more $R^{17a}$;

$R^{20}$ is the following group optionally substituted with one or more $R^{20a}$: 5- to 10-membered heteroaryl, -C(O)-5- to 10-membered heteroaryl, 5- to 10-membered heterocyclyl, $C_6$-$C_{10}$ aryl, or -C(O)-$C_6$-$C_{10}$ aryl;

$R^{20a}$ is F, Cl, Br, I, OH, CN, =O, $NO_2$, $NH_2$, SH, $C_1$-$C_{10}$ alkyl, $C_1$-$C_{10}$ alkoxy, $C_3$-$C_{10}$ cycloalkyl, 3- to 10-membered heterocyclyl, $C_6$-$C_{10}$ aryl, or 5- to 10-membered heteroaryl; said $C_1$-$C_{10}$ alkyl, $C_1$-$C_{10}$ alkoxy, $C_3$-$C_{10}$ cycloalkyl, 3- to 10-membered heterocyclyl, $C_6$-$C_{10}$ aryl, or 5- to 10-membered heteroaryl is optionally substituted with one or more $R^{20b}$;

$R^{20b}$ is F, Cl, Br, I, OH, CN, =O, $NO_2$, $NH_2$, SH, or $C_3$-$C_{10}$ cycloalkyl;

$R^{21}$, $R^{22}$, and $R^{27}$ are independently H, $C_1$-$C_{10}$ alkyl, or $C_6$-$C_{10}$ aryl, said $C_1$-$C_{10}$ alkyl or $C_6$-$C_{10}$ aryl is optionally substituted with one or more $R^{21a}$;

$R^{21a}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl,

$C_6$-$C_{10}$ aryl, or 5- to 10-membered heteroaryl;
$R^{23}$ is H, CN, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl, said $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl is optionally substituted with one or more $R^{23a}$;
$R^{23a}$ is F, Cl, Br, I, OH, CN, =O, $NO_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy; or
$R^4$ and $R^5$, together with the atoms to which they are attached, form 4- to 6-membered heterocyclyl, phenyl, or 5- to 6-membered heteroaryl, said 4- to 6-membered heterocyclyl, phenyl, or 5- to 6-membered heteroaryl is optionally substituted with one or more $R^{24a}$;
$R^{24a}$ is F, Cl, Br, I, OH, CN, =O, $NO_2$, or the following group optionally substituted with one or more $R^{24b}$: $NH_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;
$R^{24b}$ is F, Cl, Br, I, OH, CN, =O, $NO_2$, or the following group optionally substituted with one or more $R^{24c}$: $NH_2$, SH, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;
$R^{24c}$ is F, Cl, Br, I, OH, CN, =O, $NO_2$, $NH_2$, SH, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;
$R^6$ is H or the following group optionally substituted with one or more $R^{6a}$: $NH_2$, $C_1$-$C_{10}$ alkyl, $C_3$-$C_{10}$ cycloalkyl, 3- to 10-membered heterocyclyl, $C_6$-$C_{10}$ aryl, or 5- to 10-membered heteroaryl;
$R^{6a}$ is F, Cl, Br, I, OH, CN, =O, $NO_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy,
provided that when $L_1$ is phenyl, or when $R^5$ is 3- to 10-membered heterocyclyl or 5- to 10-membered heteroaryl, $R^7$ is not

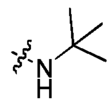.

2. The compound or the pharmaceutically acceptable salt thereof according to claim 1, being a compound of formula (Ia) or a pharmaceutically acceptable salt thereof,

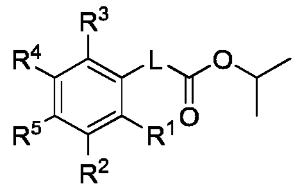

(Ia)

wherein $R^1$, $R^2$, $R^3$, $R^4$, and $R^5$ are as defined in claim 1, L is

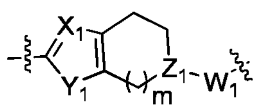

or

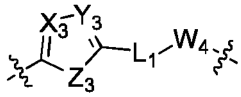

;

$X_1$, $X_3$, $Y_1$, $Y_3$, $Z_1$, $Z_3$, $W_1$, and m are as defined in claim 1;
said

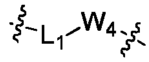

is

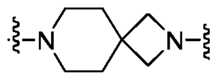,
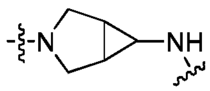,
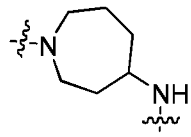,

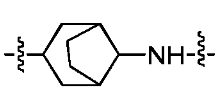,
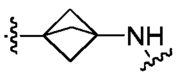,
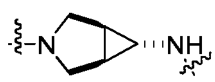, or
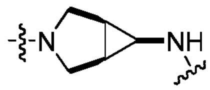;

provided that when $L_1$ is 3- to 10-membered monocyclic heterocyclyl optionally substituted with $R^{28}$, $W_4$ is not a chemical bond, wherein $R^{28}$ is as defined in claim 1.

**3.** The compound or the pharmaceutically acceptable salt thereof according to claim 1, being a compound of formula (Ia) or a pharmaceutically acceptable salt thereof,

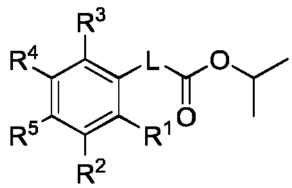

(Ia)

wherein L is

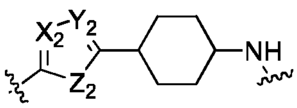;

$R^1$, $R^2$, $R^3$, $R^4$, $R^5$, $X_2$, $Y_2$ and $Z_2$ are as defined in claim 1,
provided that when $R^1$ is tert-butylsulfamoyl, $R^5$ is

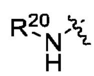,

wherein $R^{20}$ is 5- to 10-membered heteroaryl, 5- to 10-membered heterocyclyl, or C(O)-$C_6$-$C_{10}$ aryl, said 5- to 10-membered heteroaryl is pyrazolyl, isoxazolyl, 1,2,3-triazolyl, pyridazinyl, benzopyrazolyl, or pyridopyrazolyl, said 5- to 10-membered heterocyclyl is dihydropyridinyl, and said $C_6$-$C_{10}$ aryl is phenyl.

**4.** The compound or the pharmaceutically acceptable salt thereof according to claim 3, wherein the compounds of formula (Ia) do not comprise

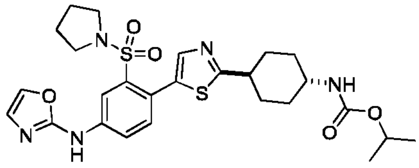,

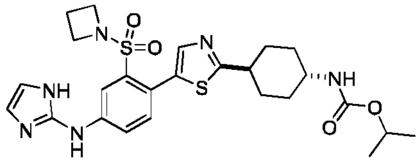, or 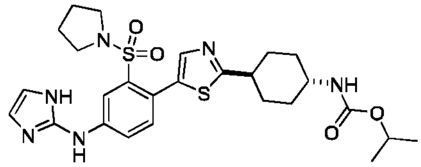.

5. The compound or the pharmaceutically acceptable salt thereof according to claim 1, being a compound of formula (Ib) or a pharmaceutically acceptable salt thereof,

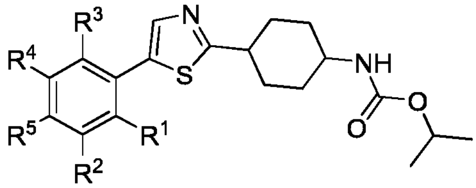

(Ib)

wherein $R^2$, $R^3$, $R^4$, and $R^5$ are as defined in claim 1;

$R^1$ is

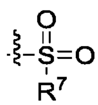, 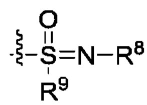, 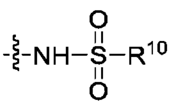, 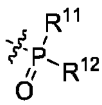, or 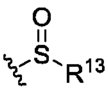,

$R^7$ is

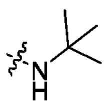,

$C_1$-$C_{10}$ alkyl, $C_3$-$C_{10}$ cycloalkyl, or $C_6$-$C_{10}$ aryl, said $C_1$-$C_{10}$ alkyl is optionally substituted with one or more $R^{7a}$; wherein $R^{7a}$, $R^8$, $R^9$, $R^{10}$, $R^{11}$, $R^{12}$, and $R^{13}$ are as defined in claim 1,
provided that $R^1$ is not tert-butylsulfamoyl.

6. The compound or the pharmaceutically acceptable salt thereof according to claim 1, being a compound of formula (II) or a pharmaceutically acceptable salt thereof,

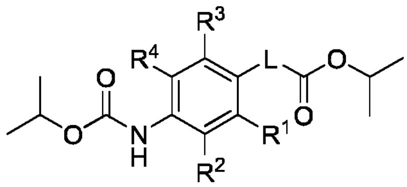

(II)

wherein L is

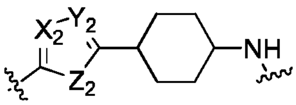

;

$X_2$, $Y_2$, $Z_2$, $R^1$, $R^2$, $R^3$, and $R^4$ are as defined in claim 1,
provided that when $R^1$ is tert-butylsulfamoyl and $X_2$ is $CR^{15}$ together with $Y_2$ is N, $Z_2$ is not S, and $R^{15}$ is as defined in claim 1.

**7.** The compound or the pharmaceutically acceptable salt thereof according to claim 1, being a compound of formula (IIa) or a pharmaceutically acceptable salt thereof,

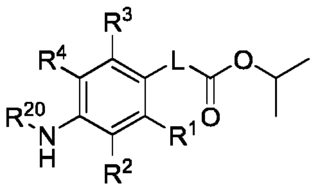

(IIa)

wherein $R^1$, $R^2$, $R^3$, $R^4$, and L are as defined in claim 1; $R^{20}$ is 5- to 10-membered heteroaryl, 5- to 10-membered heterocyclyl, or C(O)-$C_6$-$C_{10}$ aryl, said 5- to 10-membered heteroaryl is pyrazolyl, isoxazolyl, 1,2,3-triazolyl, pyridazinyl, benzopyrazolyl, or pyridopyrazolyl, said 5- to 10-membered heterocyclyl is dihydropyridinyl, and said $C_6$-$C_{10}$ aryl is phenyl,
provided that $R^1$ is not tert-butylsulfamoyl.

**8.** The compound or the pharmaceutically acceptable salt thereof according to claim 1, being a compound of formula (Ia) or a pharmaceutically acceptable salt thereof,

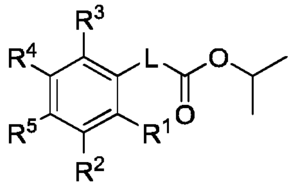

(Ia)

wherein $R^1$ is

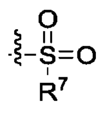 , 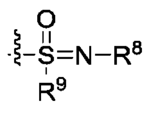 , or 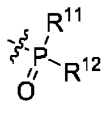

; $R^7$ is $C_1$-$C_{10}$ alkyl, $C_3$-$C_{10}$ cycloalkyl, or $C_6$-$C_{10}$ aryl, said $C_1$-$C_{10}$ alkyl is optionally substituted with one or more $R^{7a}$;

$R^2$, $R^3$, $R^4$, $R^8$, $R^9$, $R^{11}$, and $R^{12}$ are as defined in claim 1,
Lis

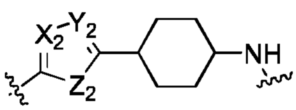

,

$X_2$, $Y_2$, and $Z_2$ are as defined in claim 1;
$R^5$ is

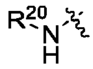,

and $R^{20}$ is as defined in claim 1.

**9.** The compound or the pharmaceutically acceptable salt thereof according to claim 1, being a compound of formula (IV) or a pharmaceutically acceptable salt thereof,

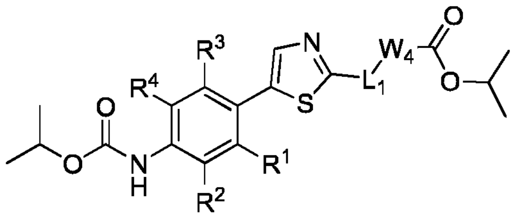

(IV)

wherein $R^1$, $R^2$, $R^3$, and $R^4$ are as defined in claim 1; said

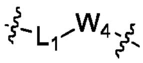

is

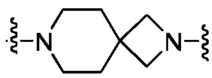

,

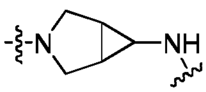, 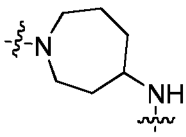, 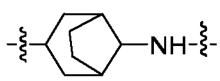, 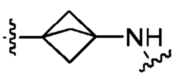, 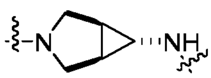,

or

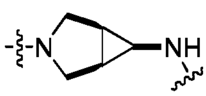.

**10.** The compound or the pharmaceutically acceptable salt thereof according to claim 1, being a compound of formula (VIII) or a pharmaceutically acceptable salt thereof,

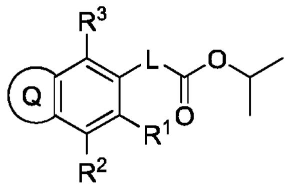

(VIII)

wherein ring Q is 4- to 6-memere heterocyclyl, phenyl, or 5- to 6-memere heteroaryl, said 4- to 6-membered heterocyclyl, phenyl, or 5- to 6-membered heteroaryl is optionally substituted with one or more $R^{24a}$; and $R^1$, $R^2$, $R^3$, L, and $R^{24a}$ are as defined in claim 1.

**11.** The compound or the pharmaceutically acceptable salt thereof according to claim 1, being a compound of formula 1-1, formula 1-7 or formula 1-11, or a pharmaceutically acceptable salt thereof,

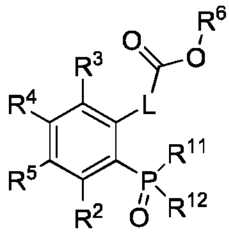

**I-1** ,

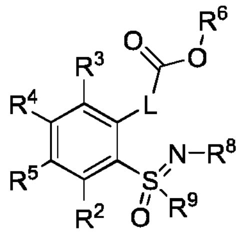

**I-7** , or

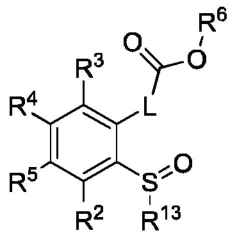

**I-11**

wherein L, $R^2$, $R^3$, $R^4$, $R^5$, $R^6$, $R^8$, $R^9$, $R^{11}$, $R^{12}$, and $R^{13}$ are as defined in claim 1.

**12.** The compound or the pharmaceutically acceptable salt thereof according to claim 1, being a compound of formula 1-13, formula 1-23 or formula 1-27, or a pharmaceutically acceptable salt thereof,

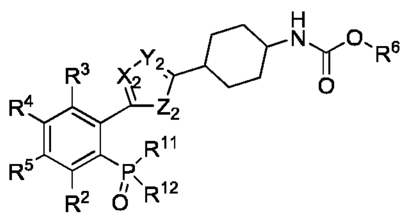

**I-13** ,

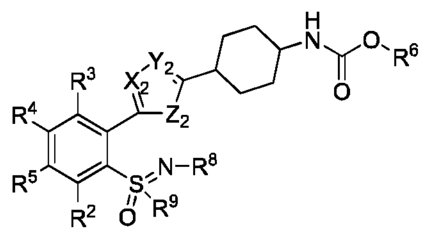

**I-23** ,

or

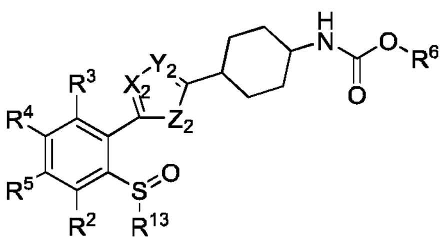

**I-27**

wherein $R^2$, $R^3$, $R^4$, $R^5$, $R^6$, $R^8$, $R^9$, $R^{11}$, $R^{12}$, $R^{13}$, $X_2$, $Z_2$, and $Y_2$ are as defined in claim 1.

**13.** The compound or the pharmaceutically acceptable salt thereof according to claim 1, being the following compound or a pharmaceutically acceptable salt thereof,

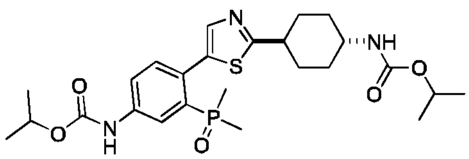 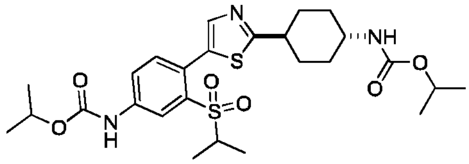

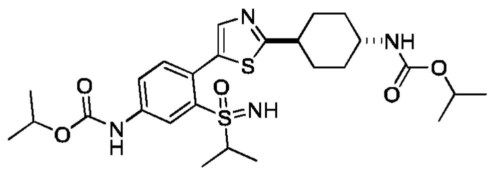 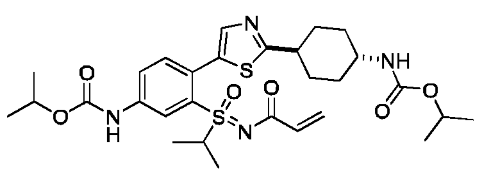

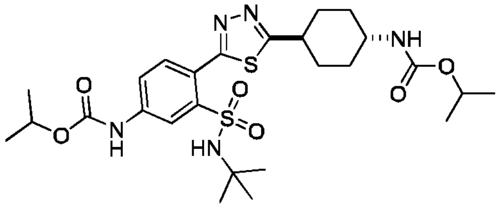 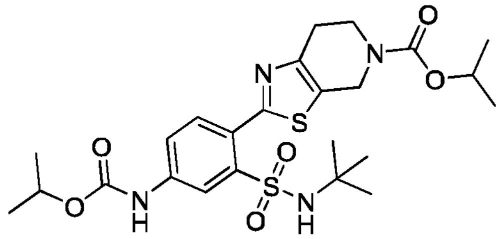

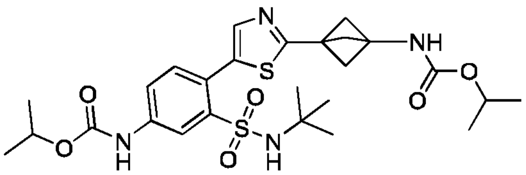 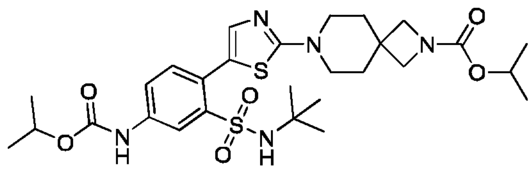

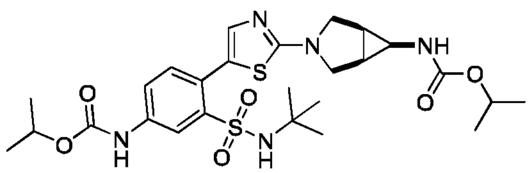 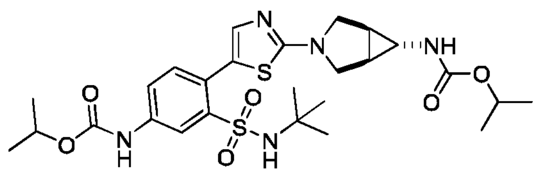

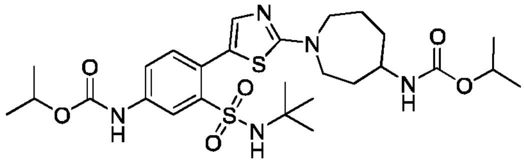 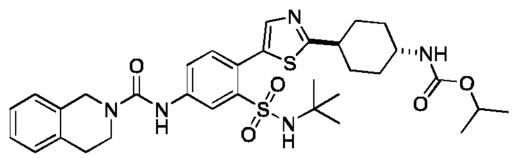

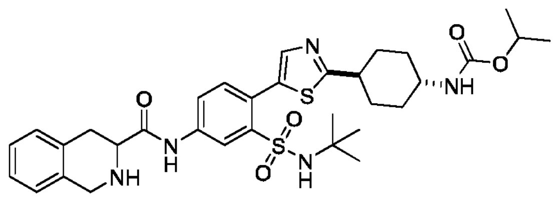 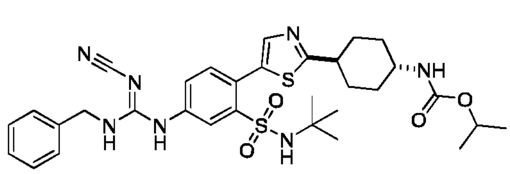

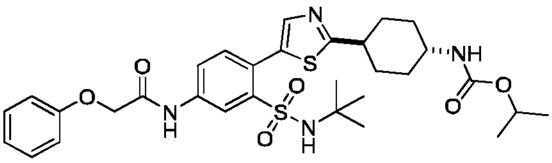
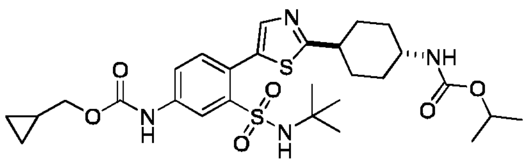

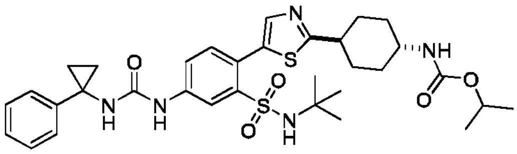
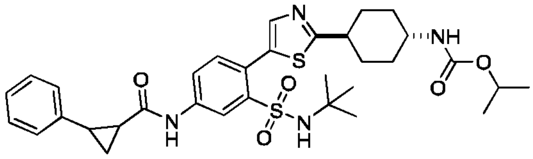

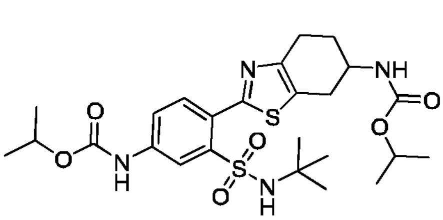
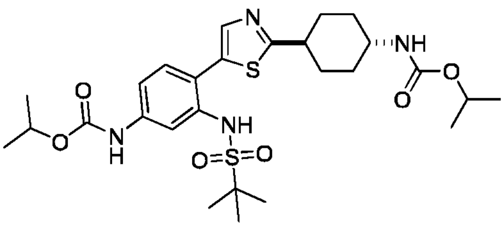

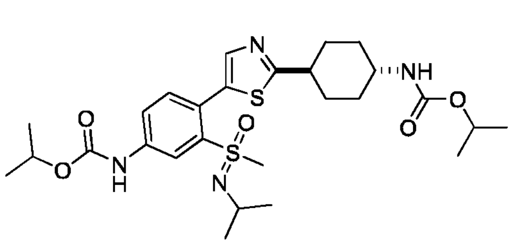
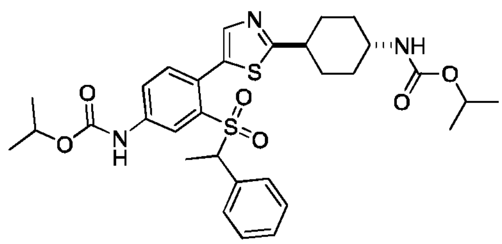

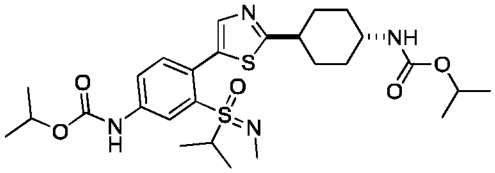
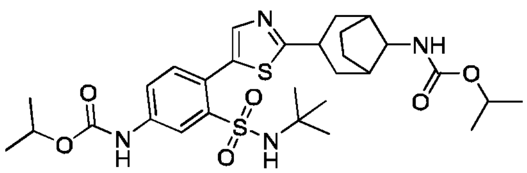

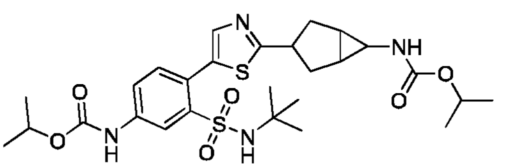
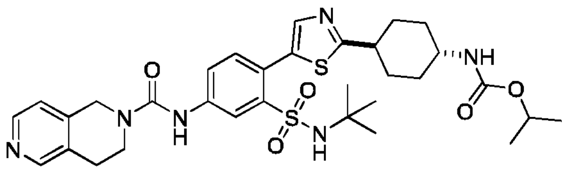

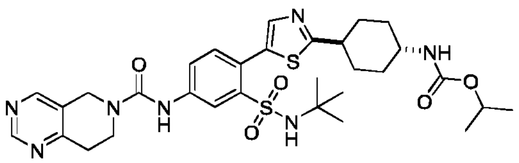
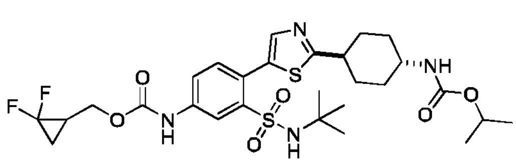

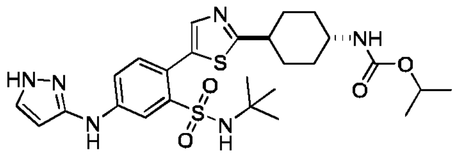 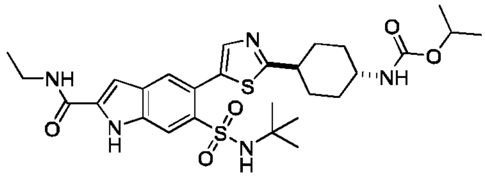

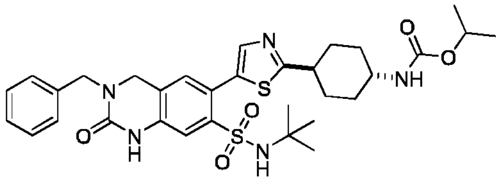 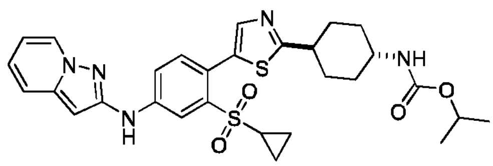

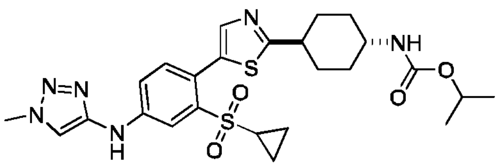 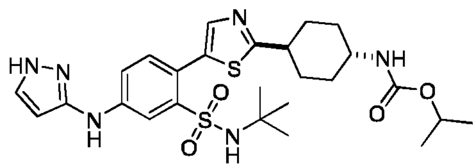

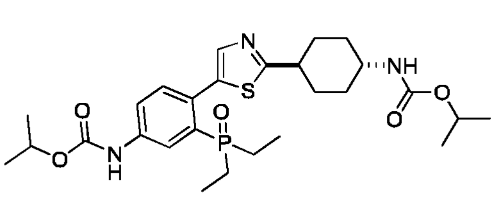 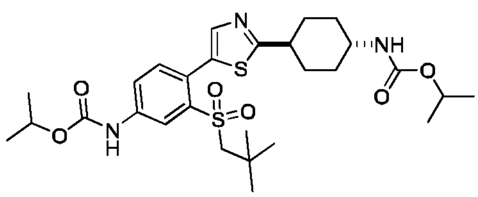

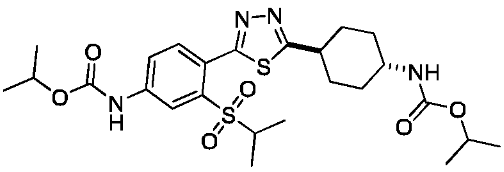 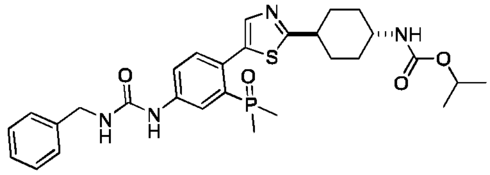

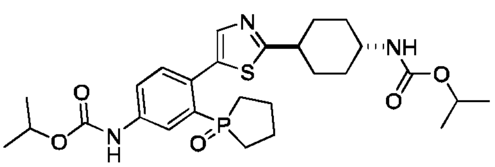 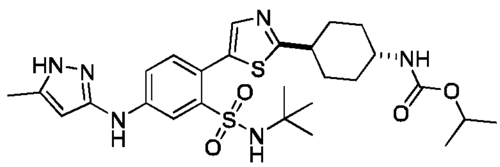

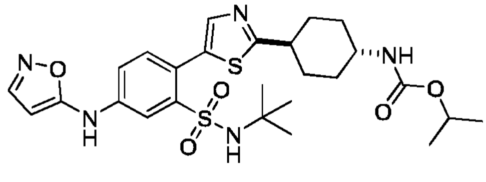 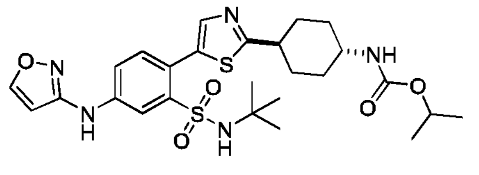

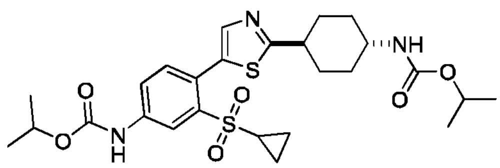
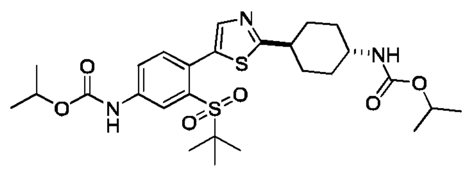

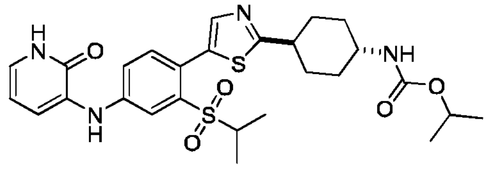
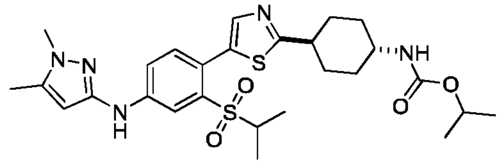

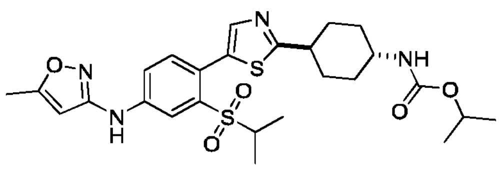
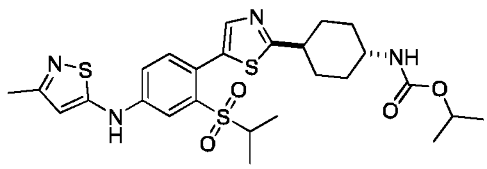

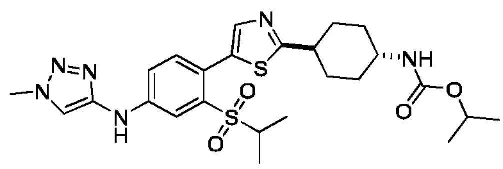
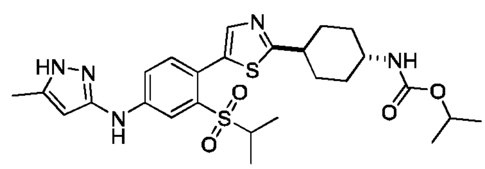

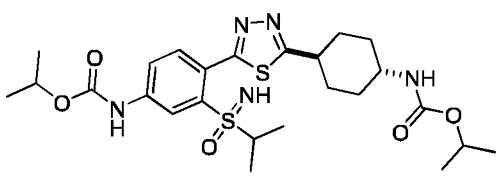
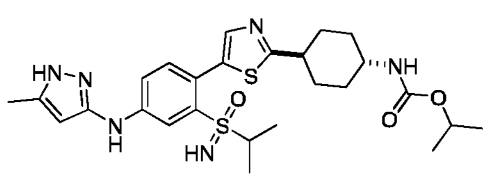

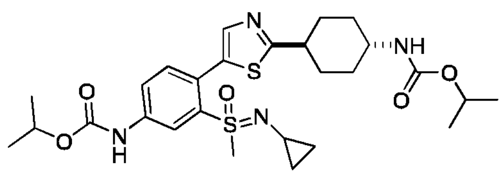
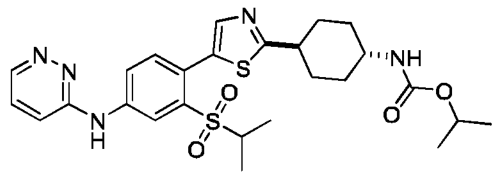

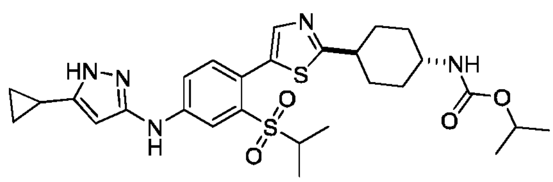
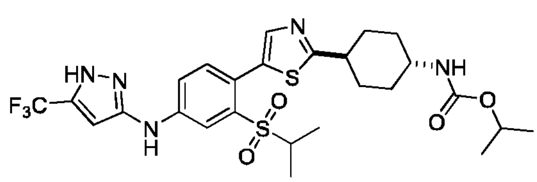

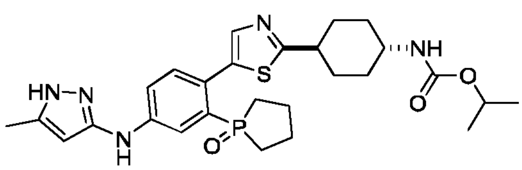
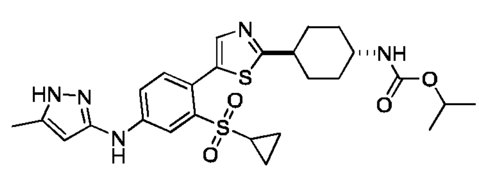

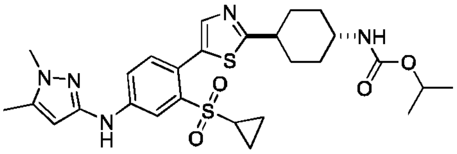
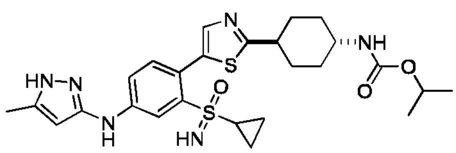

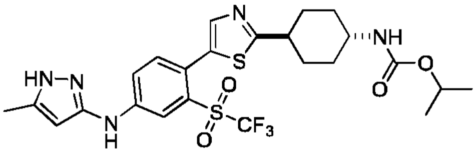
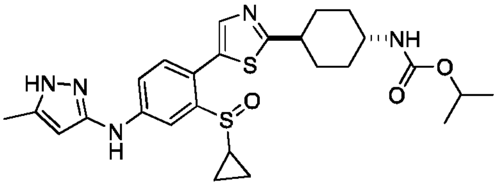

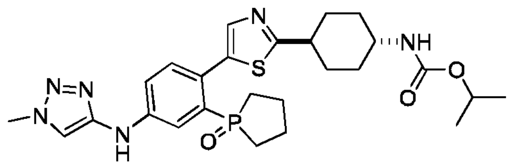
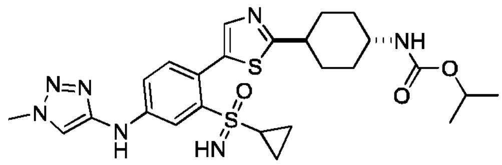

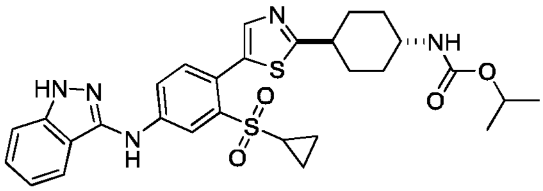
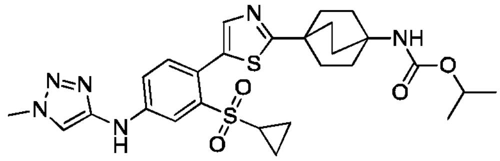

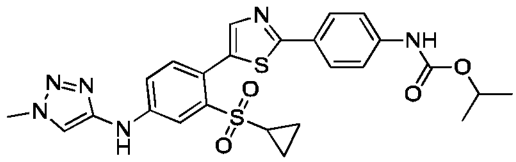
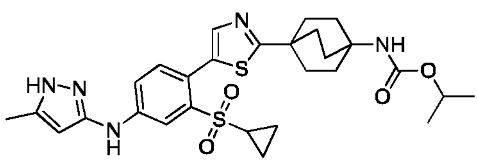

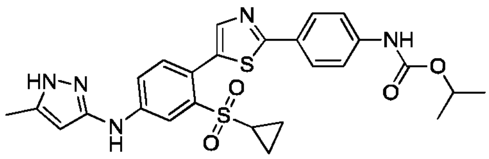
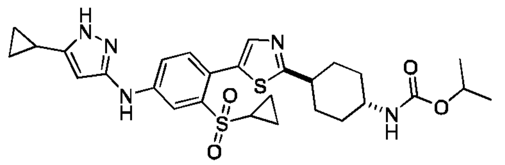

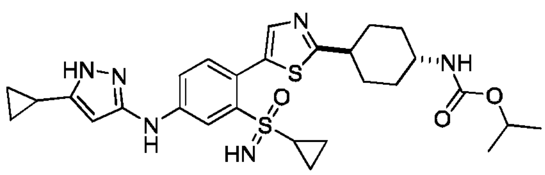
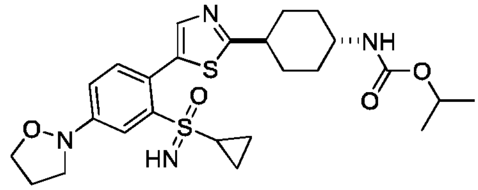

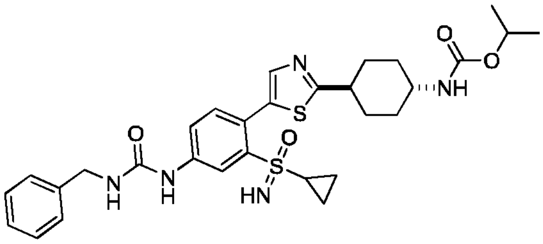

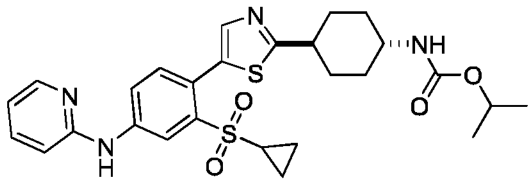

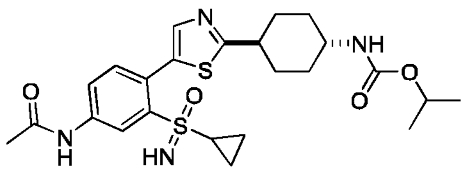

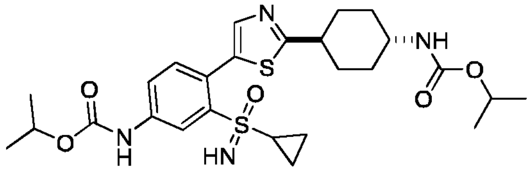

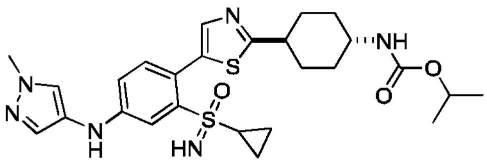

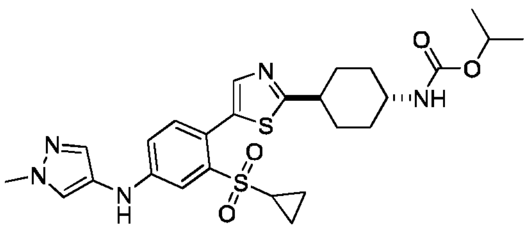

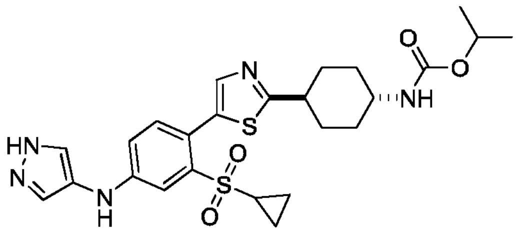

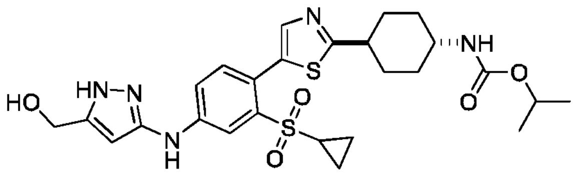

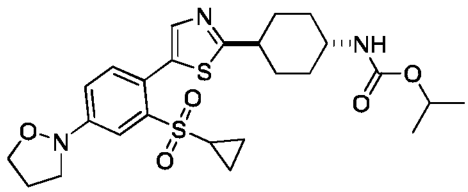

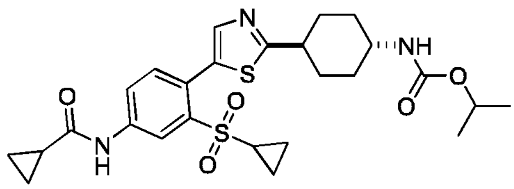

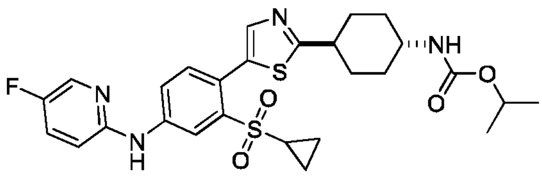

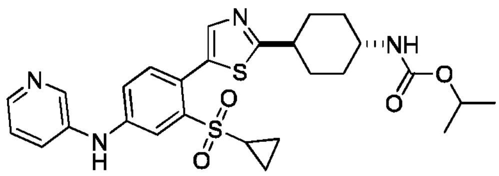

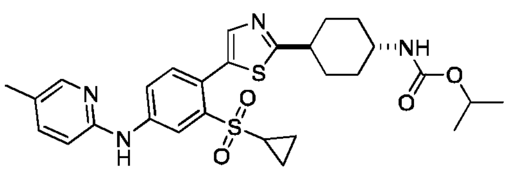

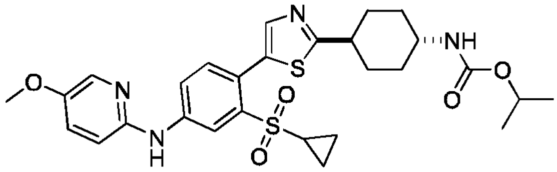

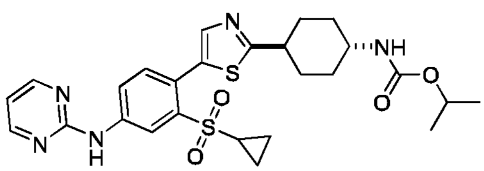
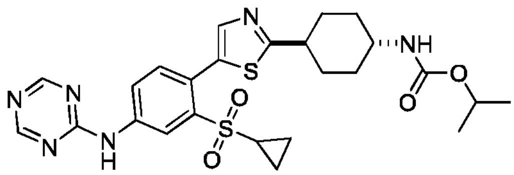

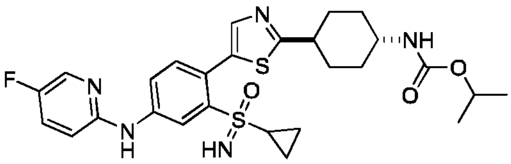
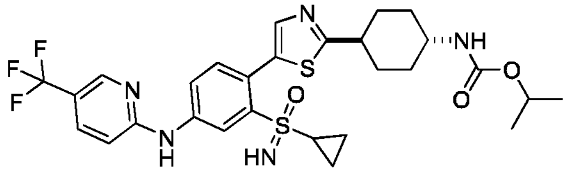

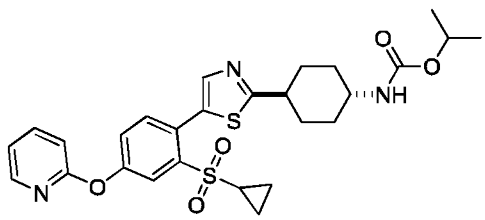
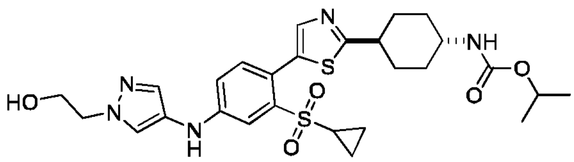

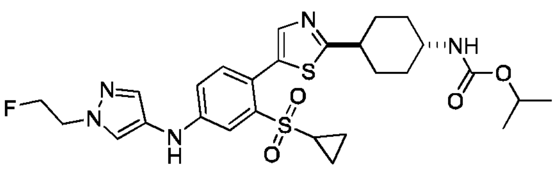
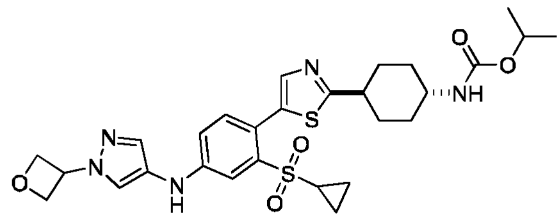

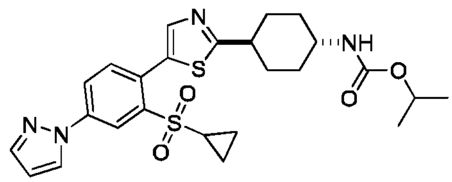
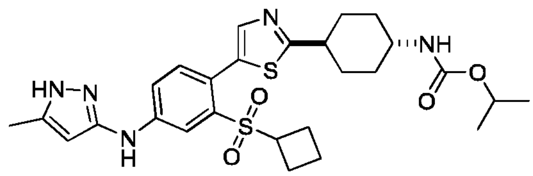

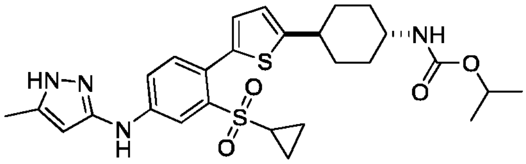
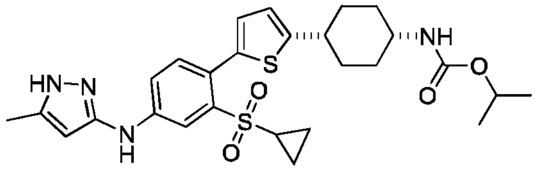

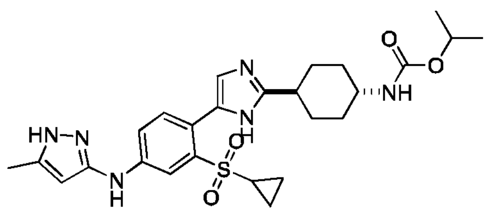
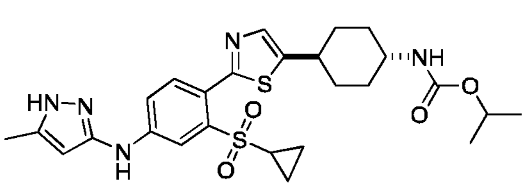

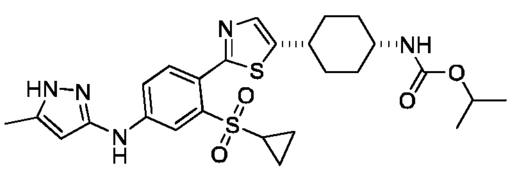
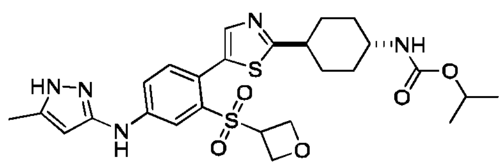

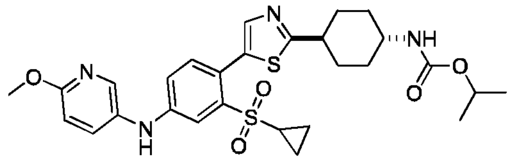
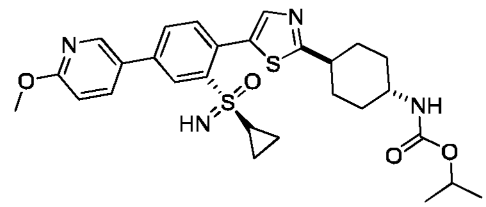

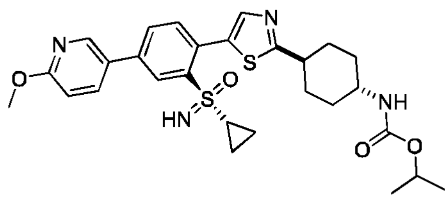
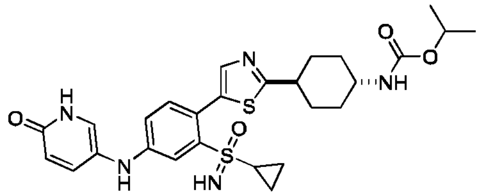

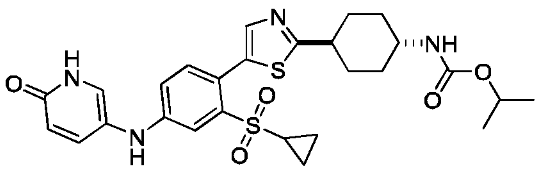
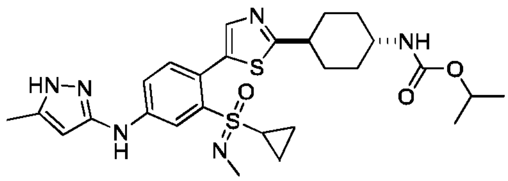

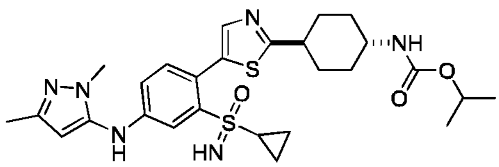
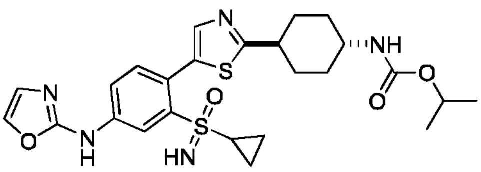

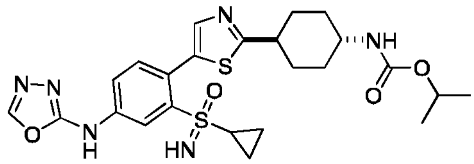
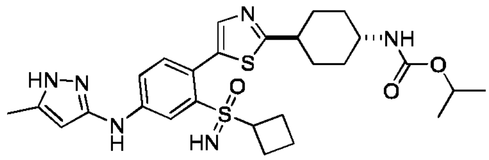

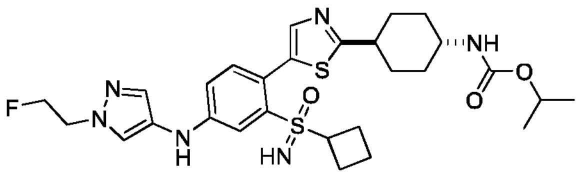
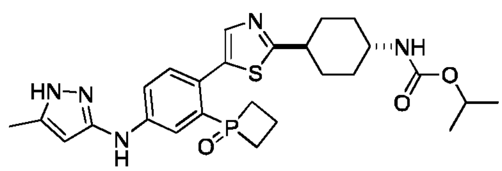

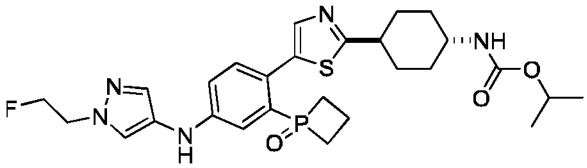
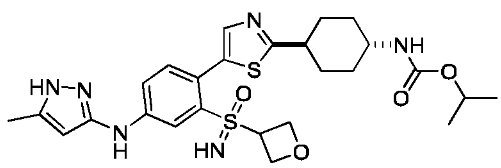

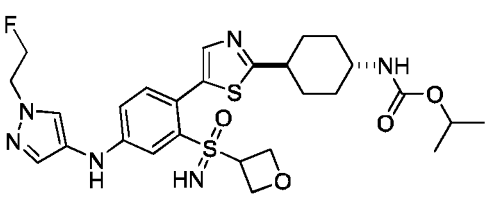
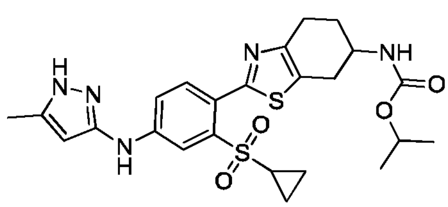

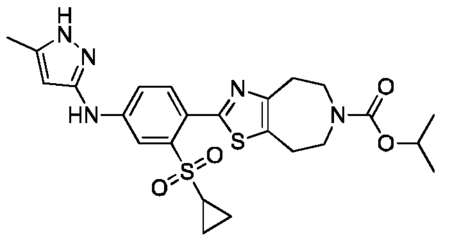

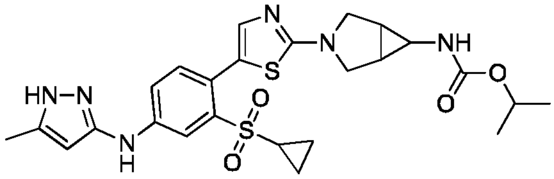

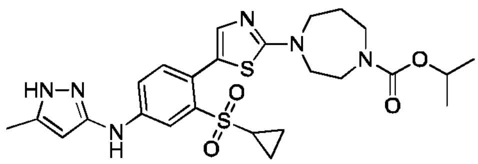

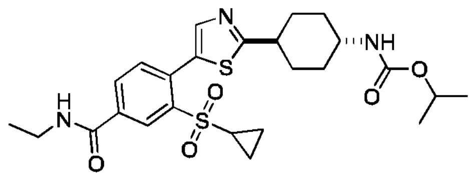

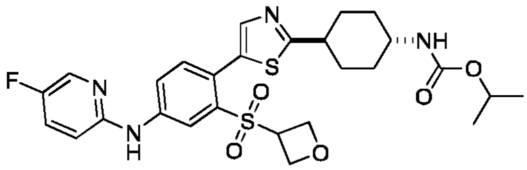

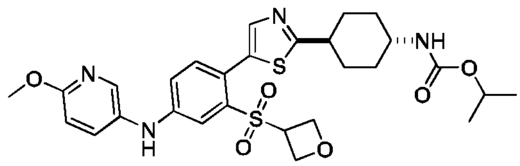

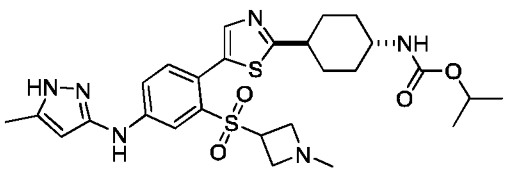

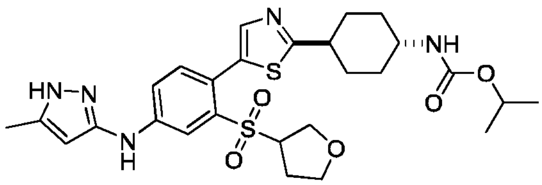

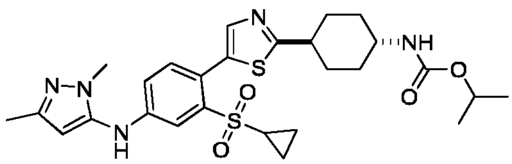

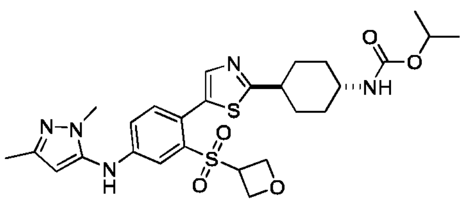

,

or

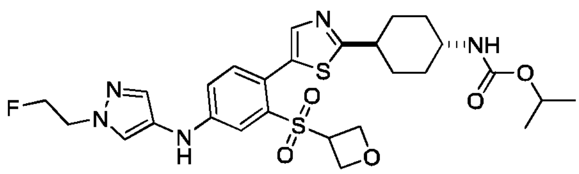

.

14. A pharmaceutical composition, comprising the compound or the pharmaceutically acceptable salt thereof according to any one of claims 1 to 13 and a pharmaceutically acceptable excipient.

15. Use of the compound or the pharmaceutically acceptable salt thereof according to any one of claims 1 to 13 in the preparation of a medicament for preventing or treating an RAD51-associated disease.

16. The use according to claim 15, wherein the RAD51-associated disease is a tumor or an autoimmune disease.

## INTERNATIONAL SEARCH REPORT

International application No.
**PCT/CN2021/076939**

**A. CLASSIFICATION OF SUBJECT MATTER**

C07D 417/04(2006.01)i; C07D 417/14(2006.01)i; C07D 277/28(2006.01)i; C07D 277/42(2006.01)i; C07D 277/60(2006.01)i; C07D 513/04(2006.01)i; C07D 471/10(2006.01)i; A61K 31/425(2006.01)i; A61P 35/00(2006.01)i; A61P 37/00(2006.01)i; A61P 25/00(2006.01)i

According to International Patent Classification (IPC) or to both national classification and IPC

**B. FIELDS SEARCHED**

Minimum documentation searched (classification system followed by classification symbols)

C07D277/-, C07D417/-, A61K31/425, A61P35/-, A61P37/-, A61P25/-

Documentation searched other than minimum documentation to the extent that such documents are included in the fields searched

Electronic data base consulted during the international search (name of data base and, where practicable, search terms used)

DWPI, SIPOABS, CNABS, CNTXT, CNKI, REGISTRY(STN), CAPLUS(STN): 噻唑, 苯基, 磺酰, 环己基, 氨基, 抑制剂, rad51, 根据式I化合物进行的结构式检索, structural formula search according to compound of formula I

**C. DOCUMENTS CONSIDERED TO BE RELEVANT**

| Category* | Citation of document, with indication, where appropriate, of the relevant passages | Relevant to claim No. |
|---|---|---|
| X | WO 2019051465 A1 (CYTEIR THERAPEUTICS, INC.) 14 March 2019 (2019-03-14) the abstract, claims, and embodiments 7, 10, 22-45, 47-55, 57-58, 66A, 66B, 67A, 67B and 70-73 | 1 (in part) and 13-16 (in part) |
| Y | WO 2019051465 A1 (CYTEIR THERAPEUTICS, INC.) 14 March 2019 (2019-03-14) the abstract, claims, and embodiments 7, 10, 22-45, 47-55, 57-58, 66A, 66B, 67A, 67B and 70-73 | 1 (in part), 3 and 4 (in part), 6 and 7 (in part), 13-16 (in part) |
| A | WO 2019051465 A1 (CYTEIR THERAPEUTICS, INC.) 14 March 2019 (2019-03-14) the abstract, claims, and embodiments 7, 10, 22-45, 47-55, 57-58, 66A, 66B, 67A, 67B and 70-73 | 5 (in part), 8 (in part), 11 and 12 (in part) |
| Y | WO 2019014315 A1 (CYTEIR THERAPEUTICS, INC.) 17 January 2019 (2019-01-17) the abstract, and claim 1 | 1 (in part), 3 and 4 (in part), 6 and 7 (in part), 13-16 (in part) |
| PX | WO 2020186006 A1 (CYTEIR THERAPEUTICS, INC.) 17 September 2020 (2020-09-17) the abstract, claims, and description, table 1 compounds 3-15, 17-22, 38-43, 92, 97, 98, 110, 148, 151, 153, 173 and 174 | 1 (in part), 3 and 4 (in part), 6 and 7 (in part), 13-16 (in part) |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 May 2021** | **21 May 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/076939**

**Box No. III Observations where unity of invention is lacking (Continuation of item 3 of first sheet)**

This International Searching Authority found multiple inventions in this international application, as follows:

[1] This application sets forth the compound shown in formula (I) or a pharmaceutically acceptable salt thereof (claims 1-13), a pharmaceutical composition thereof (claim 14) and a pharmaceutical use thereof (claims 15 and 16).

[2] When the substituent L takes a different structure, the shared structure between the compounds of formula (I) - the oxygen carbonyl and phenyl structures being attached - cannot constitute a technical feature that distinguishes them from the prior art, for example: the document WO2019051465A1 (publication date: March 14th, 2019) already discloses alkoxycarbonylaminocyclohexylthiazole benzenesulfonyl compounds that can be used as RAD51 inhibitors (see the claims and examples of this document, especially embodiment 44). Moreover, these compounds do not come under the same class of compounds, so that the technical solutions constituted in claims 1-16 when the substituents L take different structures do not share a same or corresponding specific technical feature among them, and therefore do not comply with the requirement of unity of invention as defined in PCT Rule 13.1.

[3] Based on the information specifically disclosed in the specification, the technical solutions in claims 1-16 can be divided into the following 12 groups.

[4] Group 1, relates to L selected from 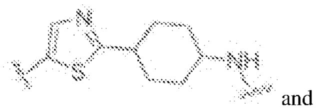 and $R^4$, $R^5$ not forming a ring structure together with the atoms to which they are attached, a compound of formula (I) or a pharmaceutically acceptable salt thereof, a pharmaceutical composition thereof, and a pharmaceutical use thereof, comprising claims 1 (in part), 3-8 (in part), and 11-16 (in part).

[5] Group 2, relates to L selected from 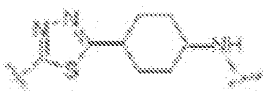 The compound of formula (I) or a pharmaceutically acceptable salt thereof, a pharmaceutical composition thereof, and a pharmaceutical use thereof, comprising claims 1 (in part), 3 and 4 (in part), 6-8 (in part), and 10-16 (in part).

[6] Group 3, relates to L selected from 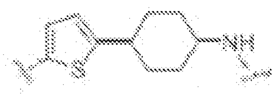 The compound of formula (I) or a pharmaceutically acceptable salt thereof, a pharmaceutical composition thereof, and a pharmaceutical use thereof, comprising claims 1 (in part), 3 and 4 (in part), 6-8 (in part), and 10-16 (in part).

[7] Group 4, relates to L selected from 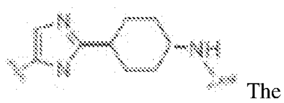 The compound of formula (I) or a pharmaceutically acceptable salt thereof, a pharmaceutical composition thereof, and a pharmaceutical use thereof, comprising claims 1 (in part), 3 and 4 (in part), 6-8 (in part), and 10-16 (in part).

[8] Group 5, relates to L selected from 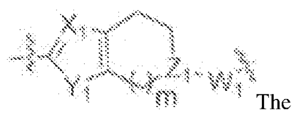 The compound of formula (I) or a pharmaceutically acceptable salt thereof, a pharmaceutical composition thereof, and a pharmaceutical use thereof, comprising claims 1 and 2 (in part), 7 (in part), 10-11 (in part), and 13-16 (in part).

Form PCT/ISA/210 (continuation of first sheet) (January 2015)

INTERNATIONAL SEARCH REPORT

International application No.
PCT/CN2021/076939

**Box No. III Observations where unity of invention is lacking (Continuation of item 3 of first sheet)**

[9]

Group 6, relates to L selected from 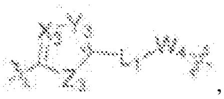, $X_3$ is CH, $Y_3$ is N, $Z_2$ is S and 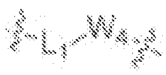 selected from 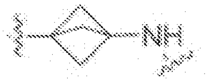 The compound of formula (I) or a pharmaceutically acceptable salt thereof, a pharmaceutical composition thereof, and a pharmaceutical use thereof, comprising claims 1 and 2 (in part), 7 (in part), 9-11 (in part), and 13-16 (in part).

[10]

Group 7, relates to L selected from 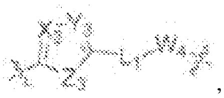, $X_3$ is CH, $Y_3$ is N, $Z_2$ is S and 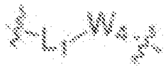 selected from 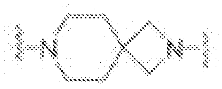 The compound of formula (I) or a pharmaceutically acceptable salt thereof, a pharmaceutical composition thereof, and a pharmaceutical use thereof, comprising claims 1 and 2 (in part), 7 (in part), 9-11 (in part), and 13-16 (in part).

[11]

Group 8, relates to L selected from 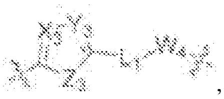, $X_3$ is CH, $Y_3$ is N, $Z_2$ is S and 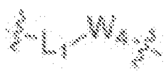 selected from 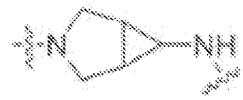 The compound of formula (I) or a pharmaceutically acceptable salt thereof, a pharmaceutical composition thereof, and a pharmaceutical use thereof, comprising claims 1 and 2 (in part), 7 (in part), 9-11 (in part), and 13-16 (in part).

[12]

Group 9, relates to L selected from 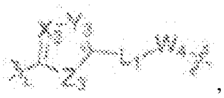, $X_3$ is CH, $Y_3$ is N, $Z_2$ is S and 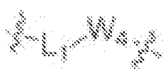 selected from 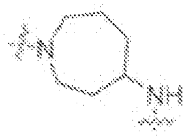 The compound of formula (I) or a pharmaceutically acceptable salt thereof, a pharmaceutical composition thereof, and a pharmaceutical use thereof, comprising claims 1 and 2 (in part), 7 (in part), 9-11 (in part), and 13-16 (in part).

[13]

Group 10, relates to L selected from 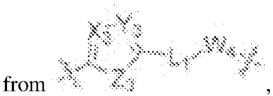, $X_3$ is CH, $Y_3$ is N, $Z_2$ is S and 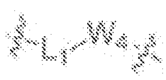 selected from 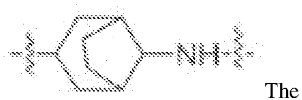 The compound of formula (I) or a pharmaceutically acceptable salt thereof, a pharmaceutical composition thereof, and or an alternate isomer thereof, comprising claims 1 and 2 (in part), 7 (in part), 9-11 (in part), 13-16 (in part).

INTERNATIONAL SEARCH REPORT

International application No.
PCT/CN2021/076939

**Box No. III Observations where unity of invention is lacking (Continuation of item 3 of first sheet)**

[14]

Group 11, relates to L selected from 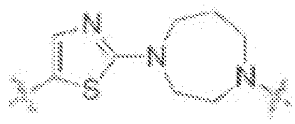 The compound of formula (I) or a pharmaceutically acceptable salt thereof, a pharmaceutical composition thereof, and a pharmaceutical use thereof, comprising claims 1 (in part), 7 (in part), 10 and 11 (in part), and 13-16 (in part).

[15]

Group 12, relates to L selected from 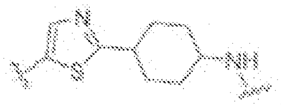, and $R^4$, $R^5$ and the atoms attached thereto collectively form 5- to 6-membered heteroaryl compounds of formula (I) or a pharmaceutically acceptable salt thereof, a pharmaceutical composition thereof, and a pharmaceutical use thereof, comprising claims 1 (in part), 3-5 (in part), 10-16 (in part).

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Group 1, relates to a compound of formula (I) or a pharmaceutically acceptable salt thereof, a pharmaceutical composition thereof, and a pharmaceutical use thereof, selected from the thiazolyl-cyclohexyl-NH- structure of L and in which R4 and R5 do not form a ring structure together with the atoms to which they are attached, comprising claims 1 (in part), 3-8 (in part), and 11-16 (in part).**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/CN2021/076939**

| Patent document cited in search report | Publication date (day/month/year) | Patent family member(s) | Publication date (day/month/year) |
|---|---|---|---|
| WO 2019051465 A1 | 14 March 2019 | PH 12020550079 A1 | 05 October 2020 |
| | | US 2019194182 A1 | 27 June 2019 |
| | | BR 112020004828 A2 | 01 December 2020 |
| | | US 2019077799 A1 | 14 March 2019 |
| | | US 10590122 B2 | 17 March 2020 |
| | | EP 3681884 A1 | 22 July 2020 |
| | | SG 11202002069W A | 29 April 2020 |
| | | TW 201917121 A | 01 May 2019 |
| | | AU 2018328818 A1 | 02 April 2020 |
| | | US 10336746 B1 | 02 July 2019 |
| | | CN 111542521 A | 14 August 2020 |
| | | KR 20200105808 A | 09 September 2020 |
| | | JP 2020533329 A | 19 November 2020 |
| | | CA 3075062 A1 | 14 March 2019 |
| | | IL 273156 D0 | 30 April 2020 |
| | | US 2020216437 A1 | 09 July 2020 |
| WO 2019014315 A1 | 17 January 2019 | CN 111263756 A | 09 June 2020 |
| | | US 2020129484 A1 | 30 April 2020 |
| | | JP 2020527602 A | 10 September 2020 |
| | | EP 3652159 A1 | 20 May 2020 |
| | | AU 2018301381 A1 | 20 February 2020 |
| | | AU 2018301381 A2 | 05 March 2020 |
| | | CA 3069003 A1 | 17 January 2019 |
| WO 2020186006 A1 | 17 September 2020 | US 2020291014 A1 | 17 September 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010102773
- CN 202010738095
- WO 2019014315 A1
- WO 2019051465 A1


### Non-patent literature cited in the description


- **KLEIN et al.** *DNA Repair (Amst).,* 03 May 2008, vol. 7 (5), 686-693
- **TAKATA et al.** *Mol Cell Biol.,* April 2001, vol. 21 (8), 2858-2866
- **GODTHELP et al.** *Nucleic Acids Res.,* 15 May 2002, vol. 30 (10), 2172-2182
- **CONNELL et al.** *Cancer Res.,* 01 May 2004, vol. 64 (9), 3002-3005
- **MAEHR.** *J. Chem. Ed.,* 1985, vol. 62, 114-120